# EUROPEAN PATENT APPLICATION

(11) **EP 4 420 682 A1**
(43) Date of publication of application: **28.08.2024**
(21) Application number: 22880341.7
(22) Date of filing: 12.10.2022
(51) Int. Cl.: A61K 47/55, A61K 47/68, A61K 47/64, C07K 16/30, A61P 35/00, A61P 35/02

(54) **HIGHLY-STABLE TARGETED LINKER-DRUG CONJUGATE**

(30) Priority: 12.10.2021 CN 202111188826; 03.03.2022 CN 202210211154; 25.04.2022 CN 202210439413; 01.06.2022 CN 202210618358
(71) Applicant: Chengdu Scimount Pharmatech Co., Ltd., Chengdu, Sichuan 610041 (CN)
(72) Inventor: HUANG, Jinkun, Chengdu, Sichuan 610041 (CN); WU, Chenglong, Chengdu, Sichuan 610041 (CN); FENG, Chaoyang, Chengdu, Sichuan 610041 (CN)
(74) Representative: Altmann Stößel Dick Patentanwälte PartG mbB
(86) International application number: PCT/CN2022/124857
(87) International publication number: WO 2023/061405

(57) **Abstract**

Disclosed is a targeted linker-drug conjugate having high stability and an excellent anti-tumor effect, belonging to the field of drugs. Specifically, provided are a drug link assembly unit having a specific linker structure, and a targeted linker-drug conjugate formed by connecting the drug link assembly unit to an antibody. The targeted linker-drug conjugate having the specific linker structure is low in polymer content and naked antibody percentage and proper in DAR value, exhibits excellent plasma stability, storage stability and anti-tumor effect, and has a wide application prospect in preparation of drugs for preventing and/or treating tumors.

## Description

### TECHNICAL FIELD

The present invention belongs to the field of drugs, and specifically relates to a highly stable targeted linker-drug conjugate.

### BACKGROUND

Antibody-drug conjugates (ADC) can selectively deliver drugs to cancer cells and kill the cancer cells, but have little impact on normal cells, and usher in a new era of tumor therapy. A plurality of drugs in ADC have obtained FDA approval for marketing, such as Mylotarg formed by connecting a CD33 antibody to calicheamicin, Adcetris formed by connecting a CD30 antibody to auristatin E and used for treating Hodgkin's lymphoma and undifferentiated large cell lymphoma patients, DS-8201 formed by connecting a Her2 antibody to a camptothecin derivative Dxd and used for treating HER2 positive breast cancer patients, and Sacituzumab govitecan targeting a TROP-2 antigen (also referred to as epithelial glycoprotein 1, EGP-1) and used for triple negative breast cancer.

So far, the drugs recognized by the FDA in ADC mainly target DNA or microtubule proteins. As anti-tumor small molecule compounds, known camptothecin derivatives (such as SN-38, Dxd, and Dx-8951) that inhibit DNA topoisomerase I to achieve anti-tumor effects have been confirmed to have killing effects on multiple cancer cells in vivo and in vitro, showing strong anti-tumor effects. Compounds that inhibit microtubule proteins to achieve anti-tumor effects, such as Eribulin, MMAE, MMAF, and maytansine, have been confirmed to have killing effects on multiple cancer cells in vivo and in vitro, showing strong anti-tumor effects. The structural formulas of compounds known to act on DNA or microtubule proteins are as follows:

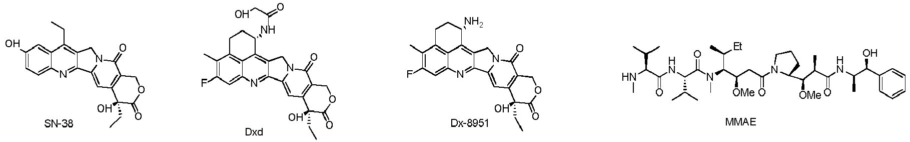

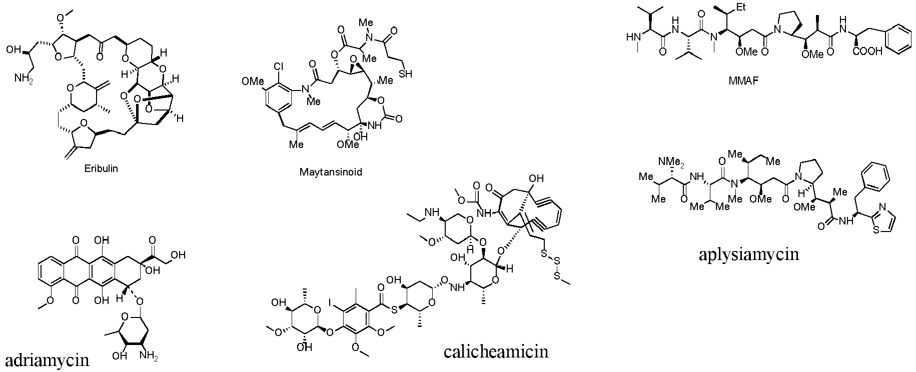

According to the most classic mechanism of action of ADC drugs, antibody-drug conjugates can specifically bind to cell surface proteins, and the produced conjugates are endocytosed by cells, thereby achieving targeted delivery of drug molecules into cells. Therefore, the concentration of intracellular drugs is directly related to the distribution density of targets on the cell surface that can be specifically recognized by antibodies. Unfortunately, the density of targets recognized by antibodies on the molecular surface is usually low, resulting in a low drug concentration in target cells. To solve this problem, a commonly used method is to increase the drug-antibody ratio (DAR) of ADC, thereby increasing the quantity of drugs entering the cells. However, according to the study by Hamblett et al. (Clin Cancer Res. 2004, 10, 7063), increasing the DAR value of ADC usually has the opposite effect as expected in pharmacokinetic exposure, where ADC molecules aggregate severely, and its stability decreases. As a result, small molecule toxins in the blood increase, leading to toxic and side effects.

Since 2013, Daiichi Sankyo Company Limited has filed many patent applications (CN201380053256.2, CN201910768778. X, CN201980061665.4, etc.), disclosing a series of antibody-drug conjugates having specific linker-toxin structures, and specifically disclosing ADCs that have the following typical structure and exhibit excellent performance. However, its stability and therapeutic effect still need to be further improved.

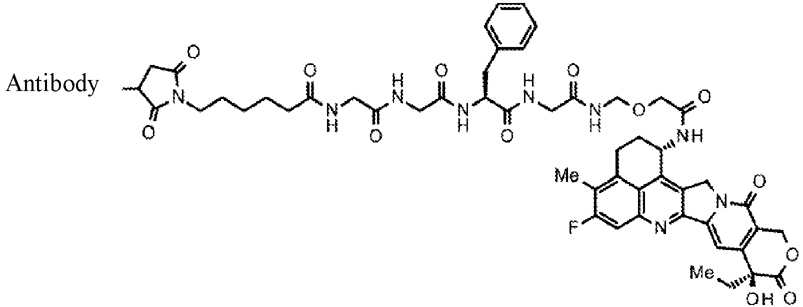

Therefore, it is of great significance to develop a drug conjugate that can significantly improve aggregation phenomenon, improve stability and therapeutic effect, and target lesion sites.

### SUMMARY

The present invention aims to provide a highly stable drug link assembly unit and a targeted linker-drug conjugate.

The present invention provides a drug link assembly unit or a stereoisomer, optical isomer, salt, or deuterated compound thereof. The drug link assembly unit consists of a linker and a drug, a linker end can be connected to a targeted linker, and the targeted linker is a substance capable of targeted binding to a lesion site; the linker has a structure represented by formula A, as shown in formula A-1: 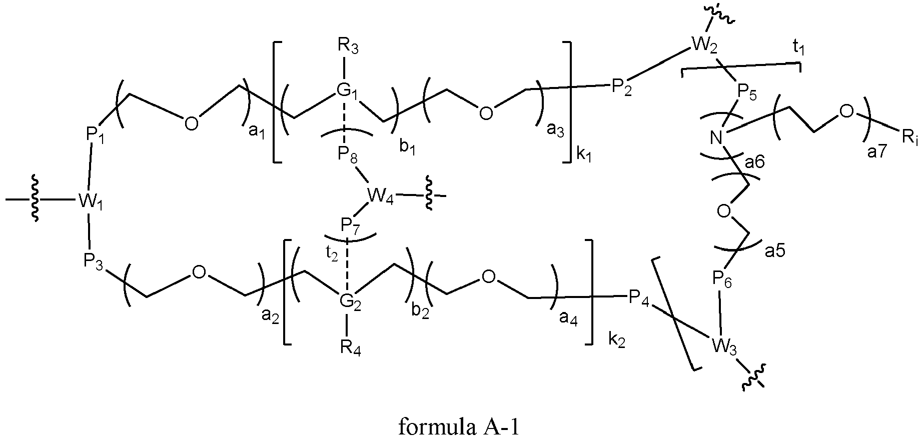
wherein W₁, W₃, and W₄ are independently selected from N, N⁺E⁻R₁, or CR₁; R₁ is selected from hydrogen, C₁₋₁₀ alkyl, or C₁₋₁₀ alkoxy; N⁺ is a monovalent nitrogen cation, and E⁻ is a monovalent anion;
W₂ is selected fromN, N⁺E⁻R₂, or CR₂; R₂ is selected from hydrogen, C₁₋₁₀ alkyl, or C₁₋₁₀ alkoxy;
P₁, P₂, P₃, P₄, P₅, P₆, P₇, and P₈ are independently none or selected from C₁₋₁₀ alkylene that is unsubstituted or substituted by 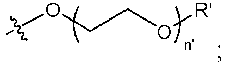 n' is an integer from 1 to 50; R' is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R_{5'})₂; R_{5'} is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
G₁ and G₂ are independently selected from N or N⁺B⁻R₇; N⁺ is a monovalent nitrogen cation, and B⁻ is a monovalent anion; R₇ is selected from C₁₋₁₀ alkyl, C₁₋₁₀ alkoxy, or 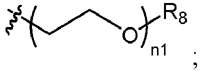 n1 is an integer from 1 to 50; R₈ is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₉)₂; R₈ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
R₃ and R₄ are independently selected from hydrogen, C₁₋₁₀ alkyl, C₁₋₁₀ alkoxy, or 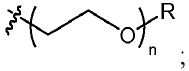 n is an integer from 1 to 50; R is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₅)₂; R₅ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl; or R₃ and R₄ are connected into 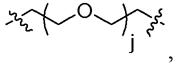 and j is an integer selected from 0 to 10;
t₁ is selected from 0 or 1;
------ represents none or a covalent bond; when ------ represents none, t₂ is 0; when ------ represents the covalent bond, t₂ is 1;
t₁ and t₂ are not simultaneously 1;
k₁ and k₂ are integers independently selected from 0 to 10;
a₁, a₂, a₃, a₄, a₅, and a₆ are integers independently selected from 0 to 10;
a₇ is an integer selected from 1 to 50;
Rᵢ is selected from hydrogen or C₁₋₁₀ alkyl;
b₁ and b₂ are integers independently selected from 0 to 10.

The present invention further provides a drug link assembly unit or a stereoisomer, optical isomer, salt, or deuterated compound thereof. The drug link assembly unit consists of a linker and a drug, a linker end can be connected to a targeted linker, and the targeted linker is a substance capable of targeted binding to a lesion site; the linker has a structure represented by formula A, as shown in formula A-1: 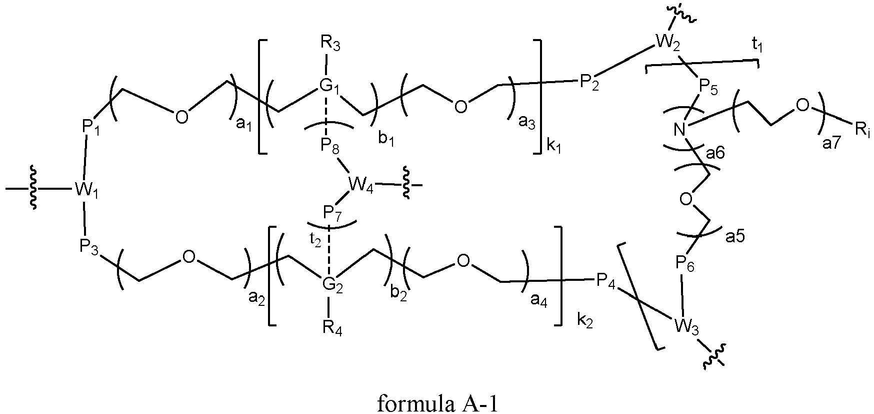
wherein W₁, W₃, and W₄ are independently selected from N, N⁺E⁻R₁, or CR₁; R₁ is selected from hydrogen, C₁₋₁₀ alkyl, or C₁₋₁₀ alkoxy; N⁺ is a monovalent nitrogen cation, and E⁻ is a monovalent anion;
W₂ is selected fromN, N⁺E⁻R₂, or CR₂; R₂ is selected from hydrogen, C₁₋₁₀ alkyl, or C₁₋₁₀ alkoxy;
P₁, P₂, P₃, P₄, P₅, P₆, P₇, and P₈ are independently none or selected from C₁₋₁₀ alkylene that is unsubstituted or substituted by 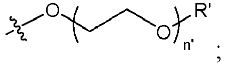 n' is an integer from 1 to 50; R' is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R_{5'})₂; R_{5'} is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
G₁ and G₂ are independently selected from N or N⁺B⁻R₇; N⁺ is a monovalent nitrogen cation, and B⁻ is a monovalent anion; R₇ is selected from C₁₋₁₀ alkyl, C₁₋₁₀ alkoxy, or 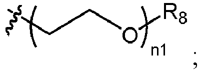 n1 is an integer from 1 to 50; Rs is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₉)₂; R₈ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
R₃ and R₄ are independently selected from hydrogen, C₁₋₁₀ alkyl, C₁₋₁₀ alkoxy, or 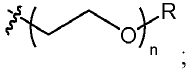 n is an integer from 1 to 50; R is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₅)₂; R₅ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl; or R₃ and R₄ are connected into 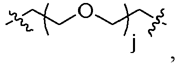 and j is an integer selected from 0 to 10;
t₁ is selected from 0 or 1;
------ represents none or a covalent bond; when ------ represents none, t₂ is 0; when ------ represents the covalent bond, t₂ is 1;
t₁ and t₂ are not simultaneously 1;
k₁ and k₂ are integers independently selected from 0 to 10;
a₁, a₂, a₃, a₄, a₅, and a₆ are integers independently selected from 0 to 10;
a₇ is an integer selected from 1 to 50;
Rᵢ is selected from hydrogen or C₁₋₁₀ alkyl;
b₁ and b₂ are integers independently selected from 0 to 10;
when W_{I} is N, W₂ is N, a₁, a₂, a₃, a₄, b₁, b₂, t₁, and t₂ are simultaneously 0,represents none, and P₂ and P₄ are none, P₁ is not C₁₋₂ alkylene, and P₃ is not C₁₋₂ alkylene; and
when W₁ is N, W₂ is N, W₃ is N, a₁, a₂, a₃, a₄, b₁, b₂, a₅, a₆, and t₂ are simultaneously 0, ------ represents none, and P₂ and P₄ are none, P₁ is not C₁₋₂ alkylene, P₃ is not C₁₋₂ alkylene, P₅ is not C₁₋₂ alkylene, and P₆ is not C₁₋₂ alkylene.

Further, the structure represented by formula A is as shown in formula B-1: 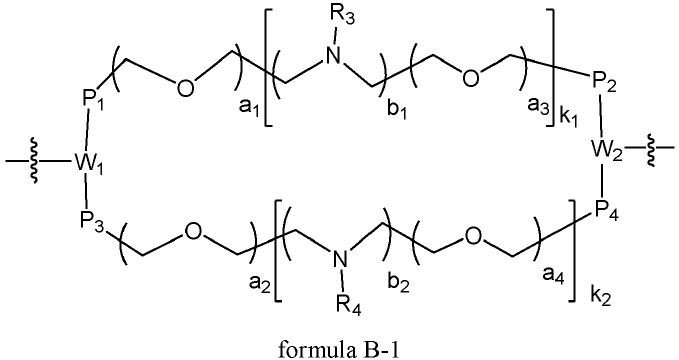
wherein W₁ is selected from N, N⁺E⁻R₁, or CR₁; R₁ is selected from hydrogen, C₁₋₁₀ alkyl, or C₁₋₁₀ alkoxy; N⁺ is a monovalent nitrogen cation, and E⁻ is a monovalent anion;
W₂ is selected from N or CR₂; R₂ is selected from hydrogen, C₁₋₁₀ alkyl, or C₁₋₁₀ alkoxy;
P₁, P₂, P₃, and P₄ are independently none or selected from C₁₋₁₀ alkylene that is unsubstituted or substituted by 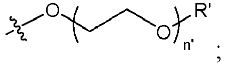 n' is an integer from 1 to 50; R' is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R_{5'})₂; R_{5'} is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
R₃ and R₄ are independently selected from hydrogen, C₁₋₁₀ alkyl, C₁₋₁₀ alkoxy, or 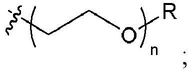 n is an integer from 1 to 50; R is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₅)₂; R₅ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl; or R₃ and R₄ are connected into 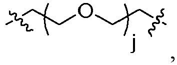 and j is an integer selected from 0 to 10;
k₁ and k₂ are integers independently selected from 0 to 10;
a₁, a₂, a₃, and a₄ are integers independently selected from 0 to 10;
b₁ and b₂ are integers independently selected from 0 to 10; and
when W₁ is N, W₂ is N, a₁, a₂, a₃, a₄, b₁, and b₂ are simultaneously 0, and P₂ and P₄ are none, P₁ is not none or C₁₋₂ alkylene, and P₃ is not none or C₁₋₂ alkylene.

Further, the structure represented by formula A is as shown in formula B-2: 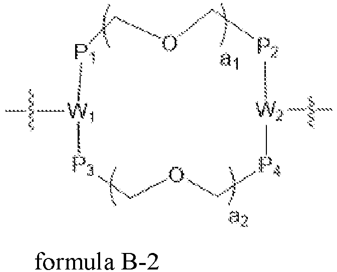
wherein W₁ is selected from N or CR₁, and W₂ is selected from N or CR₂; R₁ is hydrogen or C₁₋₃ alkyl, and R₂ is hydrogen or C₁₋₃ alkyl;
P₁, P₂, P₃, and P₄ are independently selected from none or C₁₋₂alkylene;
a₁ and a₂ are integers independently selected from 1 to 6;
preferably, W₁ is selected from N or CR₁, and W₂ is selected from N or CR₂; R₁ is hydrogen, and R₂ is hydrogen;
P₁, P₂, P₃, and P₄ are independently selected from none or methylene; and
a₁ and a₂ are integers independently selected from 1 to 4.

Further, the structure represented by formula A is as shown in formula B-3: 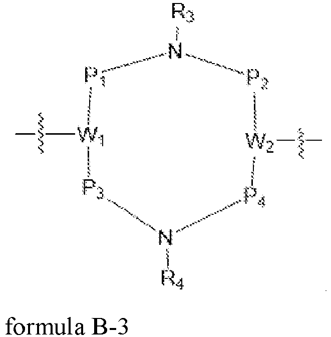
wherein W₁ is selected from N or CR₁, and W₂ is selected from N or CR₂; R₁ is hydrogen or C₁₋₃ alkyl, and R₂ is hydrogen or C₁₋₃ alkyl;
P₁, P₂, P₃, and P₄ are independently selected from C₁₋₃ alkylene;
R₃ and R₄ are independently selected from hydrogen, C₁₋₃ alkyl, C₁₋₃ alkoxy, or 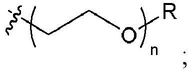 n is an integer from 1 to 30, and R is selected from hydrogen or methyl; or R₃ and R₄ are connected into 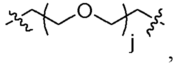 and j is an integer selected from 0 to 5;
preferably, W₁ is selected from N, and W₂ is selected from N;
P₁, P₂, P₃, and P₄ are independently selected from C₂ alkylene; and
R₃ and R₄ are independently selected from 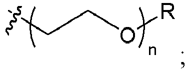 n is an integer from 1 to 30, and R is selected from hydrogen or methyl; or R₃ and R₄ are connected into 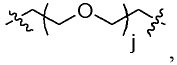 and j is 1.

Further, the structure represented by formula A is as shown in formula B-4: 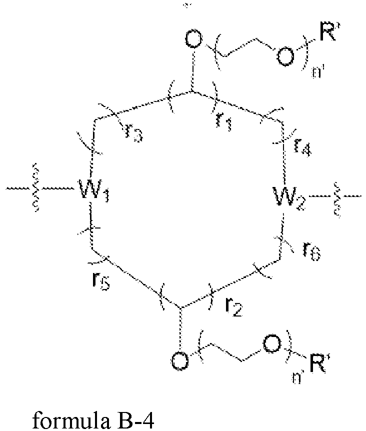
wherein W₁ is selected from N or N⁺E⁻R₁, and W₂ is selected from N or N⁺E⁻R₂; R₁ is selected from C₁₋₃ alkyl, R₂ is selected from C₁₋₃ alkyl, N⁺ is a monovalent nitrogen cation, and E⁻ is a monovalent anion;
r₃, r₄, r₅, and r₆ are integers independently selected from 1 to 3;
r₁ and r₂ are independently selected from 0 or 1;
when W₁ is N and W₂ is N, r1 and r2 are not simultaneously 0;
n' is an integer from 1 to 40, and R' is selected from hydrogen or methyl;
preferably, W₁ is selected from N or N⁺E⁻R₁, and W₂ is selected from N or N⁺E⁻R₂; R₁ is methyl, R₂ is methyl, N⁺ is a monovalent nitrogen cation, and E⁻ is a monovalent anion;
r₃, r₄, r₅, and r₆ are independently selected from 1;
r₁ and r₂ are independently selected from 0 or 1;
when W₁ is N and W₂ is N, r1 and r2 are not simultaneously 0; and
n' is an integer from 1 to 30, and R' is selected from hydrogen or methyl.

Further, the structure represented by formula A is as shown in formula C-1: 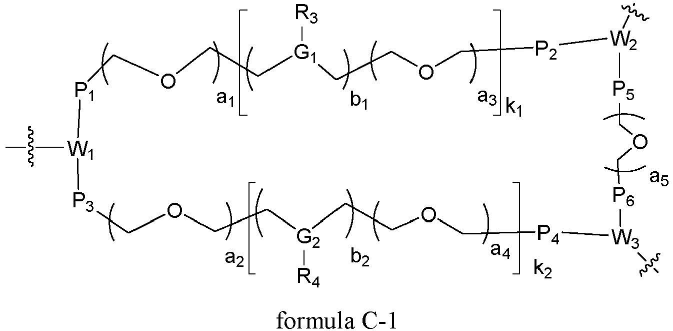
wherein W₁ and W₃ are independently selected from N, N⁺E⁻R₁, or CR₁; R₁ is selected from hydrogen, C₁₋₁₀ alkyl, or C₁₋₁₀ alkoxy; N⁺ is a monovalent nitrogen cation, and E⁻ is a monovalent anion;
W₂ is selected from N or CR₂; R₂ is selected from hydrogen, C₁₋₁₀ alkyl, or C₁₋₁₀ alkoxy;
P₁, P₂, P₃, P₄, P₅, and P₆ are independently none or selected from C₁₋₁₀ alkylene that is unsubstituted or substituted by 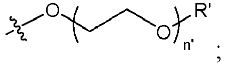 n' is an integer from 1 to 50; R' is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R_{5'})₂; R_{5'} is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
G₁ and G₂ are independently selected from N or N⁺B⁻R₇; N⁺ is a monovalent nitrogen cation, and B⁻ is a monovalent anion; R₇ is selected from C₁₋₁₀ alkyl, C₁₋₁₀ alkoxy, or 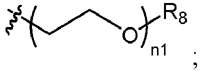 n1 is an integer from 1 to 50; R₈ is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₉)₂; R₈ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
R₃ and R₄ are independently selected from hydrogen, C₁₋₁₀ alkyl, C₁₋₁₀ alkoxy, or 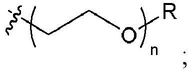 n is an integer from 1 to 50; R is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₅)₂; R₅ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl; or R₃ and R₄ are connected into 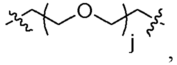 and j is an integer selected from 0 to 10;
k₁ and k₂ are integers independently selected from 0 to 10;
a₁, a₂, a₃, a₄, and as are integers independently selected from 0 to 10; and
b₁ and b₂ are integers independently selected from 0 to 10.

Further, the structure represented by formula A is as shown in formula C-2: 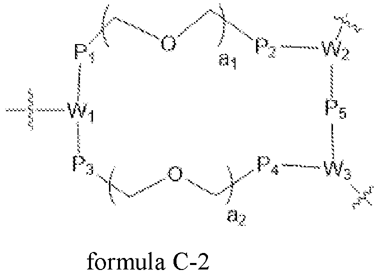
wherein W₁ is N, W₂ is N, and W₃ is N;
P₁, P₂, P₃, P₄, and P₅ are independently selected from C₁₋₃ alkylene;
a₁ and a₂ are integers independently selected from 1 to 6;
preferably, W₁ is N, W₂ is N, and W₃ is N;
P₁, P₂, P₃, P₄, and P₅ are independently selected from C₁₋₂ alkylene; and
a₁ and a₂ are integers independently selected from 1 to 4.

Further, the structure represented by formula A is as shown in formula C-3: 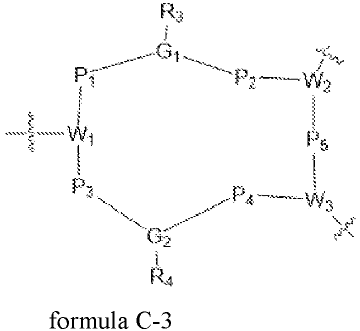
wherein W₁ is N, W₂ is N, and W₃ is N;
P₁, P₂, P₃, P₄, and P₅ are independently selected from C₁₋₃ alkylene;
G₁ and G₂ are independently selected from N or N⁺B⁻R₇; N⁺ is a monovalent nitrogen cation, and B⁻ is a monovalent anion; R₇ is selected from C₁₋₁₀ alkyl, C₁₋₁₀ alkoxy, or 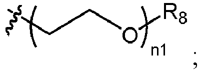 n1 is an integer from 1 to 50; R₈ is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₉)₂; R₉ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
R₃ and R₄ are independently selected from hydrogen, C₁₋₃ alkyl, C₁₋₃ alkoxy, or 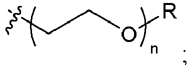 n is an integer from 1 to 30, and R is selected from hydrogen or methyl; or R₃ and R₄ are connected into 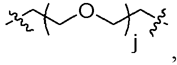 and j is an integer selected from 0 to 5;
preferably, W₁ is N, W₂ is N, and W₃ is N;
P₁, P₂, P₃, P₄, and P₅ are independently selected from C₂ alkylene; and
R₃ and R₄ are independently selected from 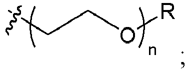 n is an integer from 1 to 30, and R is selected from hydrogen or methyl; or R₃ and R₄ are connected into 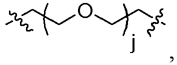 and j is 1.

Further, the structure represented by formula A is as shown in formula C-4: 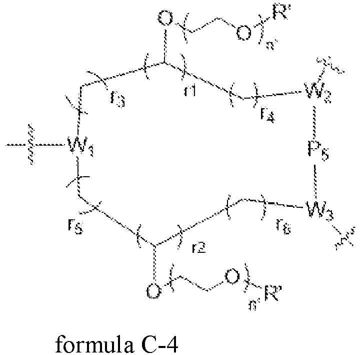
wherein W₁ is N, W₂ is N, and W₃ is N;
r₃, r₄, r₅, and r₆ are integers independently selected from 1 to 3;
P₅ is selected from C₁₋₃ alkylene;
r₁ and r₂ are independently selected from 0 or 1;
n' is an integer from 1 to 40, and R' is selected from hydrogen or methyl;
preferably, the structure represented by formula A is as shown in formula C-5: 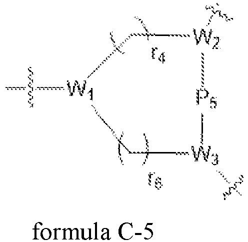
W₁ is N, W₂ is N, and W₃ is N;
r₄ and r₆ are integers independently selected from 1 to 3; and
P₅ is selected from C₁₋₃ alkylene.

Further, the structure represented by formula A is as shown in formula D-1: 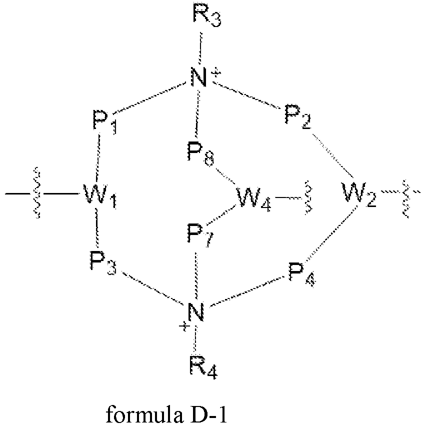
wherein W₁ and W₄ are independently selected from N, N⁺E⁻R₁, or CR₁; R₁ is selected from hydrogen, C₁₋₁₀ alkyl, or C₁₋₁₀ alkoxy; N⁺ is a monovalent nitrogen cation, and E⁻ is a monovalent anion;
W₂ is selected from N or CR₂; R₂ is selected from hydrogen, C₁₋₁₀ alkyl, or C₁₋₁₀ alkoxy;
P₁, P₂, P₃, P₄, P₇, and P₈ are independently selected from C₁₋₁₀ alkylene that is unsubstituted or substituted by 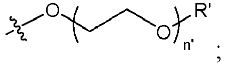 n' is an integer from 1 to 50; R' is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R_{5'})₂; R_{5'} is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
R₃ and R₄ are independently selected from hydrogen, C₁₋₁₀ alkyl, C₁₋₁₀ alkoxy, or 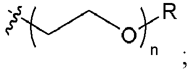 n is an integer from 1 to 50; R is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₅)₂; R₅ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl; or R₃ and R₄ are connected into 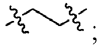
preferably, W₁ is N, W₂ is N, and W₄ is N;
P₁, P₂, P₃, P₄, P₆, and P₇ are independently selected from C₁₋₂ alkylene; and
R₃ and R₄ are independently selected from 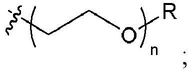 n is an integer from 1 to 30, and R is selected from hydrogen or methyl.

Further, the structure represented by formula A is as follows: 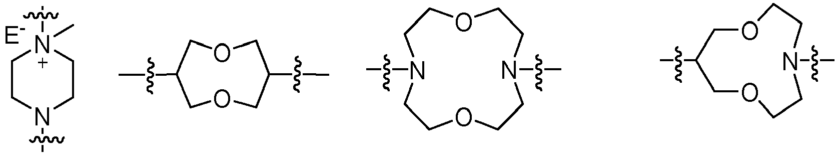 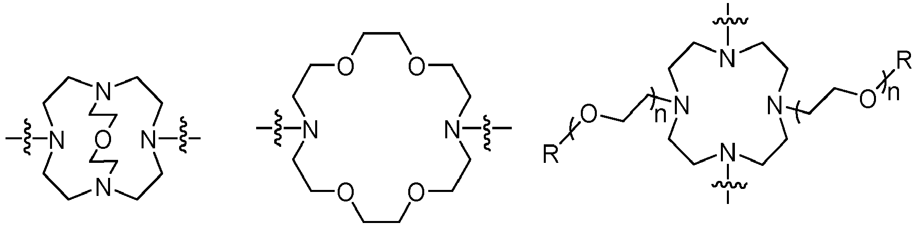 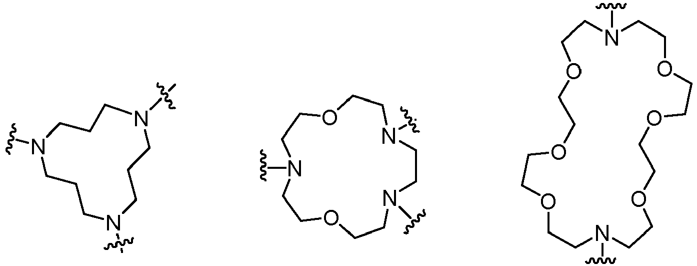 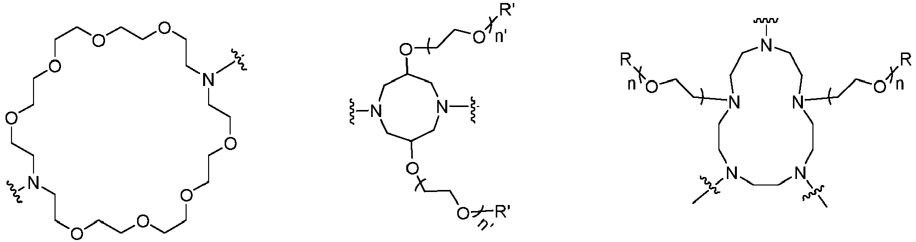 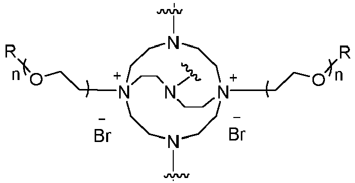 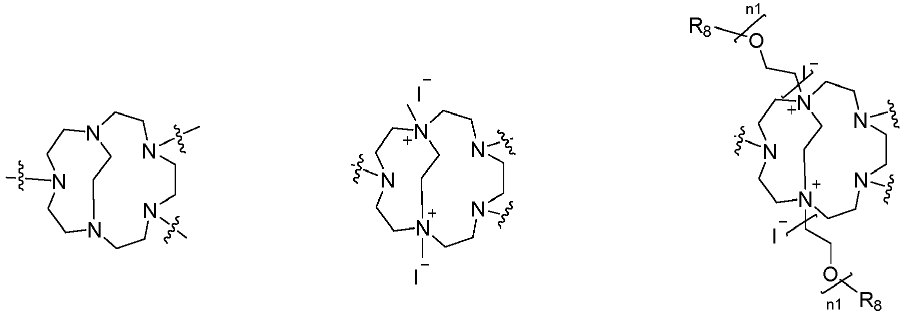 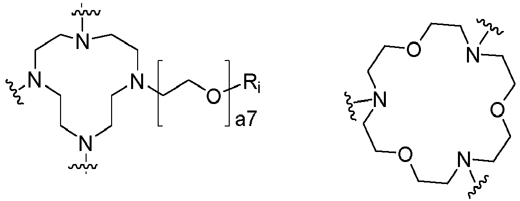 wherein n is an integer selected from 1 to 24, R is hydrogen or methyl, n' is an integer selected from 1 to 24, R' is hydrogen or methyl, a7 is an integer selected from 1 to 24, Rᵢ is hydrogen or methyl, n1 is an integer selected from 1 to 24, R₈ is hydrogen or methyl, and E is halogen.

The present invention further provides a drug link assembly unit or a stereoisomer, optical isomer, salt, or deuterated compound thereof. The drug link assembly unit consists of a linker and a drug, a linker end can be connected to a targeted linker, and the targeted linker is a substance capable of targeted binding to a lesion site; the linker has a structure represented by formula A, as shown in formula E-0: 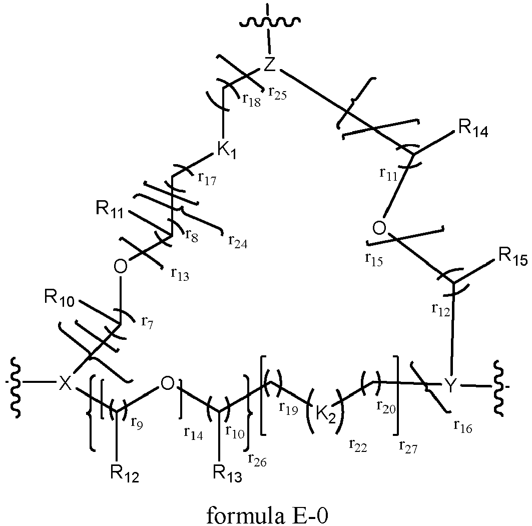
wherein X, Y, and Z are independently selected from N, N⁺B₀⁻R₀, or CH; R₁₀, R₁₁, R₁₂, R₁₃, R₁₄, and R₁₅ are independently selected from hydrogen or 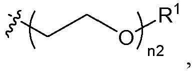 n2 is an integer from 1 to 40, and R¹ is hydrogen or methyl; N⁺ is a monovalent nitrogen cation, B₀⁻ is a monovalent anion, and R₀ is C₁₋₅ alkyl;
r₇, r₈, r₉, r₁₀, r₁₃, and r₁₄ are integers independently selected from 1 to 5; r₁₁, r₁₂, and r₁₅ are integers independently selected from 0 to 5; r₁₆ is 0 or 1;
r₁₇, r₁₈, r₁₉, and r₂₀ are integers independently selected from 1 to 3;
r₂₂ is selected from 0 or 1;
r₂₄ is selected from 0 or 1, r₂₅ is selected from 0 or 1, and r₂₄ and r₂₅ are not simultaneously 0;
r₂₆ is selected from 0 or 1, r₂₇ is selected from 0 or 1, and r₂₆ and r₂₇ are not simultaneously 0;
K₁ is selected from NR₁₆, N⁺R₁₆B⁻R₁₈, or CHR₂₀; K₂ is selected from NR₁₇, N⁺R₁₇B⁻R₁₈, or CHR₂₁;
N⁺ is a monovalent nitrogen cation, B⁻ is a monovalent anion, and R₁₈ is selected from C₁₋₃ alkyl, C₁₋₃ alkoxy, or 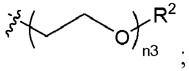 n3 is an integer from 1 to 50, and R² is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₁₉)₂; R₁₉ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
R₁₆ and R₁₇ are independently selected from C₁₋₃ alkyl, C₁₋₃ alkoxy, or 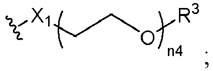 n4 is an integer from 1 to 40, and R³ is selected from hydrogen or methyl; or R₁₆ and R₁₇ are connected into 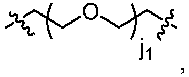 and j₁ is an integer selected from 0 to 5; X₁ is -CO-, -CONH-, or none; and
R₂₀ and R₂₁ are independently selected from 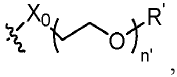 n' is an integer from 1 to 40, R' is selected from hydrogen or methyl, and X₀ is O or none.

Further, the structure represented by formula A is as shown in formula E-1: 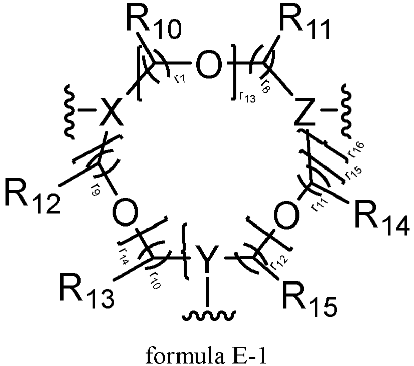
wherein X, Y, and Z are independently selected from N, N⁺B₀⁻R₀, or CH; R₁₀, R₁₁, R₁₂, R₁₃, R₁₄, and R₁₅ are independently selected from hydrogen or 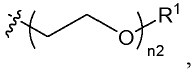 n2 is an integer from 1 to 40, and R¹ is hydrogen or methyl; N⁺ is a monovalent nitrogen cation, B₀⁻ is a monovalent anion, and R₀ is C₁₋₅ alkyl; and
r₇, r₈, r₉, r₁₀, r₁₃, and r₁₄ are integers independently selected from 1 to 5; r₁₁, r₁₂, and r₁₅ are integers independently selected from 0 to 5; and r₁₆ is 0 or 1.

Further, R₁₀, R₁₁, R₁₂, R₁₃, R₁₄, and R₁₅ are all hydrogen.

Further, the structure represented by formula A is as shown in formula E-1-a or E-1-a': 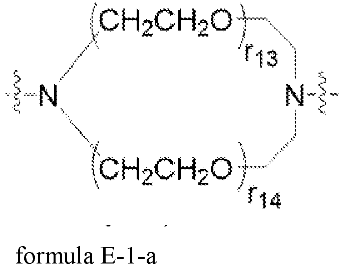 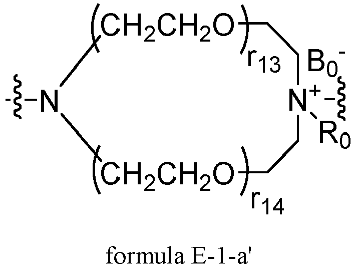
wherein r₁₃ and r₁₄ are integers independently selected from 1 to 5, preferably integers from 1 to 4; and
B₀⁻ is a monovalent anion, and R₀ is C₁₋₅ alkyl.

Further, the structure represented by formula A is as shown in formula E-1-b: 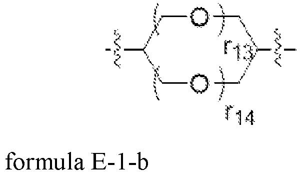 wherein r₁₃ and r₁₄ are integers independently selected from 1 to 5.

Further, the structure represented by formula A is as shown in formula E-1-c: 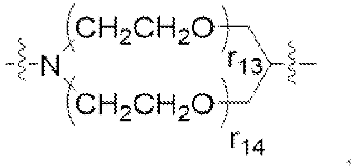 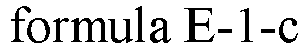 wherein r₁₃ and r₁₄ are integers independently selected from 1 to 5.

Further, the structure represented by formula A is as shown in formula E-1-d: 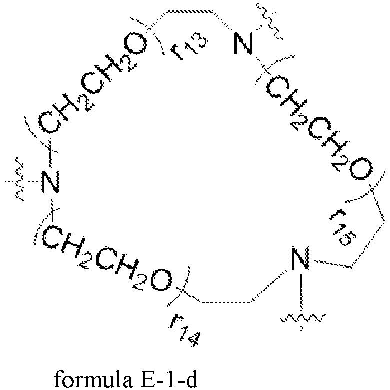 wherein r₁₃ and r₁₄ are integers independently selected from 1 to 5, and r₁₅ is an integer selected from 0 to 5.

Further, the structure represented by formula A is as shown in formula F-1: 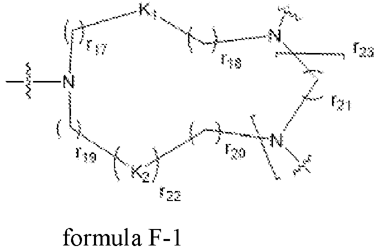
wherein r₁₇, r₁₈, r₁₉, r₂₀, and r₂₁ are integers independently selected from 1 to 3;
r₂₂ is selected from 0 or 1;
r₂₃ is selected from 0 or 1;
K₁ is selected from NR₁₆, N⁺R₁₆B⁻R₁₈, or CHR₂₀; K₂ is selected from NR₁₇, N⁺R₁₇B⁻R₁₈, or CHR₂₁;
N⁺ is a monovalent nitrogen cation, B⁻ is a monovalent anion, and R₁₈ is selected from C₁₋₃ alkyl, C₁₋₃ alkoxy, or 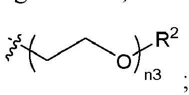 n3 is an integer from 1 to 50, and R² is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₁₉)₂; R₁₉ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
R₁₆ and R₁₇ are independently selected from C₁₋₃ alkyl, C₁₋₃ alkoxy, or 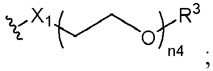 n4 is an integer from 1 to 40, and R³ is selected from hydrogen or methyl; or R₁₆ and R₁₇ are connected into 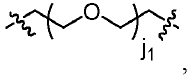 and j₁ is an integer selected from 0 to 5; X₁ is -CO-, -CONH-, or none; and
R₂₀ and R₂₁ are independently selected from 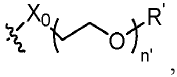 n' is an integer from 1 to 40, R' is selected from hydrogen or methyl, and X₀ is O or none.

Further, the structure represented by formula A is as shown in formula F-1-a: 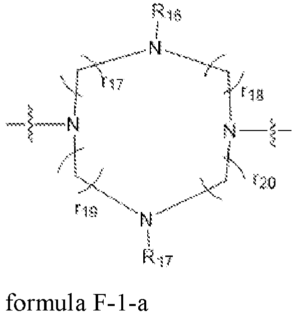
wherein r₁₇, r₁₈, r₁₉, and r₂₀ are integers independently selected from 1 to 3; and
R₁₆ and R₁₇ are independently selected from C₁₋₃ alkyl, C₁₋₃ alkoxy, or 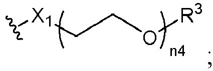 n4 is an integer from 1 to 40, preferably an integer from 9 to 16, and R³ is selected from hydrogen or methyl; or R₁₆ and R₁₇ are connected into 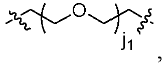 and j 1 is an integer selected from 0 to 5; X1 is -CO-, -CONH-, or none.

Further, the structure represented by formula A is as shown in formula F-1-b: 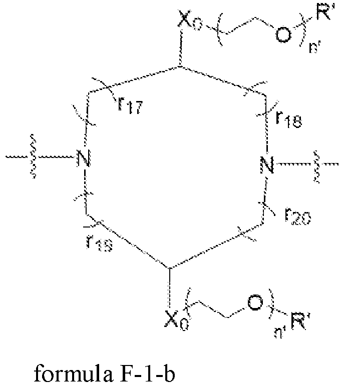
wherein r₁₇, ris, r₁₉, and r₂₀ are integers independently selected from 1 to 3;
n' is an integer from 1 to 40, preferably an integer from 9 to 16, and R' is selected from hydrogen or methyl; and
X₀ is O or none.

Further, the structure represented by formula A is as shown in formula F-1-c: 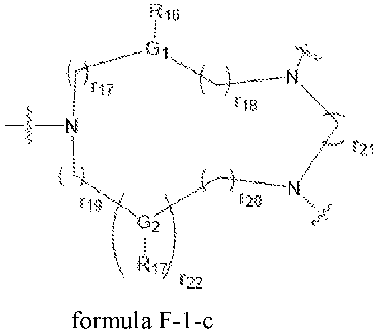
wherein r₁₇, ris, r₁₉, r₂₀, and r₂₁ are integers independently selected from 1 to 3;
r₂₂ is selected from 0 or 1;
G₁ is selected from Nor N⁺B⁻R₁₈; G₂ is selected from N or N⁺B⁻R₁₈;
N⁺ is a monovalent nitrogen cation, B- is a monovalent anion, and R₁₈ is selected from C₁₋₃ alkyl, C₁₋₃ alkoxy, or 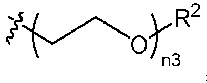 ; n3 is an integer from 1 to 50, preferably an integer from 3 to 16, and R² is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₁₉)₂; R₁₉ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl; and
R₁₆ and R₁₇ are independently selected from C₁₋₃ alkyl, C₁₋₃ alkoxy, or 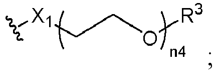 n4 is an integer from 1 to 40, preferably an integer from 9 to 16, and R³ is selected from hydrogen or methyl; or R₁₆ and R₁₇ are connected into 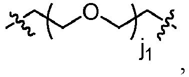 and j 1 is an integer selected from 0 to 5; X1 is -CO-, -CONH-, or none.

Further, the structure represented by formula A is as follows: 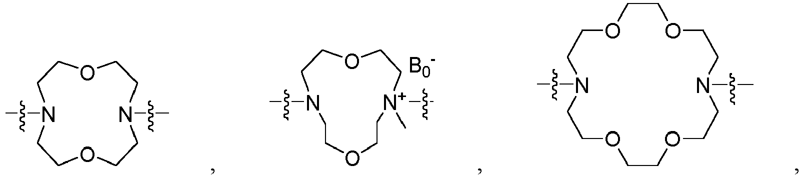 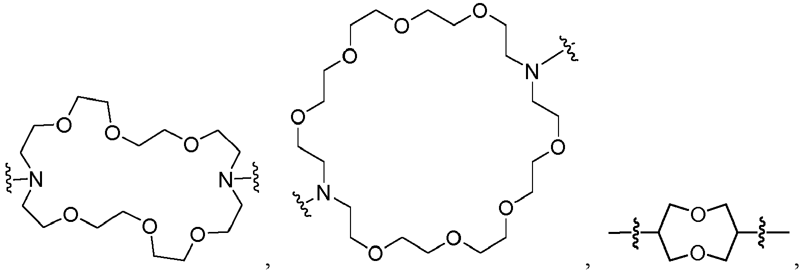 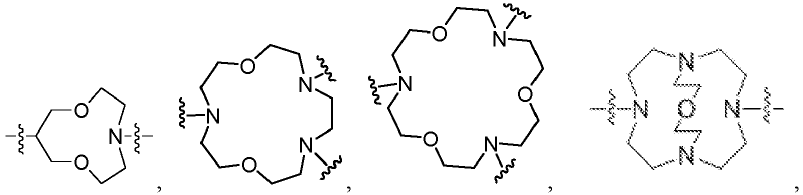 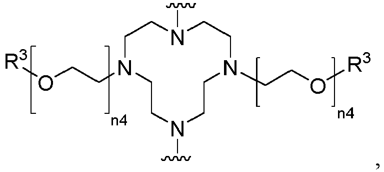 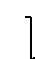 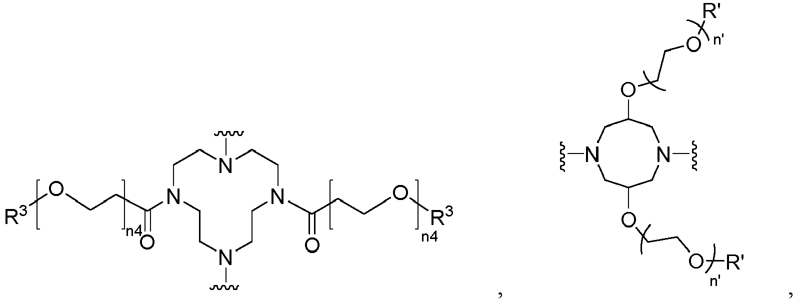 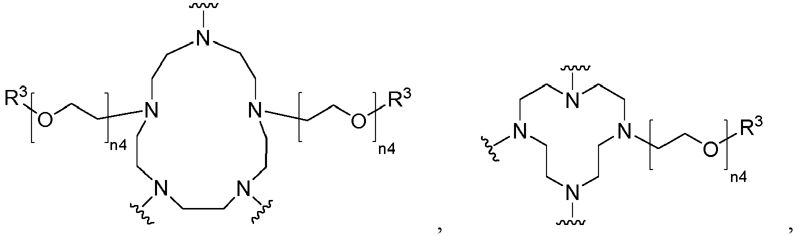 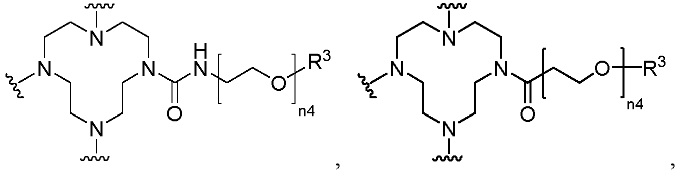 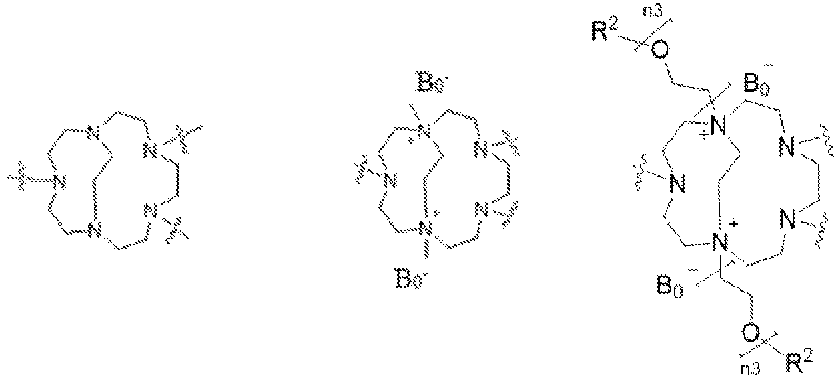 n4 is an integer selected from 1 to 24, preferably an integer from 9 to 16; R³ is hydrogen or methyl; n' is an integer selected from 1 to 24, preferably an integer from 9 to 16; R' is hydrogen or methyl; n3 is an integer selected from 1 to 24, preferably an integer from 3 to 16; R² is hydrogen or methyl; and B₀⁻ is a monovalent anion.

Further, the structure of the linker is as shown in formula G-1 or G-2: 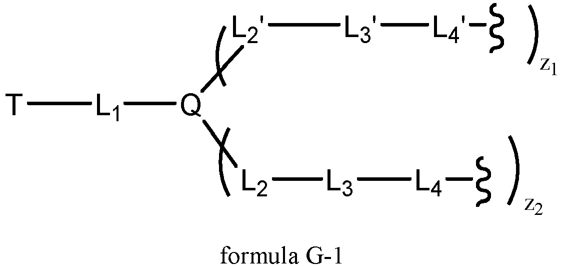 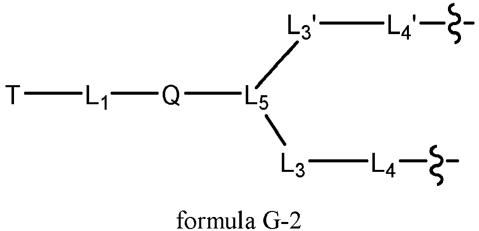
wherein T is a tying group, which can be connected to the targeted linker; the targeted linker is a substance capable of targeted binding to a lesion site;
L₁ is none or a breakable or unbreakable linker fragment;
L₂ is none or a breakable or unbreakable linker fragment;
L₂' is none or a breakable or unbreakable linker fragment;
L₃ is none or a breakable or unbreakable linker fragment;
L₃' is none or a breakable or unbreakable linker fragment;
L₄ is none or a breakable or unbreakable linker fragment;
L₄' is none or a breakable or unbreakable linker fragment;
L₅ is none or a breakable or unbreakable trident linker fragment;
Z₁ is 0 or 1, Z₂ is 0 or 1, and Z₁ and Z₂ are not simultaneously 0; and
the structure of Q is as shown in formula A.

Further, the structure of L₅ is selected from 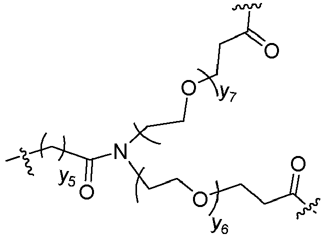 or 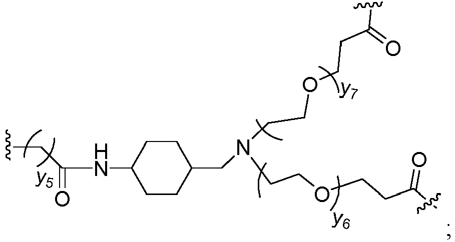
y₅ is an integer selected from 1 to 10, preferably 1;
y₆ is an integer selected from 1 to 50, preferably an integer from 2 to 9; and
y₇ is an integer selected from 1 to 50, preferably an integer from 2 to 9.

Further, the structure of the linker is as shown in formula G-3: 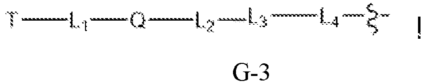
wherein T is a tying group, which can be connected to the targeted linker; the targeted linker is a substance capable of targeted binding to a lesion site;
L₁ is a breakable or unbreakable linker fragment;
L₂ is none or a breakable or unbreakable linker fragment;
L₃ is a breakable or unbreakable linker fragment;
L₄ is none or a breakable or unbreakable linker fragment; and
the structure of Q is as shown in formula A.

Further, the structure of T is as follows:

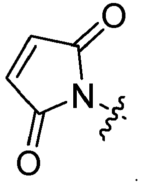

Further, the structure of L₁ is as follows: 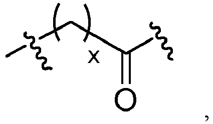 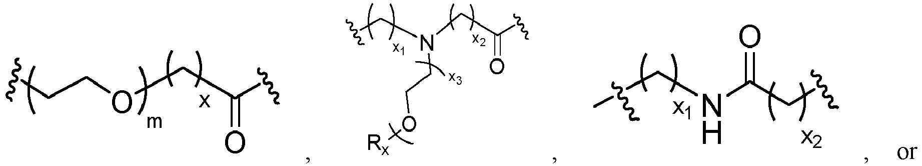 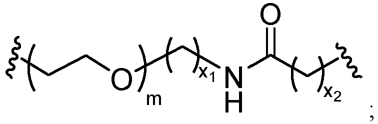
wherein x is an integer selected from 1 to 10, preferably an integer from 2 to 6;
m is an integer selected from 1 to 25, preferably an integer from 2 to 10, and more preferably an integer from 2 to 9;
X₁ is an integer selected from 1 to 10, preferably an integer from 2 to 6, and more preferably 2;
x₂ is an integer selected from 1 to 10, preferably an integer from 1 to 5, and more preferably 1;
x₃ is an integer selected from 0 to 50, preferably an integer from 8 to 30, and more preferably 9; and
Rₓ is selected from hydrogen or methyl.

Further, the structure of L₂ is 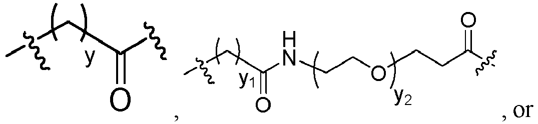 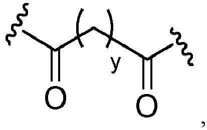 or L₂ is none;
the structure of L₂' is 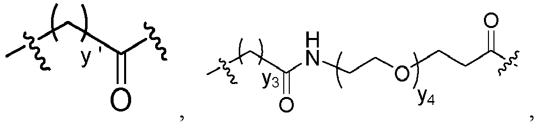 or 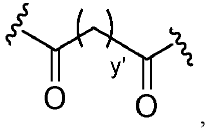 or L₂' is none;
wherein y and y' are integers independently selected from 1 to 10, preferably 1 or 2;
y₁ and y₃ are integers independently selected from 0 to 10, preferably 0, 1 or 2, and more preferably 0 or 1; and
y₂ and y₄ are integers independently selected from 1 to 50, preferably integers from 1 to 20, and more preferably integers from 2 to 12.

Further, the structures of L₃ and L₃' are independently selected from: 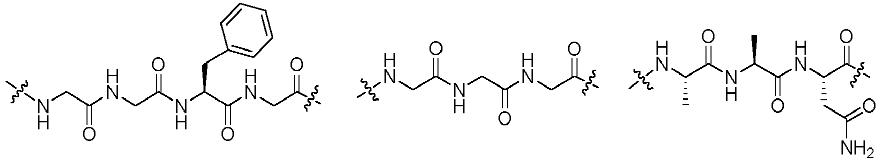 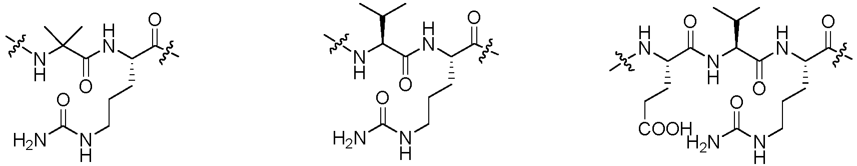 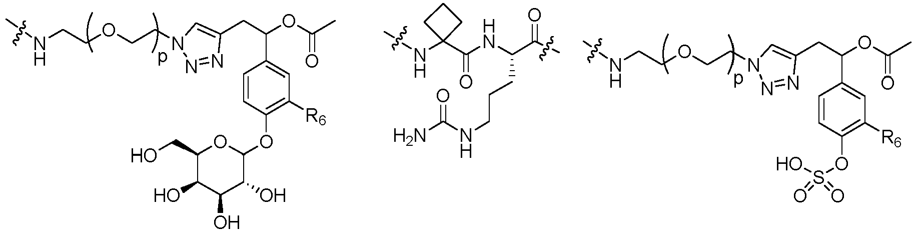 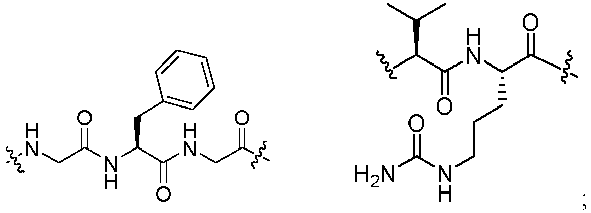 wherein p is an integer selected from 1 to 24, and R₆ is selected from hydrogen or nitro.

Further, the structures of L₄ and L₄' are independently selected from none,

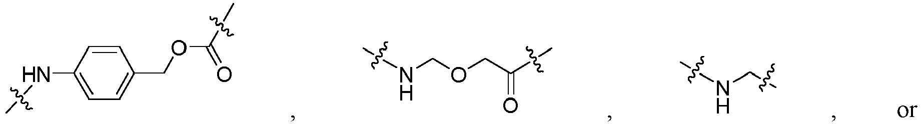

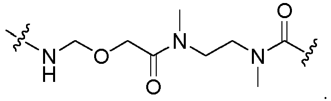

Further, the structure of the linker is selected from: 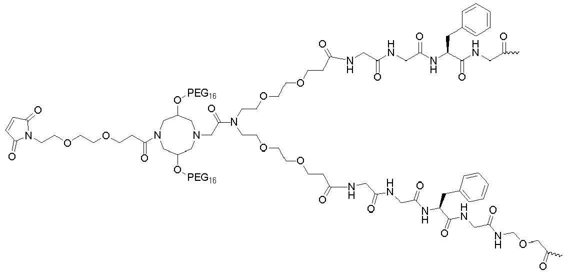 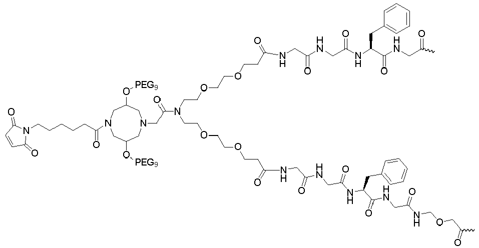 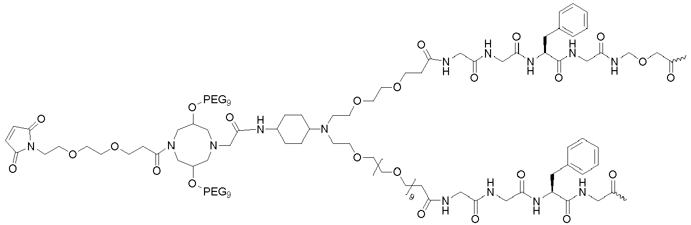 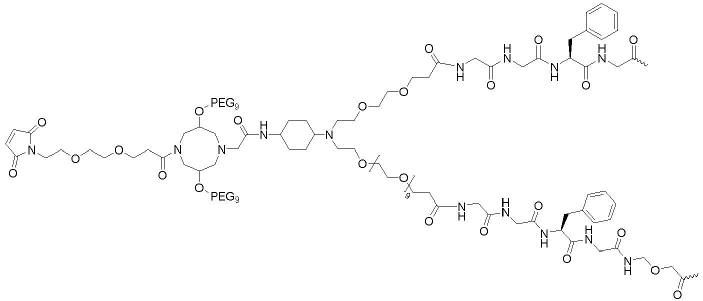 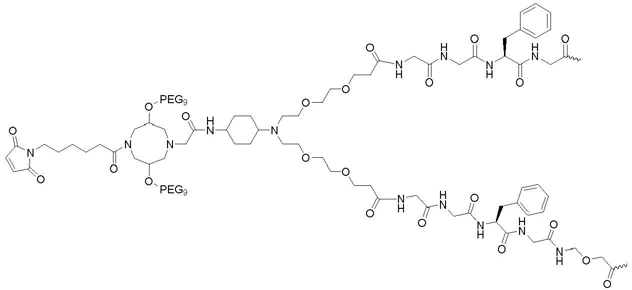 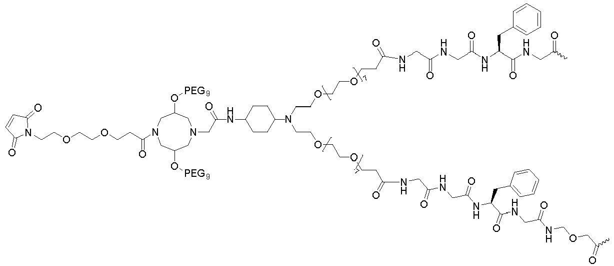 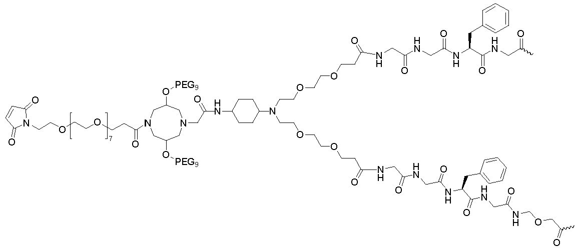 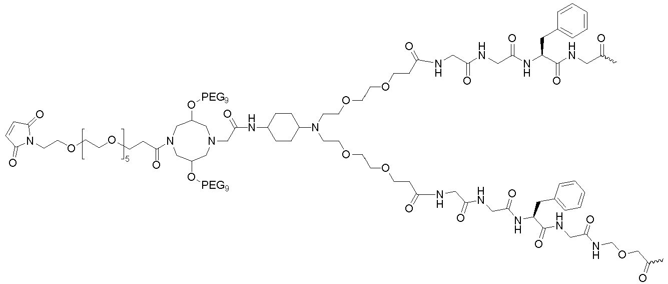 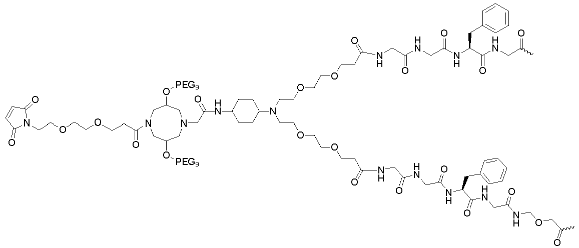 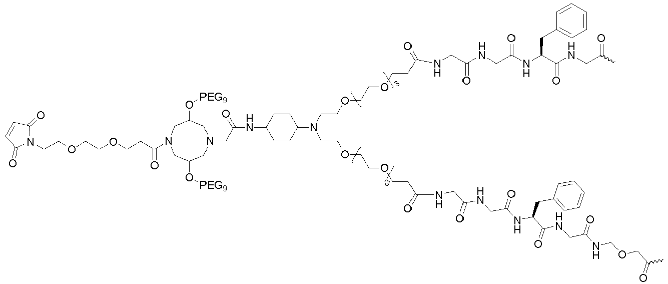 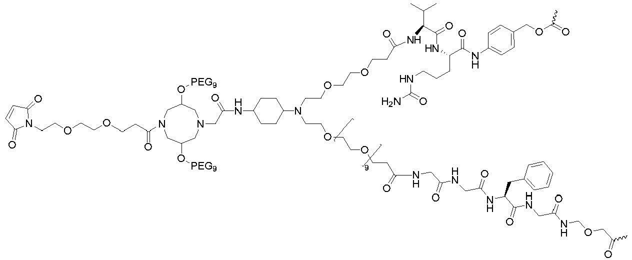 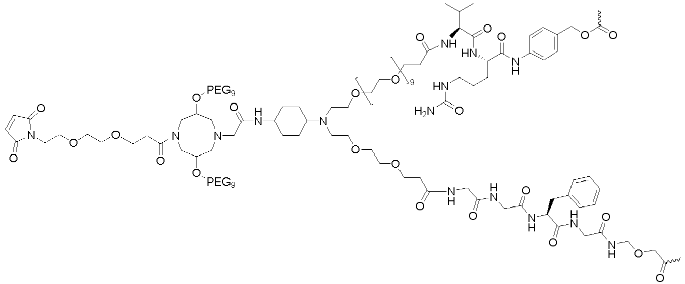 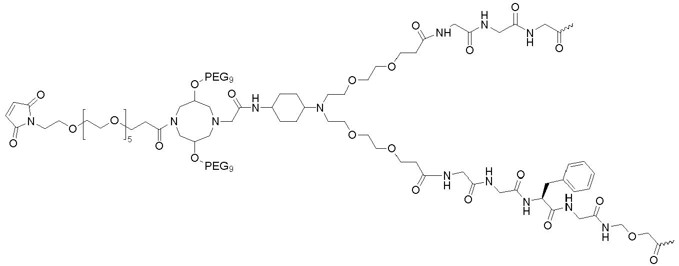 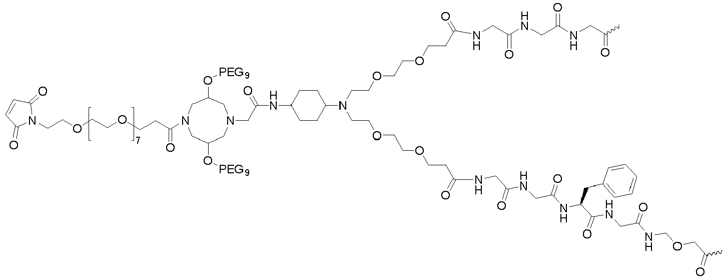 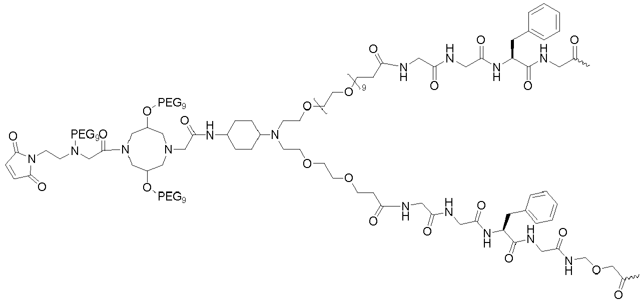 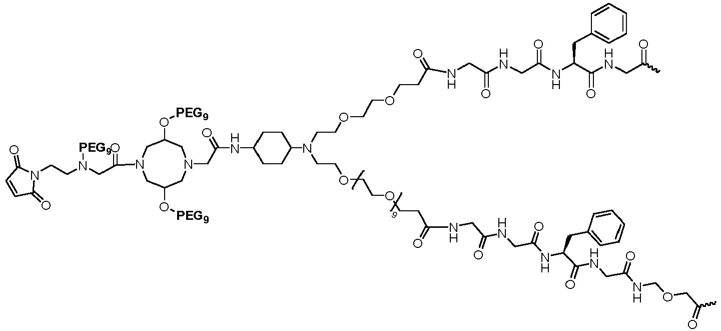 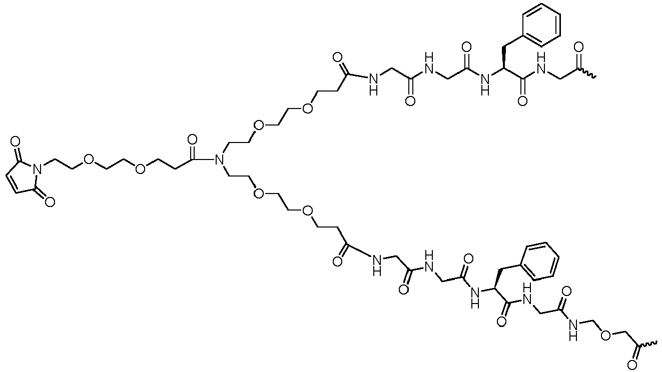 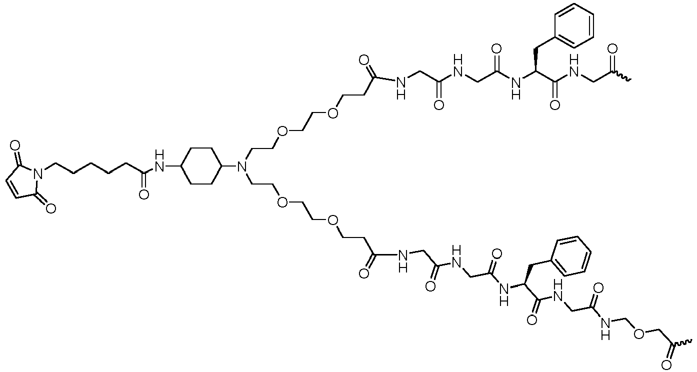 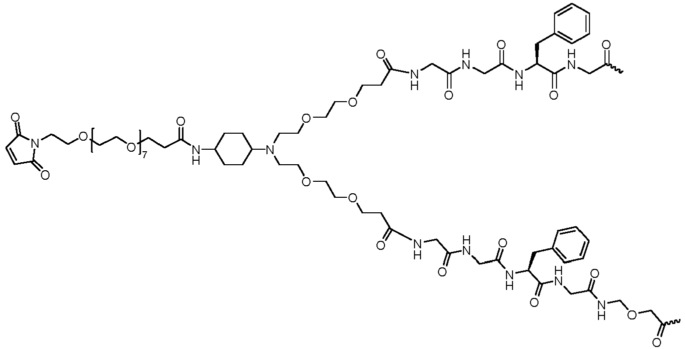 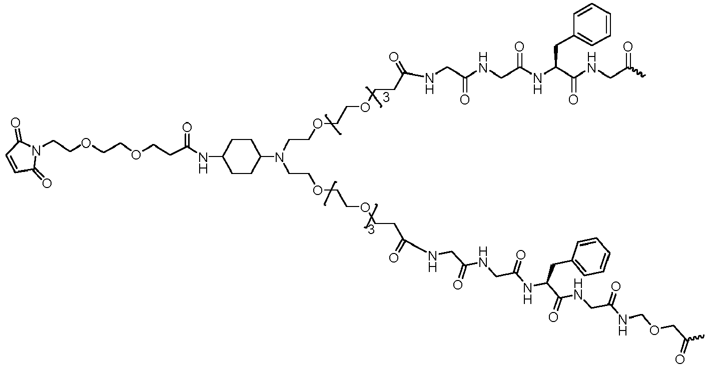 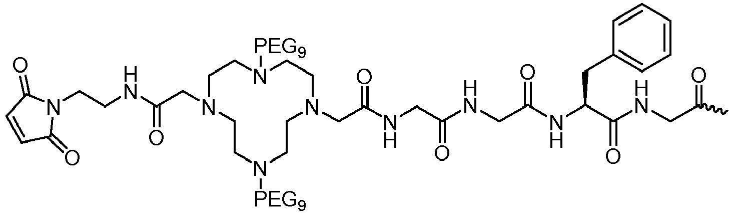 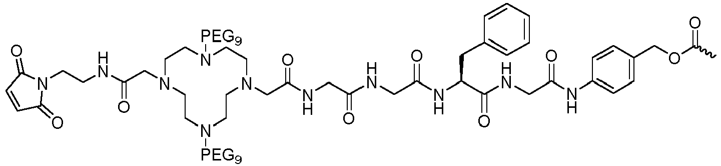 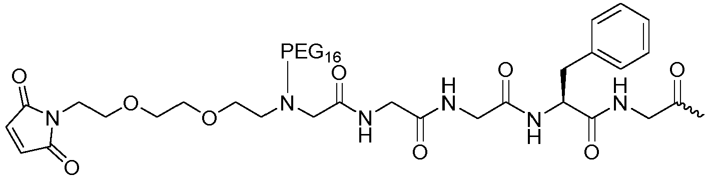 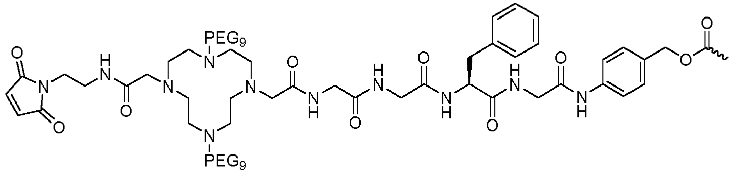 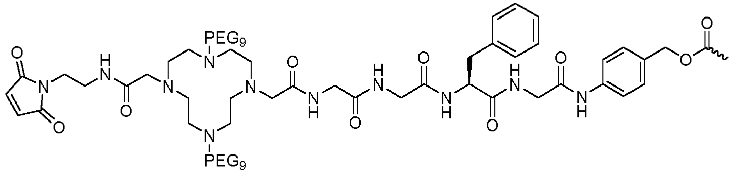 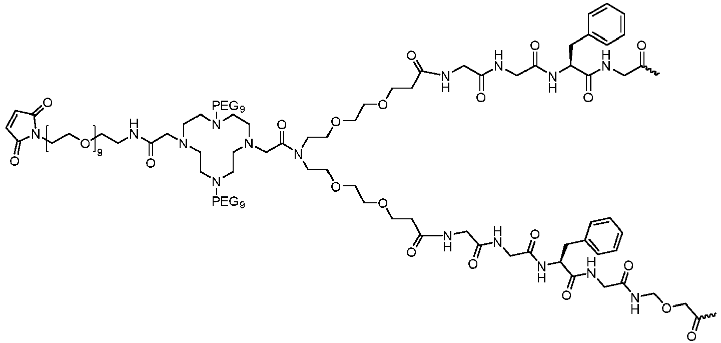 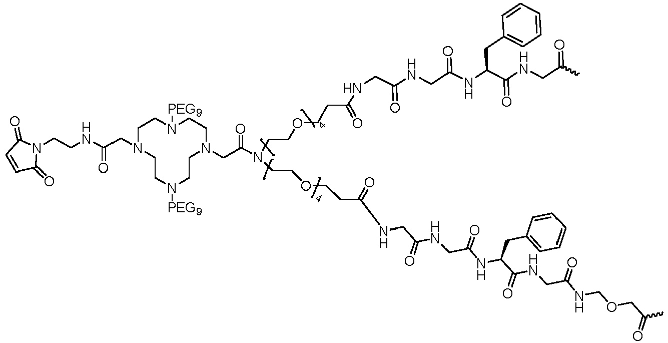 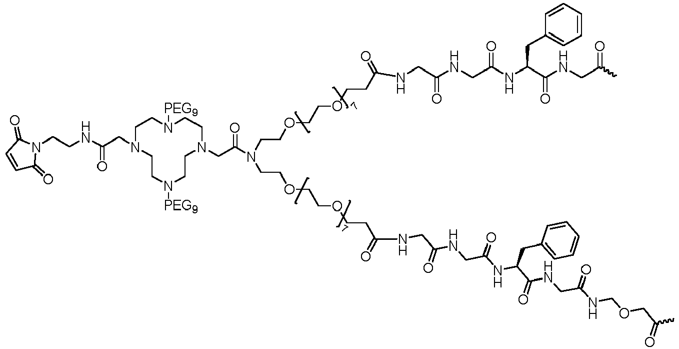 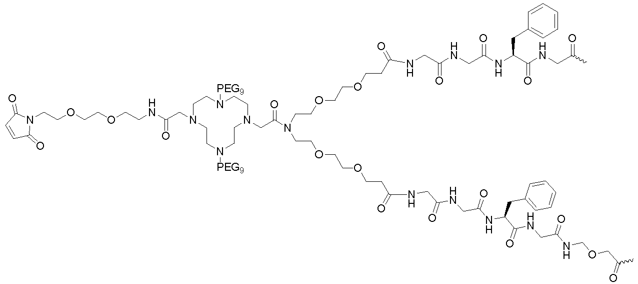 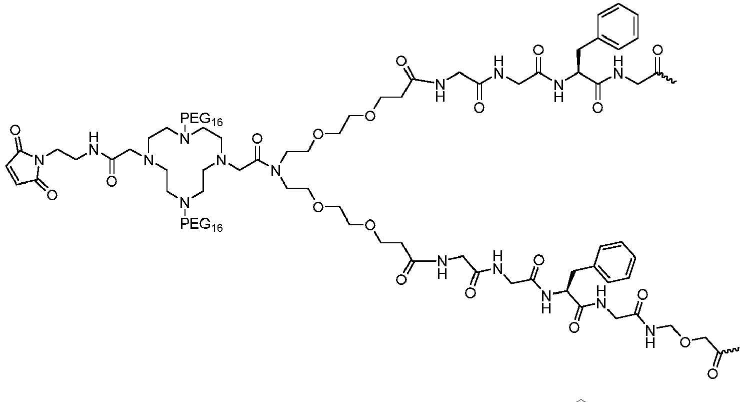 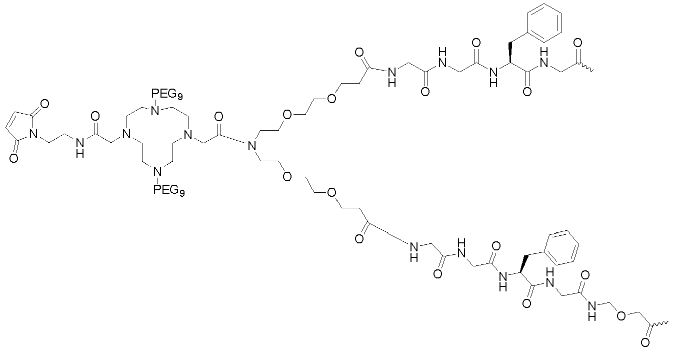 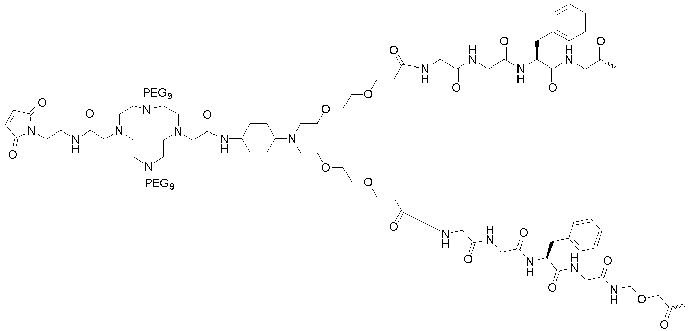 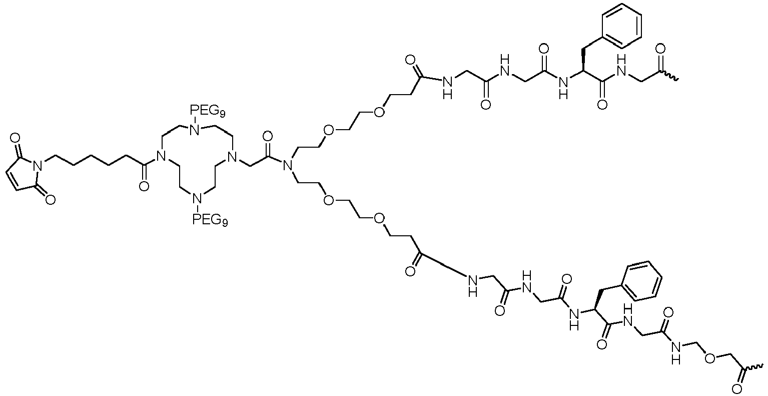 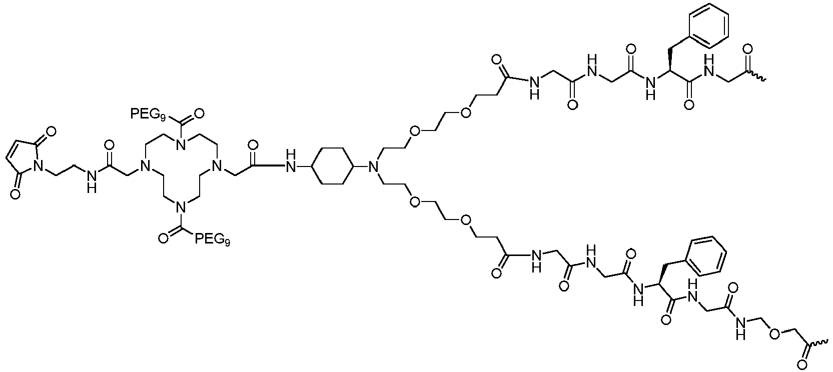 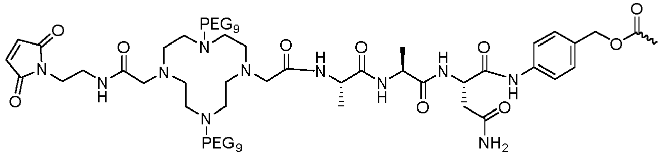 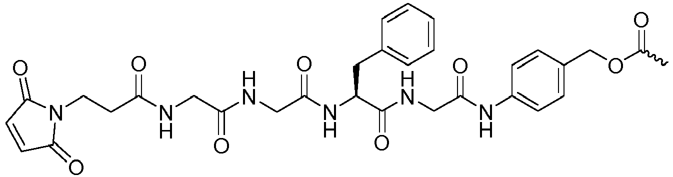 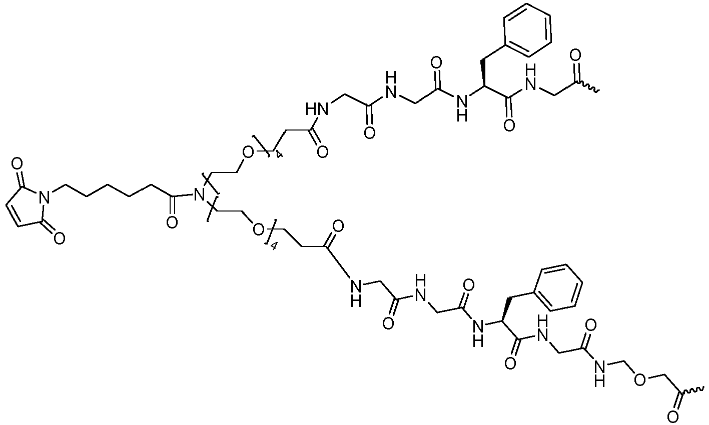 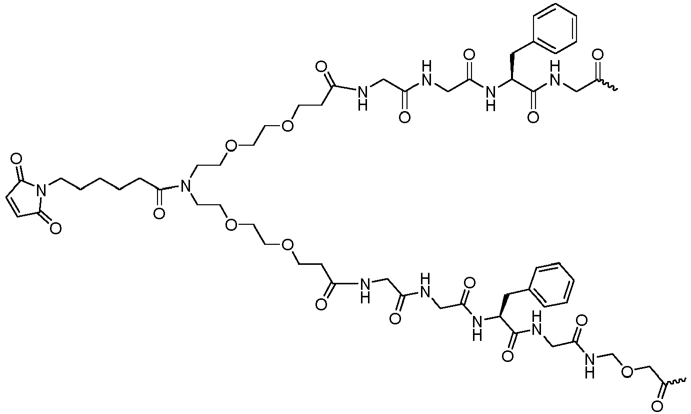 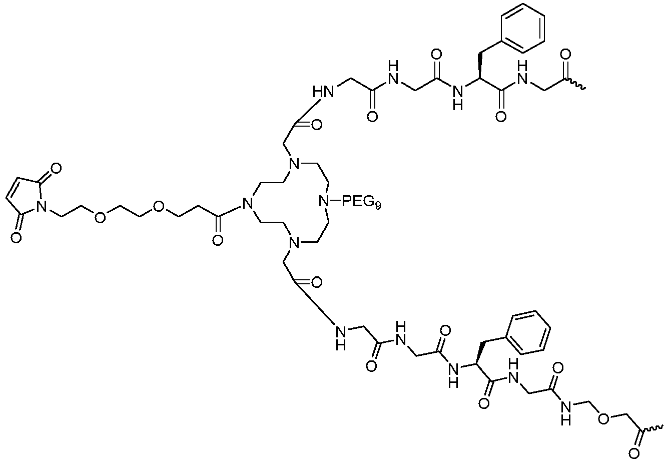 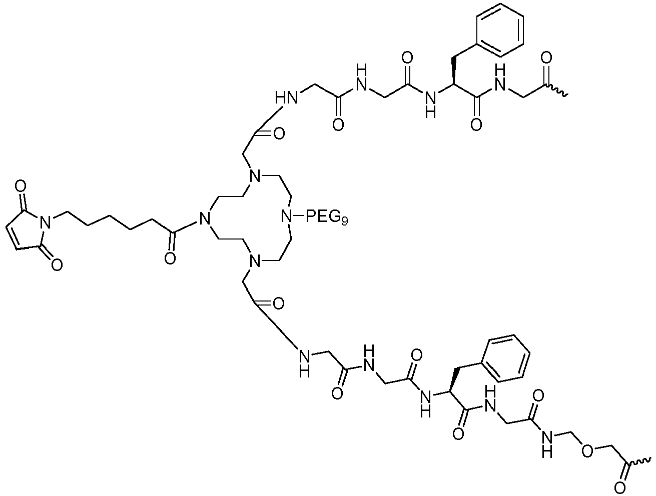 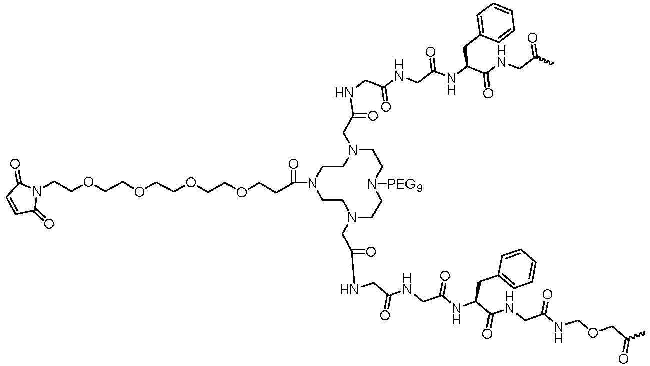 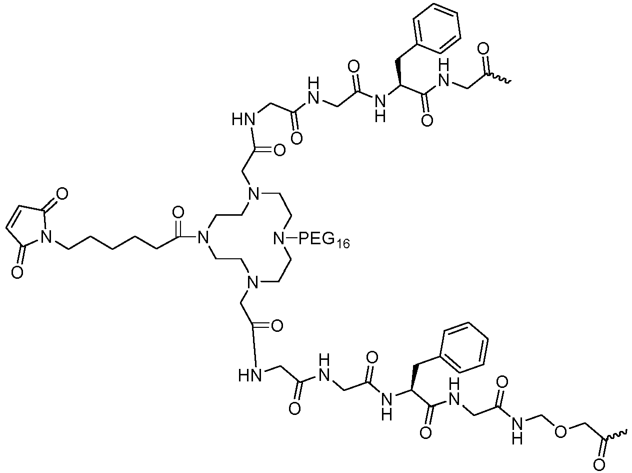 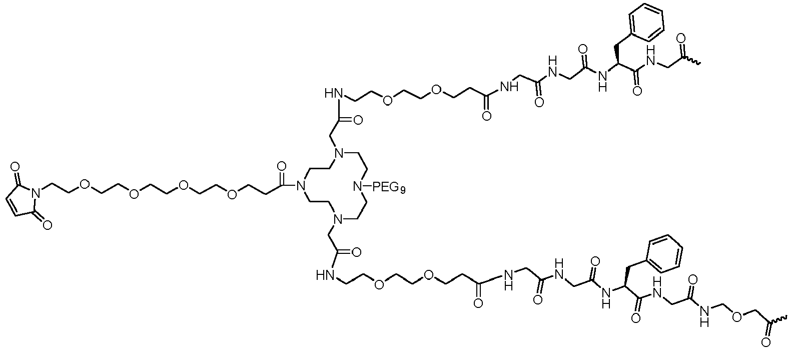 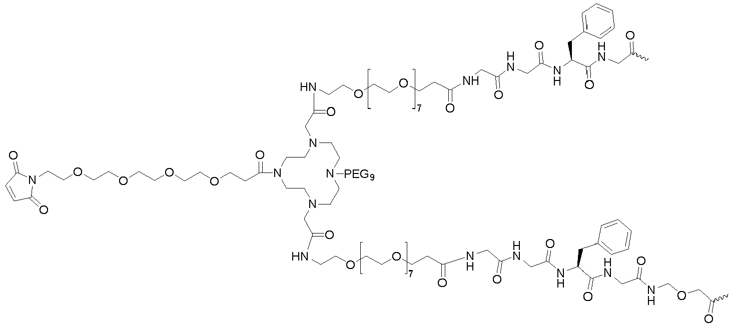 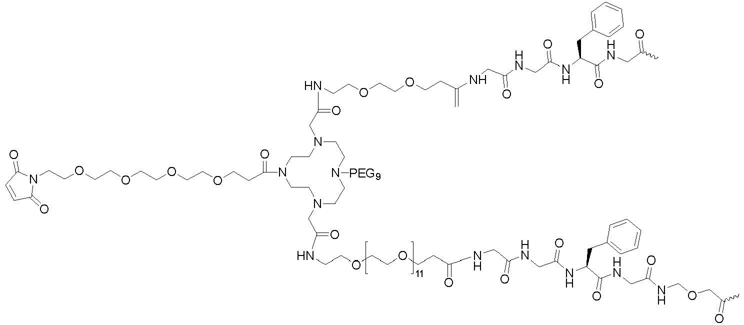 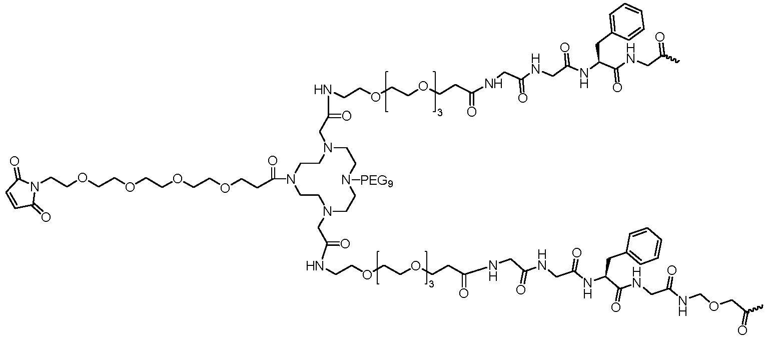 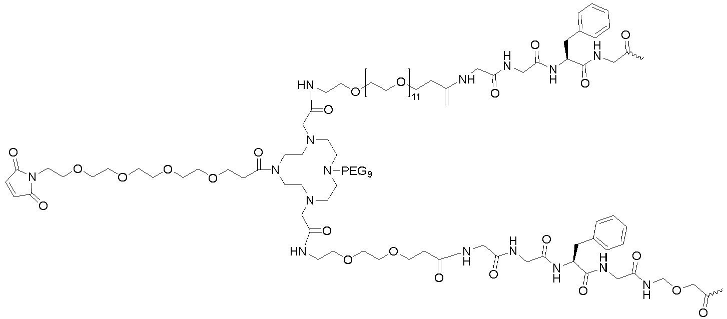 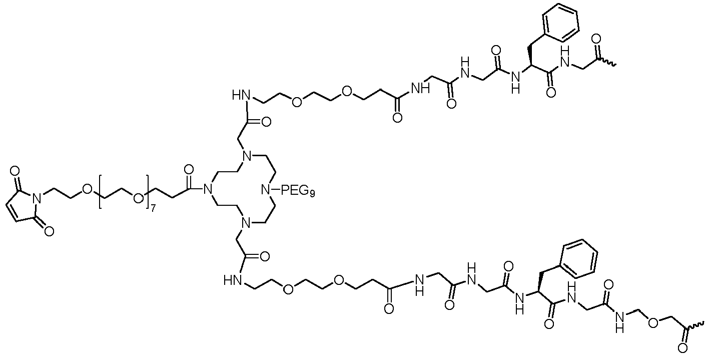 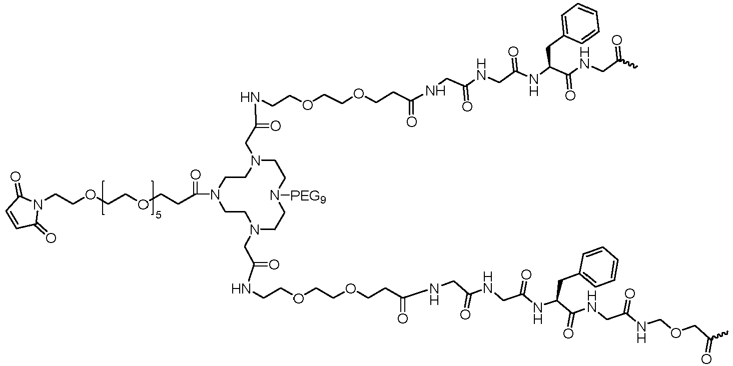 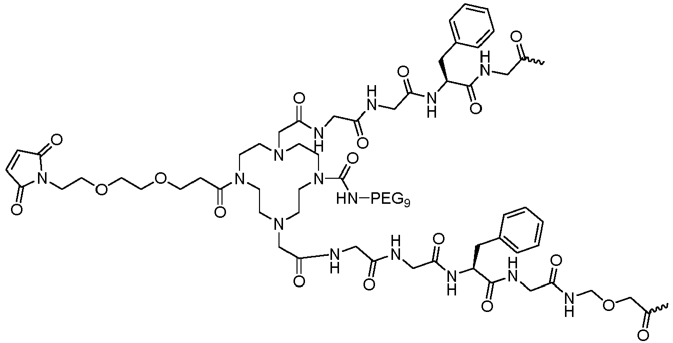 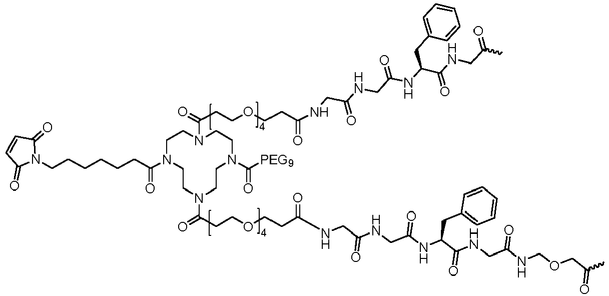 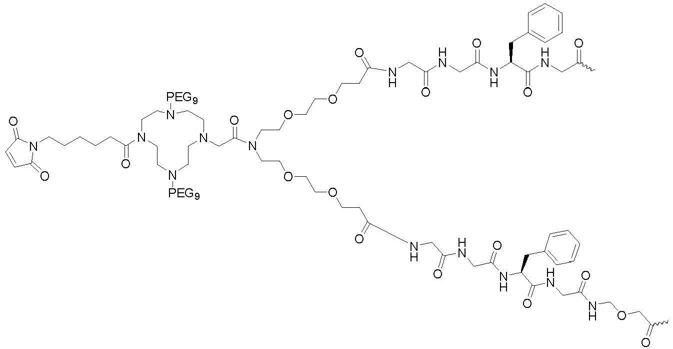 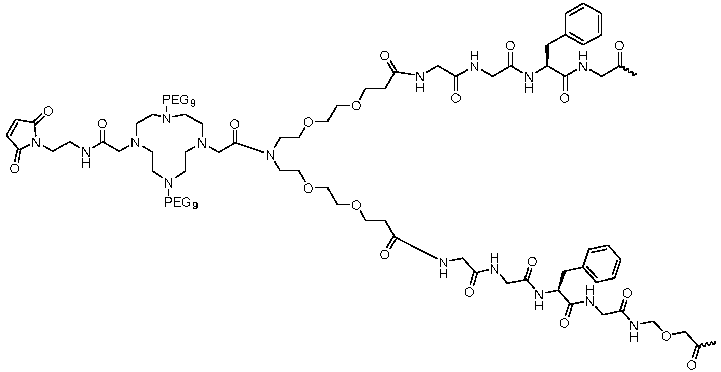 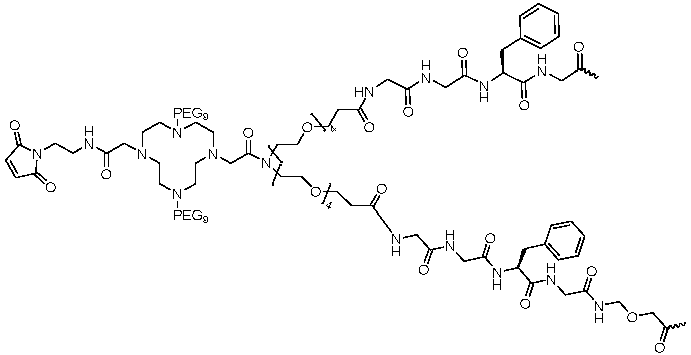 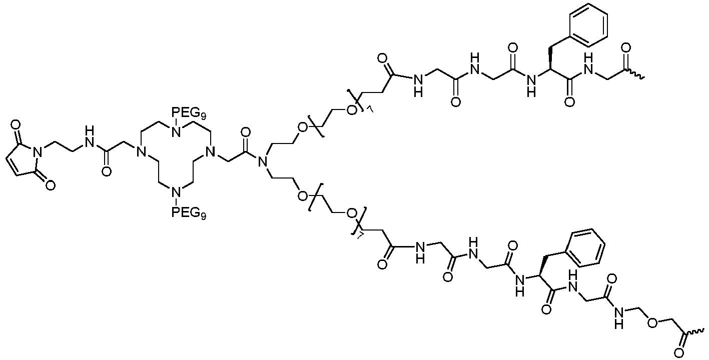 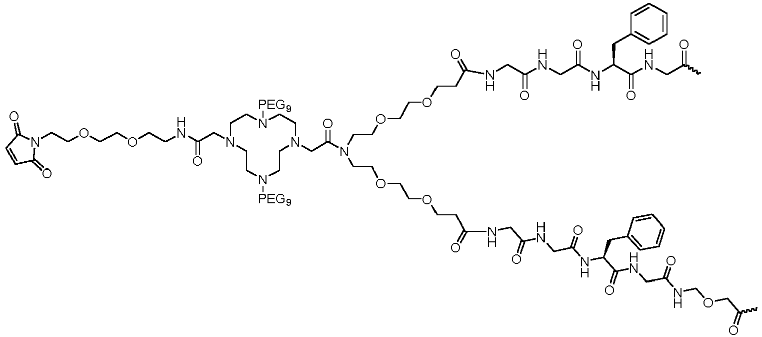 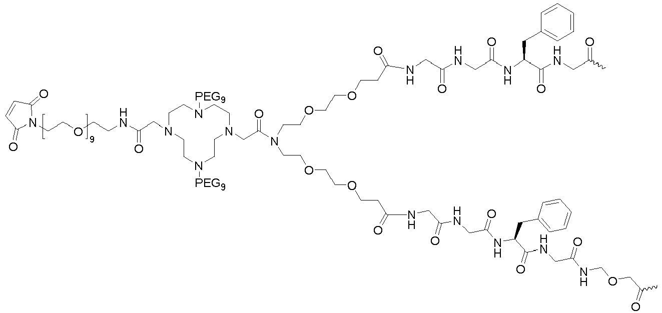 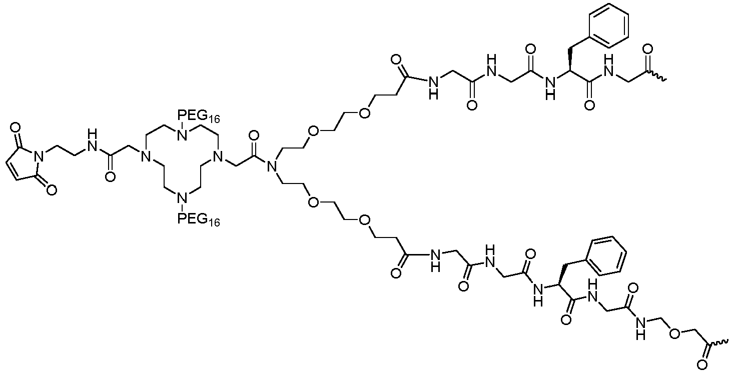 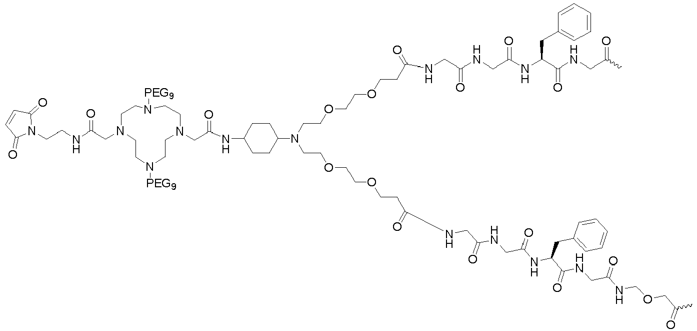 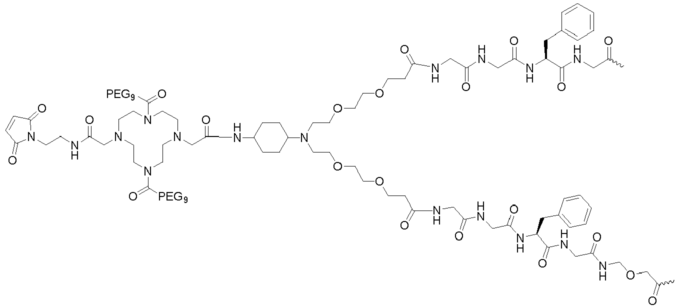 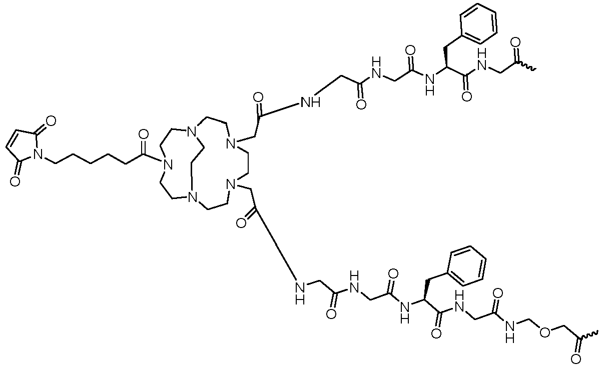 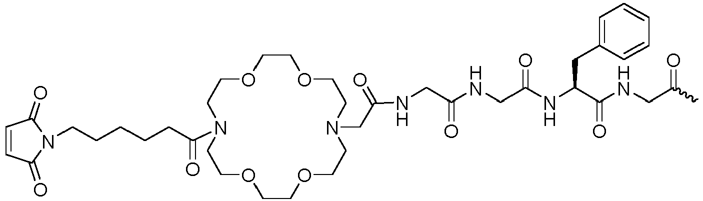 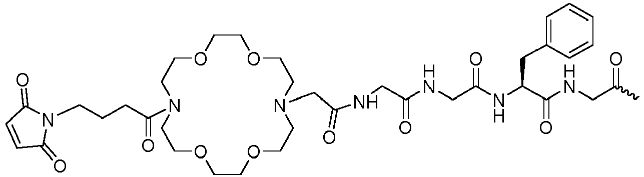 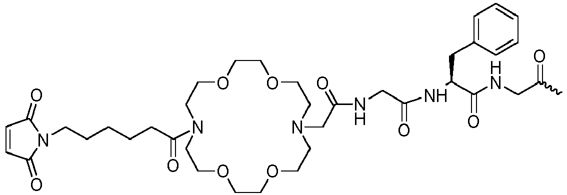 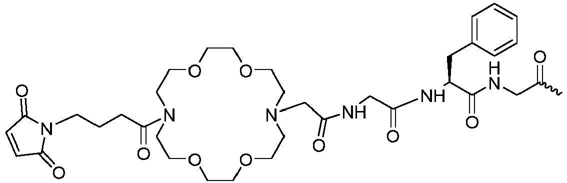 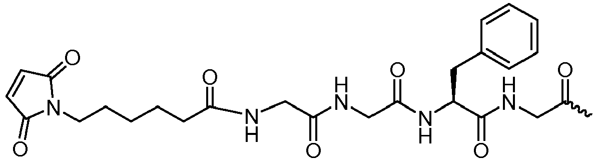 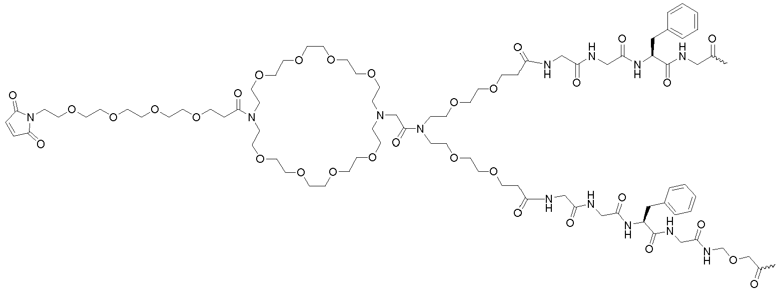 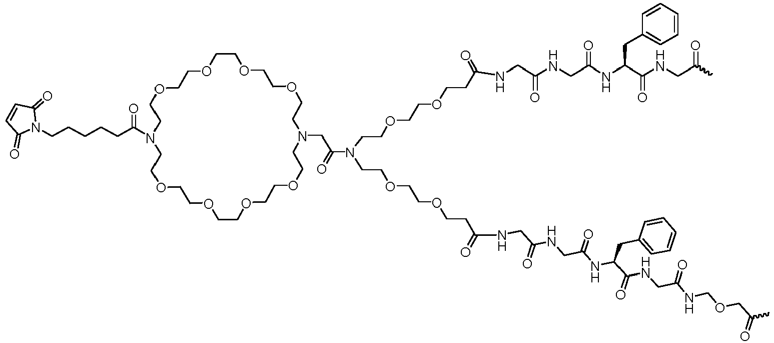 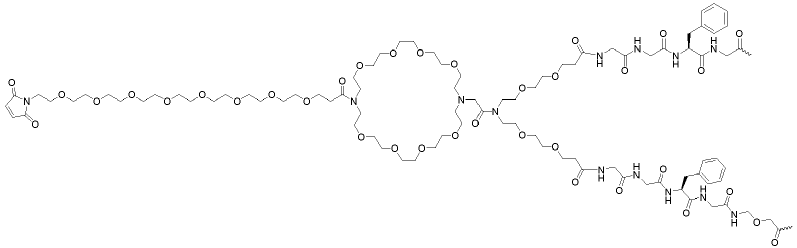 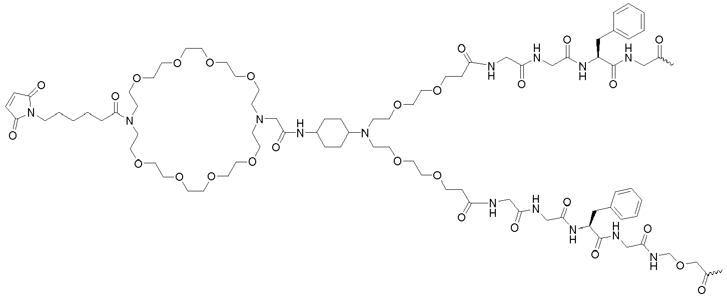 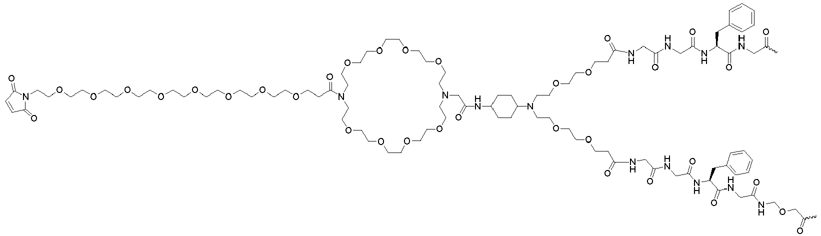 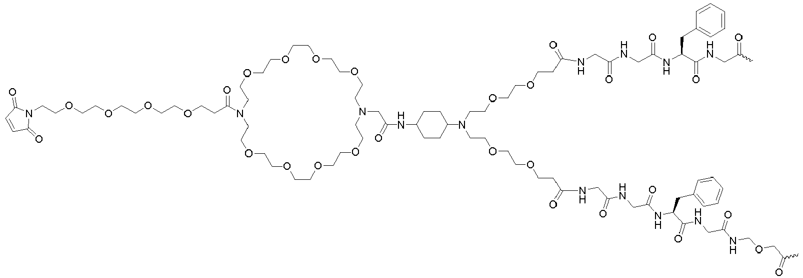 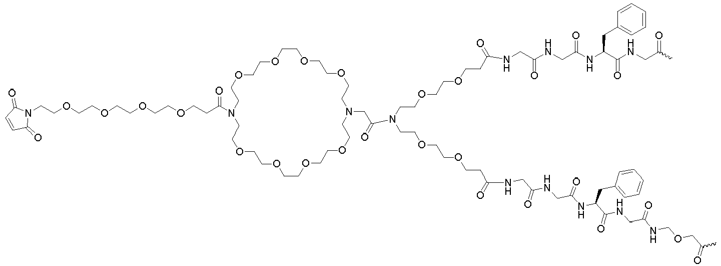 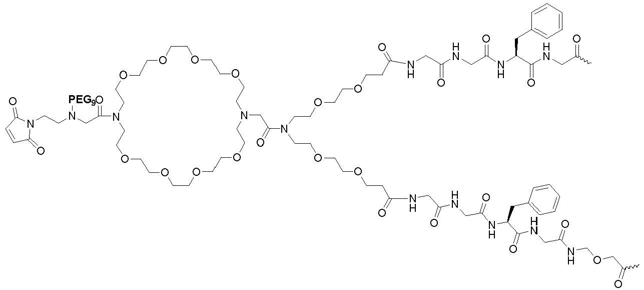 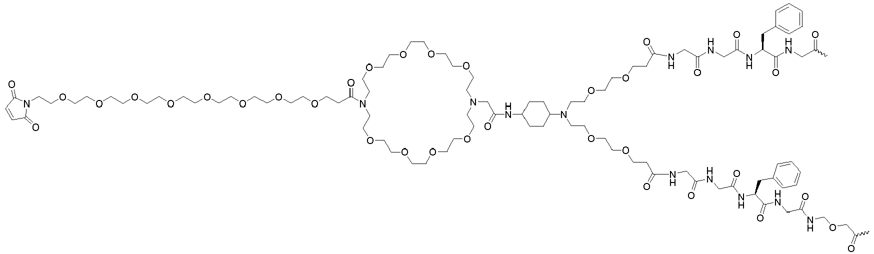 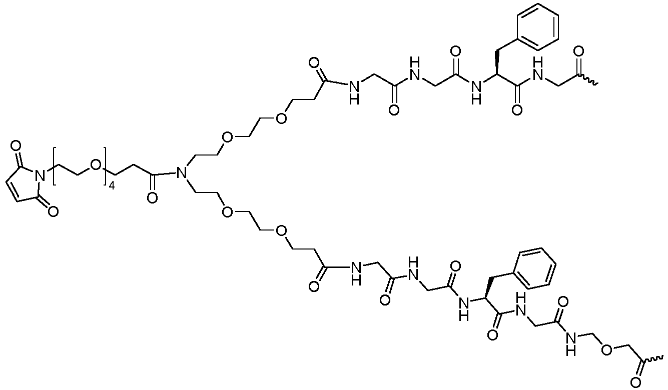 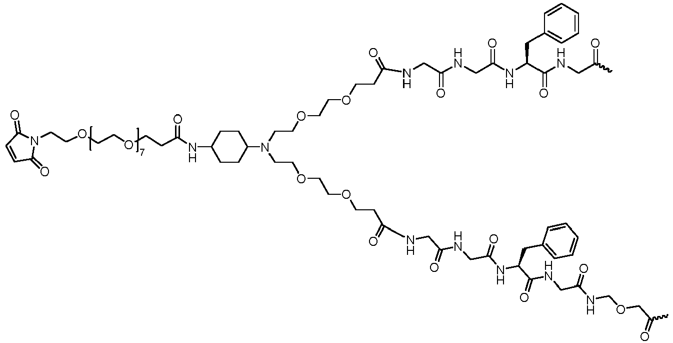 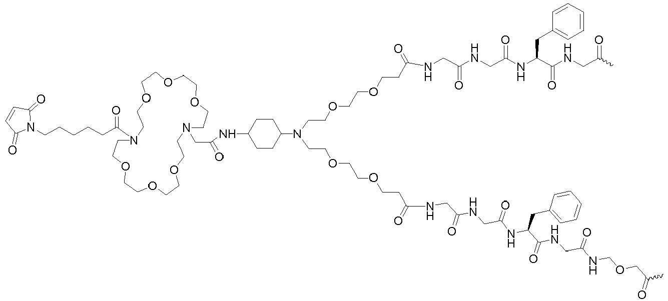 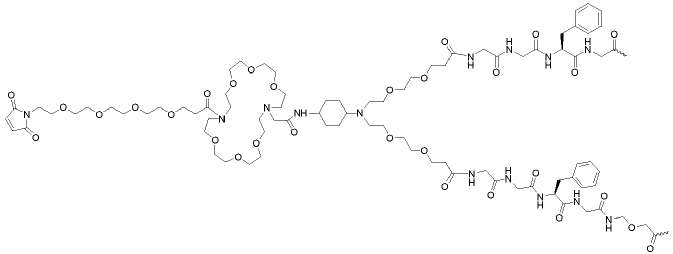 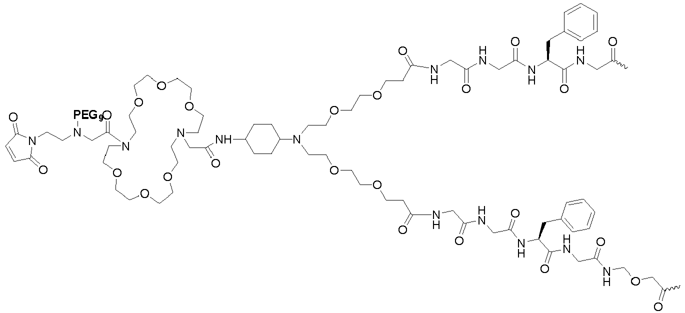 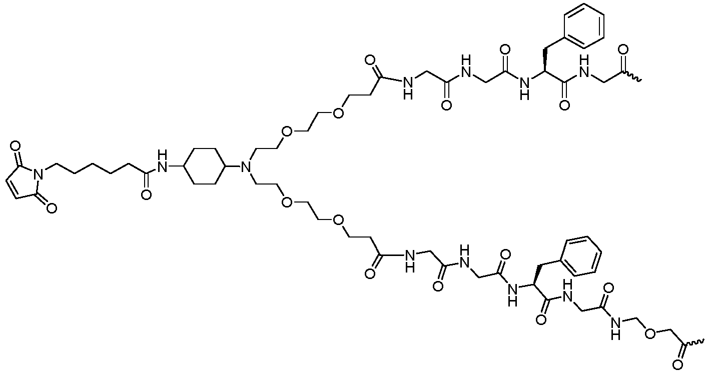 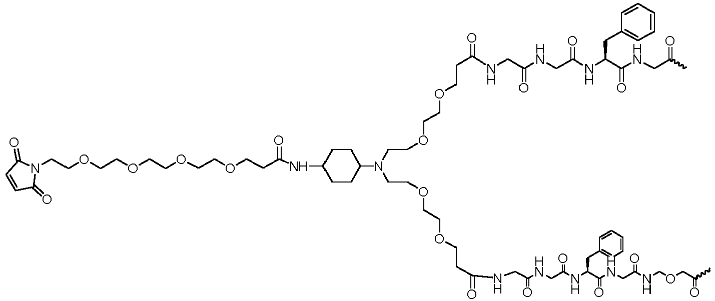 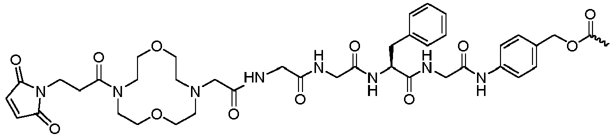 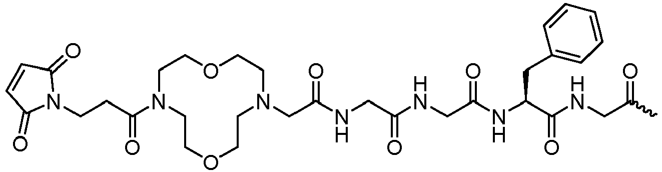 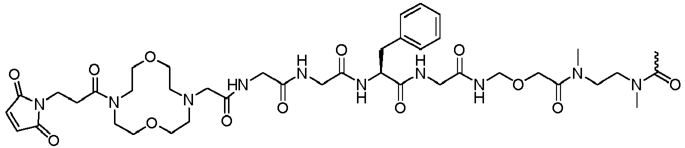 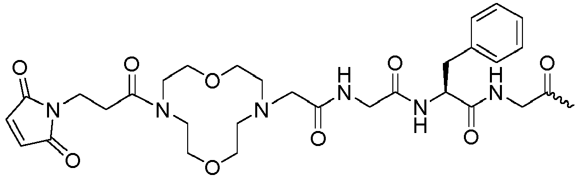 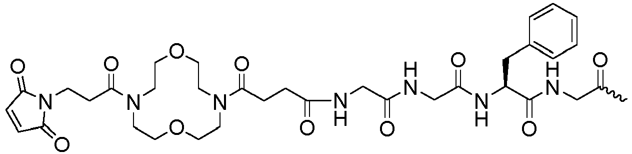 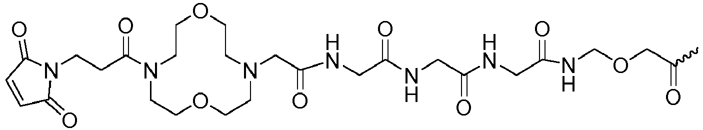 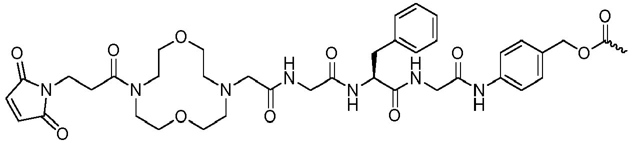 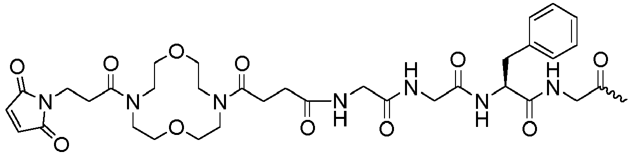 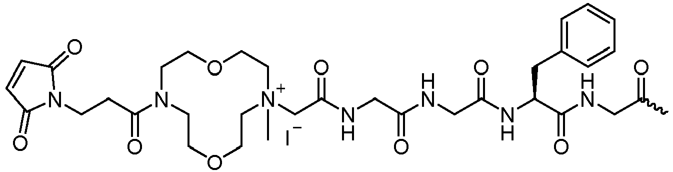 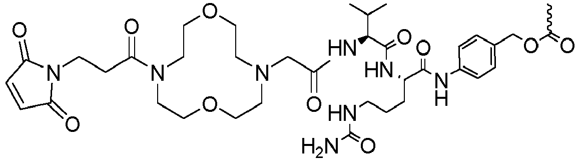 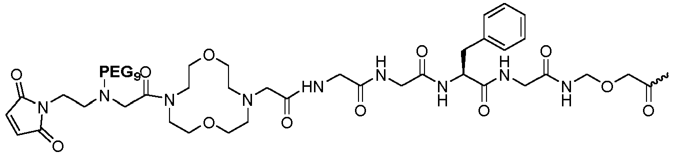 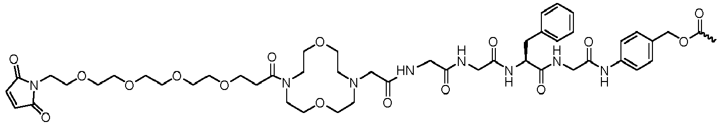 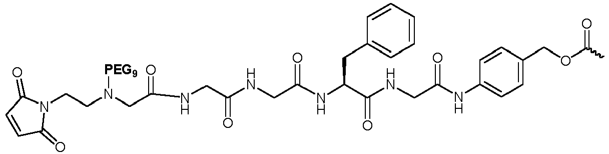 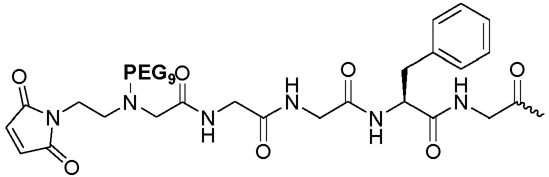 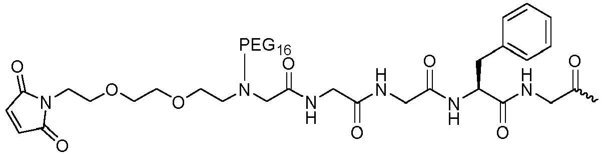 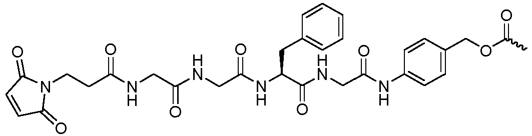 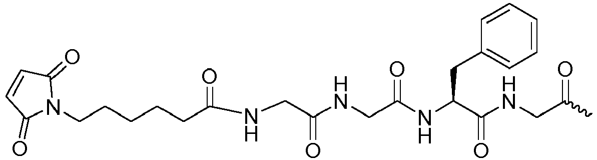 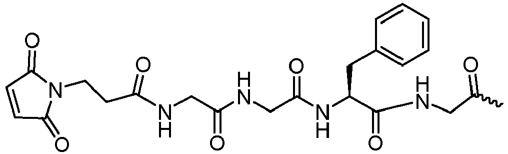
-PEG₉ represents 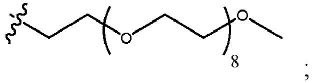 and -PEG₁₆ represents 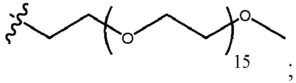 and
right sides of the above structures are all 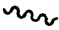 , indicating that the structures can be connected to other functional groups.

Further, the structure of the drug link assembly unit is as shown in formula G-4 or G-5: 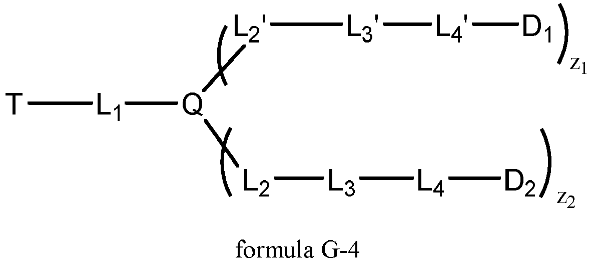 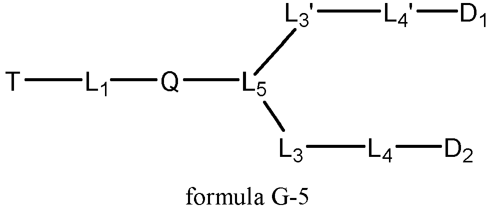
wherein T, Q, L₁, L₂, L₂', L₃, L₃', L₄, L₄', L₅, Z₁, and Z₂ are as described above;
D₁ is a drug; and
D₂ is a drug.

Further, D₁ and D₂ are independently selected from a cytotoxic drug, a drug for treating autoimmune toxicity, or an anti-inflammatory drug;
preferably, D₁ and D₂ are independently selected from a drug targeting DNA or a drug targeting a microtubule protein; and
more preferably, D₁ and D₂ are independently selected from one of the following compounds or derivatives thereof

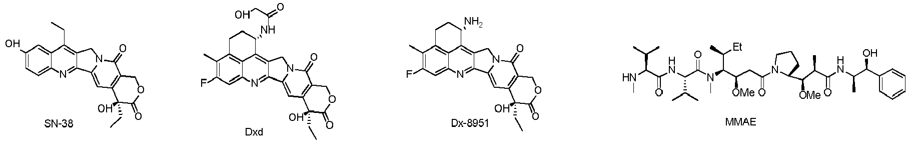

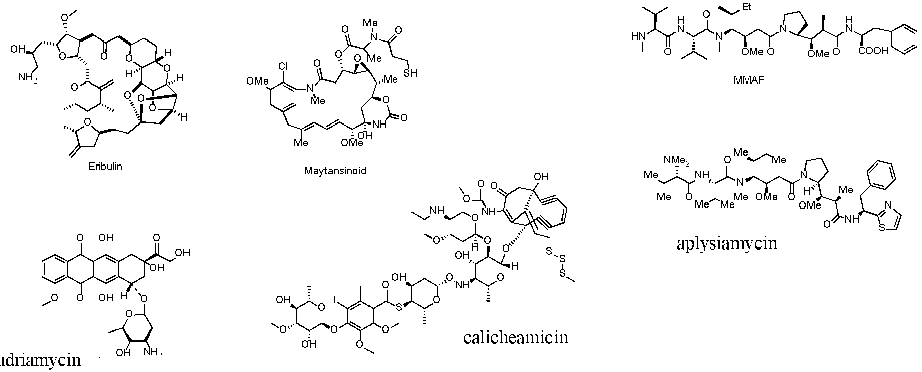

Further, the structure of the drug link assembly unit is selected from:

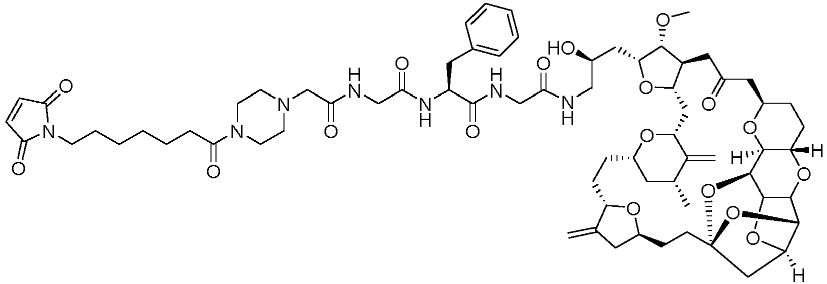

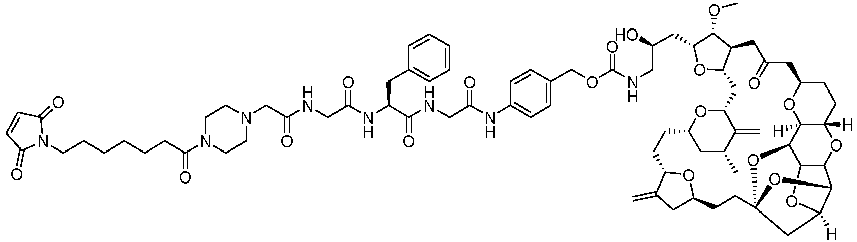

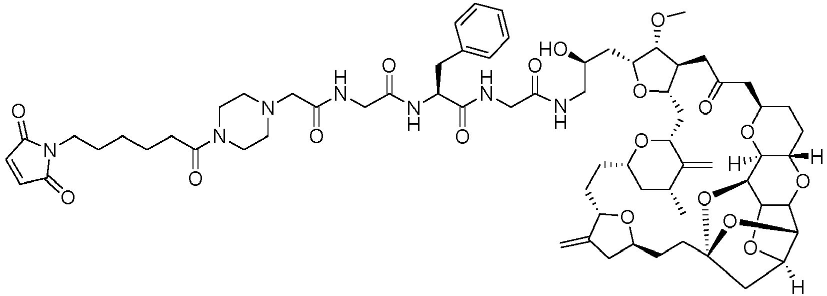

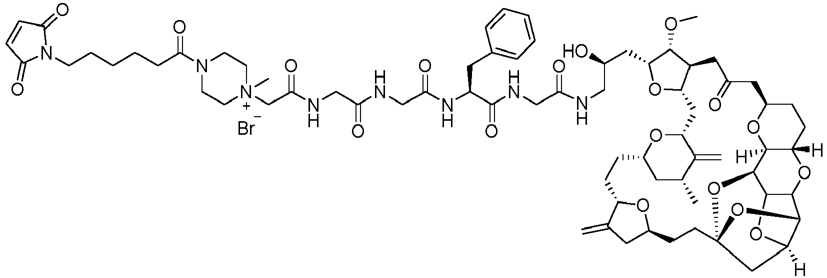

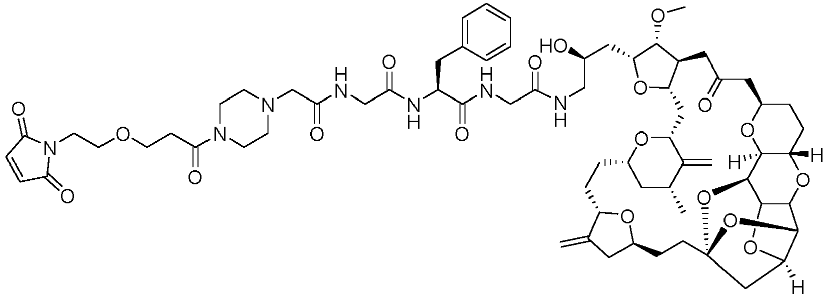

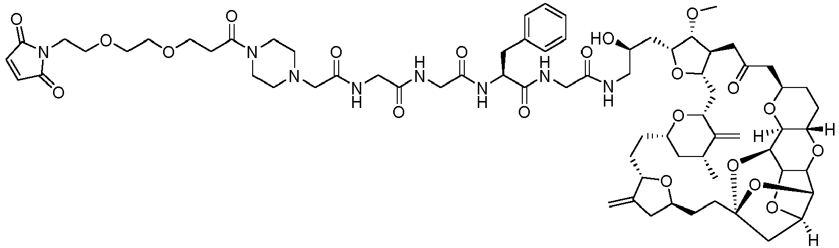

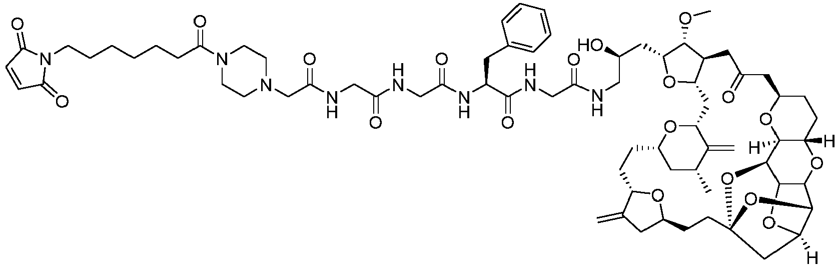

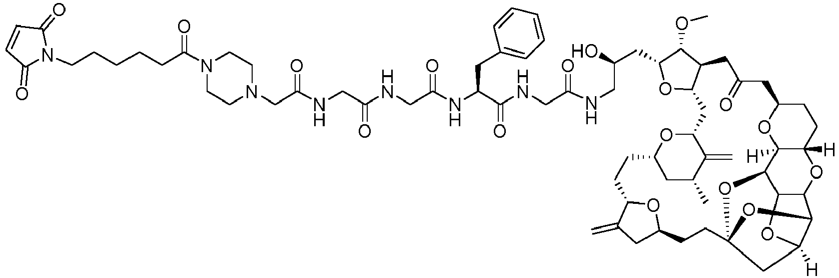

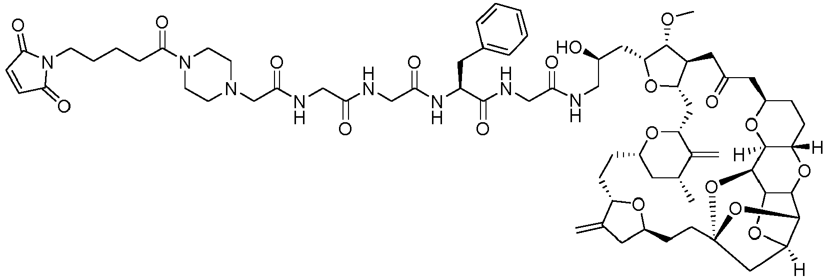

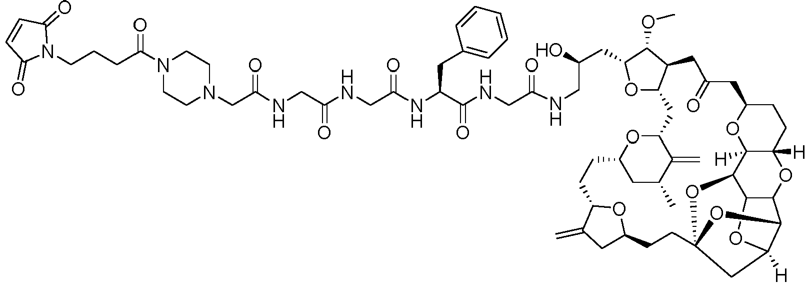

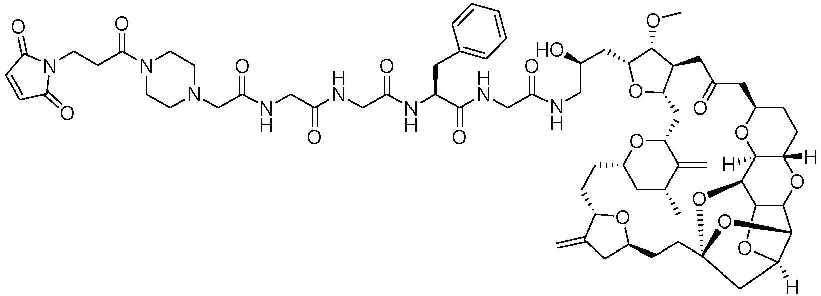

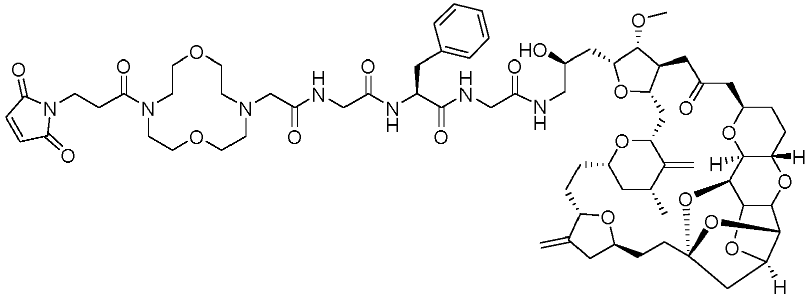

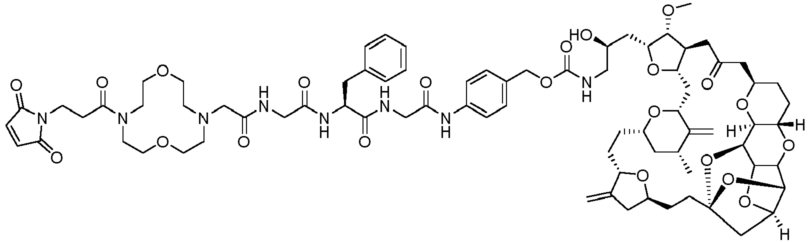

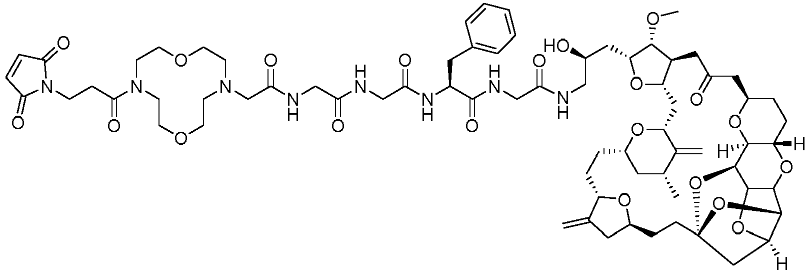

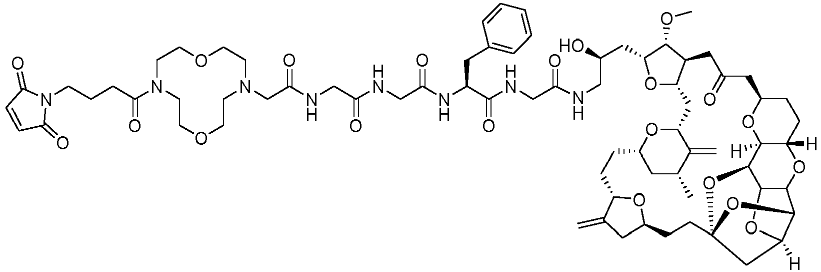

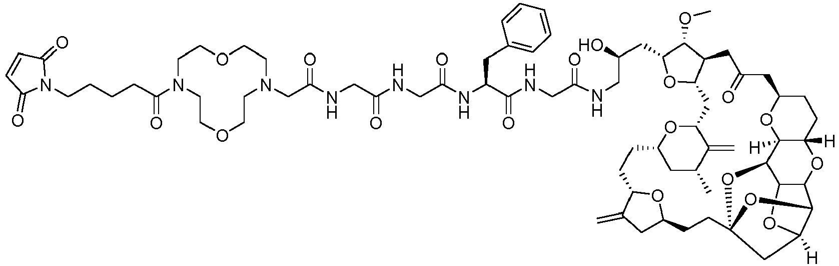

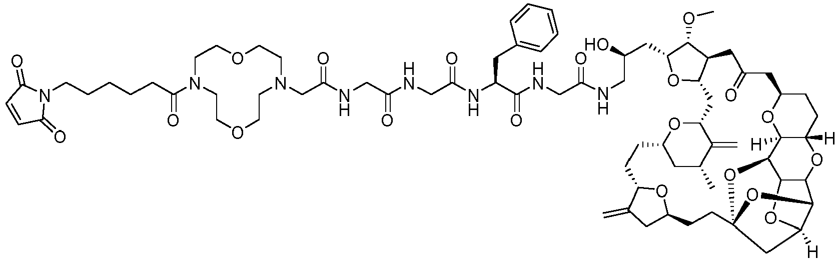

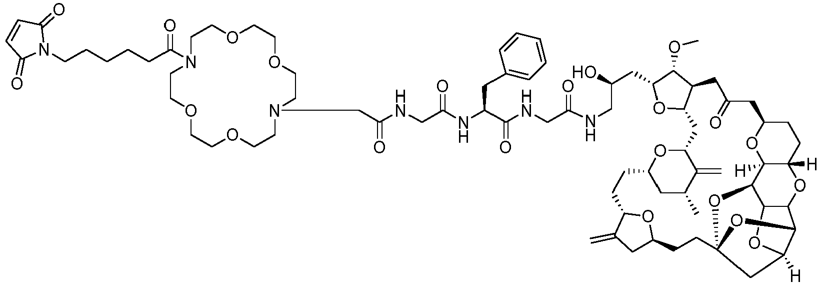

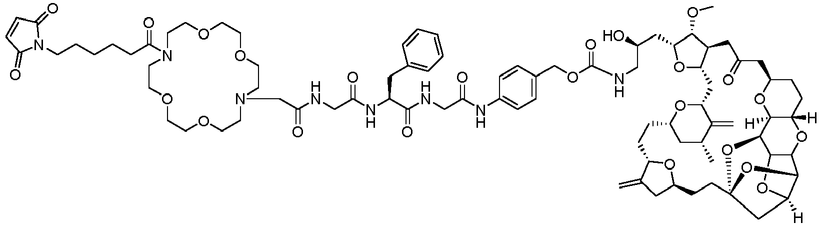

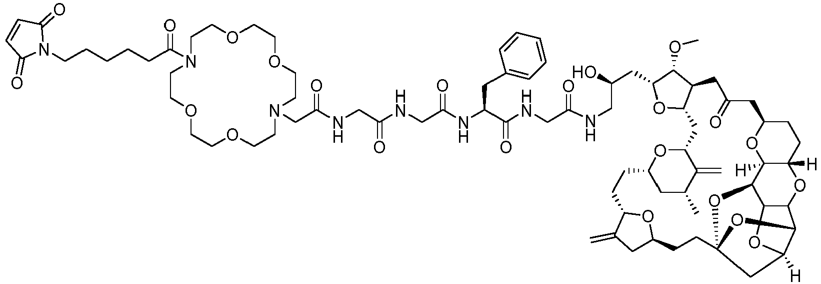

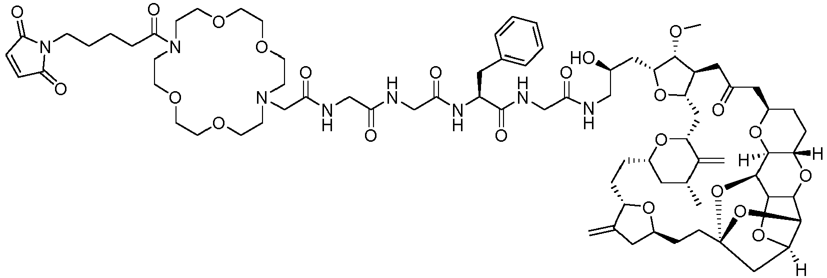

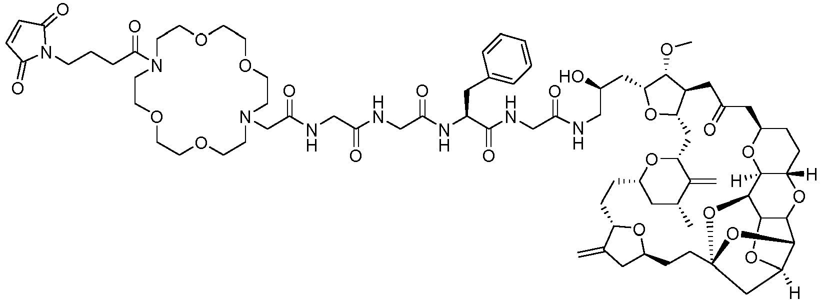

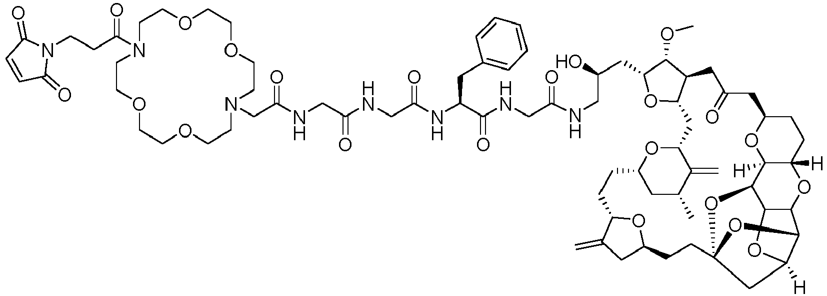

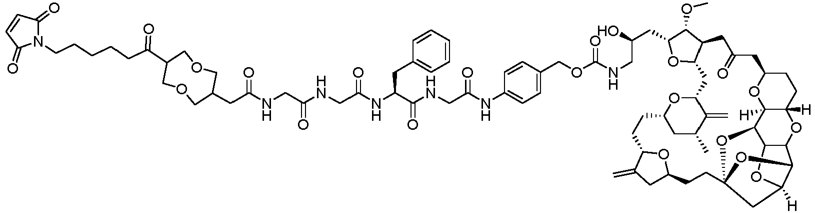

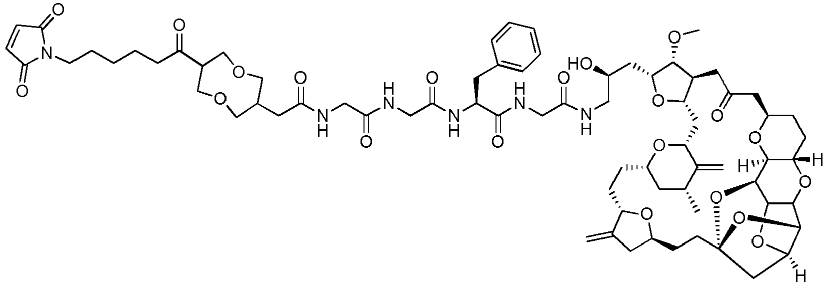

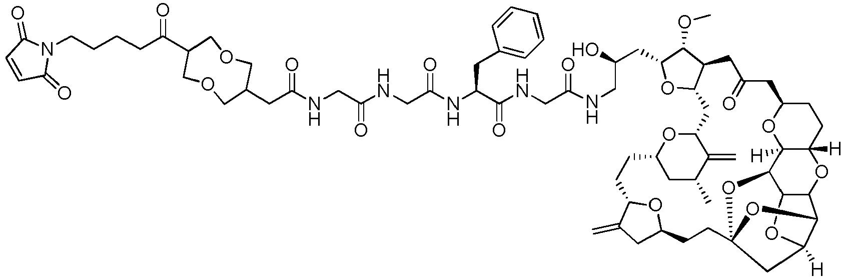

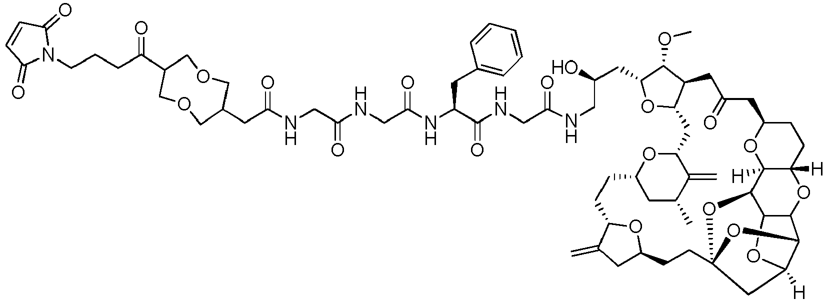

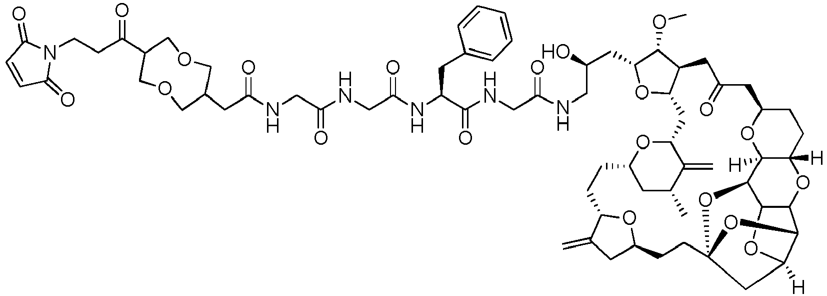

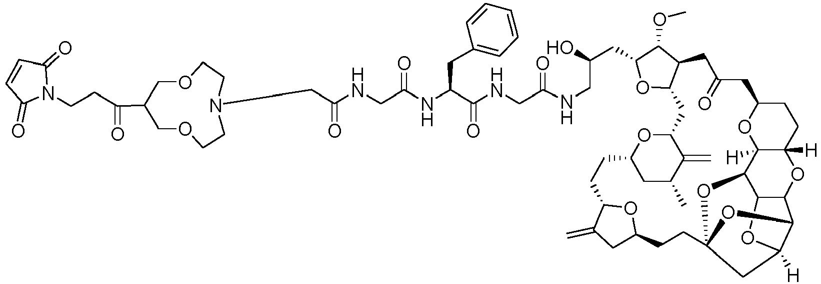

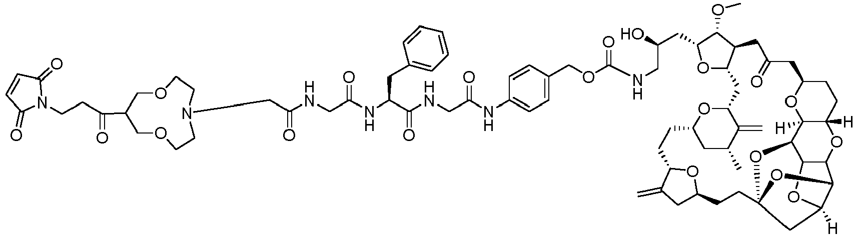

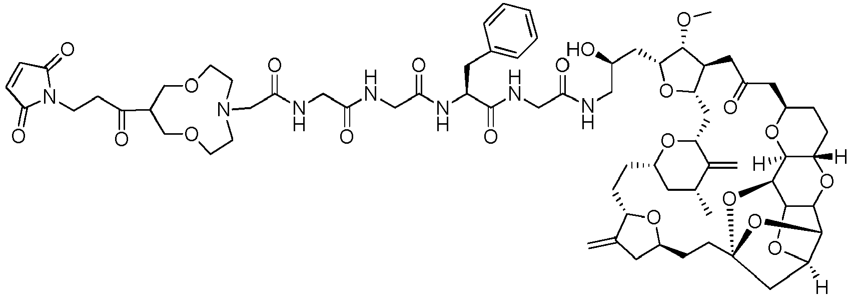

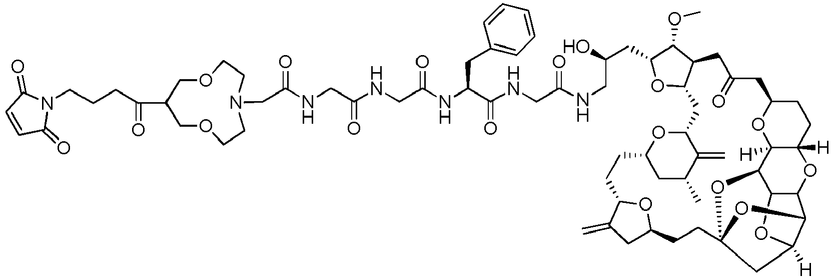

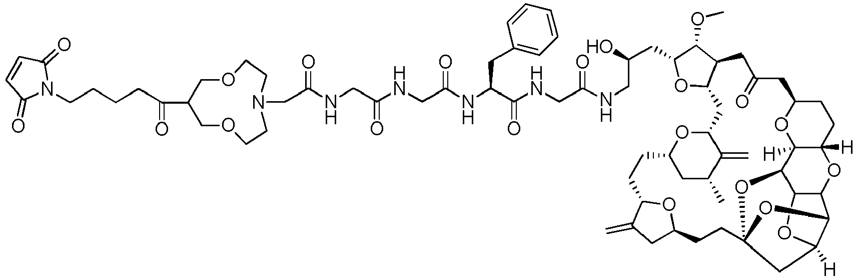

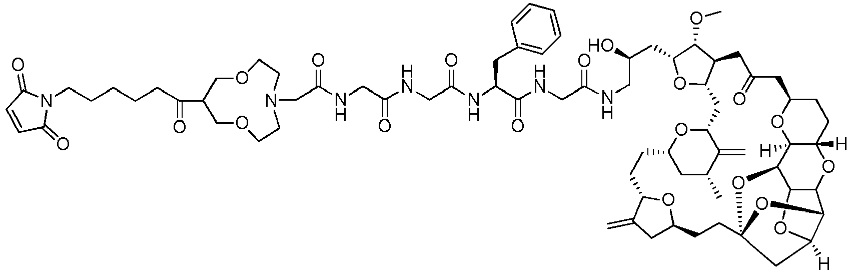

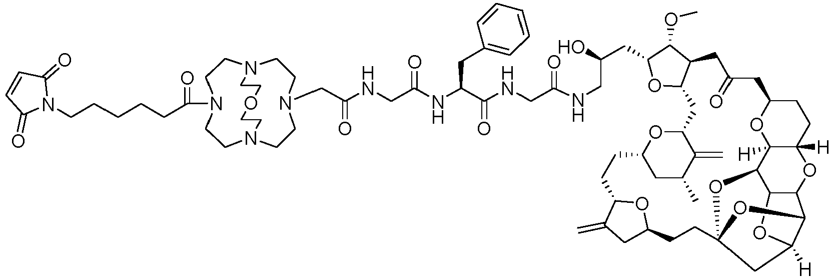

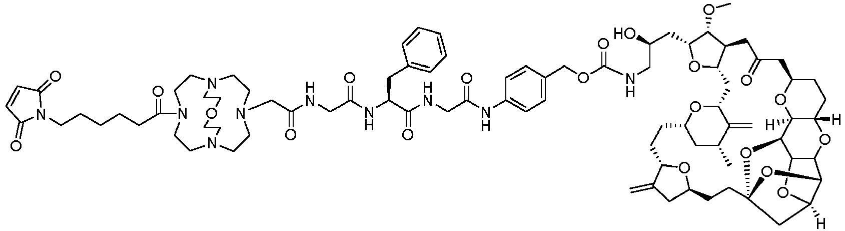

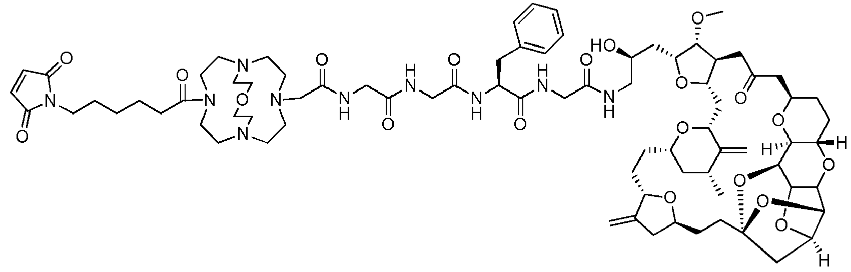

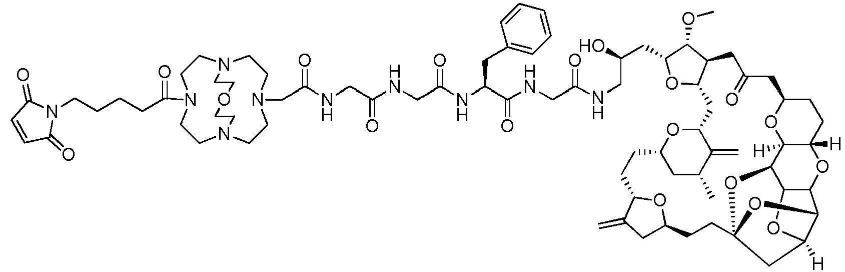

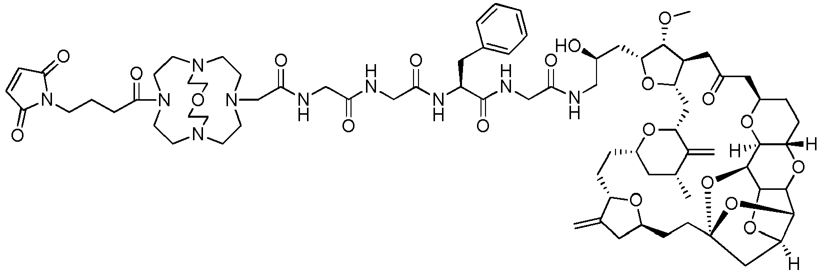

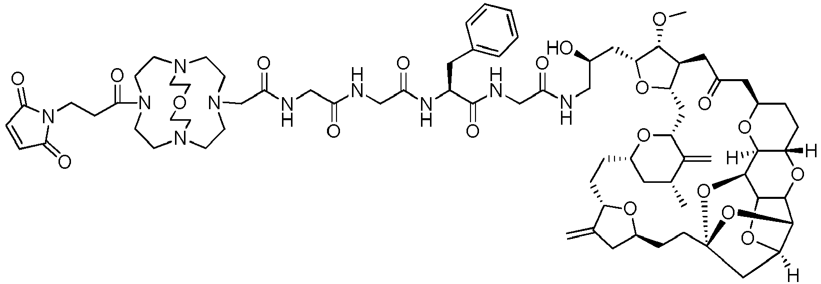

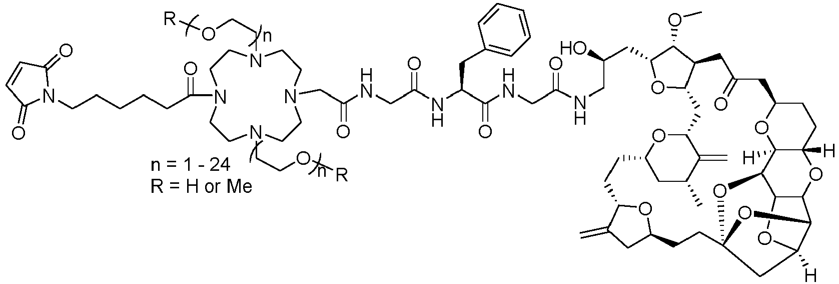

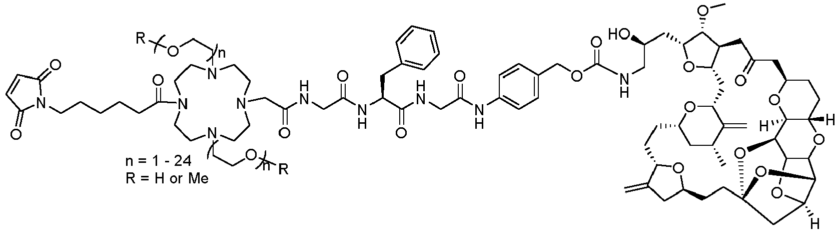

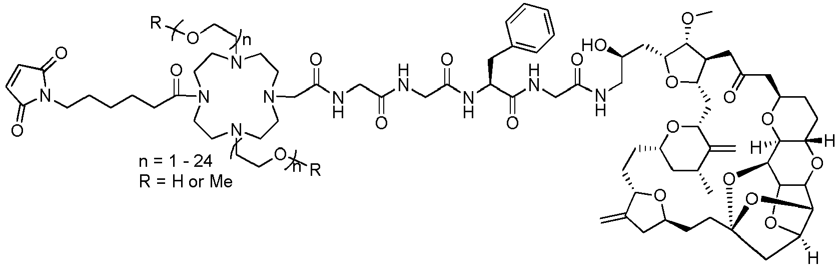

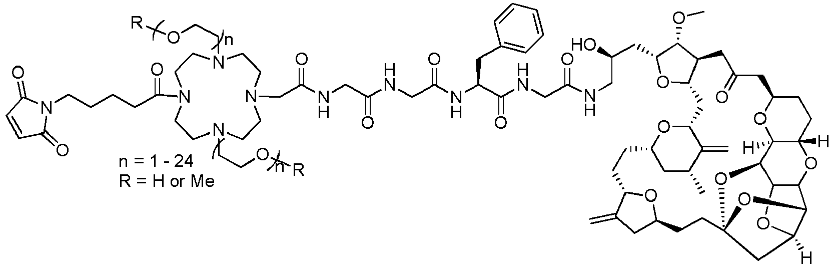

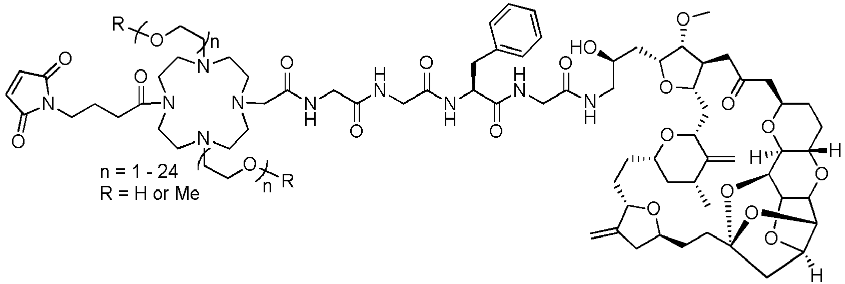

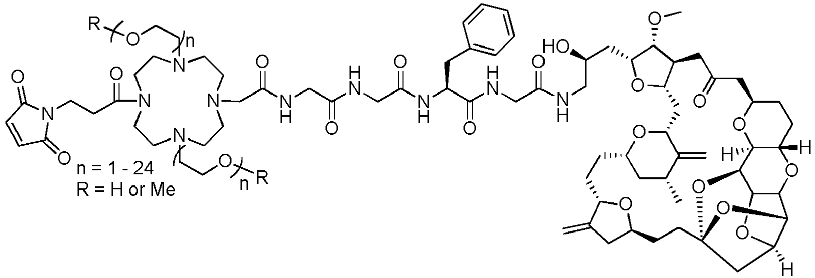

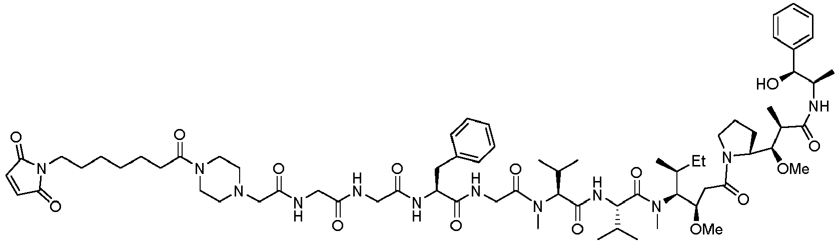

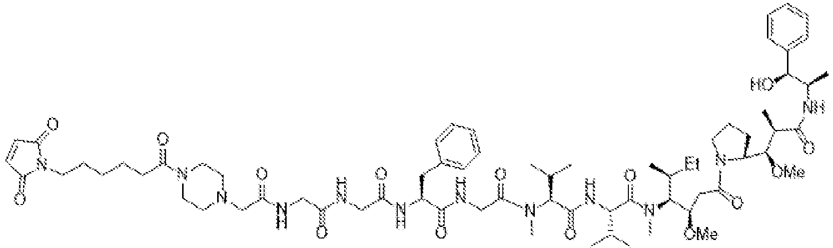

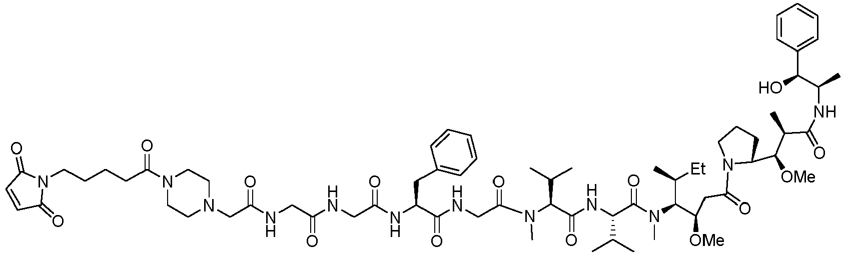

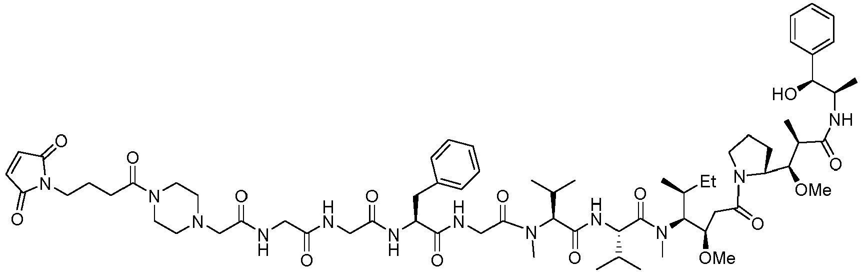

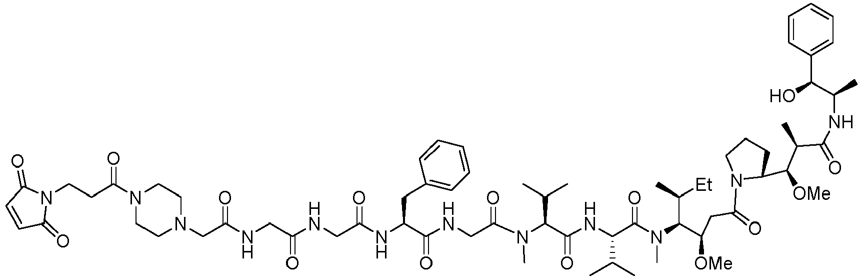

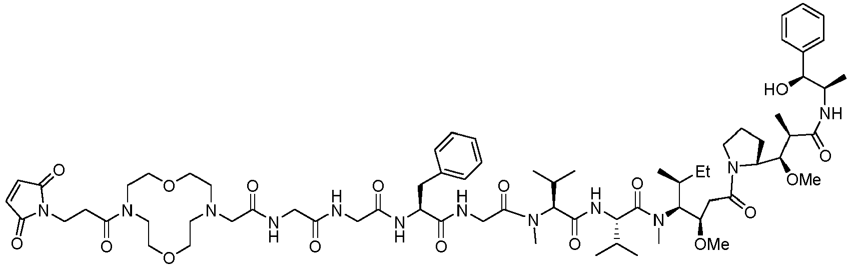

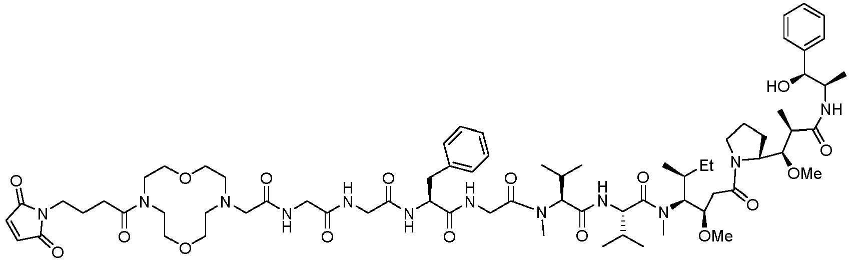

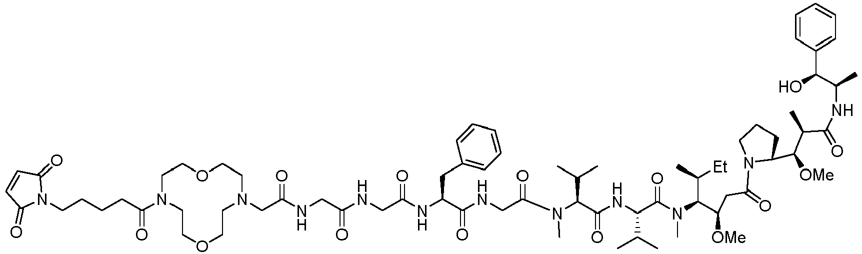

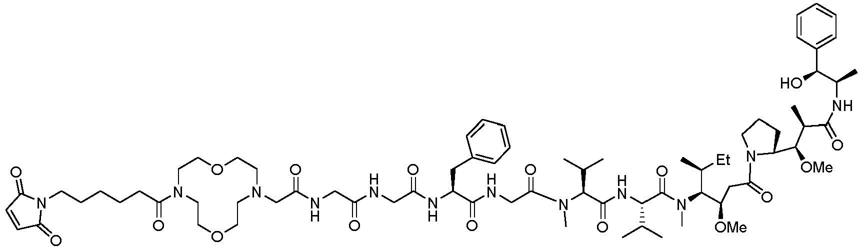

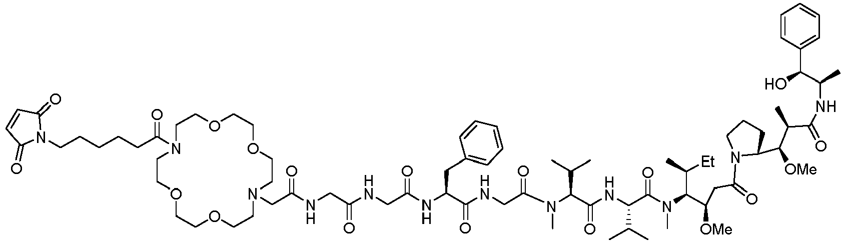

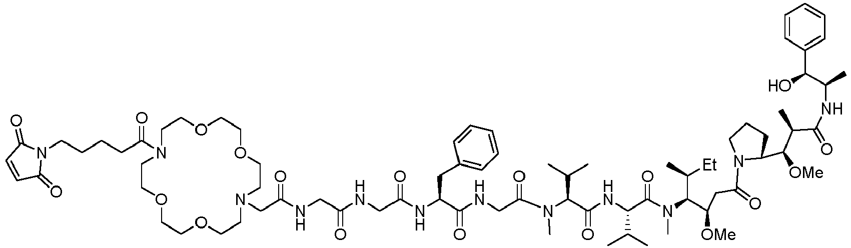

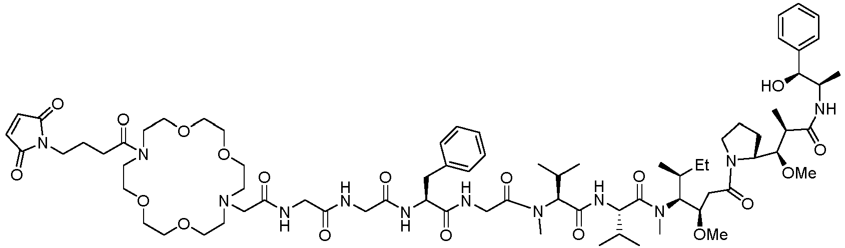

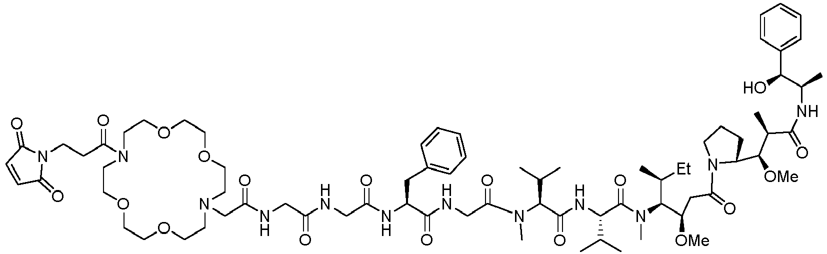

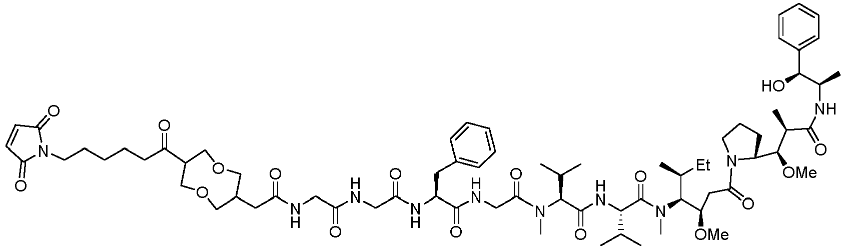

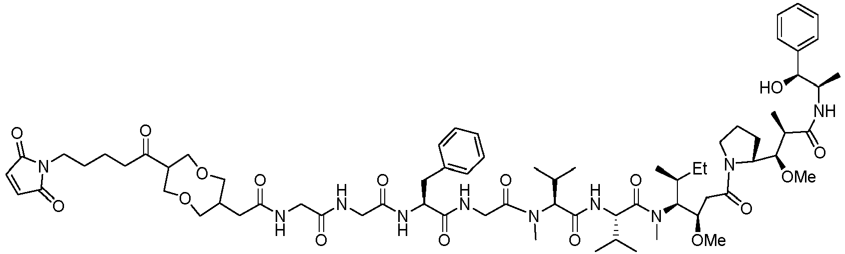

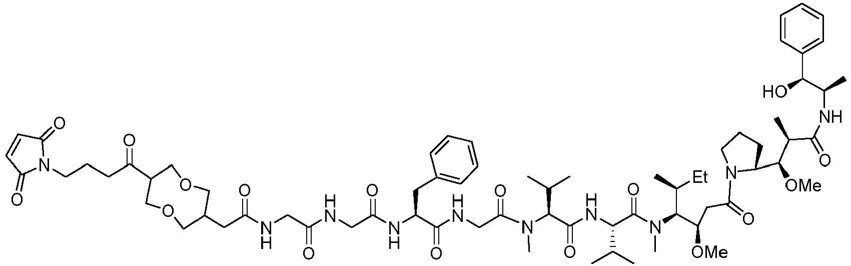

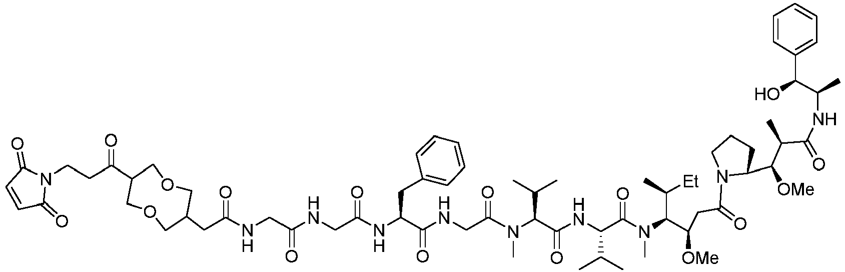

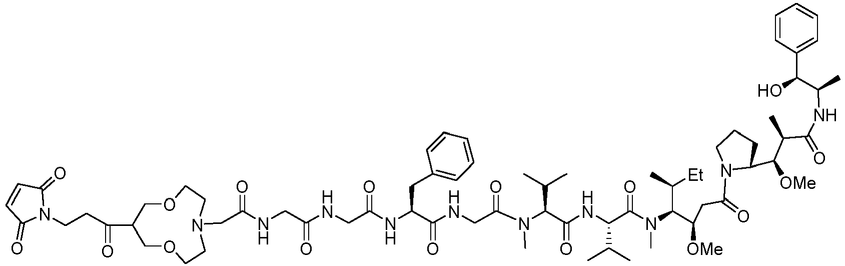

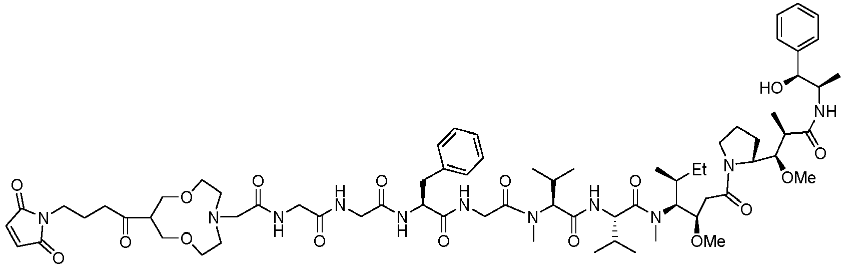

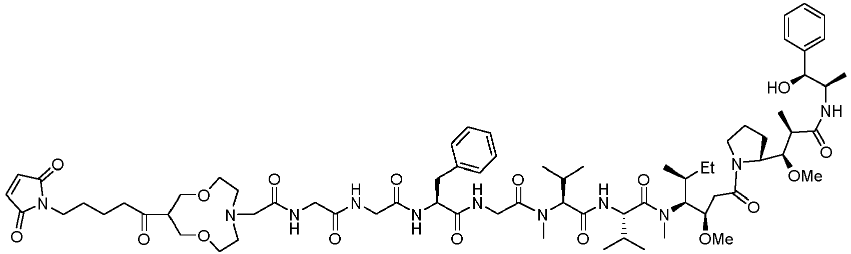

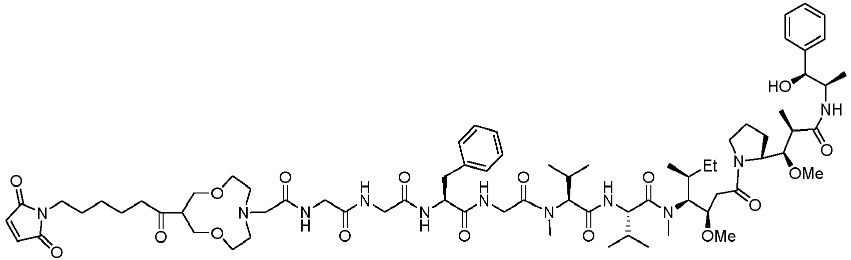

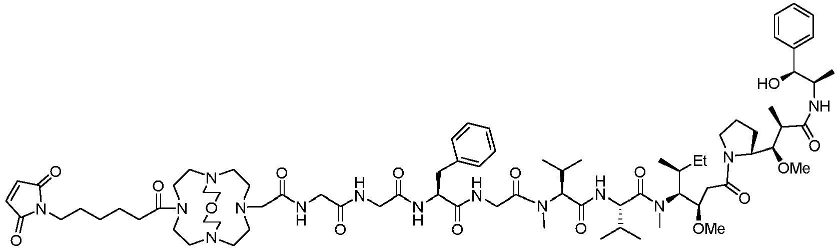

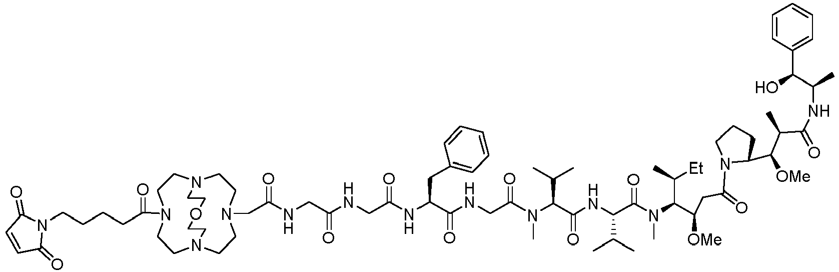

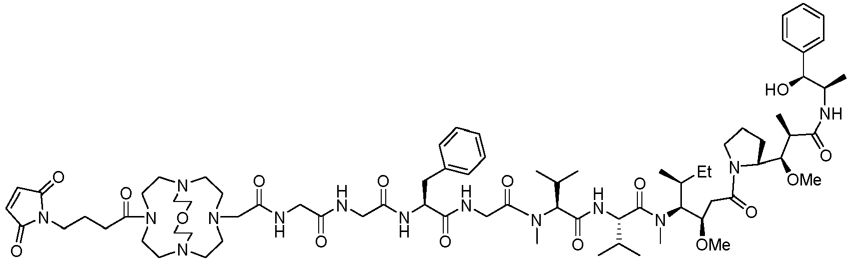

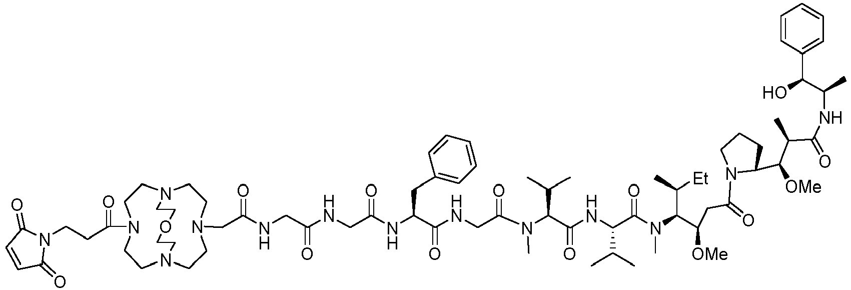

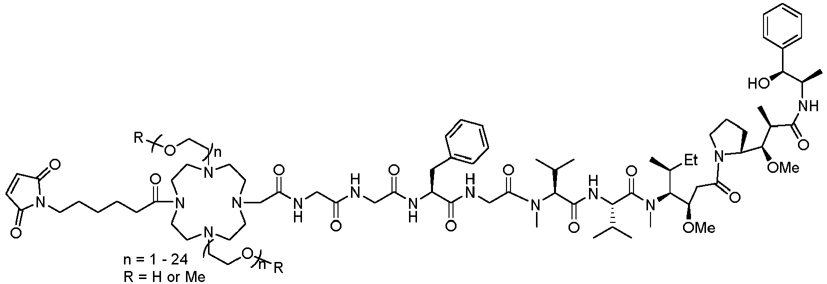

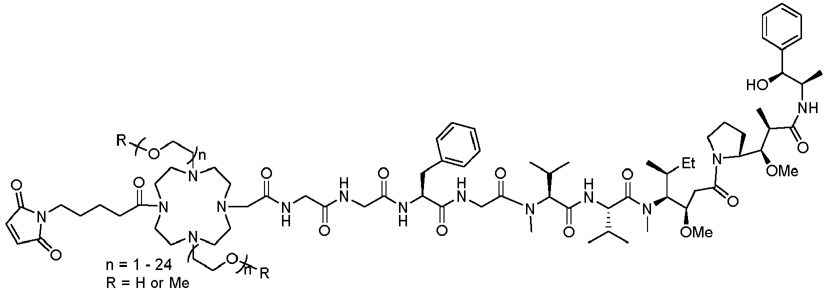

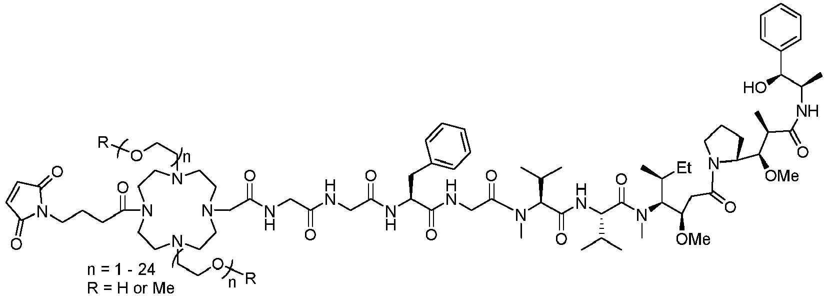

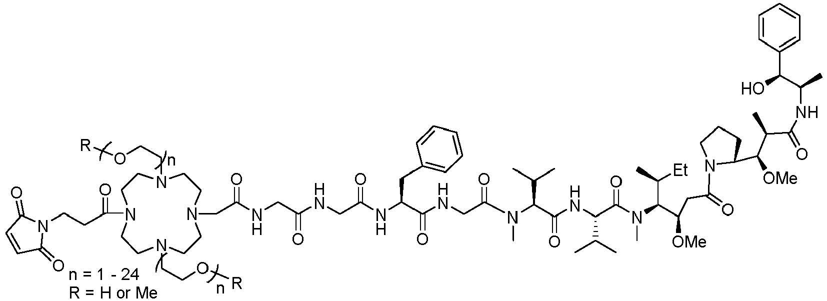

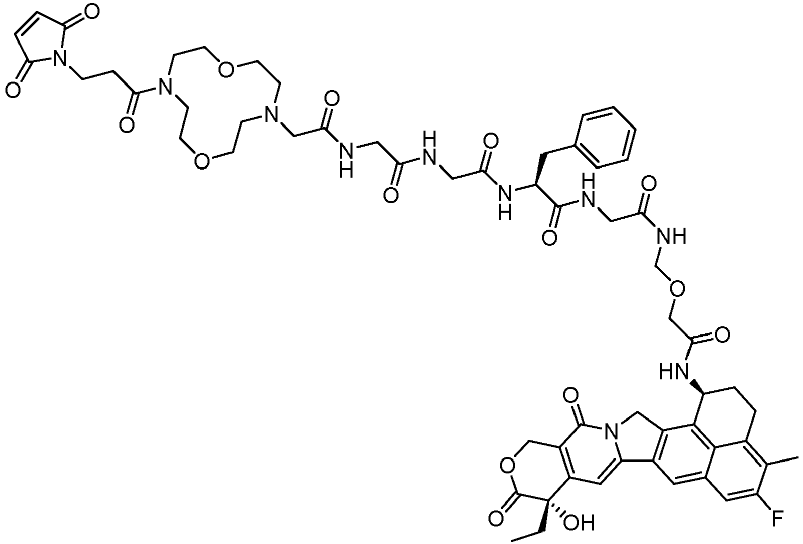

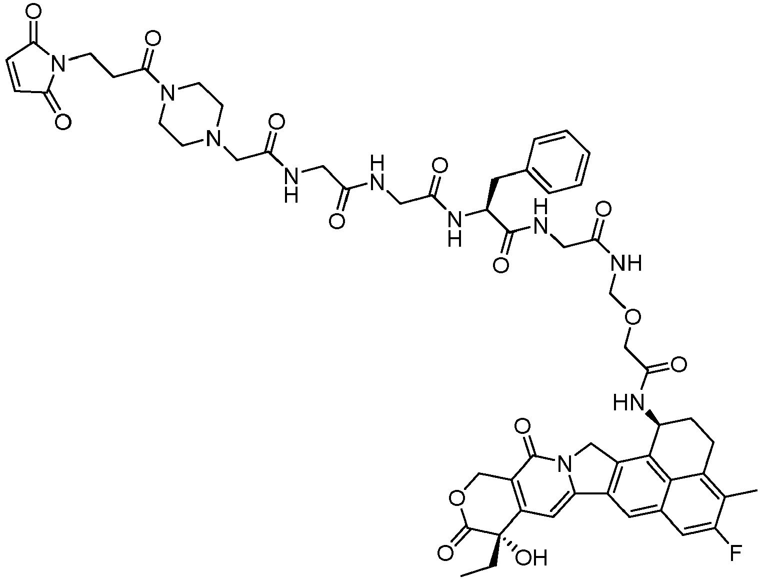

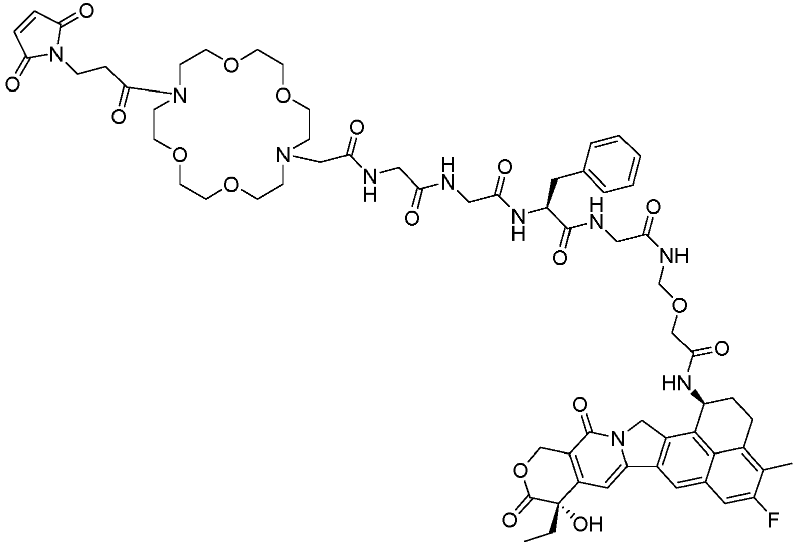

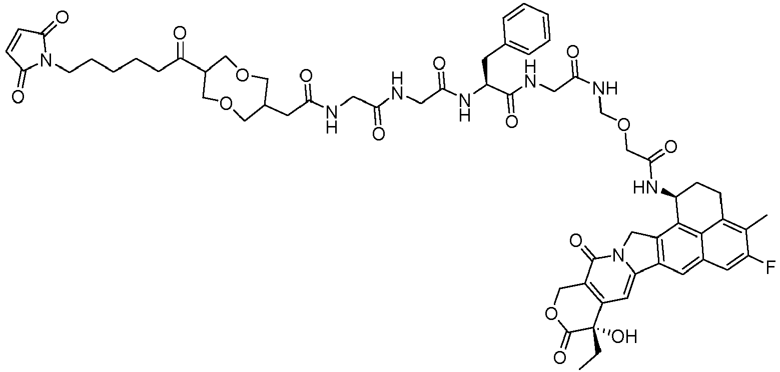

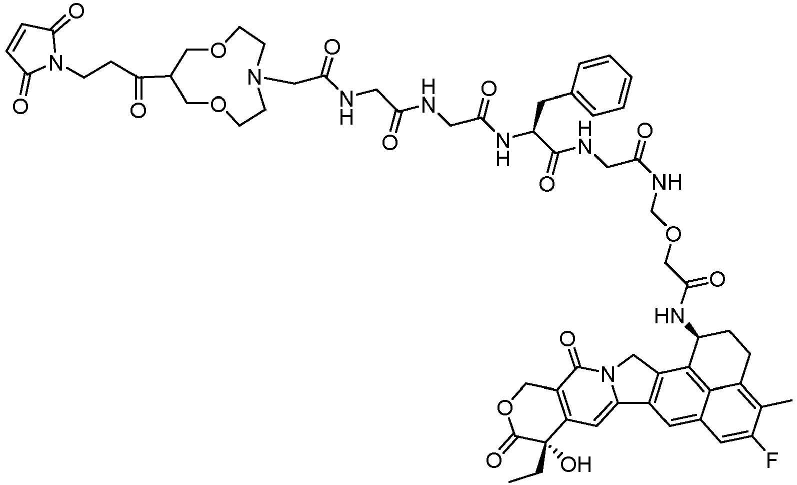

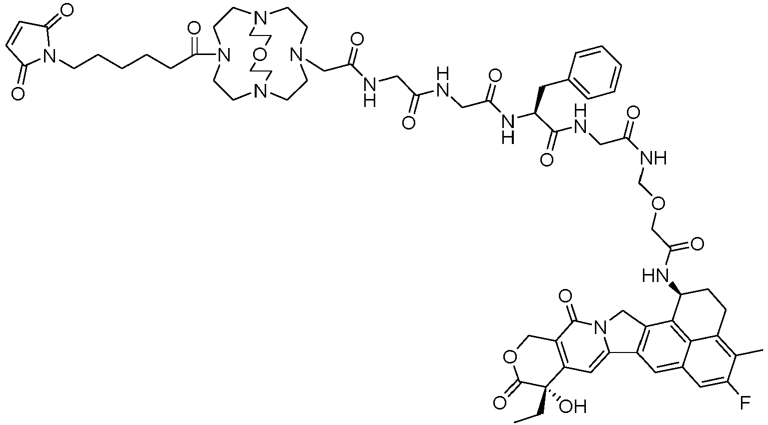

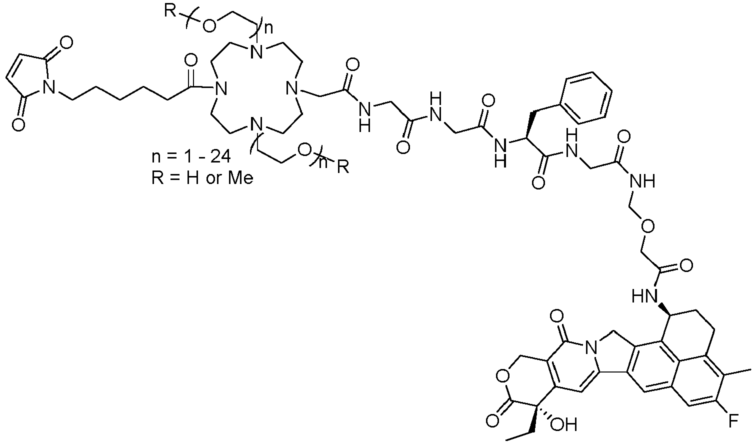

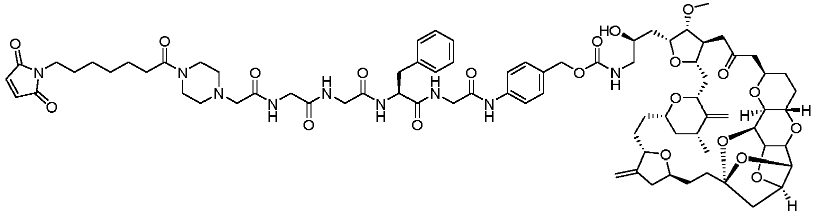

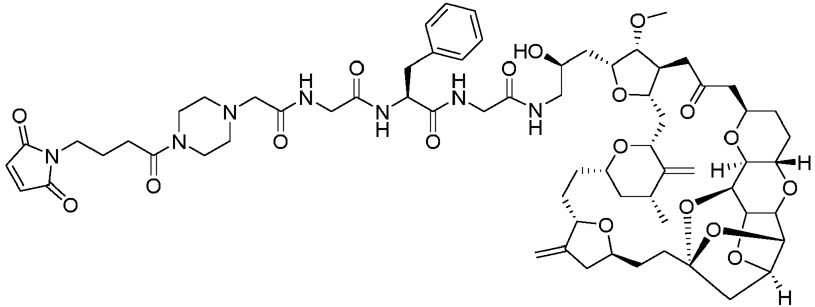

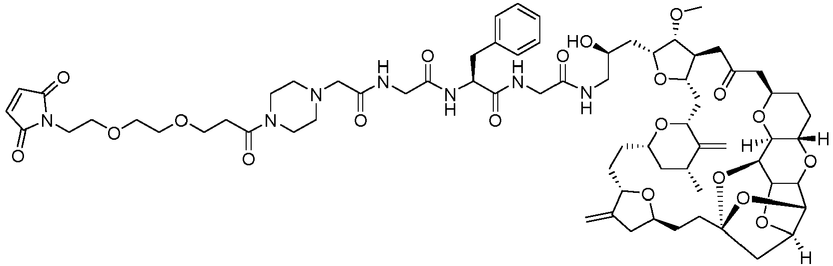

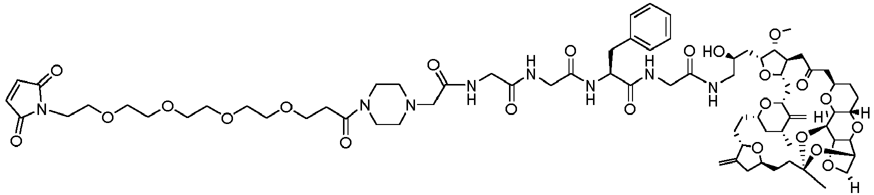

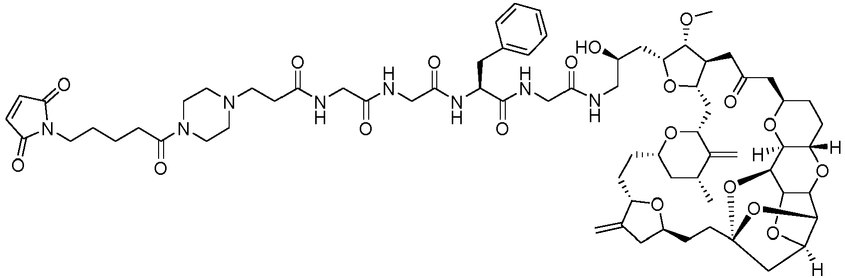

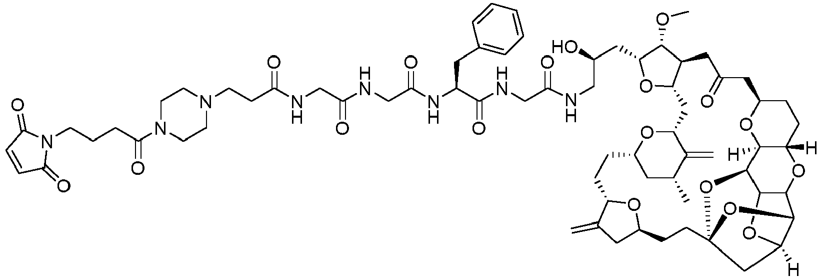

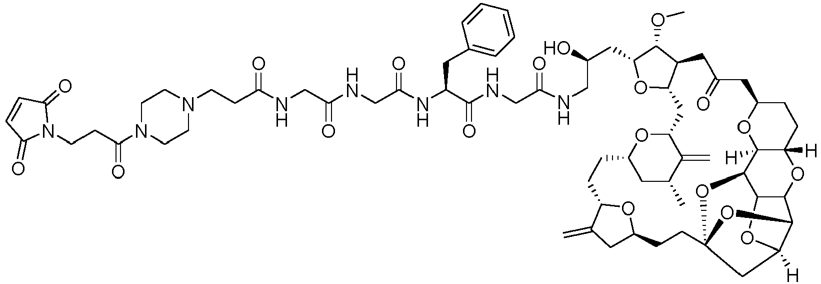

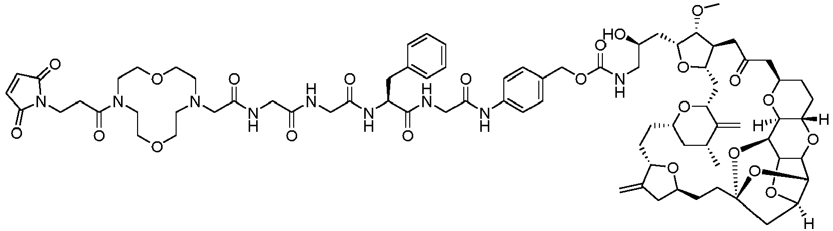

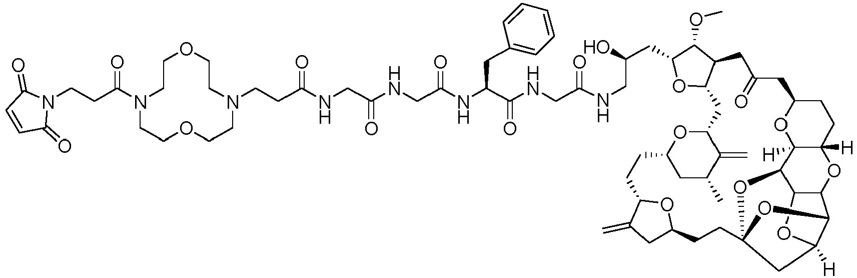

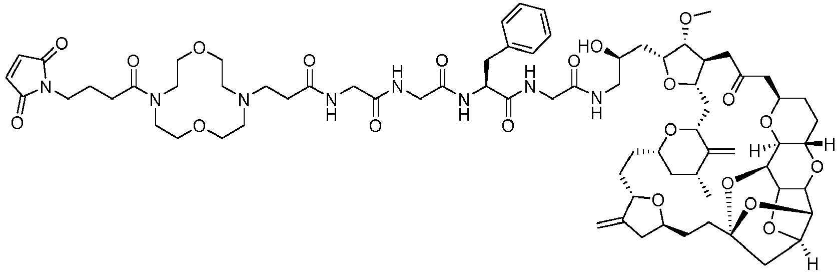

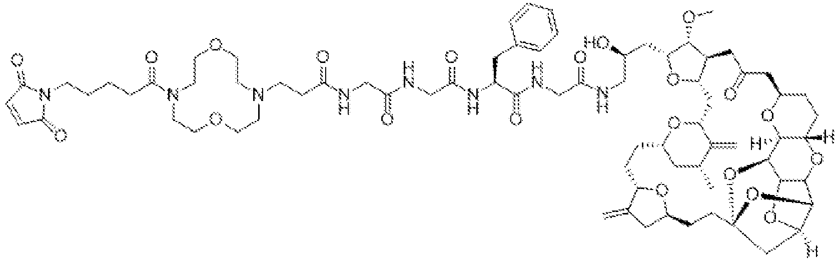

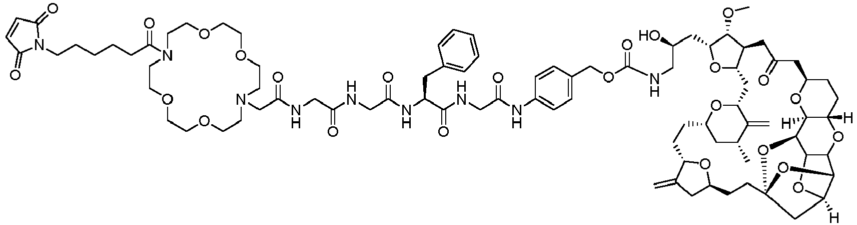

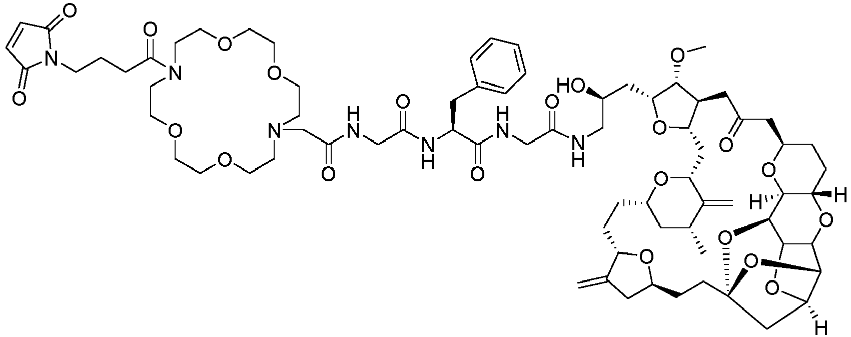

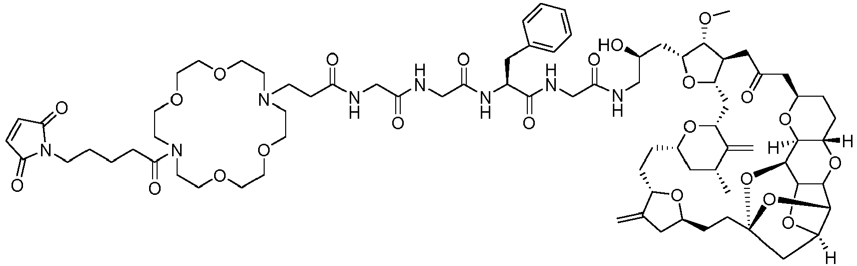

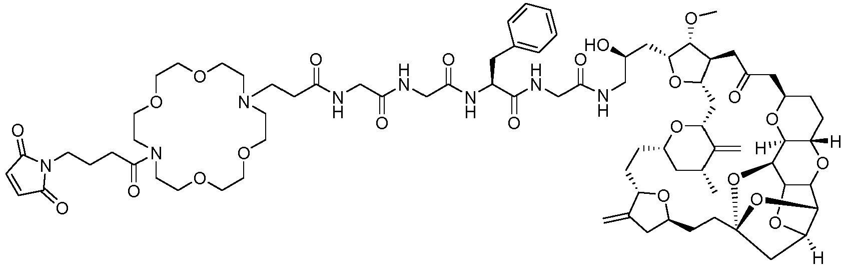

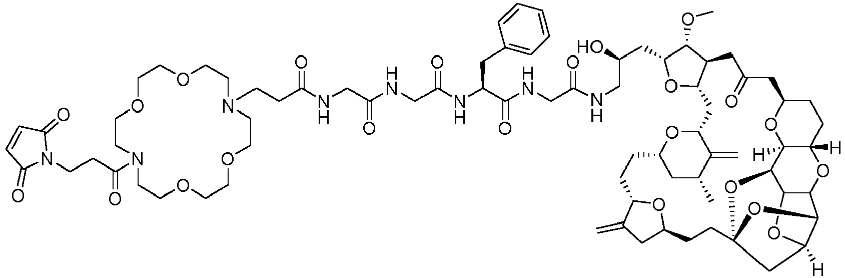

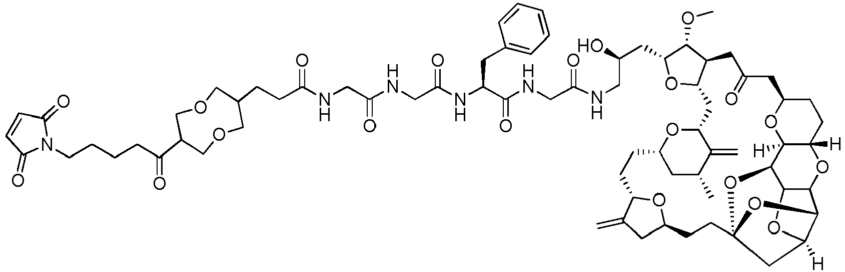

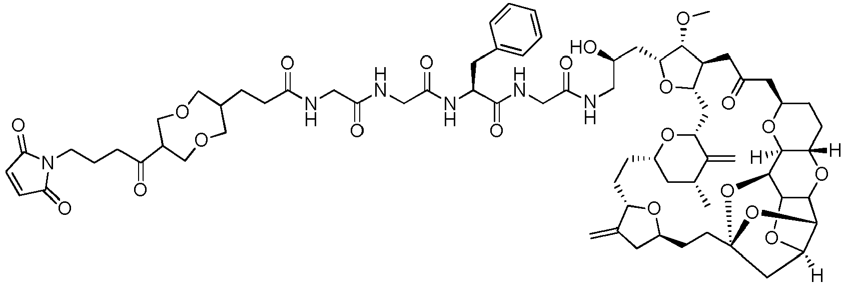

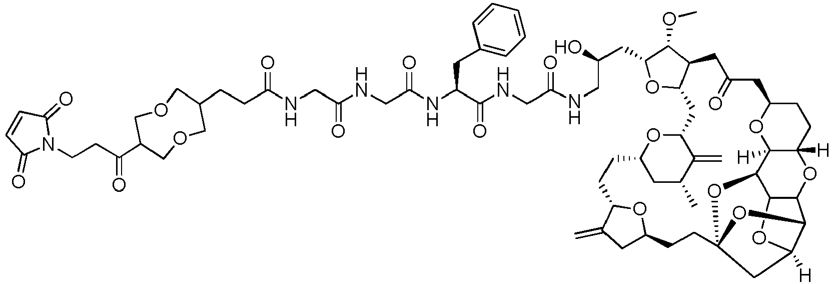

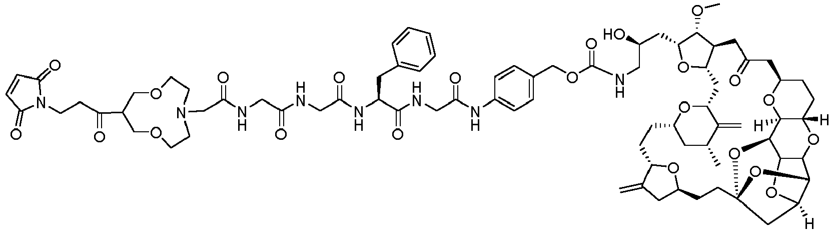

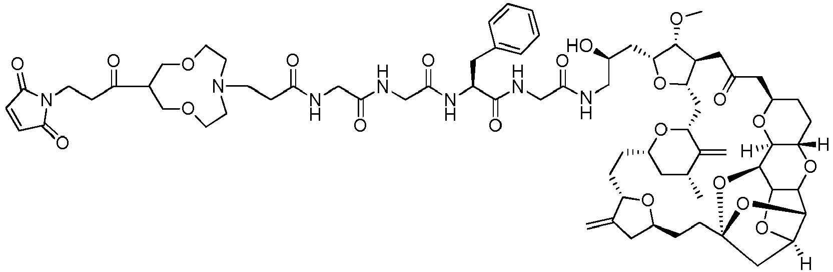

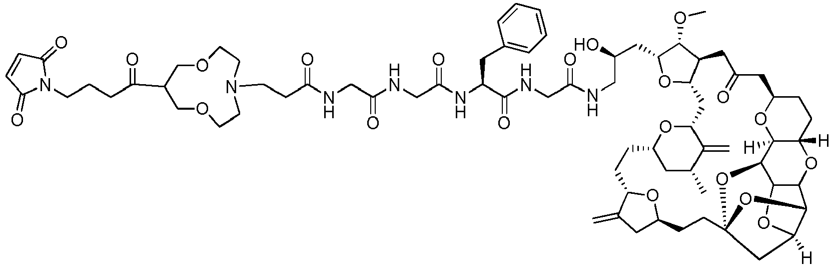

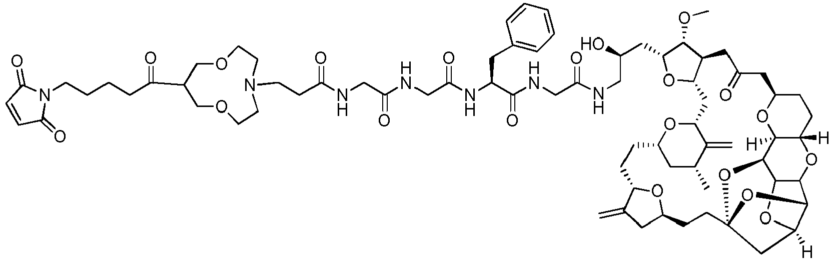

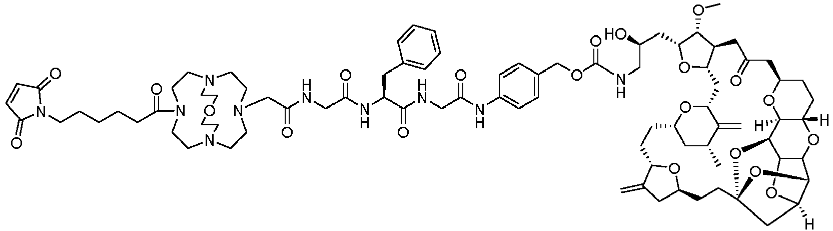

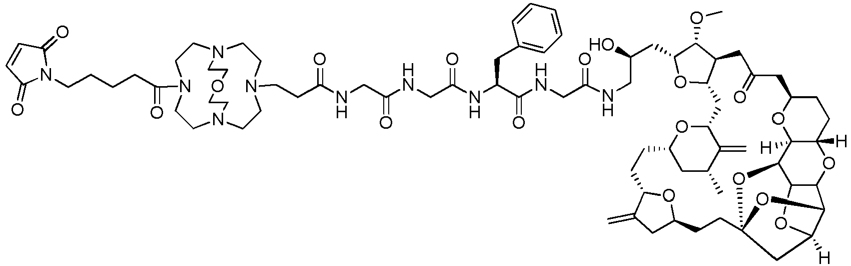

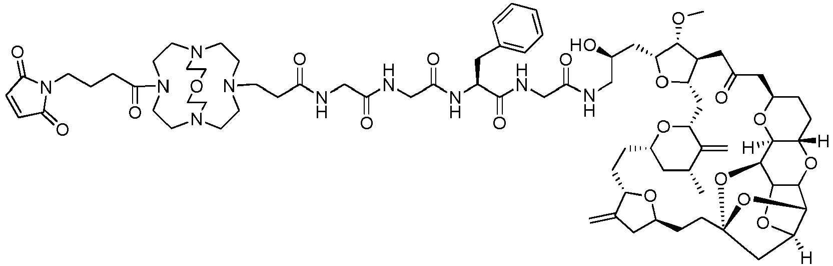

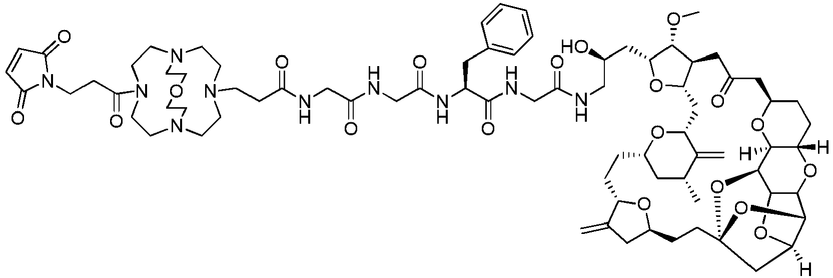

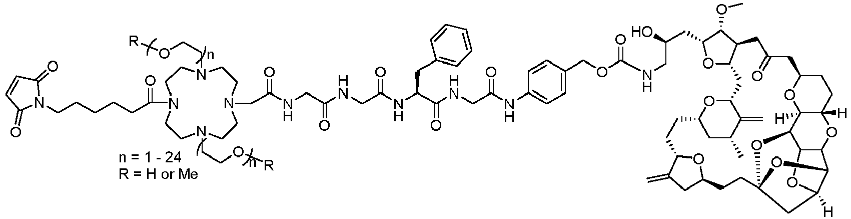

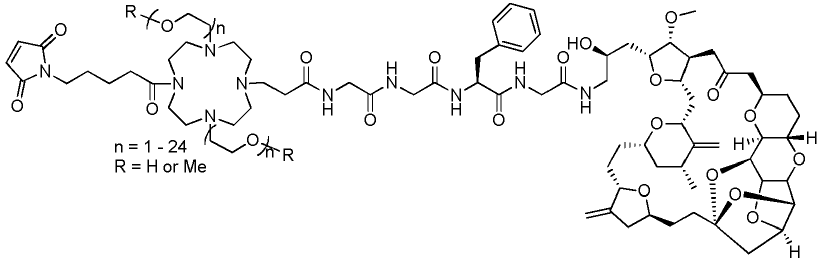

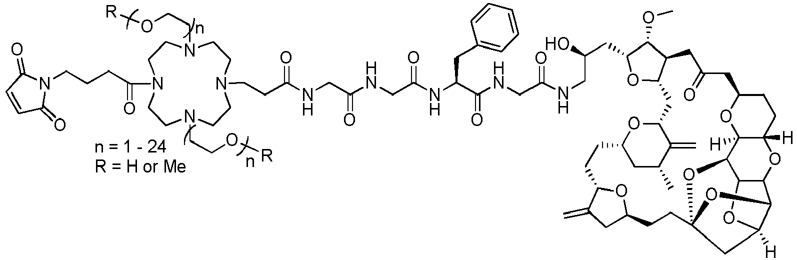

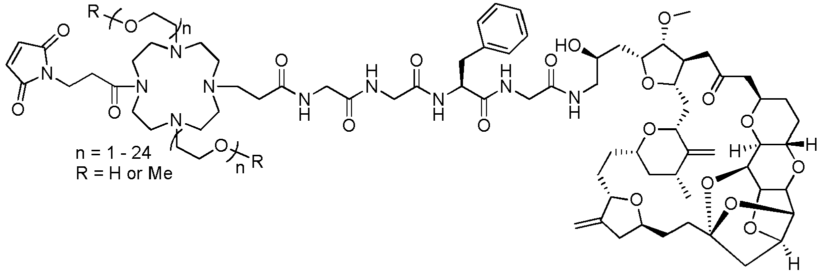

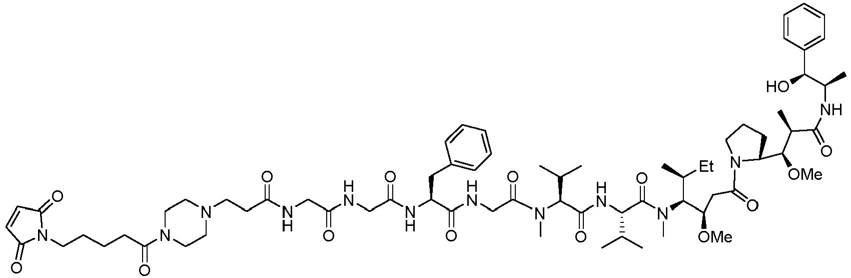

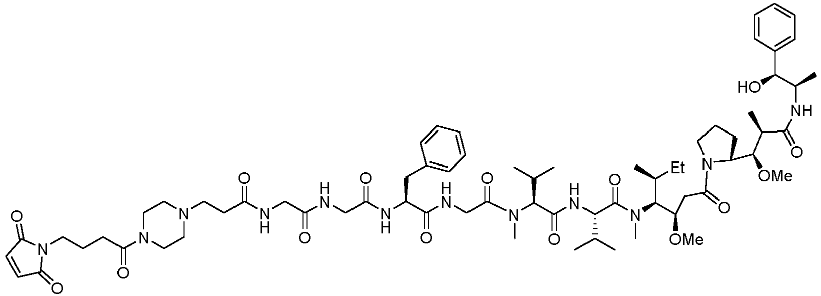

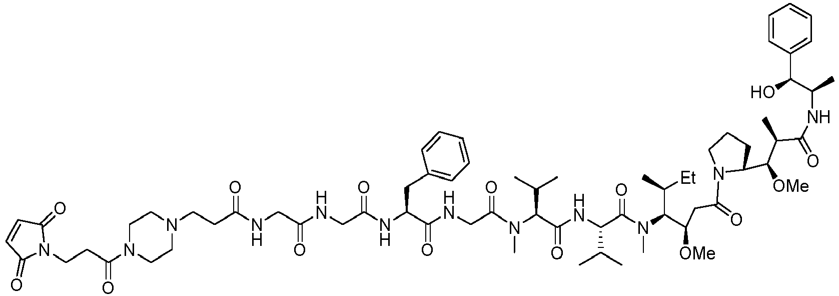

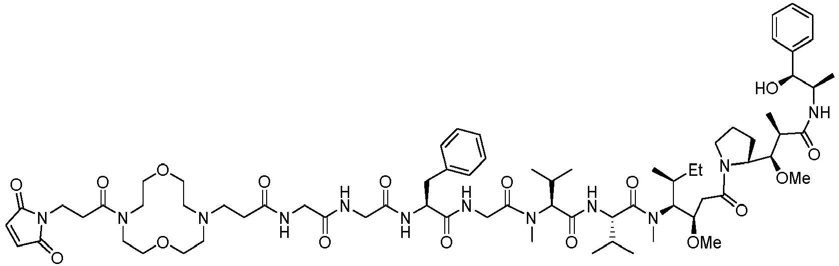

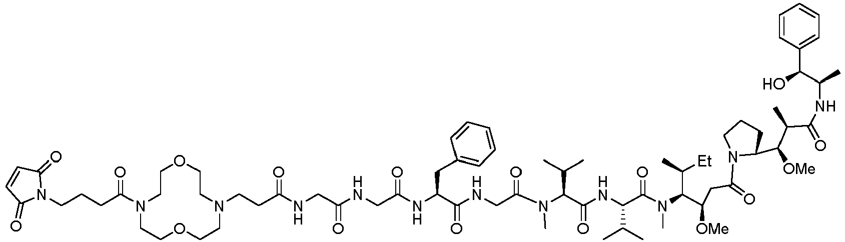

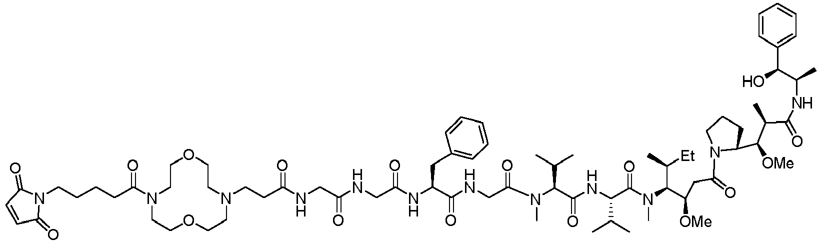

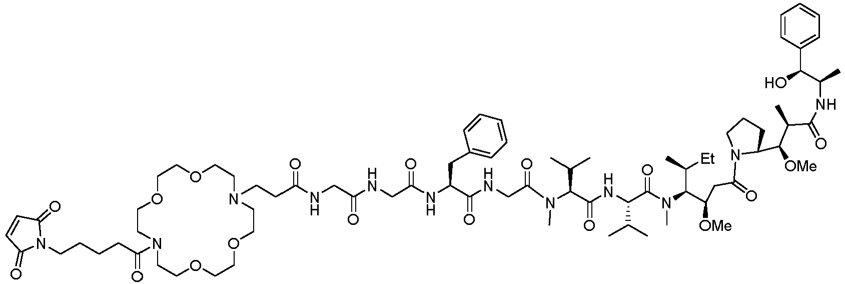

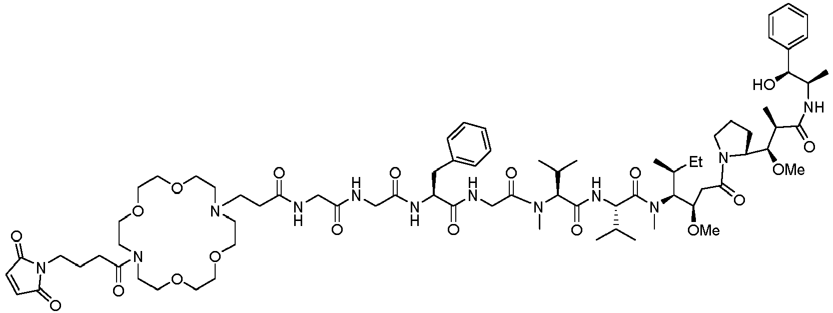

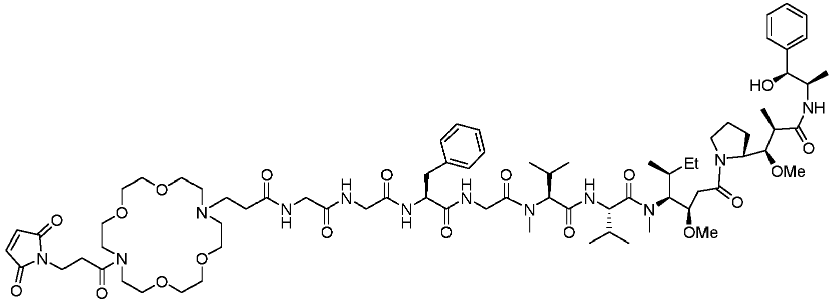

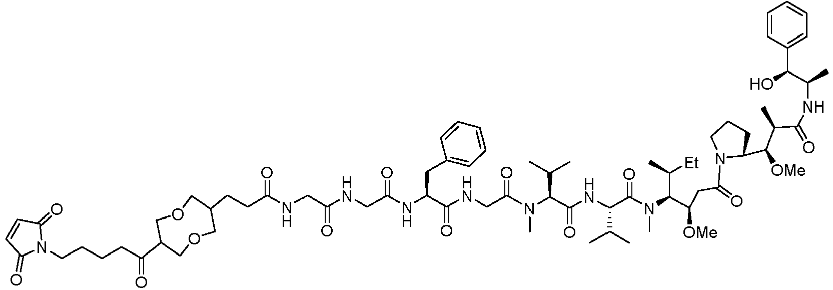

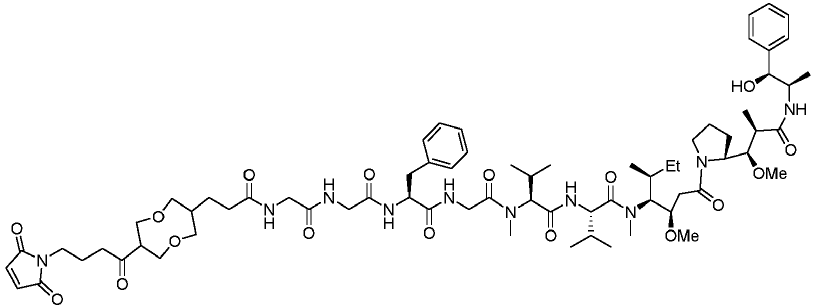

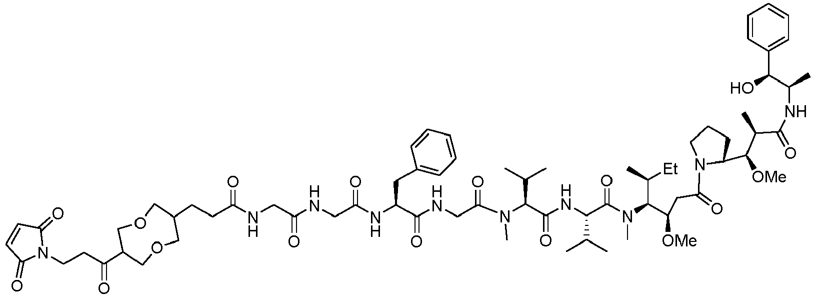

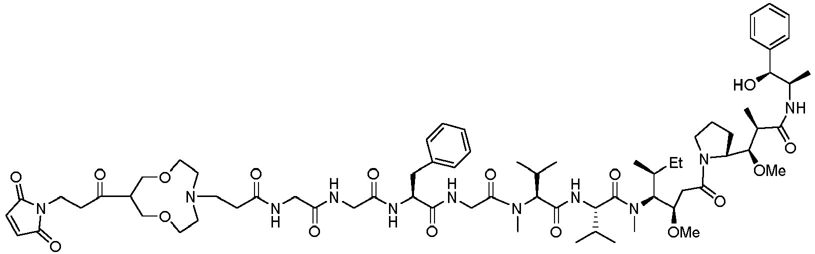

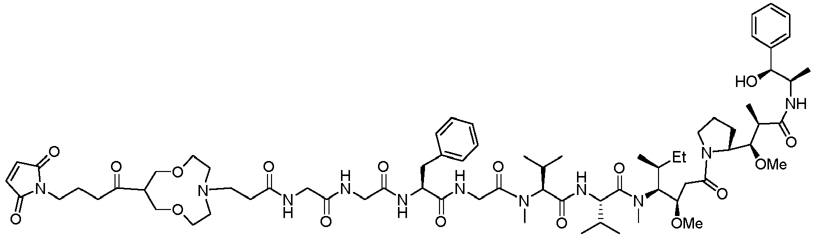

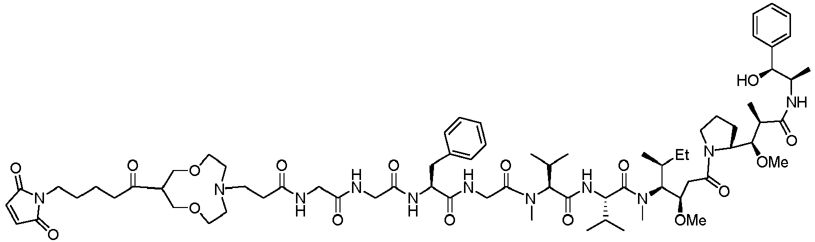

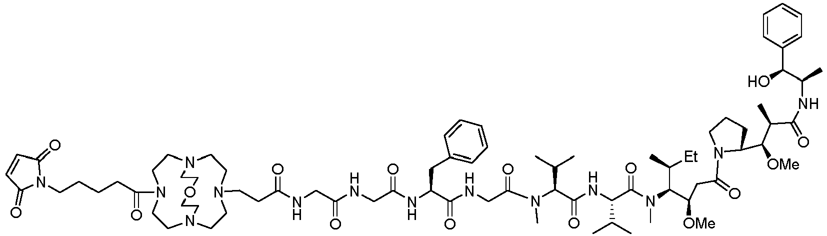

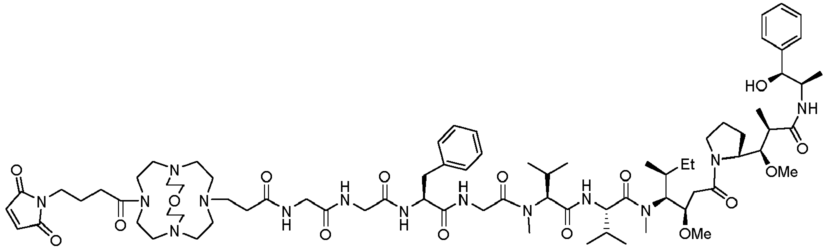

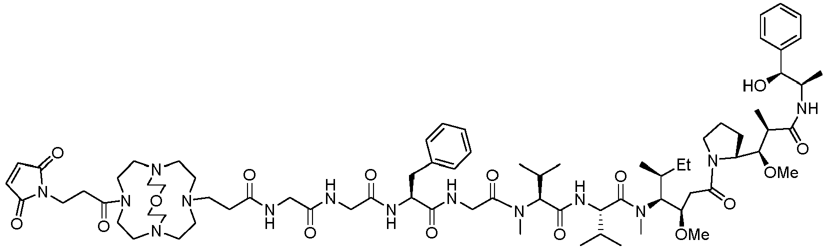

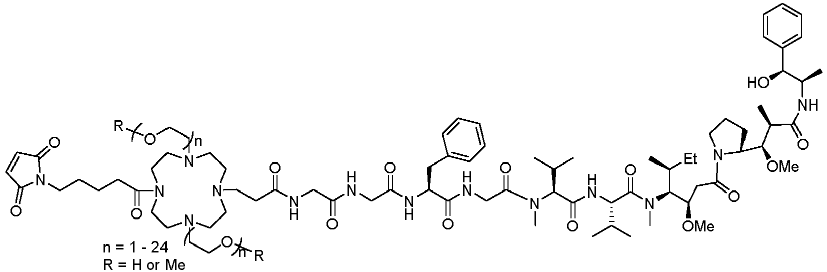

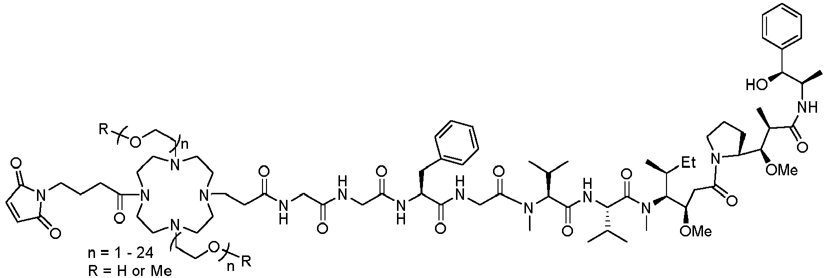

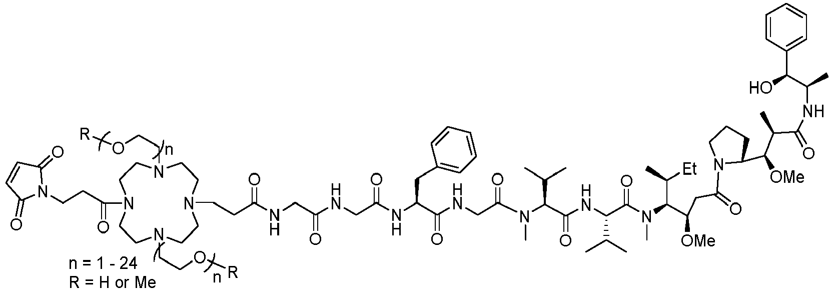

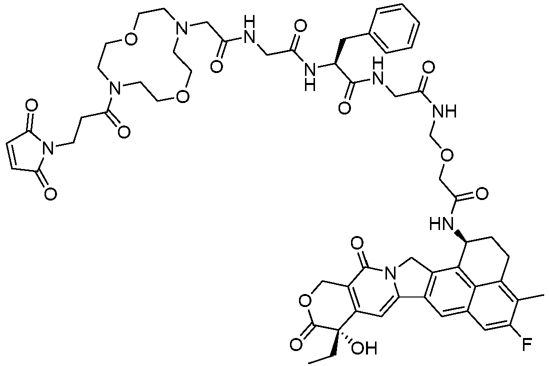

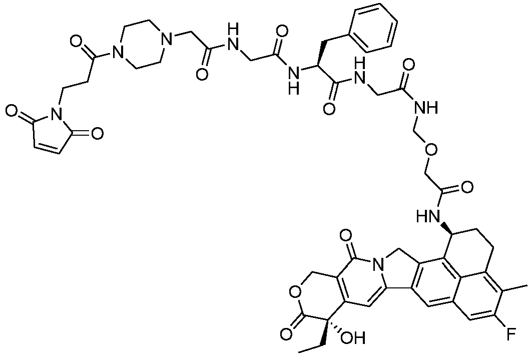

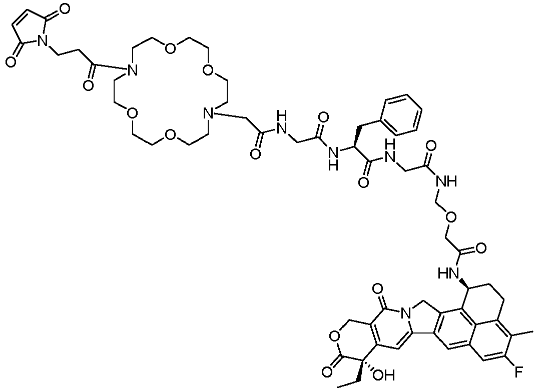

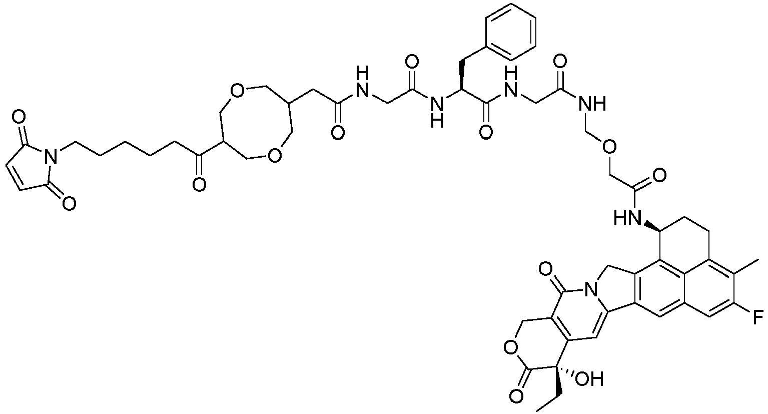

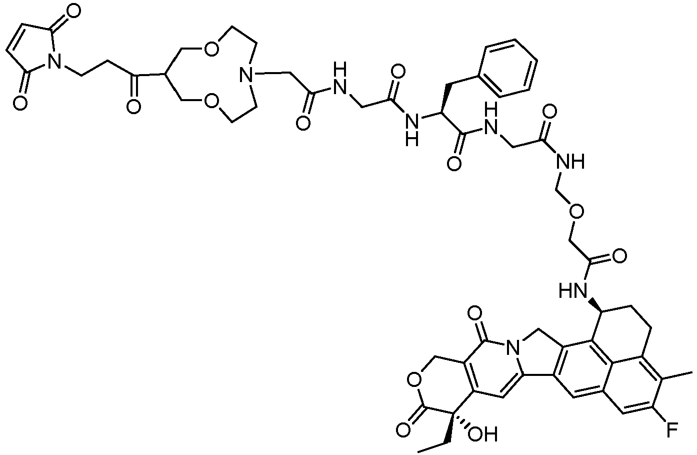

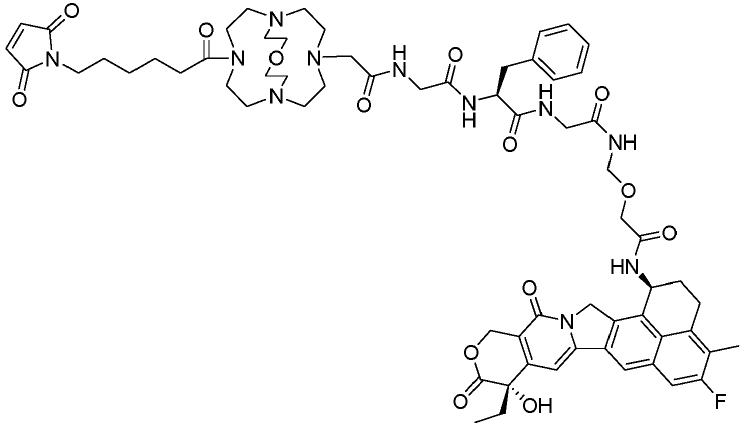

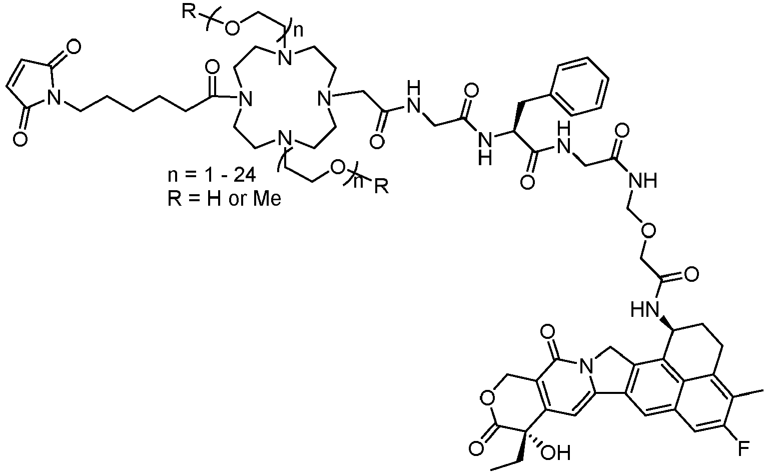

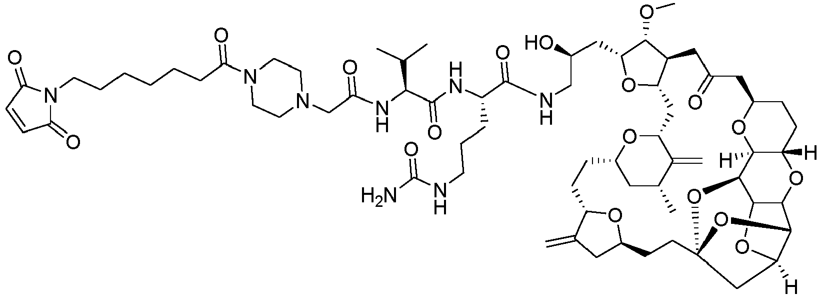

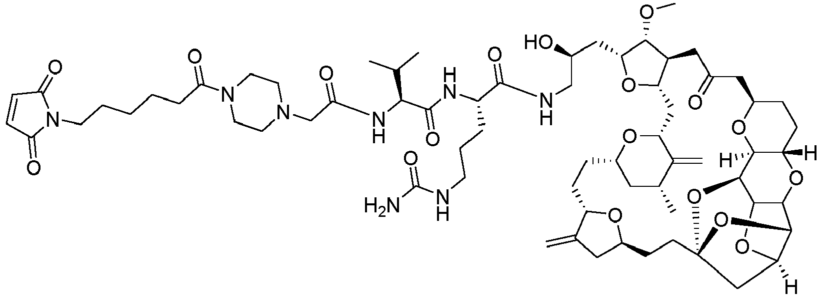

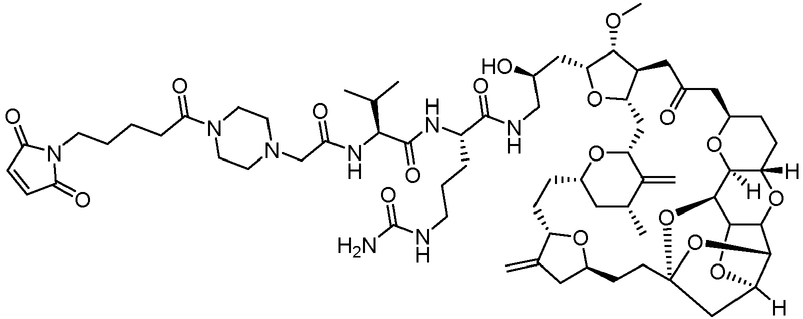

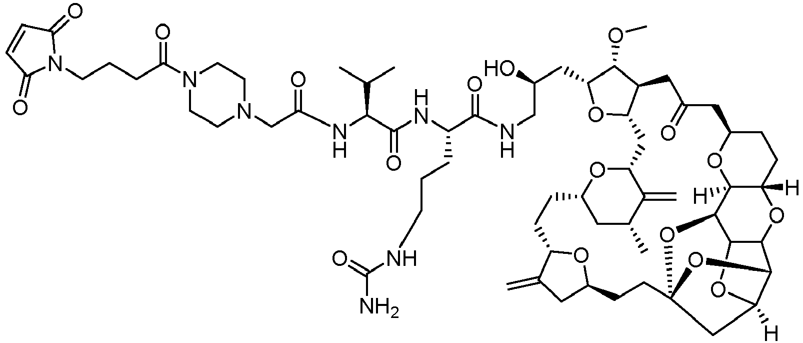

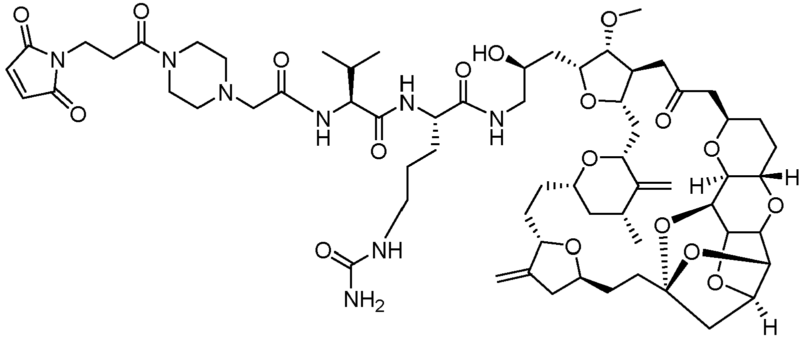

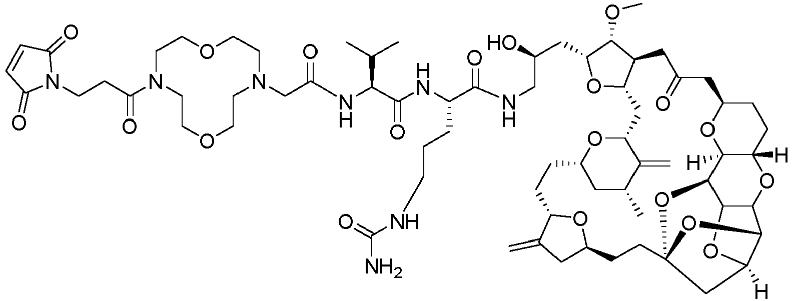

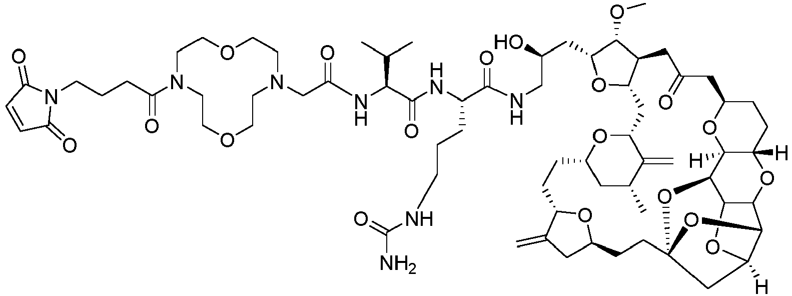

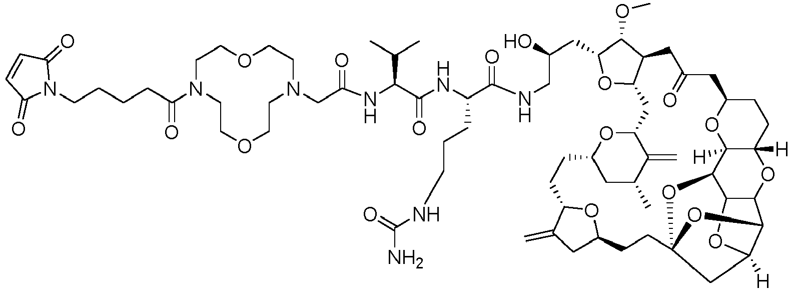

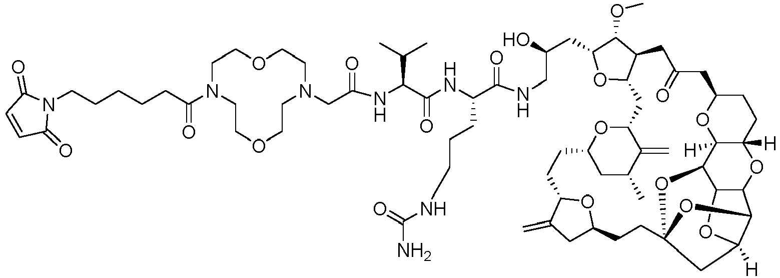

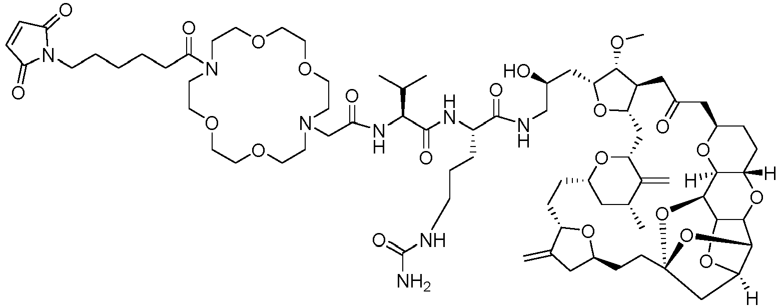

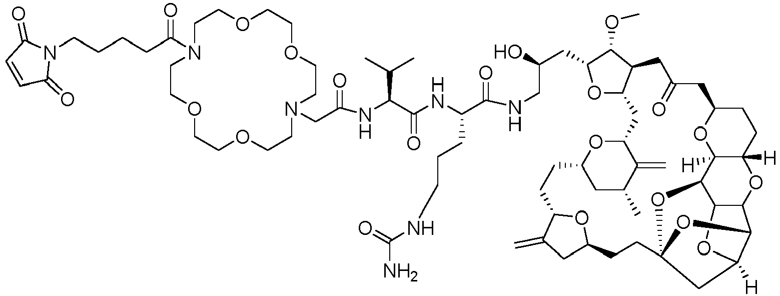

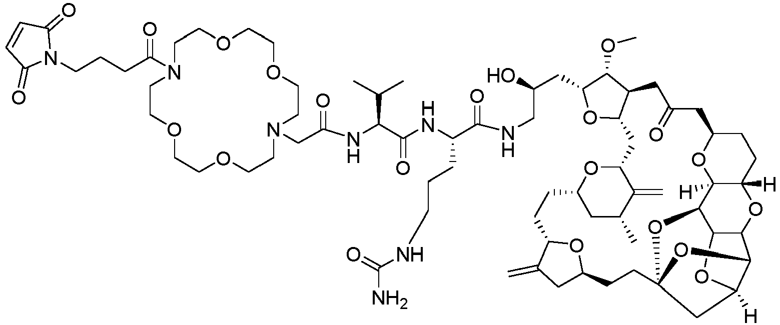

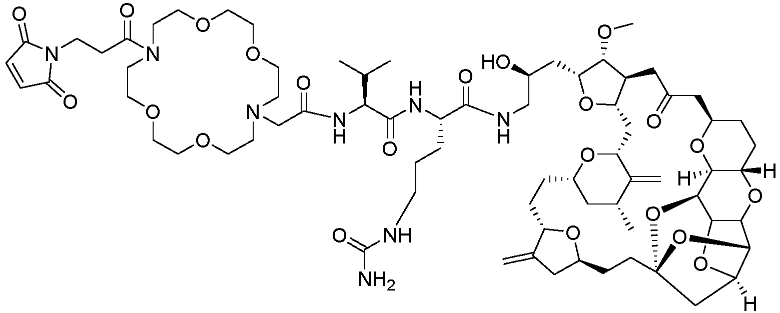

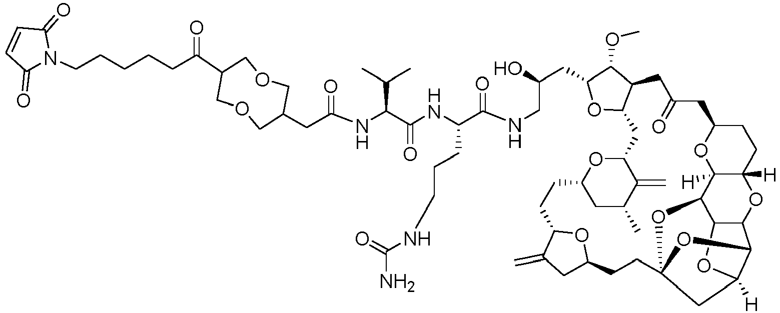

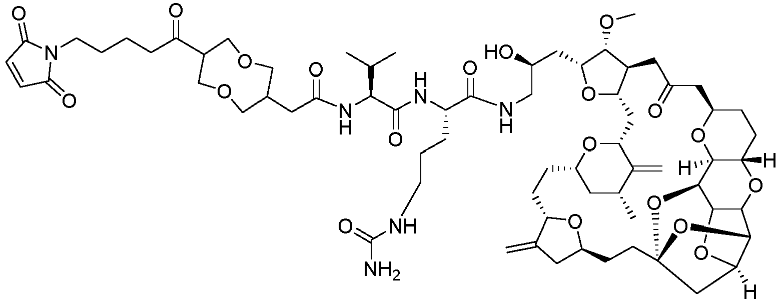

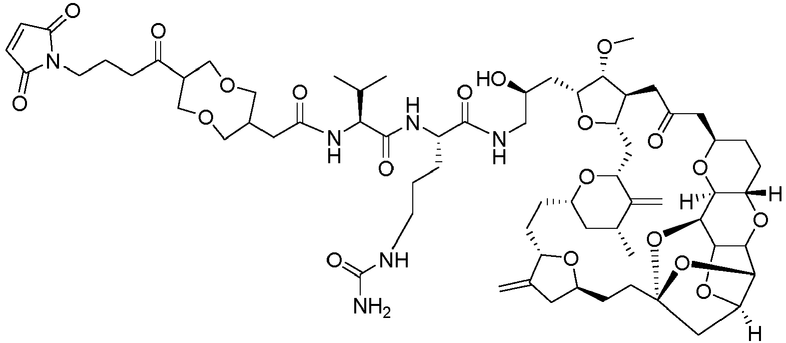

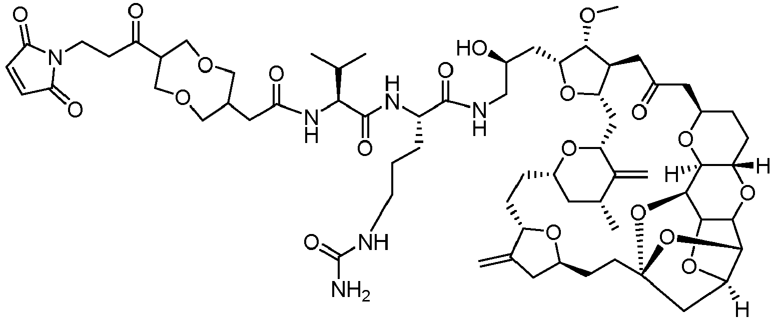

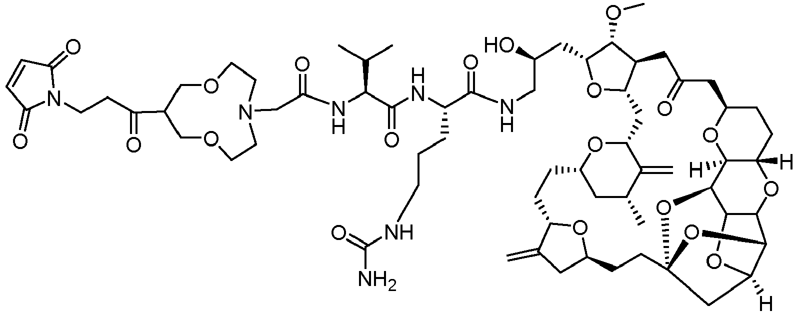

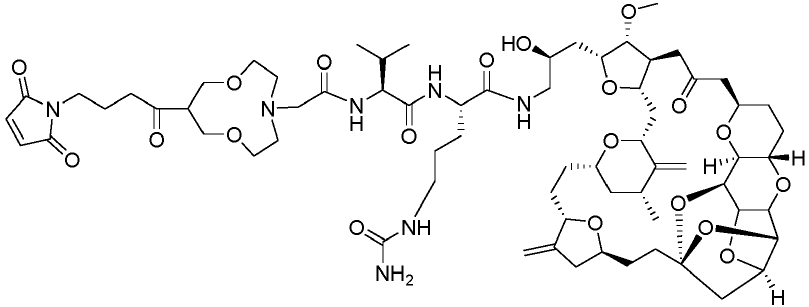

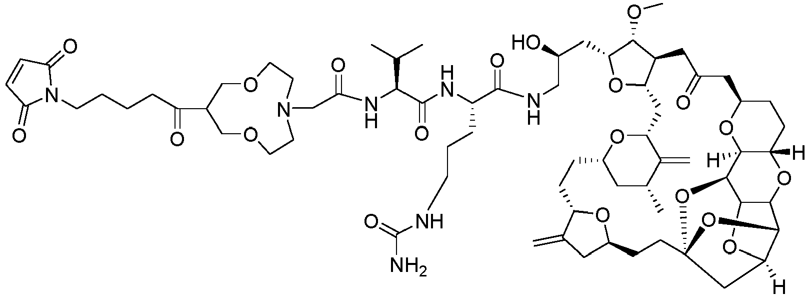

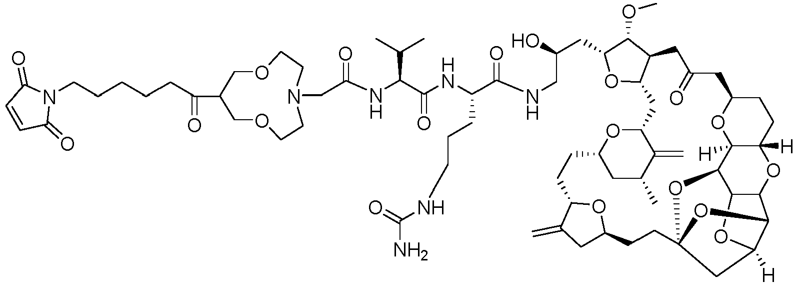

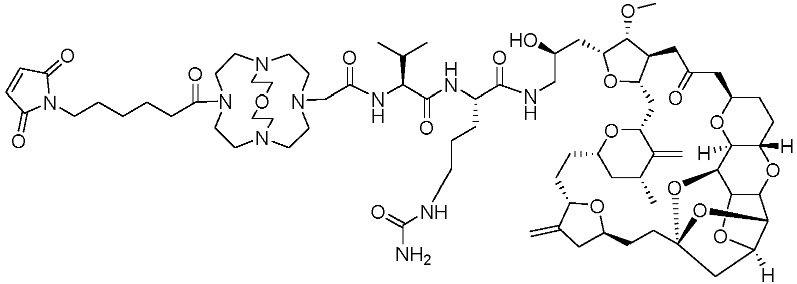

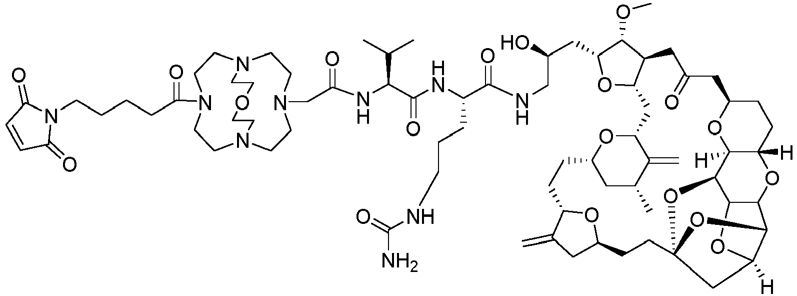

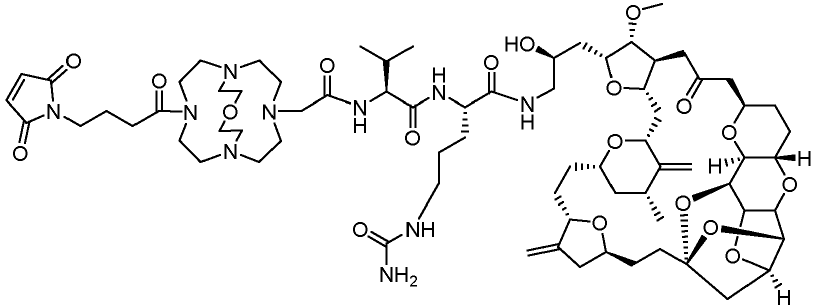

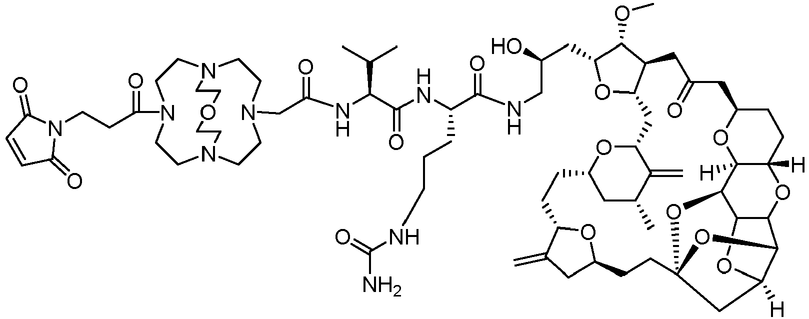

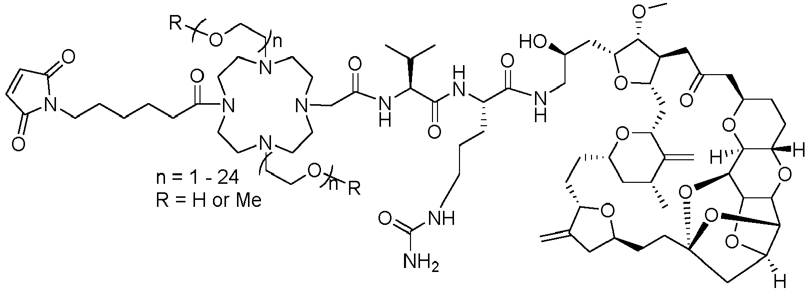

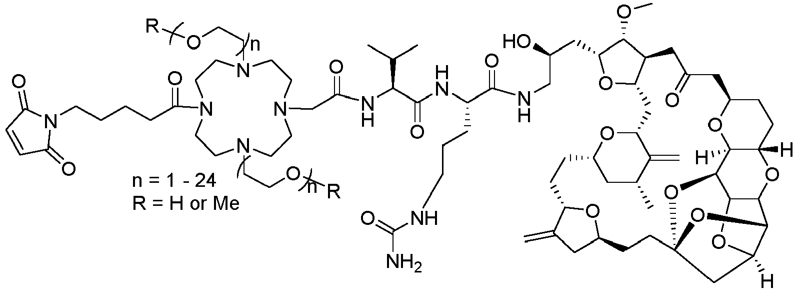

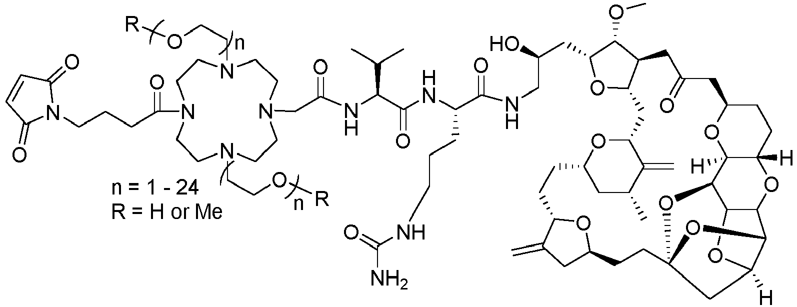

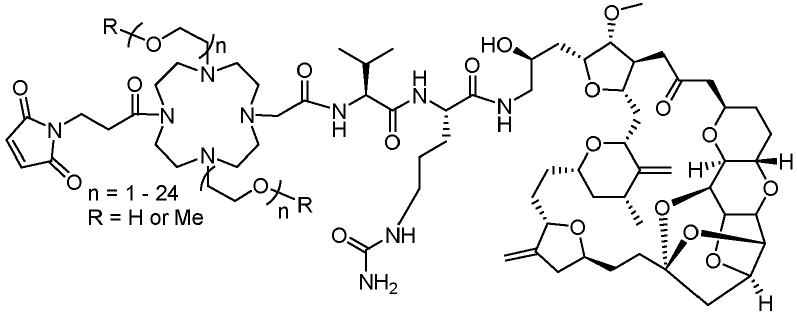

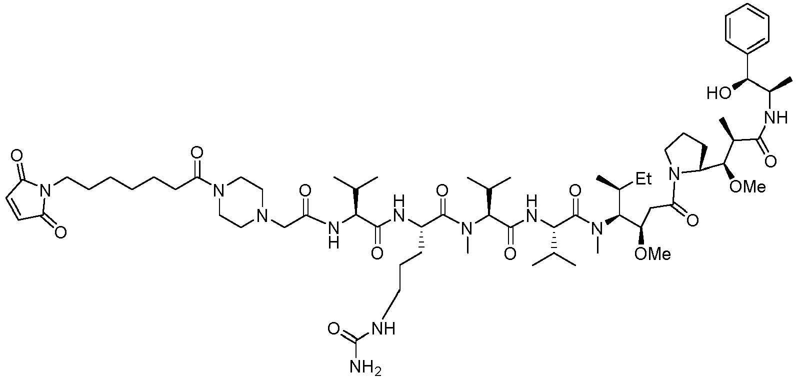

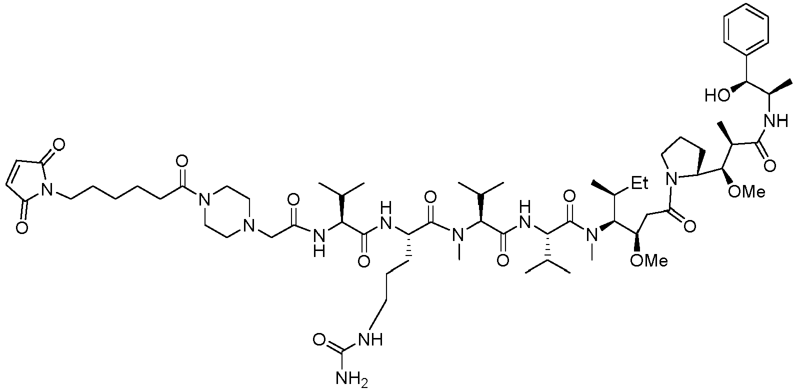

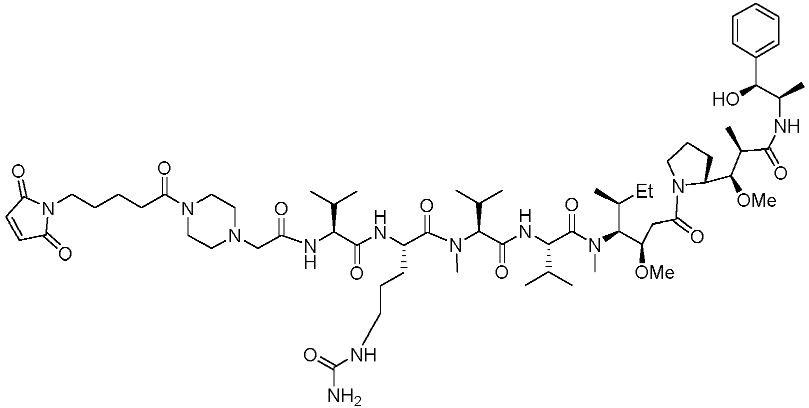

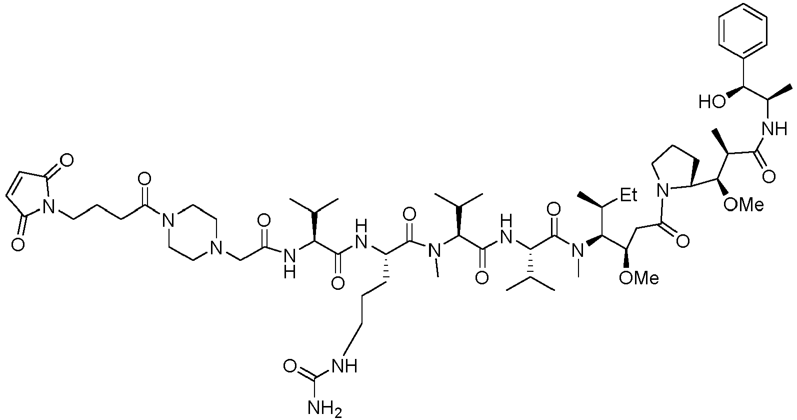

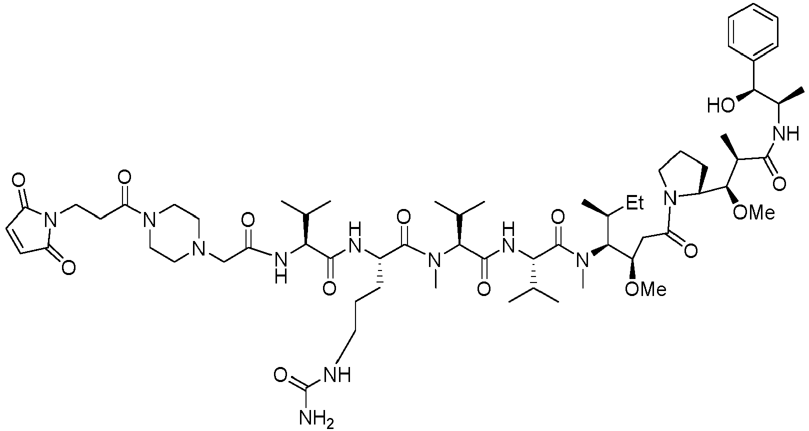

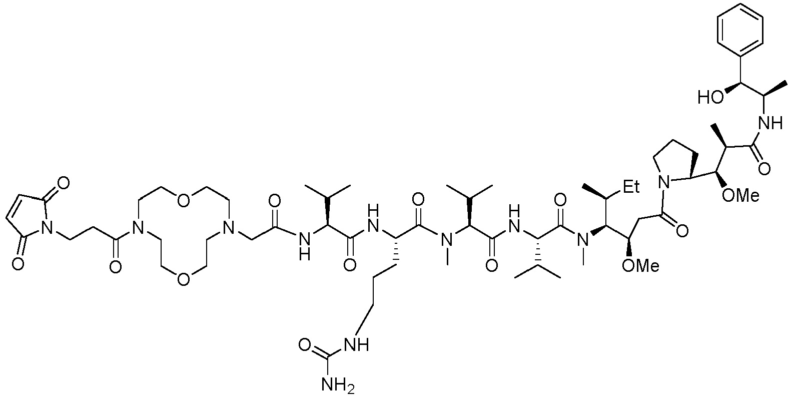

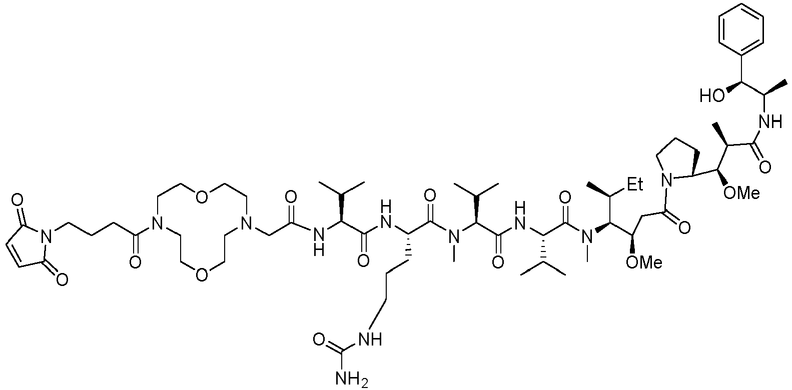

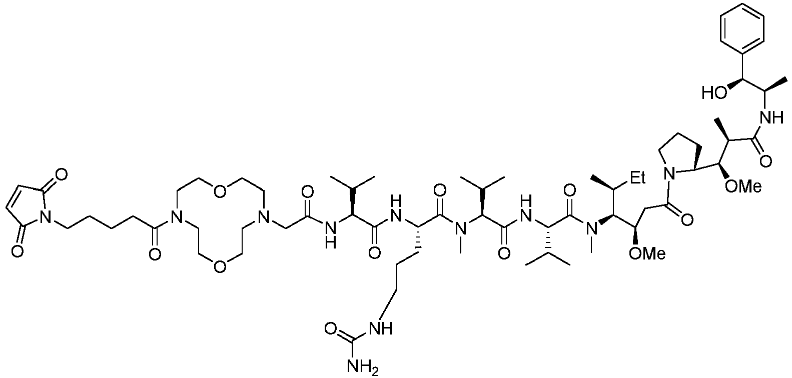

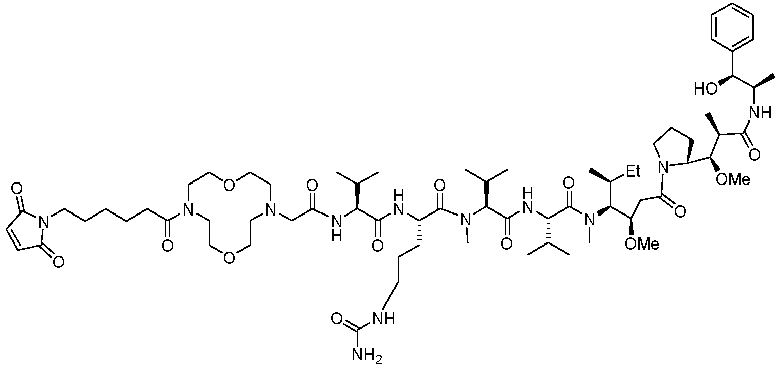

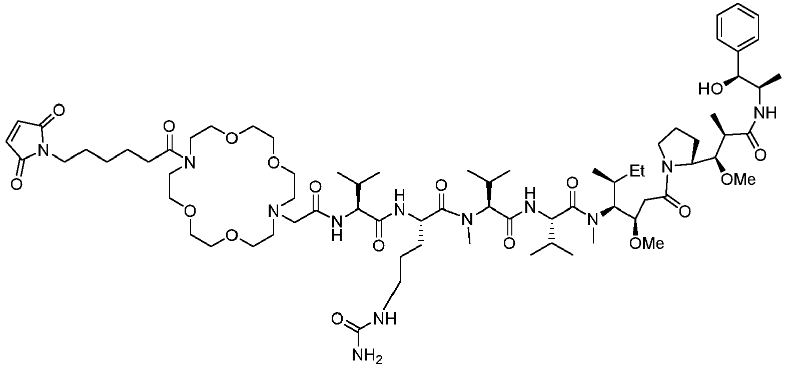

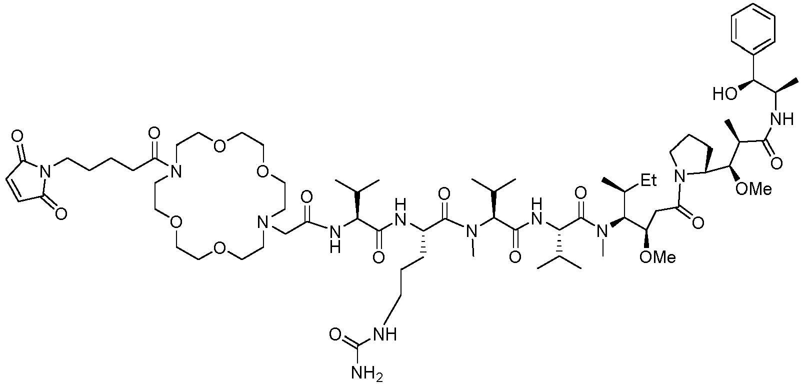

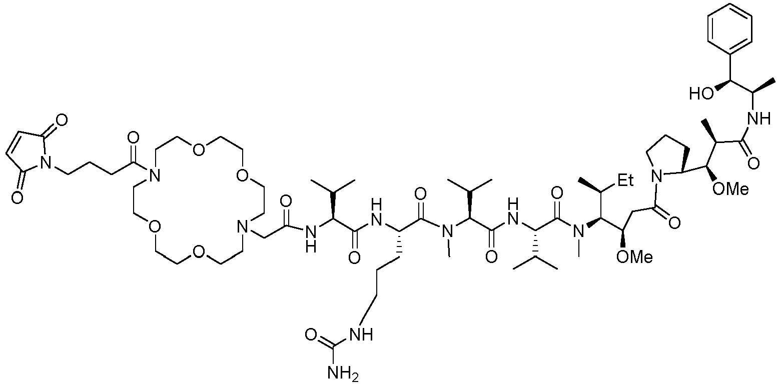

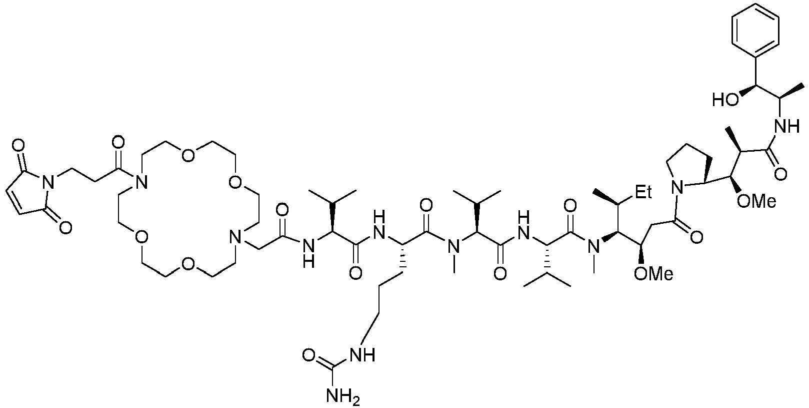

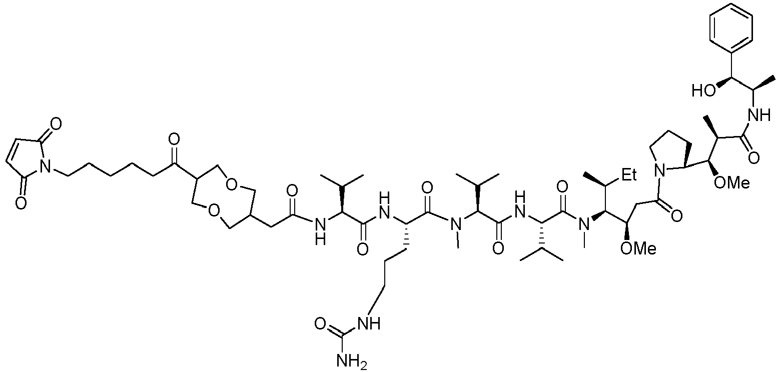

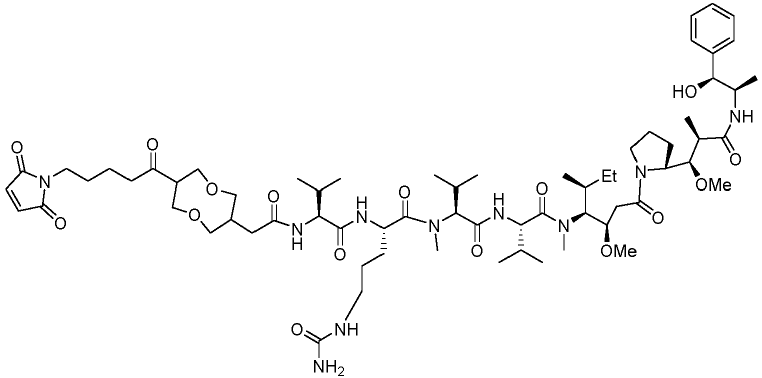

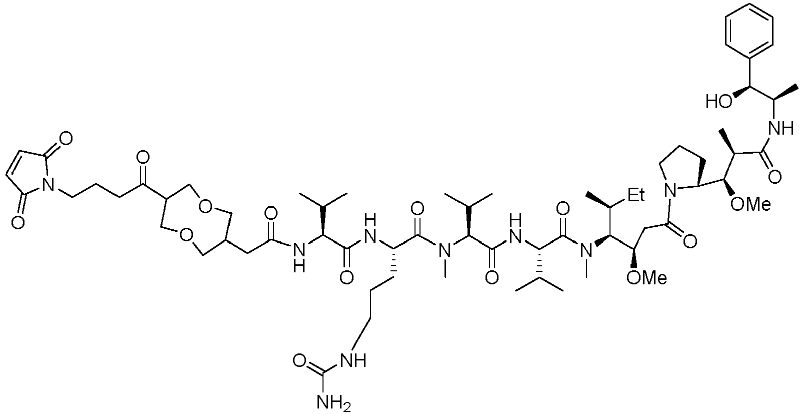

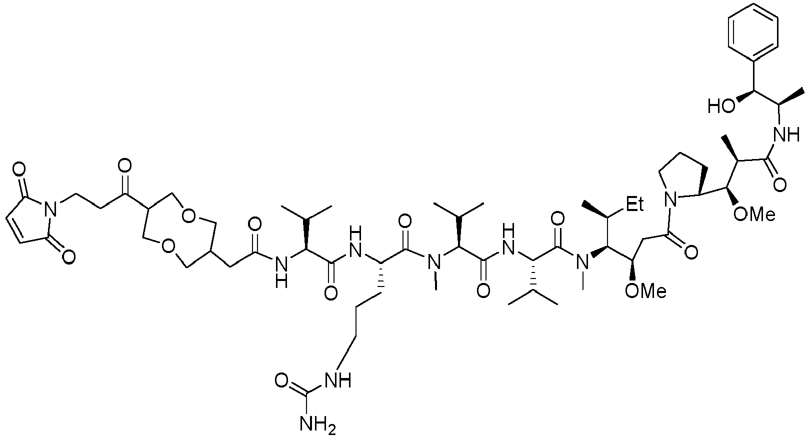

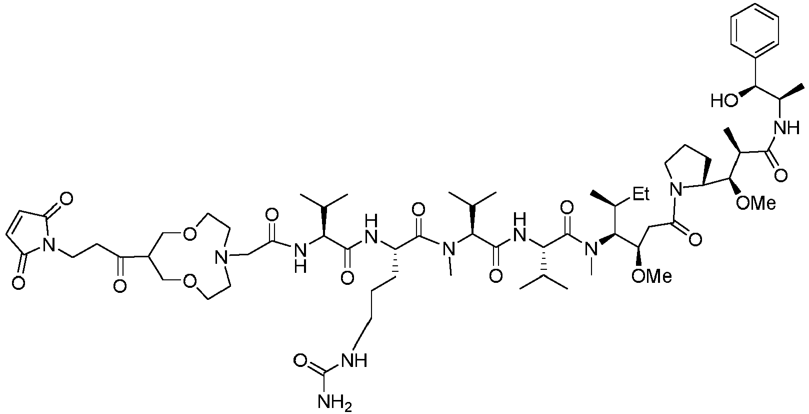

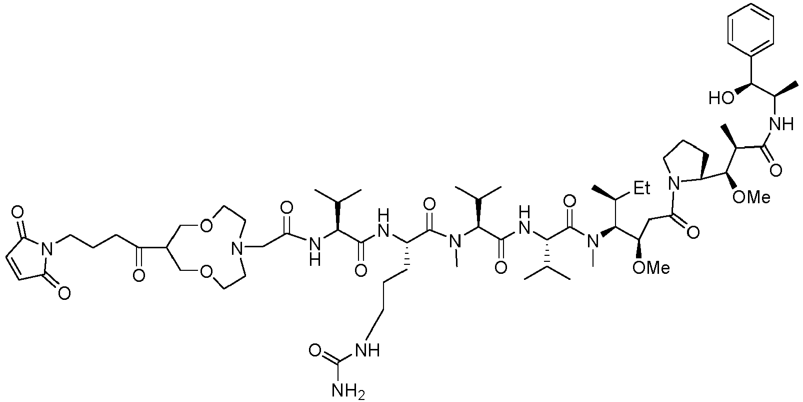

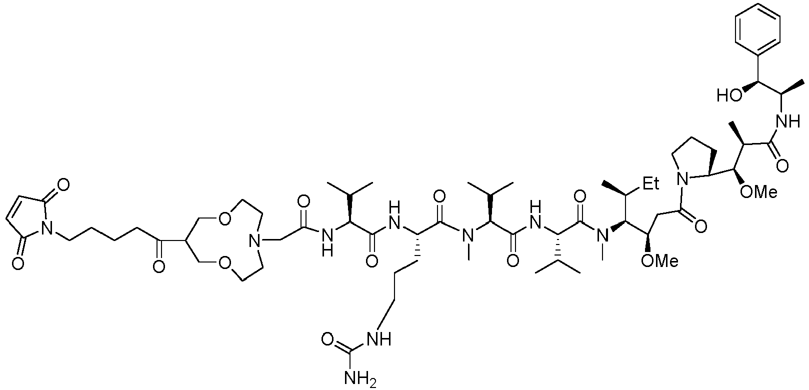

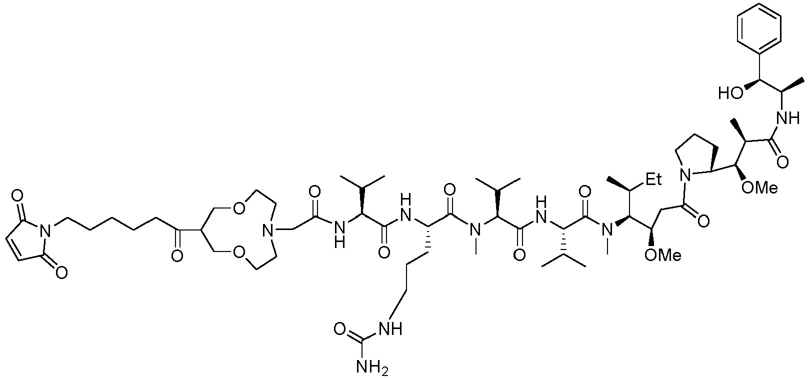

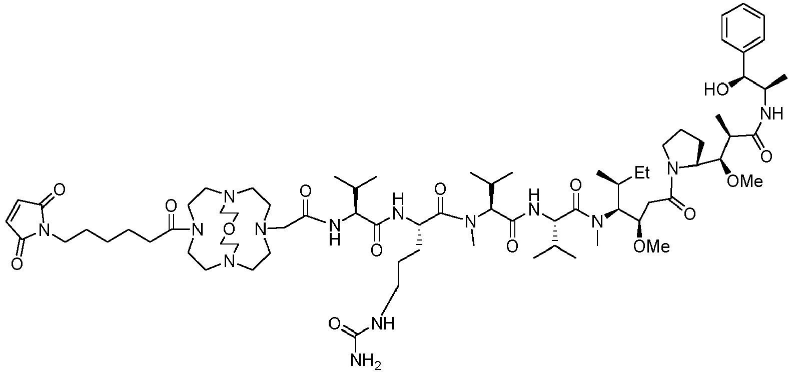

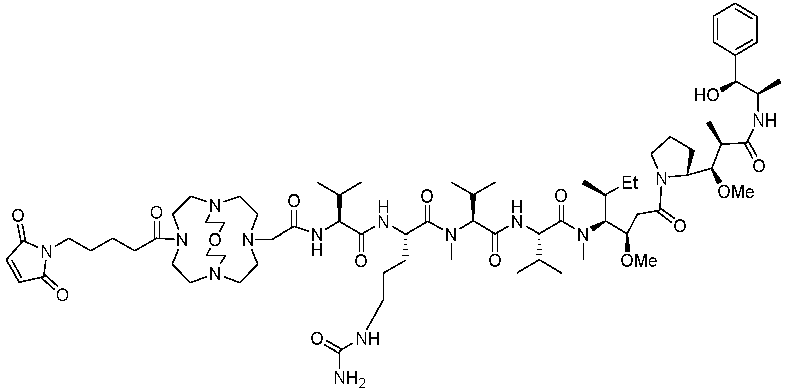

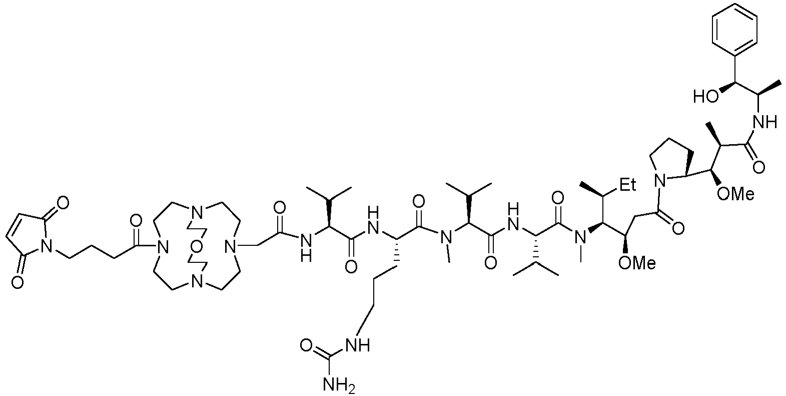

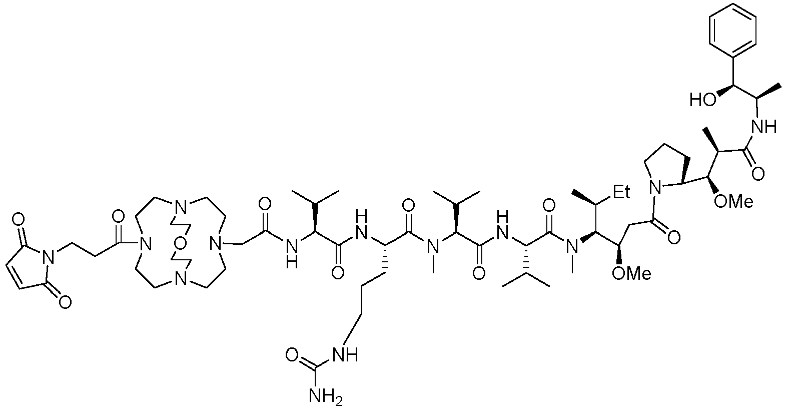

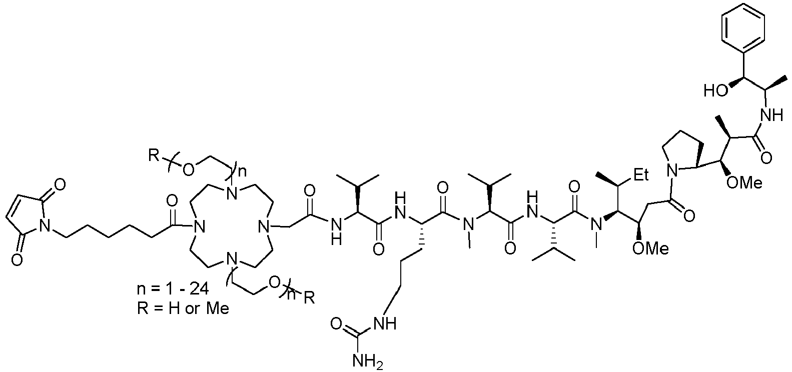

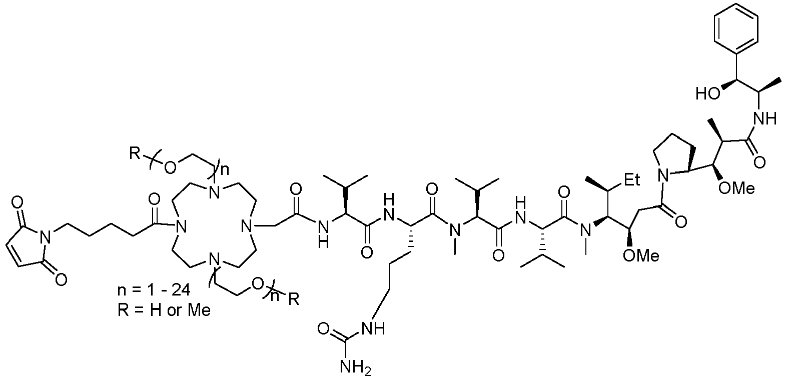

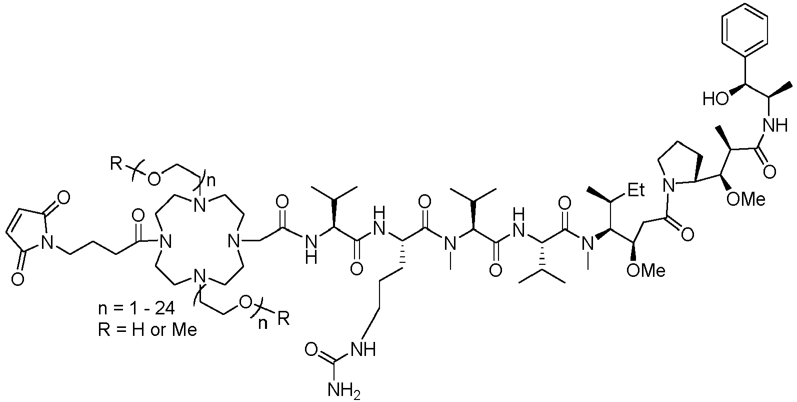

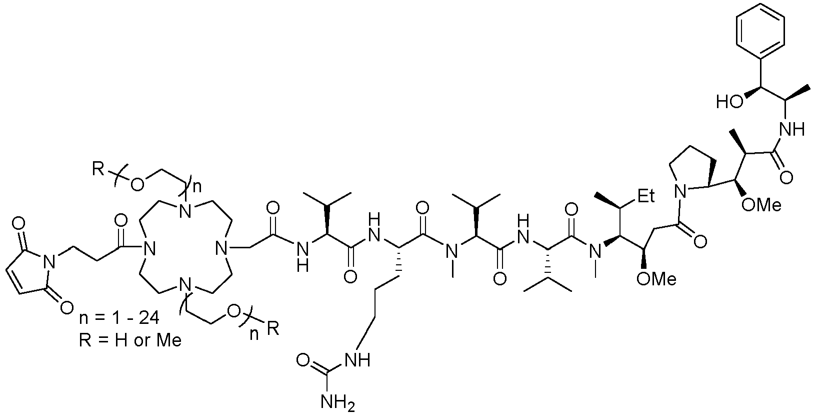

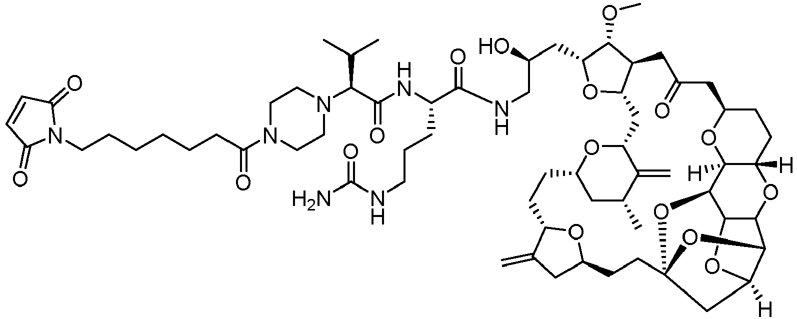

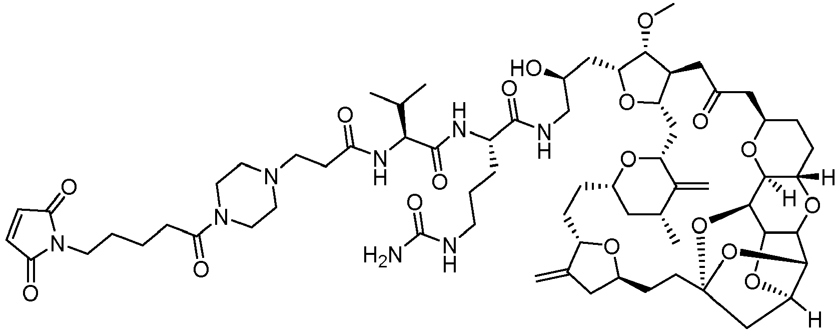

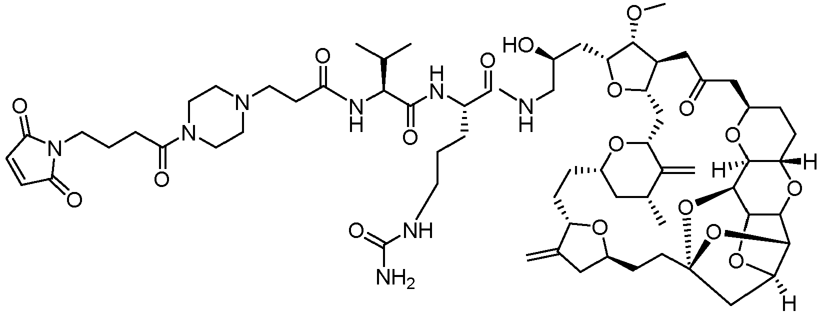

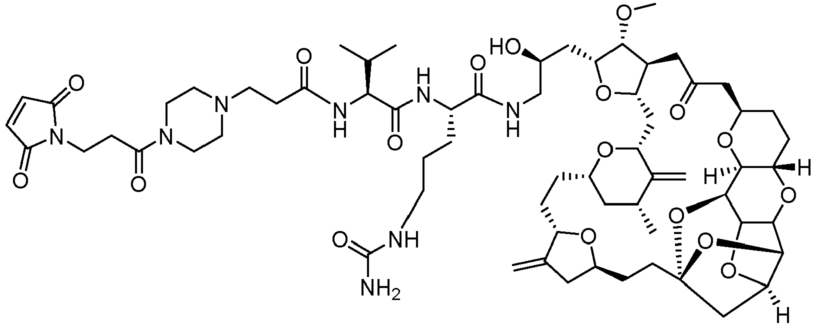

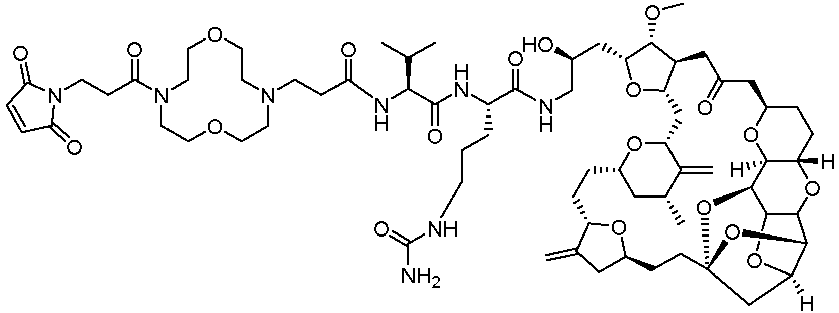

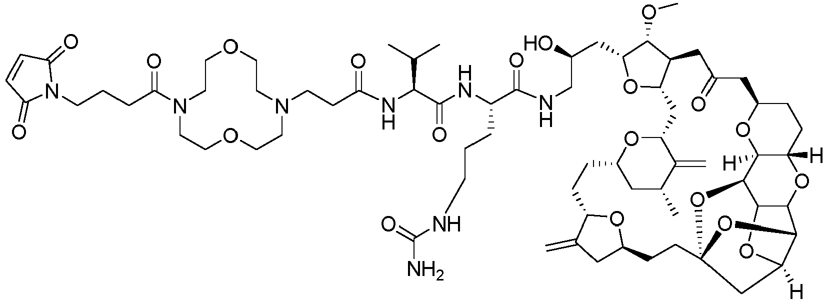

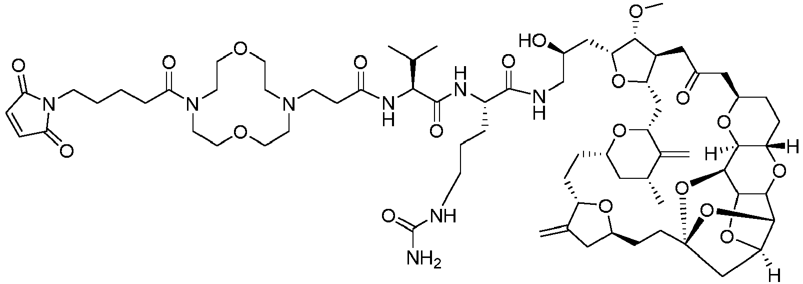

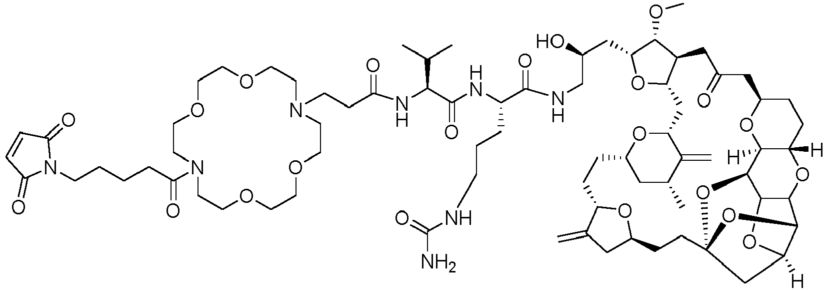

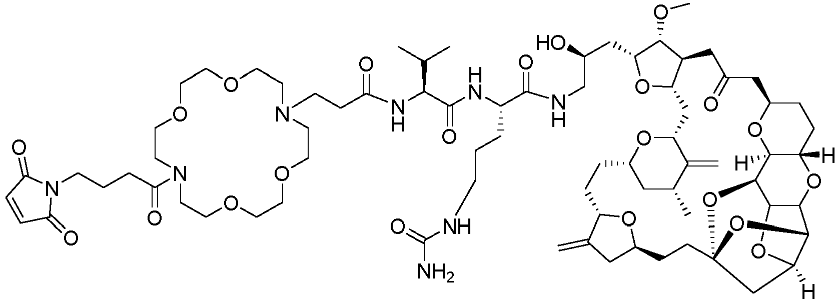

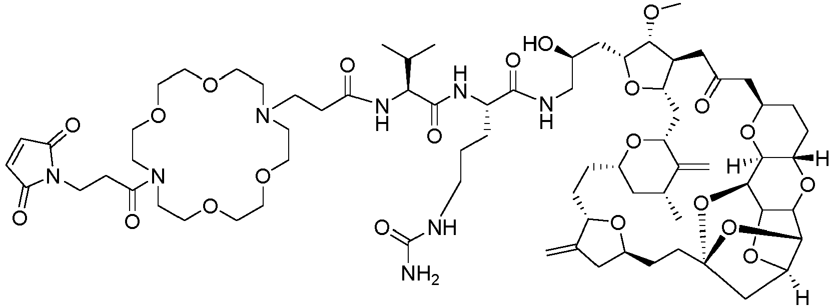

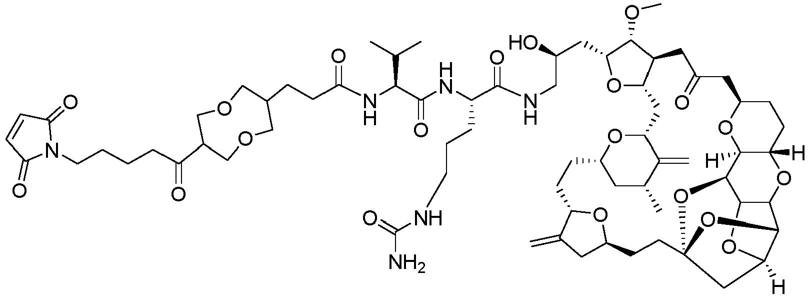

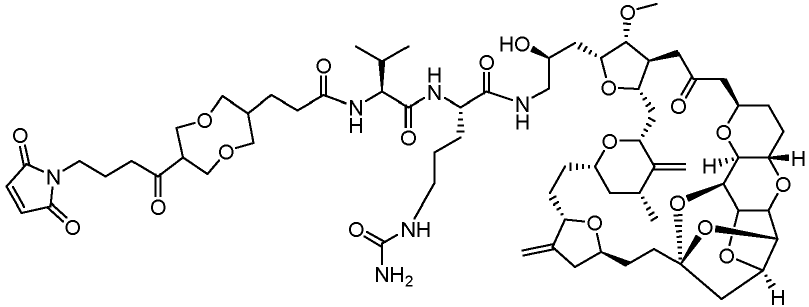

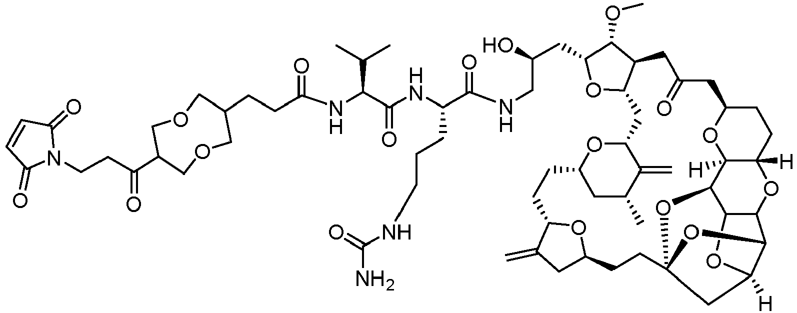

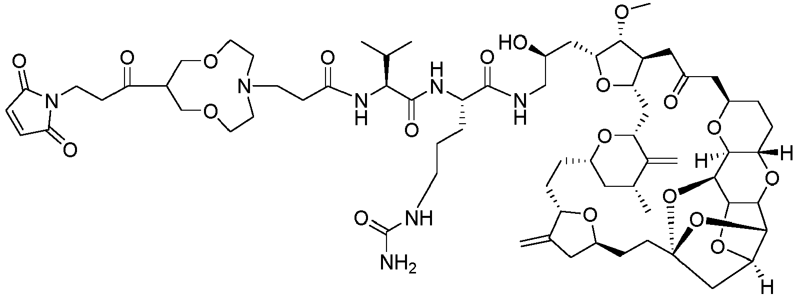

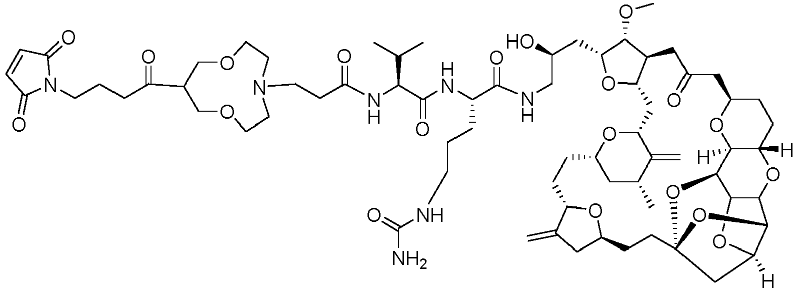

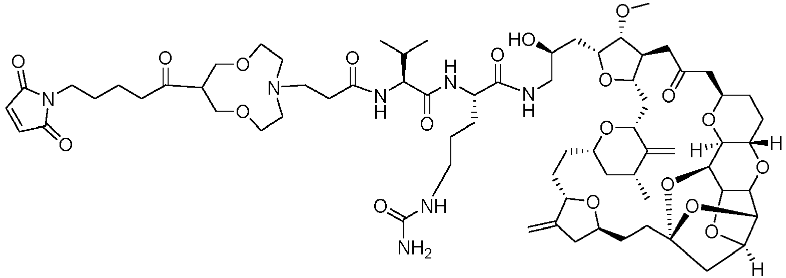

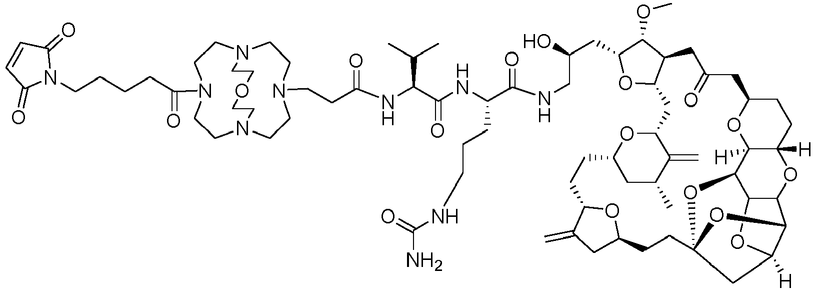

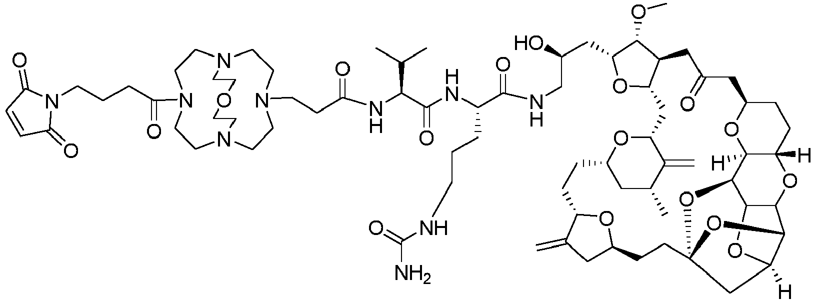

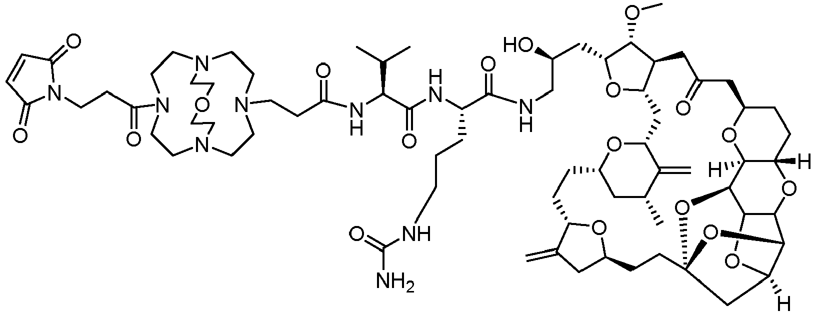

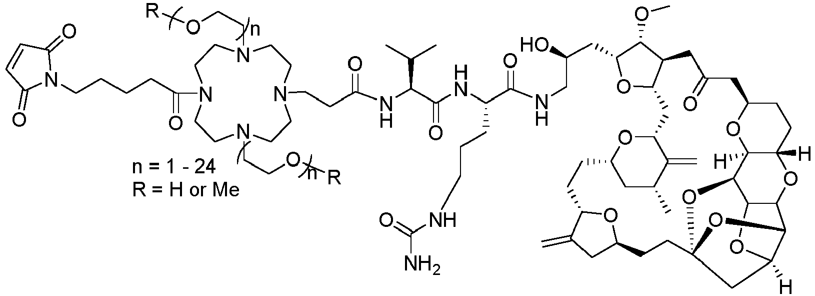

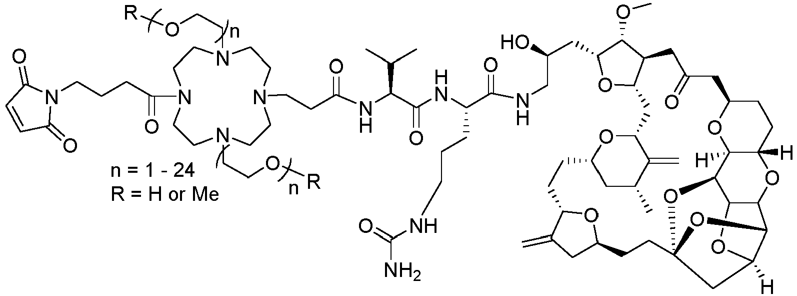

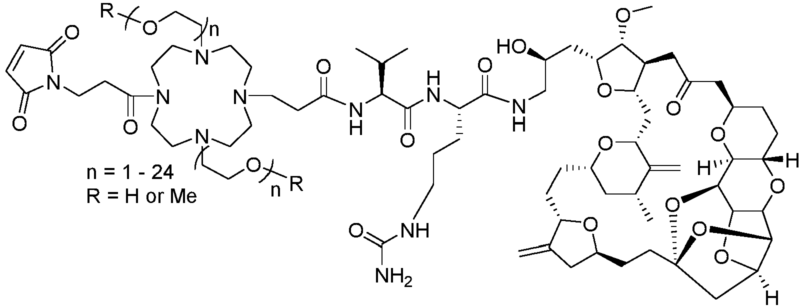

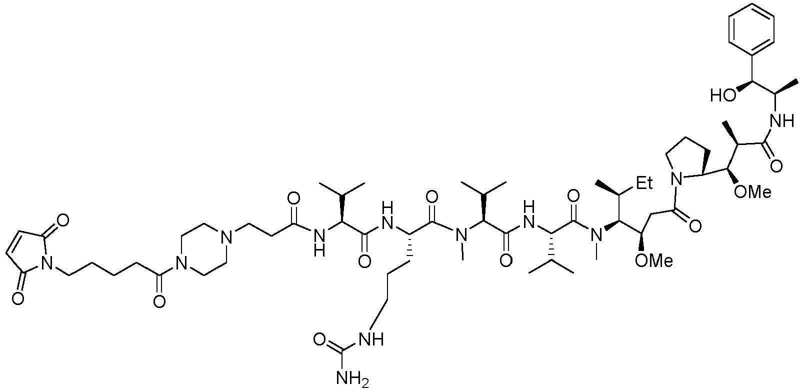

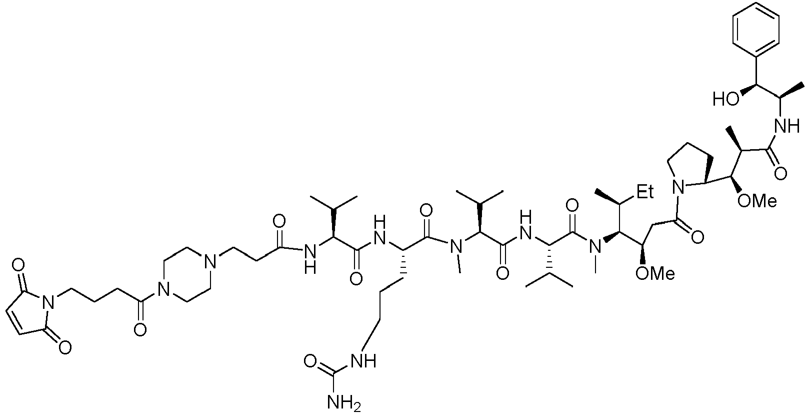

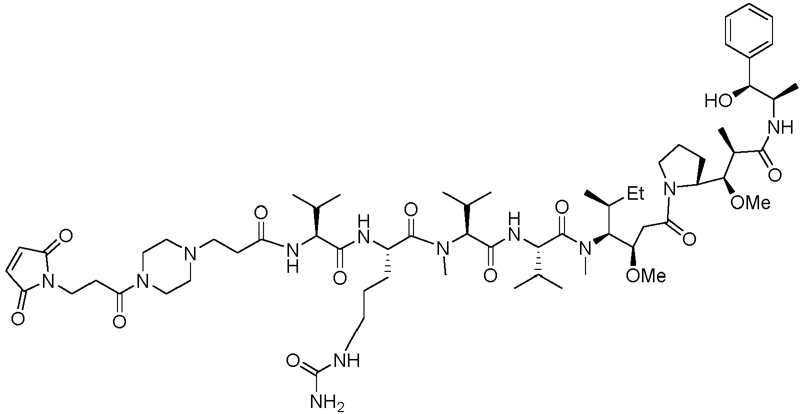

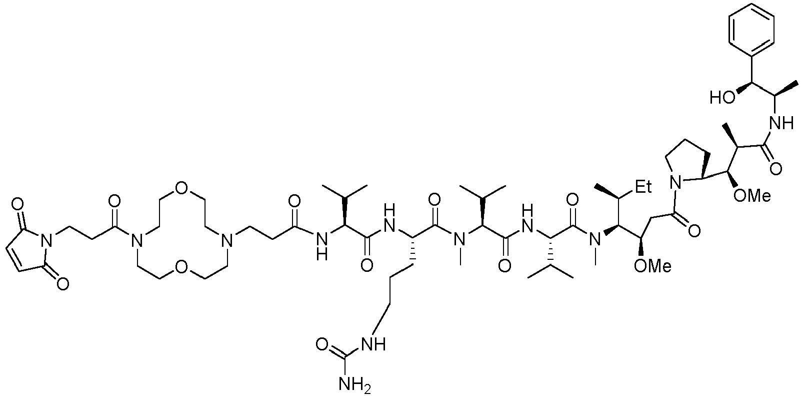

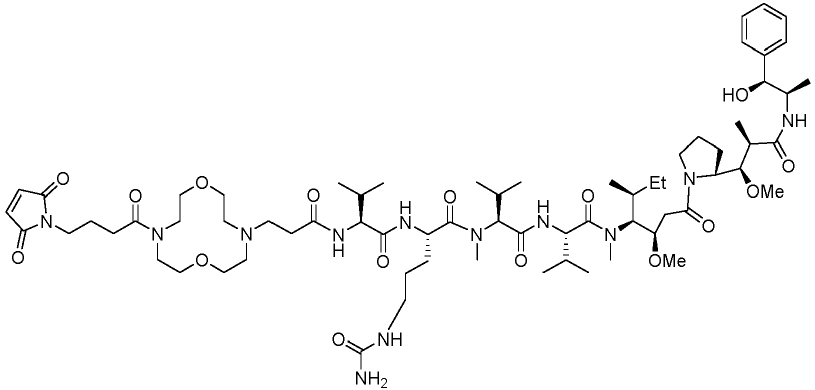

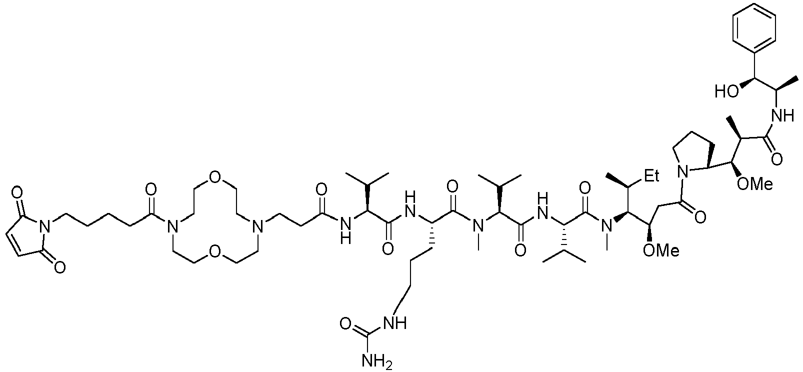

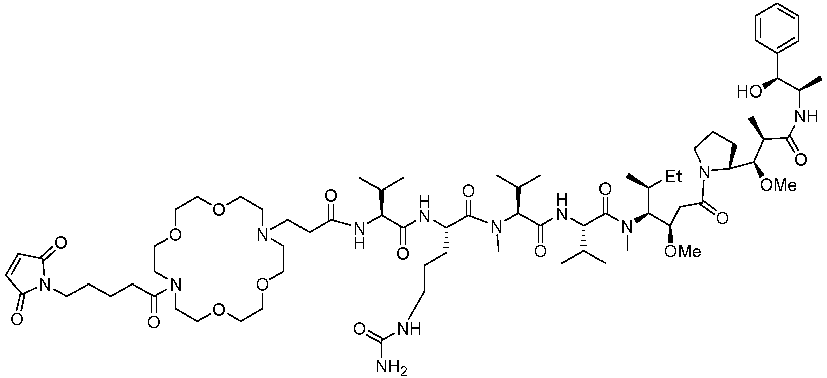

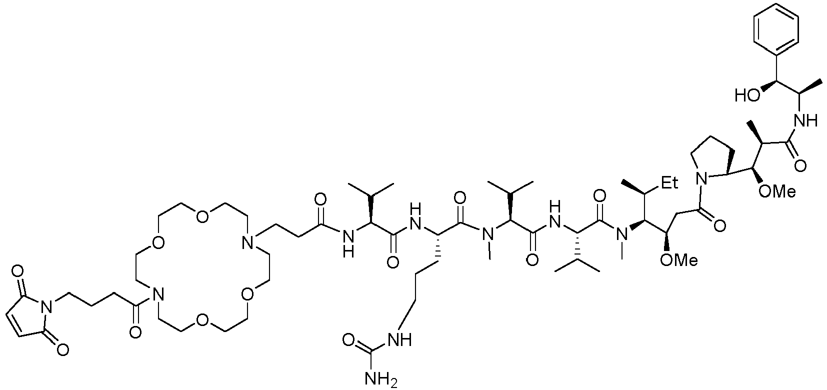

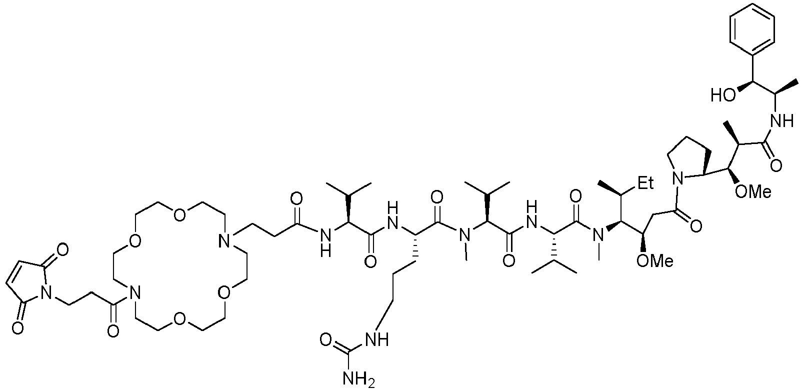

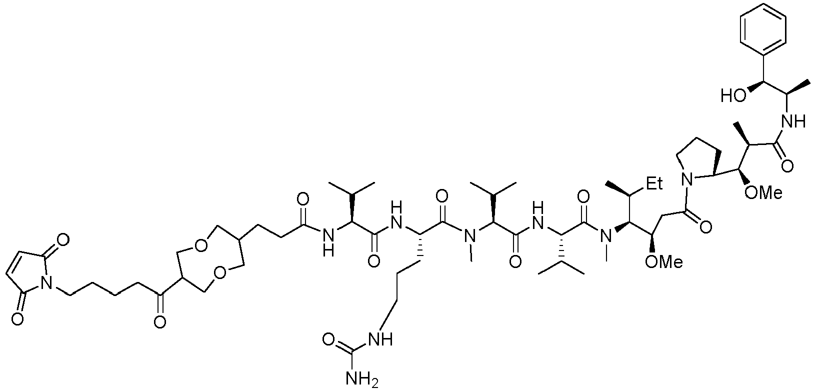

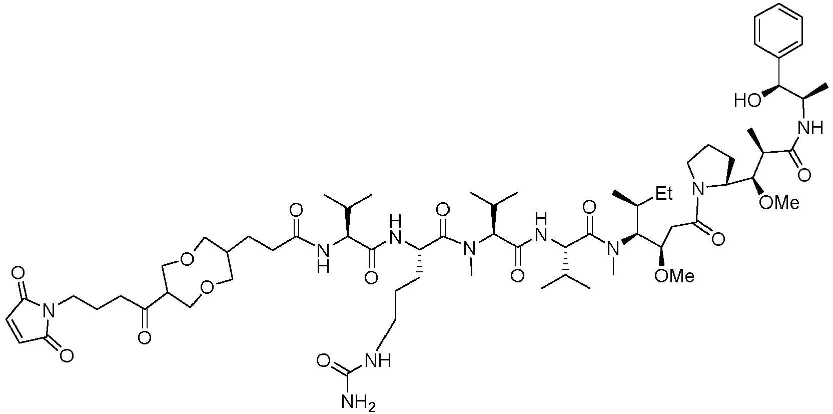

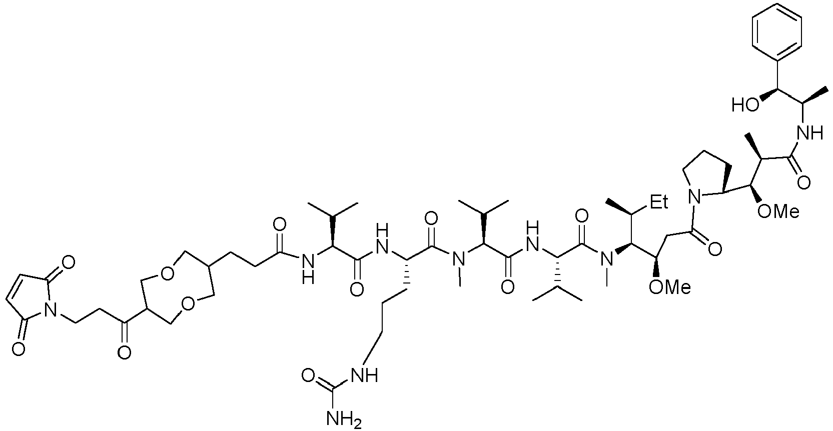

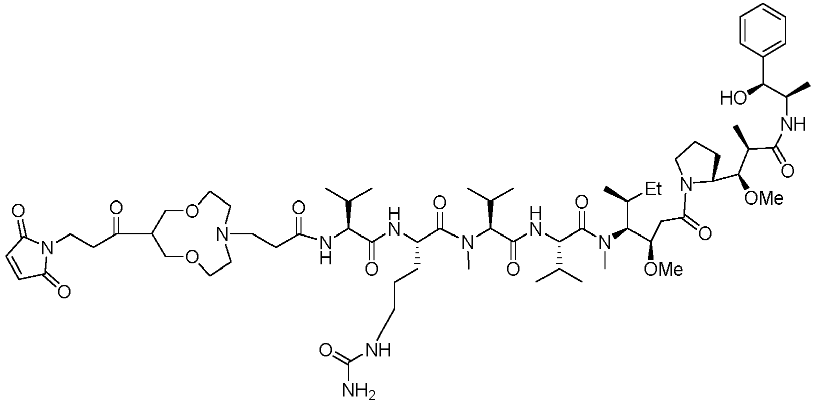

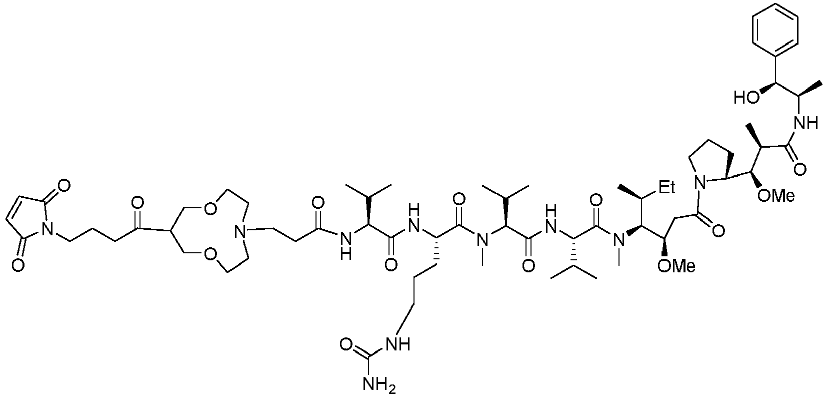

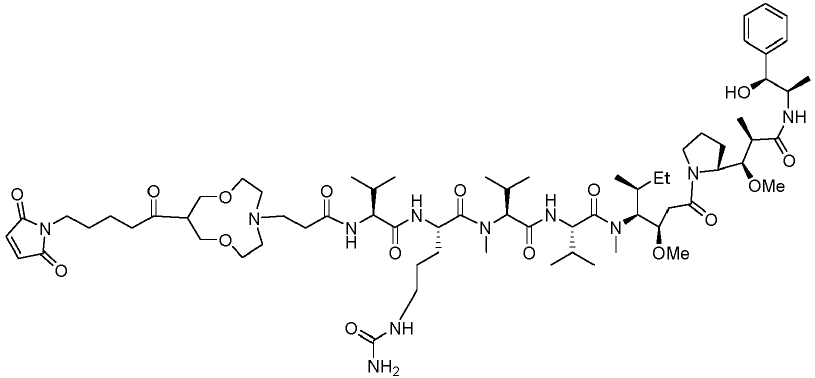

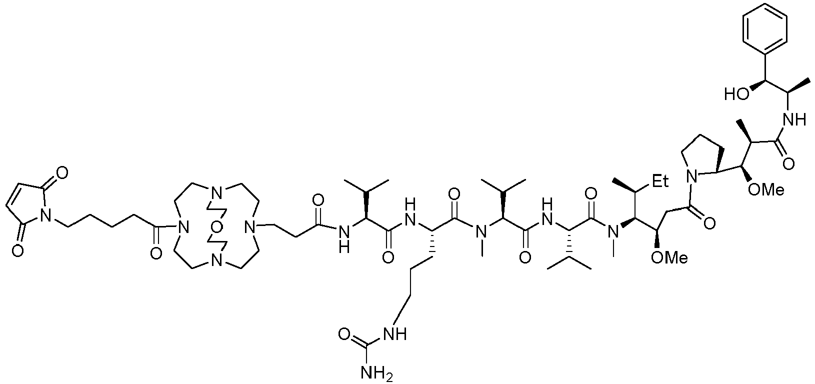

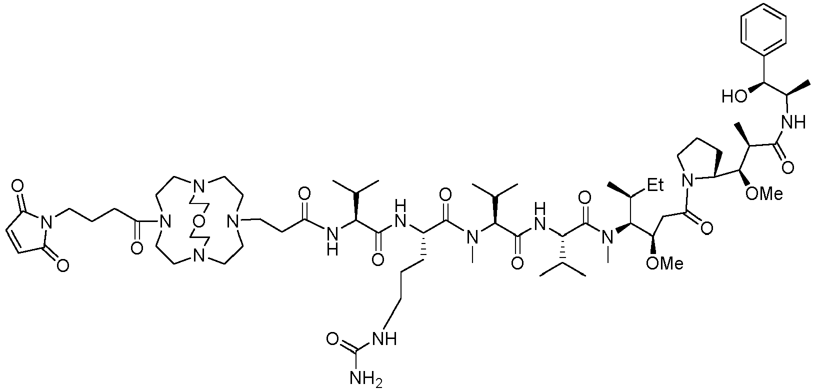

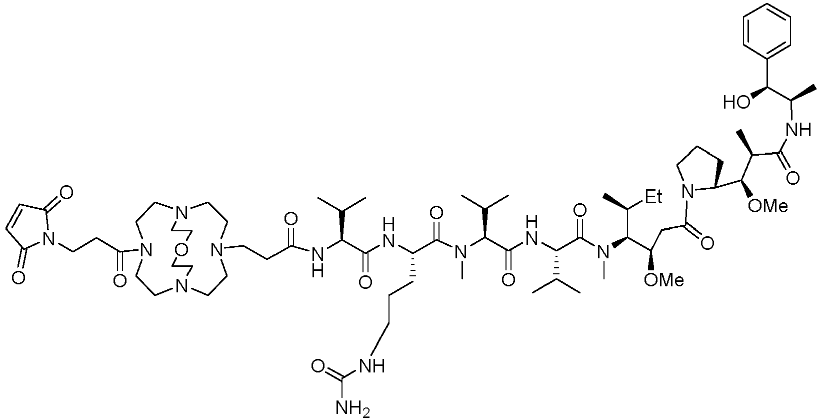

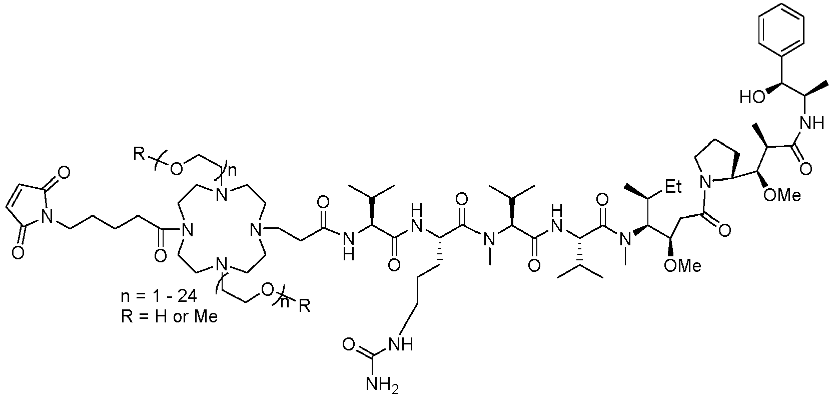

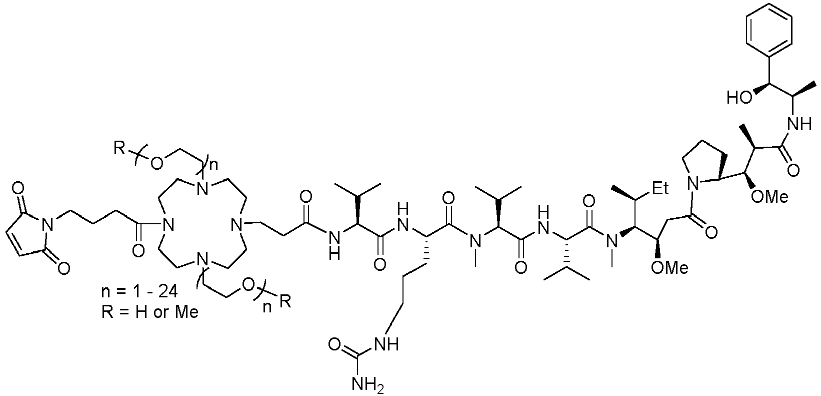

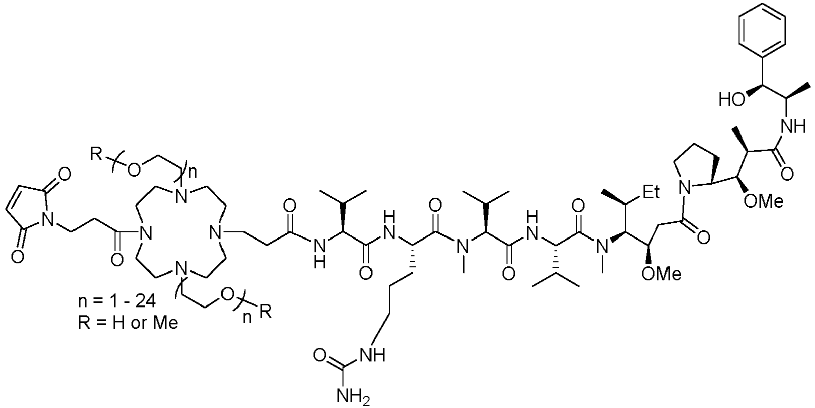

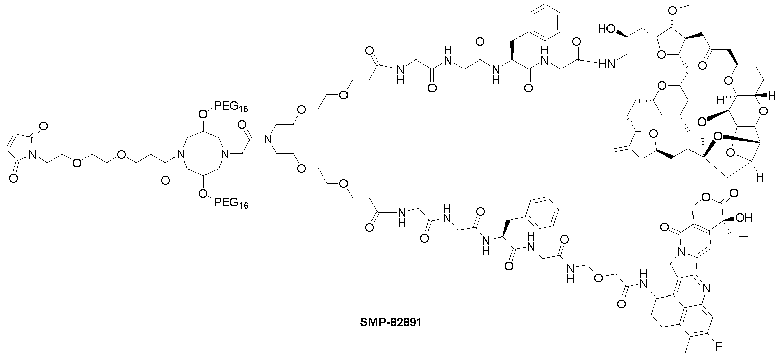

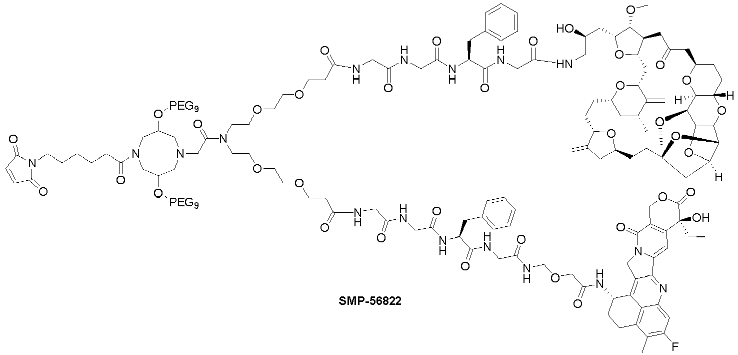

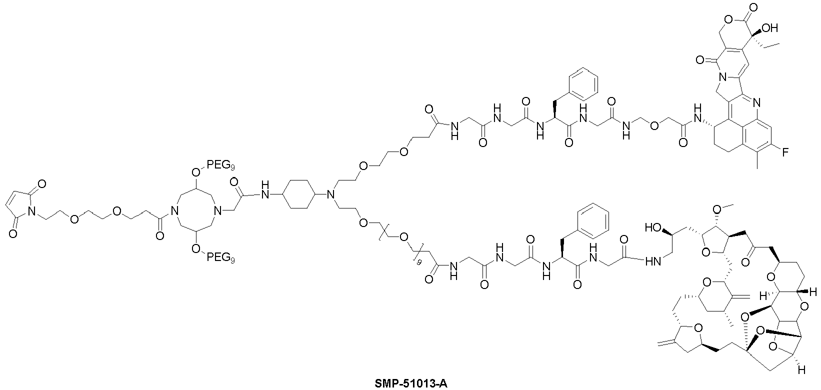

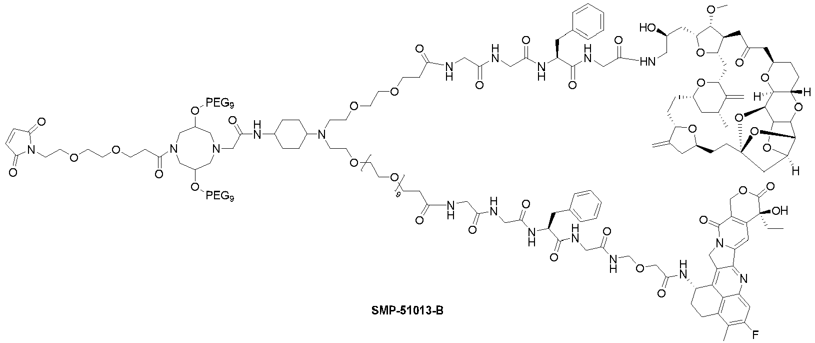

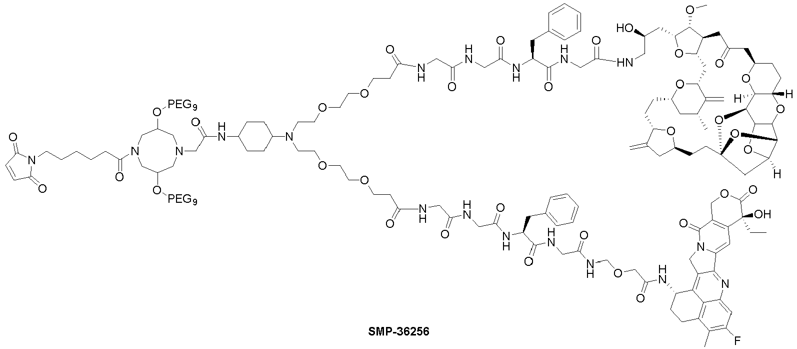

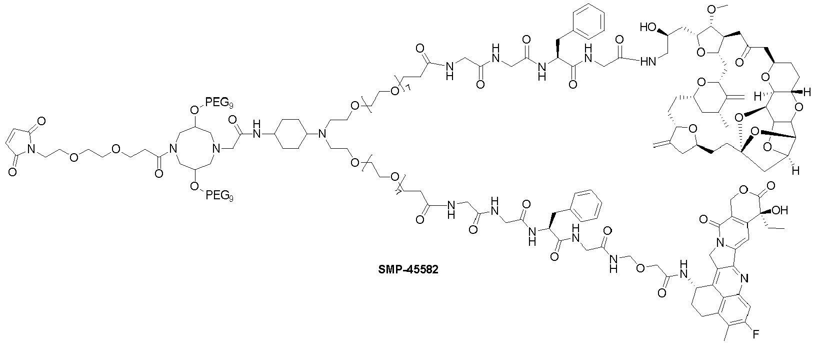

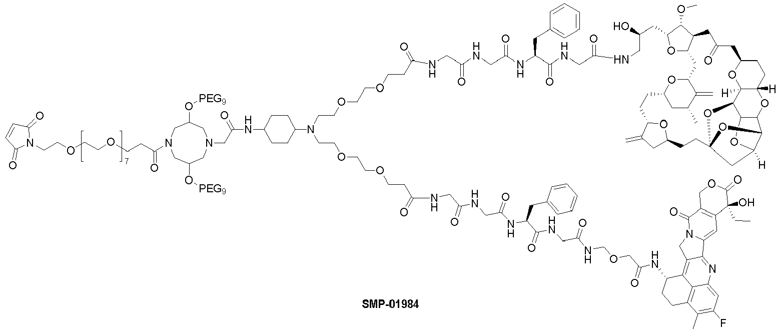

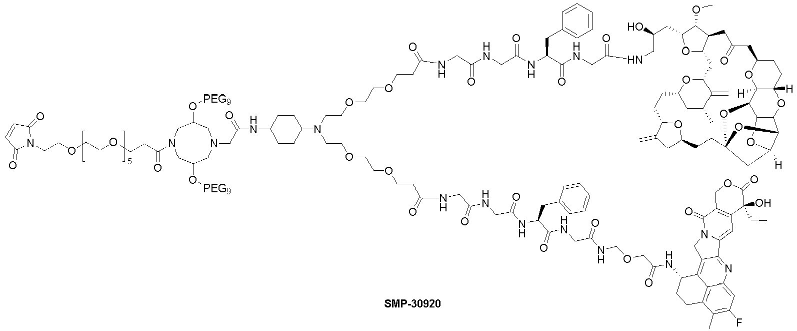

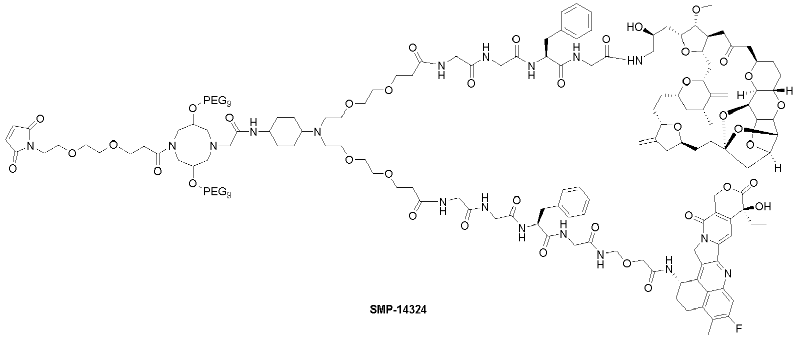

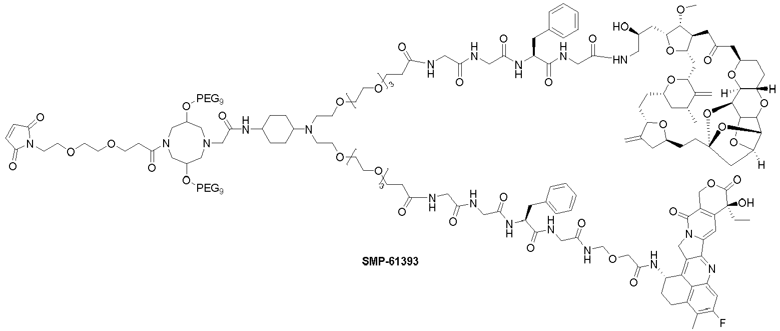

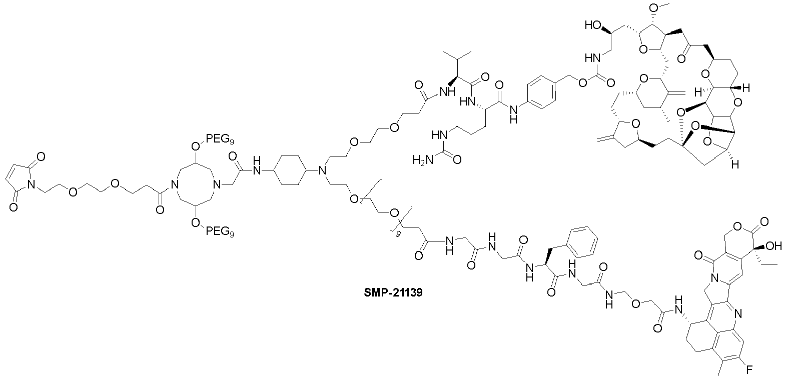

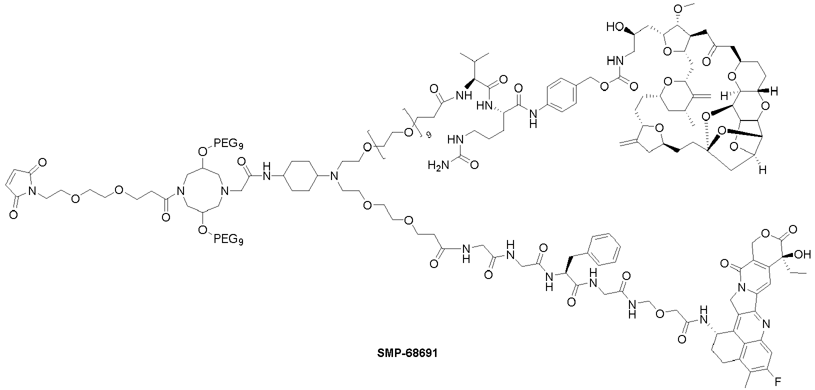

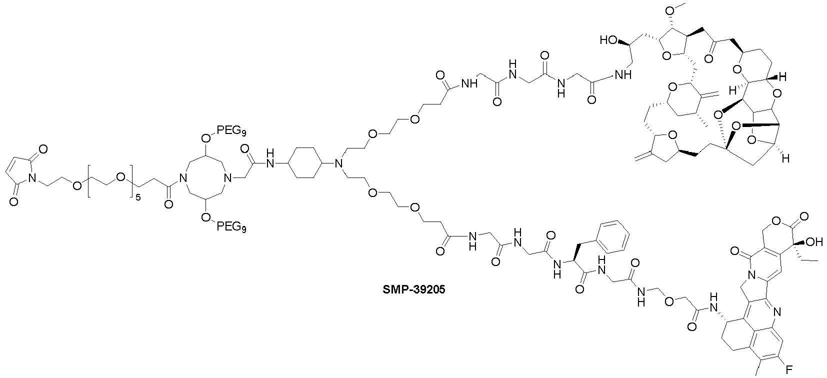

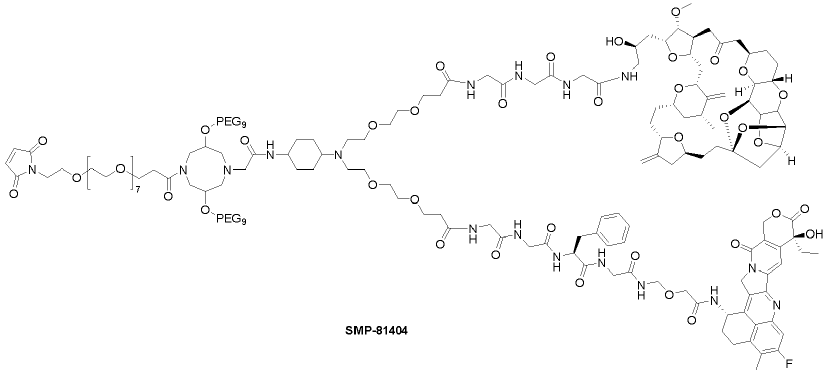

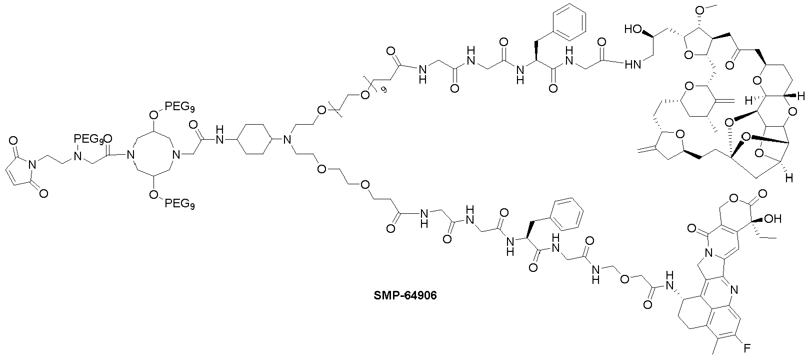

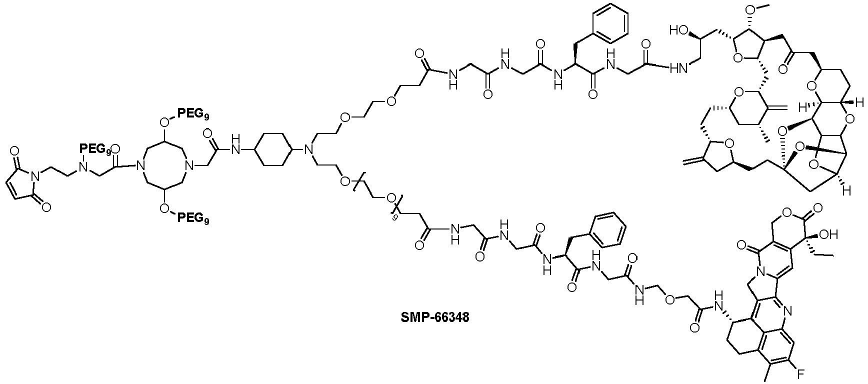

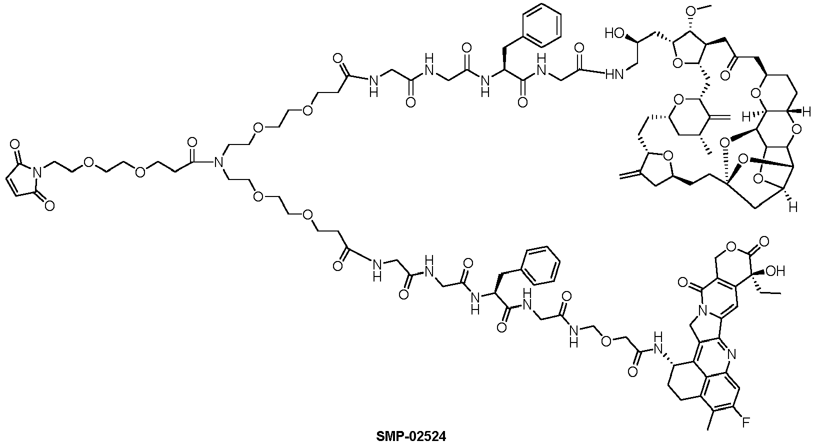

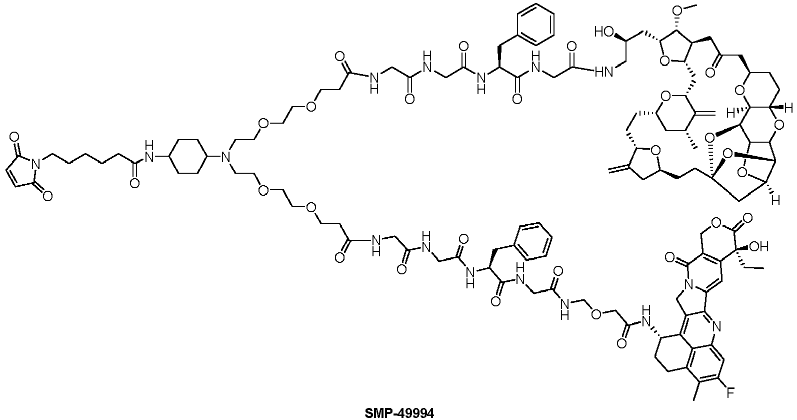

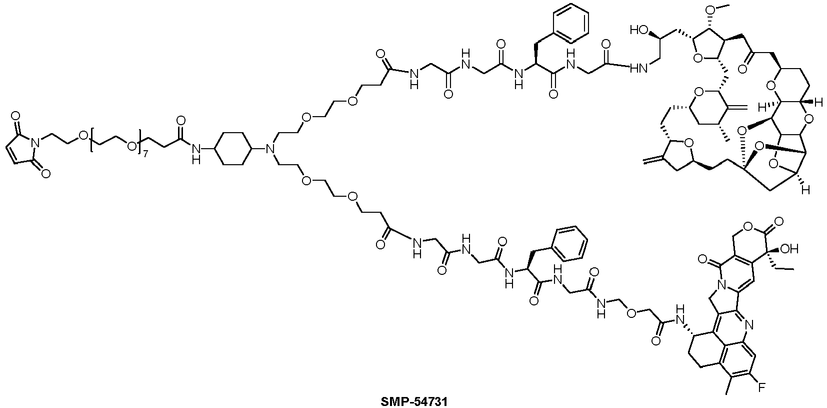

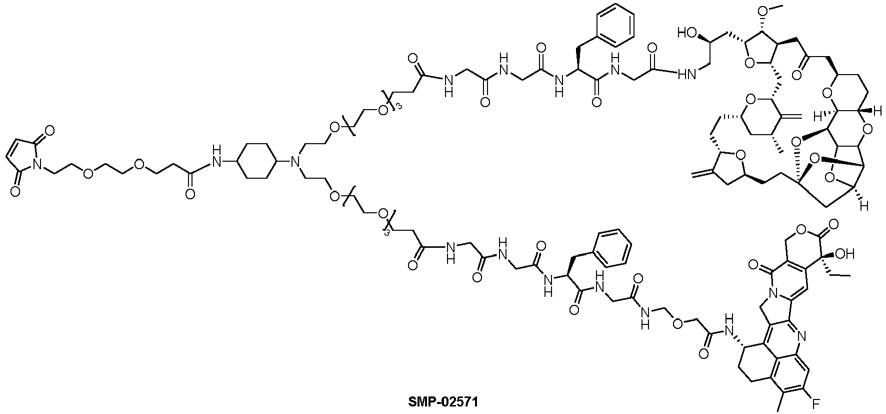

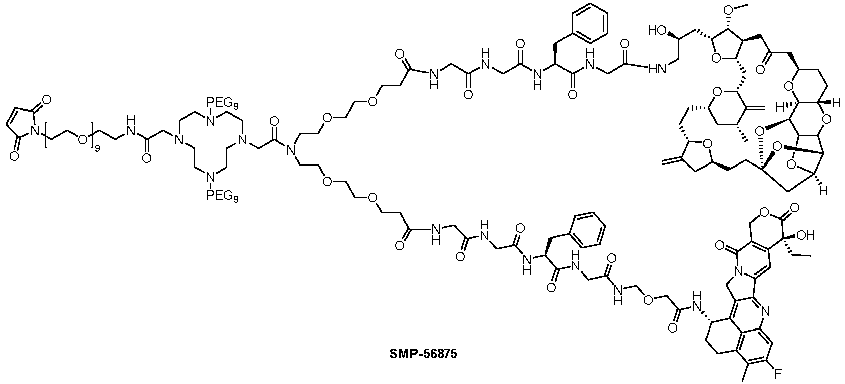

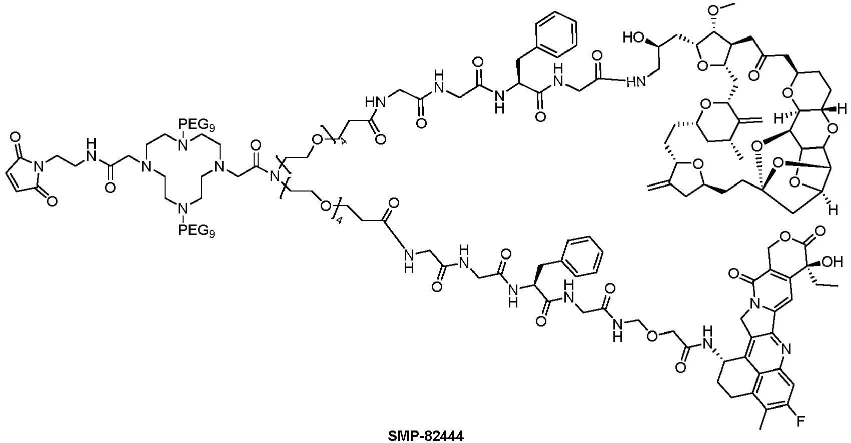

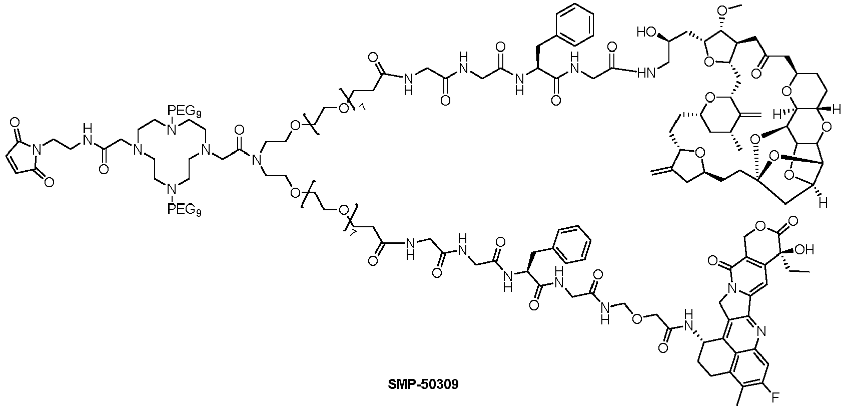

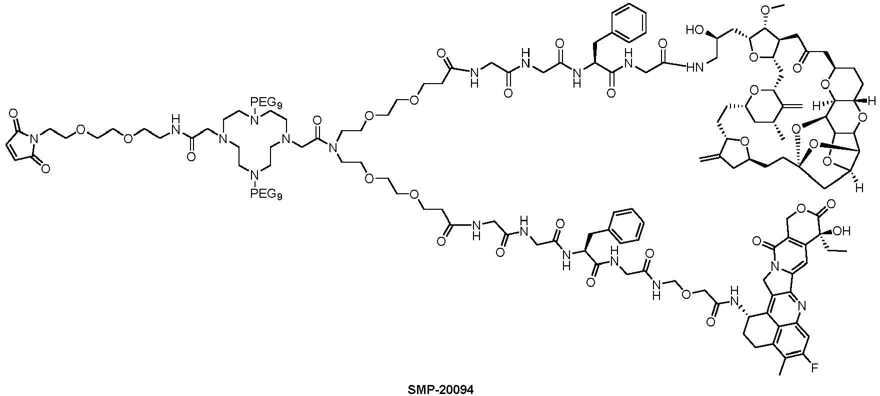

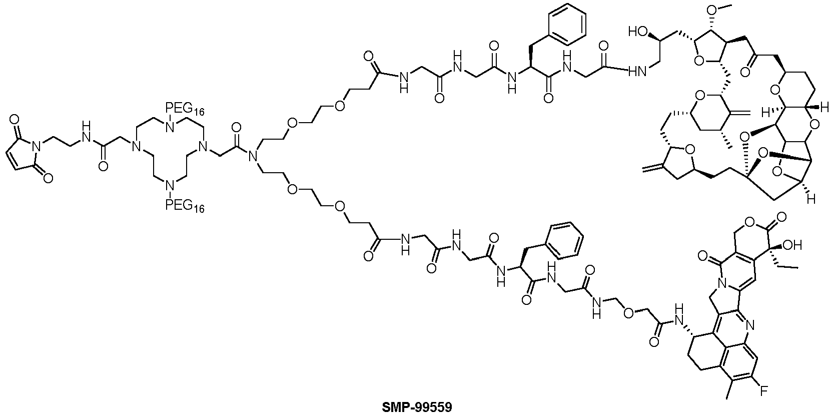

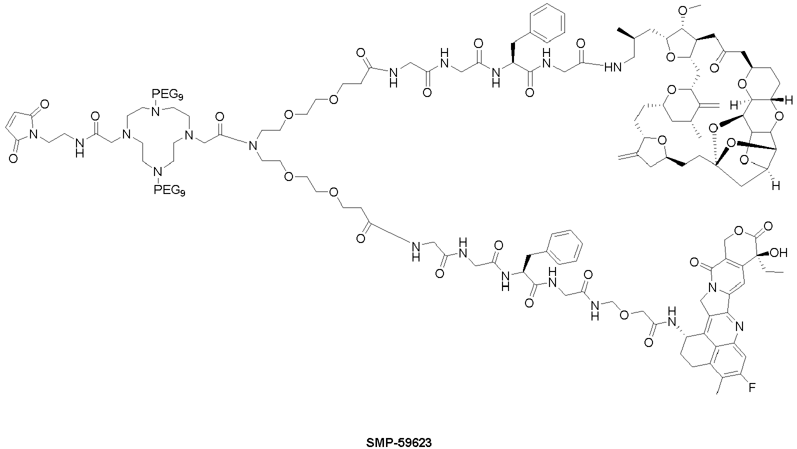

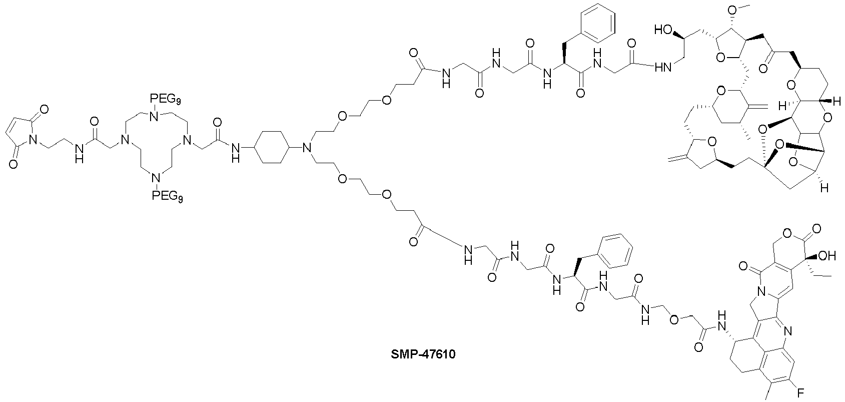

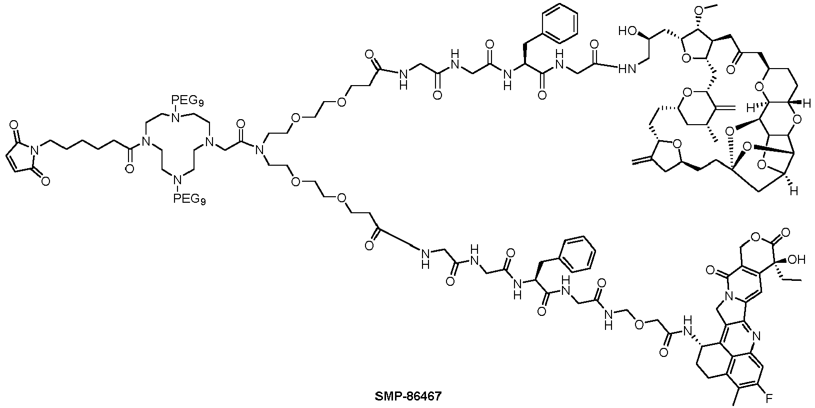

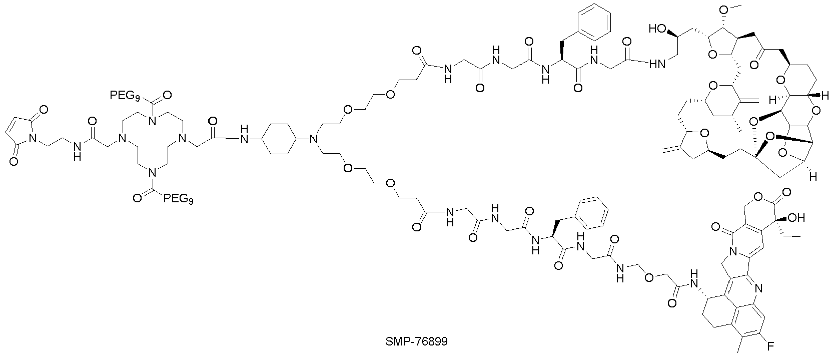

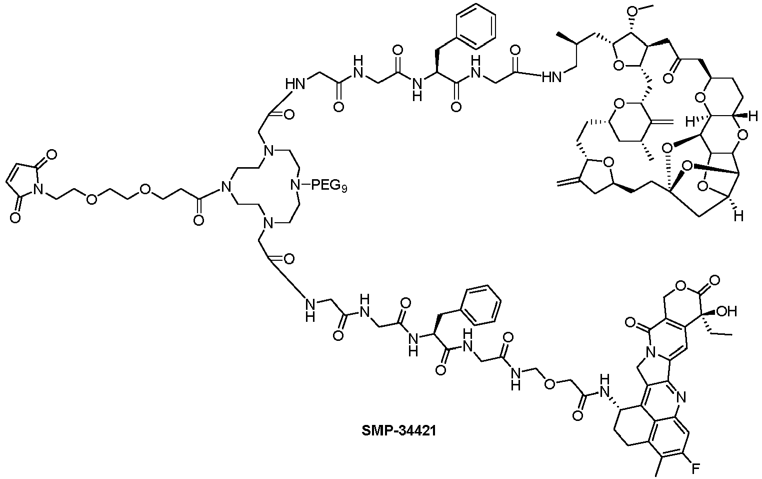

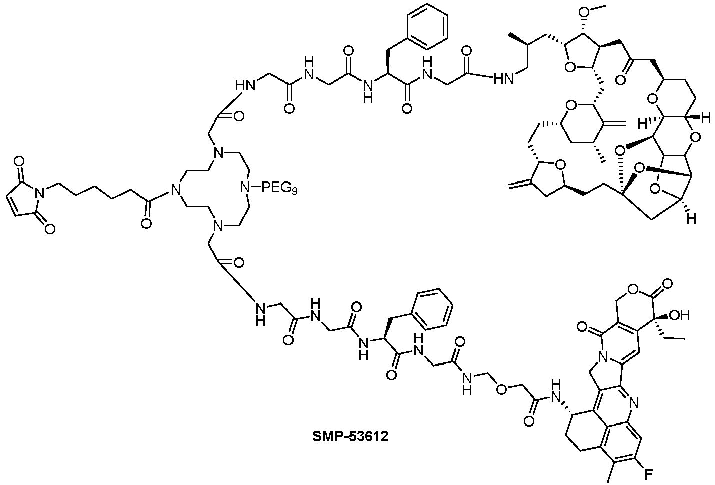

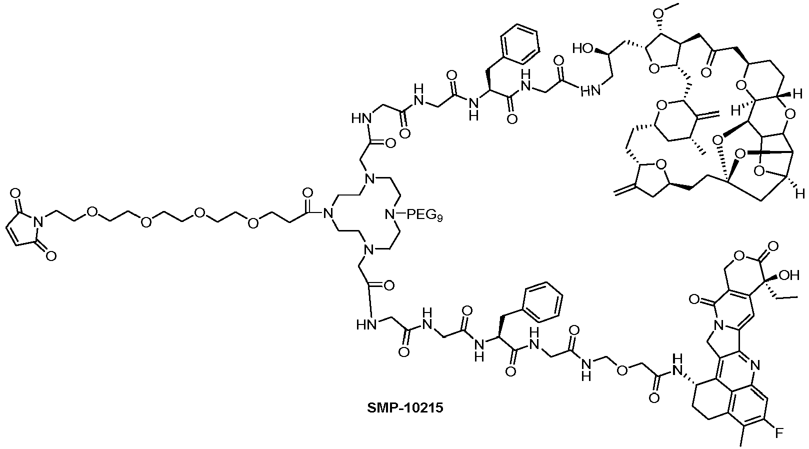

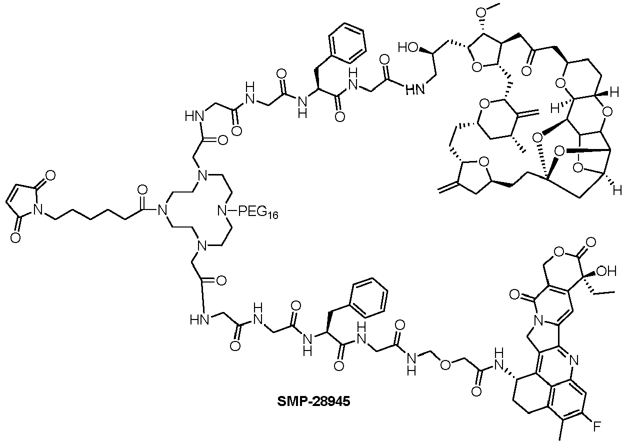

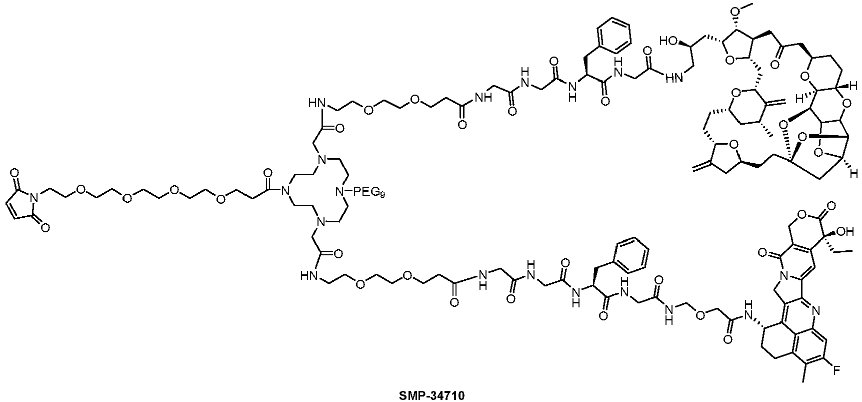

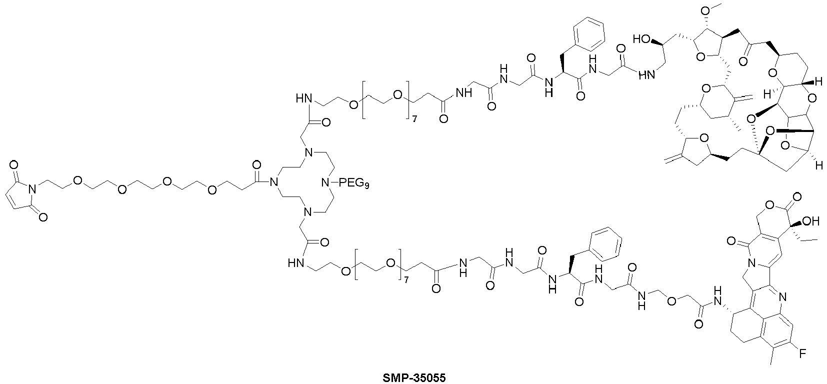

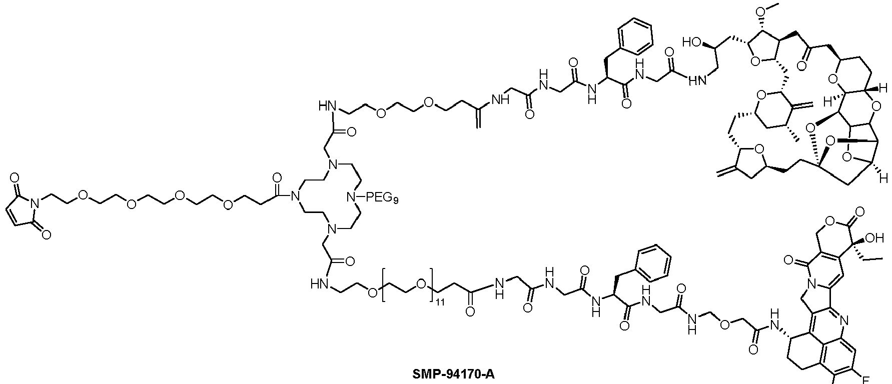

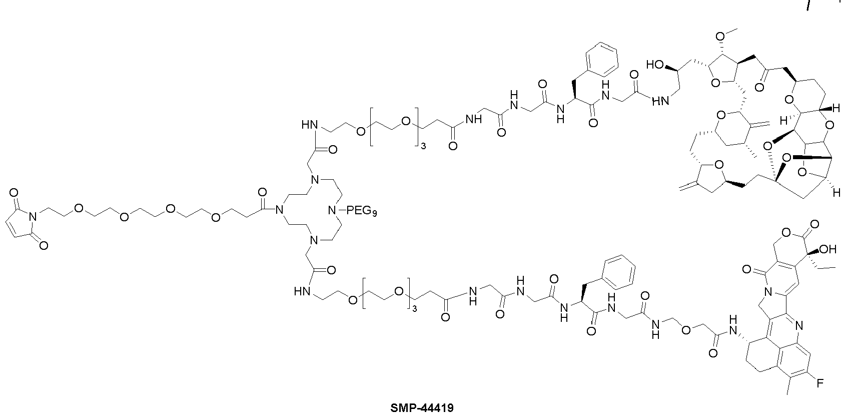

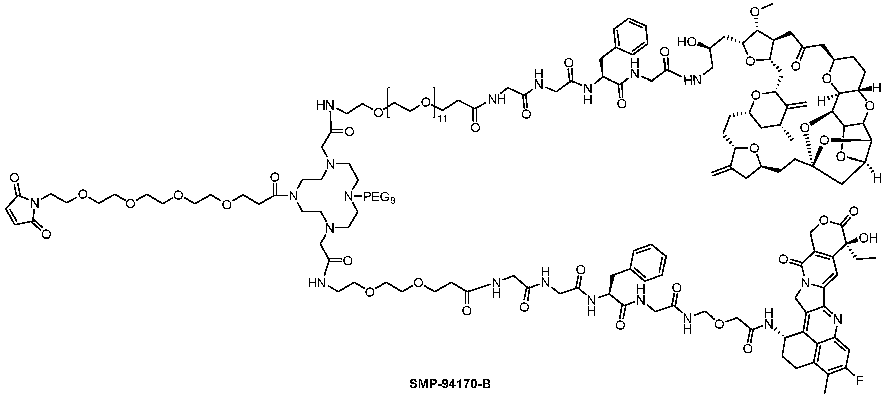

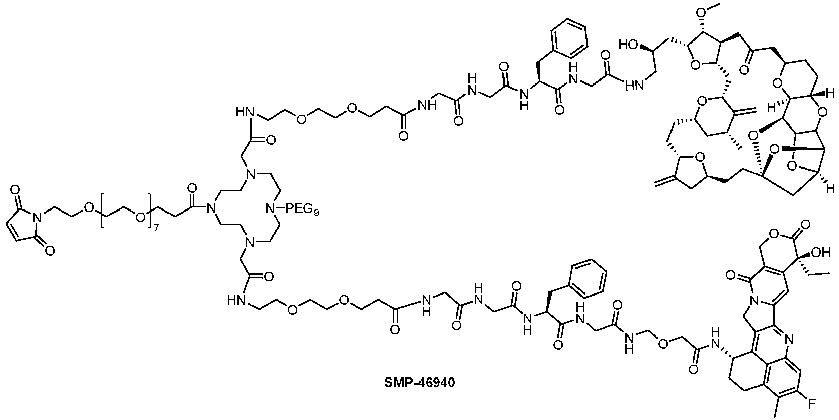

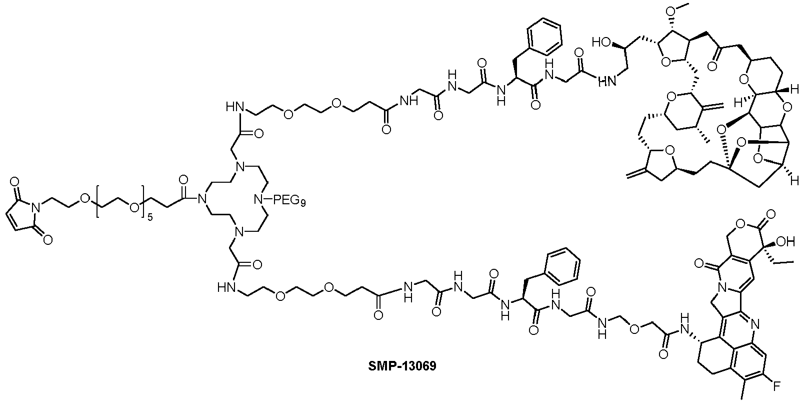

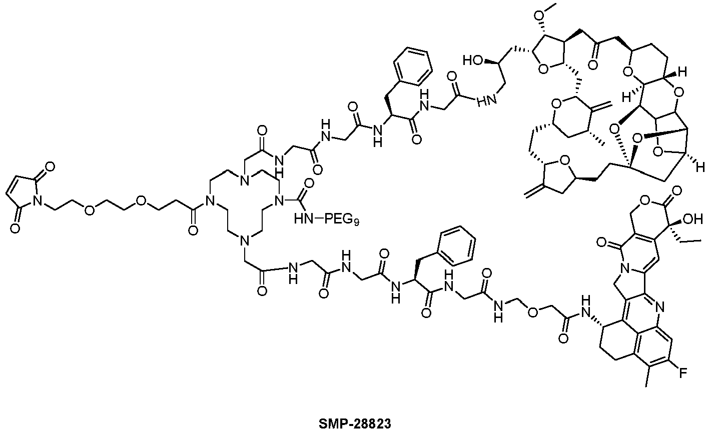

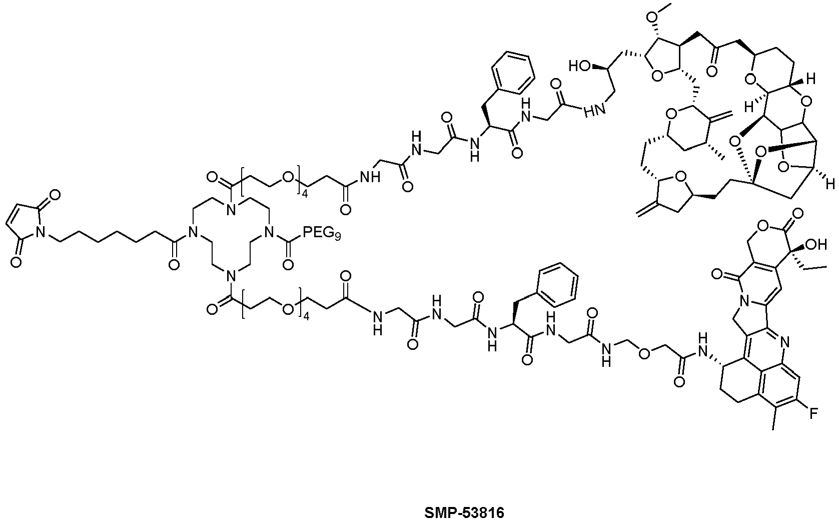

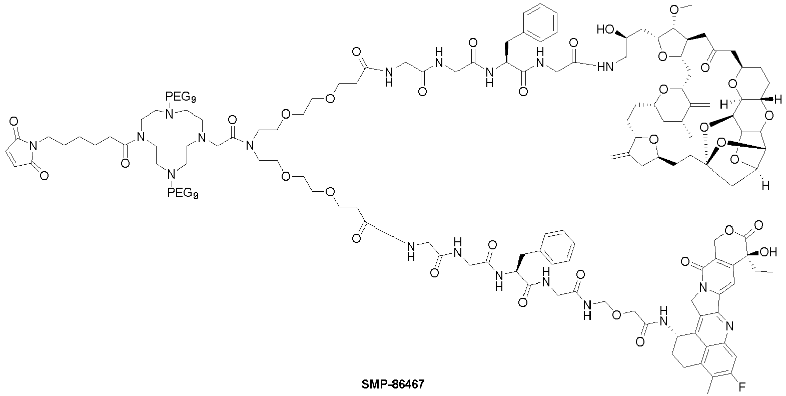

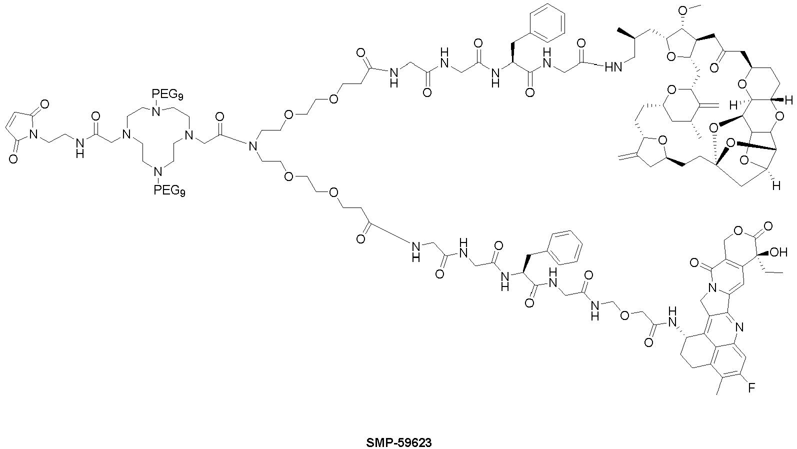

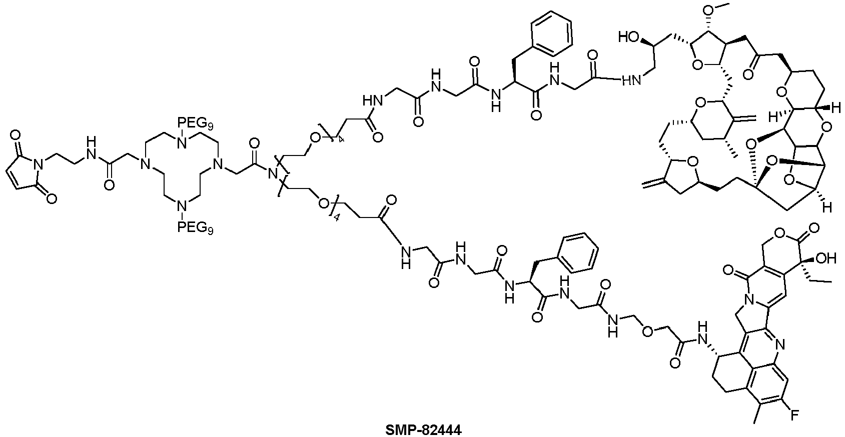

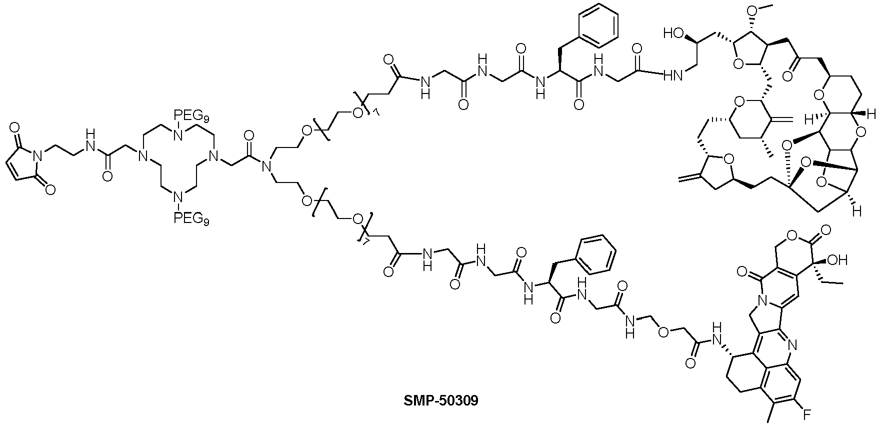

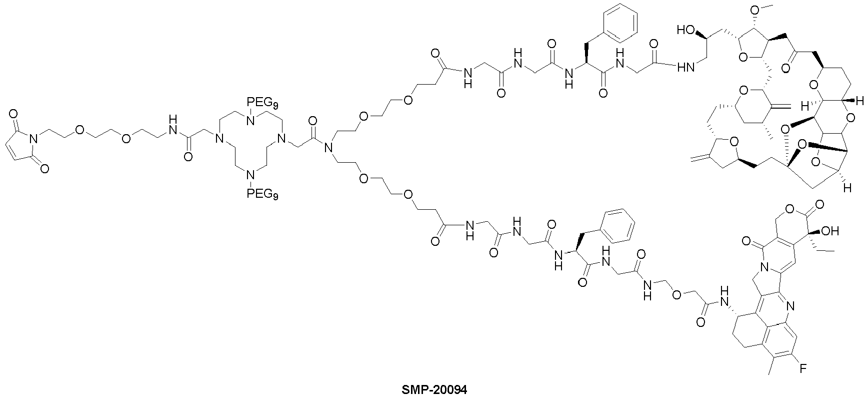

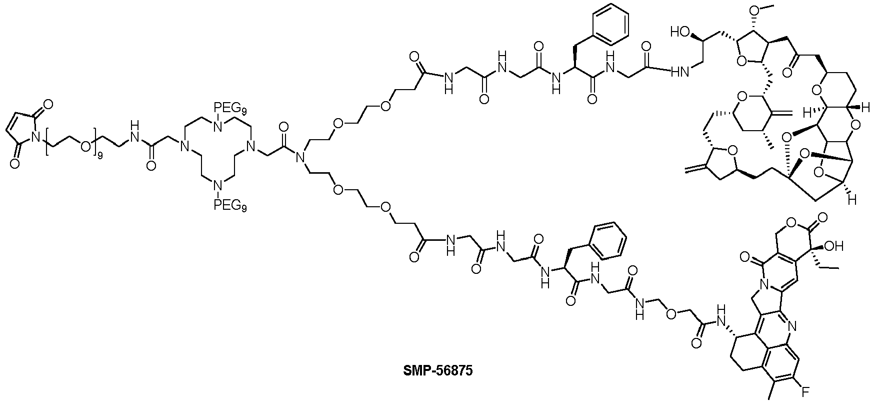

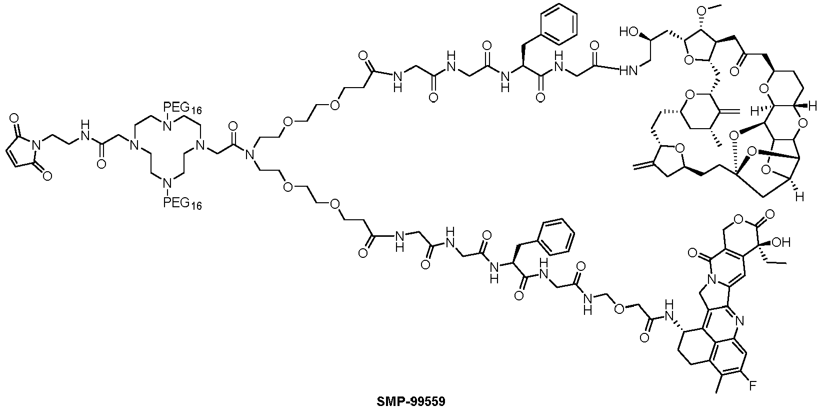

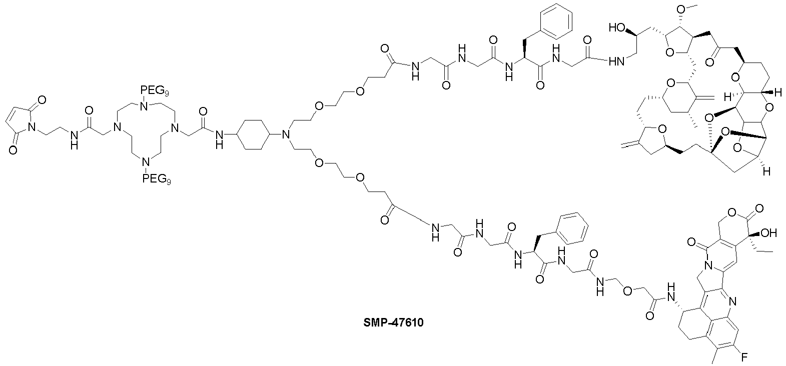

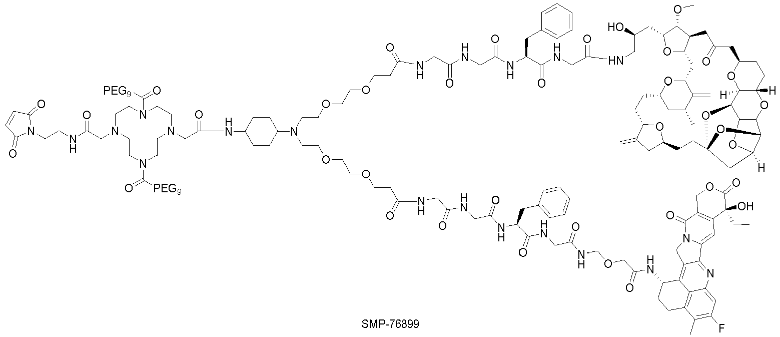

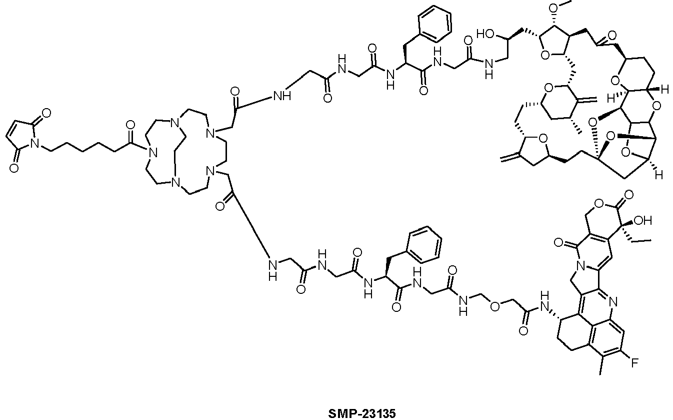

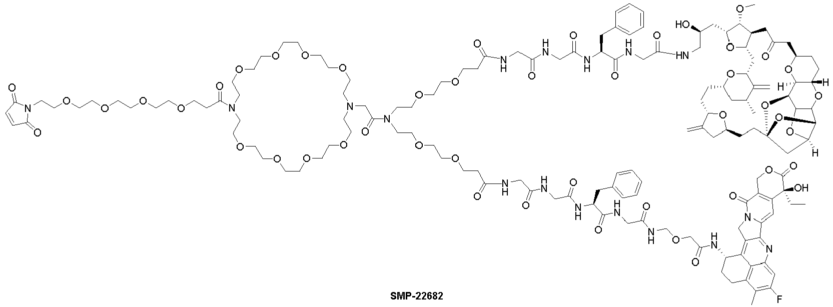

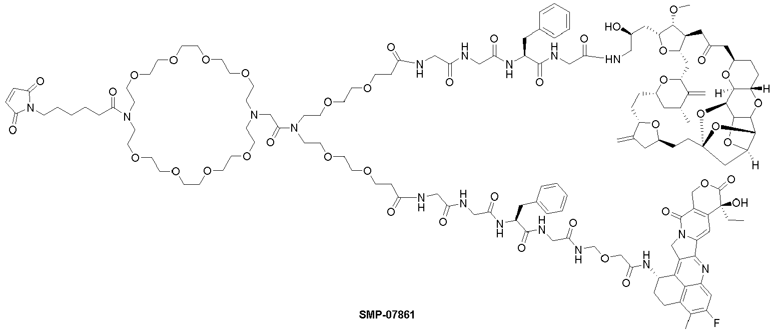

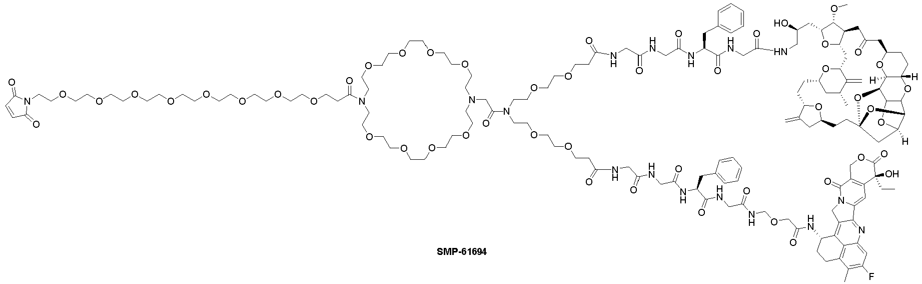

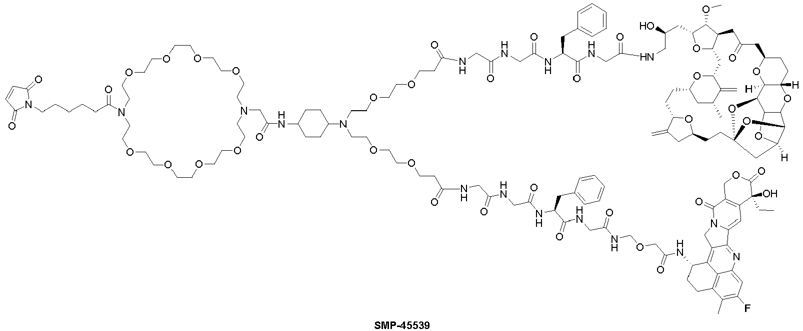

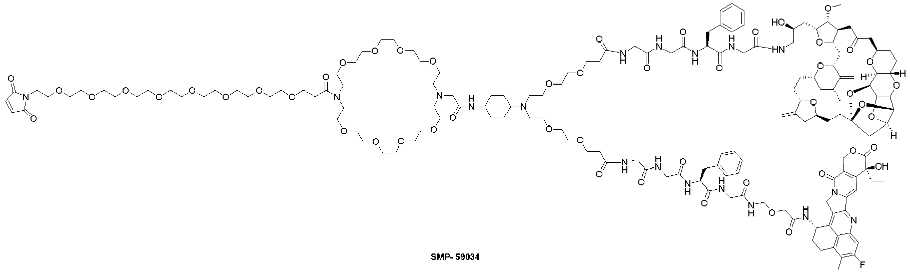

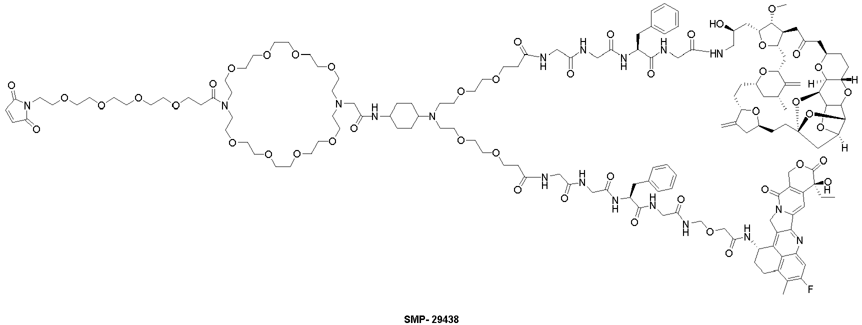

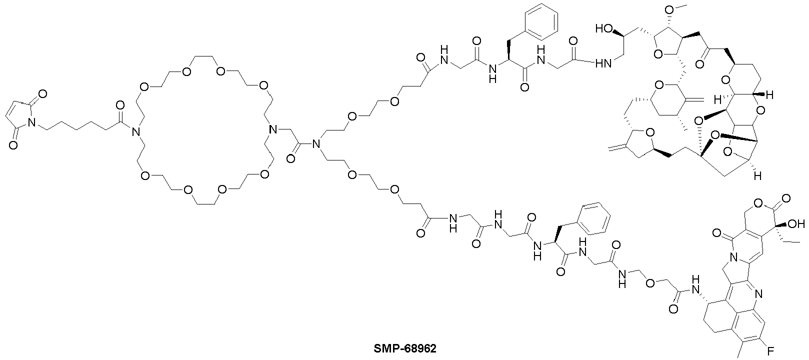

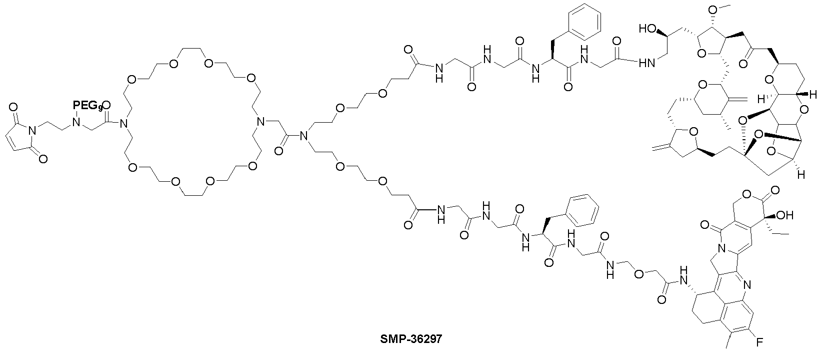

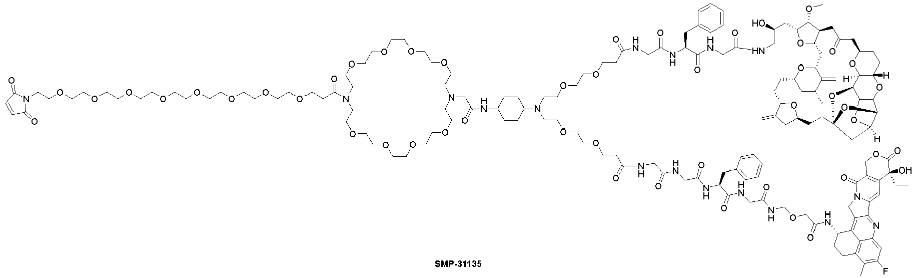

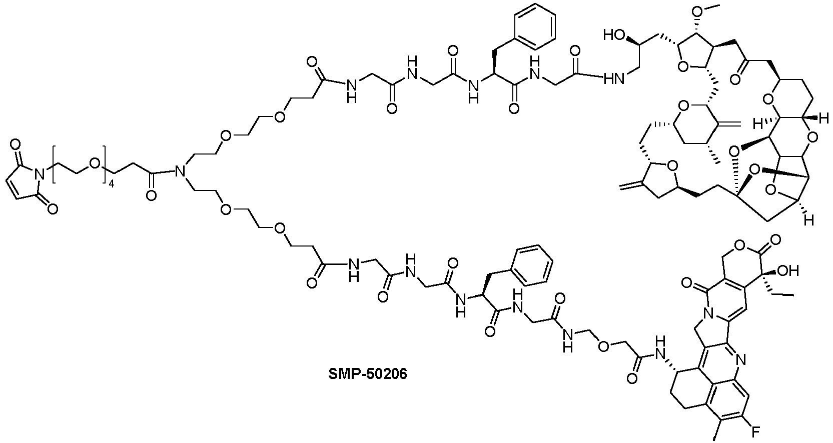

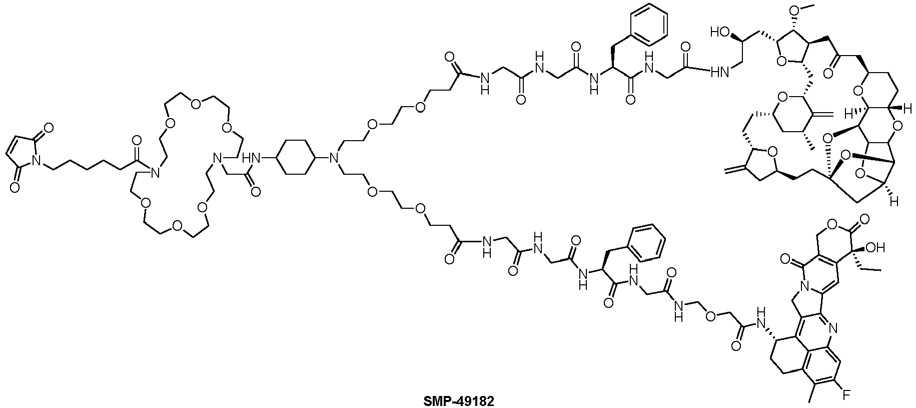

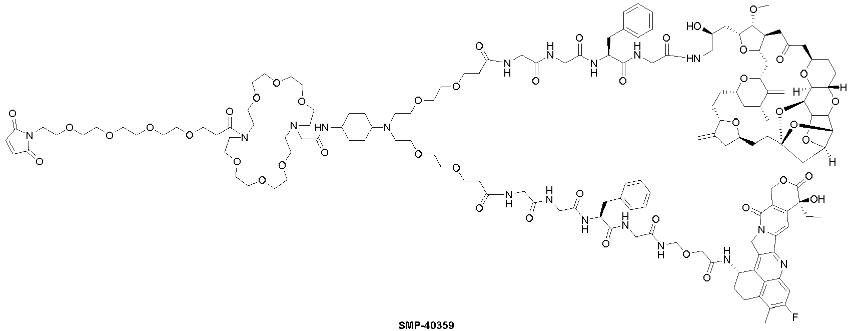

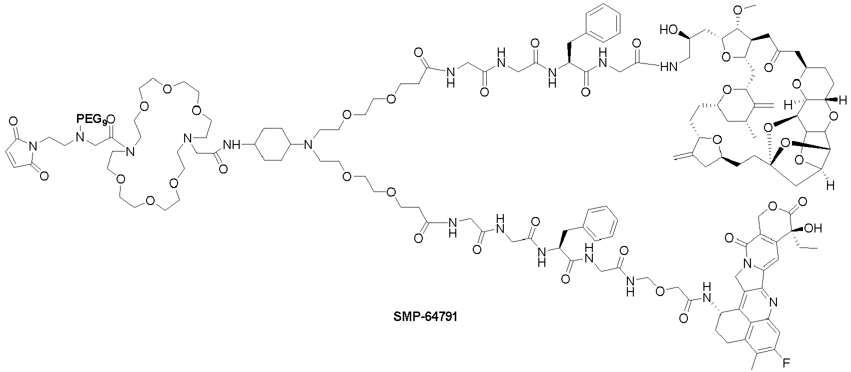

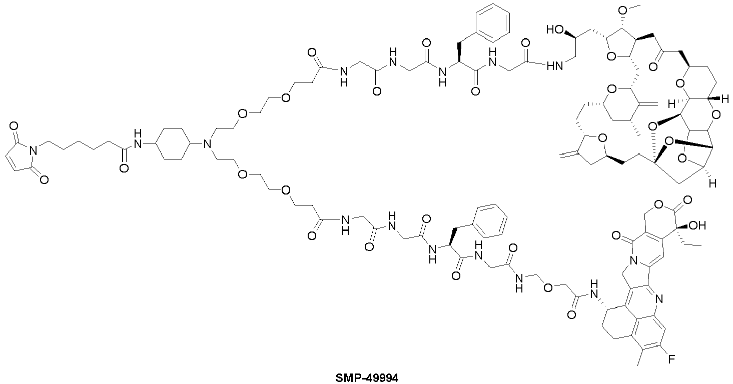

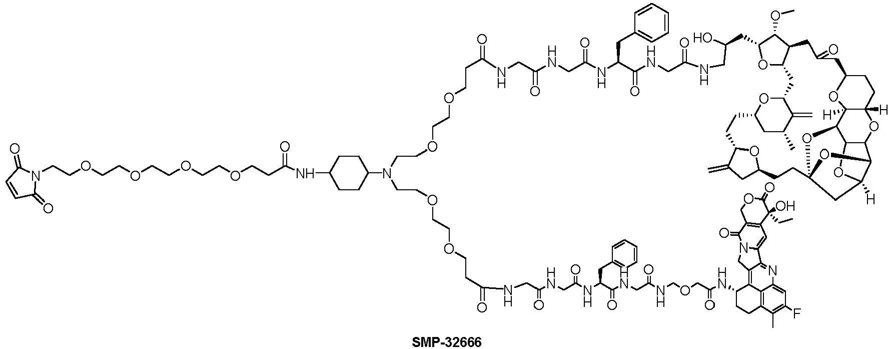

-PEG₉ represents 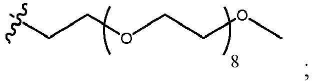 and -PEG₁₆ represents

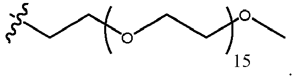

The present invention further provides a targeted linker-drug conjugate or a stereoisomer, optical isomer, salt, or deuterated compound thereof. The targeted linker-drug conjugate is obtained by connecting a targeted linker to the above-mentioned drug link assembly unit, and has a structure as shown in formula J-1; 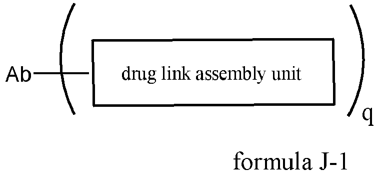
Ab is the targeted linker; q is an integer from 1 to 20; and
the targeted linker is a substance capable of targeted binding to a lesion site.

Further, the structure of the targeted linker-drug conjugate is as shown in formula J-2 or J-3: 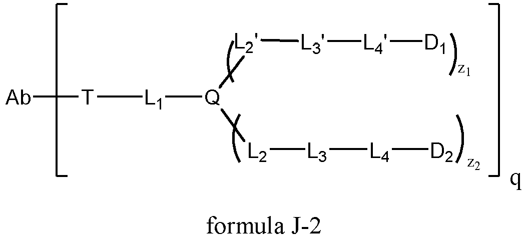 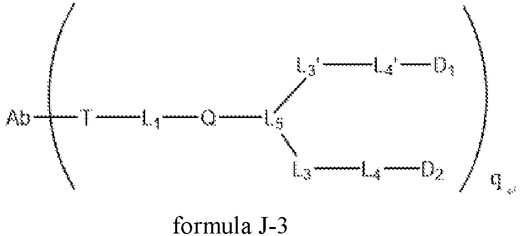
wherein Ab is the targeted linker; q is an integer from 1 to 20;
T, L₁, Q, L₂, L₃, L₄, L₂', L₃', L₄', L₅, D₁, D₂, Z₁, and Z₂ are as described above;
preferably, the structure of the targeted linker-drug conjugate is as shown in formula J-4:

   Ab-(T-L₁-Q-L₂-L₃-L₄-D₂)_{q} formula J-4
wherein Ab is the targeted linker; q is an integer from 1 to 20; and
T, L₁, Q, L₂, L₃, L₄, and D₂ are as described above.

Further, a DAR value of the targeted linker-drug conjugate is 1.00 to 20.00, preferably 2.0 to 8.0, and more preferably 2.0 to 5.0.

Further, the structure of the targeted linker-drug conjugate is selected from:

| | |
|---|---|
| SMP-82891a | 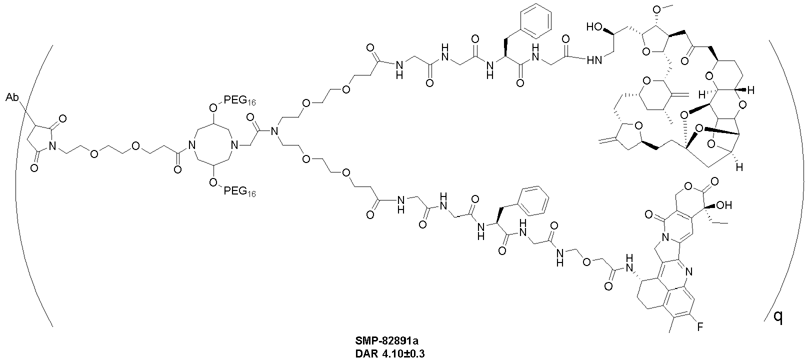 |
| SMP-56822a | 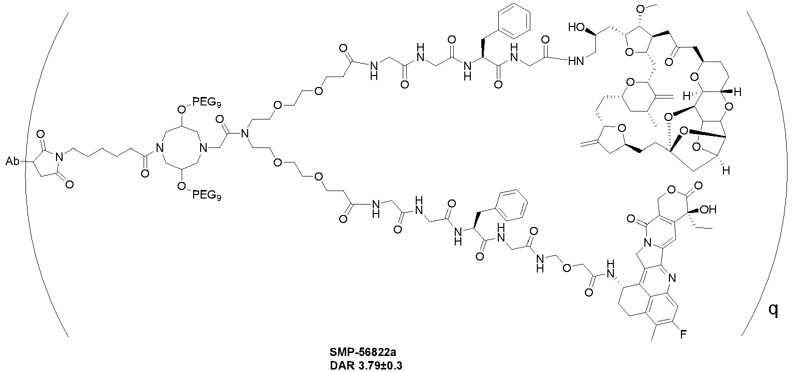 |
| SMP-51013-Aa | 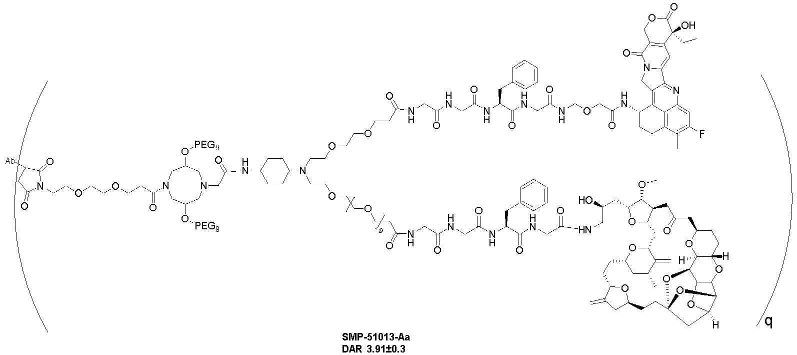 |
| SMP-51013-Ba | 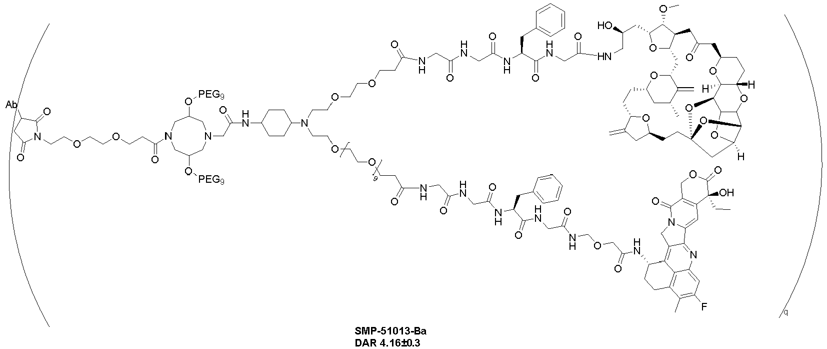 |
| SMP-36256a | 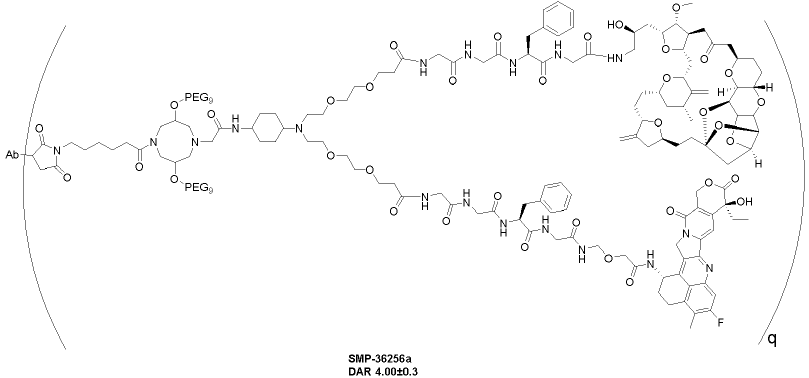 |
| SMP-45582a | 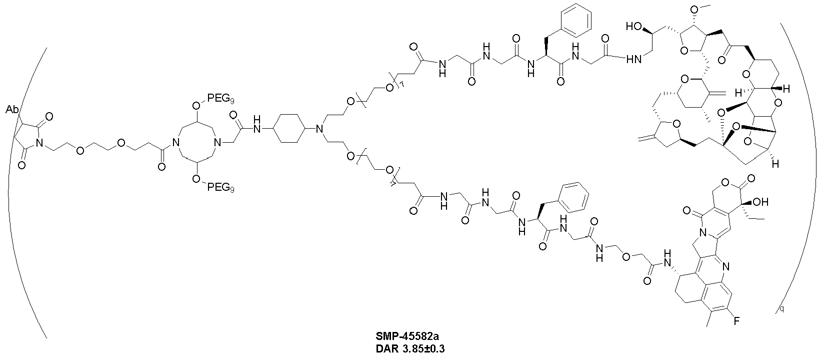 |
| SMP-01984a | 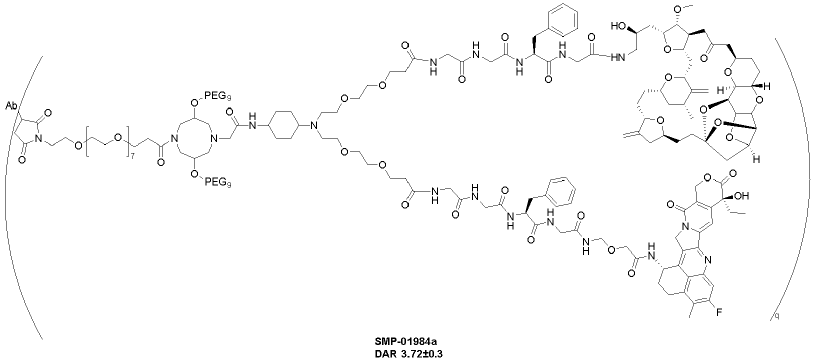 |
| SMP-30920a | 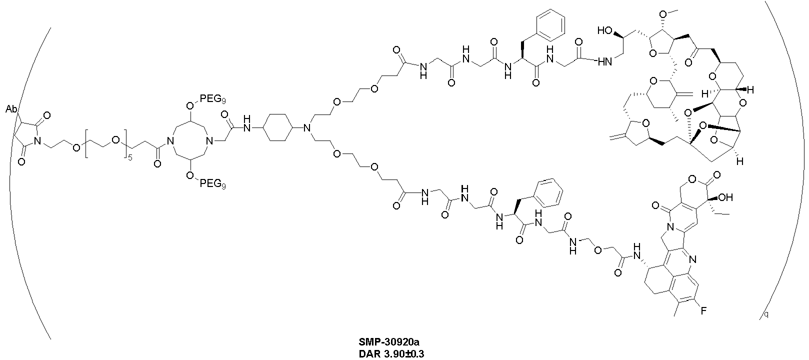 |
| SMP-14324a | 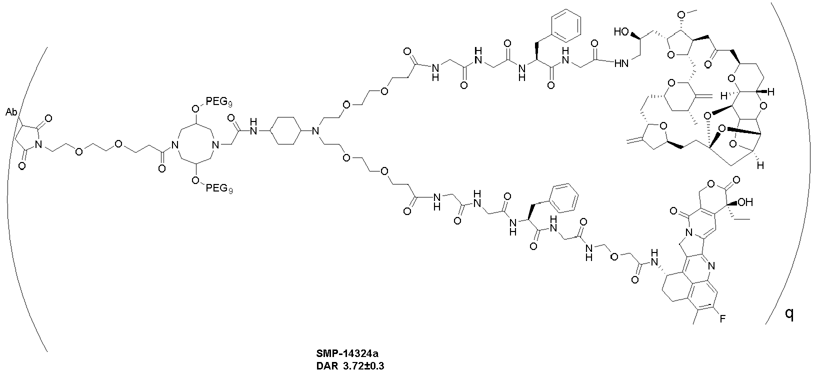 |
| SMP-61393a | 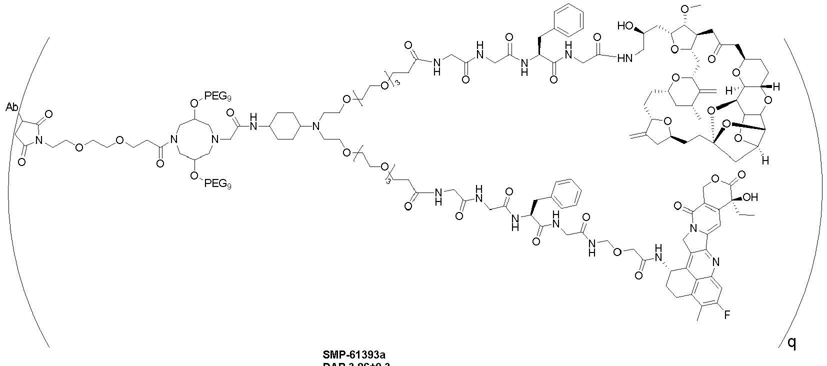 |
| SMP-21139a | 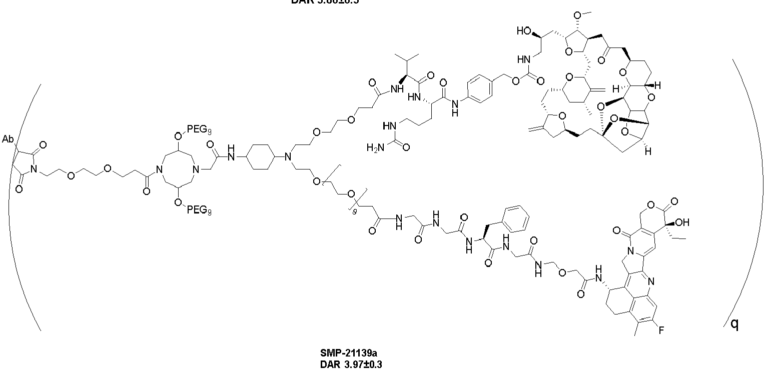 |
| SMP-68691a | 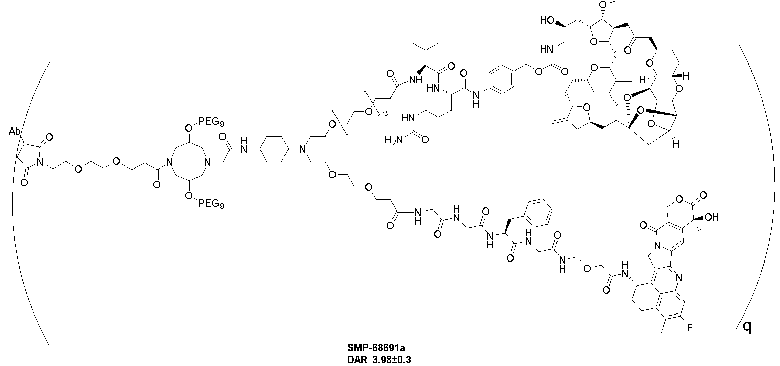 |
| SMP-39205a | 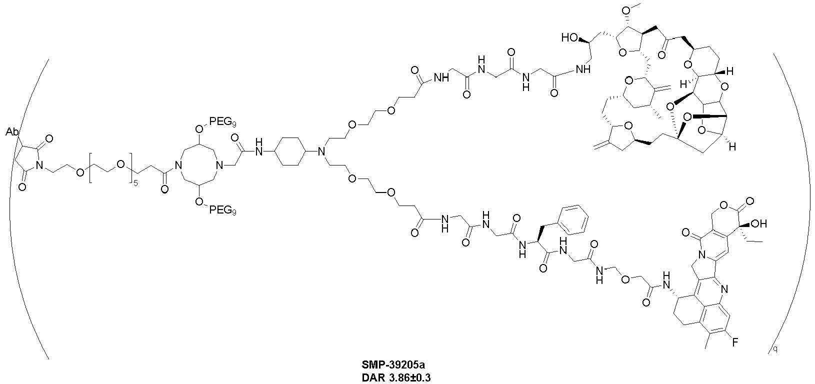 |
| SMP-81404a | 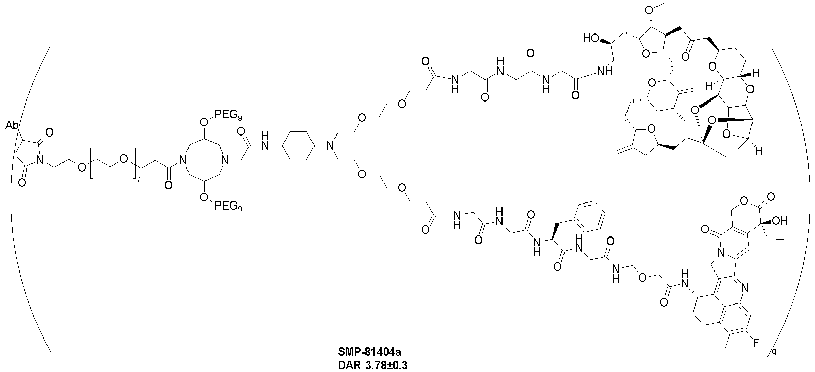 |
| SMP-64906a | 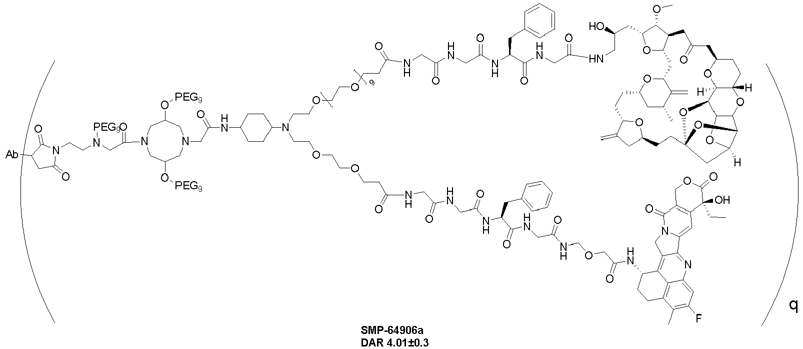 |
| SMP-66348a | 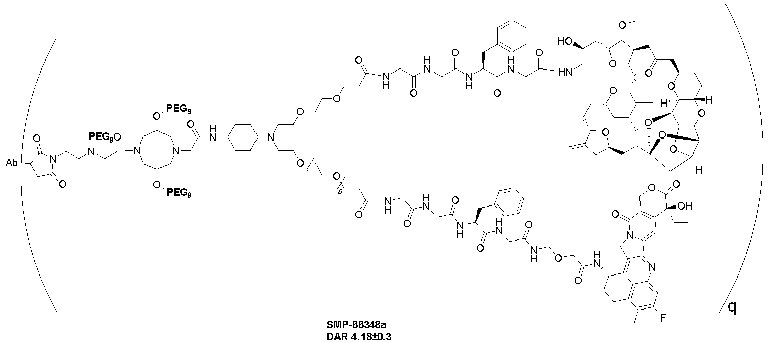 |
| SMP-37860a | 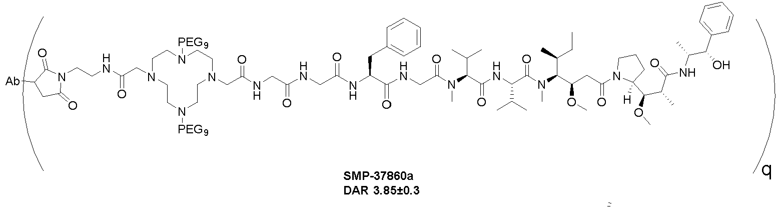 |
| SMP-18777a | 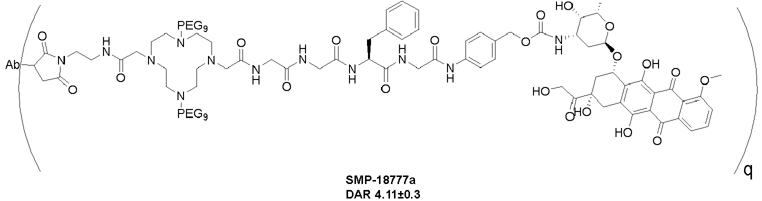 |
| SMP-56368a | 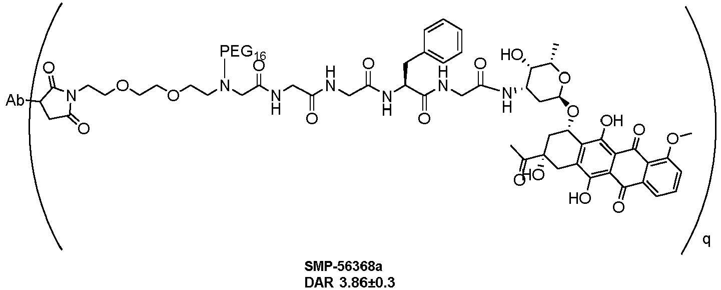 |
| SMP-58054a | 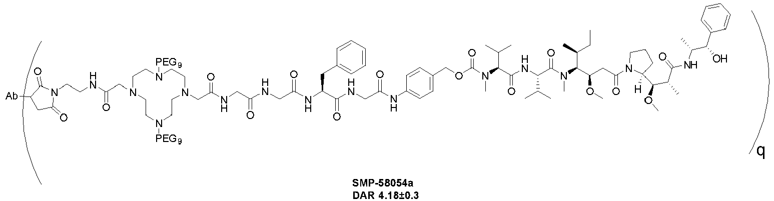 |
| SMP-25274a | 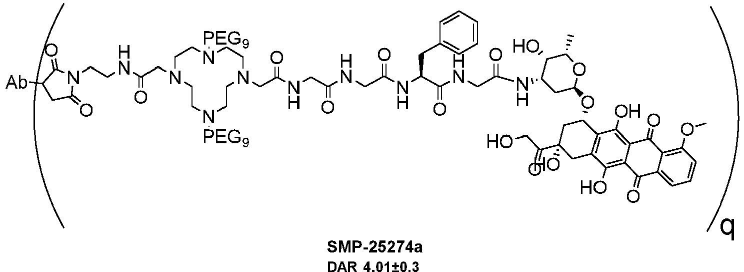 |
| SMP-56875a | 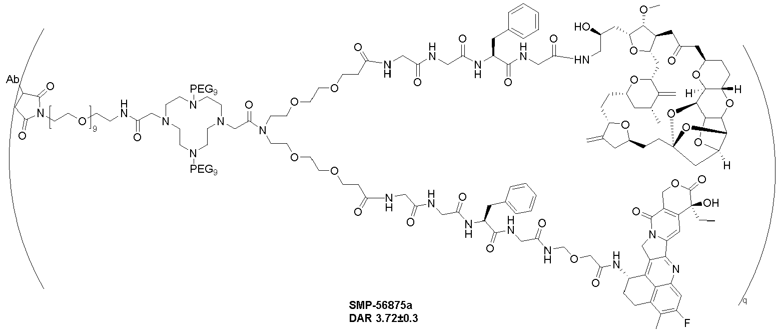 |
| SMP-82444a | 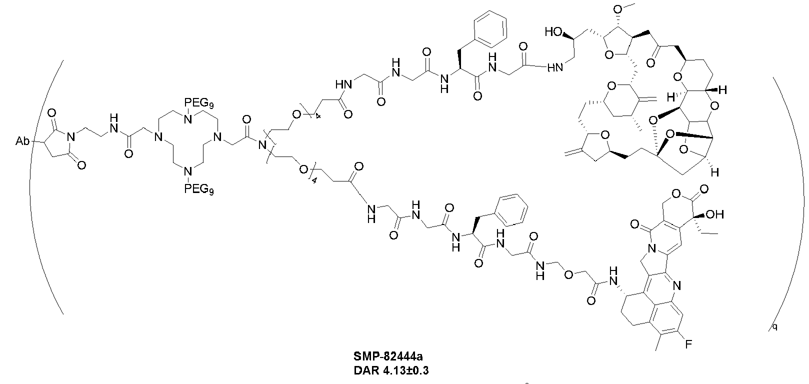 |
| SMP-50309a | 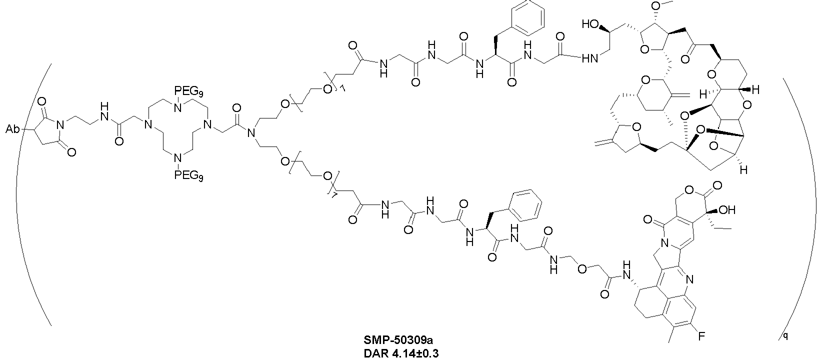 |
| SMP-20094a | 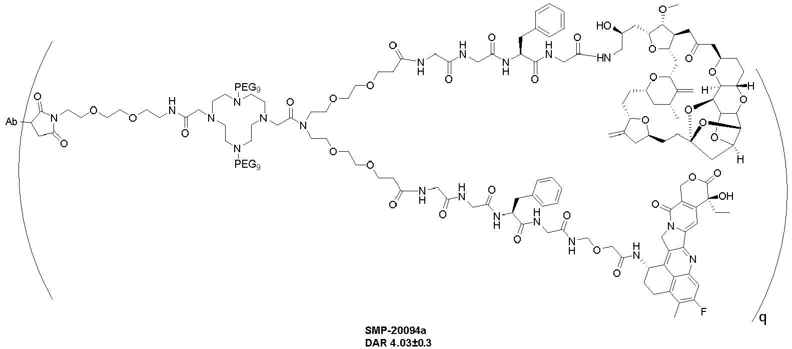 |
| SMP-99559a | 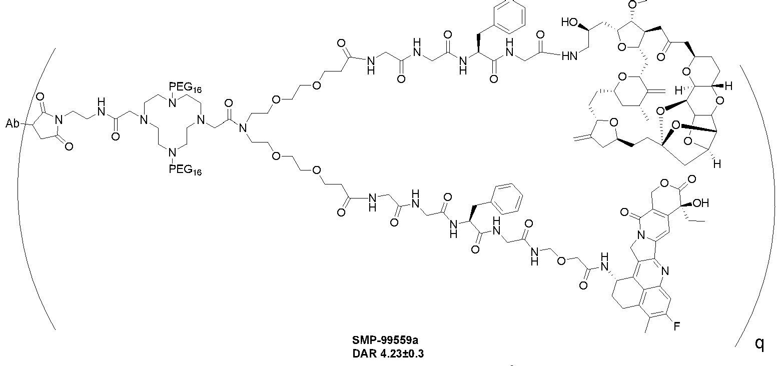 |
| SMP-59623a | 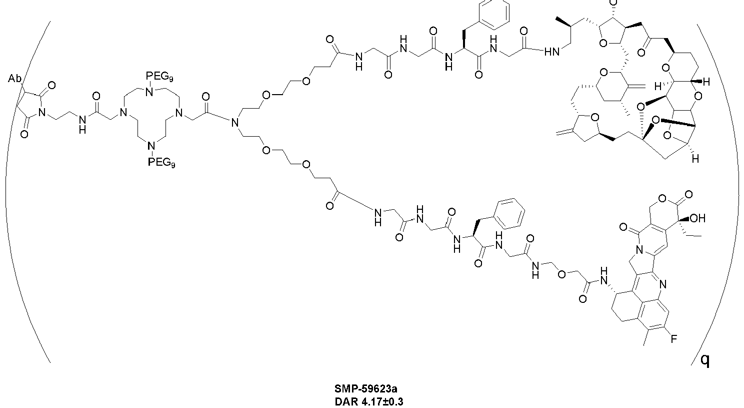 |
| SMP-47610a | 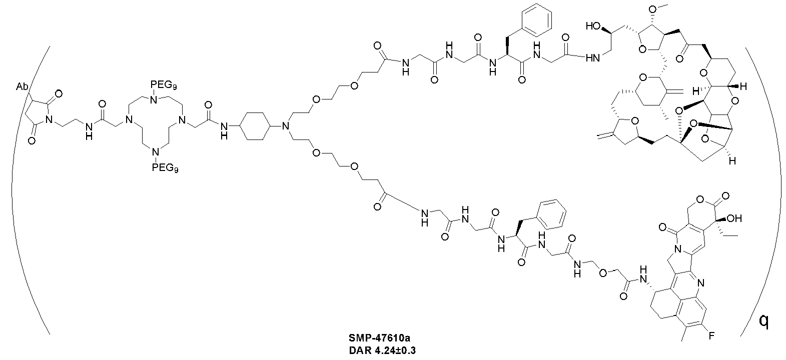 |
| SMP-86467a | 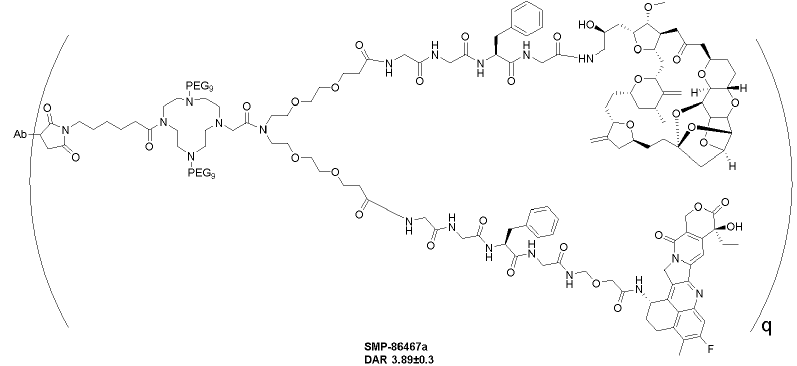 |
| SMP-76899a | 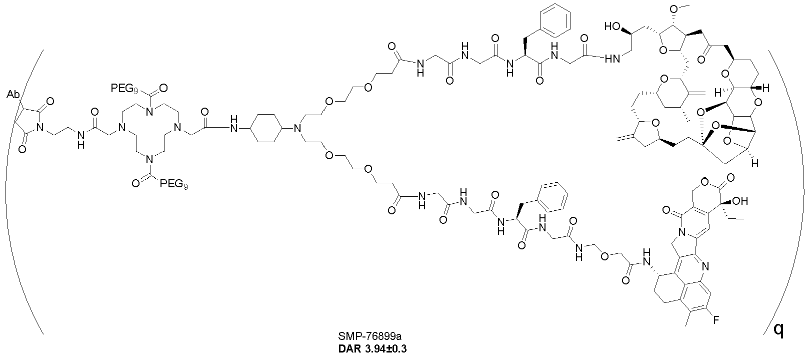 |
| SMP-27341a | 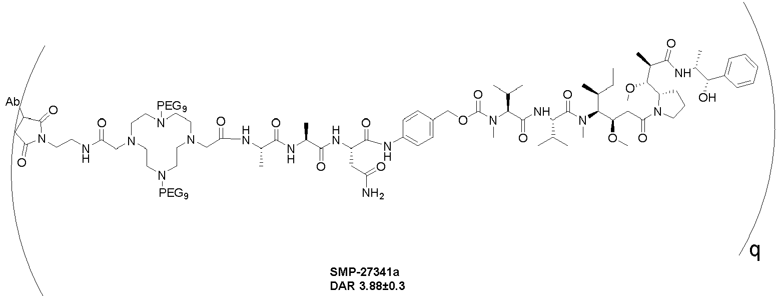 |
| SMP-34421a | 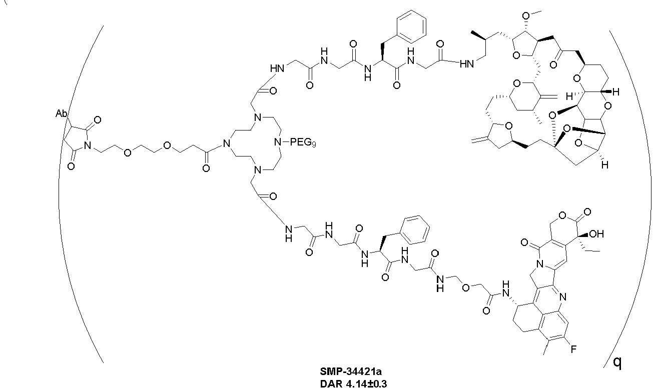 |
| SMP-53612a | 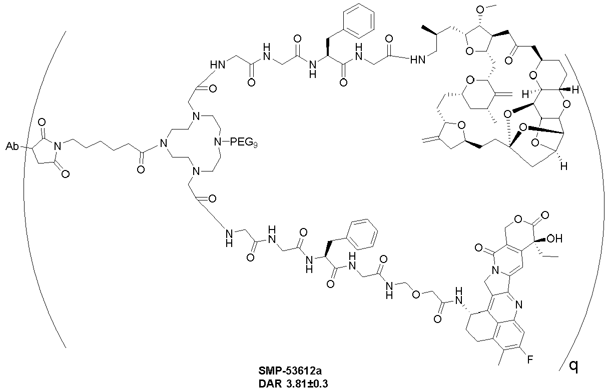 |
| SMP-10215a | 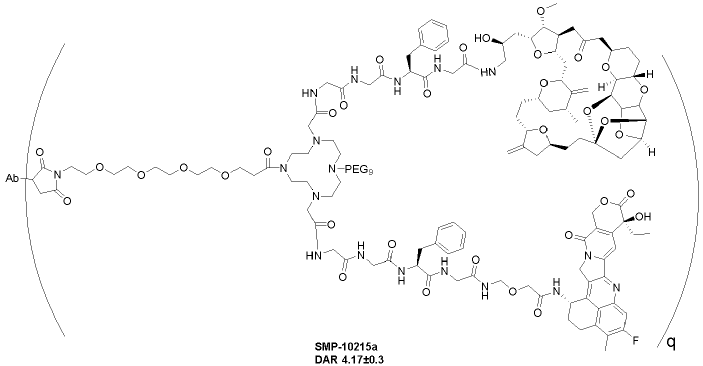 |
| SMP-28945a | 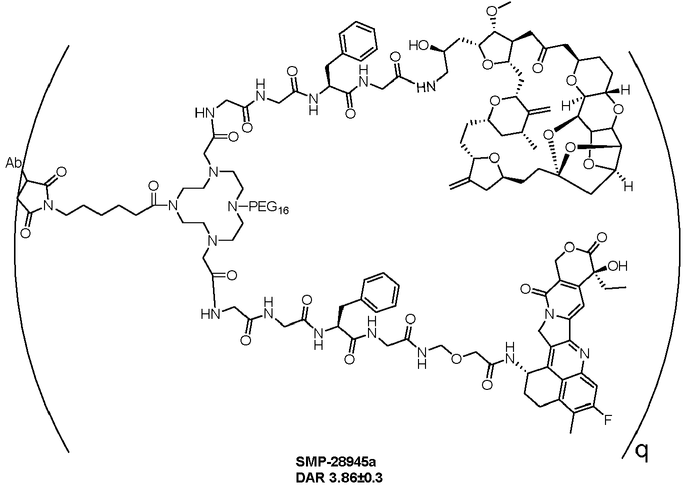 |
| SMP-34710a | 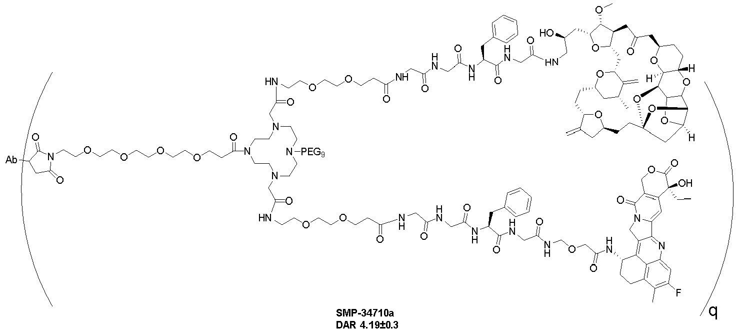 |
| SMP-35055a | 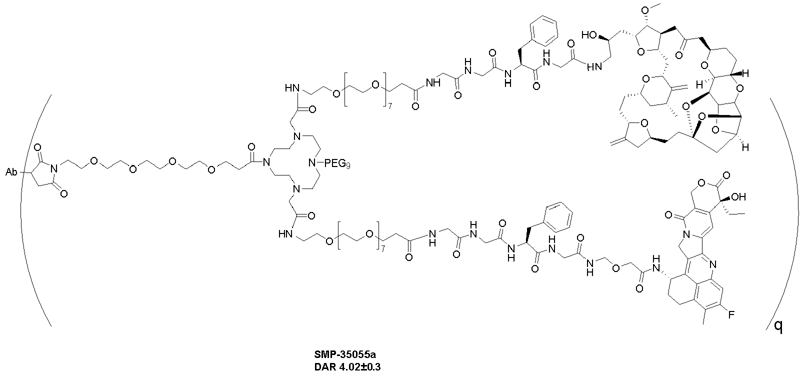 |
| SMP-94170-Aa | 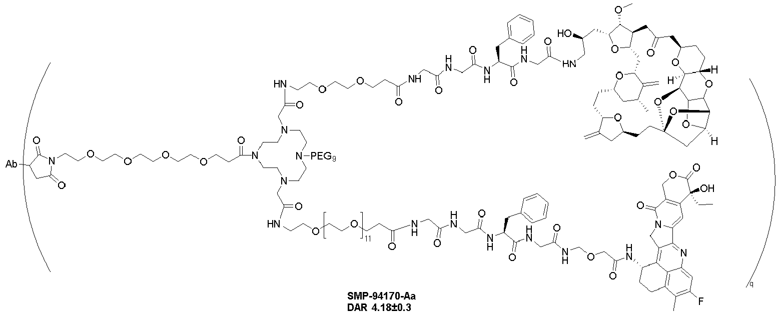 |
| SMP-44419a | 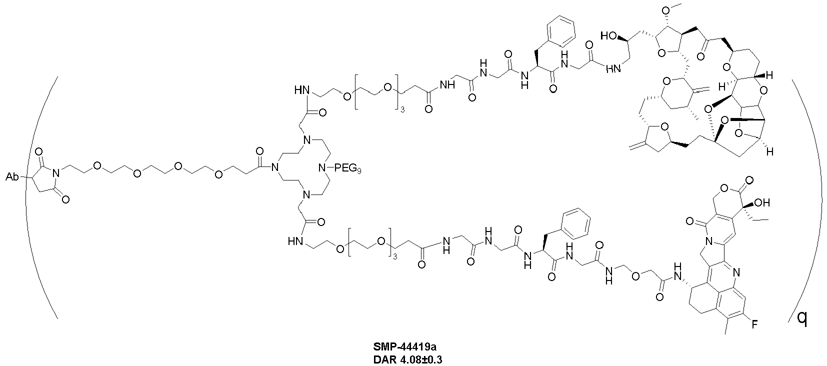 |
| SMP-94170-Ba | 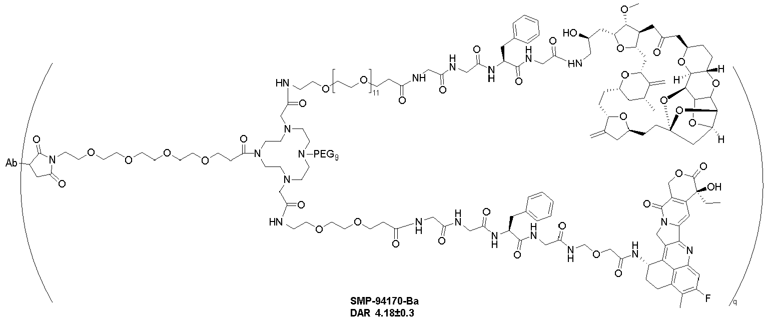 |
| SMP-46940a | 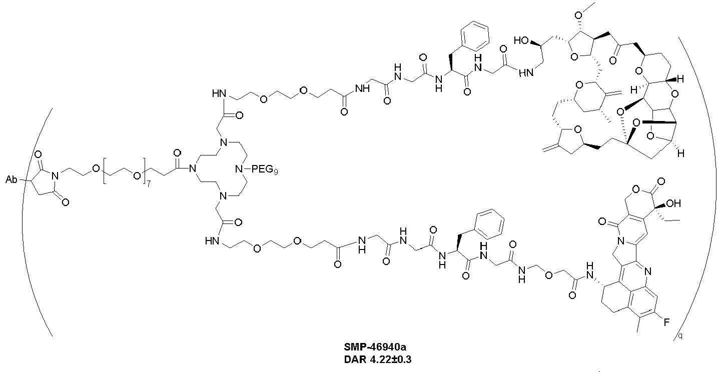 |
| SMP-13069a | 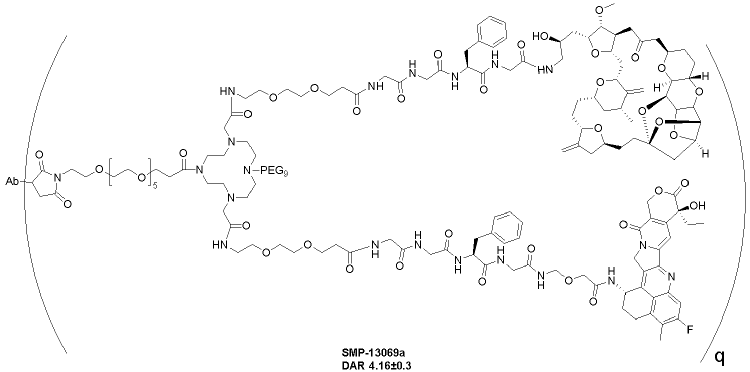 |
| SMP-28823a | 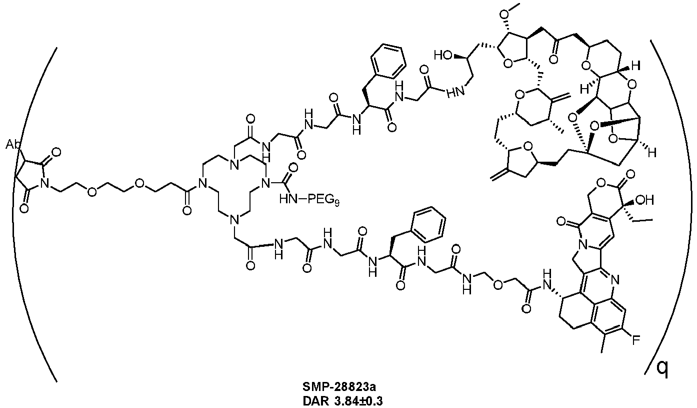 |
| SMP-53816a | 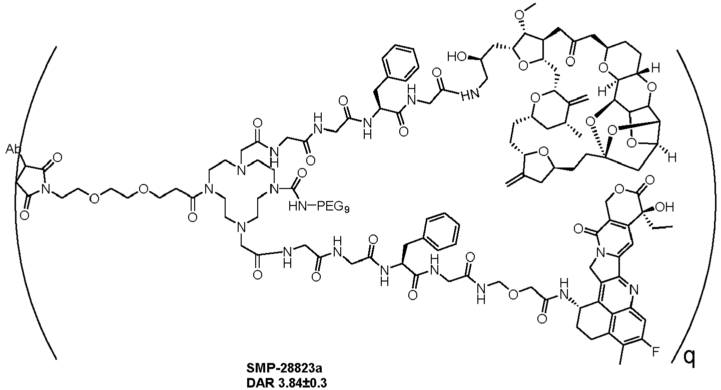 |
| SMP-23135a | 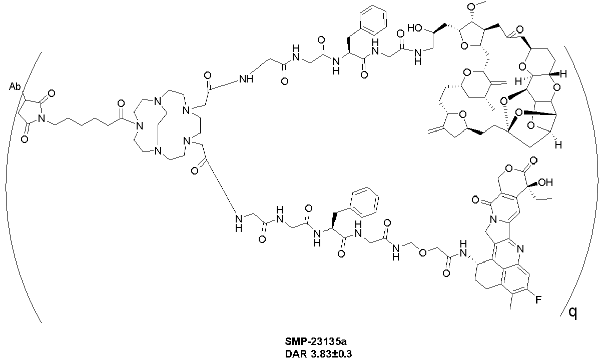 |
| SMP-33693a | 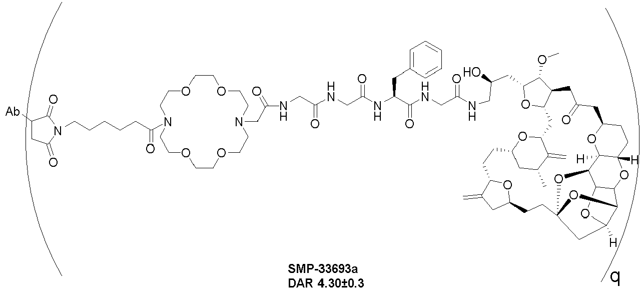 |
| SMP-42930a | 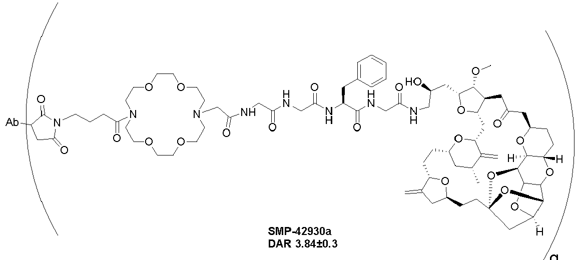 |
| SMP-52573a | 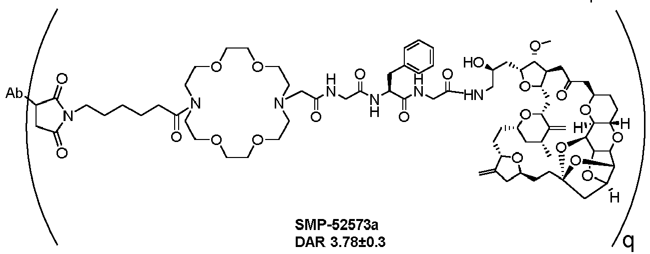 |
| SMP-80439a | 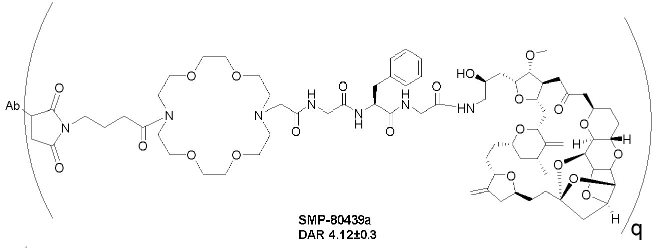 |
| SMP-22682a | 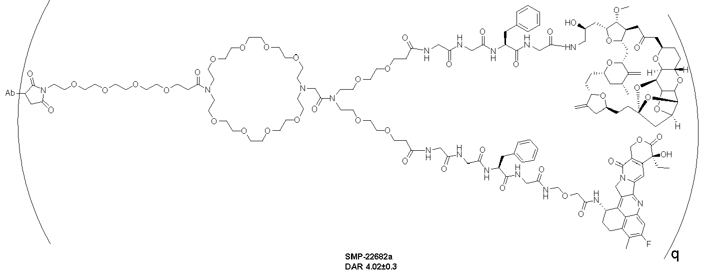 |
| SMP-07861a | 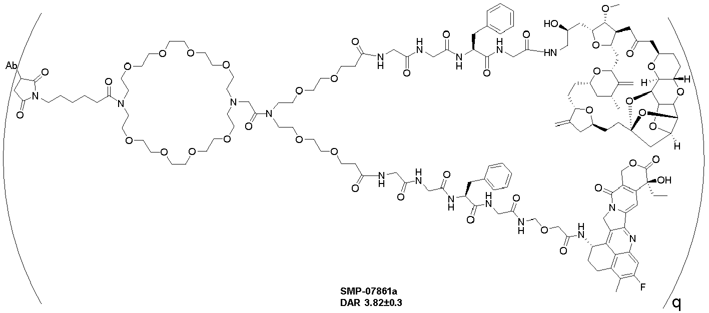 |
| SMP-61694a | 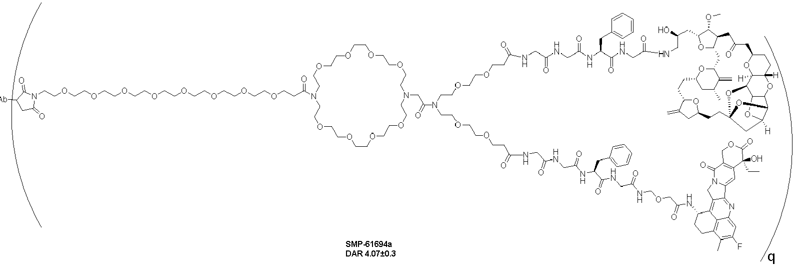 |
| SMP-45539a | 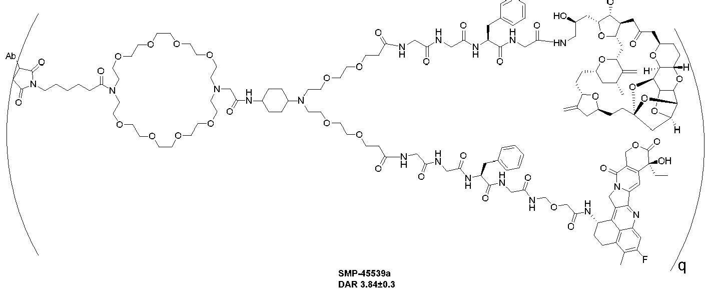 |
| SMP-59034a | 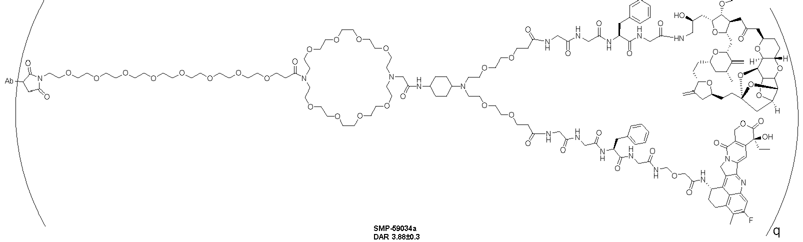 |
| SMP-29438a | 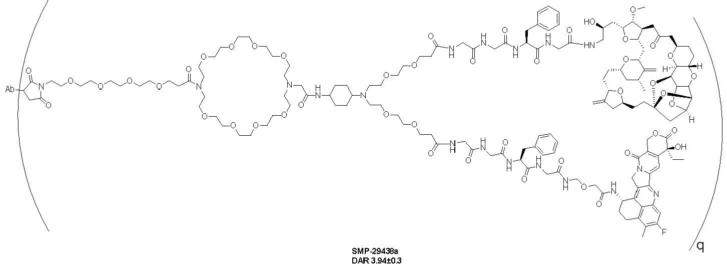 |
| SMP-68962a | 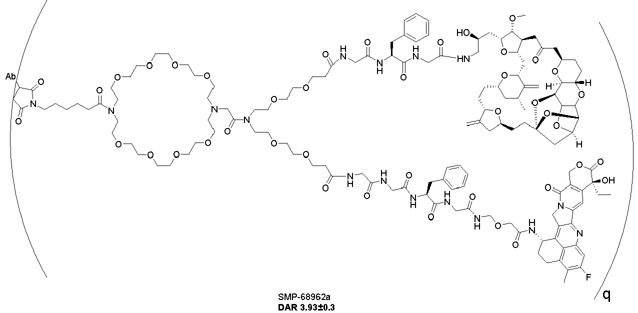 |
| SMP-36297a | 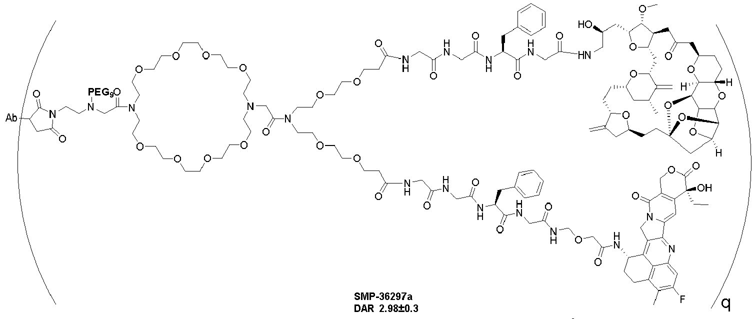 |
| SMP-31135a | 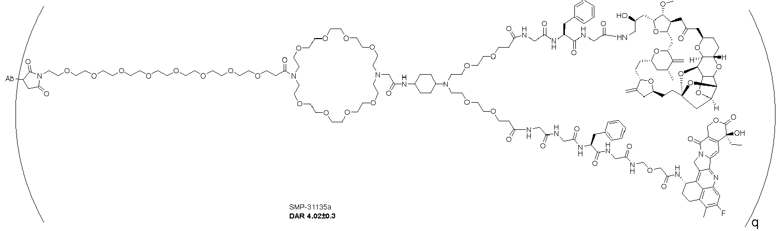 |
| SMP-49182a | 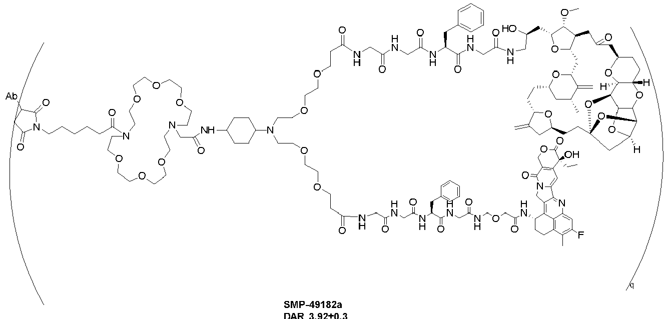 |
| SMP-40359a | 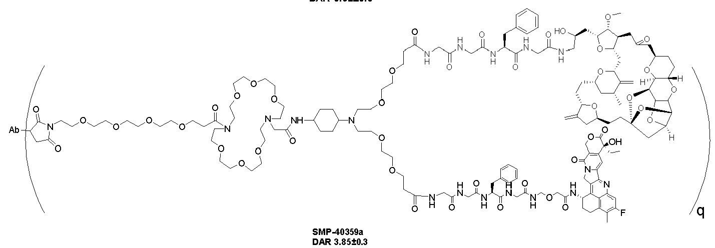 |
| SMP-64791a | 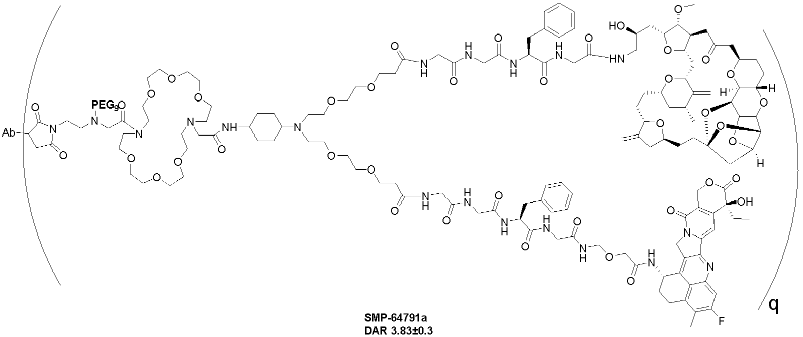 |
| SMP-00171a | 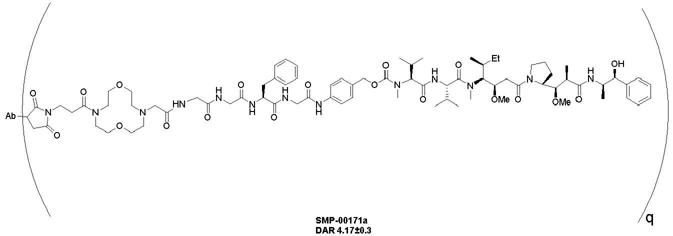 |
| SMP-93566a | 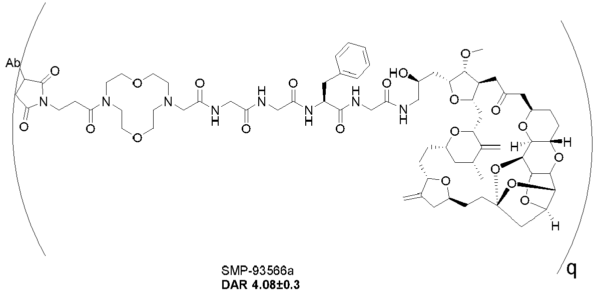 |
| SMP-88480a | 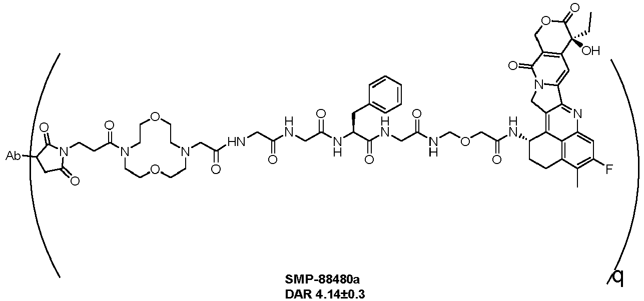 |
| SMP-34048a | 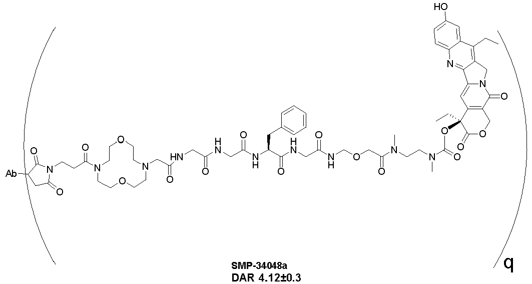 |
| SMP-97962a | 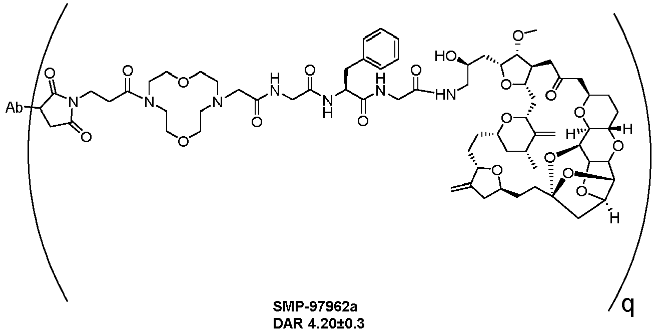 |
| SMP-24600a | 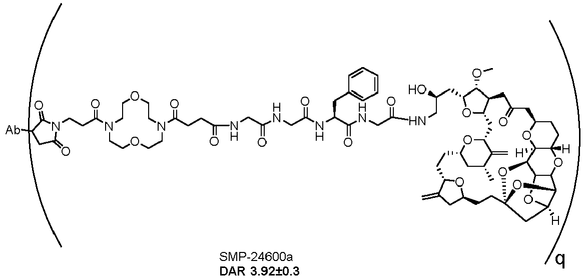 |
| SMP-12395a | 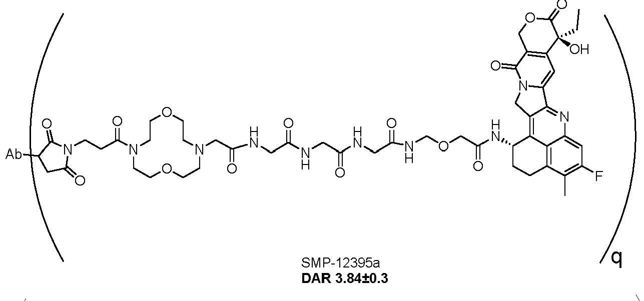 |
| SMP-87055a | 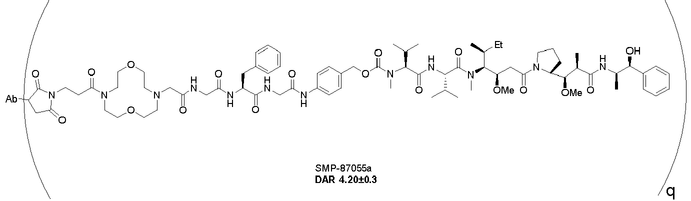 |
| SMP-02692a | 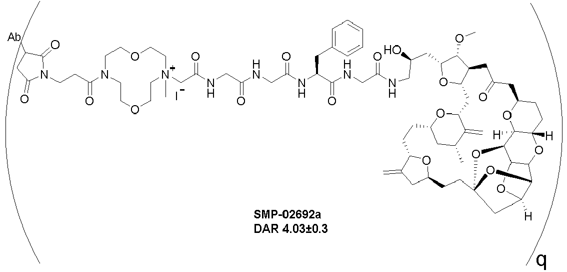 |
| SMP-34146a | 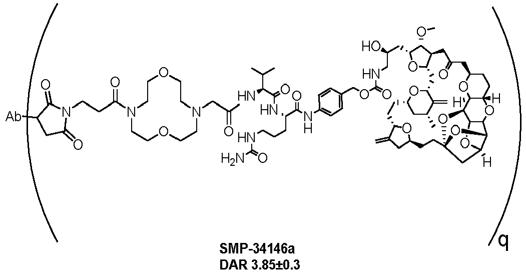 |
| SMP-26598a | 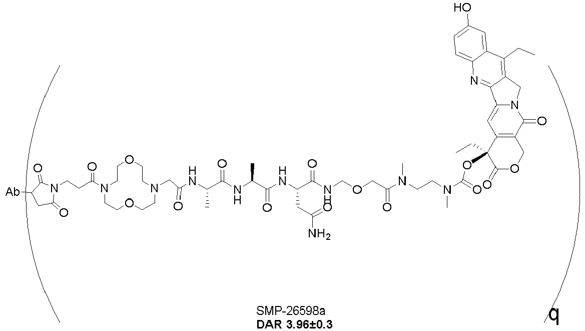 |
| SMP-60226a | 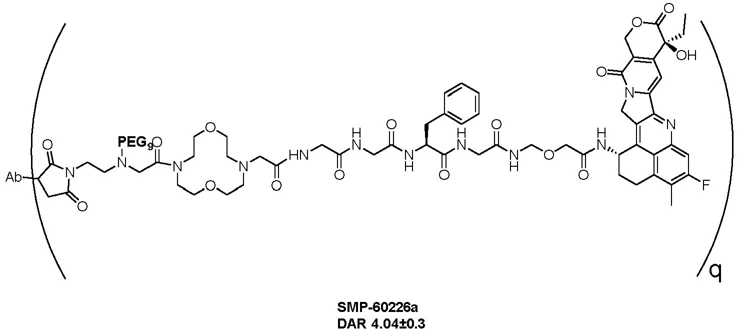 |
| SMP-09168a | 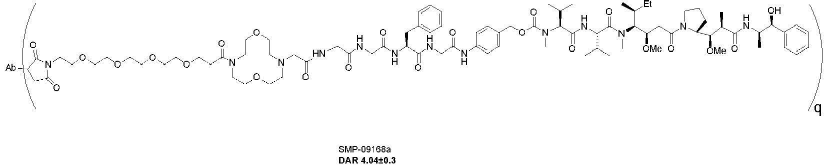 |
| SMP-79575a | 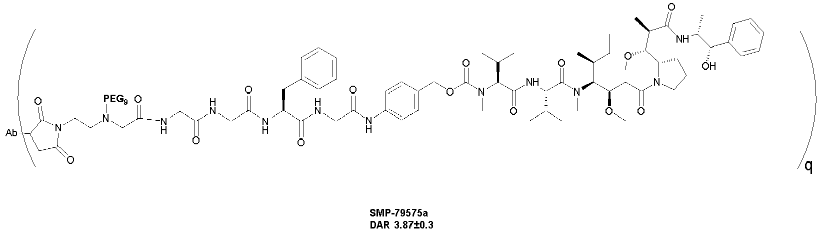 |
| SMP-80035a | 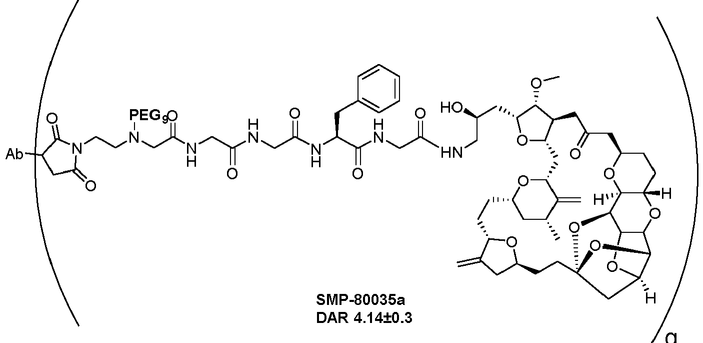 |

q is an integer from 1 to 20; and
-PEG₉ represents 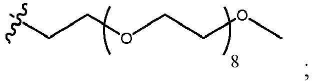 and -PEG₁₆ represents

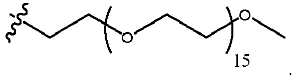

The specific targeted linker-drug conjugates listed above are labeled with DAR values according to test results of examples.

The DAR values have an error range due to the influence of test conditions and testers. According to current test means, the error range of DAR values is usually ± 0.3, but due to changes in test means, the error range may also vary. The key core of the targeted linker-drug conjugates lies in their structural formula. As long as the structural formula is the same as the specific targeted linker-drug conjugates listed above, they fall within the scope of protection of the present invention.

The specific targeted linker-drug conjugates listed above use HER2 antibodies as Ab in the examples.

Further, the targeted linker is an antibody, antibody fragment, protein, small molecular polypeptide, glycopeptide, mimetic peptide, small molecular compound, or nucleic acid oligonucleotide adapter; and
preferably, the antibody is an antibody targeting cell surface receptors and tumor associated antigens.

The present invention further provides a linker used for a targeted linker-drug conjugate, or a stereoisomer, optical isomer, salt, or deuterated compound thereof. The structure of the linker is as shown in formula K-1 or K-2: 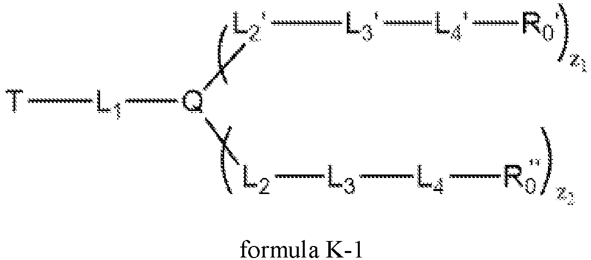 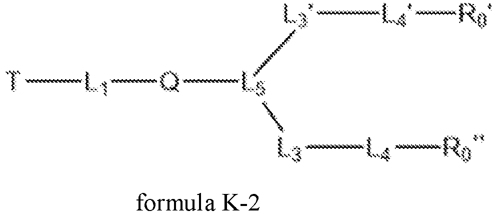
wherein T is a tying group, which can be connected to the targeted linker; the targeted linker is a substance capable of targeted binding to a lesion site;
L₁ is none or a breakable or unbreakable linker fragment;
L₂ is none or a breakable or unbreakable linker fragment;
L₂' is none or a breakable or unbreakable linker fragment;
L₃ is none or a breakable or unbreakable linker fragment;
L₃' is none or a breakable or unbreakable linker fragment;
L₄ is none or a breakable or unbreakable linker fragment;
L₄' is none or a breakable or unbreakable linker fragment;
L₅ is none or a breakable or unbreakable trident linker fragment;
R₀" is a leaving group;
R₀' is a leaving group;
Z₁ is 0 or 1, Z₂ is 0 or 1, and Z₁ and Z₂ are not simultaneously 0; and
the structure of Q is shown in formula A as described above.

Further, the leaving group is hydroxyl.

Further, the structure of L₅ is selected from 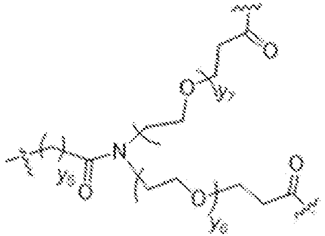 or 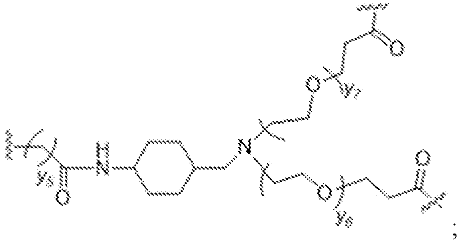
y₅ is an integer selected from 1 to 10, preferably 1;
y₆ is an integer selected from 1 to 50, preferably an integer from 2 to 9; and
y₇ is an integer selected from 1 to 50, preferably an integer from 2 to 9.

Further, the structure of the linker is as shown in formula K-3:

T-L₁-Q-L₂-L₃-L₄-R₀" formula K-3

wherein T is a tying group, which can be connected to the targeted linker; the targeted linker is a substance capable of targeted binding to a lesion site;
L₁ is a breakable or unbreakable linker fragment;
L₂ is none or a breakable or unbreakable linker fragment;
L₃ is a breakable or unbreakable linker fragment;
L₄ is none or a breakable or unbreakable linker fragment;
R₀" is a leaving group; and
the structure of Q is shown in formula A as described above.

Further, the structure of T is as follows:

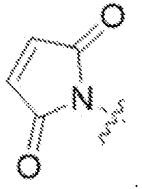

Further, the structure of L₁ is as follows: 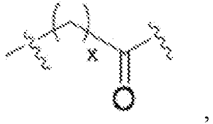 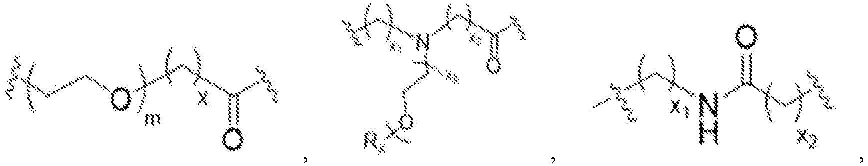 or 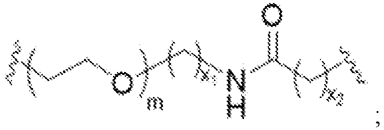
wherein x is an integer selected from 1 to 10, preferably an integer from 2 to 6;
m is an integer selected from 1 to 25, preferably an integer from 2 to 10, and more preferably an integer from 2 to 9;
x₁ is an integer selected from 1 to 10, preferably an integer from 2 to 6, and more preferably 2;
x₂ is an integer selected from 1 to 10, preferably an integer from 1 to 5, and more preferably 1;
x₃ is an integer selected from 0 to 50, preferably an integer from 8 to 30, and more preferably 9; and
Rₓ is selected from hydrogen or methyl.

Further, the structure of L₂ is 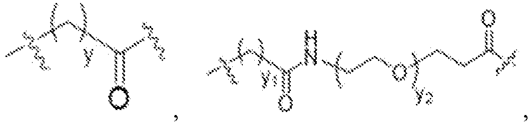 or 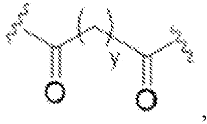 or L₂ is none;
the structure of L₂' is 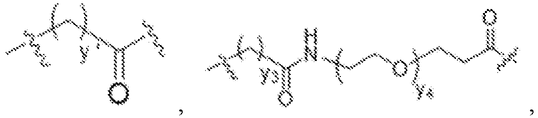 or 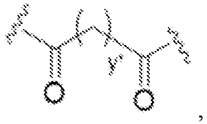 or L₂' is none;
wherein y and y' are integers independently selected from 1 to 10, preferably 1 or 2;
y₁ and y₃ are integers independently selected from 0 to 10, preferably 0, 1 or 2, and more preferably 0 or 1; and
y₂ and y₄ are integers independently selected from 1 to 50, preferably integers from 1 to 20, and more preferably integers from 2 to 12.

Further, the structures of L₃ and L₃' are independently selected from: 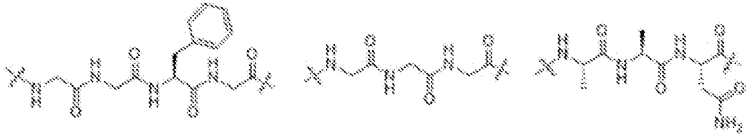 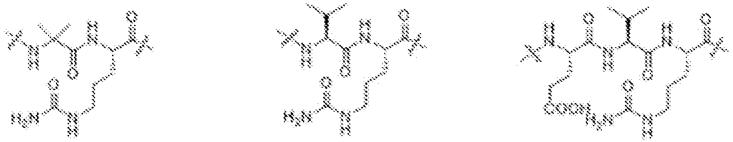 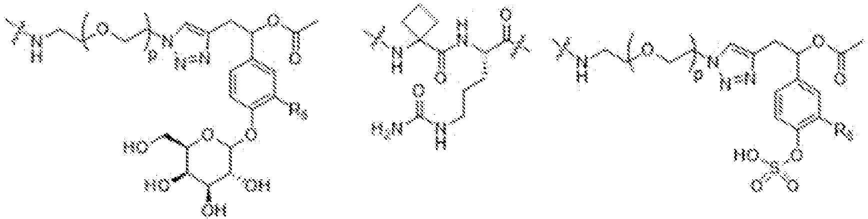 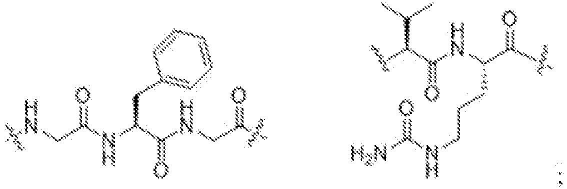 wherein p is an integer selected from 1 to 24, and R₆ is selected from hydrogen or nitro.

Further, the structures of L₄ and L₄' are independently selected from none,

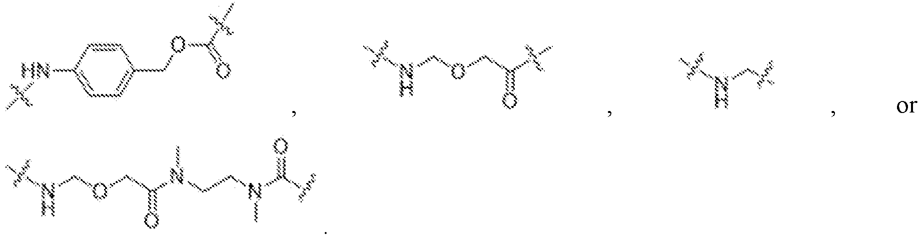

Further, the structure of the linker is as follows: 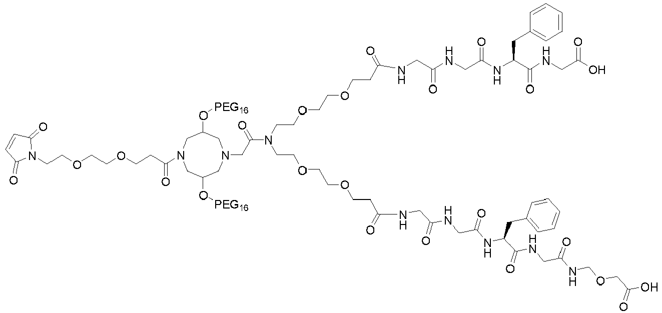 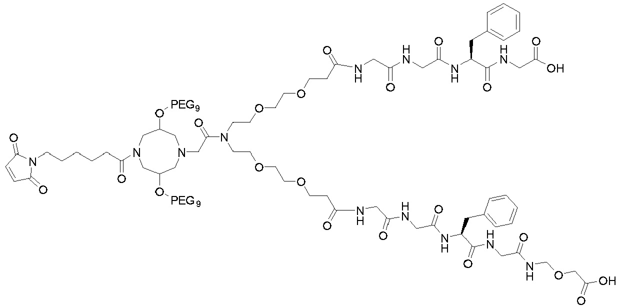 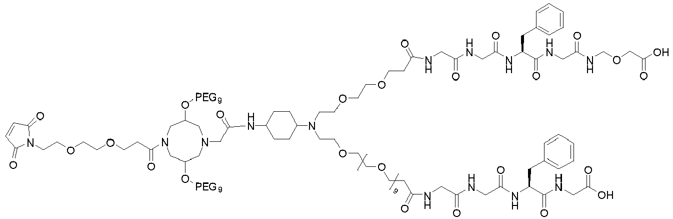 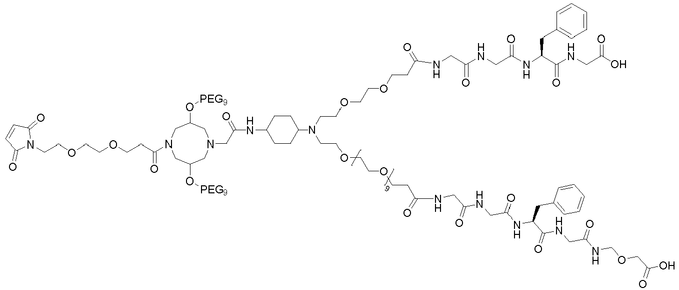 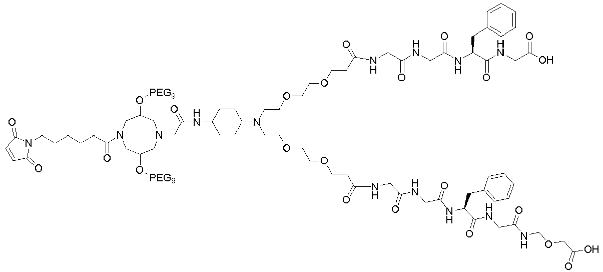 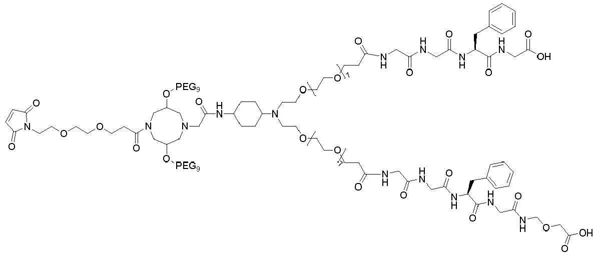 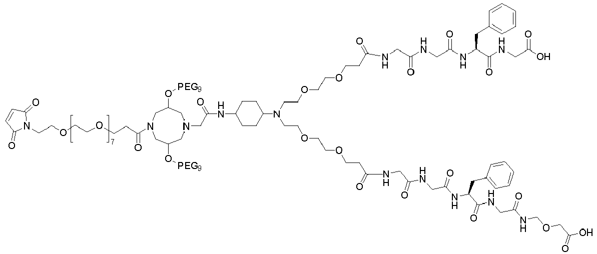 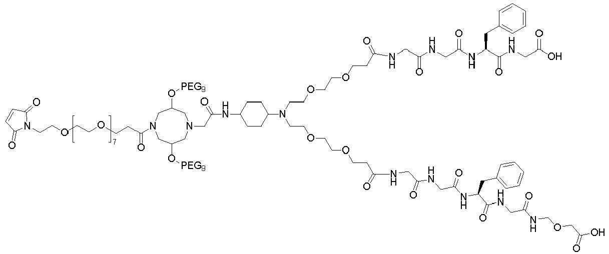 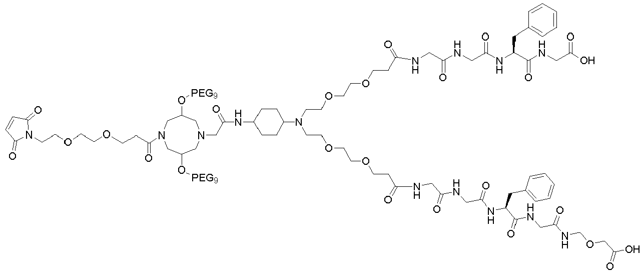 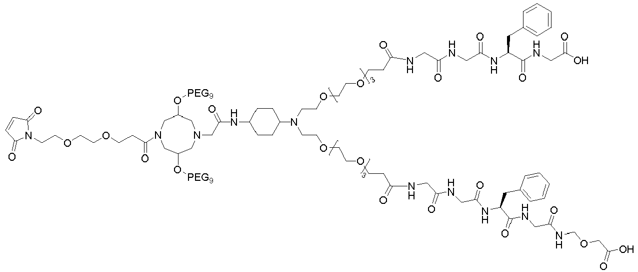 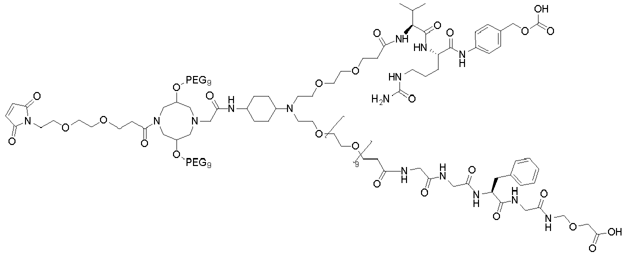 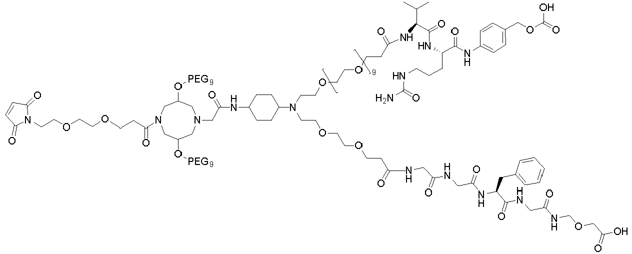 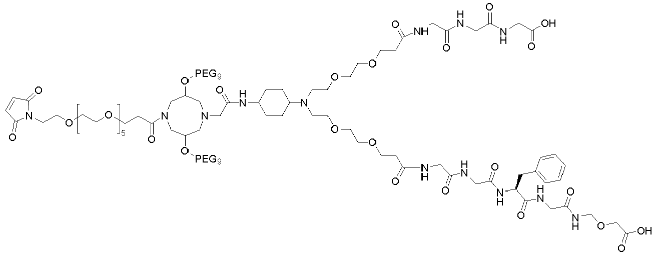 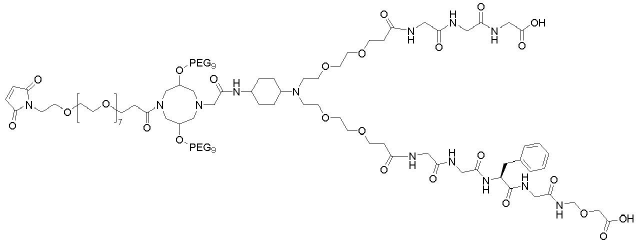 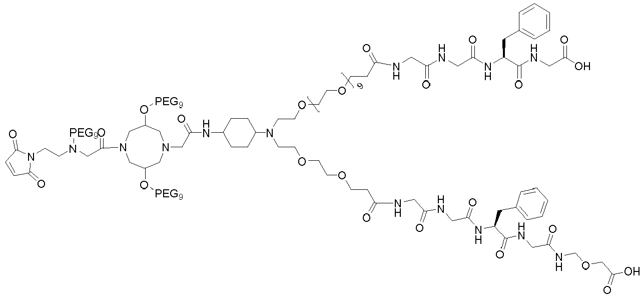 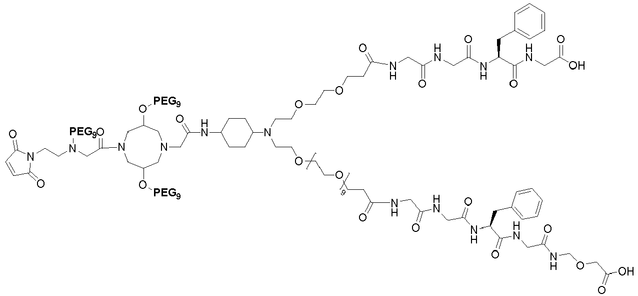 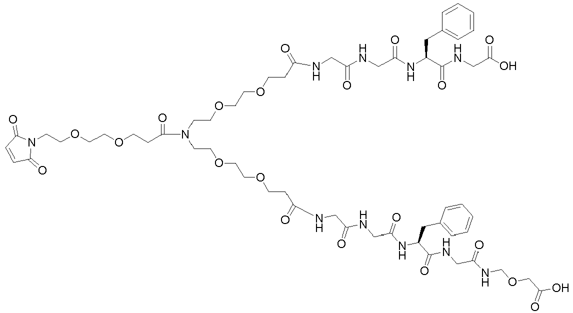 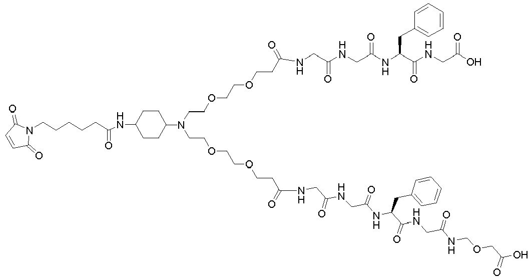 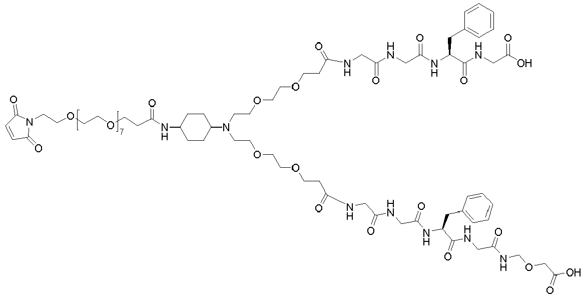 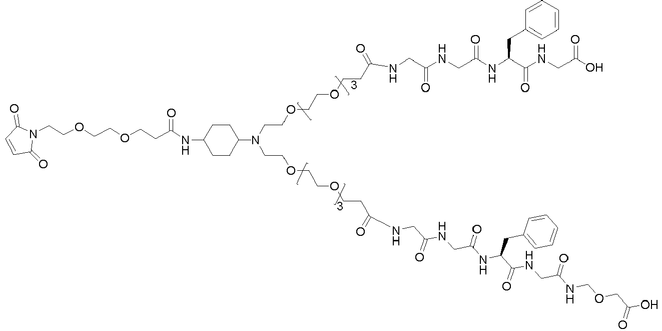 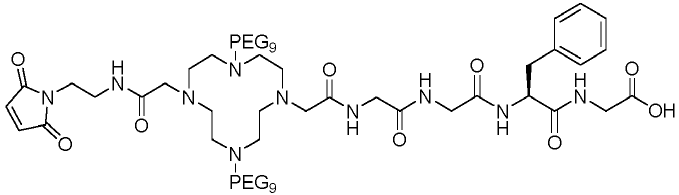 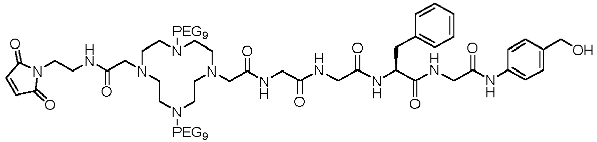 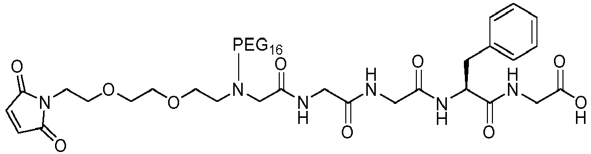 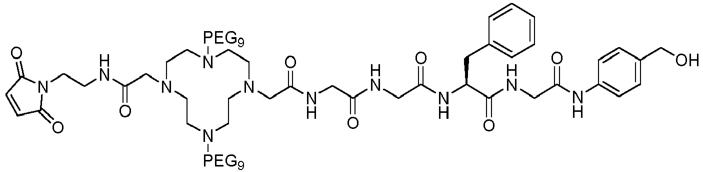 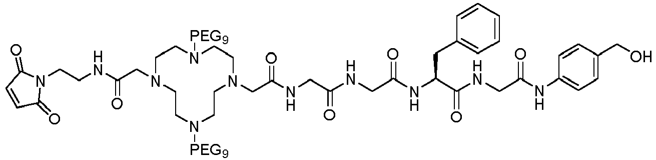 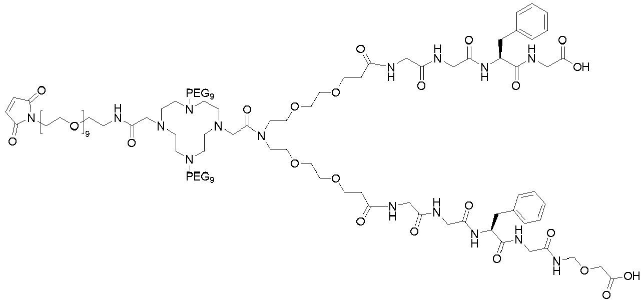 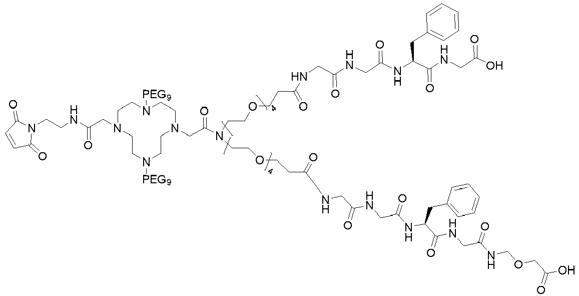 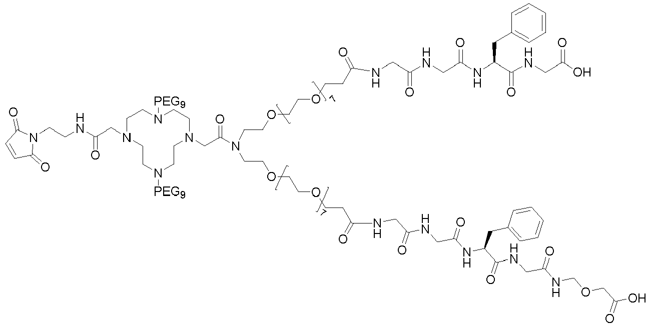 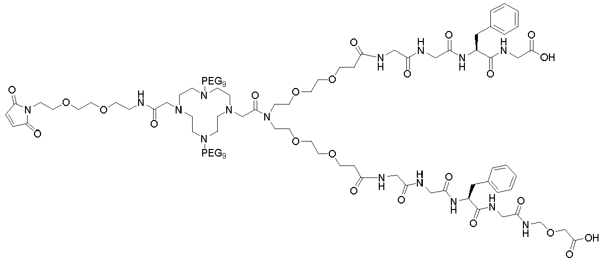 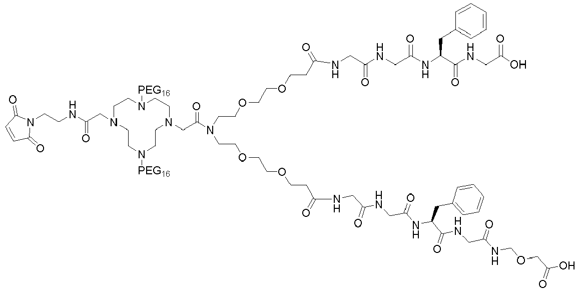 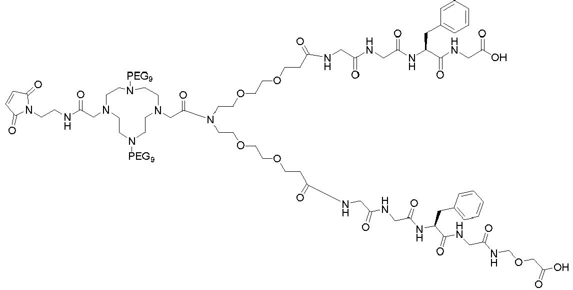 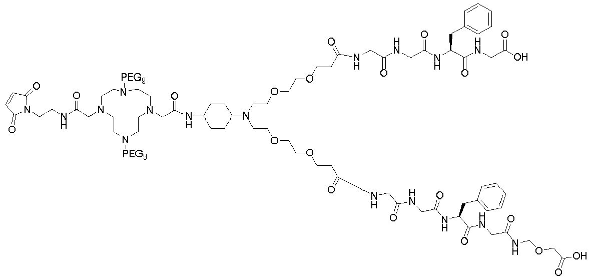 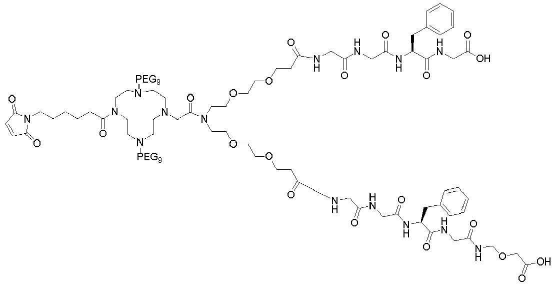 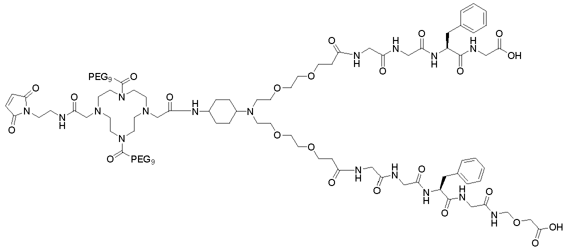 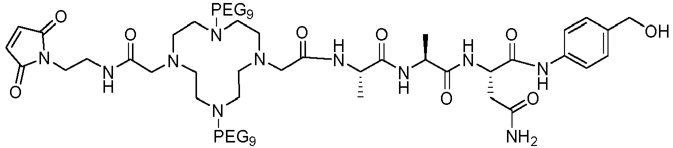 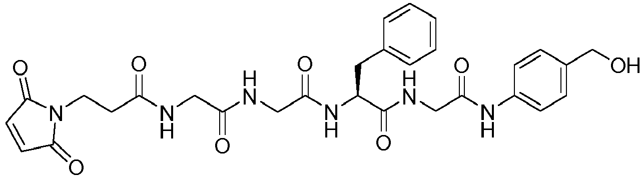 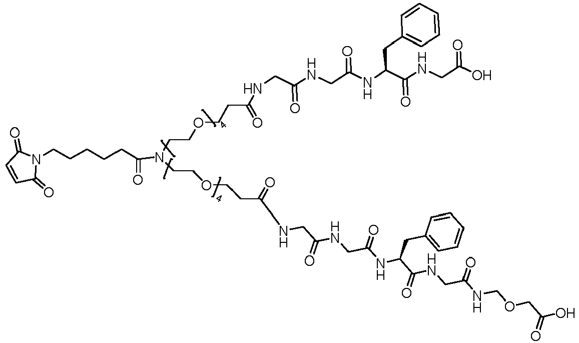 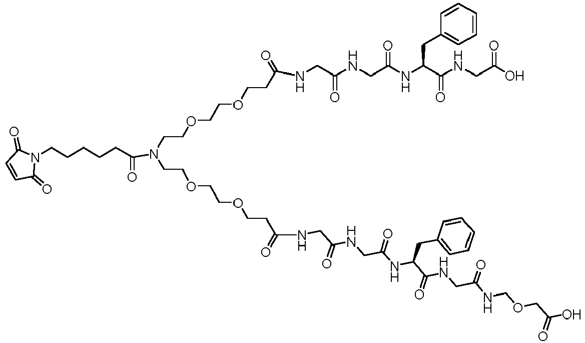 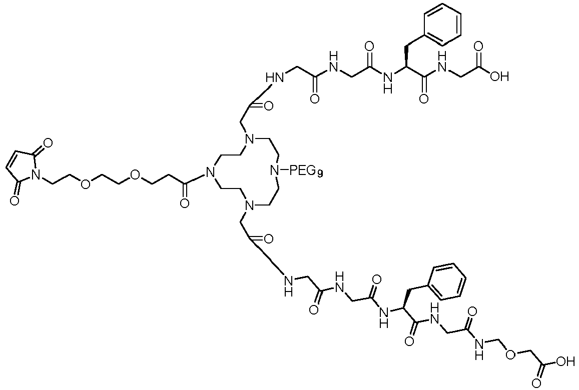 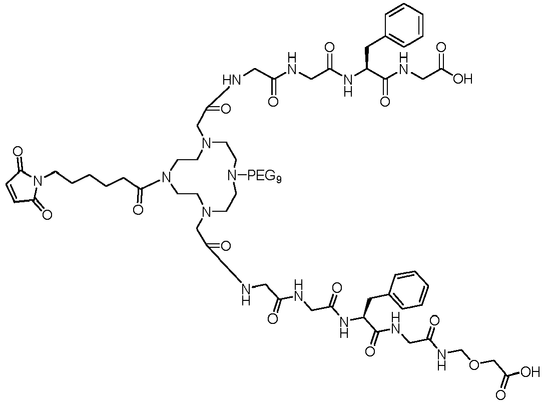 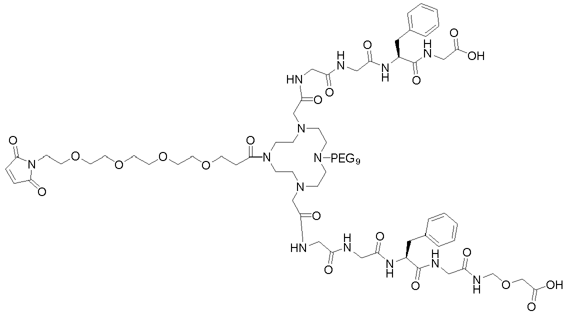 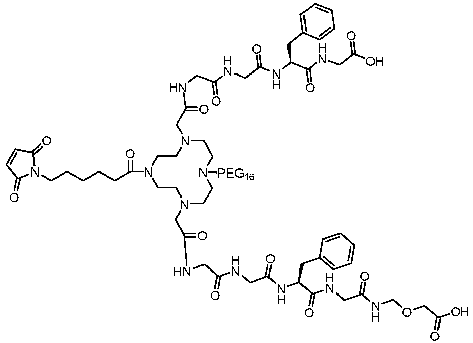 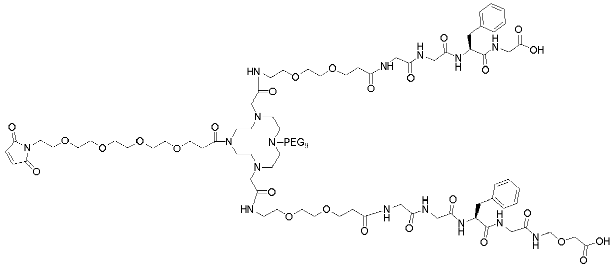 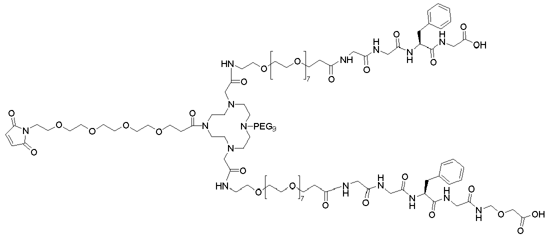 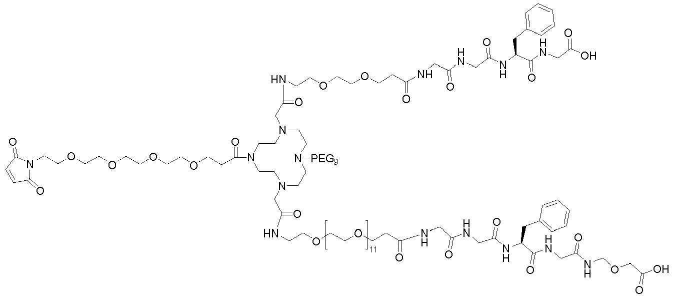 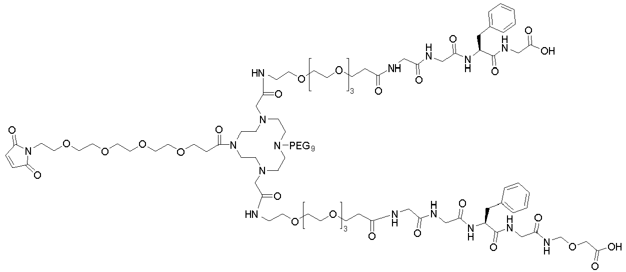 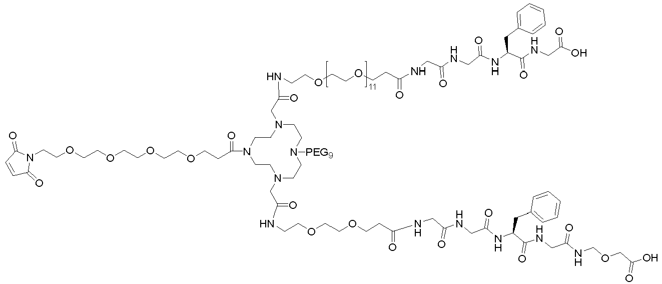 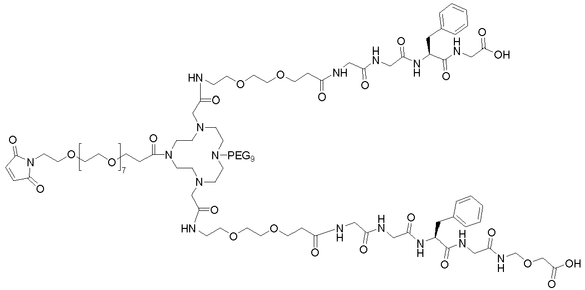 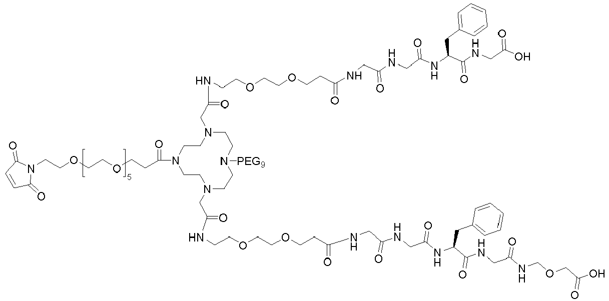 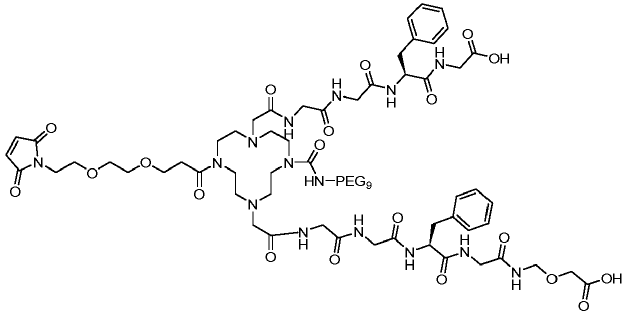 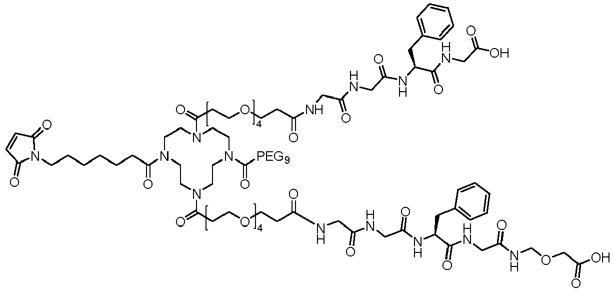 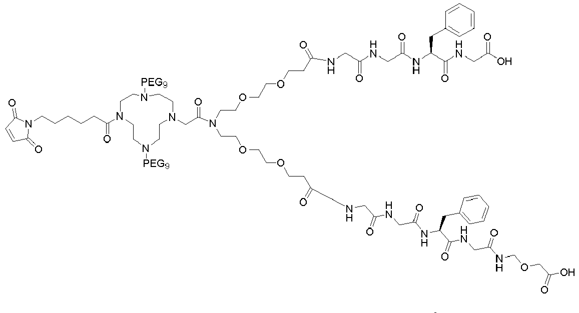 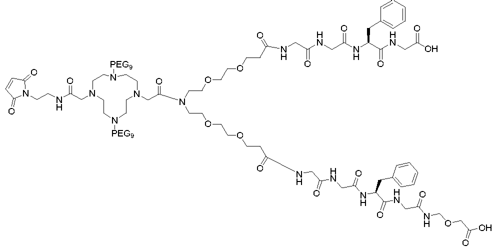 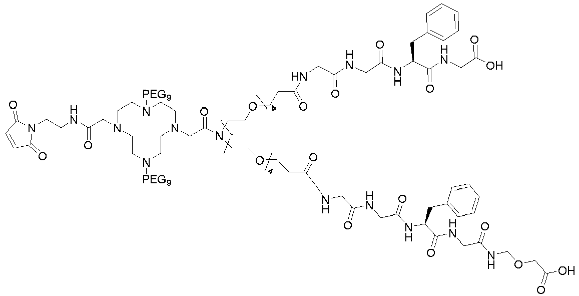 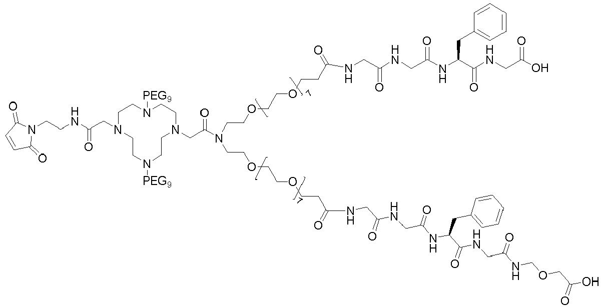 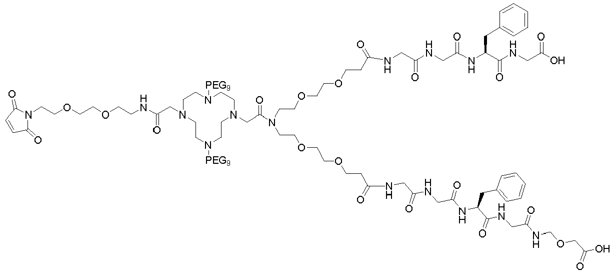 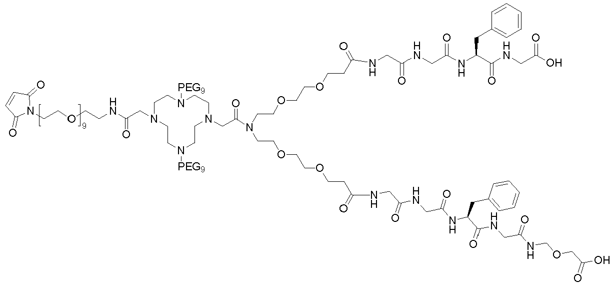 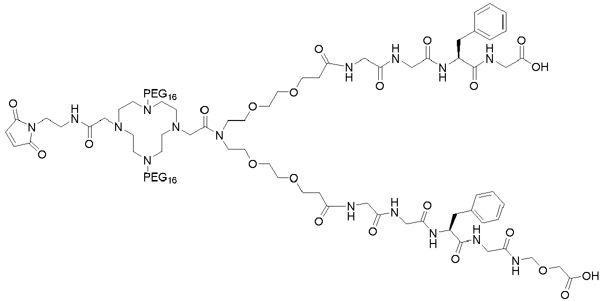 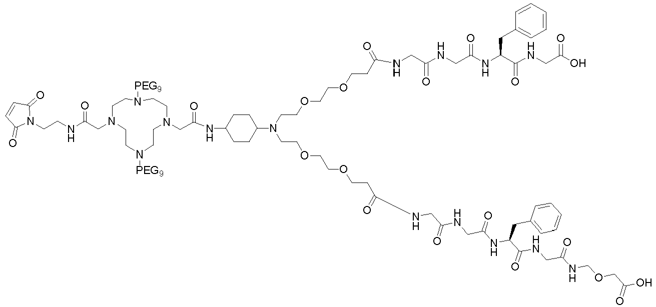 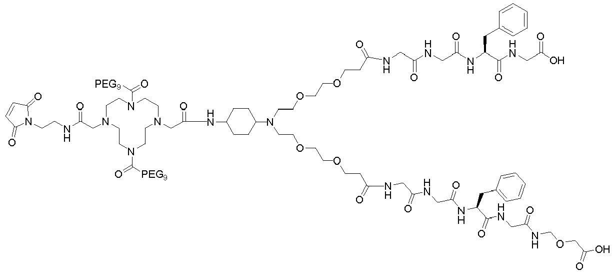 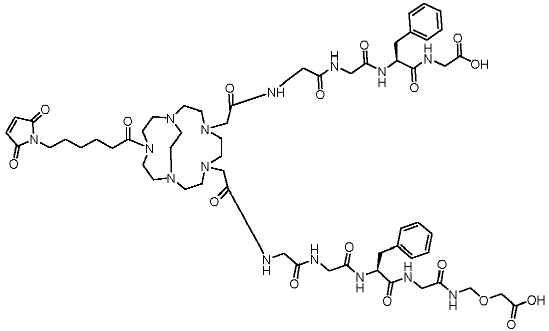 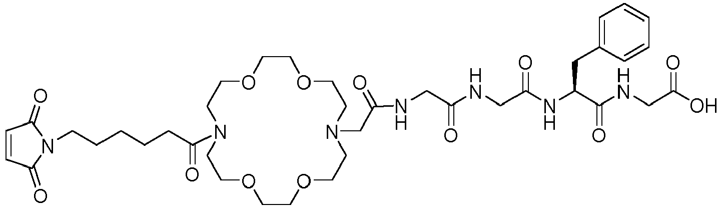 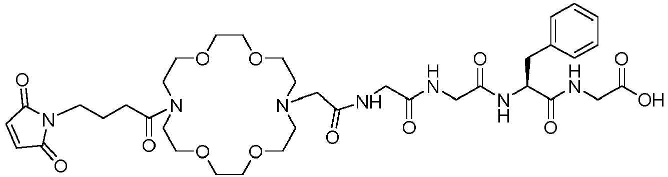 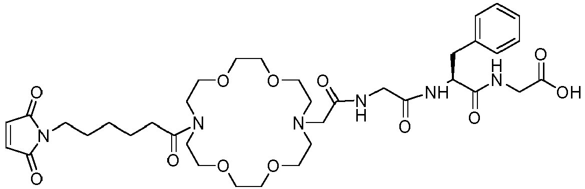 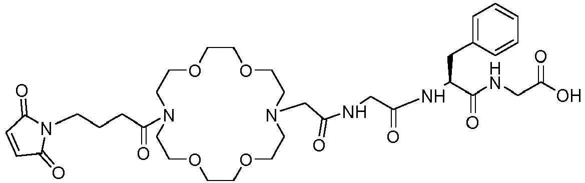 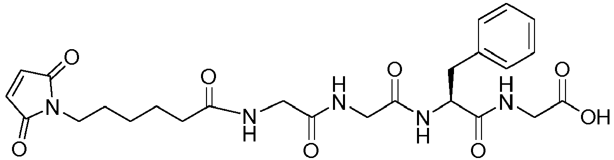 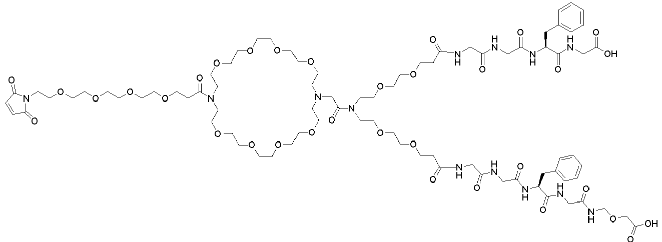 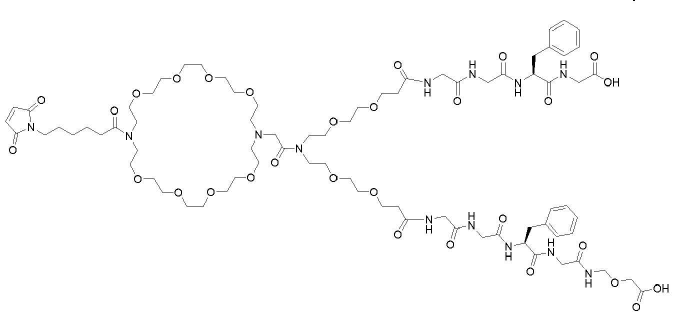 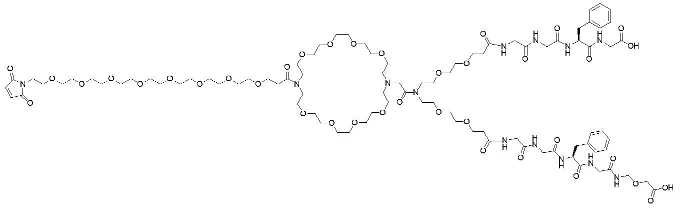 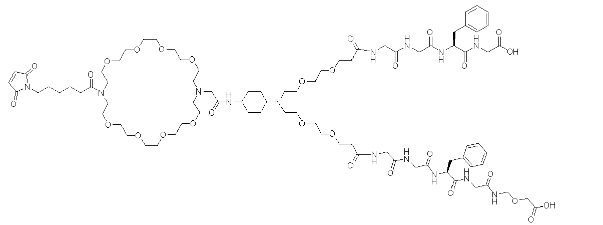 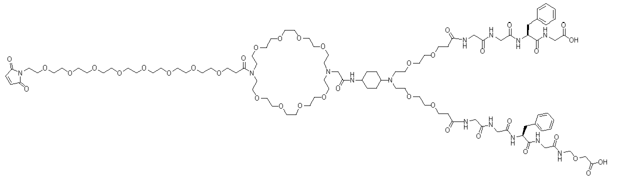 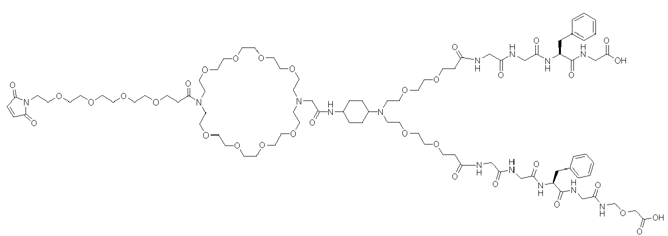 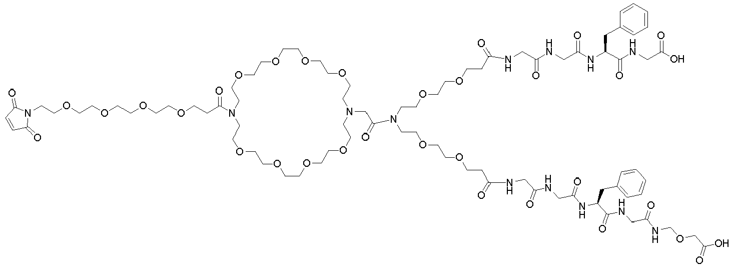 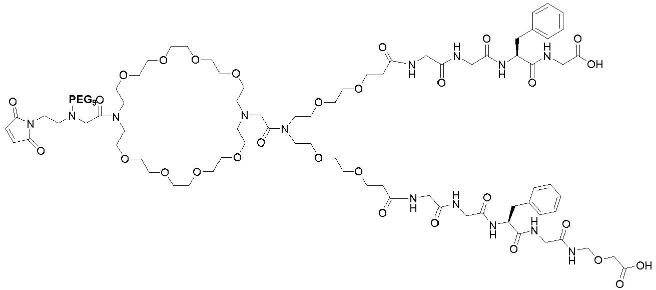 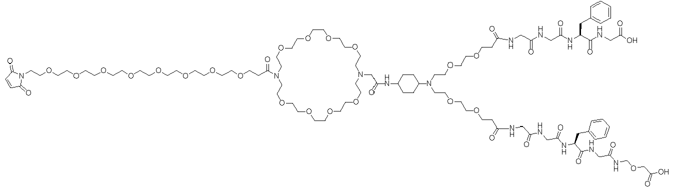 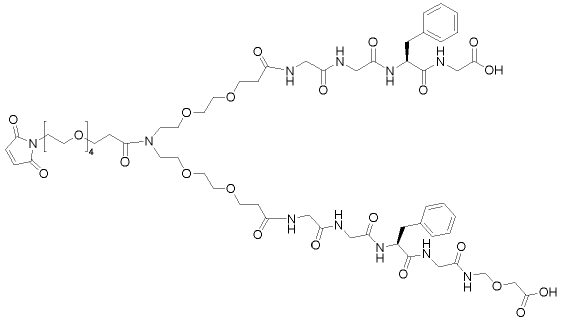 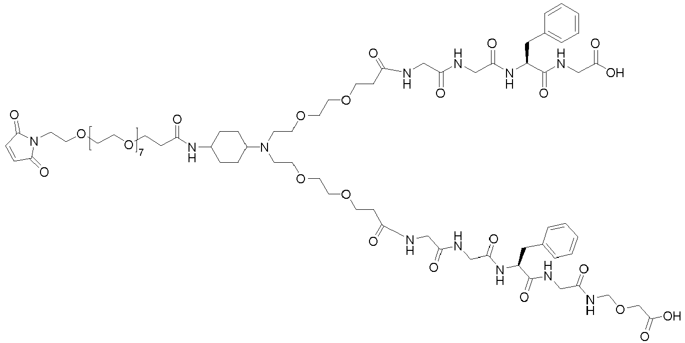 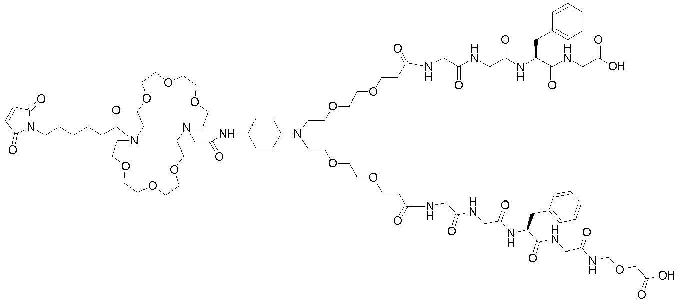 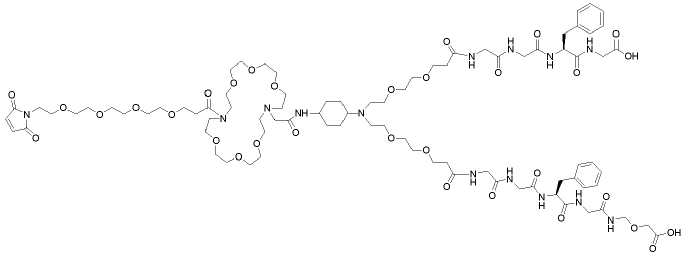 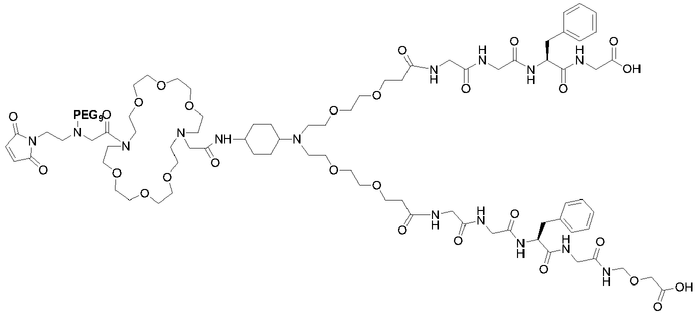 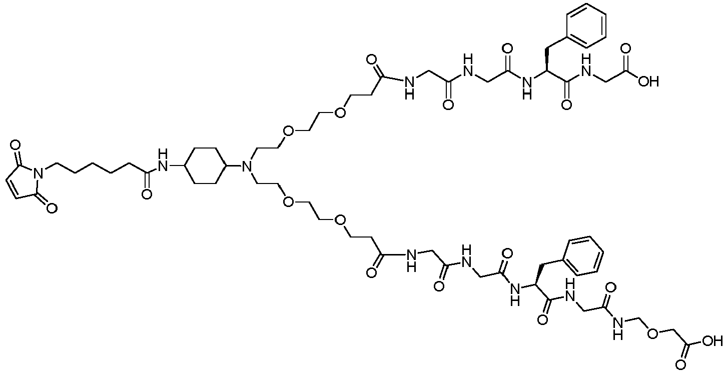 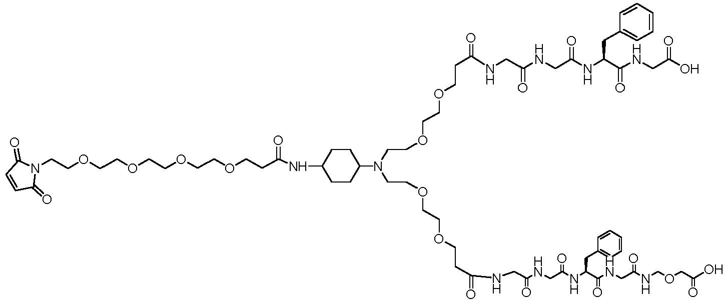 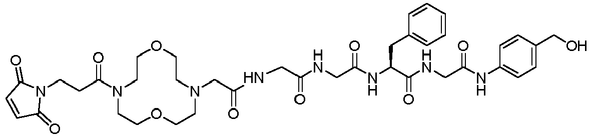 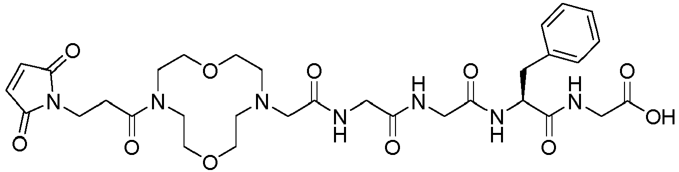 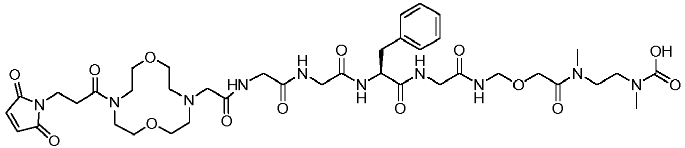 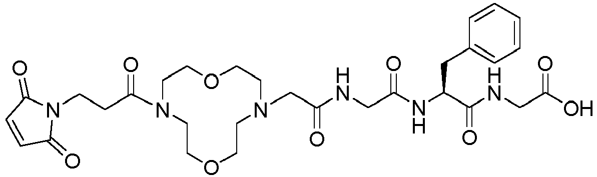 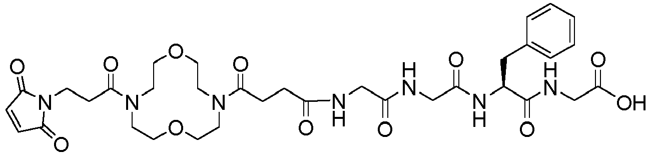 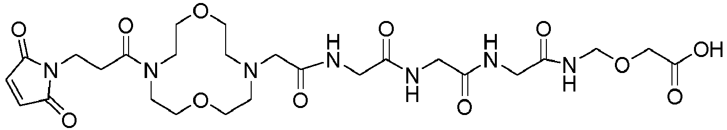 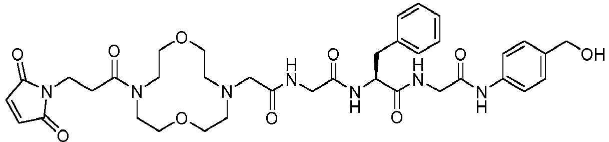 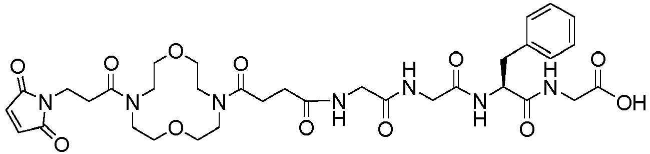 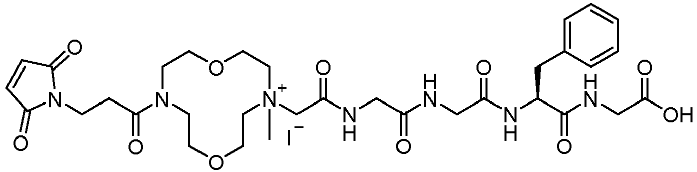 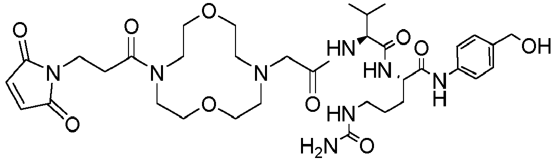 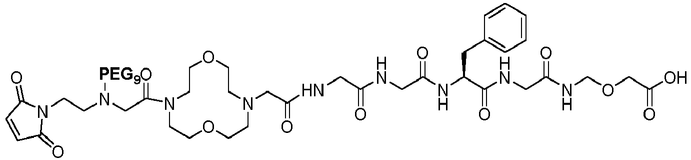 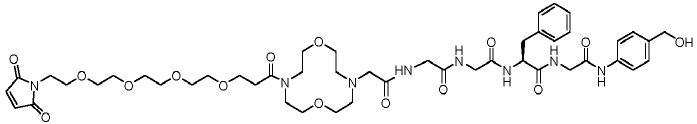 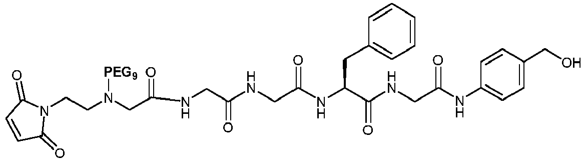 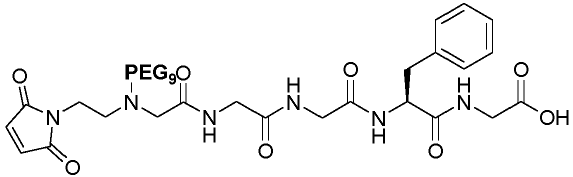 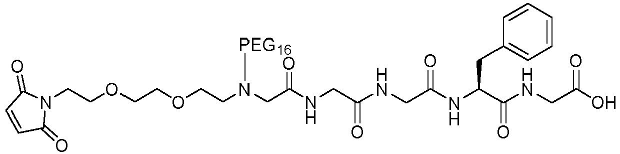 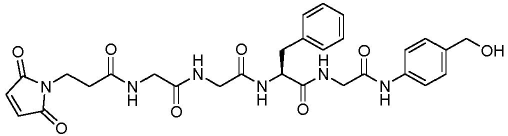 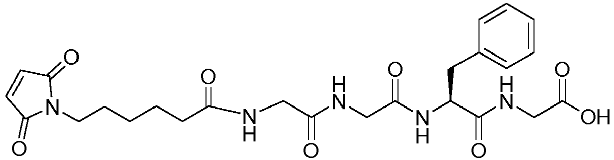 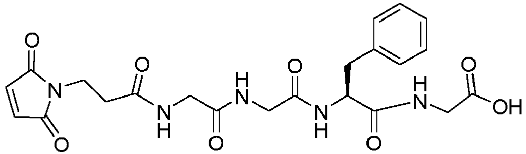
- PEG₉ represents 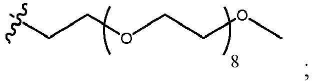 and -PEG₁₆ represents

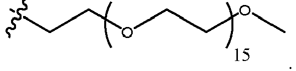

The present invention further provides a drug formulation for preventing and/or treating a tumor. The drug formulation is a formulation prepared from the above-mentioned targeted linker-drug conjugate or stereoisomer, optical isomer, salt, or deuterated compound thereof as an active ingredient, and pharmaceutically acceptable accessories.

The present invention further provides a use of the above-mentioned targeted linker-drug conjugate or stereoisomer, optical isomer, salt, or deuterated compound thereof in preparation of a drug formulation for preventing and/or treating a tumor.

Further, the tumor is selected from lung cancer, urethral cancer, large intestine cancer, prostate adenocarcinoma, ovarian cancer, pancreatic cancer, breast cancer, bladder cancer, stomach cancer, gastrointestinal stromal tumor, cervical cancer, esophageal cancer, squamous cell cancer, abdominal membrane cancer, liver cancer, colon cancer, rectal cancer, colorectal cancer, uterine cancer, salivary gland cancer, kidney cancer, vulva cancer, thyroid cancer, penis cancer, leukemia, malignant lymphoma, plasma cell tumor, myeloma, or sarcoma.

The present invention further provides a method for preparing the above-mentioned targeted linker-drug conjugate or stereoisomer, optical isomer, salt, or deuterated compound thereof. The method includes the following steps:
(1) conjugating a drug with a linker to obtain a drug link assembly unit; and
(2) conjugating the drug link assembly unit with a targeted linker to obtain the targeted linker-drug conjugate; or the method includes the following steps:
(1') conjugating a targeted linker with a linker to obtain a targeted linker-linker unit; and
(2') conjugating the targeted linker-linker unit with a drug to obtain the targeted linker-drug conjugate.

The drug link assembly unit of the present invention can be connected to a targeted linker, which is a substance capable of targeted binding to a lesion site; and the drug link assembly unit consists of a linker and a drug.

The targeted linker and 1 to 20 drug link assembly units can covalently connect to form a targeted linker-drug conjugate. When the targeted linker is an antibody, the targeted linker-drug conjugate is an antibody-drug conjugate (ADC).

In the present invention, the "DAR value" represents an average number of drug link assembly units conjugated to one targeted linker in the targeted linker-drug conjugate, and is equivalent to an average value of q values. The DAR value may not be an integer.

Definitions of terms used in the present invention: Unless otherwise specified, an initial definition provided for a group or term herein applies to the group or term throughout the entire specification. For terms that are not specifically defined herein, their meanings should be given to those skilled in the art according to the disclosure and context.

The minimum and maximum values of carbon atom content in hydrocarbon groups are represented by prefixes, for example, the prefix C_{a-b} alkyl represents any alkyl containing "a" to "b" carbon atoms. For example, C₁₋₁₀ alkyl refers to straight or branched alkyl containing 1-10 carbon atoms.

"Aryl" refers to an all-carbon monocyclic or fused polycyclic (i.e., rings sharing adjacent carbon atom pairs) group having a conjugated π electron system, such as phenyl and naphthyl. An aryl ring may be fused to other cyclic groups (including saturated and unsaturated rings), but cannot contain heteroatoms such as nitrogen, oxygen, or sulfur, and a point connected to a parent must be on a carbon atom of the ring having the conjugated π electron system. The aryl may be substituted or unsubstituted.

"Heteroaryl" refers to a heteroaromatic group containing one to multiple heteroatoms. The heteroatoms referred to here include oxygen, sulfur, and nitrogen, such as furyl, thienyl, pyridyl, pyrazolyl, pyrrolyl, N-alkylpyrrolyl, pyrimidyl, pyrazinyl, imidazolyl, and tetrazolyl. A heteroaryl ring may be fused to an aryl, heterocyclic, or cycloalkyl ring, where the ring connected to the parent structure is the heteroaryl ring. The heteroaryl may be optionally substituted or unsubstituted.

"Deuterated compound" refers to a compound obtained by replacing one or more hydrogen atoms in a compound with deuterium.

"Targeted linker-drug conjugate" refers to a conjugate formed by conjugating a drug with a targeted linker that has an effect of targeted binding to a lesion site. For example, "antibody-drug conjugate (ADC)" is a type of targeted linker-drug conjugate of the present invention, and its targeted linker is an antibody or antibody fragment.

"Drug link assembly unit" is a product obtained by connecting the linker in the aforementioned targeted linker-drug conjugate to a drug, is an intermediate for the preparation of the targeted linker-drug conjugate, and can be conjugated with the targeted linker to obtain the targeted linker-drug conjugate.

"Drug" or code name "D", "D₁", or "D₂" refers to a compound having desired bioactivity (such as therapeutic effect). For example, the drug may be a cytotoxic compound used for cancer treatment, or a protein or polypeptide having bioactivity. The drug of the present invention includes, but is not limited to, existing compounds having therapeutic effects, including camptothecin derivatives, such as SN-38, Dxd, and Dx-8951, and compounds that act on microtubule proteins, such as Eribulin, MMAE, MMAF, and maytansine.

"Targeted linker" refers to a substance having an effect of targeted binding to a lesion site, such as an antibody, antibody fragment, protein, small molecular polypeptide, glycopeptide, mimetic peptide, small molecular compound, or nucleic acid oligonucleotide adapter. The targeted linker can bind to a target (such as an antigen) on the lesion site, so that the targeted linker-drug conjugate is capable of targeted binding to the lesion site.

"Antibody" refers to a protein that is produced by the body due to the stimulation of an antigen and has a protective effect. When the targeted linker is an antibody or antibody fragment, the obtained targeted linker-drug conjugate is an antibody-drug conjugate. The antibody or antibody fragment of the present invention can bind to the antigen of the lesion site, so that the antibody-drug conjugate has an effect of targeted binding to the lesion site. The antibody can be any protein or protein molecule that can bind or chelate, or react with a portion of a cell population to be treated or biologically modified.

The examples provided by the present invention use Her2 as an antibody to prepare targeted linker-drug conjugates.

In the present invention, the antibody constituting the antibody-drug conjugate preferably maintains its antigen binding capability in its original wild state. Therefore, the antibody in the present invention can specifically bind to the antigen. The involved antigen includes, but is not limited to, tumor associated antigens (TAA), cell surface receptor proteins and other cell surface molecules, cell survival regulatory factors, cell proliferation regulatory factors, molecules related to tissue growth or differentiation, lymphokines, cytokines, molecules involved in cell cycle regulation, molecules involved in angiogenesis, and factors related to angiogenesis. Tumor associated factors may also be cluster differentiation factors (such as CD proteins). The antigen bound to the antibody in the present invention may be one or a subset of the aforementioned classes, while other subsets include other molecules/antigens with special properties.

The antibody applied to the antibody-drug conjugate includes, but is not limited to, antibodies targeting cell surface receptors and tumor associated antigens. Such tumor associated antigens are well-known in the art and can be prepared by commonly known antibody preparation methods and information in the art. These target substances can be specifically expressed on the surface of one or more cancer cells, but are seldom or not expressed on the surface of one or more non-cancer cells. Generally, compared to the surface of non-cancer cells, such as tumor associated polypeptides are excessively expressed on the surface of cancer cells. Confirming such tumor associated factors can greatly improve the targeted specificity of antibody-based cancer treatment.

The tumor associated antigens include, but are not limited to, the tumor associated antigens listed below. Corresponding tumor associated antigens targeted by antibodies include all amino acid sequence variants and homologies, which have at least 70%, 80%, 85%, 90%, or 95% homology to the confirmed sequences in the references, or have the same biological properties and characteristics as the tumor associated antigen sequences in the references.

The tumor associated antigens include: BMPR1B (Genbank accession number: NM-001203), E16 (Genbank accession number: NM-003486), STEAP1 (Genbank accession number: NM-012449), 0772P (Genbank accession number: AF361486), MPF (Genbank accession number: NM-005823), Napi3b (Genbank accession number: NM-006424), Sema 5b (Genbank accession number: AB040878), PSCA hlg (Genbank accession number: AY358628), ETBR (Genbank accession number: AY275463), MSG783 (Genbank accession number: NM-017763), STEAP2 (Genbank accession number: AF455138), TrpM4 (Genbank accession number: NM-017636), CRIPTO (Genbank accession number: NP-003203 or NM-003212), CD21 (Genbank accession number: M26004), CD79B (Genbank accession number: NM-000626), FcRH2 (Genbank accession number: NM-030764), HER2 (Genbank accession number: M11730), NCA (Genbank accession number: M18728), MDP (Genbank accession number: BC017023), IL20Rα (Genbank accession number: AF184971), Brevican (Genbank accession number: AF229053), EphB2R (Genbank accession number: NM-004442), GEDA (Genbank accession number: AY260763), BAFF-R (Genbank accession number: AF1164546), CD22 (Genbank accession number: AK026467), CD79a (Genbank accession number: NP-001774.1), CXCR5 (Genbank accession number: NP-001701.1), HLA-DOB (Genbank accession number: NP-002111.1), P2X5 (Genbank accession number: NP-002552.2), CD72 (Genbank accession number: NP-001773.1), LY64 (Genbank accession number: NP-005573.1), FcRH1 (Genbank accession number: NP-443170.1), IRTA2 (Genbank accession number: NP-112571.1), TENB2 (Genbank accession number: AF179274), CEA, B7H3, Her3, 5T4, Claudin 18.2, FGFR2b, FRα, TROP2, B7H4, Nectin4, CD30, c-MET, EGFR, carcinoembryonic antigen associated cell adhesion molecules 5 (CEACAM5), ROR1, CD20, hepatocyte growth factor receptors (HGFR), ROR2, IL3RA, mesothelin receptors, CD74, and PD-L1.

The antibodies used in the present invention may be antibodies corresponding to the aforementioned antigens.

"Linker", also known as "Jie Tou", is a substance used for connecting a therapeutic compound to a targeted linker of targeted binding to a lesion site. "Linker fragment" refers to a partial structure having a connecting function in the linker.

According to the intracellular drug release mechanism, "linkers" may be divided into two classes: unbreakable linkers and breakable linkers.

For a targeted linker-drug conjugate containing an unbreakable linker (such as an antibody-drug conjugate), its drug release mechanism may be as follows: after the conjugate binds to an antigen and is endocytosed by cells, the antibody is enzymatically hydrolyzed in the lysosome to release drugs, and the linker and antibody amino acid residues together form active small molecules.

A targeted linker-drug conjugate containing a breakable linker (such as an antibody-drug conjugate) can break and release drugs (such as small molecule drugs) within target cells. The unbreakable linkers may be divided into two main classes: chemically unstable linkers and enzymatic unstable linkers. The chemically unstable linkers may selectively break due to different properties of plasma and cytoplasm, including pH value, glutathione concentration, etc. The enzymatic unstable linkers, such as peptide linkers, can be effectively cleaved by proteases in the lysosome, such as tissue proteases and fibrinolytic enzymes. The peptide linkers are considered to be very stable in plasma.

The linkers are core components of targeted linker-drug conjugates (such as antibody-drug conjugates), and can greatly affect the pharmacokinetics, therapeutic index, and therapeutic effects of the targeted linker-drug conjugates (such as antibody-drug conjugates).

The key of the present invention is improvement on the linker, specifically by adding the structure represented by formula A to the linker, so that the linker can significantly improve the safety, stability, effectiveness, and controllability of the prepared targeted linker-drug conjugate (such as antibody-drug conjugate) in addition to traditional linker functions, and can effectively promote clinical use of targeted linker-drug conjugate (such as antibody-drug conjugate) drugs.

The targeted linker-drug conjugate provided in the examples of the present invention includes a targeted linker and 1 to 20 covalently connected drug link assembly units, where the drug link assembly unit may be connected to thiol generated by an inter-chain disulfide bond in a reducing antibody and/or each linkage assembly unit may be connected to thiol from cysteine residues.

For ease of connection, the drug link assembly unit is usually constructed before connecting to the targeted linker. However, the order of construction may be changed. For example, the assembly unit having a protective group is first connected to the targeted linker, then the protective group is removed, and other drug units are added.

The targeted linker-drug conjugate provided by the present invention targets special cells, such as tumor cells, and binds to cell surface specific proteins (such as antigens). The conjugate can enter a cell in an endocytic form, and the drug is released into the cell in an active form to exert its effect, or is released outside the cell and penetrates into the cell to exert its effect.

Beneficial effects of the present invention are as follows:
By introducing the structure represented by formula A into the link unit, the polymer content and naked antibody percentage of the targeted linker-drug conjugate formed by connecting the connecting unit to various different drug units and targeted linkers can be reduced, the DAR value of the targeted linker-drug conjugate can be maintained within an appropriate range, and the plasma stability and storage stability of the targeted linker-drug conjugate and the therapeutic effect can be improved.

The drug link assembly unit provided by the present invention, which contains the above-mentioned connecting unit structure, can also play a role in reducing the polymer content and naked antibody percentage of the targeted linker-drug conjugate and maintaining the DAR value of the targeted linker-drug conjugate within an appropriate range.

The finally prepared targeted linker-drug conjugate (such as antibody-drug conjugate) has low polymer content and naked antibody percentage and an appropriate DAR value, exhibits excellent plasma stability, storage stability and anti-tumor effect, and has good clinical application prospects.

Apparently, based on the above content of the present invention, various other forms of modifications, replacements, or changes can be made in accordance with common technical knowledge and customary means in the art, without departing from the basic technical idea of the present invention.

The above content of the present invention will be described in detail below through specific implementations in a form of examples. However, this should not be understood as the scope of the above subject matter of the present invention is limited to the following examples. All technologies implemented based on the above content of the present invention fall into the scope of the present invention.

### DESCRIPTION OF THE DRAWINGS

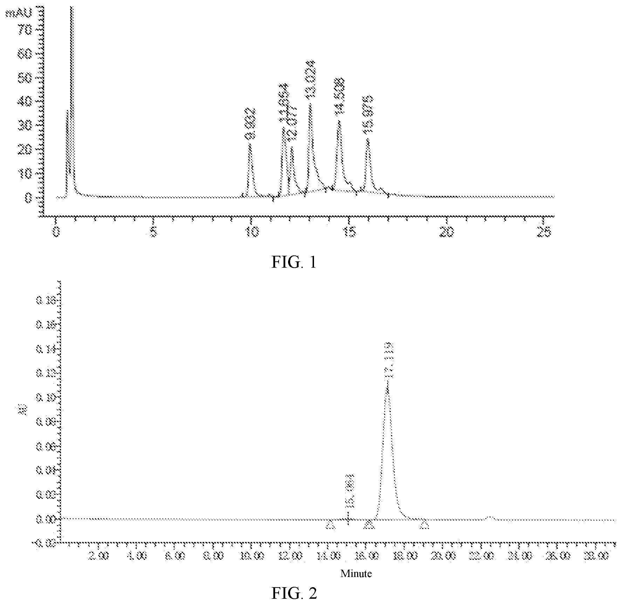

FIG. 1: DAR value test results of SMP-93566a (RP-HPLC).
FIG. 2: polymer test results of SMP-93566a (SE-HPLC).

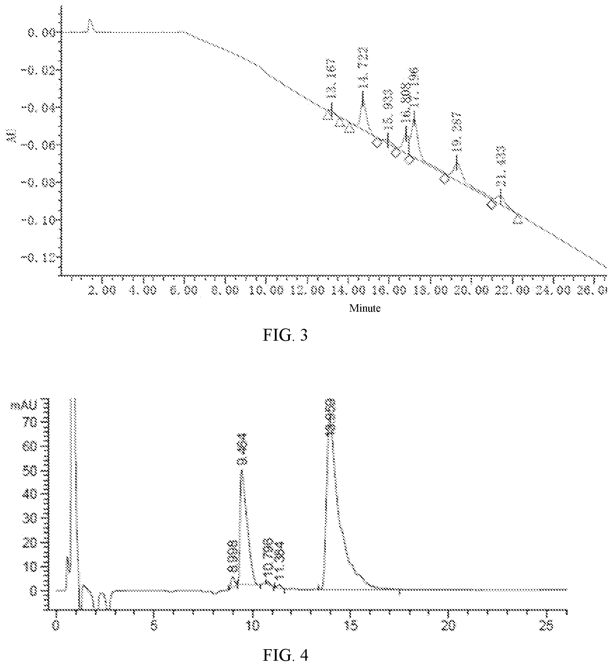

FIG. 3: naked antibody test results of SMP-93566a (HIC).
FIG. 4: DAR value test results of SMP-88480a (RP-HPLC).

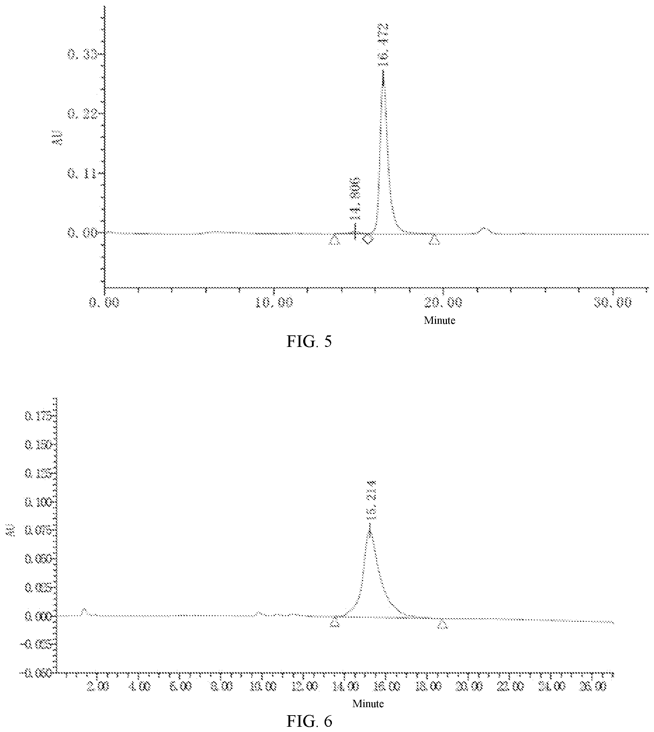

FIG. 5: polymer test results of SMP-88480a (SE-HPLC).
FIG. 6: naked antibody test results of SMP-88480a (HIC).
FIG. 7: plasma stability results of single toxin ADC in human, monkey, and mouse plasma.
FIG. 8: plasma stability results of Dxd in dual toxin ADC in human, monkey, and mouse plasma.
FIG. 9: plasma stability results of Eribulin in dual toxin ADC in human, monkey, and mouse plasma.
FIG. 10: in vivo tumor inhibitory effect of single toxin ADC on ovarian cancer, gastric cancer, and breast cancer.
FIG. 11: in vivo tumor inhibitory effect of dual toxin ADC on gastric cancer.

The raw materials and equipment used in the present invention are known products obtained by purchasing commercially available products.

The structure of the targeted linker-drug conjugate provided by the present invention can be synthesized by those skilled in the art through different methods and strategies according to conventional technical knowledge and means in the art, such as:
(1) A complete linker structure is first synthesized and then further conjugated with a targeted linker and a drug to obtain a targeted linker-drug conjugate;
Alternatively, (2) starting with a drug, the drug is gradually conjugated with a linker fragment to obtain a drug link assembly unit, which is then further conjugated with a targeted linker to obtain a targeted linker-drug conjugate;
Alternatively, (3) starting with a targeted linker, the targeted linker is gradually conjugated with a linker fragment to obtain a targeted linker-linker unit, which is then further conjugated with a drug to obtain a targeted linker-drug conjugate.

The present invention provides a specific method for preparing a targeted linker-drug conjugate in the following examples:

### Examples

### I. Preparation of targeted linker-drug conjugates

### Example 1: Preparation of intermediate A

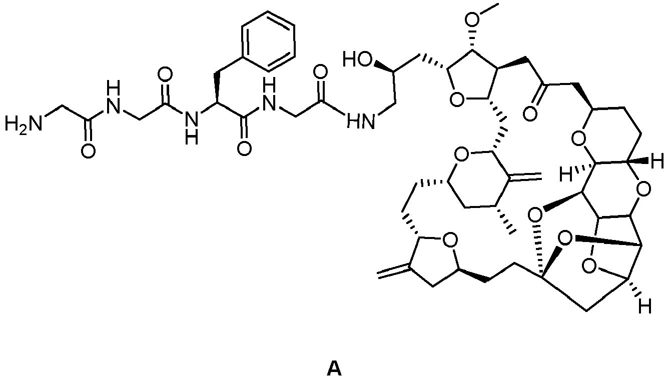

### Step 1: Preparation of compound A-2

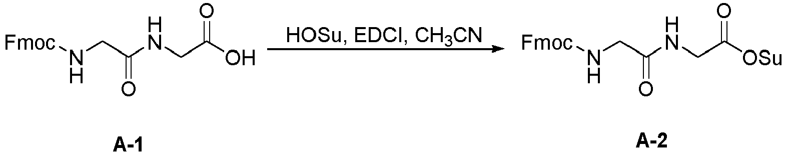

A-1 (20.0 g, 56.49 mmol) and acetonitrile (200 mL) were added to a 500 mL reaction flask and stirred at room temperature. After complete clarification, 1-(3-dimethylaminopropyl)-3-ethylcarbodiimide hydrochloride (11.0 g, 57.38 mmol) and N-hydroxysuccinimide (7.0 g, 60.82 mmol) were added, the stirring at room temperature continued for reaction for 12 hours, and then TLC showed complete reaction. The reaction system was filtered, and the obtained solid was dried in vacuum to obtain a compound A-2, weighing 22.0 g, with a yield of 86%.

### Step 2: Preparation of compound A-3

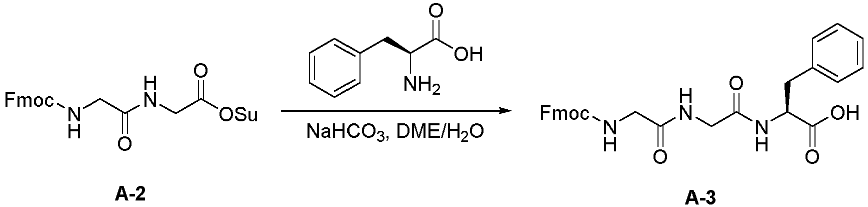

L-phenylalanine (8.0 g, 48.48 mmol), sodium bicarbonate (8.0 g, 95.24 mmol), and water (200 mL) were added to a 500 mL reaction flask and stirred at room temperature. After complete clarification, the compound A-2 (22.0 g, 48.78 mmol) was dissolved in ethylene glycol dimethyl ether (50 mL), and the solution was slowly added dropwise to the above reaction solution. After adding, the stirring at room temperature continued for reaction for 12 hours, and then TLC showed complete reaction. The ethylene glycol dimethyl ether was removed by concentration under reduced pressure, and the remaining reaction solution was added dropwise to a 0.5 M hydrochloric acid aqueous solution (500 mL) to precipitate a large amount of solid. The reaction system was filtered, and the obtained solid was dried in vacuum to obtain a compound A-3, weighing 15.0 g, with a yield of 61%.

¹HNMR (400 MHz, CDCl3) 12.51 (s, 1H), 9.04 (s, 1H), 8.31 (s, 1H), 7.95 (s, 1H), 7.90(d, J=8.0 Hz, 2H), 7.56(d, J=7.8 Hz, 2H), 7.38-7.28 (m, 4H), 7.19-7.14(m, 5H), 4.85(t, J=8.2 Hz, 1H), 4.71(d, J=8.2 Hz, 2H), 4.39(t, J=8.4 Hz, 1H), 4.10-3.83(m, 4H), 3.12(d, J=9.6 Hz, 1H), 2.85(d, J=9.6 Hz, 1H).

### Step 3: Preparation of compound A-4

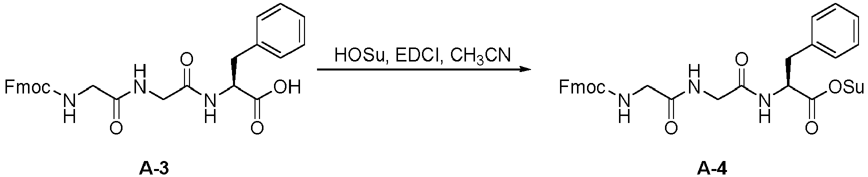

The A-3 (15.0 g, 29.94 mmol), acetonitrile (200 mL), 1-(3-dimethylaminopropyl)-3-ethylcarbodiimide hydrochloride (6 g, 31.30 mmol), and N-hydroxysuccinimide (4 g, 34.78 mmol) were sequentially added to a 500 mL reaction flask and stirred at room temperature for reaction for 12 hours. TLC showed complete reaction. The reaction system was filtered, and the obtained solid was dried in vacuum to obtain a compound A-4, weighing 13.0 g, with a yield of 73%.

### Step 4: Preparation of compound A-5

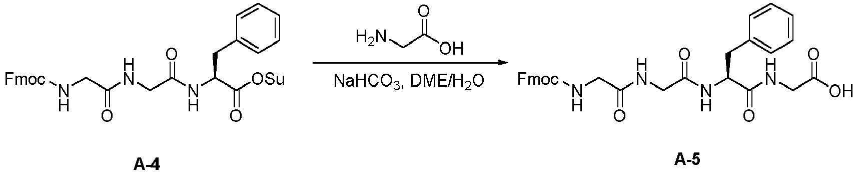

Glycine (3.0 g, 40.00 mmol), sodium bicarbonate (6.7 g, 79.76 mmol), and water (150 mL) were added to a 500 mL reaction flask and stirred at room temperature. After complete clarification, the compound A-4 (13.0 g, 21.74 mmol) was dissolved in ethylene glycol dimethyl ether (40 mL), and the solution was slowly added dropwise to the above reaction solution. After adding, the reaction continued at room temperature for 12 hours while stirring, and then TLC showed complete reaction. The organic solvent was removed by concentration under reduced pressure, and the remaining reaction solution was added dropwise to a 0.5 M hydrochloric acid aqueous solution (300 mL) to precipitate a large amount of solid. The reaction system was filtered, and the obtained solid was dried in vacuum to obtain a compound A-5, weighing 10.0 g with a yield of 83%.

¹HNMR (400 MHz, CDCl3) 13.01 (s, 1H), 9.01 (s, 1H), 8.27 (s, 1H), 7.98 (s, 1H), 7.89(d, J=8.0 Hz, 2H), 7.54(d, J=7.8 Hz, 2H), 7.34-7.23 (m, 4H), 7. 19-7. 14(m, 5H), 4.75(t, J=8.2 Hz, 1H), 4.61(d, J=8.2 Hz, 2H), 4.30(t, J=8.4 Hz, 1H), 4.04-3.83(m, 6H), 3.10(d, J=9.6 Hz, 1H), 2.75(d, J=9.6 Hz, 1H).

### Step 5: Preparation of compound A-6

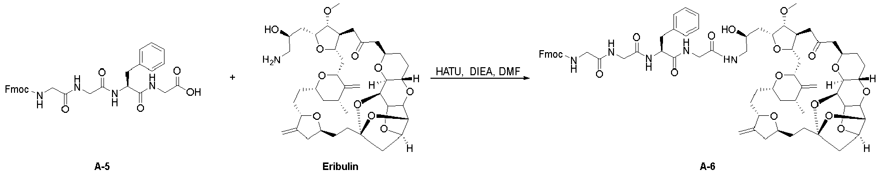

The A-5 (200.0 mg, 0.36 mmol), N,N,N',N'-tetramethyl-O-(7-azabenzotriazol-1-yl)hexafluorophosphourea (163.0 mg, 0.43 mmol), N,N-diisopropylethylamine (66.9 mg, 0.52 mmol), N,N-dimethylformamide (5 mL) were sequentially added to a 25 mL reaction flask and stirred at room temperature for reaction for 5 minutes, and then Eribulin (263.1 mg, 0.36 mmol) was added. The stirring continued at room temperature for ten minutes. TLC showed complete reaction. The N,N-dimethylformamide was removed by concentration under reduced pressure, and the residue was purified by thin layer chromatography (dichloromethane: methanol=10:1) to obtain a compound A-6, weighing 300.0 mg, with a yield of 66%.

### Step 6: Preparation of compound A

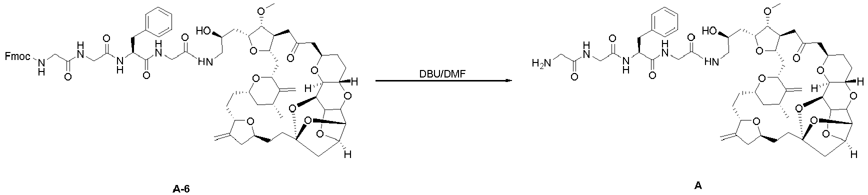

N,N-dimethylformamide (2 mL), the A-6 (20.0 mg, 0.016 mmol), and 1,8-diazabicyclo[5.4.0]undec-7-ene (5.0 mg, 0.032 mmol) were sequentially added to a 25 mL reaction flask and stirred at room temperature for reaction for 30 minutes. TLC showed complete reaction. The N,N-dimethylformamide was removed by concentration under reduced pressure, and the residue was purified by HPLC to obtain a compound A, weighing 10.0 mg, with a yield of 60%.

¹HNMR (400 MHz, DMSO-*d*₆) δ 8.48 (t, *J* = 5.5 Hz, 1H), 8.31 (dd, *J* = 10.1, 4.6 Hz, 2H), 7.98 (s, 3H), 7.74 (t, *J* = 5.6 Hz, 1H), 7.30 - 7.21 (m, 4H), 7.22 - 7.15 (m, 1H), 5.02 (d, *J* = 22.6 Hz, 2H), 4.79 (d, *J* = 29.3 Hz, 2H), 4.63 (dd, *J* = 5.7, 3.7 Hz, 1H), 4.59 - 4.50 (m, 2H), 4.24 (t, *J* = 13.5 Hz, 1H), 4.21 - 4.12 (m, 1H), 4.12 - 4.06 (m, 3H), 4.02 (s, 1H), 3.86 (dd, *J* = 16.8, 5.7 Hz, 1H), 3.68 (dd, *J* = 16.7, 5.5 Hz, 4H), 3.54 (dd, *J* = 19.0, 7.3 Hz, 12H), 3.11 (dd, *J* = 16.3, 11.0 Hz, 1H), 3.07 - 2.99 (m, 2H), 2.84 (d, *J* = 9.6 Hz, 1H), 2.80 - 2.65 (m, 3H), 2.62 - 2.54 (m, 1H), 2.24 (ddd, *J* = 48.1, 23.9, 9.6 Hz, 6H), 1.98 (dd, *J* = 29.5, 15.1 Hz, 6H), 1.73 - 1.57 (m, 5H), 1.55 - 1.40 (m, 2H), 1.37 - 1.25 (m, 3H), 1.22 - 1.13 (m, 1H), 1.03 (d, *J* = 6.4 Hz, 3H), 1.00 - 0.89 (m, 1H).

### Example 2: Preparation of intermediate B

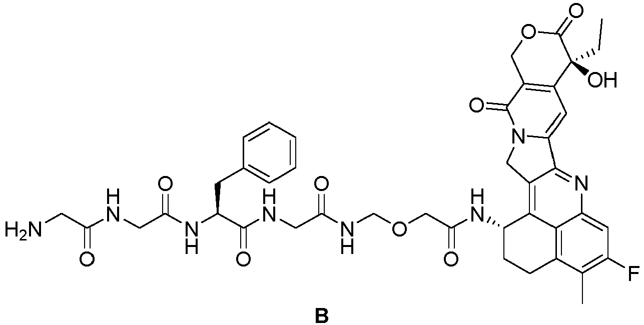

### Step 1: Preparation of compound B-2

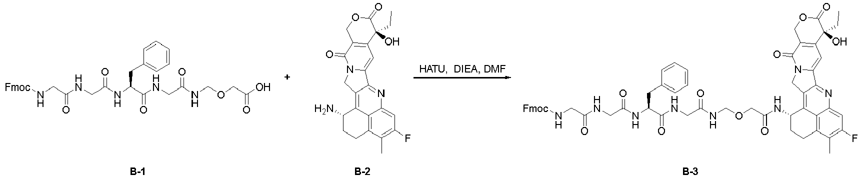

N,N-dimethylformamide (5 mL), B-1 (200.0 mg, 0.31 mmol), N,N,N',N'-tetramethyl-O-(7-azabenzotriazol-1-yl)hexafluorophosphourea (118.0 mg, 0.31 mmol), N,N-diisopropylethylamine (50.0 mg, 0.39 mmol) were sequentially added to a 25 mL reaction flask and stirred at room temperature for 5 minutes, and then exatecan (B-2, 112.0 mg, 0.26 mmol) was added. The solution was stirred for ten minutes. TLC showed complete reaction. The N,N-dimethylformamide was removed by concentration under reduced pressure, and the residue was purified by thin layer chromatography (dichloromethane: methanol=10:1) to obtain a compound B-3, weighing 140.0 mg, with a yield of 50%.

MS (ESI) m/z: 1063 [M+H]⁺.

### Step 2: Preparation of compound B

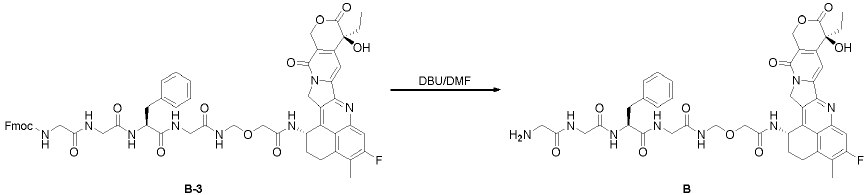

N,N-dimethylformamide (3 mL), the B-3 (140.0 mg, 0.13 mmol), and 1,8-diazabicyclo[5.4.0]undec-7-ene (30.0 mg, 0.20 mmol) were added to a 25 mL reaction flask and stirred at room temperature for 30 minutes. TLC showed complete reaction. Most of the N,N-dimethylformamide was concentrated under reduced pressure, and the residue was purified by HPLC to obtain a compound B, weighing 70.0 mg, with a yield of 64%.

MS (ESI) m/z: 841[M+H]⁺.

¹H NMR (400 MHz, DMSO-*d*₆) δ 8.70 (t, *J* = 6.6 Hz, 1H), 8.58 - 8.45 (m, 2H), 8.39 (t, *J* = 5.8 Hz, 1H), 8.33 (d, *J* = 8.2 Hz, 1H), 7.97 (d, *J* = 12.0 Hz, 3H), 7.77 (d, *J* = 10.9 Hz, 1H), 7.31 (d, *J* = 3.5 Hz, 1H), 7.28 - 7.20 (m, 4H), 7.20 - 7.12 (m, 1H), 6.54 (s, 1H), 5.59 (dd, *J* = 14.3, 5.8 Hz, 1H), 5.42 (s, 2H), 5.18 (s, 2H), 4.64 (d, *J* = 6.6 Hz, 2H), 4.53 (td, *J* = 9.4, 4.5 Hz, 1H), 4.02 (s, 2H), 3.89 - 3.79 (m, 1H), 3.79 - 3.65 (m, 3H), 3.48 (d, *J* = 5.9 Hz, 1H), 3.27 - 3.07 (m, 3H), 3.03 (dd, *J* = 13.7, 4.3 Hz, 1H), 2.74 (dd, *J* = 14.3, 9.2 Hz, 1H), 2.38 (s, 2H), 2.18 (dd, *J* = 14.7, 9.5 Hz, 2H), 1.86 (td, *J* = 14.0, 7.1 Hz, 2H), 0.87 (t, *J* = 7.3 Hz, 3H).

### Example 3: Preparation of intermediate C

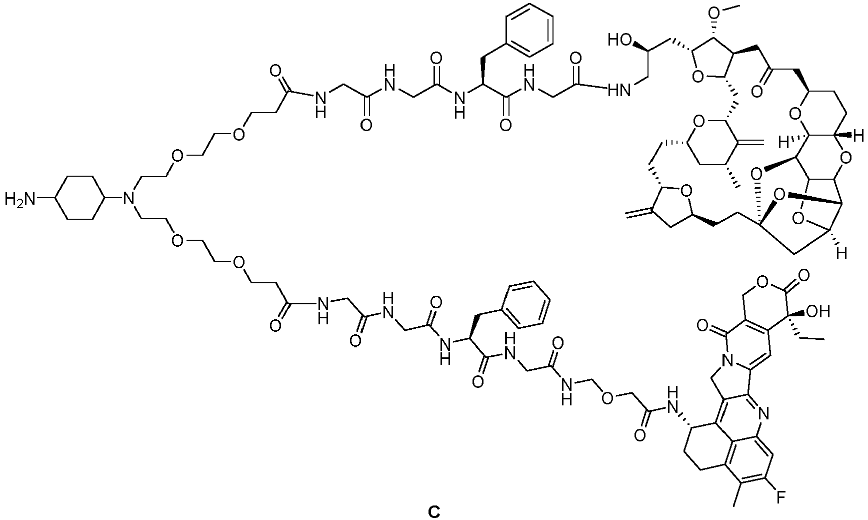

### Step 1: Preparation of compound C-2

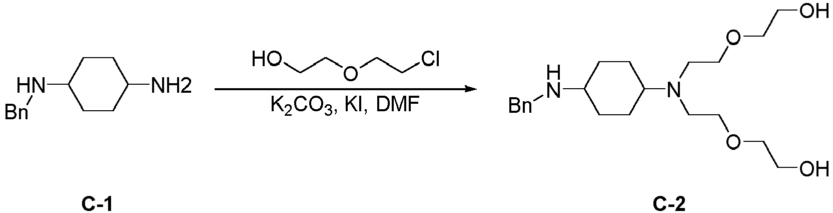

A compound C-1 (1.0 g, 4.90 mmol) and a solvent N,N-dimethylformamide (15 mL) were sequentially added to a 25 mL reaction flask and stirred at room temperature. After the reaction solution was clarified, potassium carbonate (2.4 g, 17.4 mmol), potassium iodide (1.6 g, 9.64 mmol), and 2-chloroethoxyethanol (1.6 g, 12.90 mmol) were sequentially added and stirred at 110°C for 12 hours. TLC showed complete reaction. The reaction system was filtered, the filtrate was concentrated under reduced pressure, and the residue was purified by HPLC to obtain C-2, weighing 1.5 g, with a yield of 80%.

¹HNMR (400 MHz, CDCl3) δ 7.32-7.29 (m, 5H), 4.51 (s, 2H), 4.43 (s, 1H), 3.72 - 3.69 (m, 4H), 3.55 - 3.50 (m, 8H), 2.65 - 2.50 (m, 6H), 1.59 - 1.34 (m, 8H).

### Step 2: Preparation of compound C-3

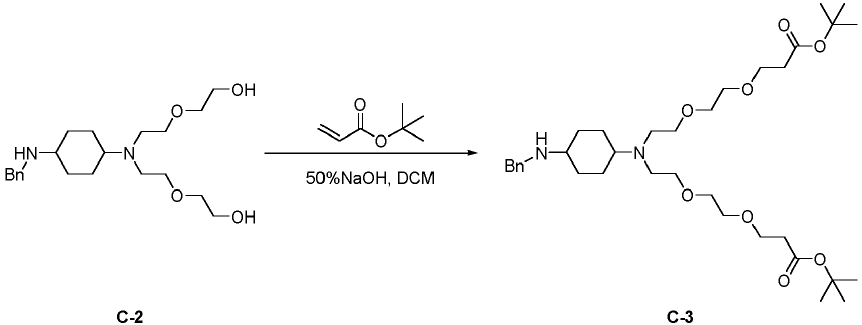

Dichloromethane (10 mL), the C-2 (1.5 g, 3.94 mmol), and tert-butyl acrylate (2.1 g, 16.40 mmol) were sequentially added to a 25 mL reaction flask in an ice bath and stirred for 30 minutes. 50% sodium hydroxide aqueous solution (2.6 g) was slowly added dropwise, and then the reaction continued for 10 minutes. The ice bath was removed, and the reaction continued at room temperature for 3 hours. LC-MS test showed complete reaction. The pH value was adjusted to 5-6 with 0.5M dilute hydrochloric acid, and the solution was extracted with dichloromethane (10 mL*3). The organic phase was washed with a saturated salt solution, dried with anhydrous sodium sulfate, filtered, and concentrated under reduced pressure to obtain a target product C-3, weighing 1.7 g, with a yield of 67%, for direct use in the next step.

### Step 3: Preparation of compound C-4

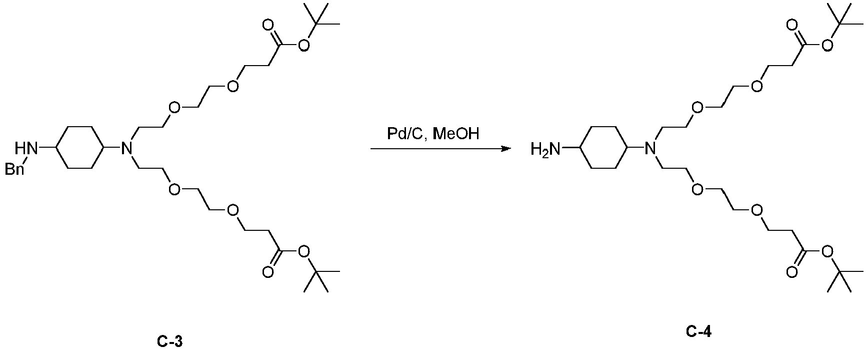

The C-3 (1.0 g, 1.57 mmol), methanol (10 mL), and palladium carbon (0.1 g) were added to a 25 mL reaction flask, followed by three times of hydrogen substitution. The reaction system was stirred at room temperature for 2 hours in a hydrogen environment. LC-MS showed complete reaction, the palladium carbon was filtered out, and the filtrate was concentrated under reduced pressure to obtain a compound C-4, weighing 800.0 mg, with a yield of 93%, for direct use in the next step.

¹HNMR (400 MHz, CDCl3) δ 3.72 - 3.69 (m, 4H), 3.55 - 3.50 (m, 12H), 2.65 - 2.42 (m, 10H), 1.59 - 1.34 (m, 8H), 1.49 (s, 18H).

### Step 4: Preparation of compound C-5

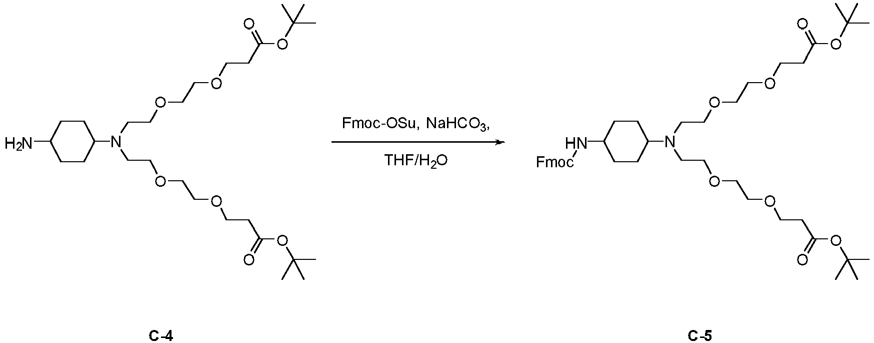

The C-4 (800.0 mg, 1.46 mmol), THF (10 mL), and water (2.5 mL) were sequentially added to a 25 mL reaction flask and stirred at room temperature. After the reaction solution was clarified, sodium bicarbonate (184.0 mg, 2.19 mmol) and N-(9-fluorenylmethoxycarbonyloxy)succinimide (529.0 mg, 1.57 mmol) were sequentially added and stirred at room temperature for 2 hours, LC-MS showed complete reaction, the reaction solution was concentrated under reduced pressure, and the residue was purified by column chromatography (dichloromethane: methanol=20: 1) to obtain a compound C-5, weighing 1.0 g, with a yield of 89%.

¹HNMR (400 MHz, CDCl3) δ 7.90 (d, J=8.9 Hz, 2H), 7.55 (d, J=9.2 Hz, 2H), 7.38-7.28 (m, 4H), 4.70 (d, J=11.2 Hz, 2H), 4.46 (t, J=11.2 Hz, 1H), 3.71 - 3.67 (m, 4H), 3.53 - 3.48 (m, 12H), 2.55 - 2.41 (m, 10H), 1.51 - 1.24 (m, 8H), 1.41 (s, 18H).

### Step 5: Preparation of compound C-6

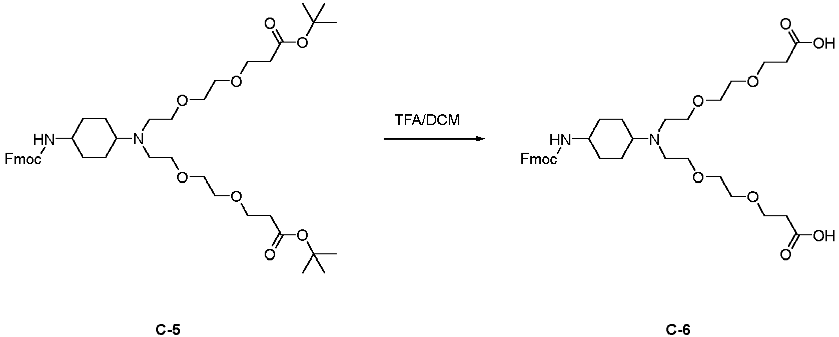

The intermediate C-5 (1.0 g, 1.30 mmol), dichloromethane (5 mL), and trifluoroacetic acid (5 mL) were sequentially added to a 25 mL reaction flask and reacted at room temperature for 2 hours. LC-MS showed complete reaction. The reaction solution was concentrated under reduced pressure, and the residue was purified by HPLC to obtain a compound C-6, weighing 600.0 mg, with a yield of 70%.

MS (ESI) m/z: 657[M+H]⁺.

### Step 6: Preparation of compound C-7

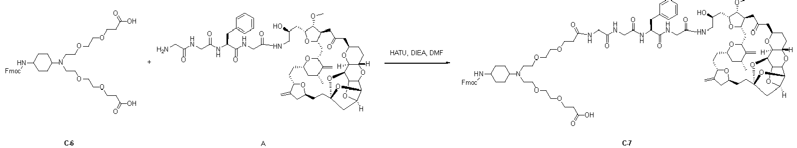

N,N-dimethylformamide (3 mL), A (50.0 mg, 0.048 mmol), the C-6 (65.0 mg, 0.10 mmol), N,N,N',N'-tetramethyl-O-(7-azabenzotriazol-1-yl)hexafluorophosphourea (19.0 mg, 0.050 mmol), and N,N-diisopropylethylamine (13.0 mg, 0.10 mmol) were sequentially added to a 25 mL reaction flask and stirred at room temperature for 30 minutes. TLC showed complete reaction. The N,N-dimethylformamide was removed by concentration under reduced pressure, and the residue was purified by HPLC to obtain a compound C-7, weighing 50.0 mg, with a yield of 62%. MS (ESI) m/z: 844[M/2+H]⁺.

### Step 7: Preparation of compound C-8

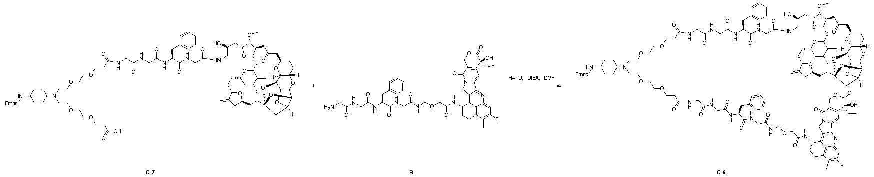

N,N-dimethylformamide (3 mL), the C-7 (30.0 mg, 0.018 mmol), B (16.0 mg, 0.020 mmol), N,N,N',N'-tetramethyl-O-(7-azabenzotriazol-1-yl)hexafluorophosphourea (7.0 mg, 0.018 mmol), and N,N-diisopropylethylamine (3.0 mg, 0.023 mmol) were sequentially added to a 25 mL reaction flask. A reaction occurred at room temperature for 30 minutes while stirring. TLC showed complete reaction. The N,N-dimethylformamide was removed by concentration under reduced pressure, and the residue was purified by HPLC to obtain a compound C-8, weighing 20.0 mg, with a yield of 44%. MS (ESI) m/z: 1255[M/2+H]⁺, 837[M/3+H]⁺.

### Step 8: Preparation of compound C

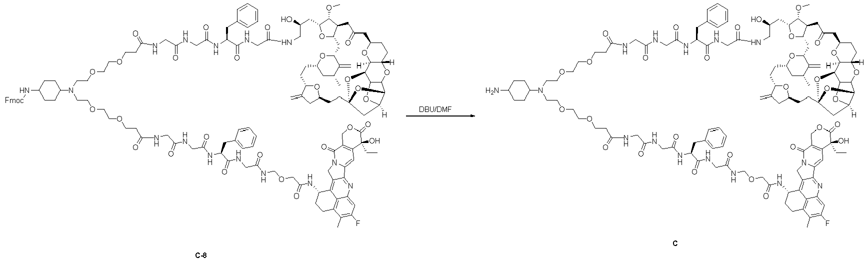

N,N-dimethylformamide (2 mL), the C-8 (20.0 mg, 0.0080 mmol), and 1,8-diazabicyclo[5.4.0]undec-7-ene (2.45 mg, 0.016 mmol) were sequentially added to a 25 mL reaction flask and stirred at room temperature for 30 minutes. TLC showed complete reaction. The N,N-dimethylformamide was removed by concentration under reduced pressure, and the residue was purified by HPLC to obtain a compound C, weighing 15.0 mg, with a yield of 82%.

MS (ESI) m/z: 1144[M/2+H]⁺, 763[M/3+H]⁺.

### Example 4: Preparation of intermediate D

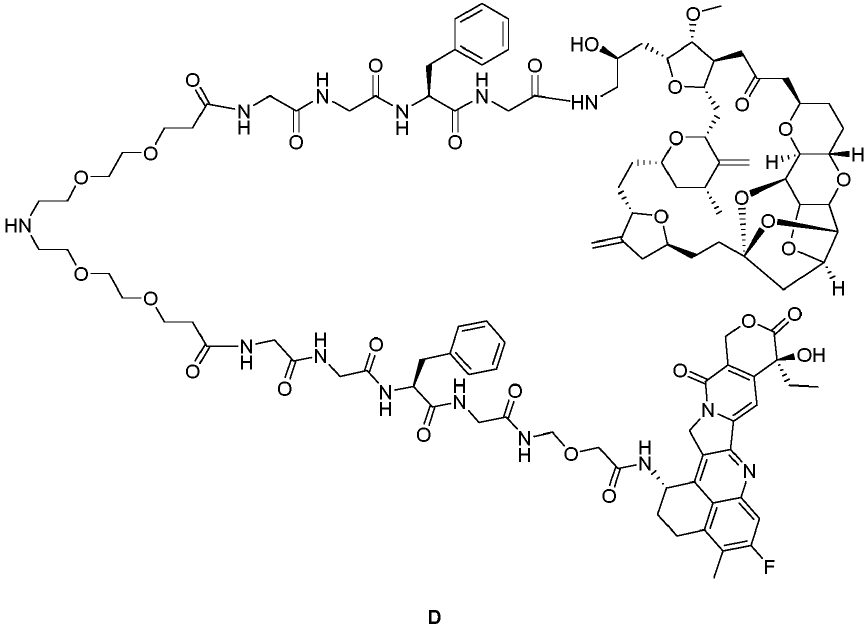

The synthesis method of intermediate D referred to the synthesis of compound C.

### Example 5: Preparation of intermediate E

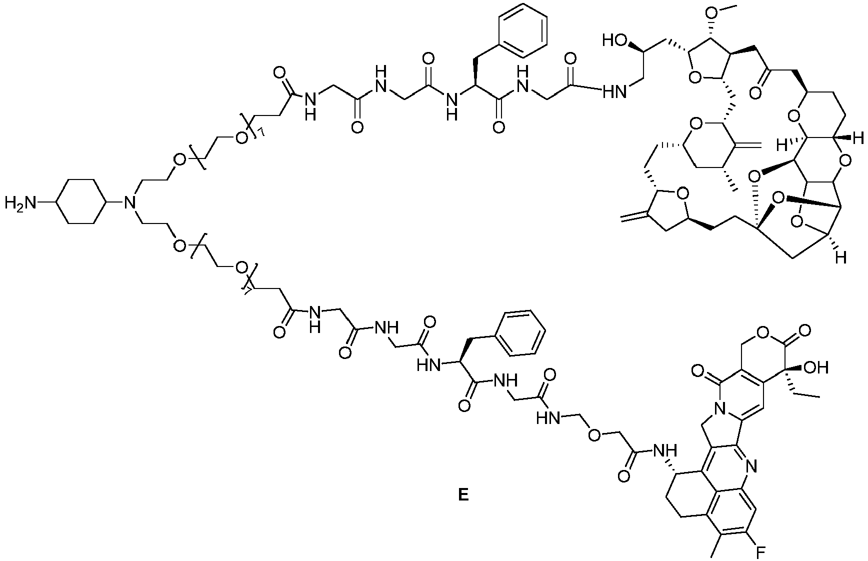

### Step 1: Preparation of compound E-2

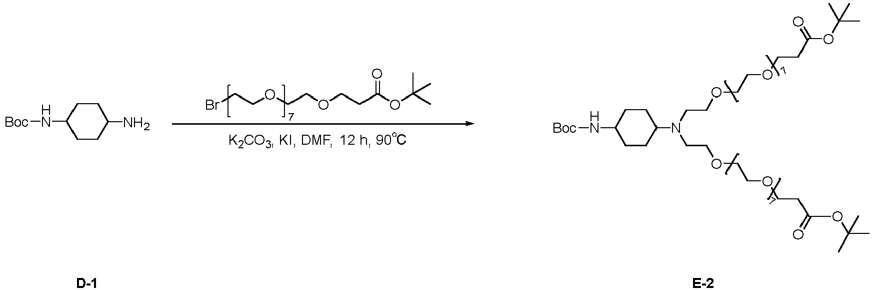

A compound D-1 (1.2 g, 5.61 mmol) and a solvent N,N-dimethylformamide (15 mL) were sequentially added to a 25 mL reaction flask and stirred at room temperature. After the reaction solution was clarified, potassium carbonate (2.6 g, 18.84 mmol), potassium iodide (1.9 g, 11.40 mmol), and bromo-heptaethylene glycol-tert-butyl propionate (6.3 g, 11.22 mmol) were sequentially added. A reaction occurred at 90°C for 12 hours while stirring. TLC showed complete reaction. The reaction system was filtered, the filtrate was concentrated under reduced pressure, and the residue was purified by HPLC to obtain an intermediate E-2, weighing 2.0 g, with a yield of 30%. MS (ESI) m/z: 1175 [M+H]⁺.

### Step 2: Preparation of compound E-3

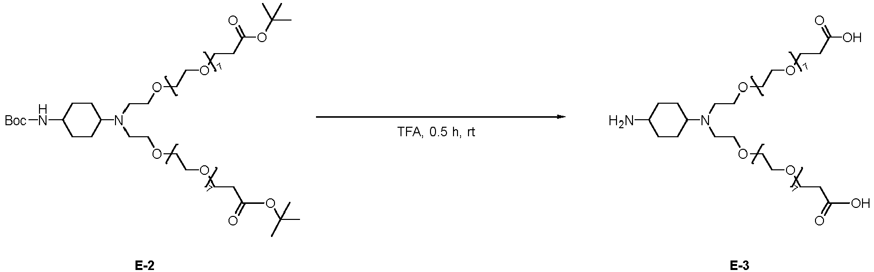

The compound E-2 (2.0 g, 1.70 mmol) and trifluoroacetic acid (10mL) were added to a 25 mL reaction flask and stirred at room temperature for 0.5 hours. LC-MS showed complete reaction. The trifluoroacetic acid was removed by concentration under reduced pressure to obtain an intermediate E-3, weighing 1.0 g, with a yield of 61%, for direct use in the next step.
MS (ESI) m/z: 964[M+H]⁺

### Step 3: Preparation of compound E-4

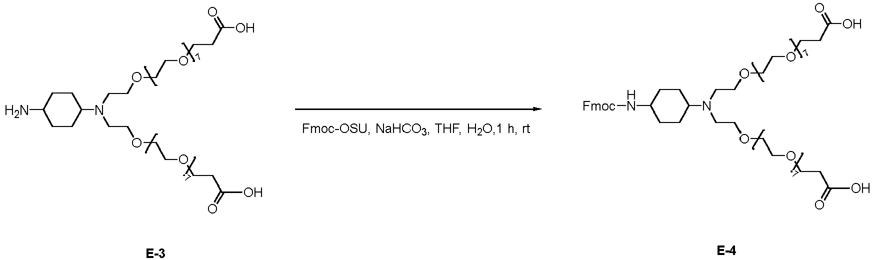

The intermediate E-3 (600.0 mg, 0.62 mmol), tetrahydrofuran (20 mL), and water (2.5 mL) were sequentially added to a 25 mL reaction flask and stirred at room temperature. After the reaction solution was clarified, sodium bicarbonate (105.0 mg, 1.25 mmol) and N-(9-fluorenylmethoxycarbonyloxy)succinimide (252.0 mg, 0.75 mmol) are sequentially added. The solution was stirred at room temperature for 1 hour. LC-MS showed complete reaction. The solution was concentrated under reduced pressure, and the residue was purified by HPLC to obtain a compound E-4, weighing 550.0 mg, with a yield of 75%.
MS (ESI) m/z: 1185 [M+H]⁺

### Step 4: Preparation of compound E-5

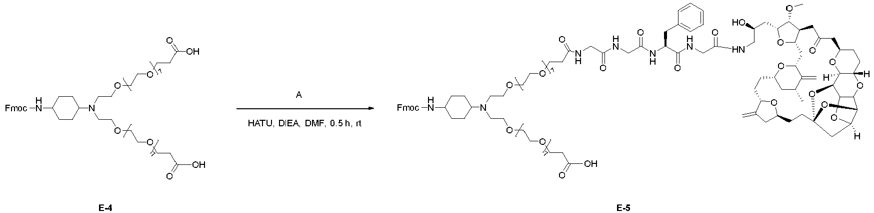

N,N-dimethylformamide (4 mL), A (50.0 mg, 0.048 mmol), the E-4 (118.0 mg, 0.10 mmol), N, N,N,N',N'-tetramethyl-O-(7-azabenzotriazol-1-yl)hexafluorophosphourea (19.0 mg, 0.05 mmol), and N,N-diisopropylethylamine (13.0 mg, 0.10 mmol) were sequentially added to a 25 mL reaction flask and stirred at room temperature for 30 minutes. TLC showed complete reaction. The N,N-dimethylformamide was removed by concentration under reduced pressure, and the residue was purified by HPLC to obtain a compound E-5, weighing 80.0 mg, with a yield of 72%. MS (ESI) m/z: 1108 [M/2+H]⁺.

### Step 5: Preparation of compound E-6

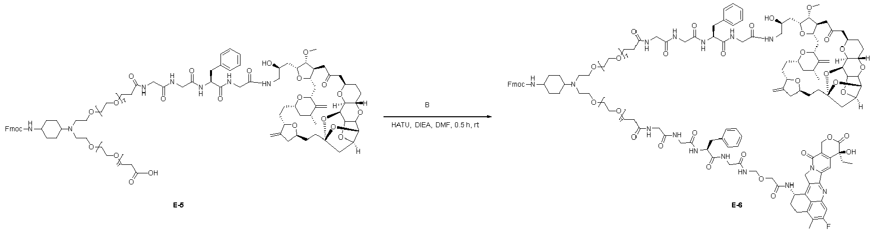

N,N-dimethylformamide (3 mL), the E-6 (35.0 mg, 0.016 mmol), B (16.0 mg, 0.019 mmol), N,N,N',N'-tetramethyl-O-(7-azabenzotriazol-1-yl)hexafluorophosphourea (7.0 mg, 0.018 mmol), and N,N-diisopropylethylamine (3.0 mg, 0.023 mmol) were sequentially added to a 25 mL reaction flask and stirred at room temperature for 30 minutes. TLC showed complete reaction. The N,N-dimethylformamide was removed by reduced pressure concentration, and the residue was purified by HPLC to obtain a compound E-6, weighing 20.0 mg, with a yield of 41%.

MS (ESI) m/z: 1013 [M/3+H]⁺.

### Step 6: Preparation of compound E

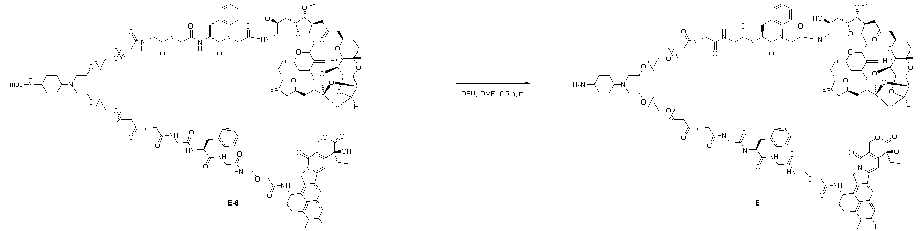

N,N-dimethylformamide (2 mL), the E-6 (20.0 mg, 0.0066 mmol), and 1,8-diazabicyclo[5.4.0]undec-7-ene (2.0 mg, 0.014 mmol) were sequentially added to a 25 mL reaction flask and stirred at room temperature for 30 minutes. TLC showed complete reaction. The N,N-dimethylformamide was removed by concentration under reduced pressure, and the residue was purified by HPLC to obtain a compound E, weighing 15.0 mg, with a yield of 76%.

MS (ESI) m/z: 1408 [M/2+H]⁺.

### Example 6: Preparation of intermediate Fa

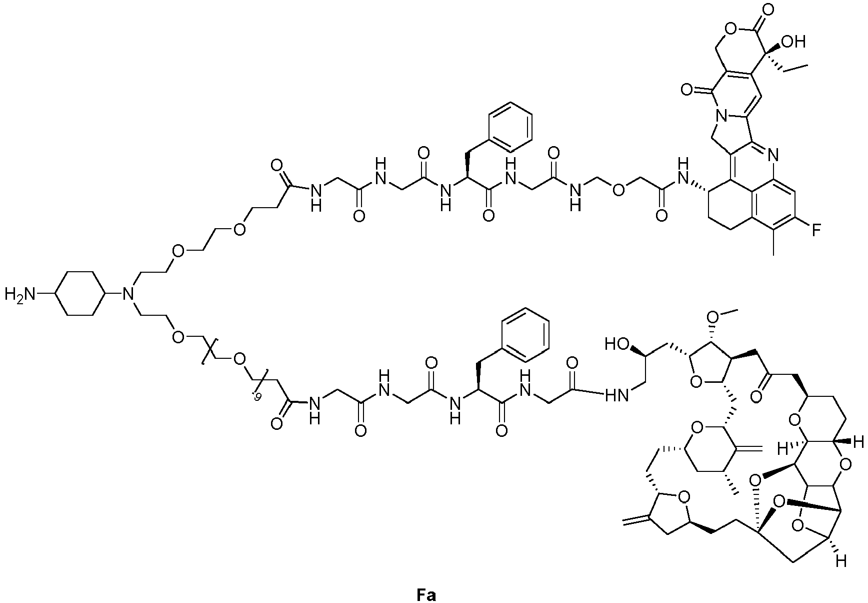

### Step 1: Preparation of compound F-2

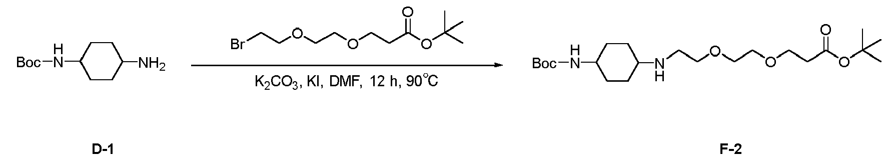

A compound D-1 (1.2 g, 5.61 mmol) and N,N-dimethylformamide (15 mL) were sequentially added to a 25 mL reaction flask and stirred at room temperature. After the reaction solution was clarified, potassium carbonate (2.8 g, 0.29 mmol), potassium iodide (1.8 g, 10.84 mmol), and bromo-dipolyethylene glycol-tert-butyl propionate (2.0 g, 6.73 mmol) were sequentially added and stirred at 90°C for 12 hours. TLC showed complete reaction. The reaction system was filtered, the filtrate was concentrated under reduced pressure, and the residue was purified by HPLC to obtain an intermediate F-2, weighing 0.6 g, with a yield of 25%.

MS (ESI) m/z: 431 [M+H]⁺.

¹HNMR (400 MHz, CDCl3) 7.64 (s, 1H), 5.50 (s, 1H), 3.73 (m, 2H), 3.50-3,54 (m, 7H), 2.72(m, 2H), 2.42-2.55(m, 3H), 1.59-1.72(m, 8H), 1.42(s, 18H).

### Step 2: Preparation of compound F-3

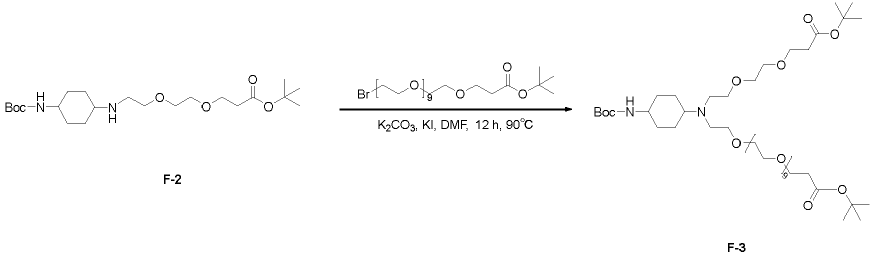

The compound F-2 (600.0 mg, 1.40 mmol) and N,N-dimethylformamide (15 mL) were sequentially added to a 25 mL reaction flask and stirred at room temperature. After the reaction solution was clarified, potassium carbonate (386.0 mg, 2.80 mmol), potassium iodide (232.0 mg, 1.40 mmol), and bromo-nonapolyethylene glycol-tert-butyl propionate (1.8 g, 2.77 mmol) were sequentially added and stirred at 90°C for 12 hours. TLC showed complete reaction. The reaction system was filtered, and the filtrate was concentrated under reduced pressure. The residue was purified by HPLC to obtain an intermediate F-3, weighing 870.0 mg, with a yield of 62%. MS (ESI) m/z: 999 [M+H]⁺.

### Step 3: Preparation of compound F-4

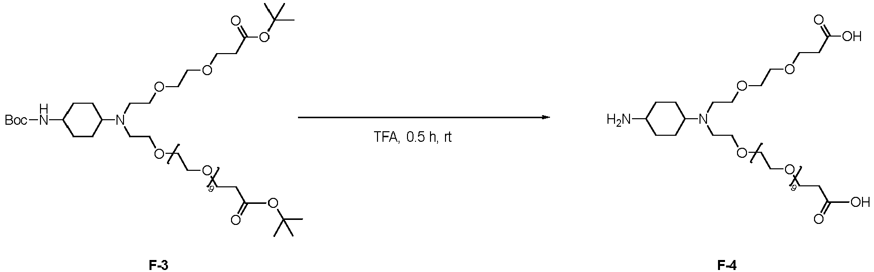

The compound F-3 (870.0 mg, 0.87 mmol) and trifluoroacetic acid (3mL) were added to a 25 mL reaction flask and stirred at room temperature for 0.5 hour. LC-MS showed complete reaction. After concentration under reduced pressure, an intermediate F-4 was obtained, weighing 600.0 mg, with a yield of 87%. The intermediate was directly used in the next step.
MS (ESI) m/z: 787 [M+H]⁺

### Step 4: Preparation of compound F-5

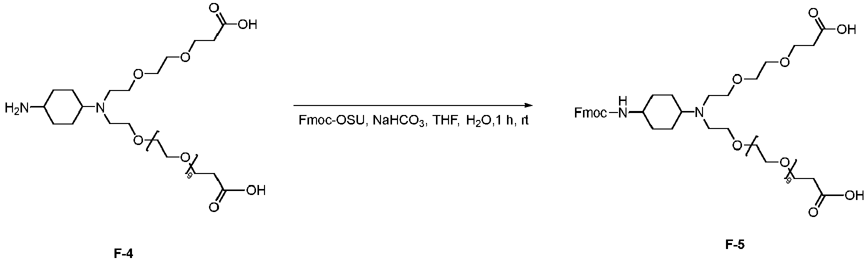

The F-4 (600.0 mg, 0.76 mmol), tetrahydrofuran (10 mL), and water (2.5 mL) were sequentially added to a 25 mL reaction flask and stirred at room temperature. After the reaction solution was clarified, sodium bicarbonate (128.0 mg, 1.52 mmol) and N-(9-fluorenylmethoxycarbonyloxy)succinimide (257.0 mg, 0.76 mmol) are sequentially added. The solution was stirred at room temperature for 2 hour. LC-MS showed complete reaction. After concentration under reduced pressure, the residue was purified by HPLC to obtain a compound F-5, weighing 660 mg, with a yield of 86%.

MS (ESI) m/z: 1009 [M+H]⁺.

### Step 5: Preparation of compound F-6a/F6-b

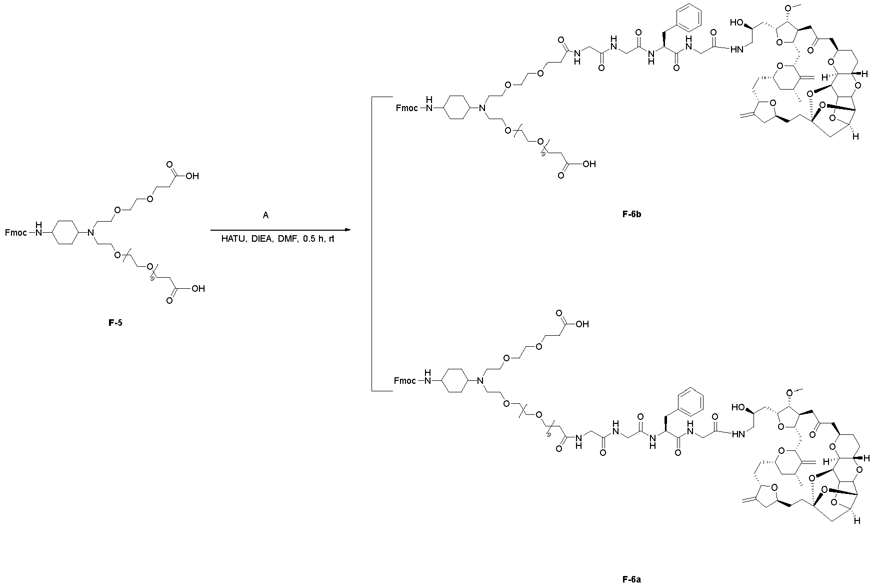

N,N-dimethylformamide (4 mL), A (50 mg, 0.05 mmol), the F-5 (101 mg, 0.10 mmol), N,N,N',N'-tetramethyl-O-(7-azabenzotriazol-1-yl)hexafluorophosphourea (19 mg, 0.05 mmol), and N,N-diisopropylethylamine (13 mg, 0.10 mmol) were sequentially added to a 25 mL reaction flask and stirred at room temperature for 30 minutes. TLC showed complete reaction. The N,N-dimethylformamide was removed by concentration under reduced pressure, and the residue was purified by HPLC to obtain a compound F-6a, weighing 35 mg, with a yield of 34%, and a compound F-6b, weighing 25 mg, with a yield of 25%.

MS (ESI) m/z: 1019 [M/2+H]⁺.

### Step 6: Preparation of compound F-7a

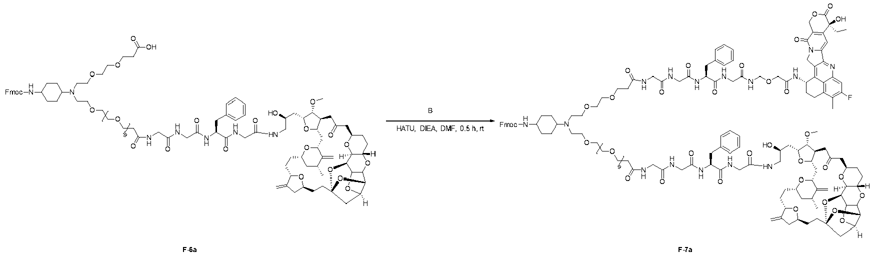

N,N-dimethylformamide (2 mL), the F-6a (35 mg, 0.017 mmol), B (17 mg, 0.021 mmol), N,N,N',N'-tetramethyl -O-(7-azabenzotriazol-1-yl)hexafluorophosphourea (8 mg, 0.021 mmol), and N,N-diisopropylethylamine (3 mg, 0.023 mmol) were sequentially added to a 25 mL reaction flask and stirred at room temperature for 30 minutes. TLC showed complete reaction. The N,N-dimethylformamide was removed by concentration under reduced pressure, and the residue was purified by HPLC to obtain a compound F-7a, weighing 20.0 mg, with a yield of 41%. MS (ESI) m/z: 1430 [M/2+H]⁺.

### Step 7: Preparation of compound Fa

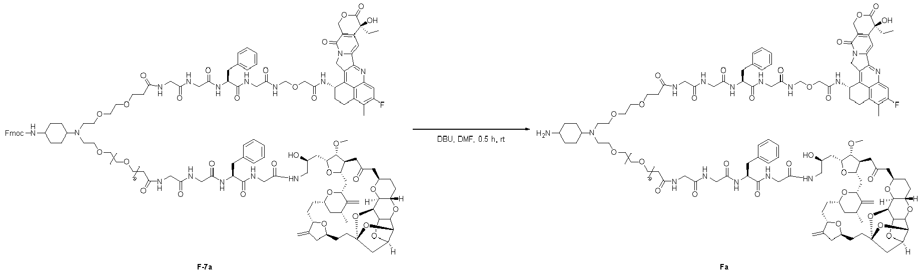

N,N-dimethylformamide (2 mL), the F-7a (20.0 mg, 0.0070 mmol), and 1,8-diazabicyclo[5.4.0]undec-7-ene (2.0 mg, 0.010 mmol) were added to a 25 mL reaction flask and stirred at room temperature for 30 minutes. TLC showed complete reaction. The N,N-dimethylformamide was removed by concentration under reduced pressure, and the residue was purified by HPLC to obtain a compound Fa, weighing 15.0 mg, with a yield of 81%.

MS (ESI) m/z: 1319 [M/2+H]⁺.

### Example 7: Preparation of intermediate Fb

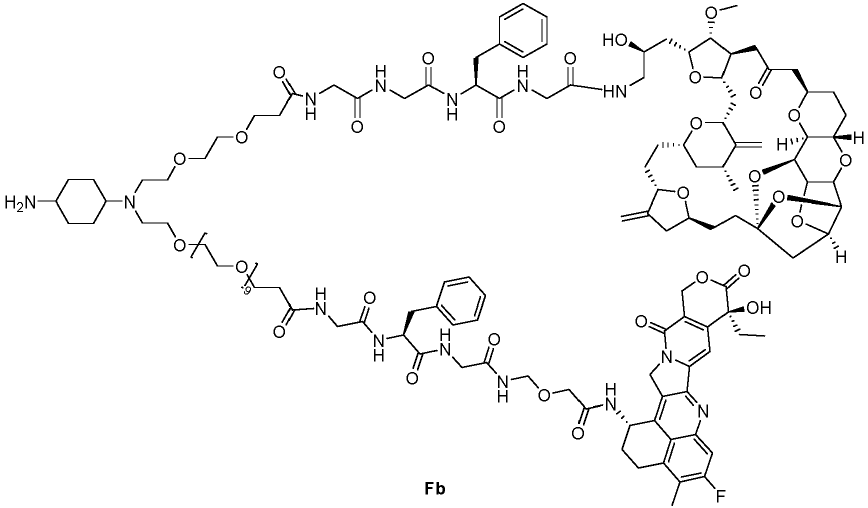

The synthesis method of intermediate Fb referred to the synthesis of compound Fa.

### Example 8: Preparation of intermediate G

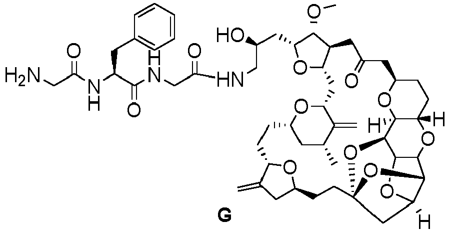

The synthesis method of intermediate G referred to the synthesis of compound A.

MS (ESI) m/z: 991 [M+H]⁺.

1HNMR (400 MHz, DMSO-*d*₆) δ 8.68 (d, J = 7.9 Hz, 1H), 8.45 (t, J = 5.6 Hz, 1H), 7.90 (s, 3H), 7.77 (d, J = 5.5 Hz, 1H), 7.28 - 7.22 (m, 4H), 7.22 - 7.17 (m, 1H), 5.03 (d, J = 22.6 Hz, 2H), 4.82 (s, 1H), 4.75 (s, 1H), 4.64 (s, 3H), 4.55 (t, J = 4.1 Hz, 1H), 4.26 (d, J = 10.2 Hz, 1H), 4.18 (d, J = 10.4 Hz, 1H), 4.10 (s, 3H), 4.02 (s, 1H), 3.83 - 3.64 (m, 5H), 3.61 - 3.46 (m, 6H), 3.26 (s, 4H), 3.16 - 3.02 (m, 3H), 2.84 (d, J = 9.6 Hz, 1H), 2.79 - 2.66 (m, 3H), 2.35 - 2.17 (m, 6H), 2.04 - 1.84 (m, 6H), 1.74 - 1.57 (m, 6H), 1.54 - 1.42 (m, 2H), 1.31 (s, 3H), 1.03 (d, J = 6.3 Hz, 3H), 1.02 - 0.90 (m, 1H).

### Example 9: Preparation of intermediate H

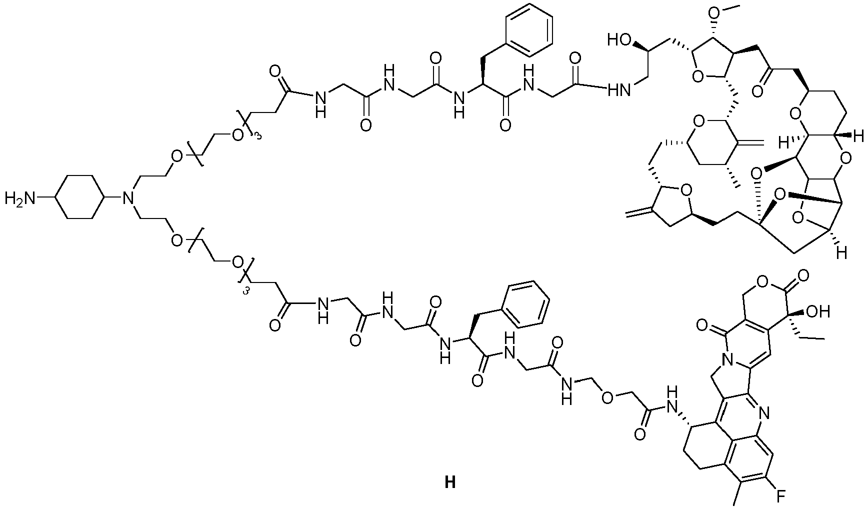

The synthesis method of intermediate H referred to the synthesis of compound E.

### Example 10: Preparation of intermediate I

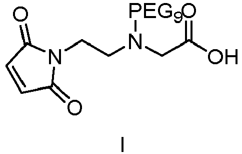

### Step 1: Preparation of compound I-2

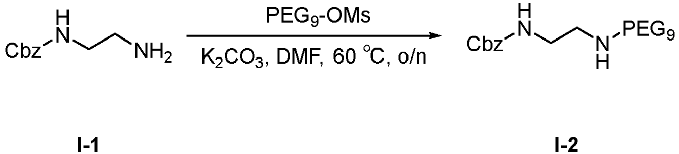

I-1 (1.0g, 5.15 mmol), potassium carbonate (1.4 g, 10.14 mmol), and N,N-dimethylformamide (25 mL) were added to a 25 mL reaction flask, and PEG₉-OMs (2.3 g, 4.55 mmol) was added at a time, followed by stirring overnight at 60°C. LC-MS showed complete reaction. The reaction system was filtered, the filtrate was concentrated under reduced pressure to remove the N,N-dimethylformamide, and the residue was purified by thin layer chromatography (petroleum ether: ethyl acetate=1:1) to obtain a compound I-2, weighing 2.5 g, with a yield of 81%.

MS (ESI) m/z: 605 [M+H]⁺.

¹HNMR (400 MHz, CDCl3) 7.42 (m, 1H), 7.33(d, J=8.0 Hz, 2H), 7.15(d, J=7.8 Hz, 2H), 6.75 (m, 1H), 5.05(s, 2H), 4.21 (m, 1H),3.43-3.54(m, 34H), 3.30(s, 3H), 3.07(m,2H), 2.66-2.75(m, 4H).

### Step 2: Preparation of compound I-3

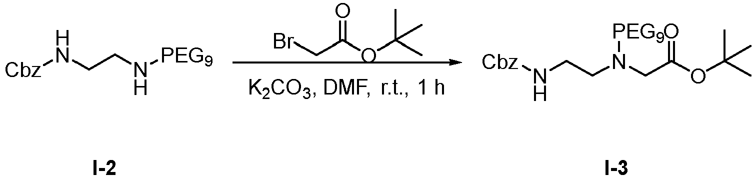

The I-2 (1.0 g, 1.66 mmol), potassium carbonate (457.0 mg, 3.31 mmol), and N,N-dimethylformamide (20 mL) were added to a 25 mL reaction flask, tert-butyl bromoacetate (483.0 mg, 2.48 mmol) was added at a time, followed by stirring at room temperature for 1 hour. LC-MS showed complete reaction. The reaction system was filtered, the filtrate was concentrated under reduced pressure to remove the N,N-dimethylformamide, and the residue was purified by HPLC to obtain a compound I-3, weighing 600.0 mg, with a yield of 50%.

MS (ESI) m/z: 719 [M+H]⁺.

¹HNMR (400 MHz, CDCl3) 7.40 (m, 1H), 7.35(d, J=8.0 Hz, 2H), 7.19(d, J=7.8 Hz, 2H), 6.76 (m, 1H), 5.12(s, 2H),3.40-3.52(m, 34H), 3.30(s, 3H), 3.07-3.21(m, 4H), 2.66-2.75(m, 4H), 1.31(s, 9H).

### Step 3: Preparation of compound I-4

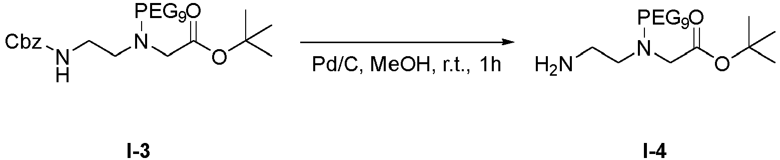

The I-3 (500.0 mg, 0.70 mmol), palladium carbon (50.0 mg), and methanol (10 mL) were added to a 25 mL reaction flask. After three times of hydrogen substitution, stirring was performed at room temperature in the hydrogen environment for 1 hour, and LC-MS showed complete reaction. The reaction system was filtered, and the filtrate was concentrated under reduced pressure to remove the methanol, so as to obtain a compound I-4, weighing 360.0 mg, with a yield of 88%.
MS (ESI) m/z: 585 [M+H]⁺

### Step 4: Preparation of compound I-5

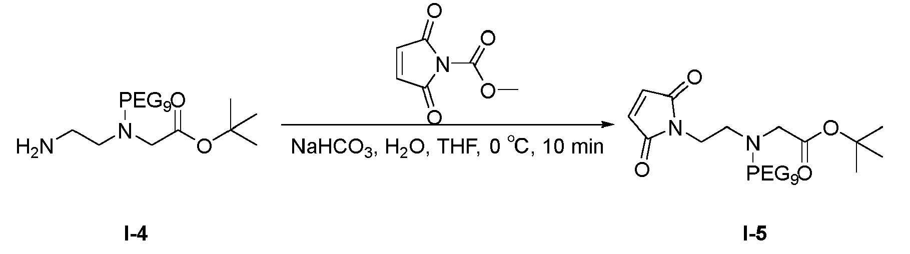

The I-4 (360.0 mg, 0.62 mmol), sodium bicarbonate (103.0 mg, 1.23 mmol), tetrahydrofuran (8mL), and water (1 mL) were added to a 25 mL reaction flask, and N-methoxycarbonyl maleimide (142.0 mg, 0.92 mmol) was slowly added at 0°C. After adding, the reaction continued at 0°C for 10 minutes, and LC-MS showed complete reaction. The tetrahydrofuran and the water were removed by concentration under reduced pressure, and the residue was purified by HPLC to obtain a compound I-5, weighing 180.0 mg, with a yield of 44%.

MS (ESI) m/z: 665 [M+H]⁺.

¹HNMR (400 MHz, CDCl3) 7.86 (s, 1H), 7.75 (s, 1H),3.32-3.52(m, 38H), 3.20(s, 3H), 2.66-2.75(m, 4H), 1.23(s, 9H).

### Step 5: Preparation of compound I

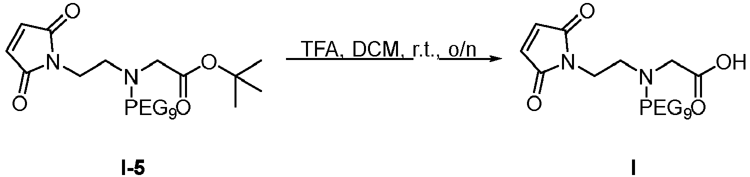

The I-5 (180.0 mg, 0.27 mmol) and trifluoroacetic acid (3 mL) were added to a 25 mL reaction flask. Stirring was performed at room temperature overnight, and LC-MS showed complete reaction. The trifluoroacetic acid was removed by concentration under reduced pressure, and the residue was purified by HPLC to obtain a compound I, weighing 120.0 mg, with a yield of 73%.

MS (ESI) m/z: 609 [M+H]⁺.

### Example 11: Preparation of intermediate J

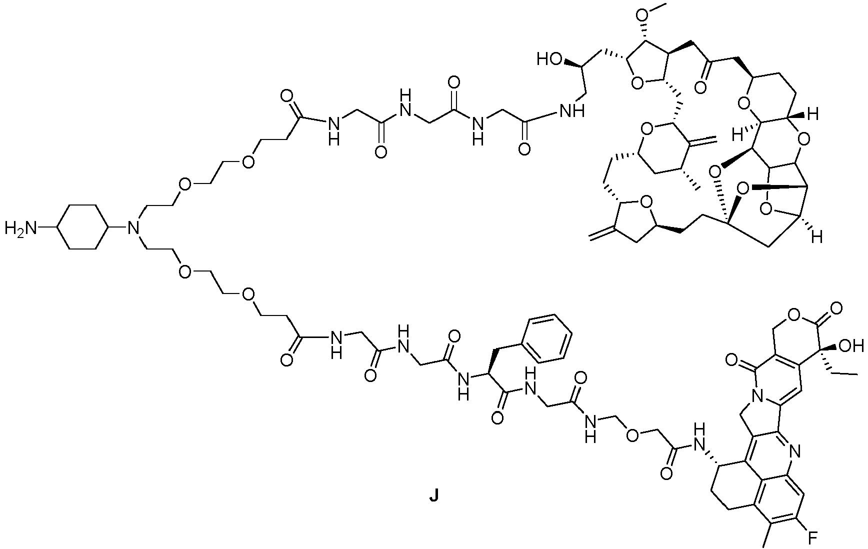

### Step 1: Preparation of compound J-2

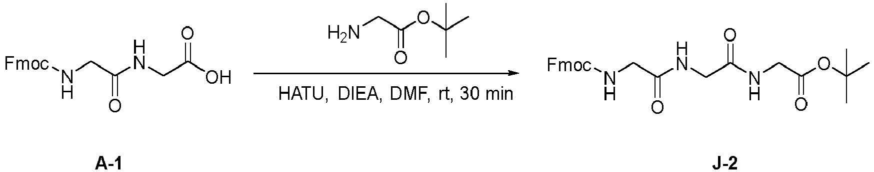

N,N-dimethylformamide (5 mL), A-1 (130.0 mg, 0.37 mmol), N,N,N',N'-tetramethyl-O-(7-azabenzotriazol-1-yl)hexafluorophosphourea (164.0 mg, 0.43 mmol), and N,N-diisopropylethylamine (70.0 mg, 0.54 mmol) were sequentially added to a 25 mL reaction flask and stirred at room temperature for reaction for 5 minutes, and then tert-butyl glycinate (56.0 mg, 0.43 mmol) was added. The reaction continued for 25 minutes, and TLC showed complete reaction. The N,N-dimethylformamide was removed by concentration under reduced pressure, and the residue was purified by thin layer chromatography (dichloromethane: methanol=20:1) to obtain a compound J-2, weighing 110.0 mg, with a yield of 65%.

MS (ESI) m/z: 468 [M+H]⁺.

¹HNMR (400 MHz, CDCl3) 9.01 (s, 1H), 8.87 (s, 1H), 8.15 (s, 1H), 7.90(d, J=8.0 Hz, 2H), 7.55(d, J=8.5 Hz, 2H), 7.29-7.35(m, 4H), 4.70 (d, 2H), 446(t, 1H), 4.09-4.17(m, 6H), 1.35(s, 9H).

### Step 2: Preparation of compound J-3

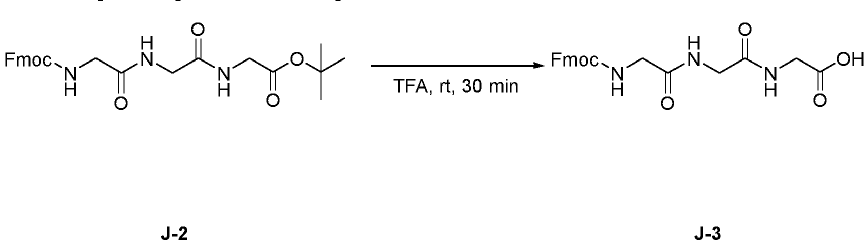

The compound J-2 (100.0 mg, 0.21 mmol) and trifluoroacetic acid (2 mL) were added to a 25 mL reaction flask and stirred at room temperature for 30 minutes. LC-MS showed complete reaction. After concentration under reduced pressure, the residue was purified by HPLC to obtain an intermediate J-3, weighing 75.0 mg, with a yield of 86%.

MS (ESI) m/z: 412 [M+H]⁺.

### Step 3: Preparation of compound J-4

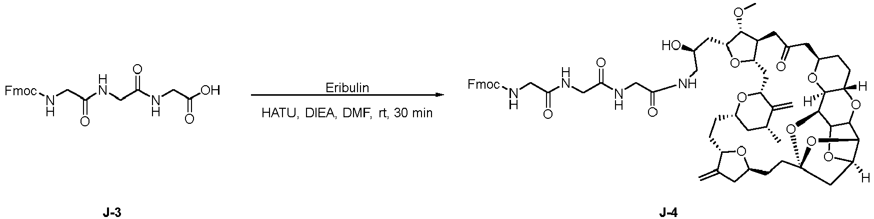

N,N-dimethylformamide (3 mL), the J-3 (75.0 mg, 0.18 mmol), N,N,N',N'-tetramethyl-O-(7-azabenzotriazol-1-yl)hexafluorophosphourea (83.0 mg, 0.22 mmol), and N,N-diisopropylethylamine (35.0 mg, 0.27 mmol) were sequentially added to a 25 mL reaction flask and stirred at room temperature for 5 minutes, and then Eribulin (138.0 mg, 0.19 mmol) was added. The stirring continued for 10 minutes, and TLC showed complete reaction. The N,N-dimethylformamide was removed by concentration under reduced pressure, and the residue was purified by thin layer chromatography (dichloromethane: methanol=10:1) to obtain a compound J-4, weighing 175.0 mg, with a yield of 87%.

MS (ESI) m/z: 1123 [M+H]⁺.

### Step 4: Preparation of compound J-5

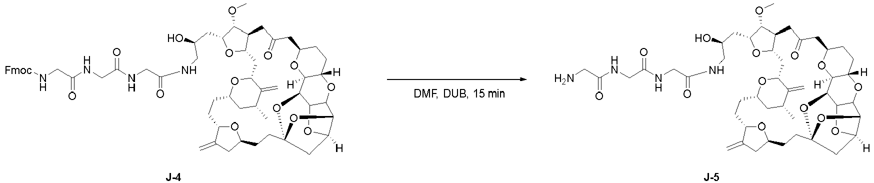

N,N-dimethylformamide (2 mL), the J-4 (175.0 mg, 0.16 mmol), and 1,8-diazabicyclo[5.4.0]undec-7-ene (49.0 mg, 0.32 mmol) were added to a 25 mL reaction flask and stirred at room temperature for 15 minutes. TLC showed complete reaction. The N,N-dimethylformamide was removed by concentration under reduced pressure, and the residue was purified by HPLC to obtain a compound J-5, weighing 108.0 mg, with a yield of 75%.

MS (ESI) m/z: 901 [M+H]⁺.

### Step 5: Preparation of compound J-6

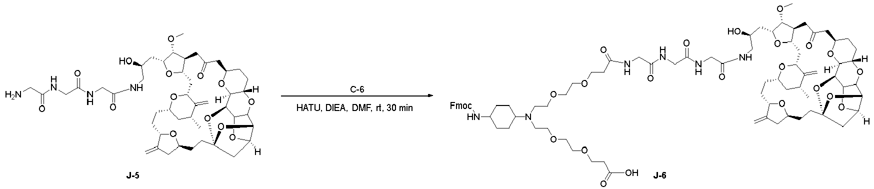

N,N-dimethylformamide (3 mL), the J-5 (45.0 mg, 0.05 mmol), C-6 (66.0 mg, 0.10 mmol), N,N,N',N'-tetramethyl-O-(7-azabenzotriazol-1-yl)hexafluorophosphourea (19.0 mg, 0.05 mmol), and N,N-diisopropylethylamine (13.0 mg, 0.10 mmol) were sequentially added to a 25 mL reaction flask and stirred at room temperature for 30 minutes. TLC showed complete reaction. The N,N-dimethylformamide was removed by concentration under reduced pressure, and the residue was purified by HPLC to obtain a compound J-6, weighing 49.0 mg, with a yield of 64%. MS (ESI) m/z: 1539 [M+H]⁺

### Step 6: Preparation of compound J-7

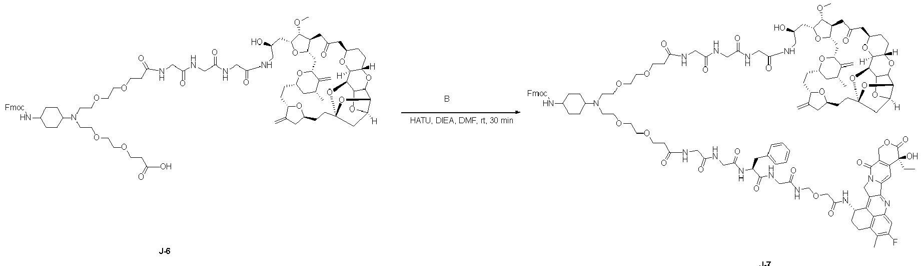

N,N-dimethylformamide (3 mL), the J-6 (40.0 mg, 0.026 mmol), B (26.0 mg, 0.031 mmol), N,N,N',N'-tetramethyl-O-(7-azabenzotriazol-1-yl)hexafluorophosphourea (12.0 mg, 0.031 mmol), and N,N-diisopropylethylamine (5.0 mg, 0.039 mmol) were sequentially added to a 25 mL reaction flask and stirred at room temperature for reaction for 30 minutes, and then TLC showed complete reaction. The N,N-dimethylformamide was removed by concentration under reduced pressure, and the residue was purified by thin layer chromatography (dichloromethane: methanol=12:1) to obtain a compound J-6, weighing 32.0 mg, with a yield of 52%.

MS (ESI) m/z: 1181 [M/2+H]⁺.

### Step 7: Preparation of compound J

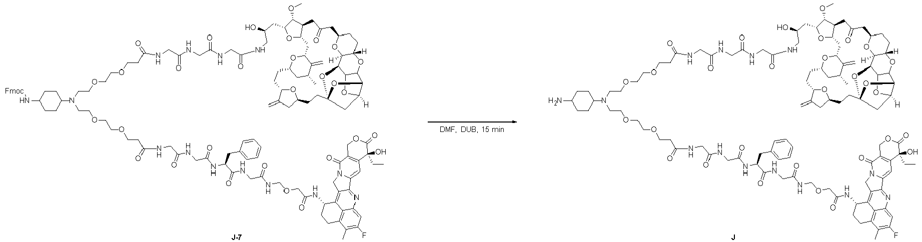

N,N-dimethylformamide (2 mL), the J-7 (32.0 mg, 0.014 mmol), and 1,8-diazabicyclo[5.4.0]undec-7-ene (3.0 mg, 0.020 mmol) were sequentially added to a 25 mL reaction flask and stirred at room temperature for 15 minutes. TLC showed complete reaction. The N,N-dimethylformamide was removed by concentration under reduced pressure, and the residue was purified by HPLC to obtain a compound J, weighing 25.0 mg, with a yield of 86%.

MS (ESI) m/z: 1070 [M/2+H]⁺.

### Example 12: Preparation of intermediate K

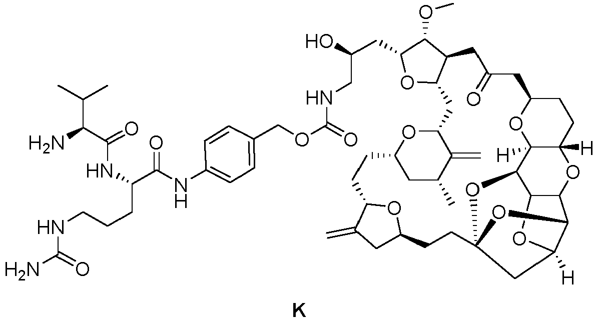

### Step 1: Preparation of compound K-2

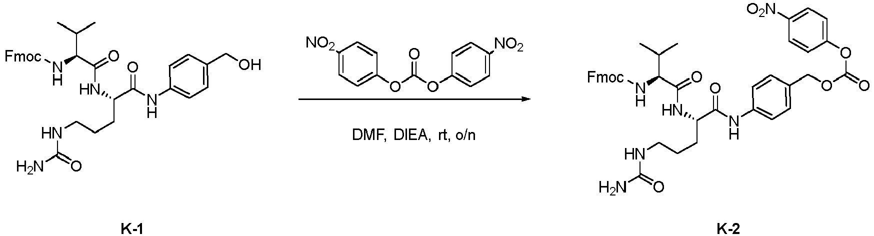

A compound K-1 (601.0 mg, 1.00 mmol) and N,N-dimethylformamide (5 mL) were added to a 25 mL reaction flask, then N,N-diisopropylethylamine (258.0 mg, 2.00 mmol) was added, bis(p-nitrophenyl)carbonate (456.0 mg, 1.50 mmol) was added at a time, and a reaction occurred at room temperature overnight while stirring. TLC showed complete reaction. The reaction solution was slowly added dropwise to methyl tert-butyl ether (25 mL) to precipitate a light yellow solid. The solid obtained by filtering was dried in vacuum to obtain a compound K-2, weighing 580.0 mg, with a yield of 76%. MS (ESI) m/z: 767 [M+H]⁺.

### Step 2: Preparation of compound K-3

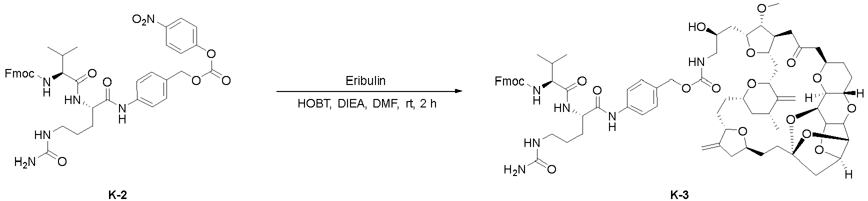

The compound K-2 (300.0 mg, 0.39 mmol) and N,N-dimethylformamide (5 mL) were added to a 25 mL reaction flask and stirred at room temperature. After complete clarification, N,N-diisopropylethylamine (101.0 mg, 0.78 mmol) and 1-hydroxybenzotriazole (27 mg, 0.20 mmol) were added, then Eribulin (292.0 mg, 0.40 mmol) was added at a time, followed by stirring at room temperature for 2 hours. LC-MS showed complete reaction. The N,N-dimethylformamide was removed by concentration under reduced pressure, and the residue was purified by thin layer chromatography (dichloromethane: methanol=15:1) to obtain a compound K-3, weighing 478.0 mg, with a yield of 90%.

MS (ESI) m/z: 1357 [M+H]⁺.

### Step 3: Preparation of compound K

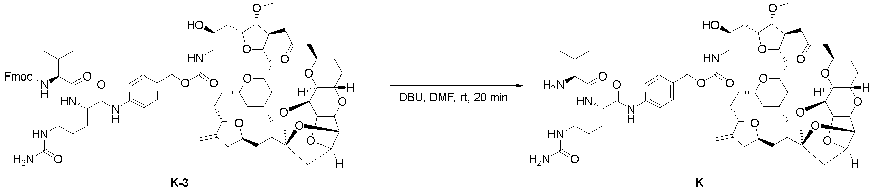

N,N-dimethylformamide (4 mL), the K-3 (245 mg, 0.18 mmol), and 1,8-diazabicyclo[5.4.0]undec-7-ene (54.0 mg, 0.36 mmol) were added to a 25 mL reaction flask and stirred at room temperature for 20 minutes. TLC showed complete reaction. The N,N-dimethylformamide was removed by concentration under reduced pressure, and the residue was purified by HPLC to obtain a compound K, weighing 180.0 mg, with a yield of 88%.

MS (ESI) m/z: 1135 [M+H]⁺.

### Example 13: Preparation of compound SMP-22682

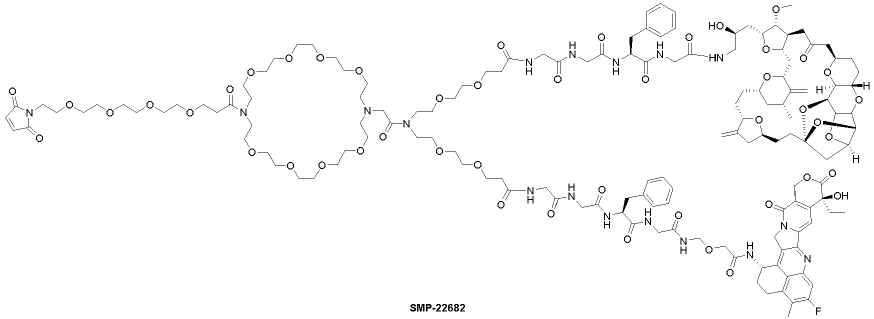

### Step 1: Preparation of compound SMP-22682-2

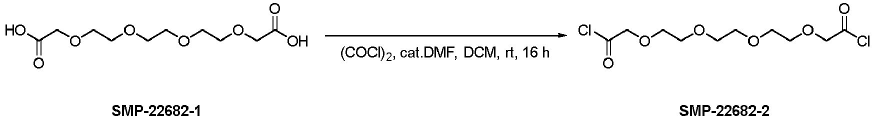

SMP-22682-1 (3.0 g, 11.28 mmol) and dichloromethane (30 mL) were added to a 250 mL reaction flask, oxalyl chloride (5.7 g, 44.88 mol) was added under ice-water bath and nitrogen protection, and then N,N-dimethylformamide (0.1 mL) was slowly added dropwise. After adding, a reaction occurred at room temperature for 16 hours. LC-MS showed substantially complete reaction of the raw materials. The solvent and excess oxalyl chloride were removed by direct distillation under reduced pressure, a small amount of toluene was added after concentration, and 3.0 g of crude product was obtained after second distillation under reduced pressure for direct use in the next step.

### Step 2: Preparation of compound SMP-22682-4

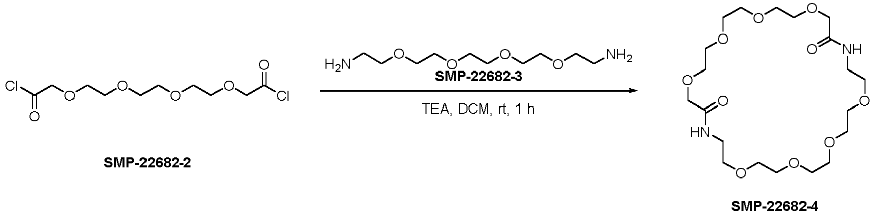

SMP-22682-3 (2.4 g, 0.01 mol), triethylamine (4.0 g, 0.040 mol), and dichloromethane (60 mL) were added to a 250 mL reaction flask. A dichloromethane suspension of the SMP-22682-2 (3.0 g, the crude product was mixed in 20 mL of dichloromethane) was slowly added dropwise in an ice-water bath. After adding, the solution was further stirred for 10 minutes and slowly heated to room temperature, and the reaction was kept at room temperature for 1 hour. LC-MS showed the molecular weight of a target compound. The reaction system was concentrated, and the crude product was purified by column chromatography (dichloromethane: methanol=10: 1) to obtain a compound SMP-22682-4, weighing 0.90 g, with a two-step yield of 17%.

MS (ESI) m/z: 467[M+H]⁺.

¹HNMR (400 MHz, CDCl3) 7.41 (s, 1H), 7.12 (s, 1H), 4.26-4.32(m, 4H),3.31-3.56(m, 32H).

### Step 3: Preparation of compound SMP-22682-5

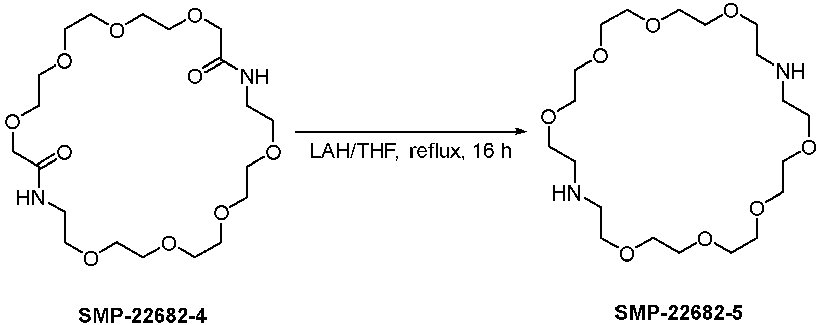

The SMP-22682-4 (0.90 g, 1.93 mmol) was added to a 100 mL reaction flask, and then a tetrahydrofuran solution of 1M lithium aluminum hydride (20 mL) was added. The reaction system was heated and refluxed for 16 hours, and LC-MS showed that most was the molecular weight of a target compound. Sodium sulfate decahydrate (5.0 g) was added in batches to the reaction solution, followed by filtration. The filtrate was concentrated, and the obtained crude product was purified by column chromatography (ethyl acetate: methanol=5:1) to obtain a compound SMP-22682-5, weighing 0.50 g, with a yield of 59%.

MS (ESI) m/z: 439[M+H]⁺.

¹HNMR (400 MHz, CDCl3) 5.91 (s, 1H), 5.32 (s, 1H), 4.26-4.32(m, 4H), 3.49-3.56(m, 32H), 2.66-2.72(m, 8H).

### Step 4: Preparation of compound SMP-22682-6

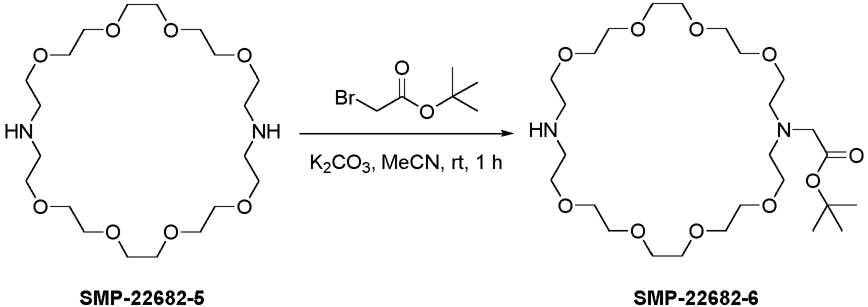

Acetonitrile (10 mL), the SMP-22682-5 (500.0 mg, 1.14 mmol), tert-butyl bromoacetate (178.0 mg, 0.91 mmol), and potassium carbonate (126.0 mg, 0.91 mmol) were added to a 100 mL reaction flask. After stirring at room temperature for 1 hour, LC-MS showed that most was the molecular weight of a target compound. The reaction solution was concentrated, and the obtained crude product was purified by column chromatography (dichloromethane: methanol=10: 1) to obtain a compound SMP-22682-6, weighing 240.0 mg, with a yield of 48%.

MS (ESI) m/z: 553[M+H]⁺.

¹HNMR (400 MHz, CDCl3) 5.91 (s, 1H), 3.33-3.54(m, 34H), 2.52-2.70(m, 8H),1.33 (s, 9H).

### Step 5: Preparation of compound SMP-22682-7

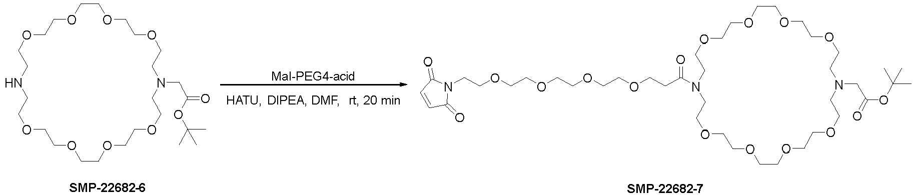

N,N-dimethylformamide (2 mL), the SMP-22682-6 (75.0 mg, 0.14 mmol), Mal-PEG4-acid (56.0 mg, 0.16 mmol), N,N,N',N'-tetramethyl-O-(7-azabenzotriazol-1-yl)hexafluorophosphourea (62.0 mg, 0.16 mmol), and N,N-diisopropylethylamine (26.0 mg, 0.20 mmol) were sequentially added to a 10 mL reaction flask and stirred at room temperature for 20 minutes. LC-MS showed that most was the molecular weight of a target compound. The reaction solution was concentrated, and the obtained crude product was purified by HPLC to obtain a compound SMP-22682-7, weighing 65 mg, with a yield of 56%.

MS (ESI) m/z: 880[M+H]⁺.

### Step 6: Preparation of compound SMP-22682-8

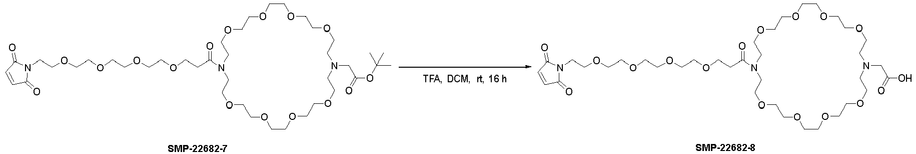

The SMP-22682-7 (65.0 mg, 0.074 mmol), dichloromethane (2 mL), and trifluoroacetic acid (2 mL) were sequentially added to a 10 mL reaction flask. After stirring at room temperature for 16 hour, LC-MS showed that most was the molecular weight of a target compound. The reaction solution was concentrated, and the obtained crude product was purified by HPLC to obtain a compound SMP-22682-8, weighing 40.0 mg, with a yield of 66%.

MS (ESI) m/z: 824[M+H]⁺.

### Step 7: Preparation of compound SMP-22682

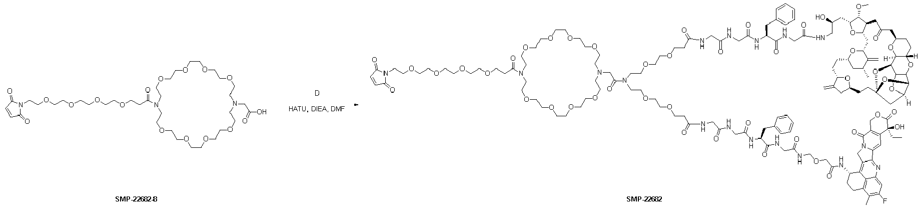

N,N-dimethylformamide (1 mL), the SMP-22682-8 (10.0 mg, 0.012 mmol), D (8.0 mg, 0.0037 mmol), N,N,N',N'-tetramethyl-O-(7-azabenzotriazol-1-yl)hexafluorophosphourea (2.0 mg, 0.0053 mmol), and N,N-diisopropylethylamine (2.0 mg, 0.016 mmol) were sequentially added to a 10 mL reaction flask and stirred at room temperature for 20 minutes. LC-MS showed that most was the molecular weight of a target compound. The reaction solution was concentrated, and the obtained crude product was purified by HPLC to obtain a compound SMP-22682, weighing 2.2 mg, with a yield of 20%.

MS (ESI) m/z: 999[M/3+H]⁺.

### Example 14: Preparation of compound SMP-07861

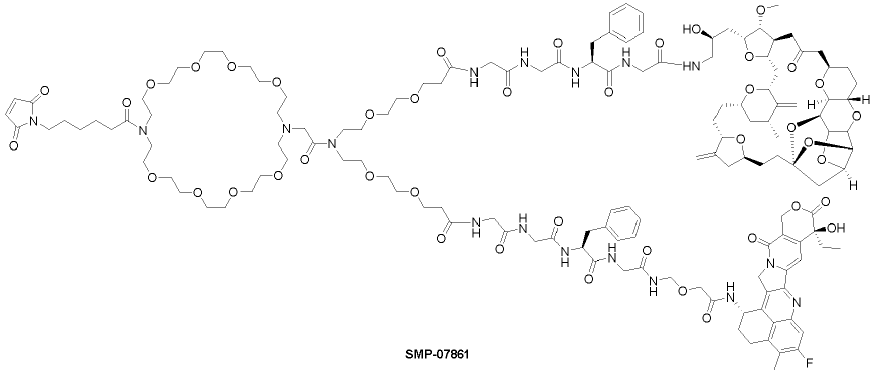

### Step 1: Preparation of compound SMP-07861-1

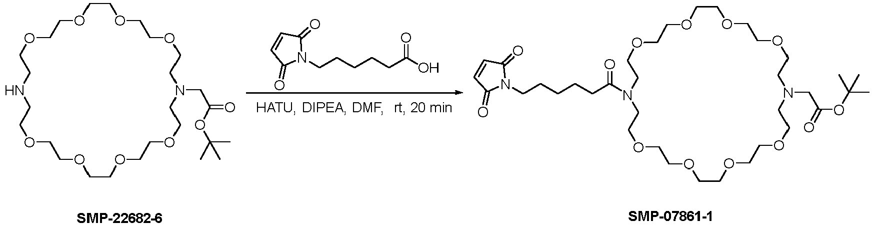

N,N-dimethylformamide (2 mL), SMP-22682-6 (50.0 mg, 0.09 mmol), maleimidocaproic acid (23.0 mg, 0.11 mmol), N,N,N',N'-tetramethyl-O-(7-azabenzotriazol-1-yl)hexafluorophosphourea (41.0 mg, 0.11 mmol), and N,N-diisopropylethylamine (18.0 mg, 0.14 mmol) were sequentially added to a 10 mL reaction flask and stirred at room temperature for 20 minutes. LC-MS showed that most was the molecular weight of a target compound. The reaction solution was concentrated, and the obtained crude product was purified by HPLC to obtain a compound SMP-07861-1, weighing 40.0 mg, with a yield of 59%.

MS (ESI) m/z: 746[M+H]⁺.

### Step 2: Preparation of compound SMP-07861-2

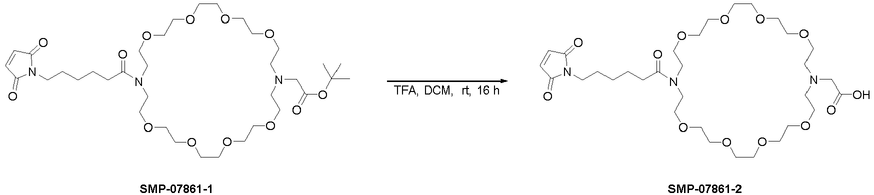

The SMP-07861-1 (40.0 mg, 0.054 mmol), dichloromethane (2 mL), and trifluoroacetic acid(2 mL) were sequentially added to a 10 mL reaction flask. After stirring at room temperature for 16 hour, LC-MS showed that most was the molecular weight of a target compound. The reaction solution was concentrated, and the obtained crude product was purified by HPLC to obtain a compound SMP-07861-2, weighing 30.0 mg, with a yield of 82%.

MS (ESI) m/z: 690[M+H]⁺.

### Step 3: Preparation of compound SMP-07861

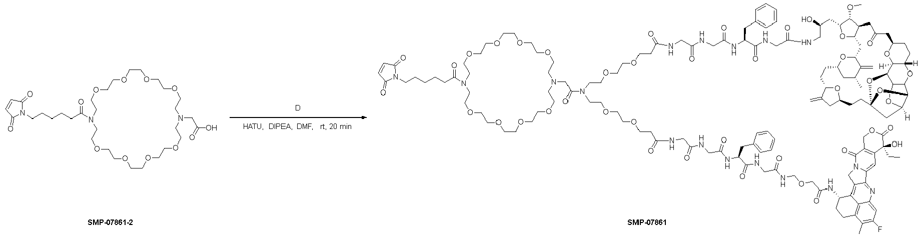

N,N-dimethylformamide (1 mL), the SMP-07861-2 (8.0 mg, 0.012 mmol), D (8.0 mg, 0.0037 mmol), N,N,N',N'-tetramethyl-O-(7-azabenzotriazol-1-yl)hexafluorophosphourea (2.0 mg, 0.0053 mmol), and N,N-diisopropylethylamine (2.0 mg, 0.016 mmol) were sequentially added to a 10 mL reaction flask and stirred at room temperature for 20 minutes. LC-MS showed that most was the molecular weight of a target compound. The reaction solution was concentrated, and the obtained crude product was purified by HPLC to obtain a compound SMP-07861, weighing 2.2 mg, with a yield of 21%.

MS (ESI) m/z: 954[M/3+H]⁺.

### Example 15: Preparation of compound SMP-61694

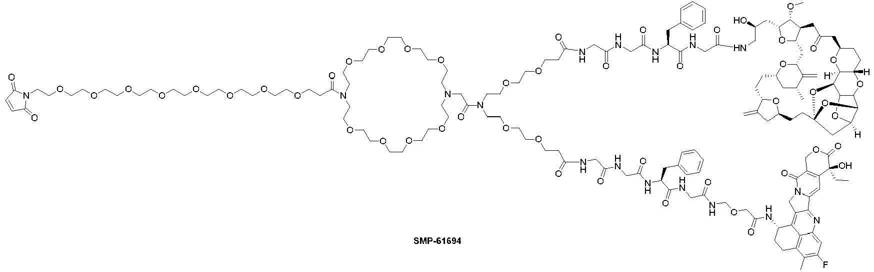

### Step 1: Preparation of compound SMP-61694-1

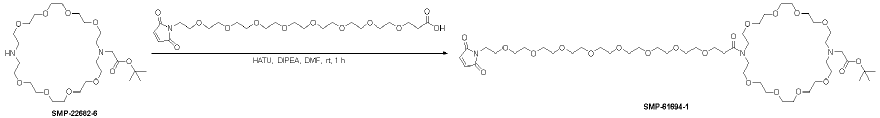

N,N-dimethylformamide (2 mL), the SMP-22682-6 (50.0 mg, 0.09 mmol), Mal-PEG8-acid (57.0 mg, 0.11 mmol), N,N,N',N'-tetramethyl-O-(7-azabenzotriazol-1-yl)hexafluorophosphourea(41.0 mg, 0.11 mmol), and N,N-diisopropylethylamine (18.0 mg, 0.14 mmol) were sequentially added to a 10 mL reaction flask and stirred at room temperature for 1 hour. LC-MS showed that most was the molecular weight of a target compound. The reaction solution was concentrated, and the obtained crude product was purified by HPLC to obtain a compound SMP-61694-1, weighing 20.0 mg, with a yield of 21%.

MS (ESI) m/z: 1056[M+H]⁺.

### Step 2: Preparation of compound SMP-61694-2

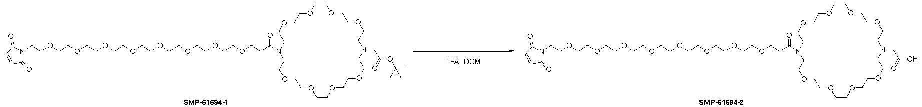

The SMP-61694-1 (20.0 mg, 0.019 mmol), dichloromethane (1 mL), and trifluoroacetic acid (1 mL) were sequentially added to a 10 mL reaction flask. After stirring at room temperature for 20 minutes, LC-MS showed that most was the molecular weight of a target compound. After concentration, a crude product SMP-61694-2 was obtained, weighing 15.0 mg, and the next step was directly performed.

MS (ESI) m/z: 1000[M+H]⁺.

### Step 3: Preparation of compound SMP-61694

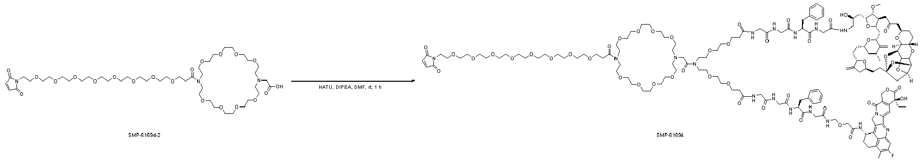

N,N-dimethylformamide (1 mL), the SMP-61694-2 (6.0 mg, 0.0060 mmol), D (10.0 mg, 0.0046 mmol), N,N,N',N'-tetramethyl-O-(7-azabenzotriazol-1-yl)hexafluorophosphourea (2.0 mg, 0.0053 mmol), and N,N-diisopropylethylamine (2.0 mg, 0.016 mmol) were sequentially added to a 10 mL reaction flask and stirred at room temperature for 1 hour. LC-MS showed that most was the molecular weight of a target compound. The reaction solution was concentrated, and the obtained crude product was purified by HPLC to obtain a compound SMP-61694, weighing 6.8 mg, with a yield of 47%.

MS (ESI) m/z: 1057[M/3+H]⁺.

### Example 16: Preparation of compound SMP-45539

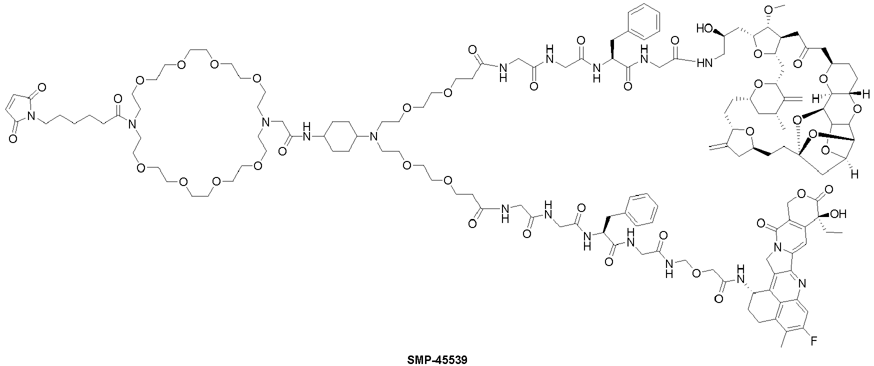

### Step 1: Preparation of compound SMP-45539

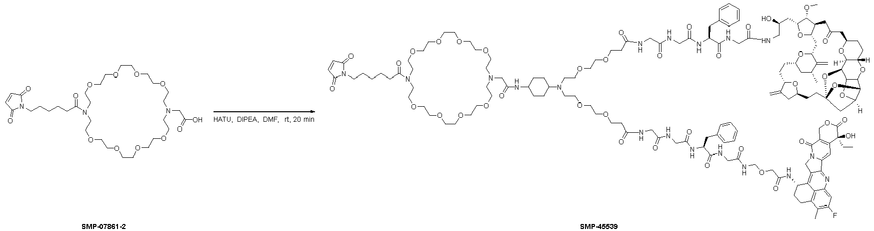

N,N-dimethylformamide (1 mL), SMP-07861-2 (5.0 mg, 0.0073 mmol), C (10.0 mg, 0.0044 mmol), N,N,N',N'-tetramethyl-O-(7-azabenzotriazol-1-yl)hexafluorophosphourea (2.0 mg, 0.0053 mmol), and N,N-diisopropylethylamine (2.0 mg, 0.016 mmol) were sequentially added to a 10 mL reaction flask and stirred at room temperature for 20 minutes. LC-MS showed that most was the molecular weight of a target compound. The reaction solution was concentrated, and the obtained crude product was purified by HPLC to obtain a compound SMP-45539, weighing 2.6 mg, with a yield of 20%.

MS (ESI) m/z: 986[M/3+H]⁺.

### Example 17: Preparation of compound SMP-59034

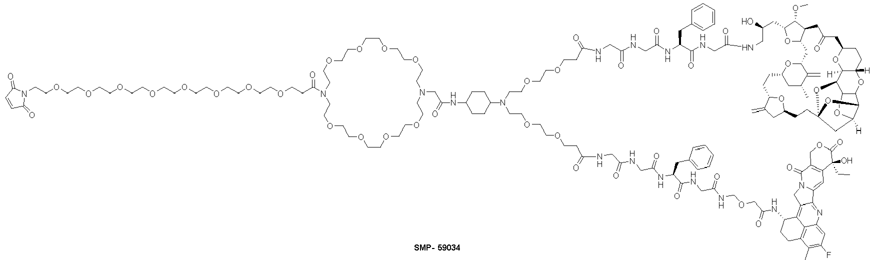

### Step 1: Preparation of compound SMP-59034

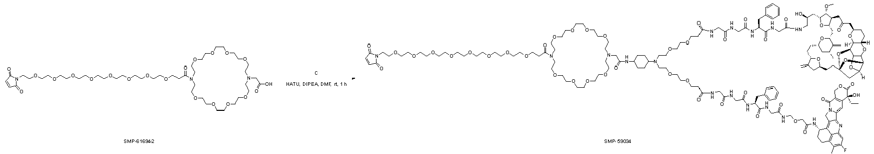

N,N-dimethylformamide (1 mL), the SMP-61694-2 (6.0 mg, 0.0060 mmol), C (10.0 mg, 0.0044 mmol), N,N,N',N'-tetramethyl-O-(7-azabenzotriazol-1-yl)hexafluorophosphourea (2.0 mg, 0.0053 mmol), and N,N-diisopropylethylamine (2.0 mg, 0.016 mmol) were sequentially added to a 10 mL reaction flask and stirred at room temperature for 1 hour. LC-MS showed that most was the molecular weight of a target compound. The reaction solution was concentrated, and the obtained crude product was purified by HPLC to obtain a compound SMP-45539, weighing 2.8 mg, with a yield of 20%.

MS (ESI) m/z: 1090[M/3+H]⁺.

### Example 18: Preparation of compound SMP-29438

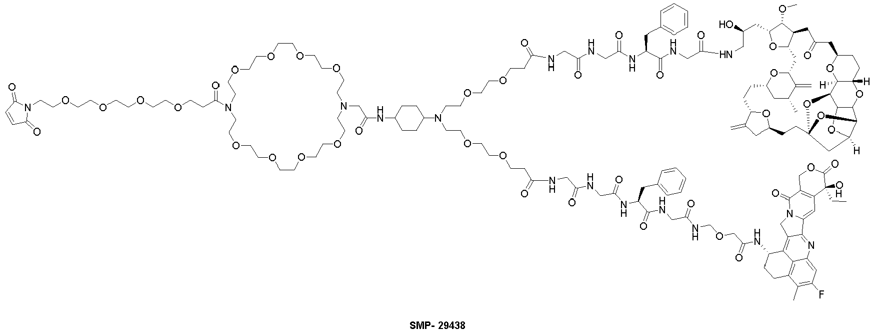

### Step 1: Preparation of compound SMP-29438

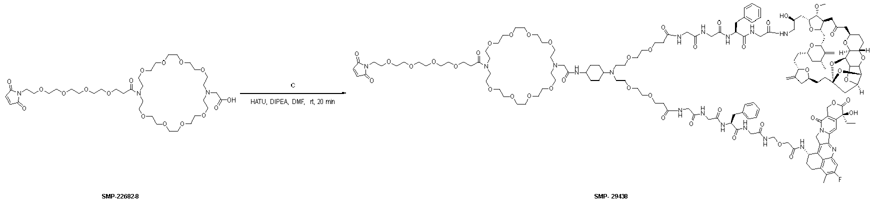

N,N-dimethylformamide (1 mL), SMP-22682-8 (5.0 mg, 0.0061 mmol), C (10.0 mg, 0.0044 mmol), N,N,N',N'-tetramethyl-O-(7-azabenzotriazol-1-yl)hexafluorophosphourea (2.0 mg, 0.0053 mmol), and N,N-diisopropylethylamine (2.0 mg, 0.016 mmol) were sequentially added to a 10 mL reaction flask and reacted at room temperature for 20 minutes, and then LC-MS showed that most was the molecular weight of a target compound. The reaction solution was concentrated, and the obtained crude product was purified by HPLC to obtain a compound SMP-45539, weighing 3.9 mg, with a yield of 29%.

MS (ESI) m/z: 1031[M/3+H]⁺.

### Example 19: Preparation of compound SMP-49182

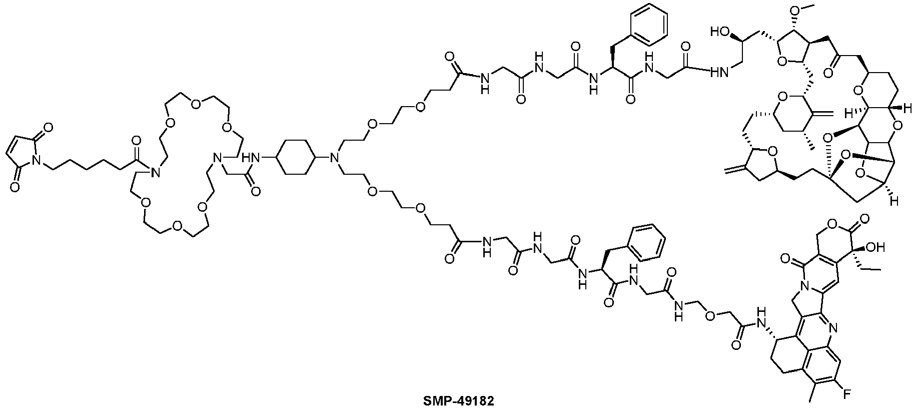

### Step 1: Preparation of compound SMP-49182-2

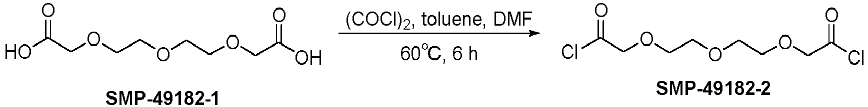

SMP-49182-1 (6.0 g, 27.03 mmol) and toluene (60 mL) were added to a 250 mL reaction flask, oxalyl chloride (13.7 g, 108.11 mol) was added under nitrogen protection in an ice bath, and then N,N-dimethylformamide (0.1 mL) was added dropwise. After adding, the raw materials were heated to 60°C and reacted for 6 hours, and then LC-MS showed that the raw materials almost reacted completely. The solvent and the excess oxalyl chloride were removed by direct distillation under reduced pressure, and 6.0 g of crude product was obtained for direct use in the next step.

### Step 2: Preparation of compound SMP-49182-4

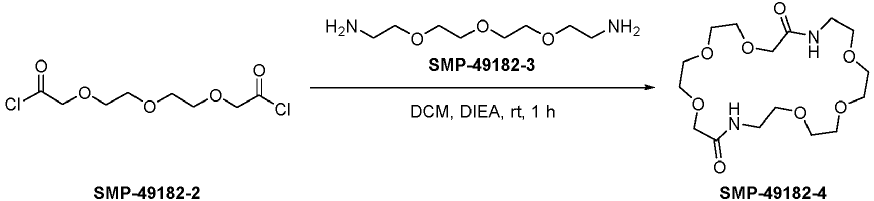

SMP-49182-3 (4.4 g, 23.16 mmol), N,N-diisopropylethylamine (11.9 g, 92.66 mmol), and dichloromethane (100 mL) were added to a 250 mL reaction flask. A dichloromethane suspension of the SMP-49182-2 (6.0 g, the crude product was mixed in 30 mL of dichloromethane) was slowly added dropwise in an ice-water bath. The solution was further stirred for 10 minutes and then slowly heated to room temperature, and the reaction was kept at room temperature for 1 hour. LC-MS showed the molecular weight of a target compound. The reaction system was concentrated. The crude product was purified by column chromatography (dichloromethane: methanol=10: 1) to obtain a compound SMP-49182-4, weighing 1.6 g, with a two-step yield of 16%.

MS (ESI) m/z: 379[M+H]⁺.

### Step 3: Preparation of compound SMP-49182-5

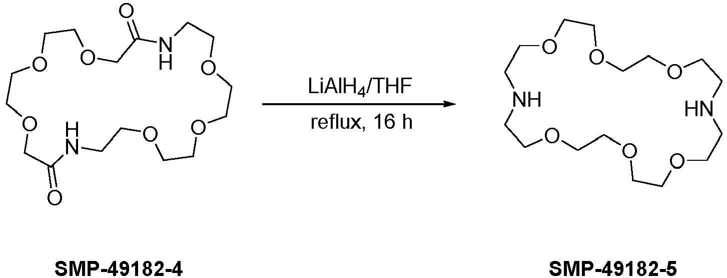

The intermediate SMP-49182-4 (1.2 g, 3.17 mmol) was added to a 100 mL reaction flask, then a tetrahydrofuran solution of 1M lithium aluminum hydride (30 mL) was added, a reflux reaction occurred in an oil bath for 16 hours, and then LC-MS showed that most was the molecular weight of a target compound. After the reaction solution was cooled to room temperature, sodium sulfate decahydrate (3.0 g) was added to the system, the reaction solution was further stirred for 30 minutes and filtered, the filtrate was concentrated under reduced pressure to remove the solvent to obtain a crude product, and the crude product was purified by column chromatography (ethyl acetate: methanol=5:1, added with 5% of ammonia) to obtain a compound SMP-49182-5, weighing 600 mg, with a yield of 53.9%.

MS (ESI) m/z: 351[M+H]⁺.

### Step 4: Preparation of compound SMP-49182-6

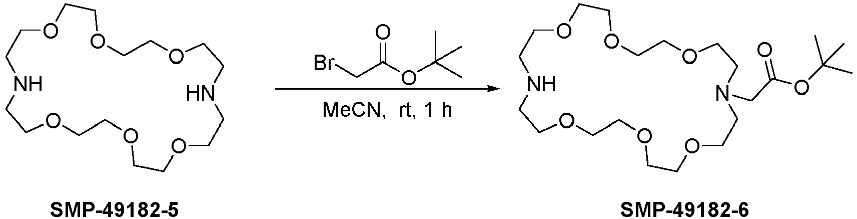

The intermediate SMP-49182-5 (0.6 g, 1.71 mmol), tert-butyl bromoacetate (0.3 g, 1.37 mmol), potassium carbonate (0.2 g, 1.37 mmol), and acetonitrile (10 mL) were added to a 100 mL reaction flask and reacted at room temperature for 1 hour, and then LC-MS showed that most was the molecular weight of a target compound. The reaction solution was filtered, the filtrate was concentrated under reduced pressure to obtain a crude product, and the crude product was purified by column chromatography (dichloromethane: methanol=10: 1) to obtain a compound SMP-49182-6, weighing 0.3 g, with a yield of 47.1%.

MS (ESI) m/z: 465[M+H]⁺.

### Step 5: Preparation of compound SMP-49182-7

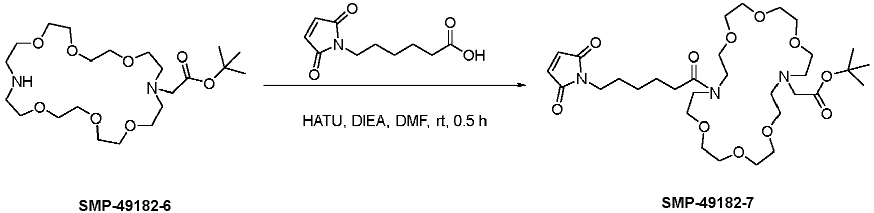

The intermediate SMP-49182-6 (110.0 mg, 0.23 mmol), N,N-dimethylformamide (5 mL), maleimidocaproic acid (60.00 mg, 0.28 mmol), N,N,N',N'-tetramethyl-O-(7-azabenzotriazol-1-yl)hexafluorophosphourea (108.0 mg, 0.28 mmol), and N,N-diisopropylethylamine (46.0 mg, 0.35 mmol) were sequentially added to a 10 mL reaction flask and reacted at room temperature for 30 minutes, and then LC-MS showed that most was the molecular weight of a target compound, The reaction solution was concentrated to obtain a crude product, and the crude product was purified by HPLC to obtain a compound SMP-49182-7, weighing 90.0 mg, with a yield of 59.5%.

MS (ESI) m/z: 658[M+H]⁺.

### Step 6: Preparation of compound SMP-49182-8

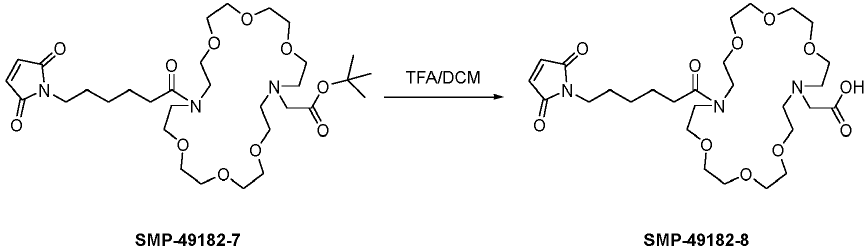

The SMP-49182-7 (90.0 mg, 0.13 mmol), dichloromethane (2 mL), and trifluoroacetic acid (2 mL) were sequentially added to a 10 mL reaction flask and reacted at room temperature for 20 minutes, and then LC-MS showed that most was the molecular weight of a target compound. The reaction solution was concentrated under reduced pressure to obtain a crude product, and the crude product was dissolved in a small amount of acetonitrile and purified by HPLC to obtain a compound SMP-49182-8, weighing 40.0 mg, with a yield of 51.1%. MS (ESI) m/z: 602[M+H]⁺.

### Step 7: Preparation of compound SMP-49182

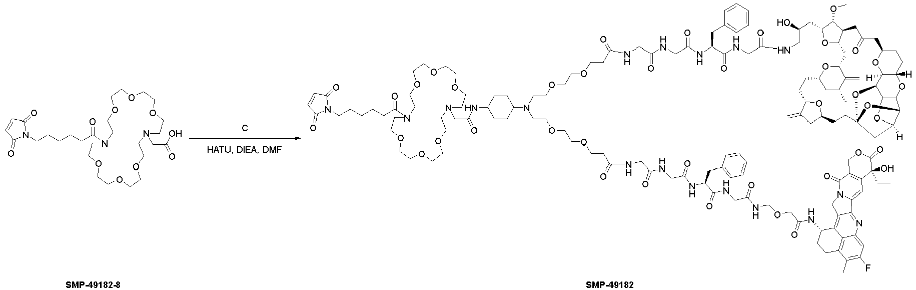

The SMP-49182-8 (2.5mg, 0.042 mmol), the intermediate C (8.0 mg, 0.0035 mmol), N,N-dimethylformamide (1 mL), N,N,N',N'-tetramethyl-O-(7-azabenzotriazol-1-yl)hexafluorophosphourea (1.6 mg, 0.0042 mmol), and N,N-diisopropylethylamine (0.7 mg, 0.053 mmol) were sequentially added to a 10 mL reaction flask and reacted at room temperature for 20 minutes, and then LC-MS showed that most was the molecular weight of a target compound. The reaction solution was concentrated under reduced pressure, a crude product was obtained after most of the solvent was removed, and the crude product was purified by HPLC to obtain a compound SMP-49182, weighing 6.4 mg, with a yield of 63.7%.

MS (ESI) m/z: 957[M/3+H]⁺.

### Example 20: Preparation of compound SMP-40359

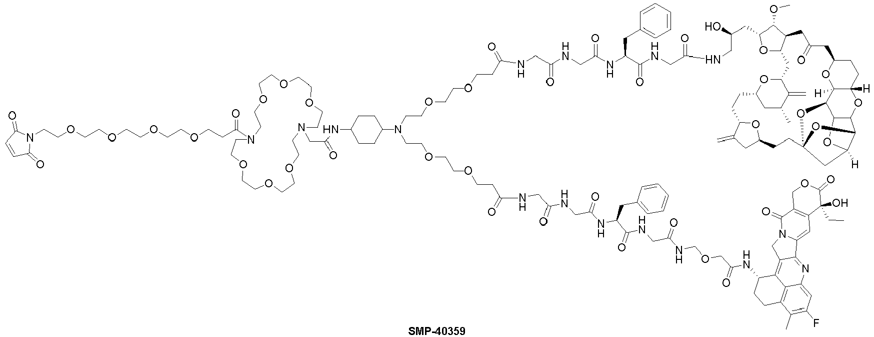

### Step 1: Preparation of compound SMP-40359-1

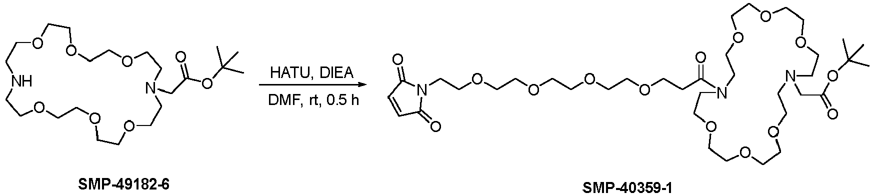

The intermediate SMP-49182-6 (120.0 mg, 0.26 mmol), Mal-PEG4-acid (107.0 mg, 0.31 mmol), N,N-dimethylformamide (5 mL), N,N,N',N'-tetramethyl-O-(7-azabenzotriazol-1-yl)hexafluorophosphourea (118.0 mg, 0.31 mmol), and N,N-diisopropylethylamine (50.0 mg, 0.38 mmol) were sequentially added to a 10 mL reaction flask and reacted at room temperature for 30 minutes, and then LC-MS showed that most was the molecular weight of a target compound. The reaction solution was concentrated under reduced pressure to obtain a crude product, and the crude product was purified by HPLC to obtain a compound SMP-40359-1, weighing 80.0 mg, with a yield of 38.9%.

MS (ESI) m/z: 792[M+H]⁺.

### Step 2: Preparation of compound SMP-40359-2

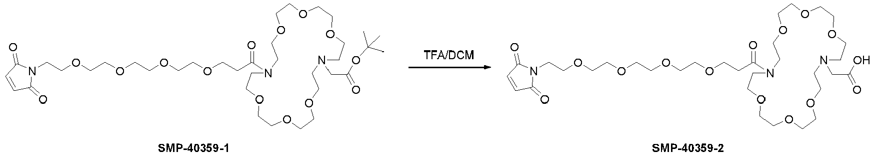

The SMP-40359-1 (80.0 mg, 0.10 mmol), dichloromethane (2 mL), and trifluoroacetic acid (2 mL) were sequentially added to a 10 mL reaction flask and reacted at room temperature for 20 minutes, and then LC-MS showed that most was the molecular weight of a target compound. The reaction solution was concentrated under reduced pressure to obtain a crude product, and the crude product was dissolved in a small amount of acetonitrile and purified by HPLC to obtain a compound SMP-40359-2, weighing 40.0 mg, with a yield of 54.4%. MS (ESI) m/z: 736[M+H]⁺.

### Step 7: Preparation of compound SMP-40359

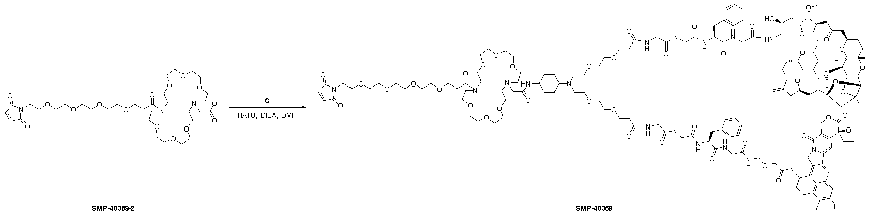

The SMP-40359-2 (5.8mg, 0.0078 mmol), the intermediate C (15.0 mg, 0.0066 mmol), N,N-dimethylformamide (1 mL), N,N,N',N'-tetramethyl-O-(7-azabenzotriazol-1-yl)hexafluorophosphourea (3.0mg, 0.0078 mmol), and N,N-diisopropylethylamine (1.3mg, 0.0098 mmol) were sequentially added to a 10 mL reaction flask and reacted at room temperature for 30 minutes, and then LC-MS showed that most was the molecular weight of a target compound. The reaction solution was concentrated under reduced pressure to obtain a crude product, and the crude product was purified by HPLC to obtain a compound SMP-49182, weighing 11.6 mg, with a yield of 58.5%.

MS (ESI) m/z: 1002[M/3+H]⁺.

### Example 21: Preparation of compound SMP-93566

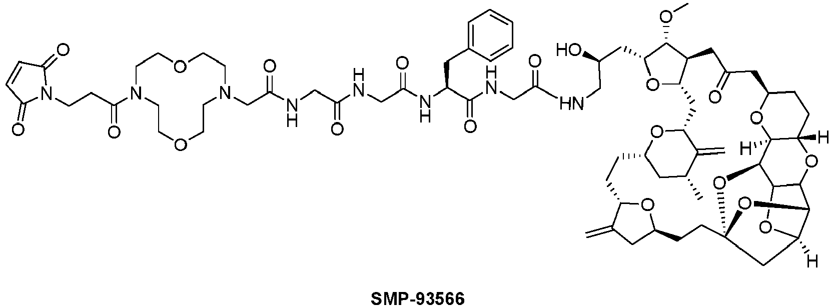

### Step 1: Preparation of compound SMP-93566-2

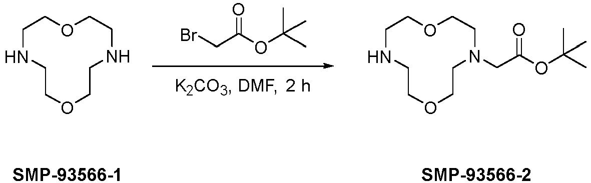

An intermediate SMP-93566-1 (1.0 g, 5.75 mmol), tert-butyl bromoacetate (0.9 g, 4.60 mmol), potassium carbonate (0.6 g, 4.60 mmol), and N,N-dimethylformamide (20 mL) were sequentially added to a 100 mL reaction flask and reacted at room temperature for 2 hours, and then LC-MS showed that most was the molecular weight of a target compound. The reaction solution was filtered, the filtrate was concentrated under reduced pressure to obtain a crude product, and the crude product was purified by column chromatography (dichloromethane: methanol=12: 1) to obtain a compound SMP-93566-2, weighing 0.2 g, with a yield of 15.1%. MS (ESI) m/z: 289[M+H]⁺.

¹HNMR (400 MHz, CDCl3) δ5.91 (s, 1H), 3.40-3.49(m, 8H), 323 (s, 2H), 2.54(m, 4H), 2.70(m, 4H),1.33 (s, 9H).

### Step 2: Preparation of compound SMP-93566-3

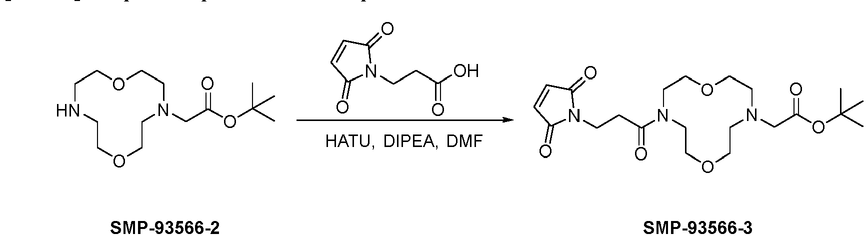

The intermediate SMP-93566-2 (200.0 mg, 0.69 mmol), maleimidopropionic acid (140.8 mg, 0.83 mmol), N,N-dimethylformamide (5 mL), N,N,N',N'-tetramethyl-O-(7-azabenzotriazol-1-yl)hexafluorophosphourea (315.4 mg, 0.83 mmol), and N,N-diisopropylethylamine (134.2 mg, 1.04 mmol) were sequentially added to a 10 mL reaction flask and reacted at room temperature for 30 minutes, and then LC-MS showed that most was the molecular weight of a target compound. The reaction solution was concentrated under reduced pressure to obtain a crude product, and the crude product was purified by HPLC to obtain a compound SMP-93566-3, weighing 220.0 mg, with a yield of 72.6%.

MS (ESI) m/z: 440[M+H]⁺.

### Step 3: Preparation of compound SMP-93566-4

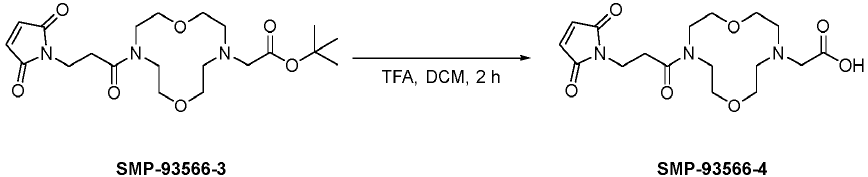

The SMP-93566-3 (220.0 mg, 0.50 mmol), dichloromethane (5 mL), and trifluoroacetic acid (5 mL) were sequentially added to a 25mL reaction flask and reacted at room temperature for 2 hours, and then LC-MS showed that most was the molecular weight of a target compound. The reaction solution was concentrated under reduced pressure to obtain a crude product, and the crude product was dissolved in a small amount of acetonitrile and purified by HPLC to obtain a compound SMP-93566-4, weighing 90.0 mg, with a yield of 47.0%. MS (ESI) m/z: 384[M+H]⁺.

### Step 4: Preparation of compound SMP-93566

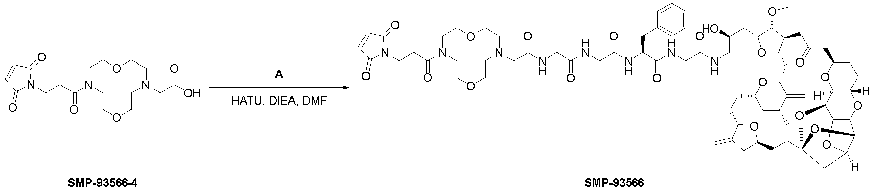

The SMP-93566-4 (8.8mg, 0.023 mmol), the intermediate A (20 mg, 0.019 mmol), N,N-dimethylformamide (2 mL), N,N,N',N'-tetramethyl-O-(7-azabenzotriazol-1-yl)hexafluorophosphourea (8.7mg, 0.023 mmol), and N,N-diisopropylethylamine (3.7mg, 0.029 mmol) were sequentially added to a 10 mL reaction flask and reacted at room temperature for 20 minutes, and then LC-MS showed that most was the molecular weight of a target compound. The reaction solution was concentrated under reduced pressure to obtain a crude product, and the crude product was purified by HPLC to obtain a compound SMP-93566, weighing 18.0 mg, with a yield of 67.0%.

MS (ESI) m/z: 1414[M+H]⁺, 707[M/2+H]⁺.

¹H NMR (400 MHz, DMSO-*d*₆) δ 8.89 (s, 1H), 8.29 (t, *J* = 5.6 Hz, 1H), 8.19 (d, *J* = 8.2 Hz, 1H), 8.12 (t, *J* = 5.6 Hz, 1H), 7.95 (s, 1H), 7.72 (s, 1H), 7.31 - 7.09 (m, 6H), 6.98 (s, 2H), 5.02 (d, *J* = 21.4 Hz, 2H), 4.82 (s, 1H), 4.75 (s, 1H), 4.63 (s, 1H), 4.58 - 4.48 (m, 2H), 4.28 - 4.06 (m, 10H), 3.87 - 3.47 (m, 30H), 3.16 - 2.99 (m, 5H), 2.85 - 2.64 (m, 6H), 2.62 - 2.53 (m, 2H), 2.34 - 2.16 (m, 6H), 2.01 - 1.87 (m, 6H), 1.73 - 1.58 (m, 6H), 1.31 (s, 3H), 1.19 (m, 1H), 1.03 (d, *J* = 6.3 Hz, 3H), 1.00 - 0.91 (m, 1H).

### Example 22: Preparation of compound SMP-88480

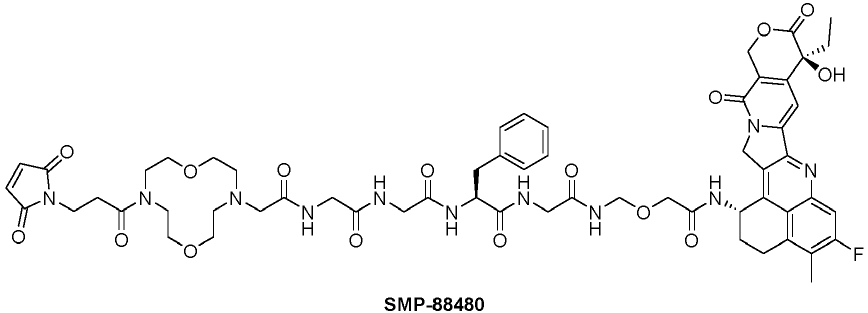

### Step 1: Preparation of compound SMP-88480

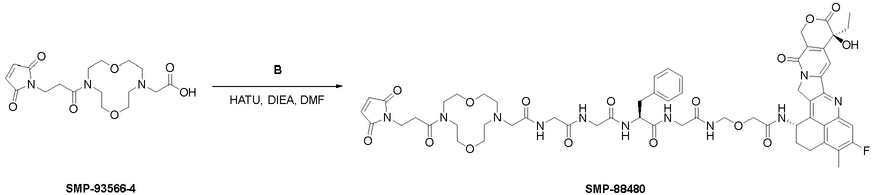

The SMP-93566-4 (9.8mg, 0.026 mmol), the intermediate B (18.0 mg, 0.021 mmol), N,N-dimethylformamide (2 mL), N,N,N',N'-tetramethyl-O-(7-azabenzotriazol-1-yl)hexafluorophosphourea (9.8mg, 0.026 mmol), and N,N-diisopropylethylamine (4.1 mg, 0.032 mmol) were sequentially added to a 10 mL reaction flask and reacted at room temperature for 30 minutes, and then LC-MS showed that most was the molecular weight of a target compound. The reaction solution was concentrated under reduced pressure to obtain a crude product, and the crude product was purified by HPLC to obtain a compound SMP-88480, weighing 12.0 mg, with a yield of 47.4%.

MS (ESI) m/z: 1206[M+H]⁺, 603[M/2+H]⁺.

¹HNMR (400 MHz, DMSO-*d₆*) δ 8.65 (s, 1H), 8.51 (d, J = 8.5 Hz, 1H), 8.33 (d, J = 24.8 Hz, 1H), 8.22 - 7.92 (m, 3H), 7.77 (d, J = 10.9 Hz, 1H), 7.30 (s, 1H), 7.28 - 7.12 (m, 5H), 6.98 (s, 2H), 6.53 (s, 1H), 5.67 - 5.54 (m, 1H), 5.49 - 5.32 (m, 2H), 5.18 (s, 2H), 4.64 (d, J = 6.5 Hz, 2H), 4.48 (s, 1H), 4.13 (s, 1H), 4.02 (s, 2H), 3.90 (s, 1H), 3.61 (dd, J = 41.9, 31.1 Hz, 15H), 3.39 (m, 5H), 3.17 (dd, J = 35.2, 18.2 Hz, 4H), 3.01 (dd, J = 13.6, 4.2 Hz, 1H), 2.71 (dd, J = 41.8, 13.0 Hz, 5H), 2.37 (s, 3H), 2.18 (d, J = 5.4 Hz, 2H), 1.92 - 1.74 (m, 2H), 0.86 (t, J = 7.3 Hz, 3H).

### Example 23: Preparation of compound SMP-34048

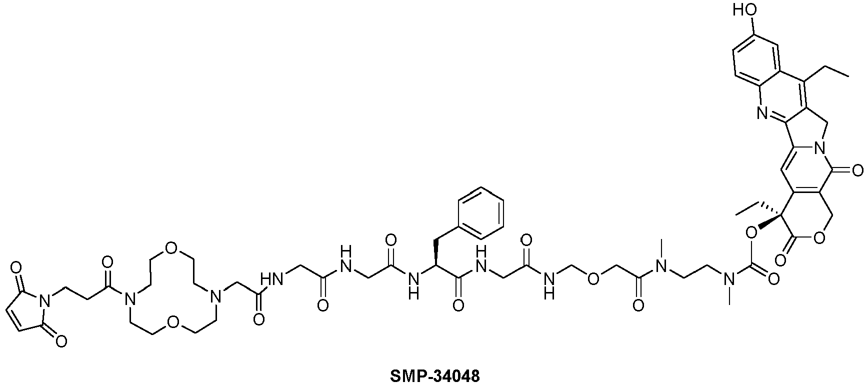

### Step 1: Preparation of compound SMP-34048-2

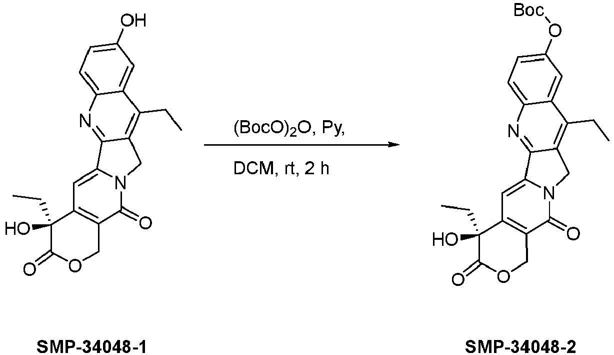

SMP-34048-1 (1.0 g, 2.55 mmol) and dichloromethane (75 mL) were added to a 250 mL reaction flask. Di-tert-butyl dicarbonate (0.7 g, 3.32 mmol) and pyridine (6.1 mL, 75 mmol) were added, and a reaction occurred at room temperature for 2 hours while stirring. TLC showed complete reaction. The reaction solution was sequentially washed with 0.5 N hydrochloric acid aqueous solution (20 mL * 3) and with saturated sodium carbonate (20 mL * 3), the organic phase was dried with anhydrous sodium sulfate and filtered, and the filtrate was concentrated under reduced pressure to obtain a crude product SMP-34048-1, weighing 1.2 g, with a yield of 95.6%.

MS (ESI) m/z: 493 [M+H]⁺.

### Step 2: Preparation of compound SMP-34048-3

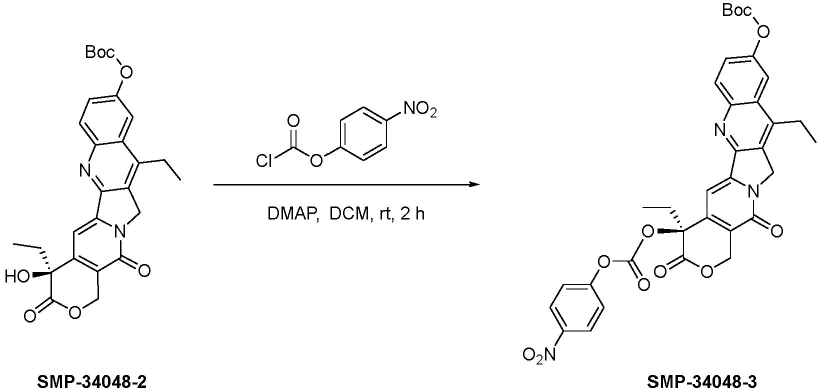

SMP-34048-2 (1.2 g, 2.44 mmol) and dichloromethane (100 mL) were added to a 250 mL reaction flask and stirred at room temperature, then p-nitrophenyl chloroformate (0.5 g, 2.68 mmol) was added in batches, 4-dimethylaminopyridine (0.3 g, 2.44 mmol) was finally added, and a reaction occurred for 2 hours while the stirring continued under the condition. TLC showed complete reaction. The reaction solution was concentrated under reduced pressure to obtain a crude product, and the crude product was purified by column chromatography (dichloromethane: methanol=15:1) to obtain SMP-34048-3, weighing 360.0 mg, with a yield of 22.4%.

MS (ESI) m/z: 658[M+H]⁺.

### Step 3: Preparation of compound SMP-34048-4

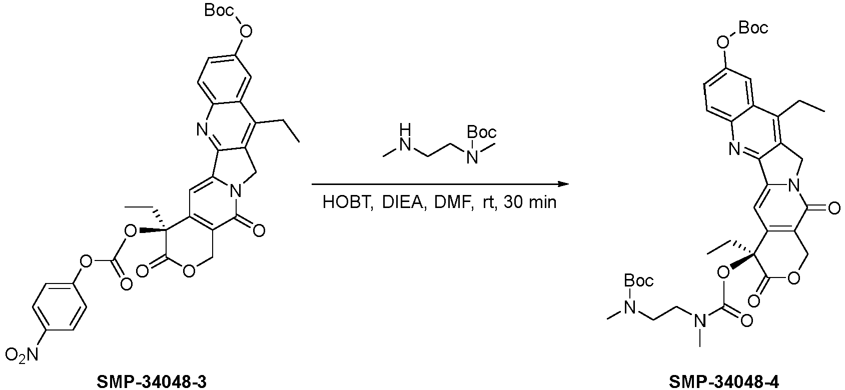

The SMP-34048-3 (360.0 mg, 0.55 mmol), methyl(2-(methylamino)ethyl)tert-butyl carbamate (155.0 mg, 0.82 mmol), N,N-dimethylformamide (5 mL), 1-hydroxybenzotriazole (74.2 mg, 0.55 mmol), and N,N-diisopropylethylamine (140.6 mg, 1.09 mmol) were sequentially added to a 50 mL reaction flask. A reaction occurred at room temperature for 30 minutes while stirring. TLC showed complete reaction. The reaction solution was concentrated under reduced pressure to obtain a crude product, and the crude product was purified by column chromatography (dichloromethane: methanol=12: 1) to obtain SMP-34048-4, weighing 300.0 mg, with a yield of 77.2%.

MS (ESI) m/z: 707[M+H]⁺.

### Step 4: Preparation of compound SMP-34048-5

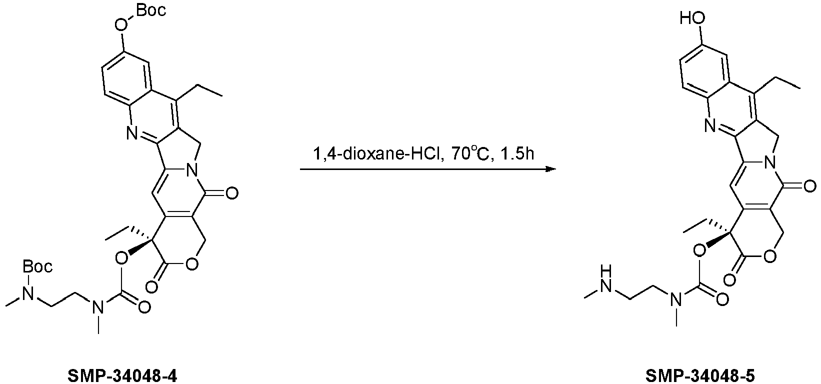

The SMP-34048-4 (300.0 mg, 0.42 mmol) and a dioxane hydrochloride solution (10 mL) were sequentially added to a 250 mL reaction flask and stirred in an oil bath at 70°C for reaction for 1.5 hours. TLC showed complete reaction. The reaction solution was cooled to room temperature and then concentrated under reduced pressure to obtain a crude product SMP-34048-5, weighing 200.0 mg, with a yield of 94.1%.

MS (ESI) m/z: 507[M+H]⁺.

### Step 5: Preparation of compound SMP-34048-6

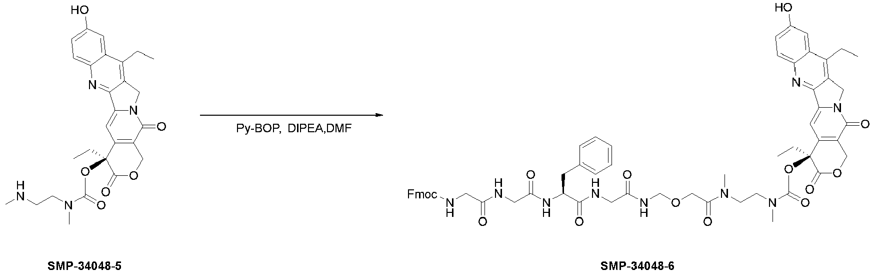

An intermediate B-1 (225.7 mg, 0.35 mmol), 1H-benzotriazol-1-yloxytripyrrolidyl hexafluorophosphate (182.0 mg, 0.35 mmol), N,N-dimethylformamide (5 mL), and N,N-diisopropylethylamine (59.8 mg, 0.46 mmol) were sequentially added to a 25 mL reaction flask and stirred at room temperature for reaction for 5 minutes, then the SMP-34048-5 (150.0 mg, 0.30 mmol) was added, the reaction solution was further stirred for 25 minutes, and then TLC detected complete reaction. The reaction solution was distilled under reduced pressure to remove the N,N-dimethylformamide, and the residue was purified by column chromatography HPLC (dichloromethane: methanol=10: 1) to obtain a compound SMP-34048-6, weighing 200.0 mg, with a yield of 58.8%.

MS (ESI) m/z: 1134[M+H]⁺, 567[M/2+H]⁺.

### Step 6: Preparation of compound SMP-34048-7

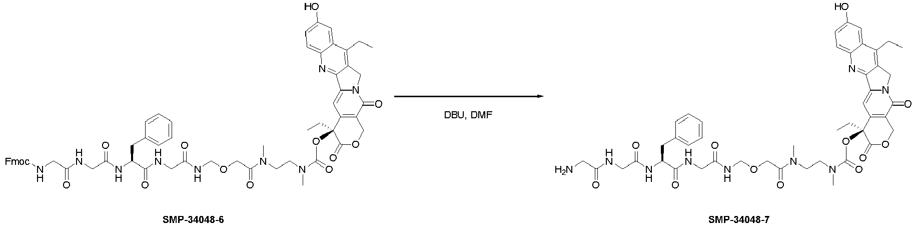

The SMP-34048-6 (200.0 mg, 0.18 mmol), N,N-dimethylformamide (2 mL), and 1,8-diazabicyclo[5.4.0]undec-7-ene (40.2 mg, 0.26 mmol) were sequentially added to a 25 mL reaction flask and reacted at room temperature for 30 minutes, and then TLC monitored complete reaction. The reaction solution was concentrated under reduced pressure to remove the N,N-dimethylformamide, and the residue was purified by HPLC to obtain a compound SMP-34048-7, weighing 100.0 mg, with a yield of 61.0%.

MS (ESI) m/z: 912[M+H]⁺.

### Step 7: Preparation of compound SMP-34048

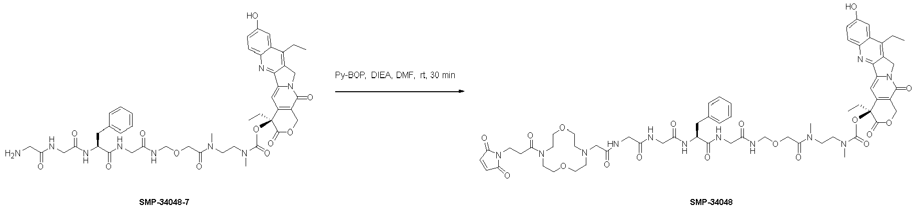

The SMP-93566-4 (9.2 mg, 0.024 mmol), the SMP-34048-7 (18 mg, 0.020 mmol), N,N-dimethylformamide (2 mL), 1H-benzotriazol-1-yloxytripyrrolidyl hexafluorophosphate (12.5 mg, 0.024 mmol), and N,N-diisopropylethylamine (3.9 mg, 0.030 mmol) were sequentially added to a 10 mL reaction flask and reacted at room temperature for 30 minutes, and then LC-MS showed that most was the molecular weight of a target compound. The reaction solution was concentrated under reduced pressure to obtain a crude product, and the crude product was purified by HPLC to obtain a compound SMP-34048, weighing 8.0 mg, with a yield of 31.3%.

MS (ESI) m/z: 1277[M+H]⁺, 639[M/2+H]⁺.

### Example 24: Preparation of compound SMP-97962

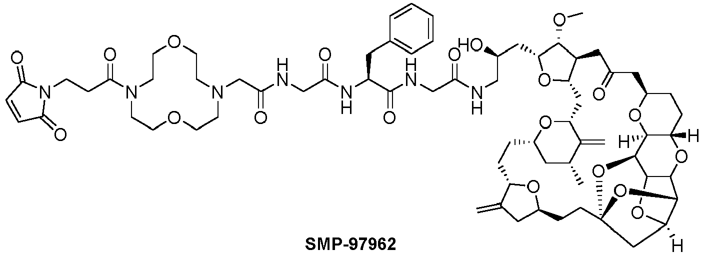

### Step 1: Preparation of compound SMP-97962

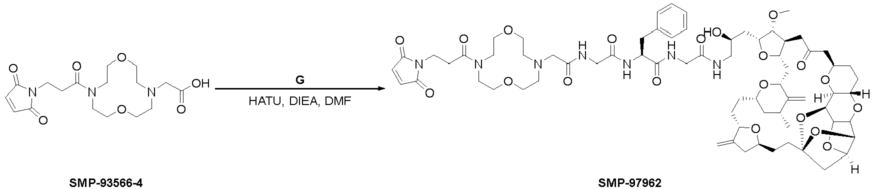

The SMP-93566-4 (4.6 mg, 0.012 mmol), the intermediate G (10 mg, 0.010 mmol), N,N-dimethylformamide (2 mL), N,N,N',N'-tetramethyl-O-(7-azabenzotriazol-1-yl)hexafluorophosphourea (4.6 mg, 0.012 mmol), and N,N-diisopropylethylamine (2.6 mg, 0.020 mmol) were sequentially added to a 10 mL reaction flask and reacted at room temperature for 20 minutes, and then LC-MS showed that most was the molecular weight of a target compound. The reaction solution was concentrated under reduced pressure to obtain a crude product, and the crude product was purified by HPLC to obtain a compound SMP-97962, weighing 5.0 mg, with a yield of 36.8%.

MS (ESI) m/z: 679[M/2+H]⁺.

¹HNMR (400 MHz, DMSO-*d*₆) δ 8.79 (s, 1H), 8.30 (d, *J* = 5.2 Hz, 2H), 7.96 (s, 1H), 7.73 (s, 1H), 7.26 - 7.19 (m, 4H), 7.17 (t, *J* = 6.3 Hz, 1H), 6.99 (s, 2H), 5.02 (d, *J* = 21.6 Hz, 2H), 4.79 (d, *J* = 29.1 Hz, 2H), 4.63 (s, 1H), 4.59 - 4.51 (m, 2H), 4.26 (d, *J* = 11.4 Hz, 1H), 4.19 - 4.05 (m, 6H), 4.02 (s, 1H), 3.92 (m, 1H), 3.84 - 3.46 (m, 28H), 3.22 - 2.98 (m, 6H), 2.75 (ddd, *J* = 20.7, 18.1, 5.9 Hz, 5H), 2.61 - 2.53 (m, 1H), 2.34 - 2.16 (m, 6H), 2.02 - 1.88 (m, 6H), 1.73 - 1.56 (m, 6H), 1.49 (d, *J* = 12.7 Hz, 2H), 1.39 - 1.13 (m, 5H), 1.03 (d, *J* = 6.3 Hz, 3H), 1.00 - 0.87 (m, 1H).

### Example 25: Preparation of compound SMP-00171

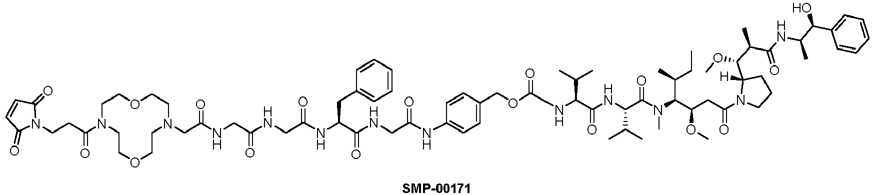

### Step 1: Preparation of compound SMP-00171

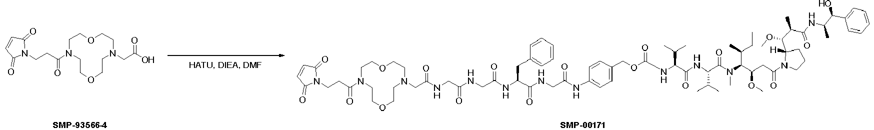

SMP-93566-4 (19.4 mg, 0.051 mmol), SMP-58054-2 (50.0 mg, 0.042 mmol), N,N-dimethylformamide (4 mL), N,N,N',N'-tetramethyl-O-(7-azabenzotriazol-1-yl)hexafluorophosphourea (19.4 mg, 0.051 mmol), and N,N-diisopropylethylamine (8.2 mg, 0.064 mmol) were sequentially added to a 10 mL reaction flask and reacted at room temperature for 20 minutes, and then LC-MS showed that most was the molecular weight of a target compound. The reaction solution was concentrated under reduced pressure to obtain a crude product, and the crude product was purified by HPLC to obtain a compound SMP-00171, weighing 59.0 mg, with a yield of 90.6%.

MS (ESI) m/z: 776[M/2+H]⁺.

### Example 26: Preparation of compound SMP-24600

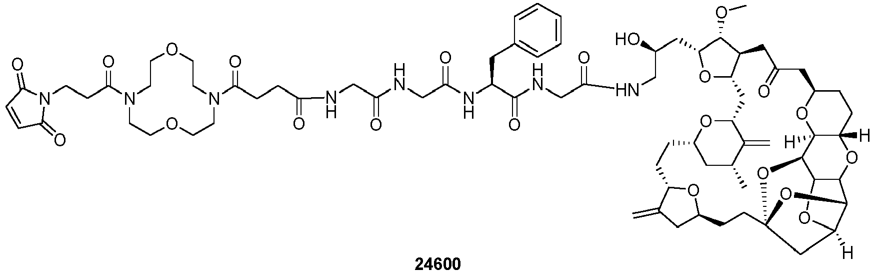

### Step 1: Preparation of compound SMP-24600-1

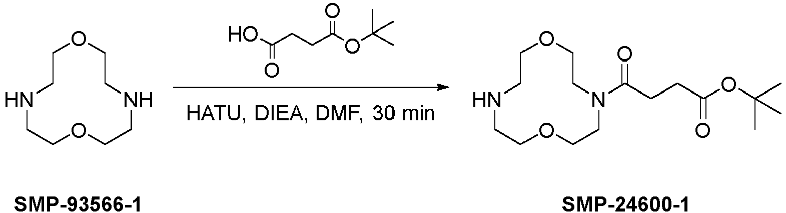

SMP-93566-1 (100.0 mg, 0.57 mmol), mono-tert-butyl succinate (99.2 mg, 0.57 mmol), N,N-dimethylformamide (5 mL), and N,N-diisopropylethylamine (89.0 mg, 0.69 mmol) were sequentially added to a 10 mL reaction flask, N,N,N',N'-tetramethyl-O-(7-azabenzotriazol-1-yl)hexafluorophosphourea (197.6 mg, 0.52 mmol, dissolved in 1 mL of N,N-dimethylformamide) was slowly added dropwise at room temperature, then the reaction continued for 30 minutes, and LC-MS showed that most was the molecular weight of a target compound. The reaction solution was concentrated under reduced pressure to obtain a crude product, and the crude product was purified by HPLC to obtain a compound SMP-24600-1, weighing 120.0 mg, with a yield of 70.0%.

MS (ESI) m/z: 331[M+H]⁺.

¹HNMR (400 MHz, CDCl3) δ5.51 (s, 1H), 3.49-3.60(m, 8H), 3.23 (m, 4H), 2.49-2.65(m, 8H),1.35 (s, 9H).

### Step 2: Preparation of compound SMP-24600-2

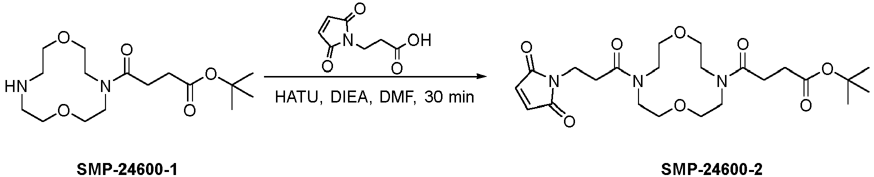

The SMP-24600-1 (120.0 mg, 0.36 mmol), maleimidopropionic acid (73.7 mg, 0.44 mmol), N,N-dimethylformamide (3 mL), N,N,N',N'-tetramethyl-O-(7-azabenzotriazol-1-yl)hexafluorophosphourea (165.8 mg, 0.44 mmol), and N,N-diisopropylethylamine (71.0 mg, 0.55 mmol) were sequentially added to a 10 mL reaction flask and reacted at room temperature for 20 minutes, and then LC-MS showed that most was the molecular weight of a target compound. The reaction solution was concentrated under reduced pressure to obtain a crude product, and the crude product was purified by HPLC to obtain a compound SMP-24600-2, weighing 95.0 mg, with a yield of 54.8%.

MS (ESI) m/z: 482[M+H]⁺.

### Step 3: Preparation of compound SMP-24600-3

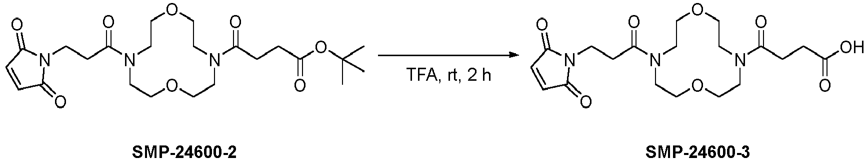

The SMP-24600-2 (55.0 mg, 0.11 mmol) and trifluoroacetic acid (3 mL) were sequentially added to a 25 mL reaction flask and reacted at room temperature for 2 hours while stirring, and then LC-MS showed complete reaction. The reaction solution was concentrated under reduced pressure, and the residue was purified by HPLC to obtain a compound SMP-24600-3, weighing 25.0 mg, with a yield of 53.5%.

MS (ESI) m/z: 426[M+H]⁺.

### Step 4: Preparation of compound SMP-24600

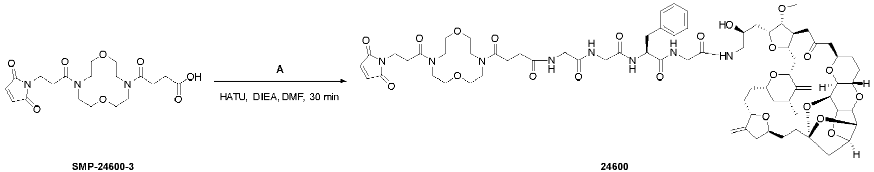

The SMP-24600-3 (7.2 mg, 0.017 mmol), the intermediate A (15.0 mg, 0.014 mmol), N,N-dimethylformamide (2 mL), N,N,N',N'-tetramethyl-O-(7-azabenzotriazol-1-yl)hexafluorophosphourea (6.5 mg, 0.017 mmol), and N,N-diisopropylethylamine (2.7 mg, 0.021 mmol) were sequentially added to a 10 mL reaction flask and reacted at room temperature for 30 minutes, and then LC-MS showed that most was the molecular weight of a target compound. The reaction solution was concentrated under reduced pressure to obtain a crude product, and the crude product was purified by HPLC to obtain a compound SMP-24600, weighing 12.0 mg, with a yield of 59.0%.

MS (ESI) m/z: 728[M/2+H]⁺.

### Example 27: Preparation of compound SMP-36297

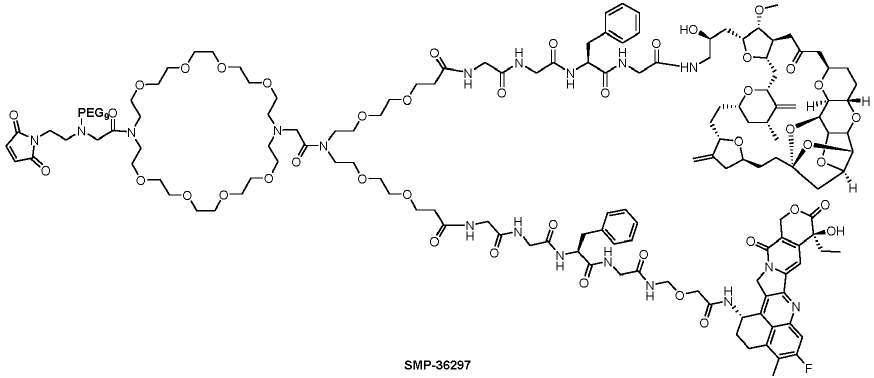

### Step 1: Preparation of compound SMP-36297-1

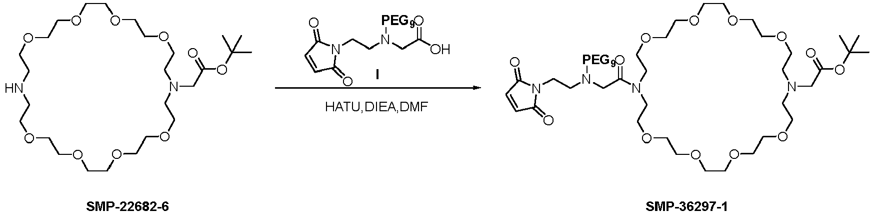

SMP-22682-6 (55.0 mg, 0.10 mmol), intermediate I (73.0 mg, 0.12 mmol), N,N-dimethylformamide (2 mL), N,N,N',N'-tetramethyl-O-(7-azabenzotriazol-1-yl)hexafluorophosphourea (45.6 mg, 0.12 mmol), and N,N-diisopropylethylamine (19.4 mg, 0.15 mmol) were sequentially added to a 10 mL reaction flask and reacted at room temperature for 20 minutes, and then LC-MS showed that most was the molecular weight of a target compound. The reaction solution was concentrated under reduced pressure to obtain a crude product, and the crude product was purified by HPLC to obtain a compound SMP-36297-1, weighing 50.0 mg, with a yield of 43.7%.

MS (ESI) m/z: 572[M/2+H]⁺.

### Step 2: Preparation of compound SMP-36297-2

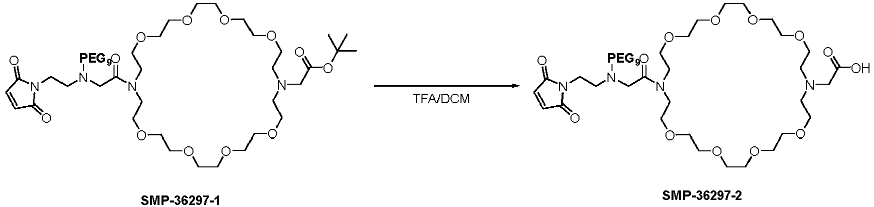

The intermediate SMP-36297-1 (50 mg, 0.044 mmol), dichloromethane (2 mL), and trifluoroacetic acid (2 mL) were sequentially added to a 25 mL reaction flask and reacted at room temperature for 2 hours while stirring, and then LC-MS showed complete reaction. The reaction solution was concentrated under reduced pressure, and the residue was purified by reversed-phase HPLC to obtain a compound SMP-36297-2, weighing 30.0 mg, with a yield of 62.7%.

MS (ESI) m/z: 543.54[M/2+H]⁺.

### Step 3: Preparation of compound SMP-36297

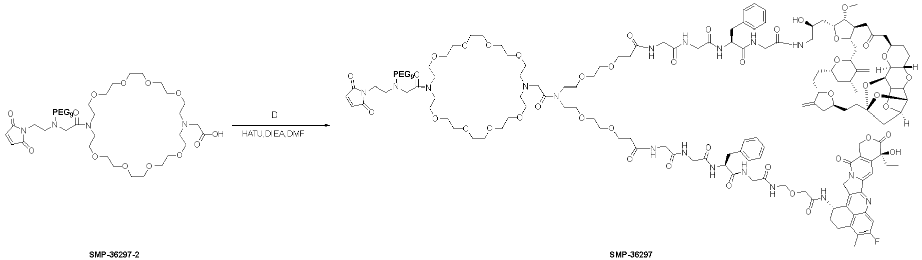

The SMP-36297-2 (6.0 mg, 0.055 mmol), the intermediate D (10 mg, 0.0045 mmol), N,N-dimethylformamide (1 mL), N,N,N',N'-tetramethyl-O-(7-azabenzotriazol-1-yl)hexafluorophosphourea (2.1 mg, 0.0055 mmol), and N,N-diisopropylethylamine (0.9 mg, 0.0068 mmol) were sequentially added to a 10 mL reaction flask and reacted at room temperature for 20 minutes, and then LC-MS showed that most was the molecular weight of a target compound. The reaction solution was concentrated under reduced pressure to obtain a crude product, and the crude product was purified by HPLC to obtain a compound SMP-36297, weighing 6.6 mg, with a yield of 45.0%.

MS (ESI) m/z: 1087[M/3+H]⁺, 815[M/4+H]⁺.

### Example 28: Preparation of compound SMP-12395

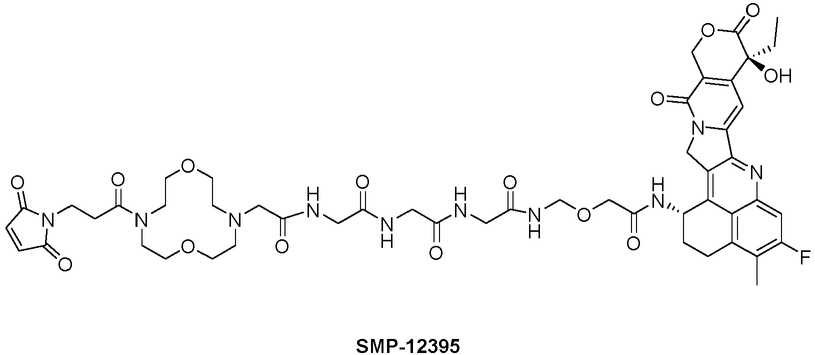

### Step 1: Preparation of compound SMP-12395-2

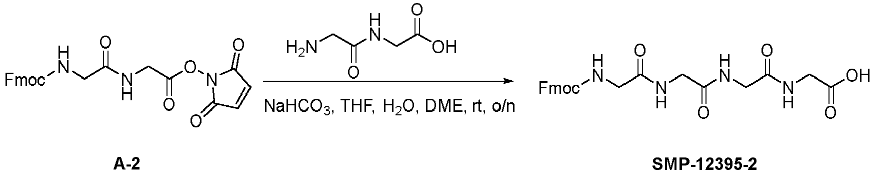

An intermediate glycylglycine (644.0 mg, 4.80 mmol), sodium bicarbonate (806.4, 9.60 mmol), water (2 mL), and tetrahydrofuran (20 mL) were added to a 100 mL reaction flask and stirred at room temperature. After the solution was completely clarified, the compound A-2 (2.2 g, 4.80 mmol) was dissolved in ethylene glycol dimethyl ether (5 mL) and slowly added to the reaction solution. The stirring continued for reaction overnight. TLC showed complete reaction. The reaction solution was concentrated under reduced pressure, and the residue was dropped into a 0.5 M hydrochloric acid aqueous solution (200 mL) to precipitate a large amount of solid. After filtration, the obtained solid was dried in vacuum to obtain a compound SMP-12395-2, weighing 1.5 g, with a yield of 66.7%. MS (ESI) m/z: 469 [M+H]⁺.

¹HNMR (400 MHz, CDCl3) δ13.01 (s, 1H), 9.01 (s, 1H),8.76 (s, 1H),8.55 (s, 1H),8.43 (s, 1H), 7.90 (d, 2H), 7.55 (d, 2H), 7.28-7.38(m, 4H), 4.71 (d, 2H), 4.45 (t, 1H), 3.85-4.01(m, 8H).

### Step 2: Preparation of compound SMP-12395-3

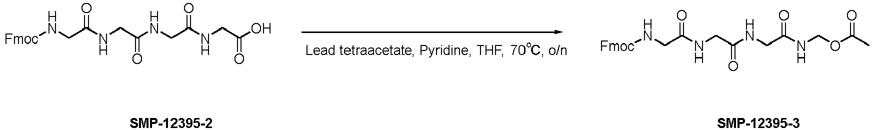

The compound SMP-12395-2 (420.0 mg, 0.90 mmol) and a solvent tetrahydrofuran (5 mL) were added to a 25 mL reaction flask and stirred at room temperature. After the solution was completely clarified, lead tetraacetate (480.0 mg, 1.07 mmol) and pyridine (107.2 mg, 1.34 mmol) were sequentially added, a reaction occurred at 70°C overnight while stirring, and then TLC showed complete reaction. The reaction solution was cooled to room temperature and then filtered, the filtrate was concentrated under reduced pressure, and the residue was purified by column chromatography (dichloromethane: ethyl acetate=2:1) to obtain an intermediate SMP-12395-3, weighing 290.0 mg, with a yield of 67.0%. MS (ESI) m/z: 482 [M+H]⁺.

¹HNMR (400 MHz, CDCl3) δ9.04 (s, 1H),8.86 (s, 1H),8.75 (s, 1H),8.23 (s, 1H), 7.87 (d, 2H), 7.52(d, 2H), 7.26-7.39(m, 4H), 5.99 (s, 2H), 4.73(d, 2H), 4.41(t, 1H), 3.83-4.02(m, 8H), 2.11 (s, 3H).

### Step 3: Preparation of compound SMP-12395-4

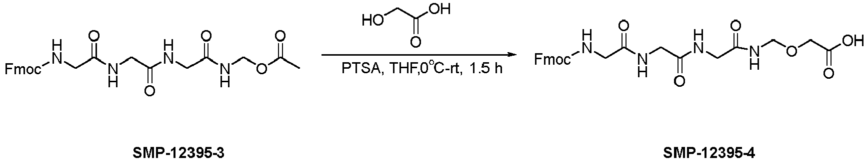

The compound SMP-12395-3 (290.0 mg, 0.60 mmol), a solvent tetrahydrofuran (25 mL), and hydroxyacetic acid (50.0 mg, 0.66 mmol) were added to a 100 mL reaction flask. After the reaction solution was clarified, p-toluenesulfonic acid (51.0 mg, 0.30 mmol) was added at a time at 0°C, a reaction occurred at room temperature for 1.5 hours while stirring, and then TLC showed complete reaction. After filtration and concentration under reduced pressure to remove excess tetrahydrofuran, the residue was purified by HPLC to obtain an intermediate SMP-12395-4, weighing 190.0 mg, with a yield of 63%.

MS (ESI) m/z: 499 [M+H]⁺.

### Step 4: Preparation of compound SMP-12395-5

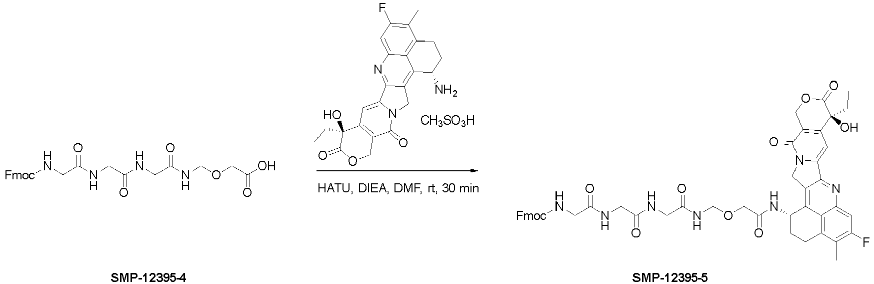

The intermediate SMP-12395-4 (168.0 mg, 0.34 mmol), N,N,N',N'-tetramethyl-O-(7-azabenzotriazol-1-yl)hexafluorophosphourea (129.0 mg, 0.34 mmol), N,N-diisopropylethylamine (55.0 mg, 0.43 mmol), and N,N-dimethylformamide (8 mL) were added to a 25 mL reaction flask and reacted at room temperature for 5 minutes while stirring, then exatecan mesylate (150.0 mg, 0.28 mmol) was added, the stirring continued for 25 minutes, and then TLC showed complete reaction. The N,N-dimethylformamide was removed by concentration under reduced pressure, and the residue was purified by thin layer chromatography (dichloromethane: methanol=8:1) to obtain a compound SMP-12395-5, weighing 200.0 mg, with a yield of 78%.

MS (ESI) m/z: 916 [M+H]⁺.

### Step 5: Preparation of compound SMP-12395-6

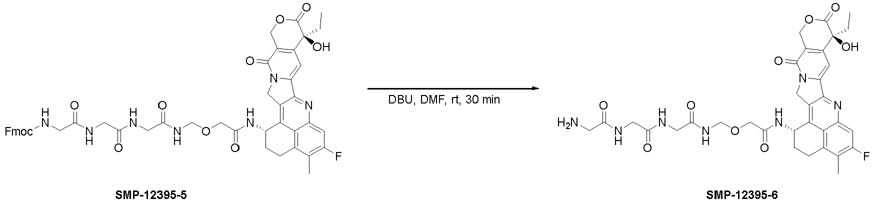

The intermediate SMP-12395-5 (120.0 mg, 0.13 mmol), 1,8-diazabicyclo[5.4.0]undec-7-ene (40.0 mg, 0.26 mmol), and N,N-dimethylformamide (3 mL) were added to a 25 mL reaction flask and reacted at room temperature for 30 minutes while stirring, and then TLC showed complete reaction. The reaction solution was concentrated under reduced pressure, and the residue was purified by HPLC to obtain a compound SMP-12395-6, weighing 65.0 mg, with a yield of 72%. MS (ESI) m/z: 694 [M+H]⁺.

### Step 6: Preparation of compound SMP-12395

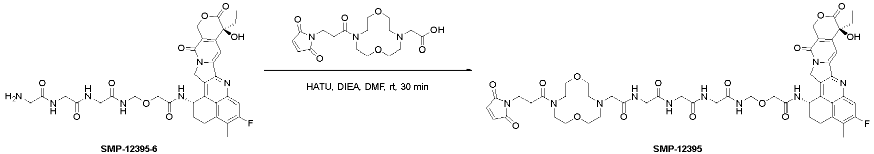

The compound SMP-93566-4 (9.0 mg, 0.020 mmol) and a solvent N,N-dimethylformamide (1.5 mL) were added to a 10 mL reaction flask. After the reaction solution was completely clarified, 2-(7-azabenzotriazole)-N,N,N',N'-tetramethylurea hexafluorophosphate (9.0 mg, 0.024 mmol) and N,N-diisopropylethylamine (4.0 mg, 0.03 mmol) were sequentially added. After reaction at room temperature for 5 minutes, the compound SMP-12395-6 (13.0 mg, 0.019 mmol) was added, the reaction continued for 25 minutes while stirring, and then TLC showed complete reaction. The N,N-dimethylformamide was removed by concentration under reduced pressure, and the residue was purified by HPLC. After freeze drying, a white solid SMP-12395 was obtained, weighing 5.8 mg, with a yield of 29%.

MS (ESI) m/z: 1059 [M+H]⁺.

¹HNMR (400 MHz, DMSO-*d*₆) δ8.93 (s, 1H), 8.72 (t, *J* = 7.3 Hz, 1H), 8.52 (d, *J* = 8.8 Hz, 1H), 8.28 (s, 1H), 8.19 (t, *J* = 5.7 Hz, 1H), 7.95 (s, 1H), 7.79 (d, *J=* 11.0 Hz, 1H), 7.32 (d, *J* = 8.3 Hz, 1H), 6.99 (s, 2H), 6.54 (s, 1H), 5.60 (dd, *J* = 14.0, 5.8 Hz, 1H), 5.40 (d, *J* = 16.8 Hz, 2H), 5.20 (s, 2H), 4.62 (d, *J* = 6.5 Hz, 2H), 4.15 (s, 2H), 4.00 (s, 2H), 3.94 (s, 2H), 3.85 - 3.69 (m, 8H), 3.69 - 3.48 (m, 14H), 2.67 (s, 1H), 2.39 (s, 4H), 2.17 (dt, *J* = 15.8, 6.2 Hz, 2H), 1.91 - 1.79 (m, 2H), 1.23 (s, 1H), 0.87 (t, *J* = 7.2 Hz, 3H).

### Example 29: Preparation of compound SMP-56822

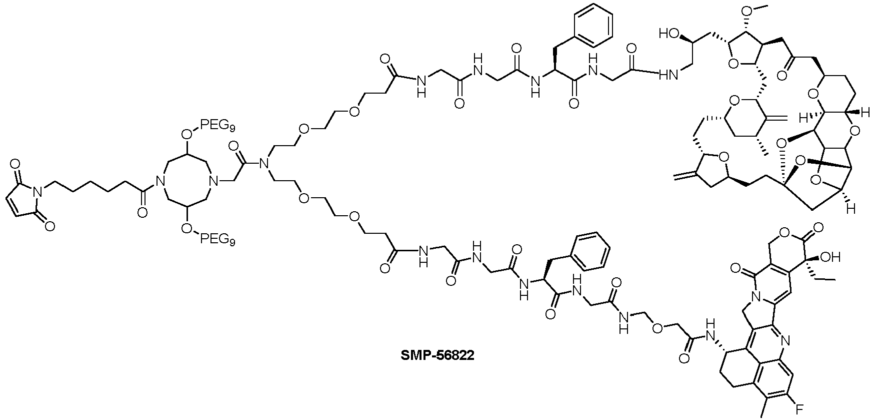

### Step 1: Preparation of compound SMP-56822-2

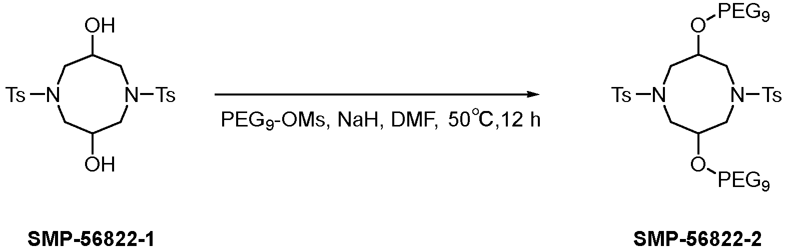

A compound SMP-56822-1 (2.0 g, 4.4 mmol) and a solvent N,N-dimethylformamide (30 mL) were sequentially added to a 100 mL reaction flask, and sodium hydride (60 wt%) (0.53 g, 13.20 mmol) was added at 0°C. After reaction at room temperature for 10 minutes, PEG₉-OMs (4.9 g, 9.70 mmol) was added at a time, the reaction continued at 50°C for 12 hours while stirring, and then TLC showed complete reaction. Water (15 mL) was slowly added at 0°C to quench the reaction, the reaction solution was extracted with dichloromethane (100 mL) 3 times, organic layers were combined, washed with a saturated salt solution, dried with anhydrous sodium sulfate, filtered and concentrated under reduced pressure, and the residue was purified by column chromatography (dichloromethane: ethyl acetate=2:1) to obtain an intermediate SMP-56822-2, weighing 4.0 g, with a yield of 71%.

MS (ESI) m/z: 1275 [M+H]⁺.

### Step 2: Preparation of compound SMP-56822-3

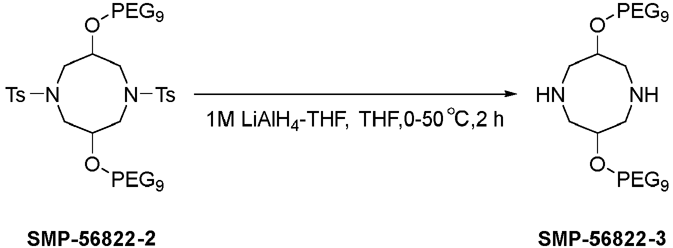

The compound SMP-56822-2 (4.0 g, 3.1 mmol) and a solvent tetrahydrofuran (32 mL) were sequentially added to a 100mL reaction flask, and a tetrahydrofuran solution of 1M lithium aluminum hydride (18.6 mL, 18.6 mmol) was slowly added dropwise at 0°C. After recovered to room temperature, the solution was heated to 50°C, a reaction occurred for 2 hours while stirring, and then LC-MS test showed complete reaction. Sodium sulfate decahydrate (5.0 g) was added in batches at 0°C. After filtration and concentration under reduced pressure to remove excess tetrahydrofuran, an intermediate SMP-56822-3 was obtained, weighing 2.4 g, with a yield of 79%.

MS (ESI) m/z: 967 [M+H]⁺.

### Step 3: Preparation of compound SMP-56822-4

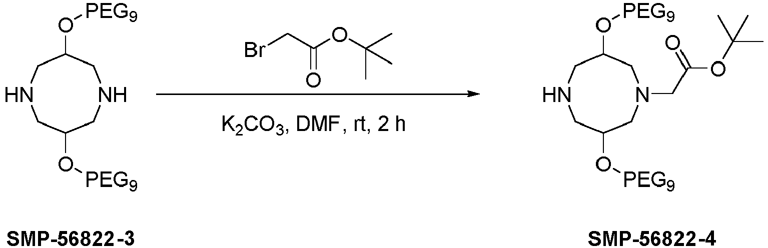

The compound SMP-56822-3 (630.0 mg, 0.65 mmol) and a solvent N,N-dimethylformamide (5 mL) were sequentially added to a 25 mL reaction flask. After the mixed solution was completely clarified, potassium carbonate (180.0 mg, 1.30 mmol) and tert-butyl bromoacetate (127.0 mg, 0.65 mmol) were sequentially added. A reaction occurred at room temperature for 2 hours while stirring, and then TLC showed complete reaction. After filtration and concentration under reduced pressure to remove excess N,N-dimethylformamide, the residue was purified by column chromatography (dichloromethane: methanol=15:1) to obtain an intermediate SMP-56822-4, weighing 170.0 mg, with a yield of 24%.

MS (ESI) m/z: 1081 [M+H]⁺.

### Step 4: Preparation of compound SMP-56822-5

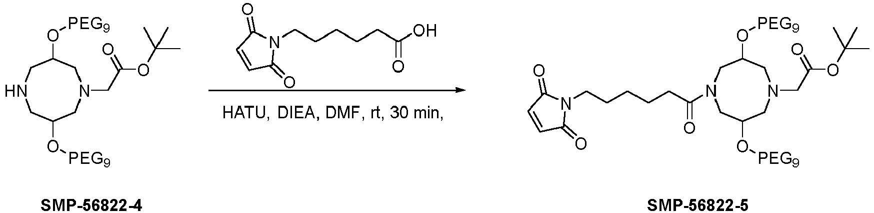

A compound maleimidocaproic acid (24.0 mg, 0.11 mmol) and a solvent N,N-dimethylformamide (4 mL) were added to a 25 mL reaction flask, and then 2-(7-azabenzotriazole)-N,N,N',N'-tetramethylurea hexafluorophosphate (42.0 mg, 0.11 mmol) and N,N-diisopropylethylamine (17.0 mg, 0.13 mmol) were sequentially added. After reaction at room temperature for 5 minutes, the compound SMP-56822-4 (102.0 mg, 0.094 mmol) was added, the reaction continued for 25 minutes while stirring, and then TLC showed complete reaction. Excess N,N-dimethylformamide was removed by concentration under reduced pressure, and the residue was purified by column chromatography (dichloromethane: methanol=20:1) to obtain an intermediate SMP-56822-5, weighing 100.0 mg, with a yield of 83%.

MS (ESI) m/z: 1274 [M+H]⁺.

### Step 5: Preparation of compound SMP-56822-6

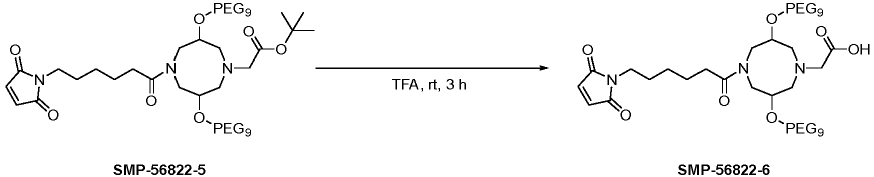

The compound SMP-56822-5 (100.0 mg, 0.08 mmol) and trifluoroacetic acid (5 mL) were added to a 25 mL reaction flask and reacted at room temperature for 3 hours while stirring, and then LC-MS showed complete reaction. Excess trifluoroacetic acid was removed by concentration under reduced pressure, and the residue was purified by HPLC to obtain an intermediate SMP-56822-6, weighing 80.0 mg, with a yield of 83%.

MS (ESI) m/z: 1218 [M+H]⁺.

### Step 6: Preparation of compound SMP-56822

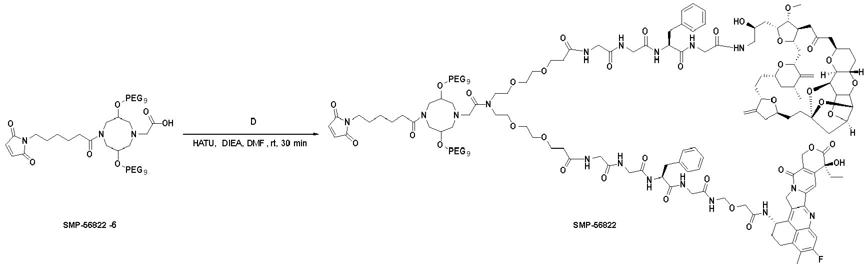

The compound SMP-56822-6 (30.0 mg, 0.025 mmol) and a solvent N,N-dimethylformamide (4 mL) were added to a 25 mL reaction flask. After the reaction solution was completely clarified, 2-(7-azabenzotriazole)-N,N,N',N'-tetramethylurea hexafluorophosphate (9.0 mg, 0.024 mmol) and N,N-diisopropylethylamine (4.0 mg, 0.032 mmol) were sequentially added. After reaction at room temperature for 5 minutes, the compound D (45.0 mg, 0.020 mmol) was added, the reaction continued for 25 minutes while stirring, and then TLC showed complete reaction. Excess N,N-dimethylformamide was removed by concentration under reduced pressure, and the residue was purified by HPLC. After freeze drying, a white solid SMP-56822 was obtained, weighing 40.0 mg, with a yield of 59%.

MS (ESI) m/z: 1131 [M/3+H]⁺.

### Example 30: Preparation of compound SMP-82891

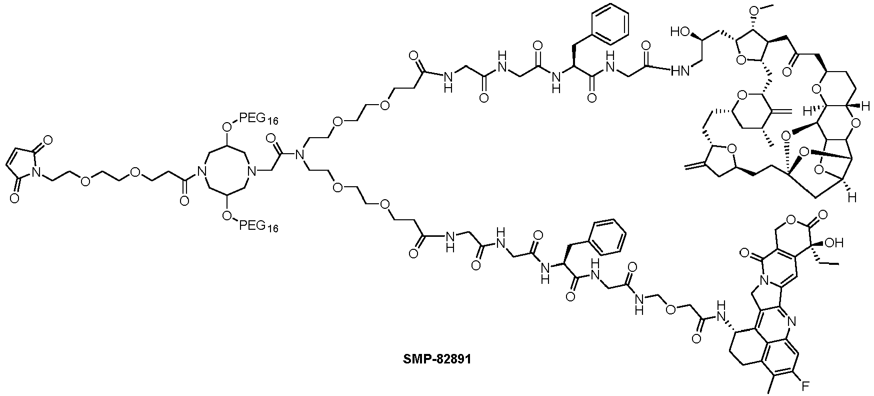

### Step 1: Preparation of compound SMP-82891-2

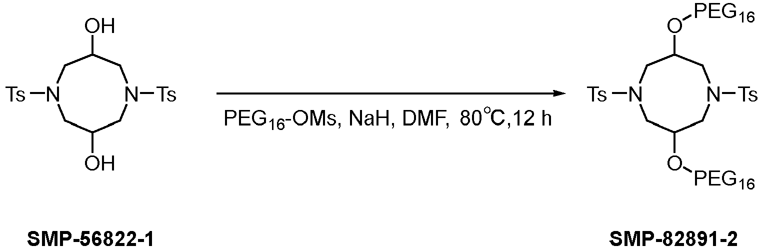

A compound SMP-56822-1 (2.0 g, 4.40 mmol) and a solvent N,N-dimethylformamide (30 mL) were sequentially added to a 100 mL reaction flask, and sodium hydride (60 wt%) (0.5 g, 13.2 mmol) was added at 0°C. After reaction at room temperature for 10 minutes, PEG₁₆-OMs (7.5 g, 9.7 mmol) was added at a time, the reaction continued at 80°C for 12 hours while stirring, and then TLC showed complete reaction. Water (15 mL) was slowly added at 0°C to quench the reaction, dichloromethane (100 mL * 3) was added for extraction, organic layers were combined, washed with a saturated salt solution, dried with anhydrous sodium sulfate, filtered and concentrated under reduced pressure, and the residue was purified by column chromatography (dichloromethane: methanol=20:1) to obtain an intermediate SMP-82891-2, weighing 5.0 g, with a yield of 63%.

MS (ESI) m/z: 1804 [M+H]⁺.

### Step 2: Preparation of compound SMP-82891-3

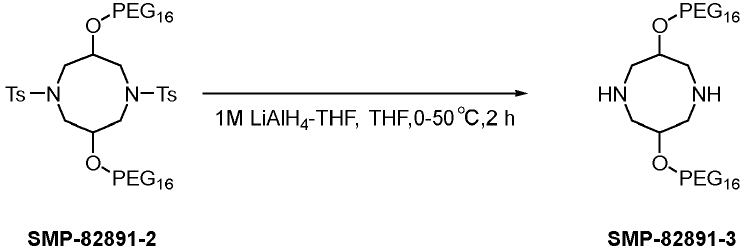

The compound SMP-82891-2 (4.0 g, 2.2 mmol) and a solvent tetrahydrofuran (40mL) were sequentially added to a 100 mL reaction flask, and a tetrahydrofuran solution of 1M lithium aluminum hydride (13.2 mL, 13.2 mmol) was slowly added dropwise at 0°C. After returning to room temperature, the solution was heated to 50°C, a reaction occurred for 2 hours while stirring, and then LC-MS test showed complete reaction. Sodium sulfate decahydrate (5.0 g) was added in batches at 0°C. After filtration and concentration under reduced pressure to remove excess tetrahydrofuran, the residue was purified by column chromatography (dichloromethane: methanol=8:1) to obtain an intermediate SMP-82891-3, weighing 2.4 g, with a yield of 73%.

MS (ESI) m/z: 1495 [M+H]⁺.

### Step 3: Preparation of compound SMP-82891-4

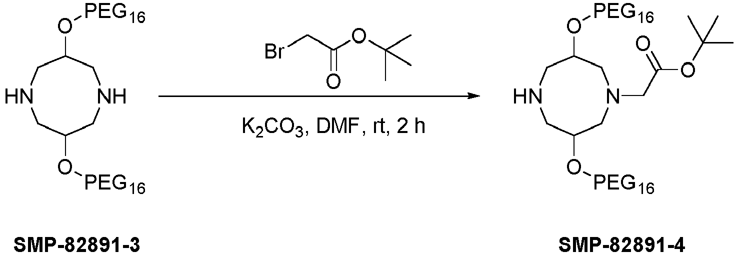

The compound SMP-82891-3 (2.4 g, 1.61 mmol), a solvent N,N-dimethylformamide (35 mL), potassium carbonate (443.0 mg, 3.21 mmol), and tert-butyl bromoacetate (314 mg, 1.61 mmol) were sequentially added to a 100 mL reaction flask and reacted at room temperature for 2 hours while stirring, and then TLC showed complete reaction. After filtration and concentration under reduced pressure to remove excess N,N-dimethylformamide, the residue was purified by column chromatography (dichloromethane: methanol=12:1) to obtain an intermediate SMP-82891-4, weighing 360.0 mg, with a yield of 14%. MS (ESI) m/z: 1609 [M+H]⁺.

### Step 4: Preparation of compound SMP-82891-5

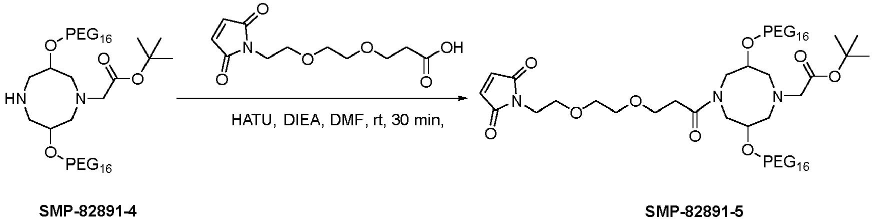

A compound 3-(2-(2,5-dioxy-2,5-dihydro-1H-pyrrol-1-yl)ethoxy)propionic acid (32.0 mg, 0.13 mmol) and a solvent N,N-dimethylformamide (8 mL) were added to a 25 mL reaction flask. After the reaction solution was completely clarified, 2-(7-azabenzotriazole)-N,N,N',N'-tetramethylurea hexafluorophosphate (49.0 mg, 0.13 mmol), and N,N-diisopropylethylamine (21.0 mg, 0.16 mmol) were sequentially added, a reaction occurred at room temperature for 5 minutes, the compound SMP-82891-4 (170.0 mg, 0.11 mmol) was added, the reaction continued for 25 minutes while stirring, and then TLC showed complete reaction. Excess N,N-dimethylformamide was removed by concentration under reduced pressure, and the residue was purified by column chromatography (dichloromethane: methanol=15:1) to obtain an intermediate SMP-82891-5, weighing 150.0 mg, with a yield of 74%.

MS (ESI) m/z: 1849 [M+H]⁺.

### Step 5: Preparation of compound SMP-82891-6

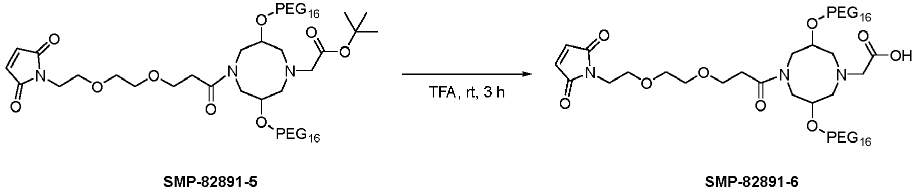

The compound SMP-82891-5 (150.0 mg, 0.08 mmol) and trifluoroacetic acid (4 mL) were added to a 25 mL reaction flask and reacted at room temperature for 3 hours while stirring, and then LC-MS showed complete reaction. Excess trifluoroacetic acid was removed by concentration under reduced pressure, and the residue was purified by HPLC to obtain an intermediate SMP-82891-6, weighing 130.0 mg, with a yield of 90%.

MS (ESI) m/z: 1793 [M+H]⁺.

### Step 6: Preparation of compound SMP-82891

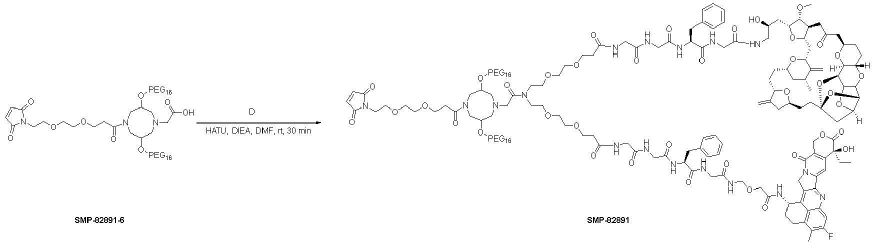

The compound SMP-82891-6 (20.0mg, 0.01 mmol) and a solvent N,N-dimethylformamide (1.5 mL) were added to a 25 mL reaction flask. After the reaction solution was completely clarified, 2-(7-azabenzotriazole)-N,N,N',N'-tetramethylurea hexafluorophosphate (11.0 mg, 0.03 mmol) and N,N-diisopropylethylamine (4.0 mg, 0.03 mmol) were sequentially added. After reaction at room temperature for 5 minutes, the compound D (22.0 mg, 0.01 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-82891, weighing 30.0 mg, with a yield of 76%.

MS (ESI) m/z: 1322 [M/3+H]⁺.

### Example 31: Preparation of compound SMP-51013-A

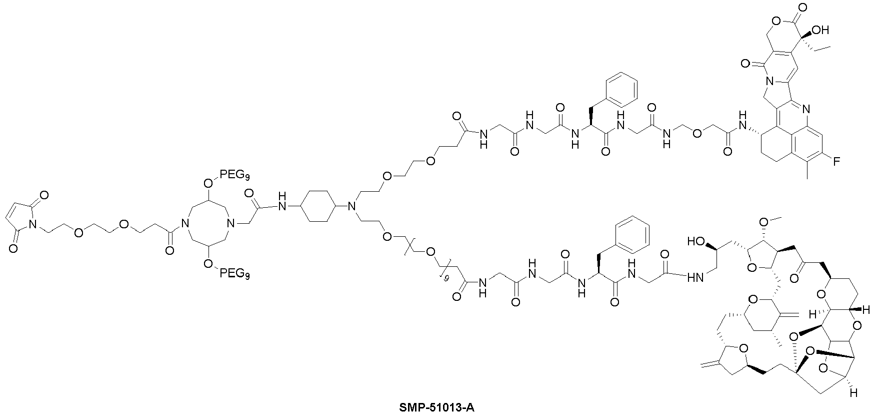

### Step 1: Preparation of compound SMP-51013-2

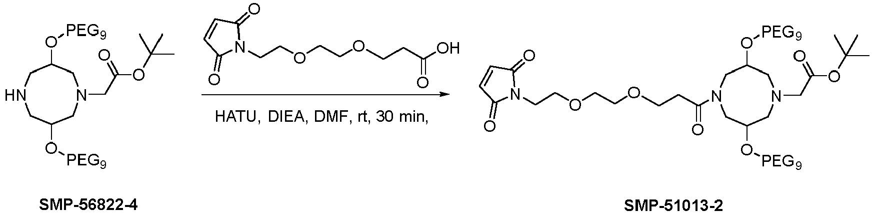

A compound maleimide-dipolyethylene glycol-carboxylic acid (29.0 mg, 0.11 mmol) and a solvent N,N-dimethylformamide (3mL) were added to a 25 mL reaction flask. After the reaction solution was completely clarified, 2-(7-azabenzotriazole)-N,N,N',N'-tetramethylurea hexafluorophosphate (41.0 mg, 0.11 mmol) and N,N-diisopropylethylamine (17.0 mg, 0.13 mmol) were sequentially added. After reaction at room temperature for 5 minutes, the compound SMP-56822-4 (102.0 mg, 0.092 mmol) was added, the reaction continued for 25 minutes while stirring, and then TLC showed complete reaction. Excess N,N-dimethylformamide was removed by concentration under reduced pressure, and the residue was purified by column chromatography (dichloromethane: methanol=12:1) to obtain an intermediate SMP-51013-2, weighing 100.0 mg, with a yield of 84%.

MS (ESI) m/z: 1320 [M+H]⁺.

### Step 2: Preparation of compound SMP-51013-3

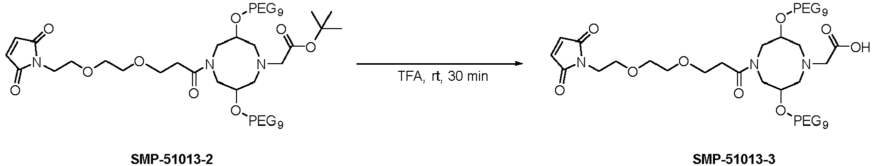

In a 25 mL reaction flask, the compound SMP-51013-2 (100. 0 mg, 0.076 mmol) and a solvent trifluoroacetic acid (4 mL) were added and stirred at room temperature for reaction for 30 minutes, and then LC-MS showed complete reaction. Excess trifluoroacetic acid was removed by concentration under reduced pressure, and the residue was purified by HPLC to obtain an intermediate SMP-51013-3, weighing 80.0 mg, with a yield of 83%.

MS (ESI) m/z: 1264 [M+H]⁺.

### Step 3: Preparation of compound SMP-51013-A

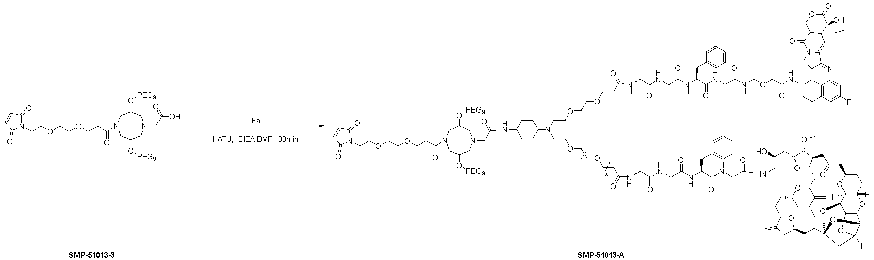

The compound SMP-51013-3 (20.0 mg, 0.016 mmol) and a solvent N,N-dimethylformamide (1.5 mL) were added to a 10 mL reaction flask, and 2-(7-azabenzotriazole)-N,N,N',N'-tetramethylurea hexafluorophosphate (11.0 mg, 0.029 mmol) and N,N-diisopropylethylamine (4.0 mg, 0.031 mmol) were sequentially added. After reaction at room temperature for 5 minutes, the compound Fa (35.0 mg, 0.013 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-51013-A, weighing 30.0 mg, with a yield of 62%. MS (ESI) m/z: 1295 [M/3+H]⁺.

### Example 32: Preparation of compound SMP-51013-B

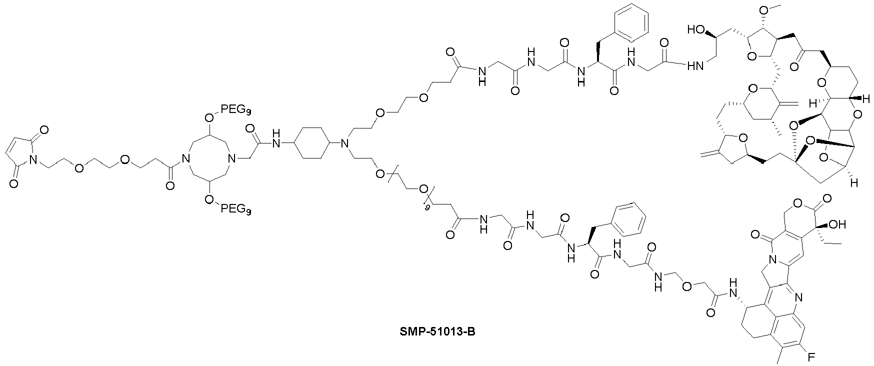

### Step 1: Preparation of compound SMP-51013-B

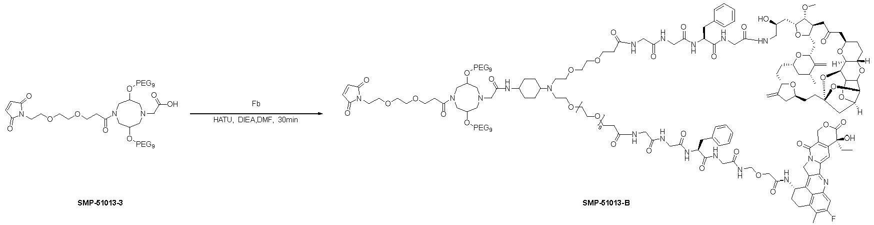

The compound SMP-51013-3 (20.0 mg, 0.016 mmol) and a solvent N,N-dimethylformamide (1.5 mL) were added to a 10 mL reaction flask, and 2-(7-azabenzotriazole)-N,N,N',N'-tetramethylurea hexafluorophosphate (11.0 mg, 0.029 mmol) and N,N-diisopropylethylamine (4.0 mg, 0.031 mmol) were sequentially added. After reaction at room temperature for 5 minutes, the compound Fb (35.0 mg, 0.013 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-51013-B, weighing 28.0 mg, with a yield of 72%. MS (ESI) m/z: 1295 [M/3+H]⁺.

### Example 33: Preparation of compound SMP-36256

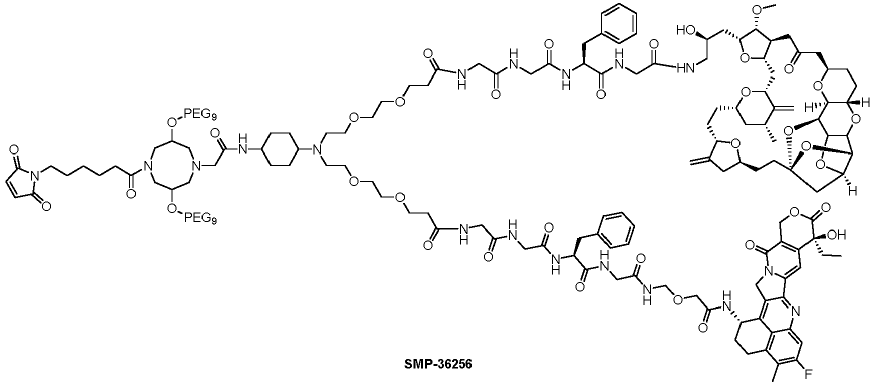

### Step 1: Preparation of compound SMP-36256

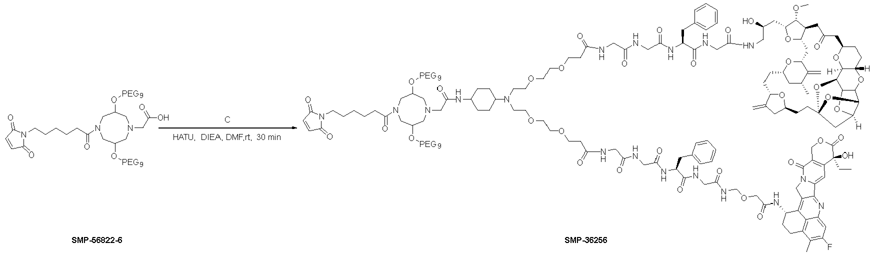

The compound SMP-56822-6 (20.0 mg, 0.016 mmol) and a solvent N,N-dimethylformamide (1.5 mL) were added to a 25 mL reaction flask. After the reaction solution was completely clarified, 2-(7-azabenzotriazole)-N,N,N',N'-tetramethylurea hexafluorophosphate (11.0 mg, 0.029 mmol) and N,N-diisopropylethylamine (4.0 mg, 0.031 mmol) were sequentially added. After reaction at room temperature for 5 minutes, the compound C (45.0 mg, 0.020 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-36256, weighing 40.0 mg, with a yield of 57%.

MS (ESI) m/z: 1162 [M/3+H]⁺.

### Example 34: Preparation of compound SMP-45582

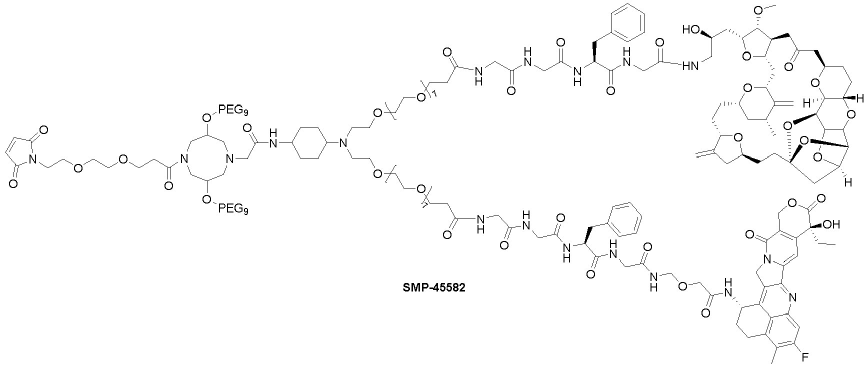

### Step 1: Preparation of compound SMP-45582

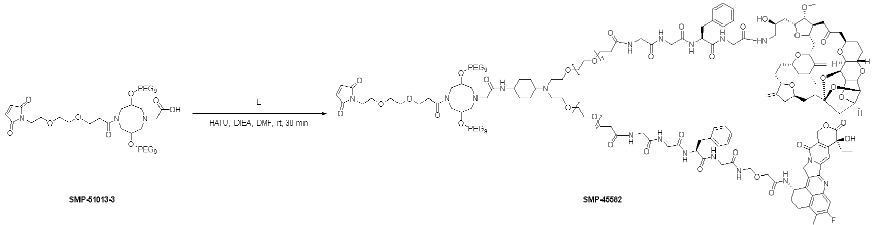

The compound SMP-51013-3 (25.0 mg, 0.020 mmol) and a solvent N,N-dimethylformamide (1.5 mL) were added to a 10 mL reaction flask, and 2-(7-azabenzotriazole)-N,N,N',N'-tetramethylurea hexafluorophosphate (11.0 mg, 0.029 mmol) and N,N-diisopropylethylamine (4.0 mg, 0.031 mmol) were sequentially added. After reaction at room temperature for 5 minutes, the compound E (60.0 mg, 0.021 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-45582, weighing 48.0 mg, with a yield of 59%. MS (ESI) m/z: 1354 [M/3+H]⁺.

### Example 35: Preparation of compound SMP-01984

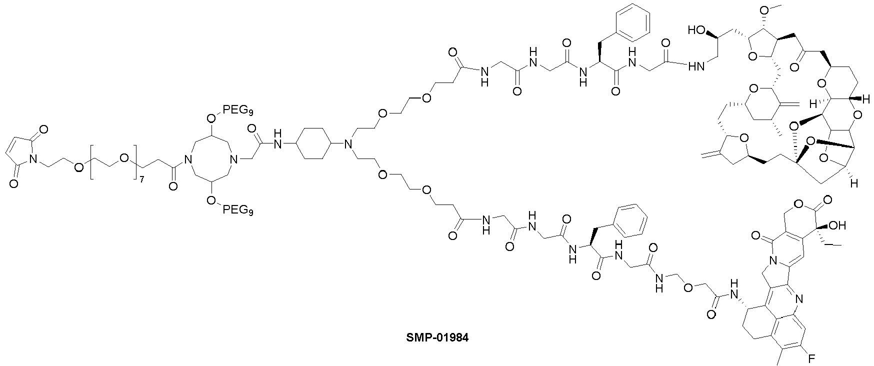

### Step 1: Preparation of compound SMP-01984-1

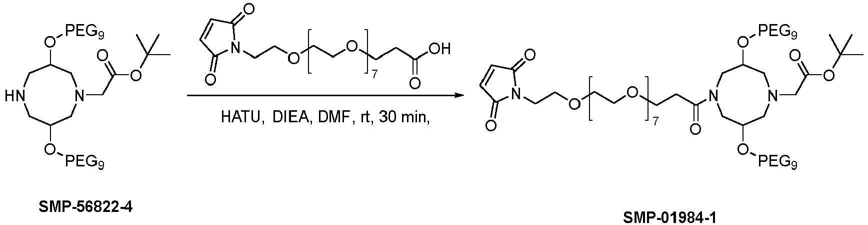

A compound maleimide-polyethylene glycol 8-carboxylic acid (57.0 mg, 0.11 mmol) and a solvent N,N-dimethylformamide (5 mL) were added to a 25 mL reaction flask, and then 2-(7-azabenzotriazole)-N,N,N',N'-tetramethylurea hexafluorophosphate (41.0 mg, 0.11 mmol) and N,N-diisopropylethylamine (17.0 mg, 0.14 mmol) were sequentially added. After reaction at room temperature for 5 minutes, the compound SMP-56822-4 (102.0 mg, 0.094 mmol) was added, the reaction continued for 25 minutes while stirring, and then TLC showed complete reaction. Excess N,N-dimethylformamide was removed by concentration under reduced pressure, and the residue was purified by column chromatography (dichloromethane: methanol=15:1) to obtain an intermediate SMP-01984-1, weighing 100.0 mg, with a yield of 67%.

MS (ESI) m/z: 1584 [M+H]⁺.

### Step 2: Preparation of compound SMP-01984-2

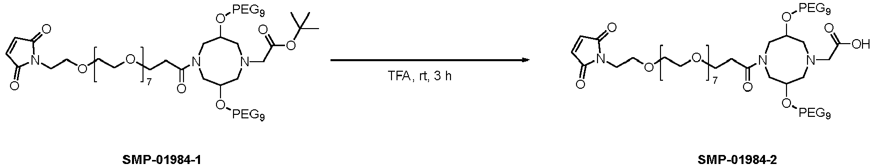

The compound SMP-01984-1 (100.0 mg, 0.063 mmol) and trifluoroacetic acid (3 mL) were added to a 25 mL reaction flask and reacted at room temperature for 3 hours while stirring, and then LC-MS showed complete reaction. Excess trifluoroacetic acid was removed by concentration under reduced pressure, and the residue was purified by HPLC to obtain an intermediate SMP-01984-2, weighing 82.0 mg, with a yield of 85%.

MS (ESI) m/z: 1528 [M+H]⁺.

### Step 3: Preparation of compound SMP-01984

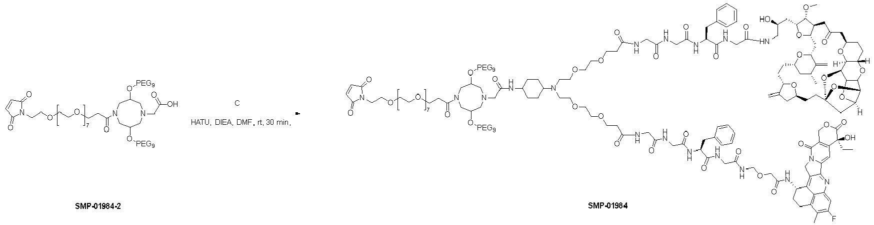

The compound SMP-01984-2 (35.0 mg, 0.023 mmol) and a solvent N,N-dimethylformamide (1.5 mL) were added to a 10 mL reaction flask, and 2-(7-azabenzotriazole)-N,N,N',N'-tetramethylurea hexafluorophosphate (11.0 mg, 0.029 mmol) and N,N-diisopropylethylamine (4.0 mg, 0.031 mmol) were sequentially added. After reaction at room temperature for 5 minutes, the compound C (60.0 mg, 0.026 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-01984, weighing 48.0 mg, with a yield of 55%. MS (ESI) m/z: 1266 [M/3+H]⁺.

### Example 36: Preparation of compound SMP-30920

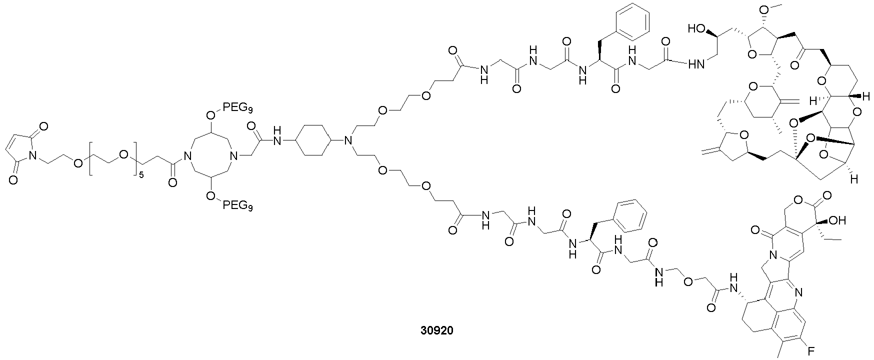

### Step 1: Preparation of compound SMP-30920-1

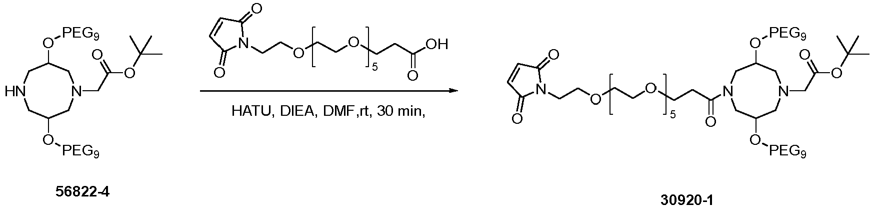

A compound maleimide-polyethylene glycol 6-carboxylic acid (48.0 mg, 0.11 mmol) and a solvent N,N-dimethylformamide (5 mL) were added to a 25 mL reaction flask, and then 2-(7-azabenzotriazole)-N,N,N',N'-tetramethylurea hexafluorophosphate (41.0 mg, 0.11 mmol) and N,N-diisopropylethylamine (17.0 mg, 0.13 mmol) were sequentially added. After reaction at room temperature for 5 minutes, the compound SMP-56822-4 (102 mg, 0.094 mmol) was added, the reaction continued for 25 minutes while stirring, and then TLC showed complete reaction. Excess N,N-dimethylformamide was removed by concentration under reduced pressure, and the residue was purified by column chromatography (dichloromethane: methanol=15:1) to obtain an intermediate SMP-30920-1, weighing 98.0 mg, with a yield of 70%.

MS (ESI) m/z: 1496 [M+H]⁺.

### Step 2: Preparation of compound SMP-30920-2

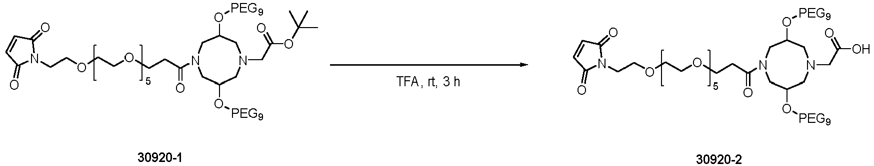

The compound SMP-30920-1 (95.0 mg, 0.063 mmol) and trifluoroacetic acid (3 mL) were added to a 25 mL reaction flask and reacted at room temperature for 3 hours while stirring, and then LC-MS showed complete reaction. Excess trifluoroacetic acid was removed by concentration under reduced pressure, and the residue was purified by HPLC to obtain an intermediate SMP-30920-2, weighing 80.0 mg, with a yield of 88%.

MS (ESI) m/z: 1440 [M+H]⁺.

### Step 3: Preparation of compound SMP-30920

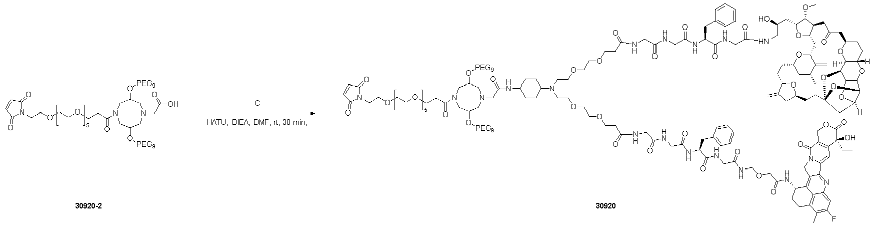

The compound SMP-30920-2 (29.0 mg, 0.020 mmol) and a solvent N,N-dimethylformamide (1.5 mL) were added to a 10 mL reaction flask. After the reaction solution was completely clarified, 2-(7-azabenzotriazole)-N,N,N',N'-tetramethylurea hexafluorophosphate (11.0 mg, 0.03 mmol) and N,N-diisopropylethylamine (4.0 mg, 0.031 mmol) were sequentially added. After reaction at room temperature for 5 minutes, the compound C (60.0 mg, 0.026 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-30920, weighing 43.0 mg, with a yield of 58%.

MS (ESI) m/z: 1236 [M/3+H]⁺.

### Example 37: Preparation of compound SMP-14324

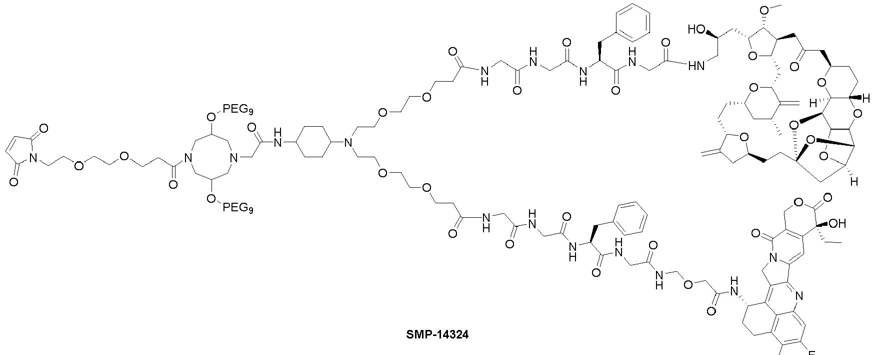

### Step 1: Preparation of compound SMP-14324

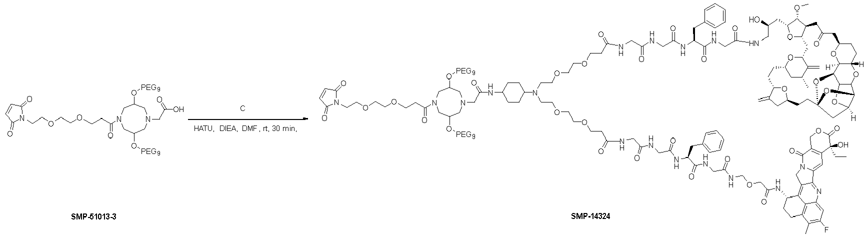

The compound SMP-51013-3 (13.0 mg, 0.010 mmol) and a solvent N,N-dimethylformamide (1.5 mL) were added to a 10 mL reaction flask, and 2-(7-azabenzotriazole)-N,N,N',N'-tetramethylurea hexafluorophosphate (11.0 mg, 0.029 mmol) and N,N-diisopropylethylamine (4.0 mg, 0.031 mmol) were sequentially added. After reaction at room temperature for 5 minutes, the compound C (22.0 mg, 0.0096 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-14324, weighing 26.0 mg, with a yield of 73%. MS (ESI) m/z: 1178 [M/3+H]⁺.

### Example 38: Preparation of compound SMP-61393

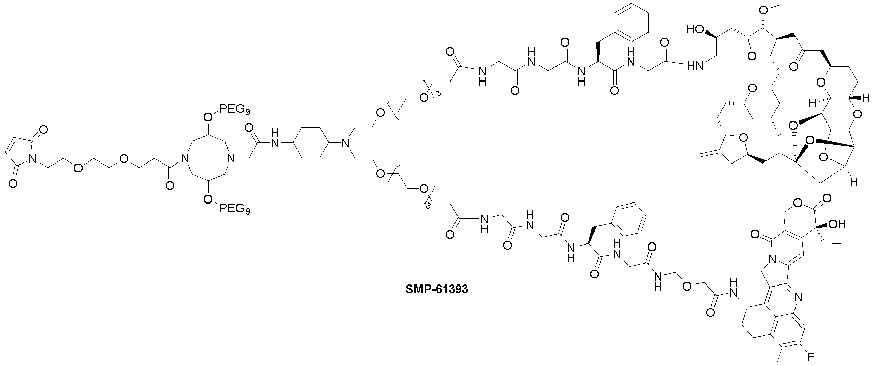

### Step 1: Preparation of compound SMP-61393

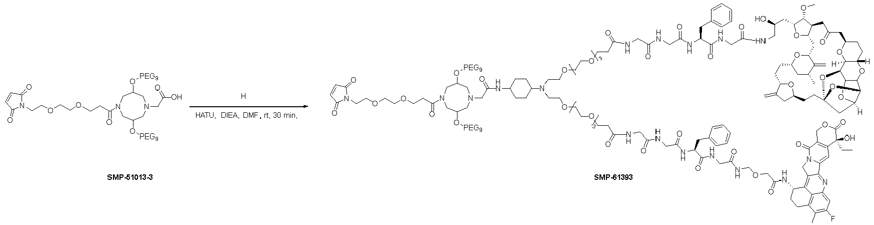

The compound SMP-51013-3 (13.0 mg, 0.011 mmol) and a solvent N,N-dimethylformamide (1.5 mL) were added to a 10 mL reaction flask, and 2-(7-azabenzotriazole)-N,N,N',N'-tetramethylurea hexafluorophosphate (4.0 mg, 0.011 mmol) and N,N-diisopropylethylamine (2.0 mg, 0.014 mmol) were sequentially added. After reaction at room temperature for 5 minutes, the compound H (22.0 mg, 0.009 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-61393, weighing 24.0 mg, with a yield of 74%. MS (ESI) m/z: 1207 [M/3+H]⁺.

### Example 39: Preparation of compound SMP-33693

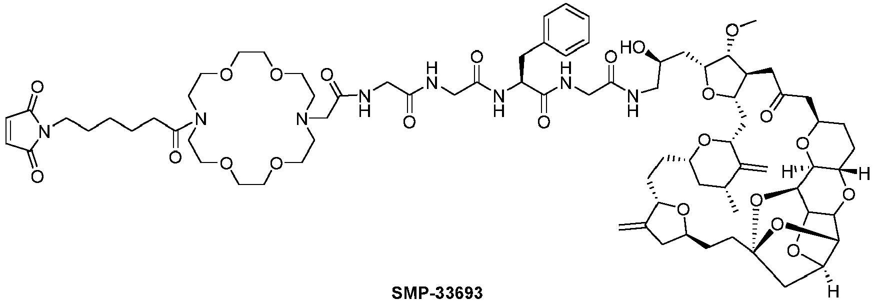

### Step 1: Preparation of compound SMP-33693-2

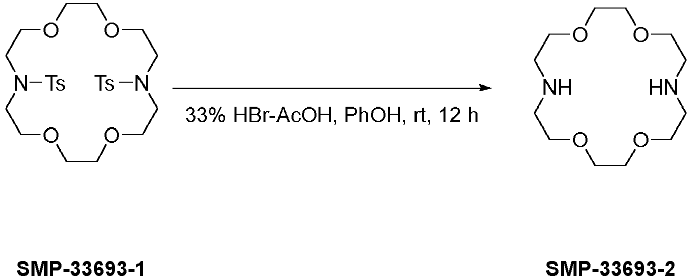

A compound SMP-33693-1 (1.0 g, 1.75 mmol) and a 33% hydrogen bromide acetic acid solution (15 mL) were sequentially added to a 50 mL reaction flask. After the reaction solution was clarified, phenol (494.0 mg, 5.25 mmol) was added. After reaction at room temperature for 12 hours, LC-MS test showed complete reaction. The reaction solution was slowly added dropwise to methyl tert-butyl ether (20 mL), and white solid precipitated. After filtration, filter cakes were collected and dried to obtain a target product SMP-33693-2, weighing 300.0 mg, with a yield of 66%.

MS (ESI) m/z: 263 [M+H]⁺.

### Step 2: Preparation of compound SMP-33693-3

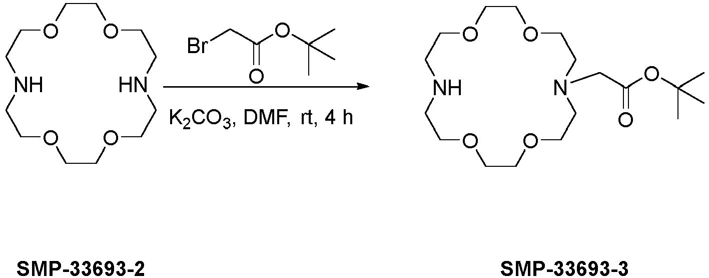

The compound SMP-33693-2 (300.0 mg, 1.15 mmol), a solvent N,N-dimethylformamide (10 mL), potassium carbonate (316.0 mg, 2.30 mmol), and tert-butyl bromoacetate (224.0 mg, 1.15 mmol) were sequentially added to a 25mL reaction flask and reacted at room temperature for 4 hours while stirring, and then TLC showed complete reaction. After filtration and concentration under reduced pressure to remove excess N,N-dimethylformamide, the residue was purified by column chromatography (dichloromethane: methanol=12:1) to obtain an intermediate SMP-33693-3, weighing 130.0 mg, with a yield of 30%. MS (ESI) m/z: 377 [M+H]⁺.

¹HNMR (400 MHz, CDCl3) δ5.51 (s, 1H), 3.33-3.56(m, 18H), 2.52-2.70(m, 8H), 1.33 (s, 9H).

### Step 3: Preparation of compound SMP-33693-4

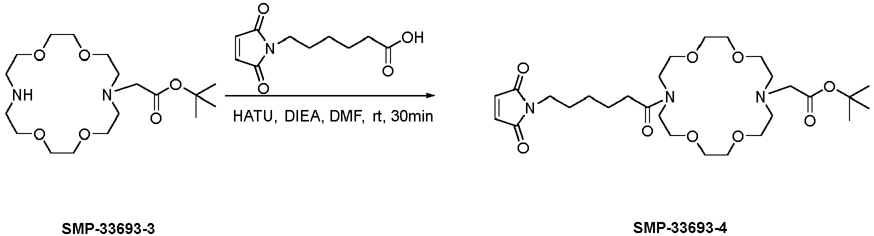

A compound maleimidocaproic acid (87.0 mg, 0.41 mmol) and a solvent N,N-dimethylformamide (5 mL) were added to a 25 mL reaction flask. After the reaction solution was clarified, 2-(7-azabenzotriazole)-N,N,N',N'-tetramethylurea hexafluorophosphate (156.0 mg, 0.41 mmol) and N,N-diisopropylethylamine (66.0 mg, 0.51 mmol) were sequentially added. After reaction at room temperature for 5 minutes, the compound SMP-33693-3 (130.0 mg, 0.34 mmol) was added, the reaction continued for 25 minutes while stirring, and then TLC showed complete reaction. Excess N,N-dimethylformamide was removed by concentration under reduced pressure, and the residue was purified by column chromatography (dichloromethane: methanol=20: 1) to obtain an intermediate SMP-33693-4, weighing 150.0 mg, with a yield of 77%.

MS (ESI) m/z: 570 [M+H]⁺.

¹HNMR (400 MHz, CDCl3) δ7.84 (s, 1H), 7.64 (s, 1H), 4.05 (t, 2H), 3.32-3.63(m, 22H), 2.52(m, 4H), 2.25 (m, 2H),1.32-1.63(m, 6H), 1.33 (s, 9H).

### Step 4: Preparation of compound SMP-33693-5

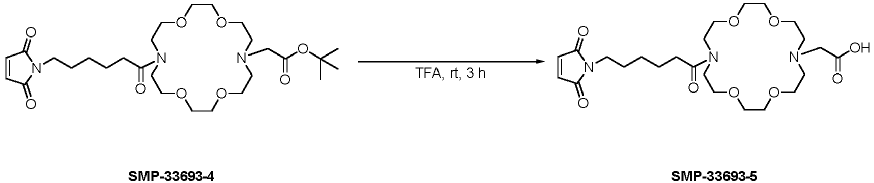

The compound SMP-33693-4 (100.0 mg, 0.18 mmol) and trifluoroacetic acid (3 mL) were added to a 25 mL reaction flask and reacted at room temperature for 3 hours while stirring, and then LC-MS showed complete reaction. Excess trifluoroacetic acid was removed by concentration under reduced pressure, and the residue was purified by HPLC to obtain an intermediate SMP-33693-5, weighing 80.0 mg, with a yield of 89%.

MS (ESI) m/z: 514 [M+H]⁺.

### Step 5: Preparation of compound SMP-33693

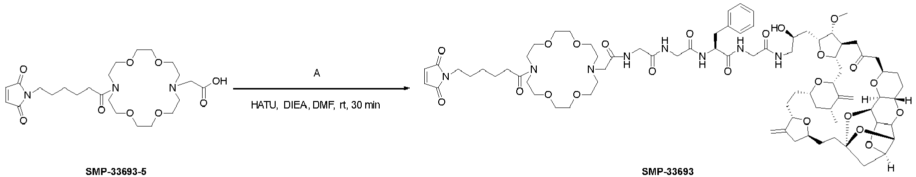

The compound SMP-33693-5 (20.0 mg, 0.039 mmol) and a solvent N,N-dimethylformamide (4 mL) were added to a 10 mL reaction flask, and 2-(7-azabenzotriazole)-N,N,N',N'-tetramethylurea hexafluorophosphate (15.0 mg, 0.039 mmol) and N,N-diisopropylethylamine (6.0 mg, 0.047 mmol) were sequentially added. After reaction at room temperature for 5 minutes, the compound A (40.0 mg, 0.038 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-33693, weighing 48.0 mg, with a yield of 78%.

MS (ESI) m/z: 1544 [M+H]⁺.

### Example 40: Preparation of compound SMP-52573

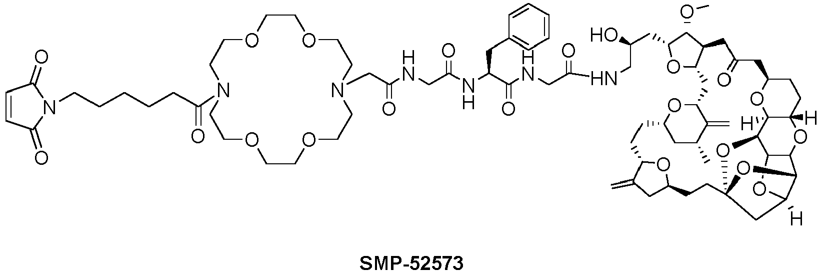

### Step 1: Preparation of compound SMP-52573

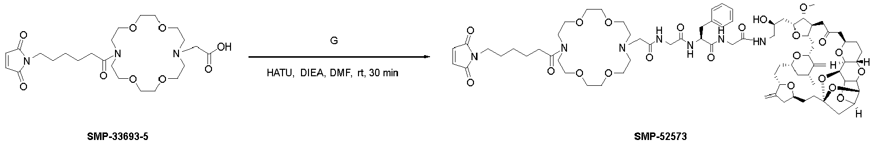

The compound SMP-33693-5 (20.0 mg, 0.039 mmol) and a solvent N,N-dimethylformamide (2 mL) were added to a 10 mL reaction flask. After the reaction solution was completely clarified, 2-(7-azabenzotriazole)-N,N,N',N'-tetramethylurea hexafluorophosphate (15.0 mg, 0.039 mmol) and N,N-diisopropylethylamine (6.0 mg, 0.047 mmol) were sequentially added. After reaction at room temperature for 5 minutes, the compound G (40.0 mg, 0.040 mmol) was added, the reaction continued for 25 minutes while stirring, and then TLC showed complete reaction. The reaction solution was concentrated under reduced pressure, and the residue was purified by HPLC to obtain a target product SMP-52573, weighing 45.0 mg, with a yield of 75%.

MS (ESI) m/z: 1487 [M+H]⁺.

### Example 41: Preparation of compound SMP-42930

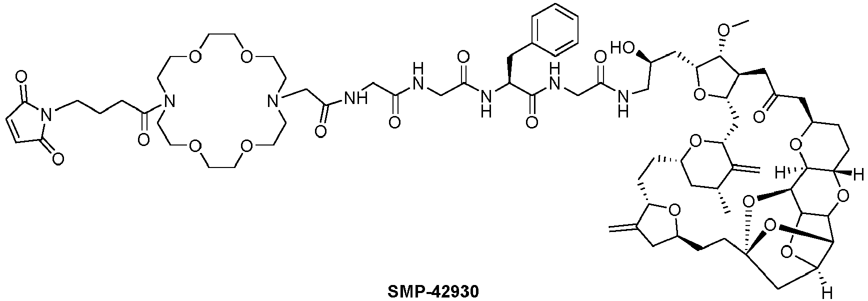

### Step 1: Preparation of compound SMP-42930-1

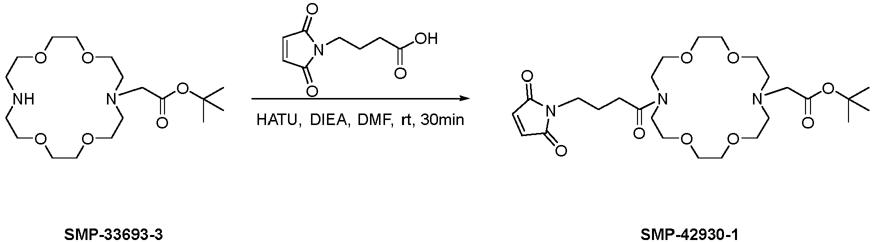

A compound maleimidobutyric acid (103.0 mg, 0.56 mmol) and a solvent N,N-dimethylformamide (5 mL) were added to a 25 mL reaction flask, and then 2-(7-azabenzotriazole)-N,N,N',N'-tetramethylurea hexafluorophosphate (212.0 mg, 0.55 mmol) and N,N-diisopropylethylamine (91.0 mg, 0.71 mmol) were sequentially added. After reaction at room temperature for 5 minutes, the compound SMP-33693-3 (176.0 mg, 0.47 mmol) was added, the reaction continued for 25 minutes while stirring, and then TLC showed complete reaction. Excess N,N-dimethylformamide was removed by concentration under reduced pressure, and the residue was purified by column chromatography (dichloromethane: methanol=20:1) to obtain an intermediate SMP-42930-1, weighing 185.0 mg, with a yield of 72%.

MS (ESI) m/z: 542 [M+H]⁺.

### Step 2: Preparation of compound SMP-42930-2

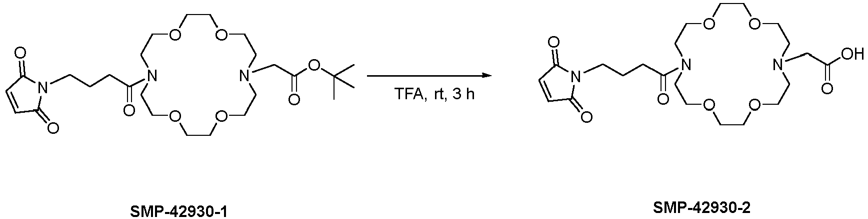

The compound SMP-42930-1 (100.0 mg, 0.18 mmol) and trifluoroacetic acid (4 mL) were added to a 25 mL reaction flask and reacted at room temperature for 3 hours while stirring, and then LC-MS showed complete reaction. Excess trifluoroacetic acid was removed by concentration under reduced pressure, and the residue was purified by HPLC to obtain an intermediate SMP-42930-2, weighing 80.0 mg, with a yield of 89%.

MS (ESI) m/z: 486 [M+H]⁺.

### Step 3: Preparation of compound SMP-42930

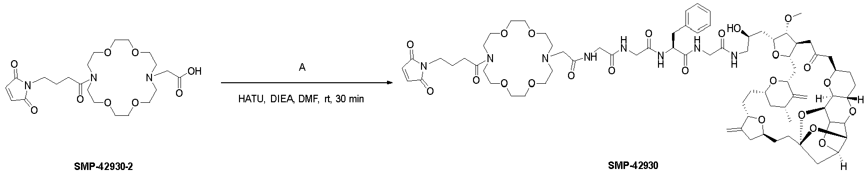

The compound SMP-42930-2 (19.0 mg, 0.039 mmol) and a solvent N,N-dimethylformamide (2 mL) were added to a 10 mL reaction flask. After the reaction solution was clarified, 2-(7-azabenzotriazole)-N,N,N',N'-tetramethylurea hexafluorophosphate (15.0 mg, 0.039 mmol) and N,N-diisopropylethylamine (6.0 mg, 0.047 mmol) were sequentially added. After reaction at room temperature for 5 minutes, the compound A (40.0 mg, 0.038 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-42930, weighing 42.0 mg, with a yield of 73%.

MS (ESI) m/z: 1516 [M+H]⁺.

### Example 42: Preparation of compound SMP-80439

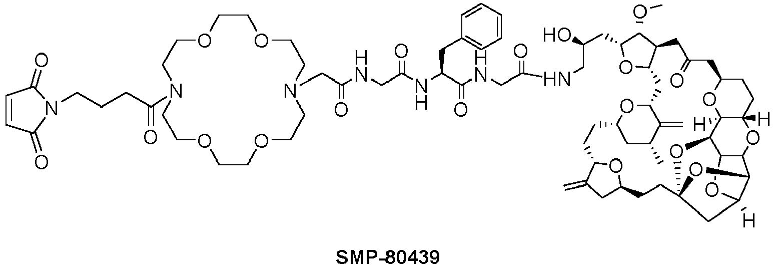

### Step 1: Preparation of compound SMP-80439

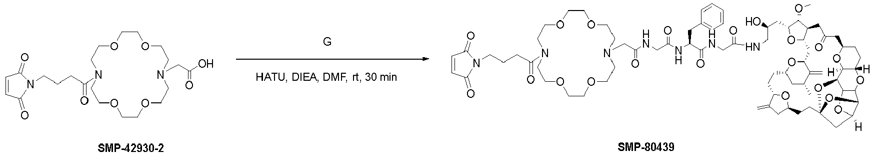

The compound SMP-42930-2 (20.0 mg, 0.041 mmol) and a solvent N,N-dimethylformamide (3 mL) were added to a 10 mL reaction flask. After the reaction solution was clarified, 2-(7-azabenzotriazole)-N,N,N',N'-tetramethylurea hexafluorophosphate (15.0 mg, 0.039 mmol) and N,N-diisopropylethylamine (6.0 mg, 0.047 mmol) were sequentially added. After reaction at room temperature for 5 minutes, the compound G (40.0 mg, 0.038 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-80439, weighing 42.0 mg, with a yield of 76%.

MS (ESI) m/z: 1458 [M+H]⁺.

### Example 43: Preparation of compound SMP-81404

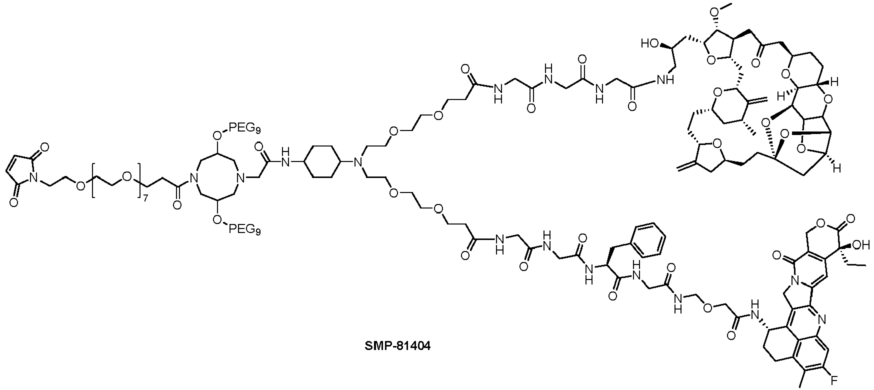

### Step 1: Preparation of compound SMP-81404

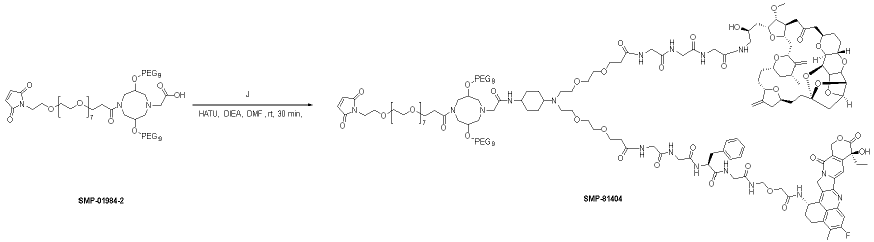

The compound SMP-01984-2 (42.0 mg, 0.024 mmol) and a solvent N,N-dimethylformamide (3 mL) were added to a 10 mL reaction flask, and 2-(7-azabenzotriazole)-N,N,N',N'-tetramethylurea hexafluorophosphate (9.0 mg, 0.024 mmol) and N,N-diisopropylethylamine (4.0 mg, 0.031 mmol) were sequentially added. After reaction at room temperature for 5 minutes, the compound J (43.0 mg, 0.020 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-81404, weighing 57.0 mg, with a yield of 78%. MS (ESI) m/z: 1217 [M/3+H]⁺.

### Example 44: Preparation of compound SMP-64906

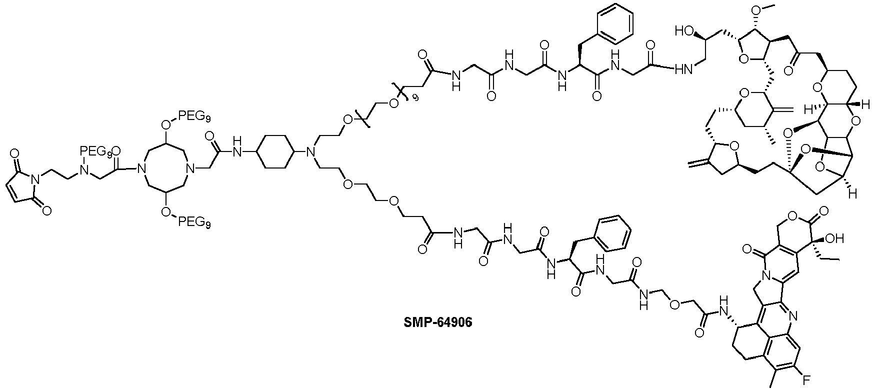

### Step 1: Preparation of compound SMP-64906

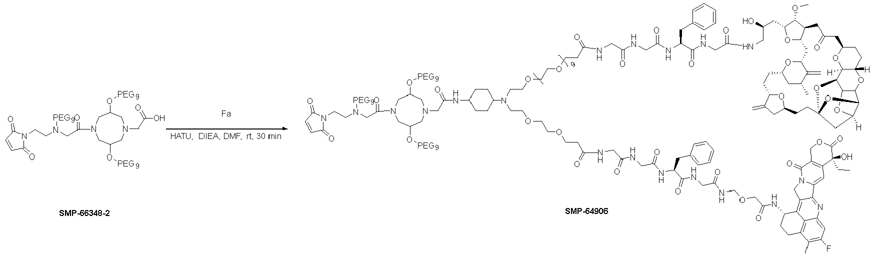

An intermediate SMP-66348-2 (39.0 mg, 0.024 mmol) and a solvent N,N-dimethylformamide (4 mL) were added to a 10 mL reaction flask. After the reaction solution was clarified, 2-(7-azabenzotriazole)-N,N,N',N'-tetramethylurea hexafluorophosphate (9.0 mg, 0.024 mmol) and N,N-diisopropylethylamine (4.0 mg, 0.031 mmol) were sequentially added. After reaction at room temperature for 5 minutes, the compound Fa (52.0 mg, 0.020 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-64906, weighing 55.0 mg, with a yield of 65%.

MS (ESI) m/z: 1413 [M/3+H]⁺.

### Example 45: Preparation of compound SMP-66348

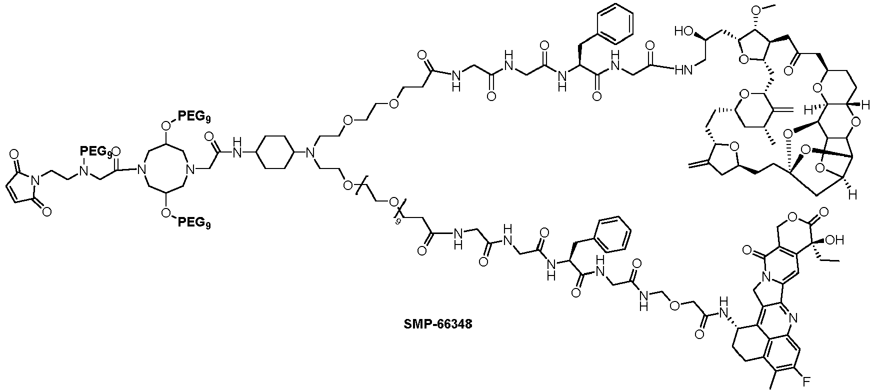

### Step 1: Preparation of compound SMP-66348-1

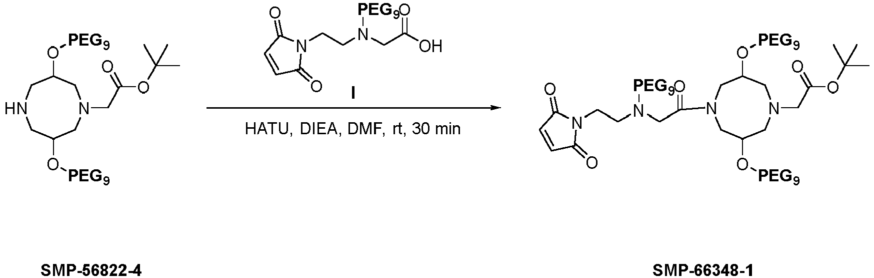

The intermediate I (61.0 mg, 0.10 mmol) and a solvent N,N-dimethylformamide (5 mL) were added to a 25 mL reaction flask, and then 2-(7-azabenzotriazole)-N,N,N',N'-tetramethylurea hexafluorophosphate (38.0 mg, 0.10 mmol) and N,N-diisopropylethylamine (15.0 mg, 0.12 mmol) were sequentially added. After reaction at room temperature for 5 minutes, the compound SMP-56822-4 (95.0 mg, 0.08 mmol) was added, the reaction continued for 25 minutes while stirring, and then TLC showed complete reaction. Excess N,N-dimethylformamide was removed by concentration under reduced pressure, and the residue was purified by column chromatography (dichloromethane: methanol=12:1) to obtain a target product SMP-66348-1, weighing 80.0 mg, with a yield of 60%.

MS (ESI) m/z: 1671 [M+H]⁺.

### Step 2: Preparation of compound SMP-66348-2

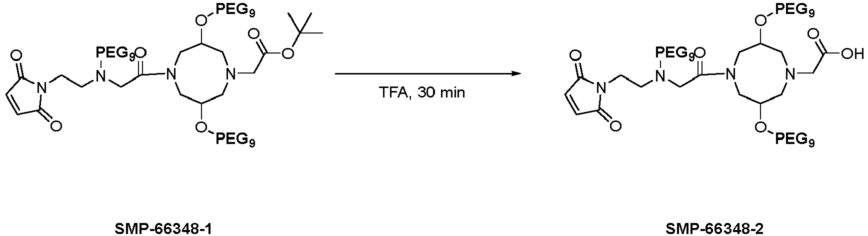

The intermediate SMP-66348-1 (50.0 mg, 0.03 mmol) and trifluoroacetic acid (3 mL) were added to a 25 mL reaction flask. A reaction occurred at room temperature for 30 minutes while, and then LC-MS showed complete reaction. After filtration, the trifluoroacetic acid was removed by concentration under reduced pressure, and the residue was purified by HPLC to obtain a compound SMP-66348-2, weighing 35.0 mg, with a yield of 72%.

MS (ESI) m/z: 1615 [M+H]⁺.

### Step 3: Preparation of compound SMP-66348

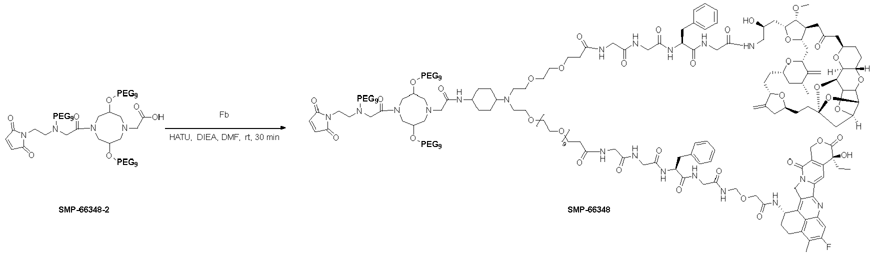

The intermediate SMP-66348-2 (39.0 mg, 0.024 mmol) and a solvent N,N-dimethylformamide (3 mL) were added to a 10 mL reaction flask. After the reaction solution was clarified, 2-(7-azabenzotriazole)-N,N,N',N'-tetramethylurea hexafluorophosphate (9.0 mg, 0.024 mmol) and N,N-diisopropylethylamine (4.0 mg, 0.031 mmol) were sequentially added. After reaction at room temperature for 5 minutes, the compound Fb (52.0 mg, 0.020 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-66348, weighing 60.0 mg, with a yield of 71%.

MS (ESI) m/z: 1413 [M/3+H]⁺.

### Example 46: Preparation of compound SMP-34421

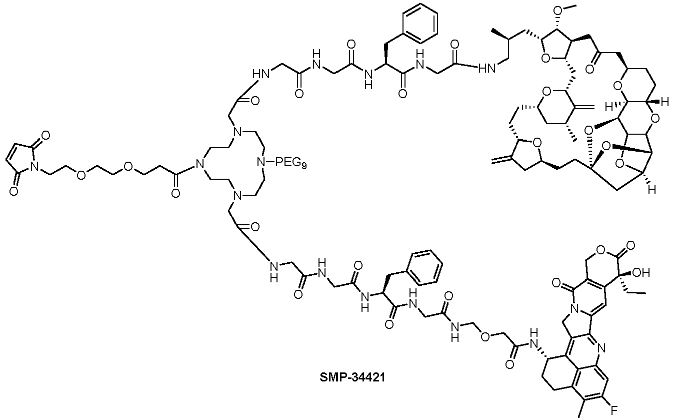

### Step 1: Preparation of compound SMP-34421-2

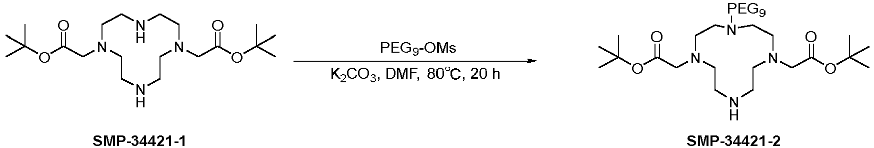

An intermediate SMP-34421-1 (200.0 mg, 0.50 mmol) and N,N-dimethylformamide (10 mL) were added to a 50 mL reaction flask. After complete clarification, PEG₉-OMs (253.0 mg, 0.50 mmol) and potassium carbonate (207.0 mg, 1.50 mmol) were added. A reaction occurred at 80°C for 20 hours while stirring, and then TLC showed complete reaction. Excess N,N-dimethylformamide was removed by concentration under reduced pressure, and the residue was purified by column chromatography (dichloromethane: methanol=10:1) to obtain a compound SMP-34421-2, weighing 60.0 mg, with a yield of 15%.

MS (ESI) m/z: 811[M+H]⁺.

¹HNMR (400 MHz, CDCl3) δ4.31 (s, 1H), 3.33-3.54(m, 38H), 3.11 (s, 3H), 2.46-2.65(m, 16H), 1.35 (s, 18H).

### Step 2: Preparation of compound SMP-34421-3

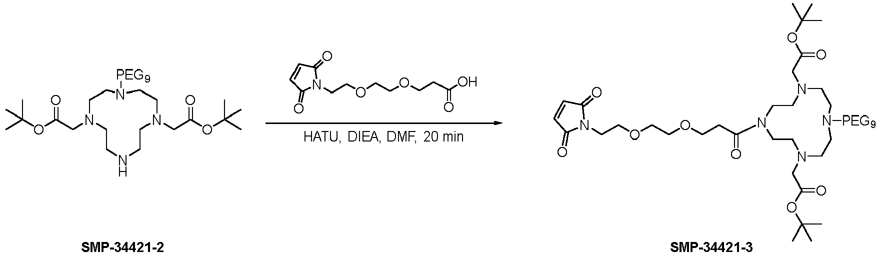

An intermediate maleimide-dipolyethylene glycol-carboxylic acid (22.0 mg, 0.086 mmol) and N,N-dimethylformamide (4 mL) were added to a 25 mL reaction flask. After complete clarification, N,N,N',N'-tetramethyl-O-(7-azabenzotriazol-1-yl)hexafluorophosphourea (34.0 mg, 0.089 mmol), N,N-diisopropylethylamine (30.0 mg, 0.22 mmol), and the SMP-34421-2 (60.0 mg, 0.074 mmol) were added. A reaction occurred at room temperature for 20 minutes while stirring, and then TLC showed complete reaction. The reaction solution was purified by HPLC to obtain a compound SMP-34421-3, weighing 50.0 mg, with a yield of 64%.

MS (ESI) m/z: 526[M/2+H]⁺.

### Step 3: Preparation of compound SMP-34421-4

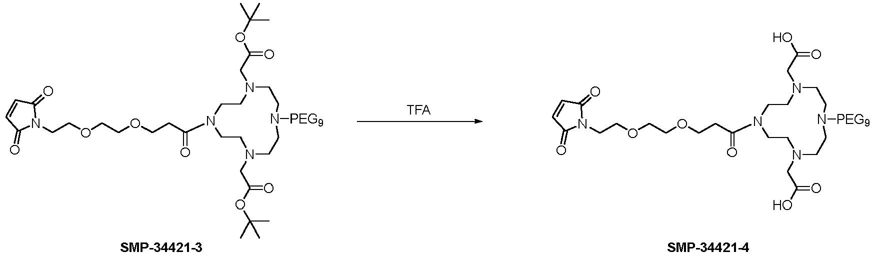

The SMP-34421-3 (50.0 mg, 0.048 mmol) was placed in a 25mL reaction flask, and trifluoroacetic acid (3mL) was added. A reaction occurred at room temperature for 3 hours while stirring, and then TLC showed complete reaction. The trifluoroacetic acid was removed by concentration under reduced pressure, and the residue was purified by HPLC to obtain a compound SMP-34421-4, weighing 25.0 mg, with a yield of 55.97%. MS (ESI) m/z: 470[M/2+H]⁺.

### Step 4: Preparation of compound SMP-34421-5

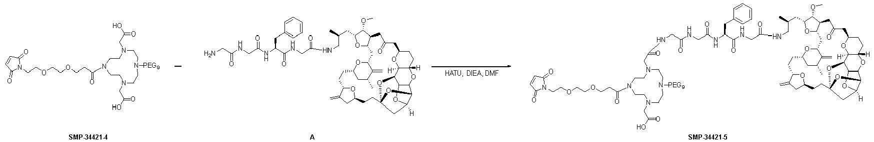

The SMP-34421-4 (25.0 mg, 0.027 mmol) was dissolved in N,N-dimethylformamide (2 mL). After complete clarification, the intermediate A(28.0 mg, 0.027 mmol) and N,N-diisopropylethylamine (7.0 mg, 0.054 mmol) were sequentially added, then an N,N-dimethylformamide (1 mL) solution of N,N,N',N'-tetramethyl-O-(7-azabenzotriazol-1-yl)hexafluorophosphourea (10.0 mg, 0.027 mmol) was slowly added dropwise, a reaction occurred at room temperature for 20 minutes while stirring, and then TLC showed complete reaction. The reaction solution was purified by HPLC to obtain a compound SMP-34421-5, weighing 20.0 mg, with a yield of 38.2%.

MS (ESI) m/z: 984[M/2+H]⁺.

### Step 5: Preparation of compound SMP-34421

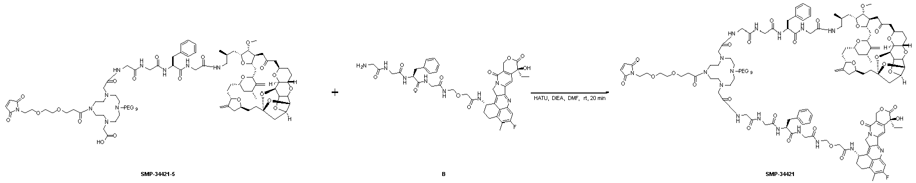

The SMP-34421-5 (20.0 mg, 0.010 mmol) was dissolved in N,N-dimethylformamide (2 mL). After complete clarification, the intermediate B (9.0 mg, 0.010 mmol), N,N,N',N'-tetramethyl-O-(7-azabenzotriazol-1-yl)hexafluorophosphourea (4.0 mg, 0.010 mmol), and N,N-diisopropylethylamine (4.0 mg, 0.031 mmol) were sequentially added, a reaction occurred at room temperature for 20 minutes while stirring, and then TLC showed complete reaction. The reaction solution was purified by HPLC to obtain a compound SMP-34421, weighing 13.0 mg, with a yield of 45.8%.

MS (ESI) m/z: 1395[M/2+H]⁺.

### Example 47: Preparation of compound SMP-53612

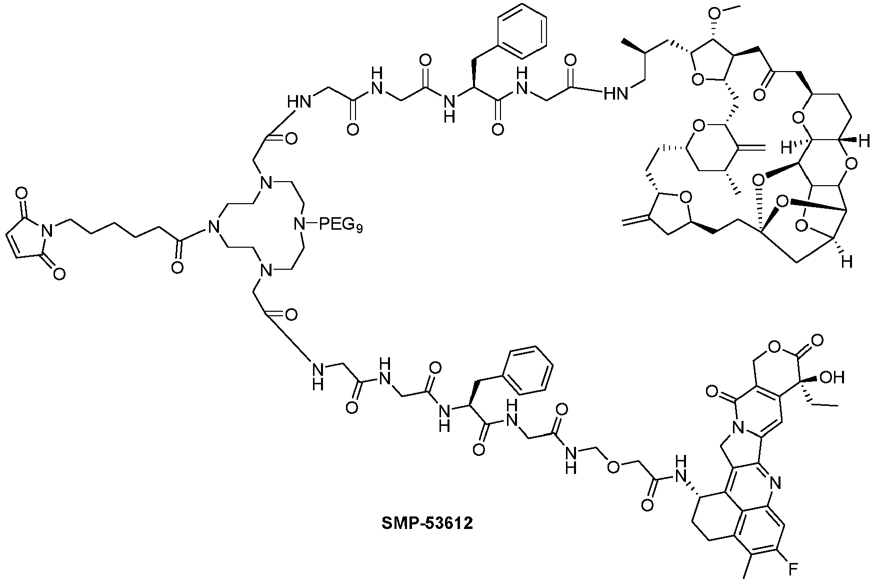

### Step 1: Preparation of compound SMP-53612-1

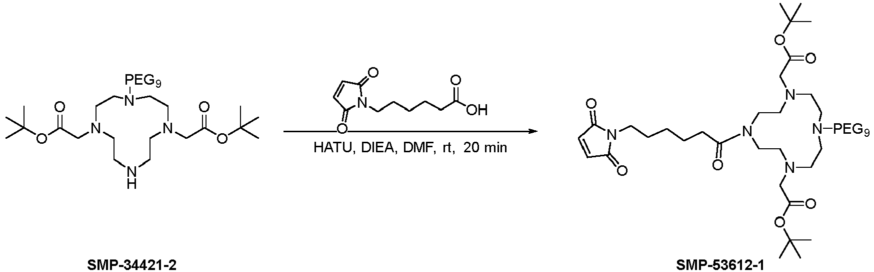

SMP-34421-2 (70. 0 mg, 0.086 mmol) was dissolved in N,N-dimethylformamide (2 mL). After complete clarification, 6-maleimidocaproic acid (22.0 mg, 0.10 mmol), N,N,N',N'-tetramethyl-O-(7-azabenzotriazol-1-yl)hexafluorophosphourea (39.0 mg, 0.10 mmol), and N,N-diisopropylethylamine (17.0 mg, 0.13 mmol) were sequentially added, a reaction occurred at room temperature for 20 minutes while stirring, and then TLC showed complete reaction. The reaction solution was purified by HPLC to obtain a compound SMP-53612-1, weighing 50 mg, with a yield of 57.7%. MS (ESI) m/z: 503[M/2+H]⁺.

### Step 2: Preparation of compound SMP-53612-2

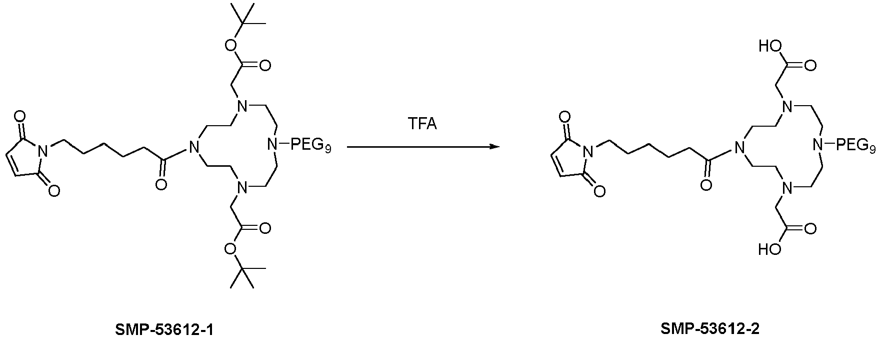

The SMP-53612-1 (50.0 mg, 0.05 mmol) was dissolved in trifluoroacetic acid (3 mL). A reaction occurred at room temperature for 3 hours while stirring, and then TLC showed complete reaction. The trifluoroacetic acid was removed by concentration under reduced pressure, and the residue was purified by HPLC to obtain a compound SMP-53612-2, weighing 34.0 mg, with a yield of 79.6%.

MS (ESI) m/z: 447[M/2+H]⁺.

### Step 3: Preparation of compound SMP-53612-3

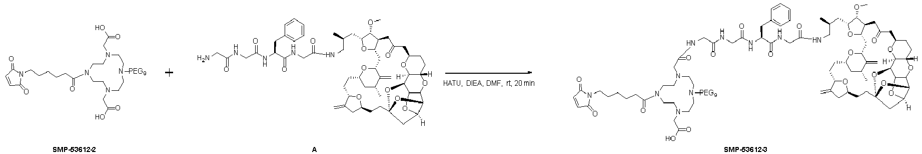

The SMP-53612-2 (34.0 mg, 0.038 mmol) was dissolved in N,N-dimethylformamide (2 mL). After complete clarification, the intermediate A(40.0 mg, 0.038 mmol) and N,N-diisopropylethylamine (15.0 mg, 0.11 mmol) were sequentially added, then an N,N-dimethylformamide (1 mL) solution of N,N,N',N'-tetramethyl-O-(7-azabenzotriazol-1-yl)hexafluorophosphourea (15.0 mg, 0.039 mmol) was slowly added dropwise, a reaction occurred at room temperature for 20 minutes while stirring, and then TLC showed complete reaction. The reaction solution was purified by HPLC to obtain a compound SMP-53612-3, weighing 24.0 mg, with a yield of 32.8%.

MS (ESI) m/z: 962[M/2+H]⁺.

### Step 4: Preparation of compound SMP-53612

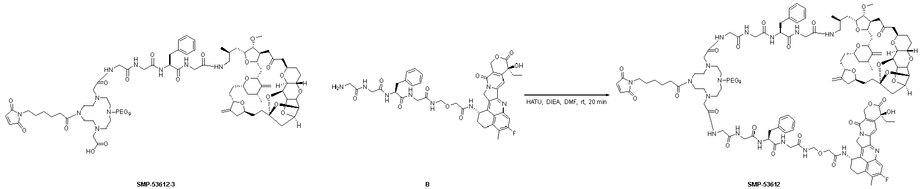

The SMP-53612-3 (24.0 mg, 0.012 mmol) was dissolved in N,N-dimethylformamide (2 mL). After complete clarification, the intermediate B (10.0 mg, 0.012 mmol), N,N,N',N'-tetramethyl-O-(7-azabenzotriazol-1-yl)hexafluorophosphourea (5.0 mg, 0.012 mmol), and N,N-diisopropylethylamine (5.0 mg, 0.039 mmol) were sequentially added, a reaction occurred at room temperature for 20 minutes while stirring, and then TLC showed complete reaction. The reaction solution was purified by HPLC to obtain a compound SMP-53612, weighing 10.0 mg, with a yield of 29.2%.

MS (ESI) m/z: 1373[M/2+H]⁺.

¹HNMR (400 MHz, DMSO-*d₆*) δ 8.68 (t, J = 6.6 Hz, 1H), 8.50 (t, J = 14.4 Hz, 3H), 8.36 (s, 1H), 8.28 (s, 1H), 8.18 (s, 2H), 8.12 (d, J = 11.6 Hz, 2H), 7.79 (d, J = 10.9 Hz, 1H), 7.71 (s, 1H), 7.31 (s, 1H), 7.26 - 7.12 (m, 10H), 7.00 (s, 2H), 5.63 - 5.56 (m, 1H), 5.41 (s, 2H), 5.20 (s, 2H), 5.02 (d, J = 21.8 Hz, 2H), 4.82 (s, 1H), 4.74 (s, 1H), 4.64 (d, J = 6.1 Hz, 3H), 4.54 (dd, J = 9.8, 5.5 Hz, 4H), 4.45 - 3.84 (m, 36H), 3.69 (m, 24H), 3.38 (m, 12H), 3.24 (d, J = 12.4 Hz, 10H), 3.17 - 2.96 (m, 8H), 2.92 - 2.62 (m, 13H), 2.38 (s, 3H), 2.33 - 2.05 (m, 12H), 2.04 - 1.81 (m, 8H), 1.67 (dd, J = 25.2, 13.1 Hz, 6H), 1.46 (dt, J = 15.1, 7.7 Hz, 6H), 1.31 (dd, J = 22.5, 8.0 Hz, 3H), 1.19 (dd, J = 21.3, 13.8 Hz, 4H), 0.99 (dd, J = 24.2, 8.9 Hz, 4H), 0.86 (t, J = 7.4 Hz, 3H).

### Example 48: Preparation of compound SMP-10215

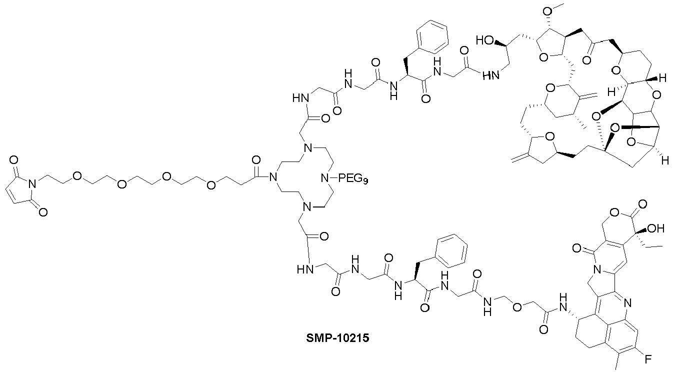

### Step 1: Preparation of compound SMP-10215-1

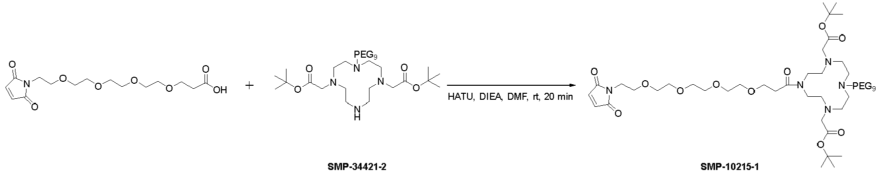

SMP-34421-2 (120.0 mg, 0.15 mmol) and maleimide-tetrapolyethylene glycol-carboxylic acid (51.0 mg, 0.15 mmol) were mixed and dissolved in N,N-dimethylformamide (4 mL). After complete clarification, N,N,N',N'-tetramethyl-O-(7-azabenzotriazol-1-yl)hexafluorophosphourea (56.0 mg, 0.15 mmol) and N,N-diisopropylethylamine (57.0 mg, 0.45 mmol) were sequentially added. A reaction occurred at room temperature for 20 minutes while stirring, and then TLC showed complete reaction. The reaction solution was purified by HPLC to obtain a compound SMP-10215-1, weighing 90.0 mg, with a yield of 53.4%.

MS (ESI) m/z: 570[M/2+H]⁺.

### Step 2: Preparation of compound SMP-10215-2

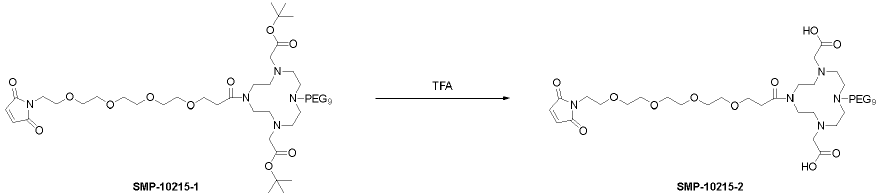

The SMP-10215-1 (90.0 mg, 0.079mmol) was dissolved in trifluoroacetic acid (3 mL). A reaction occurred at room temperature for 2 hours while stirring, and then TLC showed complete reaction. The trifluoroacetic acid was removed under reduced pressure, and the reaction solution was purified by HPLC to obtain a compound SMP-10215-2, weighing 20.0 mg, with a yield of 24.6%.

MS (ESI) m/z: 513[M/2+H]⁺.

### Step 3: Preparation of compound SMP-10215-3

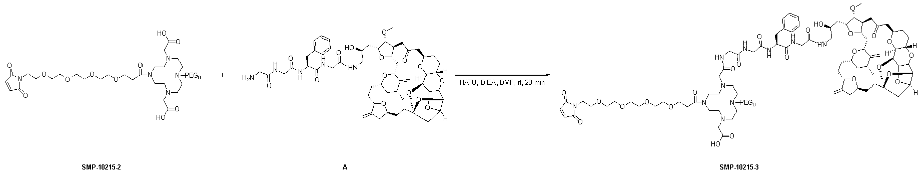

The SMP-10215-2 (20.0 mg, 0.02 mmol) was dissolved in N,N-dimethylformamide (1 mL). After complete clarification, the intermediate A (20.0 mg, 0.02 mmol) and N,N-diisopropylethylamine (7.0 mg, 0.06 mmol) were sequentially added, then an N,N-dimethylformamide (1 mL) solution of N,N,N',N'-tetramethyl-O-(7-azabenzotriazol-1-yl)hexafluorophosphourea (7.0 mg, 0.02 mmol) was slowly added dropwise, a reaction occurred at room temperature for 20 minutes while stirring, and then TLC showed complete reaction. The reaction solution was purified by HPLC to obtain a compound SMP-10215-3, weighing 19.0 mg, with a yield of 48.4%.

MS (ESI) m/z: 1027[M/2+H]⁺.

### Step 4: Preparation of compound SMP-10215

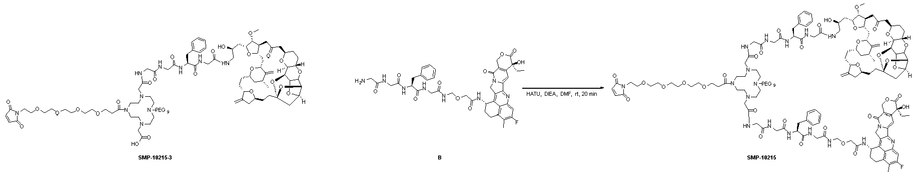

The SMP-10215-3 (19.0 mg, 0.0093 mmol) and the intermediate B (8.0 mg, 0.0095 mmol) were mixed and dissolved in N,N-dimethylformamide (1 mL). After complete clarification, N,N,N',N'-tetramethyl-O-(7-azabenzotriazol-1-yl)hexafluorophosphourea (4.0 mg, 0.011 mmol) and N,N-diisopropylethylamine (4.0 mg, 0.031 mmol) were sequentially added, a reaction occurred at room temperature for 20 minutes while stirring, and then TLC showed complete reaction. The reaction solution was purified by HPLC to obtain a compound SMP-10215, weighing 12.0 mg, with a yield of 43.8%.

MS (ESI) m/z: 1439[M/2+H]⁺.

### Example 49: Preparation of compound SMP-34710

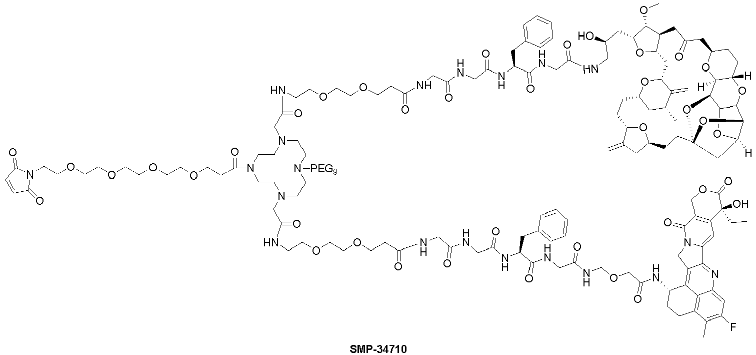

### Step 1: Preparation of compound SMP-34710-1

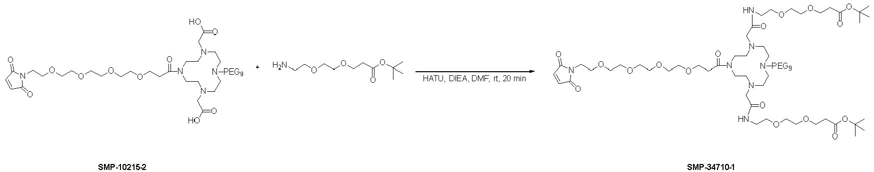

SMP-10215-2 (60.0 mg, 0.06 mmol) and an intermediate tert-butyl 9-amino-4,7-dioxanonanoate (27.0 mg, 0.12 mmol) were mixed and dissolved in N,N-dimethylformamide (2 mL). After complete clarification, N,N,N',N'-tetramethyl-O-(7-azabenzotriazol-1-yl)hexafluorophosphourea (44.0 mg, 0.12 mmol) and N,N-diisopropylethylamine (30.0 mg, 0.24 mmol) were sequentially added, a reaction occurred at room temperature for 20 minutes while stirring, and then TLC showed complete reaction. The reaction solution was purified by HPLC to obtain a compound SMP-34710-1, weighing 45.0 mg, with a yield of 52.9%.

MS (ESI) m/z: 728[M/2+H]⁺.

### Step 2: Preparation of compound SMP-34710-2

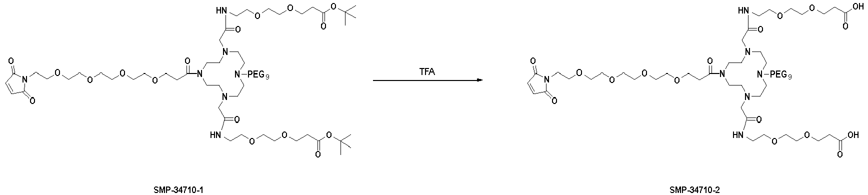

The SMP-34710-1 (45.0 mg, 0.03 mmol) was dissolved in trifluoroacetic acid (3 mL). A reaction occurred at room temperature for 2 hours while stirring, and then TLC showed complete reaction. The trifluoroacetic acid was removed by concentration under reduced pressure, and the reaction solution was purified by HPLC to obtain a compound SMP-34710-2, weighing 20.0 mg, with a yield of 48.2%.

MS (ESI) m/z: 672[M/2+H]⁺.

### Step 3: Preparation of compound SMP-34710-3

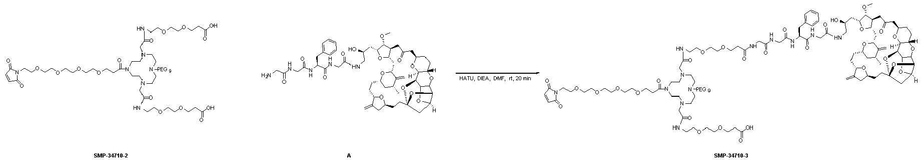

The SMP-34710-2 (20.0 mg, 0.015 mmol) and the intermediate A (16.0 mg, 0.015 mmol) were mixed and dissolved in N,N-dimethylformamide (2 mL). After complete clarification, N,N-diisopropylethylamine (4.0 mg, 0.031 mmol) was added, then an N,N-dimethylformamide (1 mL) solution of N,N,N',N'-tetramethyl-O-(7-azabenzotriazol-1-yl)hexafluorophosphourea (5.7 mg, 0.015 mmol) was slowly added dropwise, a reaction occurred at room temperature for 20 minutes while stirring, and then TLC showed complete reaction. The reaction solution was purified by HPLC to obtain a compound SMP-34710-3, weighing 12.0 mg, with a yield of 34.0%.

MS (ESI) m/z: 1187[M/2+H]⁺.

### Step 4: Preparation of compound SMP-34710

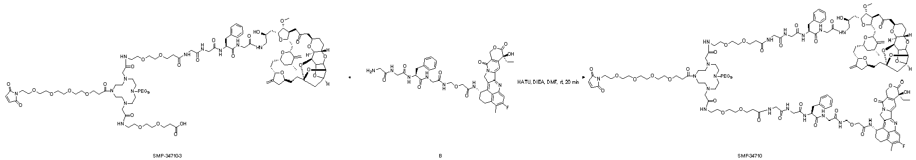

The SMP-34170-3 (12.0 mg, 0.005 mmol) and the intermediate B (4.2 mg, 0.005 mmol) were mixed and dissolved in N,N-dimethylformamide (2 mL). After complete clarification, N,N,N',N'-tetramethyl-O-(7-azabenzotriazol-1-yl)hexafluorophosphourea (1.9 mg, 0.005 mmol) and N,N-diisopropylethylamine (2.0 mg, 0.015 mmol) were sequentially added, a reaction occurred at room temperature for 20 minutes while stirring, and then TLC showed complete reaction. A compound SMP-34710 was obtained by reversed phase preparation, weighing 9.0 mg, with a yield of 55.7%.

MS (ESI) m/z: 1598[M/2+H]⁺.

### Example 50: Preparation of compound SMP-44419

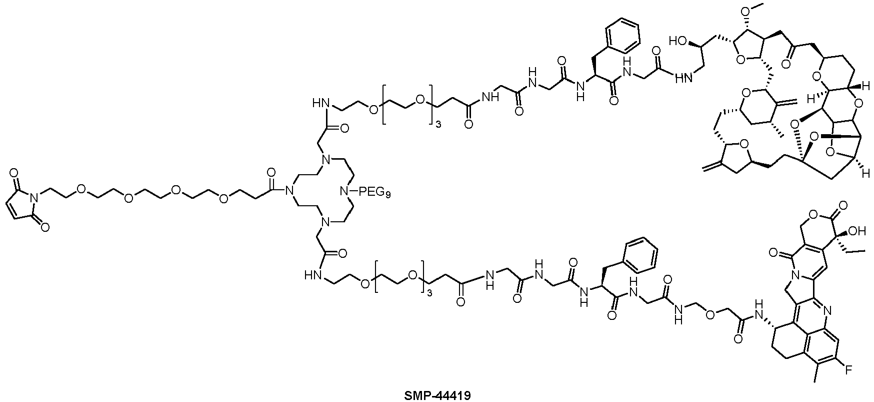

### Step 1: Preparation of compound SMP-44419-1

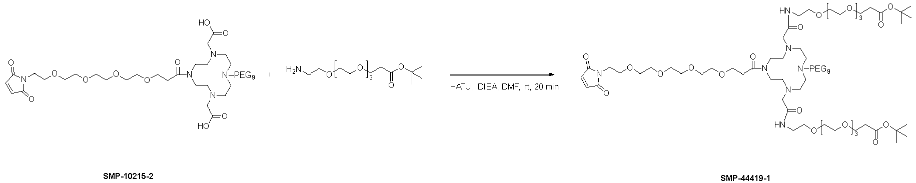

SMP-10215-2 (60.0 mg, 0.06 mmol) and an intermediate tert-butyl 15-amino-4,7,10,13-tetraoxatepentadecanoate (38.0 mg, 0.12 mmol), were mixed and dissolved in N,N-dimethylformamide (2 mL). After complete clarification, N,N,N',N'-tetramethyl-O-(7-azabenzotriazol-1-yl)hexafluorophosphourea (45.6 mg, 0.12 mmol) and N,N-diisopropylethylamine (30.9 mg, 0.24 mmol) were sequentially added, a reaction occurred at room temperature for 20 minutes while stirring, and then TLC showed complete reaction. The reaction solution was purified by HPLC to obtain a compound SMP-4419-1, weighing 48.0 mg, with a yield of 50.3%.

MS (ESI) m/z: 816[M/2+H]⁺.

### Step 2: Preparation of compound SMP-44419-2

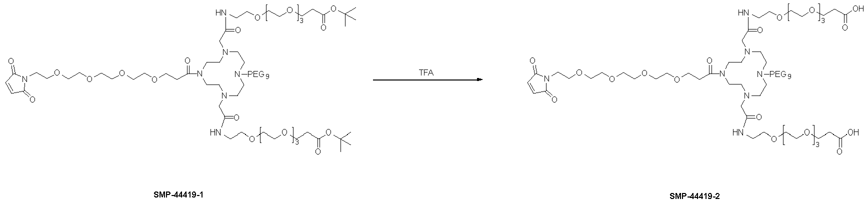

The intermediate SMP-44419-1 (48.0 mg, 0.029 mmol) was dissolved in trifluoroacetic acid (3 mL). A reaction occurred at room temperature for 2 hours while stirring, and then TLC showed complete reaction. The trifluoroacetic acid was removed by concentration under reduced pressure, and the residue was purified by HPLC to obtain a compound SMP-44419-2, weighing 20.0 mg, with a yield of 44.7%.

MS (ESI) m/z: 760[M/2+H]⁺.

### Step 3: Preparation of compound SMP-44419-3

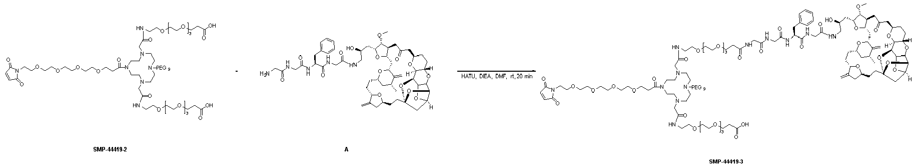

The intermediate SMP-44419-2 (20.0 mg, 0.013 mmol), the intermediate A (14.0 mg, 0.013 mmol), N,N-dimethylformamide (2 mL), and N,N-diisopropylethylamine (5.0 mg, 0.039 mmol) were added to a 10 mL reaction flask. After complete clarification, an N,N-dimethylformamide (1 mL) solution of N,N,N',N'-tetramethyl-O-(7-azabenzotriazol-1-yl)hexafluorophosphourea (5.0 mg, 0.013 mmol) was slowly added dropwise. A reaction occurred at room temperature for 20 minutes while stirring, and then TLC showed complete reaction. The reaction solution was purified by HPLC to obtain a compound SMP-44419-3, weighing 10.0 mg, with a yield of 29.8%.

MS (ESI) m/z: 1274[M/2+H]⁺.

### Step 4: Preparation of compound SMP-44419

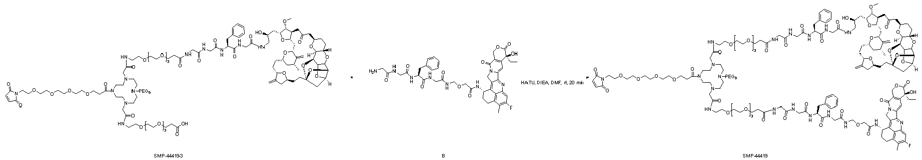

The intermediate SMP-44419-3 (9.0 mg, 0.004 mmol), the intermediate B (3.4 mg, 0.004 mmol), and N,N-dimethylformamide (1 mL) were added to a 10 mL reaction flask. After complete clarification, N,N,N',N'-tetramethyl-O-(7-azabenzotriazol-1-yl)hexafluorophosphourea (2.0 mg, 0.004 mmol) and N,N-diisopropylethylamine (1.6 mg, 0.012 mmol) were sequentially added, a reaction occurred at room temperature for 20 minutes while stirring, and then TLC showed complete reaction. The reaction solution was purified by HPLC to obtain a compound SMP-44419, weighing 2.0 mg, with a yield of 16.8%.

MS (ESI) m/z: 1124[M/3+H]⁺.

### Example 51: Preparation of compound SMP-35055

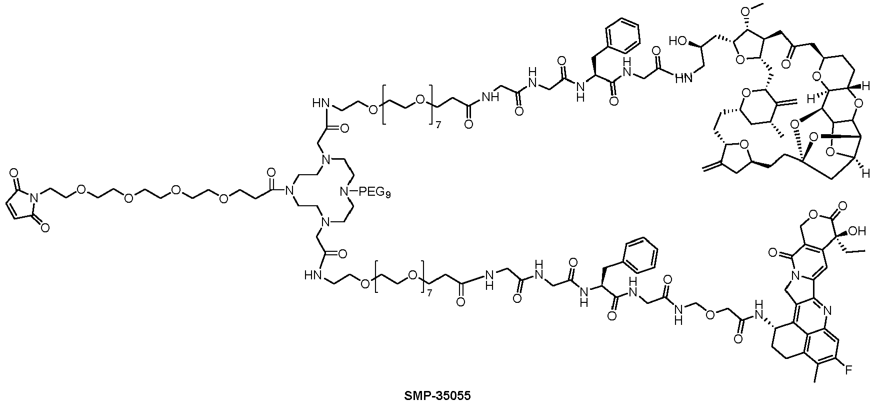

### Step 1: Preparation of compound SMP-35055-1

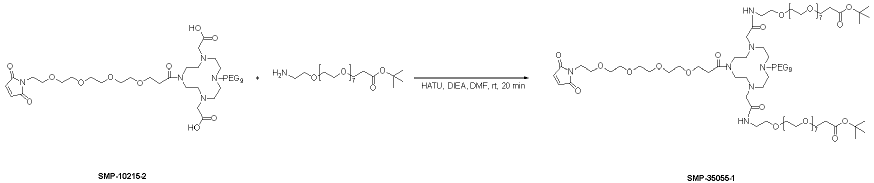

The intermediate SMP-10215-2 (60.0 mg, 0.06 mmol), amino-octapolyethylene glycol-tert-butyl propionate (58.0 mg, 0.12 mmol), and N,N-dimethylformamide (3 mL) were added to a 10 mL reaction flask. After complete clarification, N,N,N',N'-tetramethyl-O-(7-azabenzotriazol-1-yl)hexafluorophosphourea (45.6 mg, 0.12 mmol) and N,N-diisopropylethylamine (30.0 mg, 0.24 mmol) were sequentially added, a reaction occurred at room temperature for 20 minutes while stirring, and then TLC showed complete reaction. The reaction solution was purified by HPLC to obtain a compound SMP-35055-1, weighing 50.0 mg, with a yield of 43.1%.

MS (ESI) m/z: 993[M/2+H]⁺.

### Step 2: Preparation of compound SMP-35055-2

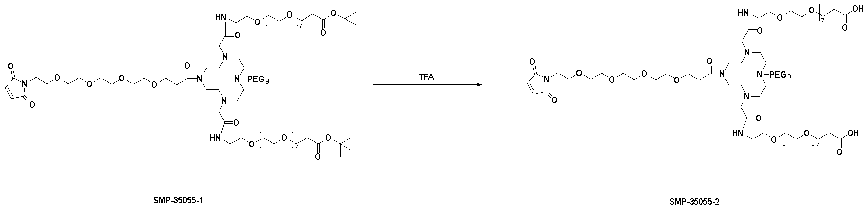

The SMP-35055-1 (50.0 mg, 0.03 mmol) was dissolved in trifluoroacetic acid (3 mL). A reaction occurred at room temperature for 2 hours while stirring, and then TLC showed complete reaction. The trifluoroacetic acid was removed by concentration under reduced pressure, and the reaction solution was purified by HPLC to obtain a compound SMP-35055-2, weighing 20.0 mg, with a yield of 42.4%.

MS (ESI) m/z: 937[M/2+H]⁺.

### Step 3: Preparation of compound SMP-35055-3

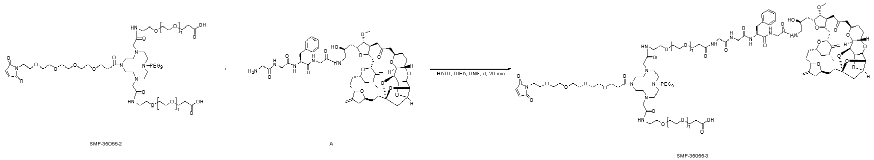

The intermediate SMP-35055-2 (20.0 mg, 0.011 mmol), the intermediate A (11.5 mg, 0.011 mmol), N,N-dimethylformamide (2 mL), and N,N-diisopropylethylamine (4.0 mg, 0.032 mmol) were added to a 10 mL reaction flask. After complete clarification, an N,N-dimethylformamide (1 mL) solution of N,N,N',N'-tetramethyl-O-(7-azabenzotriazol-1-yl)hexafluorophosphourea (4.2 mg, 0.011 mmol) was slowly added dropwise. A reaction occurred at room temperature for 20 minutes while stirring, and then TLC showed complete reaction. The reaction solution was purified by HPLC to obtain a compound SMP-35055-3, weighing 8.0 mg, with a yield of 25.8%.

MS (ESI) m/z: 968[M/3+H]⁺.

### Step 4: Preparation of compound SMP-35055

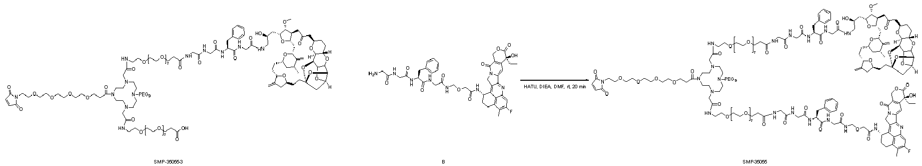

The intermediate SMP-35055-3 (8.0 mg, 0.003 mmol), the intermediate B (2.5 mg, 0.003 mmol), and N,N-dimethylformamide (1 mL) were added to a 10 mL reaction flask. After complete clarification, N,N,N',N'-tetramethyl-O-(7-azabenzotriazol-1-yl)hexafluorophosphourea (1.1 mg, 0.003 mmol) and N,N-diisopropylethylamine (1.0 mg, 0.008 mmol) were sequentially added, a reaction occurred at room temperature for 20 minutes while stirring, and then TLC showed complete reaction. The reaction solution was purified by HPLC to obtain a compound SMP-35055, weighing 1.2 mg, with a yield of 11.7%.

MS (ESI) m/z: 1242[M/2+H]⁺.

### Example 52: Preparation of compound SMP-94170-A

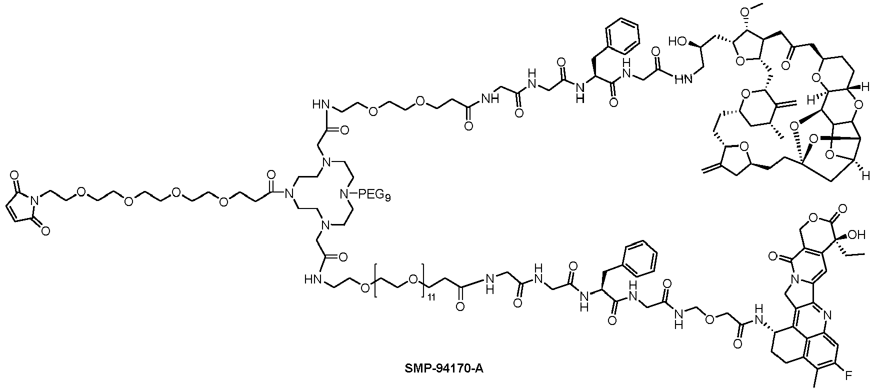

### Step 1: Preparation of compound SMP-94170-1

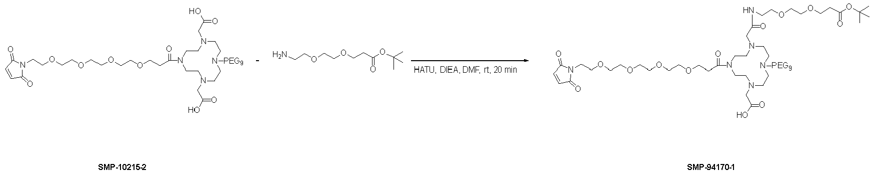

The intermediate SMP-10215-2 (250.0 mg, 0.24 mmol) was dissolved in N,N-dimethylformamide (5 mL), and an intermediate tert-butyl 9-amino-4,7-dioxanonanoate (57.0 mg, 0.24 mmol) and N,N-diisopropylethylamine (94.0 mg, 0.73 mmol) were sequentially added. After complete clarification, an N,N-dimethylformamide (1 mL) solution of N,N,N',N'-tetramethyl-O-(7-azabenzotriazol-1-yl)hexafluorophosphourea (93.0 mg, 0.24 mmol) was slowly added dropwise. A reaction occurred at room temperature for 20 minutes while stirring, and then TLC showed complete reaction. The reaction solution was purified by HPLC to obtain a compound SMP-94170-1, weighing 110 mg, with a yield of 36.4%.

MS (ESI) m/z: 621[M/2+H]⁺.

### Step 2: Preparation of compound SMP-94170-2

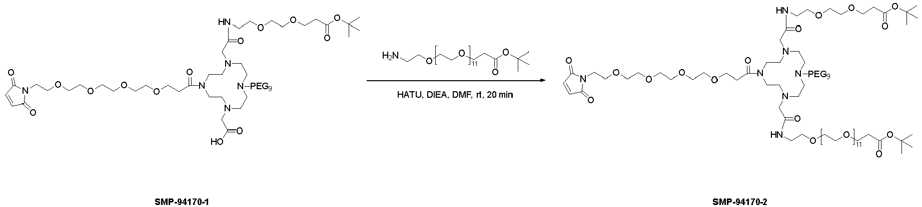

The intermediate SMP-94170-1 (110.0 mg, 0.09 mmol), amino-dodecaethylene glycol-tert-butyl propionate (59.0 mg, 0.09 mmol), and N,N-dimethylformamide (3 mL) were added to a 10 mL reaction flask. After complete clarification, N,N,N',N'-tetramethyl-O-(7-azabenzotriazol-1-yl)hexafluorophosphourea (34.2 mg, 0.09 mmol) and N,N-diisopropylethylamine (34.8 mg, 0.27 mmol) were sequentially added, a reaction occurred at room temperature for 20 minutes while stirring, and then TLC showed complete reaction. The reaction solution was purified by HPLC to obtain a compound SMP-94170-2, weighing 110 mg, with a yield of 65.5%.

MS (ESI) m/z: 948[M/2+H]⁺.

### Step 3: Preparation of compound SMP-94170-3

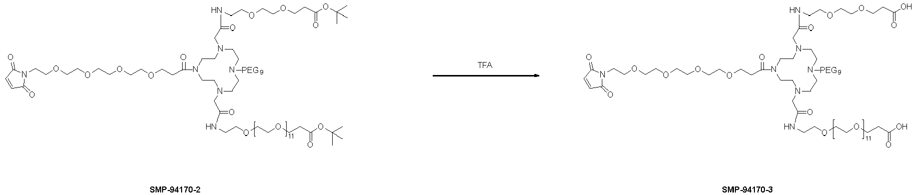

The SMP-94170-2 (110.0 mg, 0.06 mmol) was dissolved in trifluoroacetic acid (3 mL). A reaction occurred at room temperature for 2 hours while stirring, and then TLC showed complete reaction. The trifluoroacetic acid was removed by concentration under reduced pressure, and the reaction solution was purified by HPLC to obtain a compound SMP-94170-3, weighing 70 mg, with a yield of 67.6%.

MS (ESI) m/z: 893[M/2+H]⁺.

### Step 4: Preparation of compounds SMP-94170-A-1 and SMP-94170-B-1

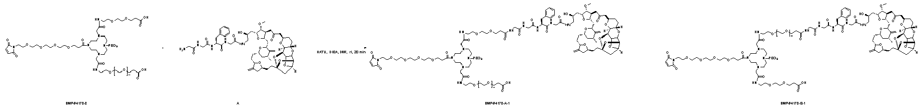

The SMP-94170-3 (70.0 mg, 0.04 mmol) was dissolved in N,N-dimethylformamide (3 mL), and A (41.0 mg, 0.04 mmol) and N,N-diisopropylethylamine (15.0mg, 0.12 mmol) were sequentially added. After complete clarification, an N,N-dimethylformamide (1 mL) solution of N,N,N',N'-tetramethyl-O-(7-azabenzotriazol-1-yl)hexafluorophosphourea (15.0 mg, 0.04 mmol) was slowly added dropwise. A reaction occurred at room temperature for 20 minutes while stirring, and then TLC showed complete reaction. The reaction solution was purified by HPLC to obtain a compound SMP-94170-A-1, weighing 18.0 mg, with a yield of 16.3%, and a compound SMP-94170-B-1, weighing 30.0 mg, with a yield of 27.2%.

MS (ESI) m/z: 1407[M/2+H]⁺.

### Step 5: Preparation of compound SMP-94170-A

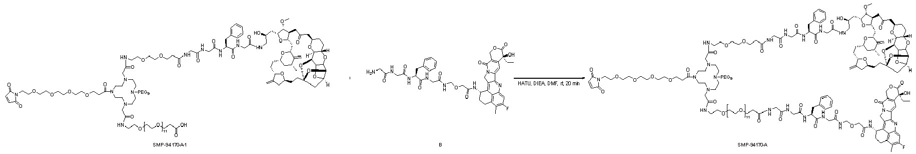

The intermediate SMP-94170-A-1 (18.0 mg, 0.006 mmol), the intermediate B (5.0 mg, 0.006 mmol), and N,N-dimethylformamide (2 mL) were sequentially added to a 10 mL reaction flask. After complete clarification, N,N,N',N'-tetramethyl-O-(7-azabenzotriazol-1-yl)hexafluorophosphourea (2.3 mg, 0.006 mmol) and N,N-diisopropylethylamine (2.5 mg, 0.019 mmol) were sequentially added, a reaction occurred at room temperature for 20 minutes while stirring, and then TLC showed complete reaction. The reaction solution was purified by HPLC to obtain a compound SMP-94170-A, weighing 10.0 mg, with a yield of 43.0%.

MS (ESI) m/z: 1212[M/3+H]⁺.

### Example 53: Preparation of compound SMP-94170-B

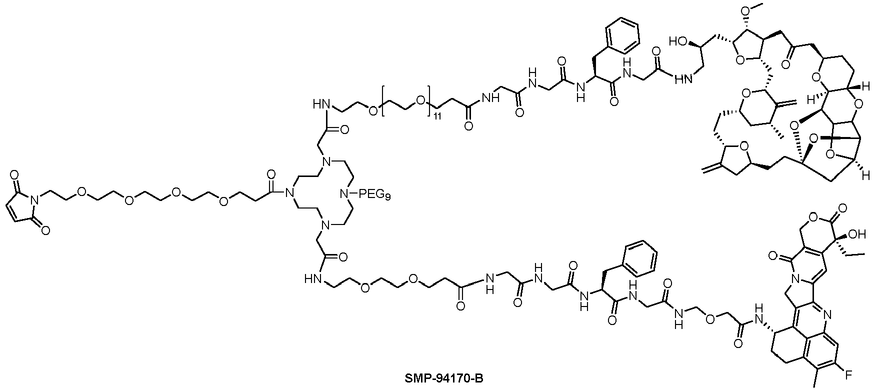

### Step 1: Preparation of compound SMP-94170-B

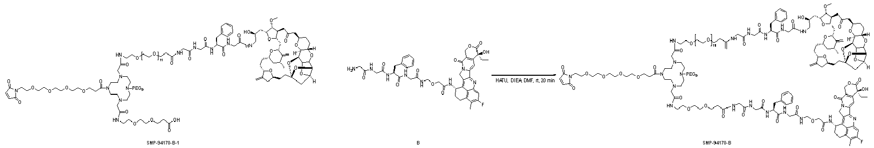

The SMP-94170-B-1 (30.0 mg, 0.011 mmol) was dissolved in N,N-dimethylformamide (2 mL). After complete clarification, the intermediate B (9.0 mg, 0.011 mmol), N,N,N',N'-tetramethyl-O-(7-azabenzotriazol-1-yl)hexafluorophosphourea (4.0 mg, 0.011 mmol), and N,N-diisopropylethylamine (4.3 mg, 0.033 mmol) were sequentially added, a reaction occurred at room temperature for 20 minutes while stirring, and then TLC showed complete reaction. The reaction solution was purified by HPLC to obtain a compound SMP-94170-B, weighing 15 mg, with a yield of 38.7%.

MS (ESI) m/z: 1212[M/3+H]⁺.

### Example 54: Preparation of compound SMP-46940

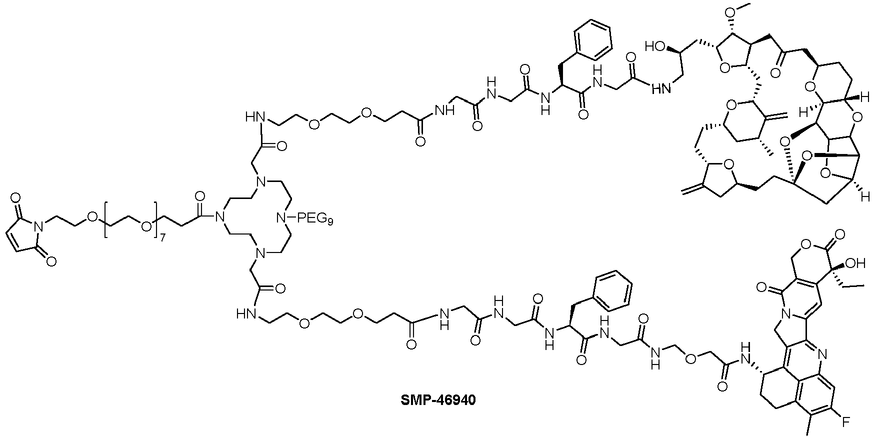

### Step 1: Preparation of compound SMP-46940-1

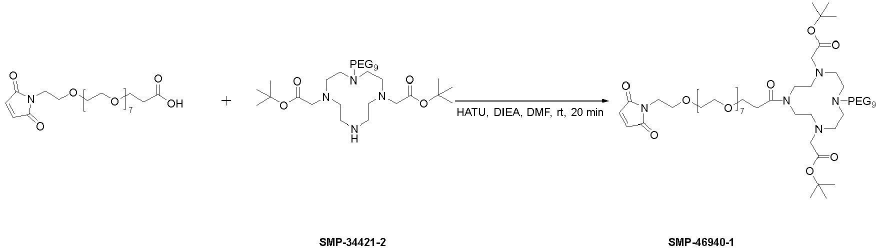

SMP-34421-2 (300.0 mg, 0.37 mmol) was dissolved in N,N-dimethylformamide (5 mL). After complete clarification, maleimide-octapolyethylene glycol-carboxylic acid (193.0 mg, 0.37 mmol), N,N,N',N'-tetramethyl-O-(7-azabenzotriazol-1-yl)hexafluorophosphourea (141.0 mg, 0.37 mmol), and N,N-diisopropylethylamine (143.0 mg, 1.11 mmol) were sequentially added, a reaction occurred at room temperature for 20 minutes while stirring, and then TLC showed complete reaction. The reaction solution was purified by HPLC to obtain a compound SMP-46940-1, weighing 132.0 mg, with a yield of 27.2%.

MS (ESI) m/z: 657[M/2+H]⁺.

### Step 2: Preparation of compound SMP-46940-2

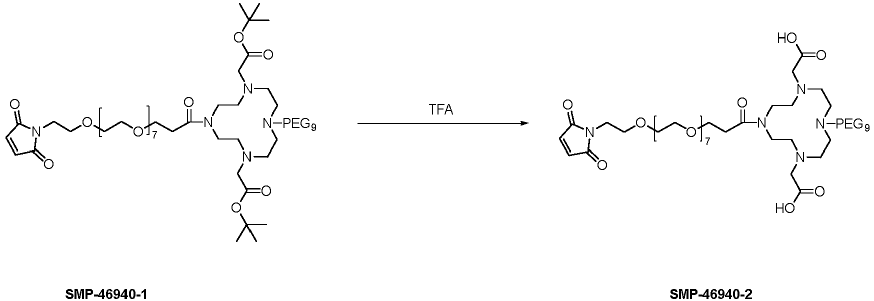

The intermediate SMP-46940-1 (132.0 mg, 0.10 mmol) and trifluoroacetic acid (3 mL) were sequentially added to a 10 mL reaction flask. A reaction occurred at room temperature for 2 hours while stirring, and then TLC showed complete reaction. The trifluoroacetic acid was removed by concentration under reduced pressure, and the reaction solution was purified by HPLC to obtain a compound SMP-46940-2, weighing 60.0 mg, with a yield of 49.7%.

MS (ESI) m/z: 601[M/2+H]⁺.

### Step 3: Preparation of compound SMP-46940-3

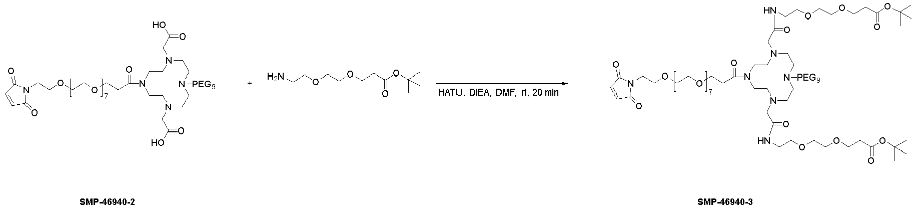

The intermediate SMP-46940-3 (60.0 mg, 0.05 mmol), tert-butyl 9-amino-4,7-dioxanonanoate (23.0 mg, 0.10 mmol), and N,N-dimethylformamide (2 mL) were added to a 10 mL reaction flask. After complete clarification, N,N,N',N'-tetramethyl-O-(7-azabenzotriazol-1-yl)hexafluorophosphourea (38.0 mg, 0.10 mmol) and N,N-diisopropylethylamine (39.0 mg, 0.30 mmol) were sequentially added. A reaction occurred at room temperature for 20 minutes while stirring, and then TLC showed complete reaction. The reaction solution was purified by HPLC to obtain a compound SMP-46940-3, weighing 40.0 mg, with a yield of 49.1%.

MS (ESI) m/z: 816[M/2+H]⁺.

### Step 4: Preparation of compound SMP-46940-4

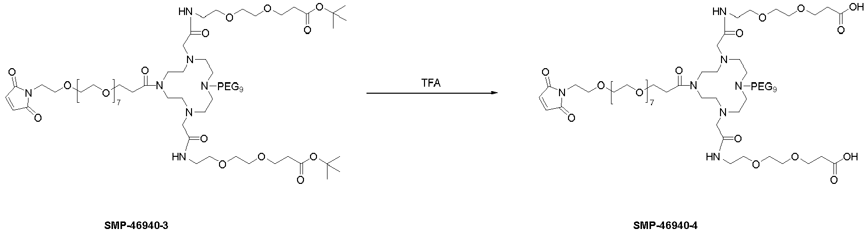

The intermediate SMP-46940-3 (40.0 mg, 0.025 mmol) and trifluoroacetic acid (3 mL) were sequentially added to a 10 mL reaction flask. A reaction occurred at room temperature for 2 hours while stirring, and then TLC showed complete reaction. The trifluoroacetic acid was removed by concentration under reduced pressure, and the reaction solution was purified by HPLC to obtain a compound SMP-46940-4, weighing 25.0 mg, with a yield of 67.1%.

MS (ESI) m/z: 760[M/2+H]⁺.

### Step 5: Preparation of compound SMP-46940-5

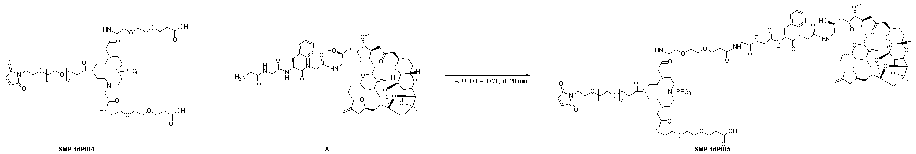

The SMP-46940-4 (25.0 mg, 0.016 mmol) was dissolved in N,N-dimethylformamide (2 mL), and the intermediate A (17.0 mg, 0.016 mmol) and N,N-diisopropylethylamine (6.2 mg, 0.048 mmol) were sequentially added. After complete clarification, an N,N-dimethylformamide (1 mL) solution of N,N,N',N'-tetramethyl-O-(7-azabenzotriazol-1-yl)hexafluorophosphourea (6.0 mg, 0.016 mmol) was slowly added dropwise. A reaction occurred at room temperature for 20 minutes while stirring, and then TLC showed complete reaction. The reaction solution was purified by HPLC to obtain a compound SMP-46940-5, weighing 14.0 mg, with a yield of 33.4%.

MS (ESI) m/z: 1275[M/2+H]⁺.

### Step 5: Preparation of compound SMP-46940

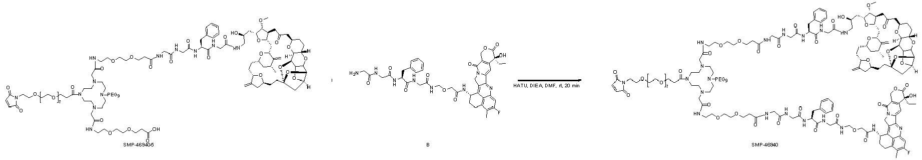

The intermediate SMP-46940-5 (14.0 mg, 0.005 mmol), the intermediate B (5.0 mg, 0.005 mmol), and N,N-dimethylformamide (2 mL) were sequentially added to a 10 mL reaction flask. After complete clarification, N,N,N',N'-tetramethyl-O-(7-azabenzotriazol-1-yl)hexafluorophosphourea (2.0 mg, 0.005 mmol) and N,N-diisopropylethylamine (2.0 mg, 0.015 mmol) were added. A reaction occurred at room temperature for 20 minutes while stirring, and then TLC showed complete reaction. The reaction solution was purified by HPLC to obtain a compound SMP-46940, weighing 9.0 mg, with a yield of 48.6%.

MS (ESI) m/z: 1124[M/3+H]⁺.

### Example 55: Preparation of compound SMP-13069

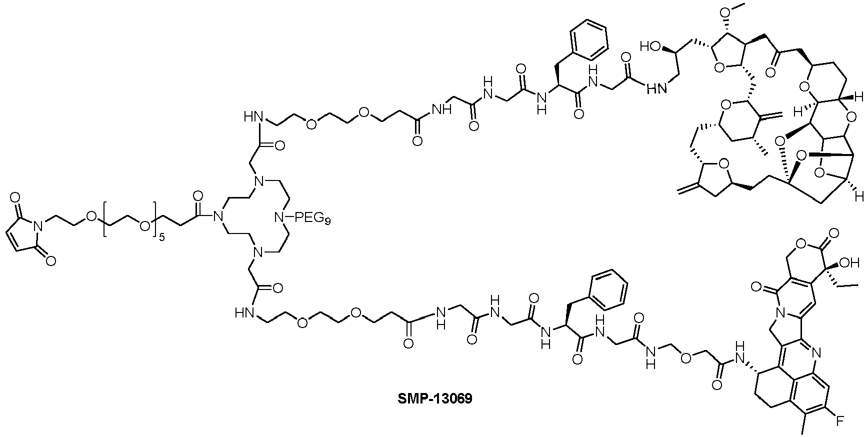

### Step 1: Preparation of compound SMP-13069-1

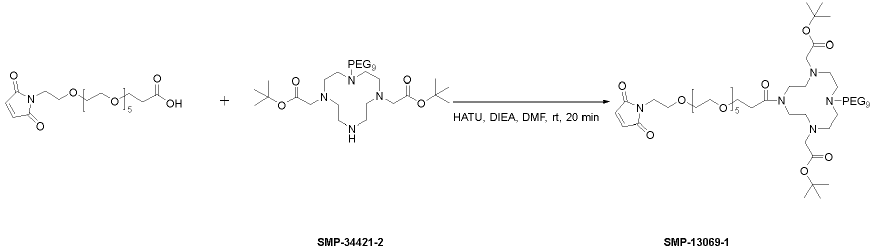

The intermediate SMP-34421-2 (300.0 mg, 0.37 mmol), maleimide-hexapolyethylene glycol-carboxylic acid (160.0 mg, 0.37 mmol), and N,N-dimethylformamide (5 mL) were sequentially added to a 25 mL reaction flask. After complete clarification, N,N,N',N'-tetramethyl-O-(7-azabenzotriazol-1-yl)hexafluorophosphourea (140.7 mg, 0.37 mmol) and N,N-diisopropylethylamine (143.2 mg, 1.11 mmol) were added. A reaction occurred at room temperature for 20 minutes while stirring, and then TLC showed complete reaction. The reaction solution was purified by HPLC to obtain a compound SMP-13069-1, weighing 153.0 mg, with a yield of 33.7%.

MS (ESI) m/z: 613[M/2+H]⁺.

### Step 2: Preparation of compound SMP-13069-2

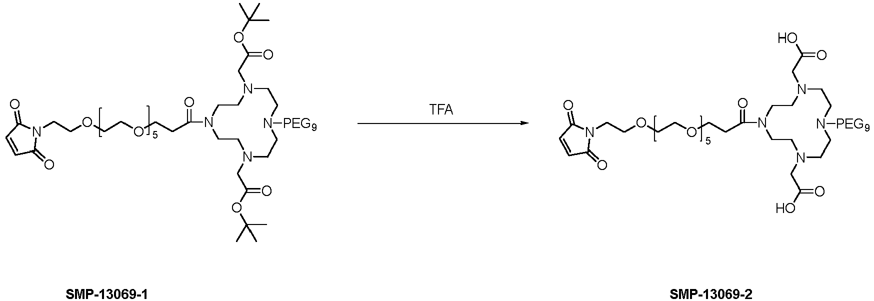

The intermediate SMP-13069-1 (153.0 mg, 0.12 mmol) and trifluoroacetic acid (3 mL) were sequentially added to a 10 mL reaction flask. A reaction occurred at room temperature for 2 hours while stirring, and then TLC showed complete reaction. The trifluoroacetic acid was removed by concentration under reduced pressure, and the reaction solution was purified by HPLC to obtain a compound SMP-13069-2, weighing 80.0 mg, with a yield of 57.6%.

MS (ESI) m/z: 557[M/2+H]⁺.

### Step 3: Preparation of compound SMP-13069-3

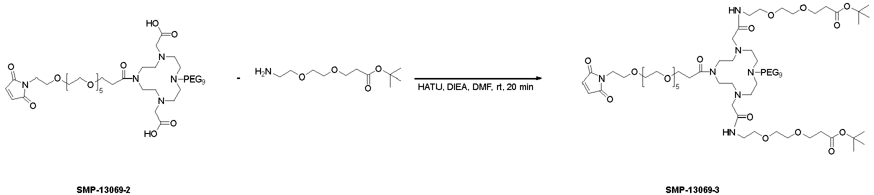

The intermediate SMP-13069-3 (80.0 mg, 0.072 mmol), tert-butyl 9-amino-4,7-dioxanonanoate (33.6 mg, 0.144 mmol), and N,N-dimethylformamide (2 mL) were sequentially added to a 10 mL reaction flask. After complete clarification, N,N,N',N'-tetramethyl-O-(7-azabenzotriazol-1-yl)hexafluorophosphourea (54.0 mg, 0.144 mmol) and N,N-diisopropylethylamine (55.0 mg, 0.43 mmol) were sequentially added. A reaction occurred at room temperature for 20 minutes while stirring, and then TLC showed complete reaction. The reaction solution was purified by HPLC to obtain a compound SMP-13069-3, weighing 60.0 mg, with a yield of 53.8%.

MS (ESI) m/z: 772[M/2+H]⁺.

### Step 4: Preparation of compound SMP-13069-4

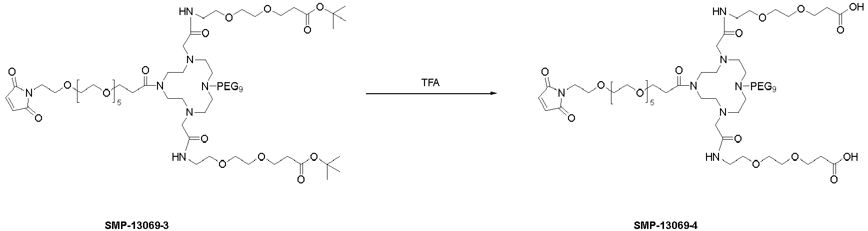

The intermediate SMP-13069-3 (60.0 mg, 0.039 mmol) and trifluoroacetic acid (3 mL) were sequentially added to a 10 mL reaction flask. A reaction occurred at room temperature for 2 hours while stirring, and then TLC showed complete reaction. The trifluoroacetic acid was removed by concentration under reduced pressure, and the reaction solution was purified by HPLC to obtain a compound SMP-13069-4, weighing 35.0 mg, with a yield of 62.9%. MS (ESI) m/z: 716[M/2+H]⁺.

### Step 5: Preparation of compound SMP-13069-5

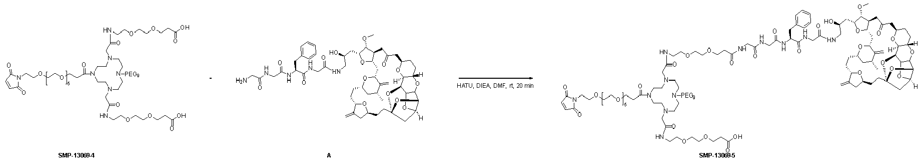

The intermediate SMP-13069-4 (35.0 mg, 0.024 mmol), the intermediate A (25.0 mg, 0.024 mmol), N,N-dimethylformamide (2 mL), and N,N-diisopropylethylamine (9.3 mg, 0.072 mmol) were sequentially added to a 10 mL reaction flask. After complete clarification, an N,N-dimethylformamide (1 mL) solution of N,N,N',N'-tetramethyl-O-(7-azabenzotriazol-1-yl)hexafluorophosphourea (9.0 mg, 0.024 mmol) was slowly added dropwise. A reaction occurred at room temperature for 20 minutes while stirring, and then TLC showed complete reaction. The reaction solution was purified by HPLC to obtain a compound SMP-13069-5, weighing 18.0 mg, with a yield of 29.9%.

MS (ESI) m/z: 1231[M/2+H]⁺.

### Step 6: Preparation of compound SMP-13069

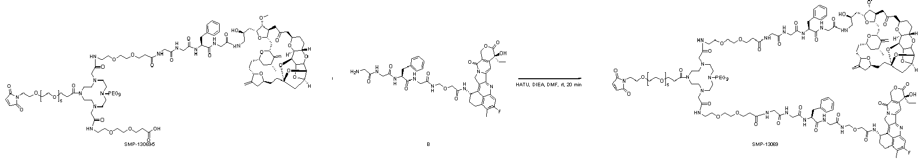

The intermediate SMP-13069-5 (18.0 mg, 0.007 mmol), the intermediate B (6.0 mg, 0.007 mmol), and N,N-dimethylformamide (2 mL) were sequentially added to a 10 mL reaction flask. After complete clarification, N,N,N',N'-tetramethyl-O-(7-azabenzotriazol-1-yl)hexafluorophosphourea (3.0 mg, 0.007 mmol) and N,N-diisopropylethylamine (3.0 mg, 0.021 mmol) were added. A reaction occurred at room temperature for 20 minutes while stirring, and then TLC showed complete reaction. The reaction solution was purified by HPLC to obtain a compound SMP-13069, weighing 9.5 mg, with a yield of 39.6%.

MS (ESI) m/z: 1095[M/3+H]⁺.

### Example 56: Preparation of compound SMP-28945

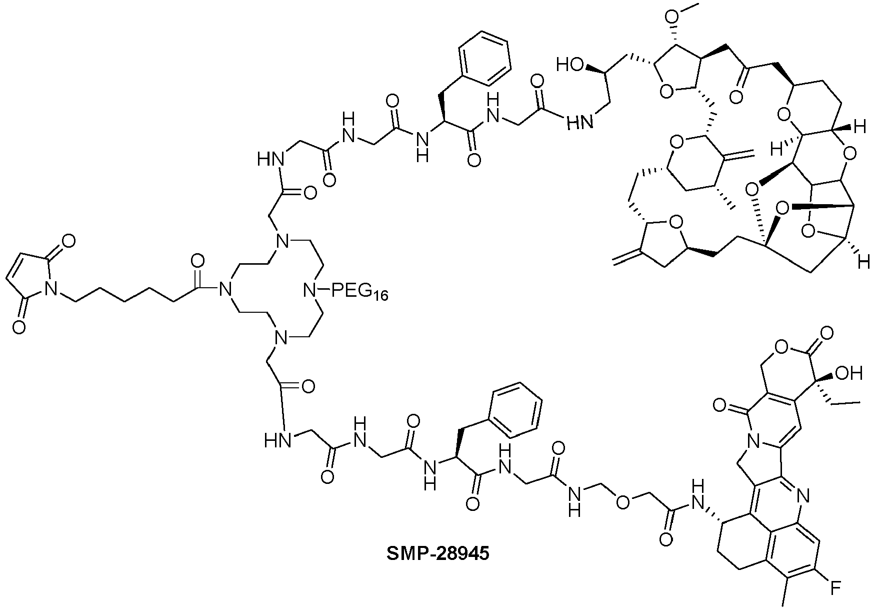

### Step 1: Preparation of compound SMP-28945-1

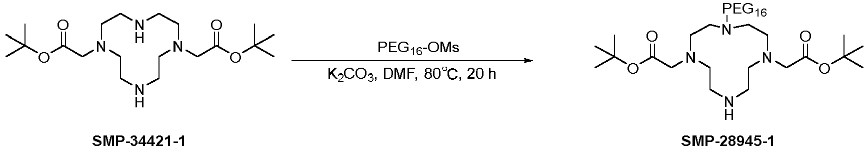

The intermediate SMP-34421-1 (250.0 mg, 0.62 mmol) and N,N-dimethylformamide (10 mL) were sequentially added to a 50 mL reaction flask. After complete clarification, PEG₁₆-OMs (525.0 mg, 0.62 mmol) and potassium carbonate (258.0 mg, 1.87 mmol) were added. A reaction occurred at 80°C for 20 hours while stirring, and then TLC showed complete reaction. The N,N-dimethylformamide was removed by concentration under reduced pressure, and the residue was purified by silica gel column chromatography (dichloromethane: methanol=10:1) to obtain a compound SMP-28945-1, weighing 180.0 mg, with a yield of 25.8%.

MS (ESI) m/z: 560[M/2+H]⁺.

### Step 2: Preparation of compound SMP-28945-2

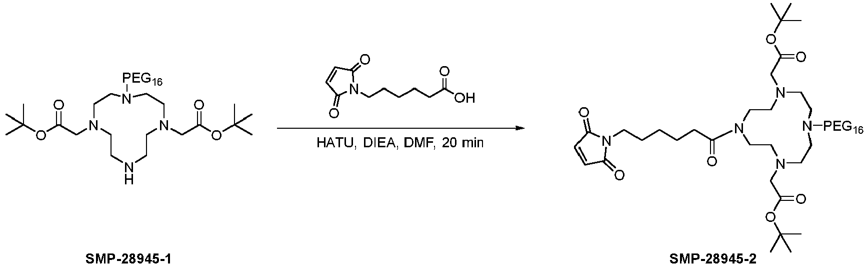

The intermediate SMP-28945-1 (180.0 mg, 0.16 mmol) and N,N-dimethylformamide (5 mL) were sequentially added to a 25mL reaction flask. After complete clarification, 6-maleimidocaproic acid (34.0 mg, 0.16 mmol), N,N,N',N'-tetramethyl-O-(7-azabenzotriazol-1-yl)hexafluorophosphourea (61.0 mg, 0.16 mmol), and N,N-diisopropylethylamine (62.0 mg, 0.48 mmol) were sequentially added. A reaction occurred at room temperature for 20 minutes while stirring, and then TLC showed complete reaction. The reaction solution was purified by HPLC to obtain a compound SMP-28945-2, weighing 120.0 mg, with a yield of 56.9%.

MS (ESI) m/z: 656[M/2+1]⁺.

### Step 3: Preparation of compound SMP-28945-3

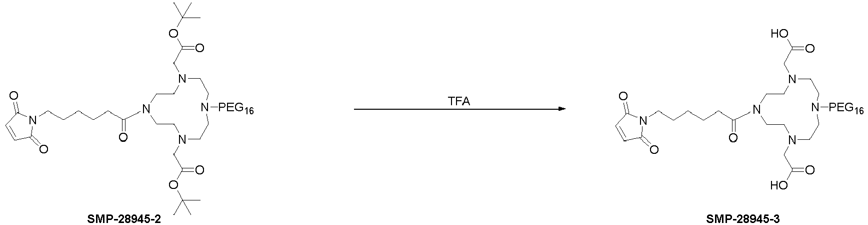

The intermediate SMP-28945-2 (120.0 mg, 0.092 mmol) and trifluoroacetic acid (3 mL) were sequentially added to a 10 mL reaction flask. A reaction occurred at room temperature for 2 hours while stirring, and then TLC showed complete reaction. The trifluoroacetic acid was removed by concentration under reduced pressure, and the reaction solution was purified by HPLC to obtain a compound SMP-28945-3, weighing 80.0 mg, with a yield of 72.9%.

MS (ESI) m/z: 600[M/2+H]⁺.

### Step 4: Preparation of compound SMP-28945-4

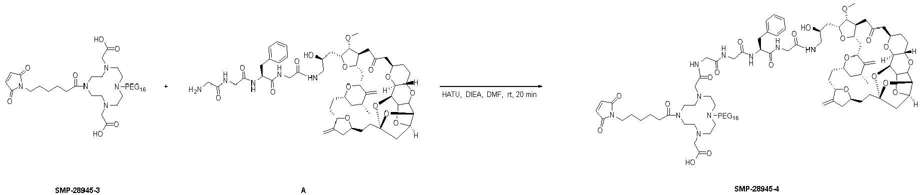

The intermediate SMP-28945-3 (30.0 mg, 0.025 mmol), the intermediate A (26.0 mg, 0.025 mmol), N,N-dimethylformamide (2 mL), and N,N-diisopropylethylamine (10.0 mg, 0.075 mmol) were sequentially added to a 10 mL reaction flask. After complete clarification, an N,N-dimethylformamide (1 mL) solution of N,N,N',N'-tetramethyl-O-(7-azabenzotriazol-1-yl)hexafluorophosphourea (10 mg, 0.025 mmol) was slowly added dropwise. A reaction occurred at room temperature for 20 minutes while stirring, and then TLC showed complete reaction. The reaction solution was purified by HPLC to obtain a compound SMP-28945-4, weighing 20.0 mg, with a yield of 35.9%.

MS (ESI) m/z: 1115[M/2+H]⁺.

### Step 5: Preparation of compound SMP-28945

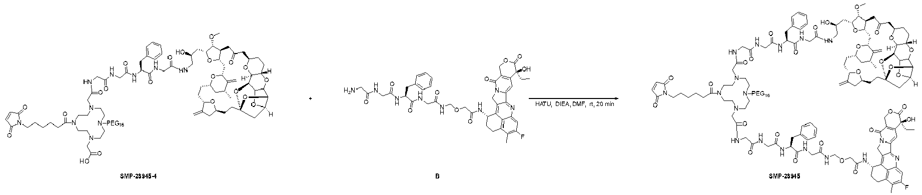

The intermediate SMP-28945-4 (20.0 mg, 0.009 mmol), the intermediate B (7.6 mg, 0.009 mmol), and N,N-dimethylformamide (2 mL) were sequentially added to a 10 mL reaction flask. After complete clarification, N,N,N',N'-tetramethyl-O-(7-azabenzotriazol-1-yl)hexafluorophosphourea (3.4 mg, 0.009 mmol) and N,N-diisopropylethylamine (3.5 mg, 0.027 mmol) were added. A reaction occurred at room temperature for 20 minutes while stirring, and then TLC showed complete reaction. The reaction solution was purified by HPLC to obtain a compound SMP-28945, weighing 2.6 mg, with a yield of 9.5%.

MS (ESI) m/z: 1017[M/3+H]⁺.

### Example 57: Preparation of compound SMP-28823

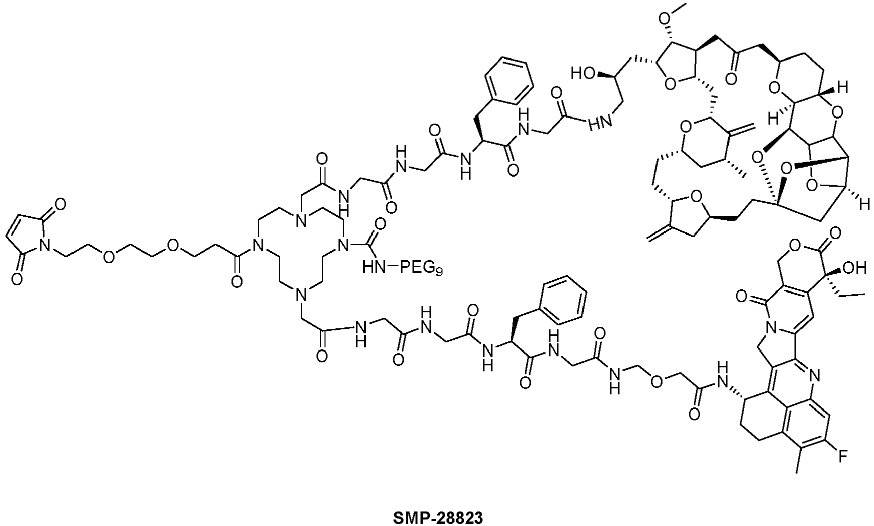

### Step 1: Preparation of compound SMP-28823-1

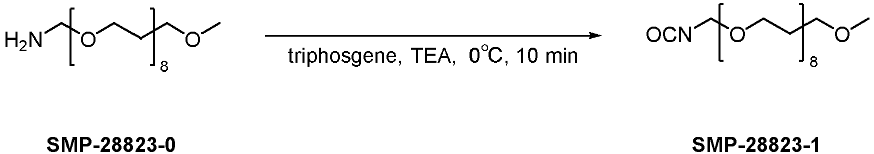

Amino nonaethylene glycol monomethyl ether (300.0 mg, 0.70 mmol), triphosgene (206.0 mg, 0.70 mmol), and dichloromethane (10 mL) were sequentially added to a 50 mL reaction flask. After complete clarification, triethylamine (1 mL) was added. A reaction occurred at 0°C for 20 minutes while stirring, and then TLC showed complete reaction. The dichloromethane was removed by concentration under reduced pressure, and a crude compound SMP-28823-1 was obtained, weighing 300.0 mg. MS (ESI) m/z: 454[M+H]⁺.

### Step 2: Preparation of compound SMP-28823-2

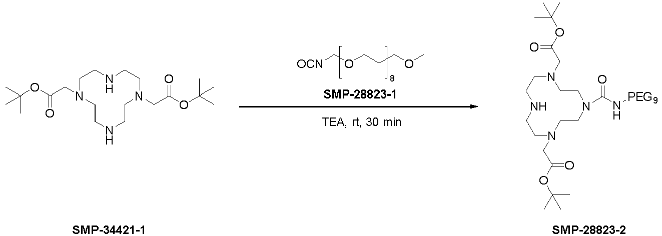

The crude intermediate SMP-28823-1 (300.0 mg, 0.66 mmol), the intermediate SMP-34421-1 (300.0 mg, 0.75 mmol), and dichloromethane (10 mL) were sequentially added to a 50 mL reaction flask. After complete clarification, triethylamine (1 mL) was added. A reaction occurred at room temperature for 20 minutes while stirring, and then LC-TLC showed complete reaction. The dichloromethane was removed by concentration under reduced pressure, and the residue was purified by HPLC to obtain a compound SMP-28823-2, weighing 300.0 mg, with a yield of 53.1%.

MS (ESI) m/z: 854[M+H]⁺.

### Step 3: Preparation of compound SMP-28823-3

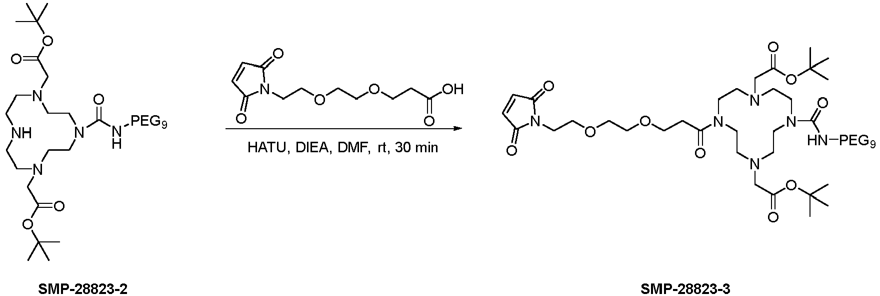

The intermediate SMP-28823-2 (230.0 mg, 0.27 mmol) and N,N-dimethylformamide (5 mL) were added to a 25 mL reaction flask. After complete clarification, maleimide-dipolyethylene glycol-carboxylic acid (69.4 mg, 0.27 mmol), N,N,N',N'-tetramethyl-O-(7-azabenzotriazol-1-yl)hexafluorophosphourea (102.7 mg, 0.27 mmol), and N,N-diisopropylethylamine (104.5 mg, 0.81 mmol) were sequentially added. A reaction occurred at room temperature for 30 minutes while stirring, and then TLC showed complete reaction. The reaction solution was purified by HPLC to obtain a compound SMP-28823-3, weighing 200.0 mg, with a yield of 67.9%.

MS (ESI) m/z: 547[M+H]⁺.

### Step 4: Preparation of compound SMP-28823-4

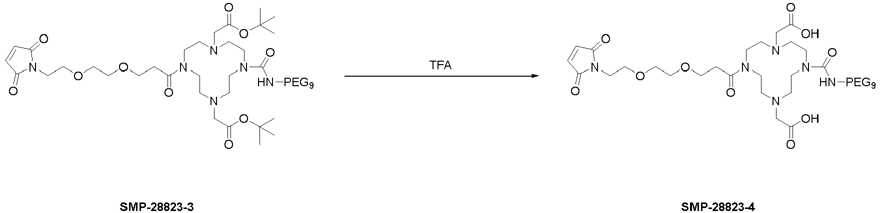

The intermediate SMP-28823-3 (200 mg, 0.18 mmol) and trifluoroacetic acid (3 mL) were sequentially added to a 10 mL reaction flask. A reaction occurred at room temperature for 2 hours while stirring, and then TLC showed complete reaction. The trifluoroacetic acid was removed by concentration under reduced pressure, and the reaction solution was purified by HPLC to obtain a compound SMP-28823-4, weighing 40.0 mg, with a yield of 22.3%. MS (ESI) m/z: 491[M/2+H]⁺.

### Step 5: Preparation of compound SMP-28823-5

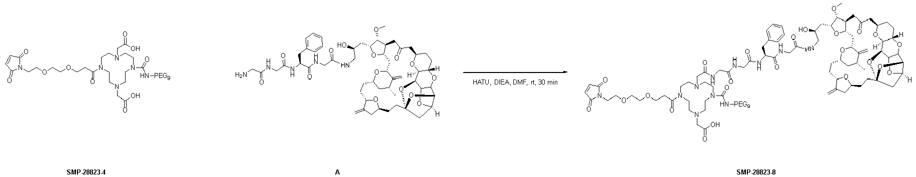

The intermediate SMP-28823-4 (40.0 mg, 0.041 mmol), the intermediate A (42.9 mg, 0.041 mmol), N,N-dimethylformamide (3 mL), and N,N-diisopropylethylamine (16.8 mg, 0.13 mmol) were sequentially added to a 10 mL reaction flask. After complete clarification, an N,N-dimethylformamide (1 mL) solution of N,N,N',N'-tetramethyl-O-(7-azabenzotriazol-1-yl)hexafluorophosphourea (15.7 mg, 0.041 mmol) was slowly added dropwise. A reaction occurred at room temperature for 30 minutes while stirring, and then TLC showed complete reaction. The reaction solution was purified by HPLC to obtain a compound SMP-28823-5, weighing 17.0 mg, with a yield of 20.7%. MS (ESI) m/z: 1005[M/2+H]⁺.

### Step 6: Preparation of compound SMP-28823

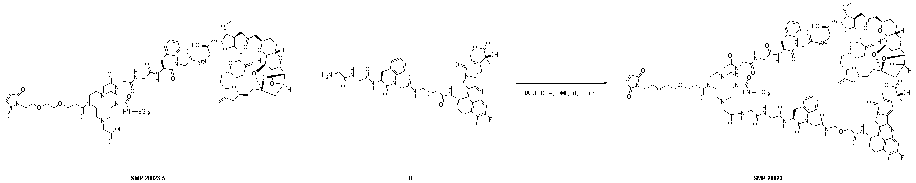

The intermediate SMP-28823-5 (17.0 mg, 0.0085 mmol), the intermediate B (7.1 mg, 0.0085 mmol), and N,N-dimethylformamide (2 mL) were sequentially added to a 10 mL reaction flask. After complete clarification, N,N,N',N'-tetramethyl-O-(7-azabenzotriazol-1-yl)hexafluorophosphourea (3.3 mg, 0.0085 mmol) and N,N-diisopropylethylamine (3.3 mg, 0.026 mmol) were added. A reaction occurred at room temperature for 30 minutes while stirring, and then TLC showed complete reaction. The reaction solution was purified by HPLC to obtain a compound SMP-28823, weighing 12 mg, with a yield of 50.1%.

MS (ESI) m/z: 944[M/3+H]⁺.

### Example 58: Preparation of compound SMP-53816

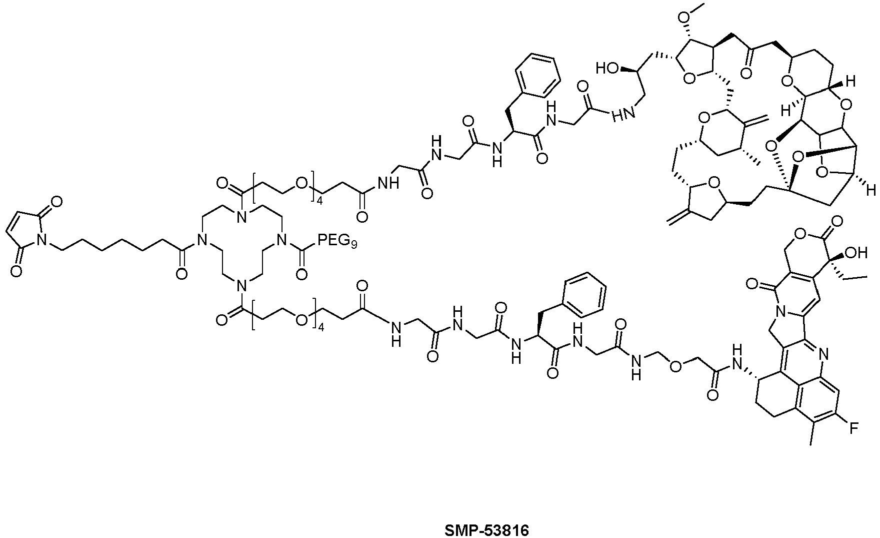

### Step 1: Preparation of compound SMP-53816-1

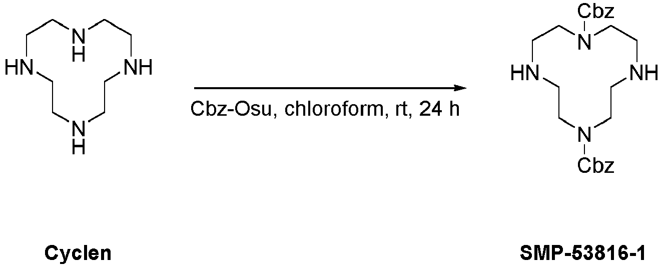

Cycleanine (1.0 g, 5.81 mmol), N-benzyloxycarbonyloxy)succinimide (2.9 g, 11.62 mmol), and trichloromethane (20 mL) were sequentially added to a 100 mL reaction flask. A reaction occurred at room temperature for 24 hours while stirring, and then TLC showed complete reaction. Excess trichloromethane was removed under reduced pressure, and the reaction solution was purified by HPLC to obtain a compound SMP-53816-1, weighing 2.0 g, with a yield of 78.1%.

MS (ESI) m/z: 441[M+H]⁺.

### Step 2: Preparation of compound SMP-53816-2

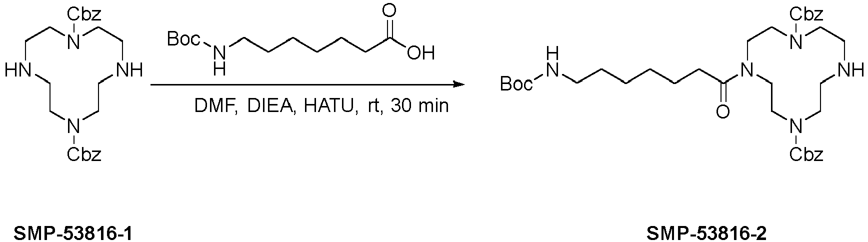

The intermediate SMP-53816-1 (1.0 g, 2.27 mmol), N-tert-butoxycarbonyl-7-aminoheptanoic acid (556.2 mg, 2.27 mmol), and N,N-dimethylformamide (20 mL) were sequentially added to a 50 mL reaction flask. After complete clarification, N,N,N',N'-tetramethyl-O-(7-azabenzotriazol-1-yl)hexafluorophosphourea (863.2 mg, 2.27 mol) and N,N-diisopropylethylamine (879.0 mg, 6.81 mol) were added. A reaction occurred at room temperature for 30 minutes while stirring, and then TLC showed complete reaction. A compound SMP-53816-2 was obtained by reversed phase preparation, weighing 300.0 mg, with a yield of 19.7%.

MS (ESI) m/z: 668[M+H]⁺.

### Step 3: Preparation of compound SMP-53816-3

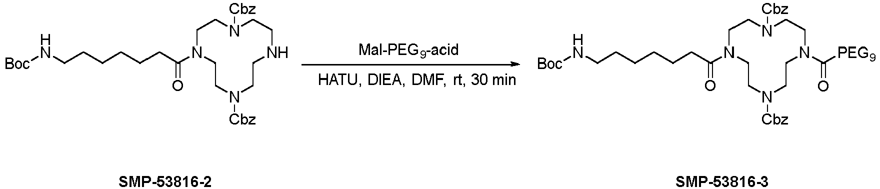

The intermediate SMP-53816-2 (300.0 mg, 0.45 mmol), maleimide-nonapolyethylene glycol-carboxylic acid (286.0 mg, 0.45 mmol), and N,N-dimethylformamide (5 mL) were sequentially added to a 25 mL reaction flask. After complete clarification, N,N,N',N'-tetramethyl-O-(7-azabenzotriazol-1-yl)hexafluorophosphourea (171.1 mg, 0.45 mol) and N,N-diisopropylethylamine (174.2 mg, 1.35 mmol) were added. A reaction occurred at room temperature for 30 minutes while stirring, and then TLC showed complete reaction. The reaction solution was purified by HPLC to obtain a compound SMP-53816-3, weighing 300.0 mg, with a yield of 60.4%.

MS (ESI) m/z: 553[M/2+H]⁺.

### Step 4: Preparation of compound SMP-53816-4

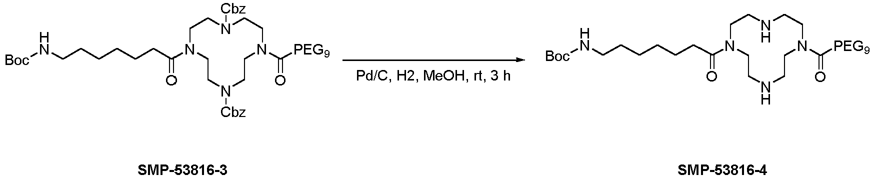

The intermediate SMP-53816-3 (300.0 mg, 0.27 mmol) and methanol (5 mL) were sequentially added to a 25 mL reaction flask. After complete clarification, palladium carbon (60 mg) was added, air was replaced with a hydrogen atmosphere, and a reaction occurred at room temperature for 3 hours while stirring. TLC showed complete reaction, the reaction solution was filtered, and the filtrate was concentrated to obtain a compound SMP-53816-4, weighing 220.0 mg, with a yield of 96.8%.

MS (ESI) m/z: 838[M+H]⁺.

### Step 5: Preparation of compound SMP-53816-5

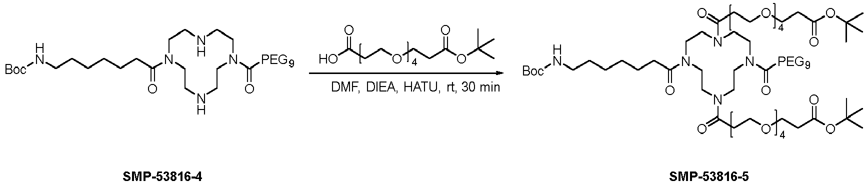

The intermediate SMP-53816-4 (220 mg, 0.26 mmol), tert-butyl-tetrapolyethylene glycol-carboxylate (182.0 mg, 0.52 mmol), and N,N-dimethylformamide (3 mL) were sequentially added to a 25 mL reaction flask. After complete clarification, N,N,N',N'-tetramethyl-O-(7-azabenzotriazol-1-yl)hexafluorophosphourea (197.8 mg, 0.52 mol) and N,N-diisopropylethylamine (100.6 mg, 0.78 mol) were added. A reaction occurred at room temperature for 30 minutes while stirring, and then TLC showed complete reaction. The reaction solution was purified by HPLC to obtain a compound SMP-53816-5, weighing 360 mg, with a yield of 91.2%.

MS (ESI) m/z: 751[M/2+H]⁺.

### Step 6: Preparation of compound SMP-53816-6

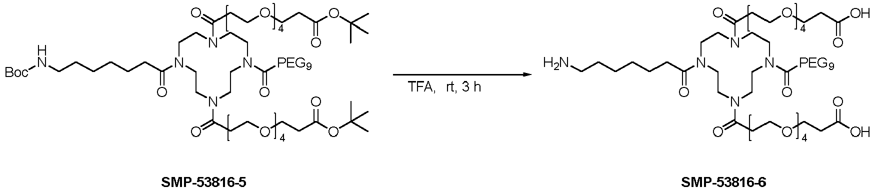

The intermediate SMP-53816-5 (360.0 mg, 0.24 mmol) and trifluoroacetic acid (5 mL) were sequentially added to a 10 mL reaction flask. A reaction occurred at room temperature for 3 hours while stirring, and then TLC showed complete reaction. The trifluoroacetic acid was removed by concentration under reduced pressure, and the reaction solution was purified by HPLC to obtain a compound SMP-53816-6, weighing 300.0 mg, with a yield of 90.0%.

MS (ESI) m/z: 695[M/2+H]⁺.

### Step 7: Preparation of compound SMP-53816-7

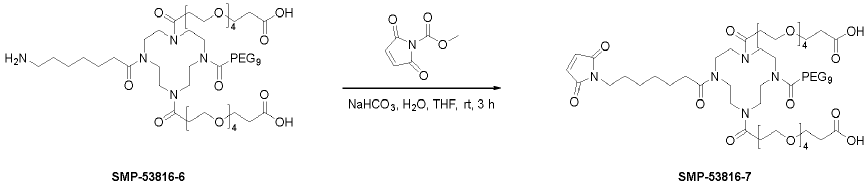

The intermediate SMP-53816-6 (300.0 mg, 0.22 mmol), N-methoxycarbonyl maleimide (68.2 mg, 0.44 mmol), THF (3 mL), and saturated sodium bicarbonate aqueous solution (3 mL) were sequentially added to a 25 mL reaction flask. A reaction occurred at room temperature for 3 hours while stirring, and then TLC showed complete reaction. The reaction solution was purified by HPLC to obtain a compound SMP-53816-7, weighing 260.0 mg, with a yield of 87.9%. MS (ESI) m/z: 685[M/2+H]⁺.

### Step 8: Preparation of compound SMP-53816-8

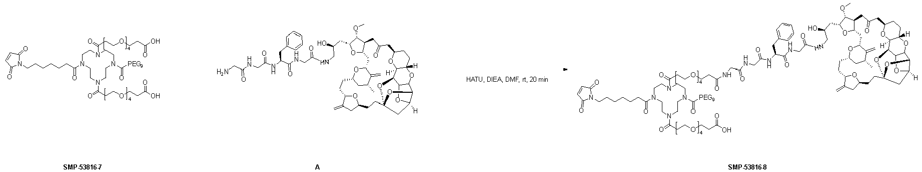

The intermediate SMP-53816-7 (100.0 mg, 0.073 mmol), the intermediate A (74.3 mg, 0.073 mmol), N,N-dimethylformamide (3 mL), and N,N-diisopropylethylamine (28.2 mg, 0.22 mmol) were sequentially added to a 25 mL reaction flask. After complete clarification, an N,N-dimethylformamide (3 mL) solution of N,N,N',N'-tetramethyl-O-(7-azabenzotriazol-1-yl)hexafluorophosphourea (27.8 mg, 0.073 mmol) was added dropwise. A reaction occurred at room temperature for 20 minutes while stirring, and then TLC showed complete reaction. The reaction solution was purified by HPLC to obtain a compound SMP-53816-8, weighing 40.0 mg, with a yield of 22.8%.

MS (ESI) m/z: 1200[M/2+H]⁺.

### Step 9: Preparation of compound SMP-53816

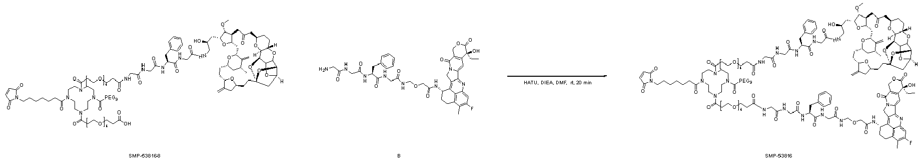

The intermediate SMP-53816-8 (40.0 mg, 0.017 mmol), the intermediate B (14.3 mg, 0.017 mmol), and N,N-dimethylformamide (2 mL) were sequentially added to a 10 mL reaction flask. After complete clarification, N,N,N',N'-tetramethyl-O-(7-azabenzotriazol-1-yl)hexafluorophosphourea (6.5 mg, 0.017 mmol) and N,N-diisopropylethylamine (6.5 mg, 0.051 mmol) were added. A reaction occurred at room temperature for 20 minutes while stirring, and then TLC showed complete reaction. The reaction solution was purified by HPLC to obtain a compound SMP-53816, weighing 14.0 mg, with a yield of 26.0%.

MS (ESI) m/z: 1074[M/3+H]⁺.

### Example 59: Preparation of compound SMP-23135

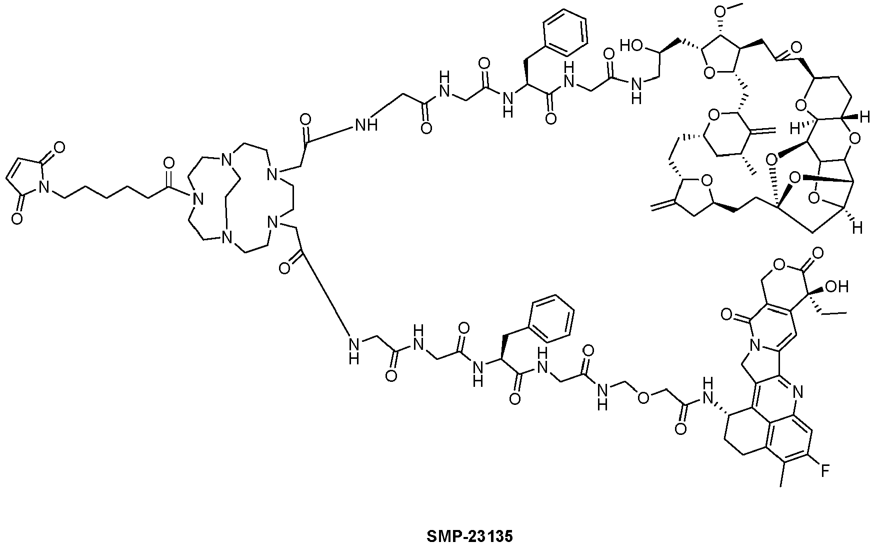

### Step 1: Preparation of compound SMP-23135-2

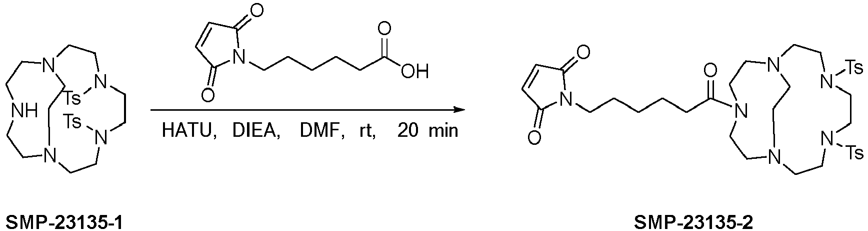

An intermediate SMP-23135-1 (2.0 g, 3.64 mmol), 6-maleimidocaproic acid (768.0 mg, 3.64 mmol), and N,N-dimethylformamide (30 mL) were sequentially added to a 100 mL reaction flask. After complete clarification, N,N,N',N'-tetramethyl-O-(7-azabenzotriazol-1-yl)hexafluorophosphourea (1.4 g, 3.64 mmol) and N,N-diisopropylethylamine (1.4 g, 10.92 mmol) were added. A reaction occurred at room temperature for 20 minutes while stirring, and then TLC showed complete reaction. The reaction solution was purified by HPLC to obtain a compound SMP-23135-2, weighing 1.1 g, with a yield of 40.7%.

MS (ESI) m/z: 743 [M+H]⁺.

### Step 2: Preparation of compound SMP-23135-3

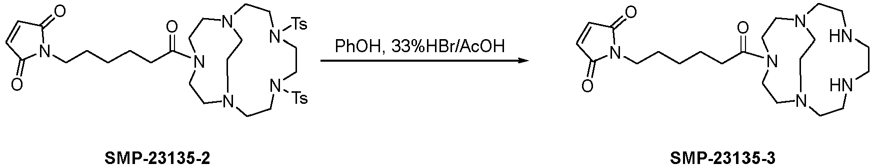

The intermediate SMP-23135-2 (1.1 g, 1.48 mmol), phenol (139.0 mg, 1.48 mmol), and a 33% hydrobromic acid acetic acid solution (30 mL) were sequentially added to a 100 mL reaction flask. A reaction occurred at 50°C overnight while stirring, and then LC-MS showed complete reaction. The reaction solution was cooled and then added dropwise to a single-necked flask containing methyl tert-butyl ether (300 mL) to precipitate a solid. After filtration, the solid was dried in vacuum to obtain a compound SMP-23135-3, weighing 350.0 mg, with a yield of 54.4%.

MS (ESI) m/z: 435[M+H]⁺.

### Step 3: Preparation of compound SMP-23135-4

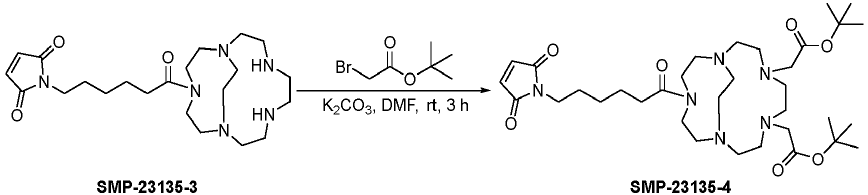

The intermediate SMP-23135-3 (350.0 mg, 0.81 mmol), tert-butyl bromoacetate (473.9 mg, 2.43 mmol), N,N-dimethylformamide (10 mL), and potassium carbonate (335.3 mg, 2.43 mmol) were sequentially added to a 50 mL reaction flask. A reaction occurred at room temperature for 3 hours while stirring, and then LC-MS showed complete reaction. After filtration, the filtrate was purified by HPLC to obtain a compound SMP-23135-4, weighing 85.0 mg, with a yield of 15.9%. MS (ESI) m/z: 663[M+H]⁺.

### Step 4: Preparation of compound SMP-23135-5

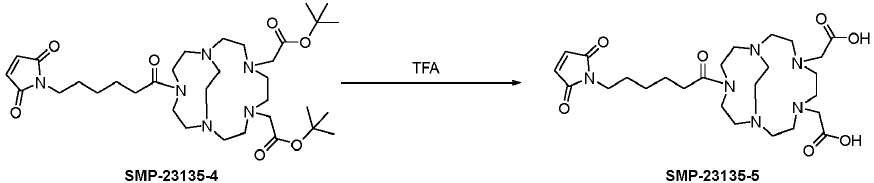

The intermediate SMP-23135-4 (85.0 mg, 0.13 mmol) and trifluoroacetic acid (5 mL) were sequentially added to a 25 mL reaction flask. A reaction occurred at room temperature for 3 hours while stirring, and then TLC showed complete reaction. The trifluoroacetic acid was removed by concentration under reduced pressure, and the reaction solution was purified by HPLC to obtain a compound SMP-23135-5, weighing 50.0 mg, with a yield of 70.8%. MS (ESI) m/z: 551[M+H]⁺.

### Step 5: Preparation of compound SMP-23135-6

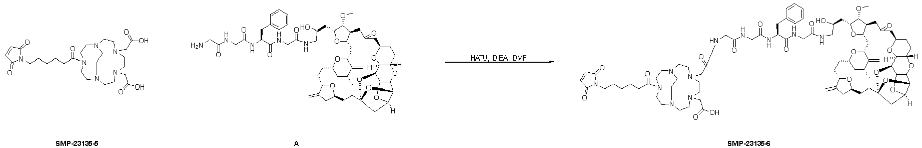

The intermediate SMP-23135-5 (50.0 mg, 0.091 mmol), the intermediate A (95.4 mg, 0.091 mmol), N,N-dimethylformamide (2 mL), and N,N-diisopropylethylamine (35.2 mg, 0.27 mmol) were sequentially to a 10mL reaction flask. After complete clarification, an N,N-dimethylformamide (1 mL) solution of N,N,N',N'-tetramethyl-O-(7-azabenzotriazol-1-yl)hexafluorophosphourea (34.6 mg, 0.091 mmol) was slowly added dropwise. A reaction occurred at room temperature for 20 minutes while stirring, and then TLC showed complete reaction. The reaction solution was purified by HPLC to obtain a compound SMP-23135-6, weighing 23.0 mg, with a yield of 16.0%.

MS (ESI) m/z: 790[M/2+H]⁺.

### Step 6: Preparation of compound SMP-23135

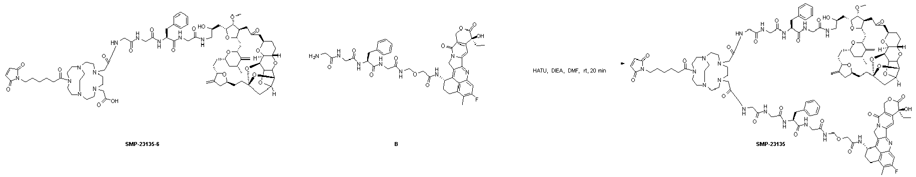

The intermediate SMP-23135-6 (23.0 mg, 0.014 mmol), the intermediate B (12.0 mg, 0.014 mmol), and N,N-dimethylformamide (2 mL) were sequentially added to a 10 mL reaction flask. After complete clarification, N,N,N',N'-tetramethyl-O-(7-azabenzotriazol-1-yl)hexafluorophosphourea (5.3 mg, 0.014 mmol) and N,N-diisopropylethylamine (5.4 mg, 0.042 mmol) were added. A reaction occurred at room temperature for 20 minutes while stirring, and then TLC showed complete reaction. The reaction solution was purified by HPLC to obtain a compound SMP-23135, weighing 12.0 mg, with a yield of 34.3%.

MS (ESI) m/z: 1201[M/2+H]⁺.

### Example 60: Preparation of compound SMP-21139

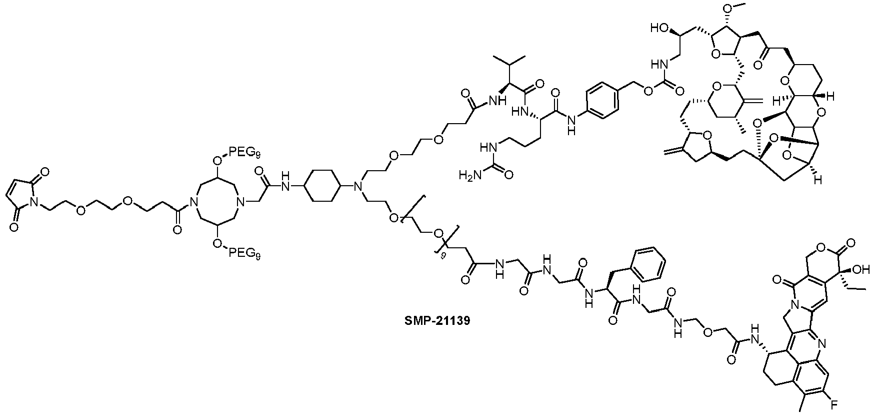

### Step 1: Preparation of compounds SMP-21139-1 and SMP-68691-1

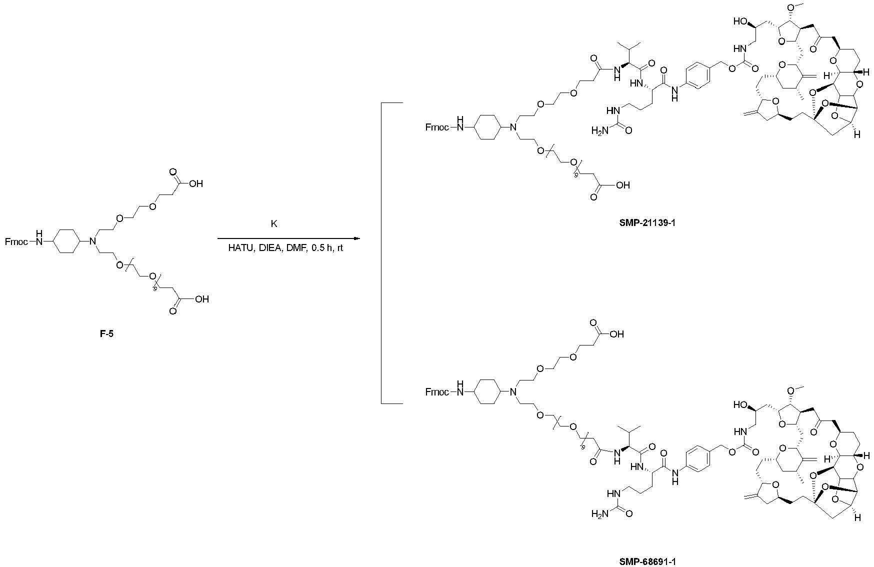

The intermediate K (56.0 mg, 0.05 mmol), the intermediate F-5 (85.0 mg, 0.10 mmol), N,N,N',N'-tetramethyl-O-(7-azabenzotriazol-1-yl)hexafluorophosphourea (19.0 mg, 0.05 mmol), N,N-diisopropylethylamine (13.0 mg, 0.10 mmol), and N,N-dimethylformamide (4 mL) were added to a 25 mL reaction flask. A reaction occurred at room temperature for 30 minutes while stirring, and then TLC showed complete reaction. The N,N-dimethylformamide was removed by concentration under reduced pressure, and the residue was purified by HPLC to obtain a compound SMP-21139-1, weighing 20.0 mg, and a compound SMP-68691-1, weighing 18.0 mg, with a total yield of 36%.

MS (ESI) m/z: 1059 [M/2+H]⁺.

### Step 2: Preparation of compound SMP-21139-2

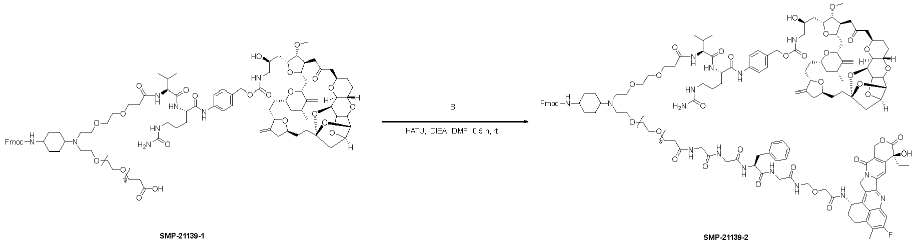

The intermediate SMP-21139-1 (20.0 mg, 0.010 mmol), the intermediate B (10.0 mg, 0.010 mmol), N,N-dimethylformamide (2 mL), N,N,N',N'-tetramethyl-O-(7-azabenzotriazol-1-yl)hexafluorophosphourea (4.0 mg, 0.010 mmol), and N,N-diisopropylethylamine (2.0 mg, 0.014 mmol) were added to a 10 mL reaction flask. A reaction occurred at room temperature for 30 minutes while stirring, and then TLC showed complete reaction. The N,N-dimethylformamide was removed by concentration under reduced pressure, and the residue was purified by HPLC to obtain a compound SMP-21139-2, weighing 15.0 mg, with a yield of 61%.

MS (ESI) m/z: 1363 [M/2+H]⁺.

### Step 3: Preparation of compound SMP-21139-3

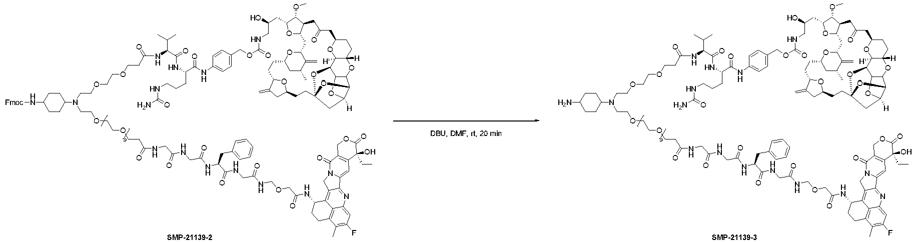

The intermediate SMP-21139-2 (15.0 mg, 0.0055 mmol), 1,8-diazabicyclo[5.4.0]undec-7-ene (2.0 mg, 0.013 mmol), and N,N-dimethylformamide (2 mL) were added to a 10 mL reaction flask. A reaction occurred at room temperature for 20 minutes while stirring, and then TLC showed complete reaction. The N,N-dimethylformamide was removed by concentration under reduced pressure, and the residue was purified by HPLC to obtain a compound SMP-21139-3, weighing 8.0 mg, with a yield of 58%.

MS (ESI) m/z: 1252 [M/2+H]⁺.

### Step 4: Preparation of compound SMP-21139

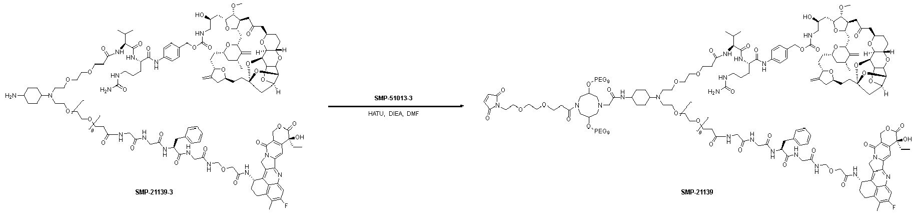

The compound SMP-51013-3 (6.0 mg, 0.0047 mmol) and a solvent N,N-dimethylformamide (2 mL) were added to a 10 mL reaction flask. After the reaction solution was clarified, 2-(7-azabenzotriazole)-N,N,N',N'-tetramethylurea hexafluorophosphate (2.0 mg, 0.0052 mmol) and N,N-diisopropylethylamine (1.0 mg, 0.0077 mmol) were sequentially added. After reaction at room temperature for 5 minutes, the compound SMP-21139-3 (8.0 mg, 0.0032 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-21139, weighing 4.5 mg, with a yield of 34%.

MS (ESI) m/z: 1324 [M/3+H]⁺.

### Example 61: Preparation of compound SMP-68691

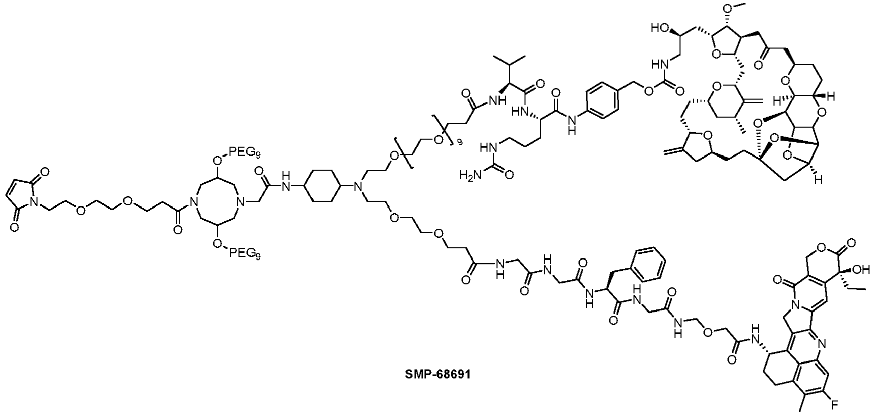

### Step 1: Preparation of compound SMP-68691-2

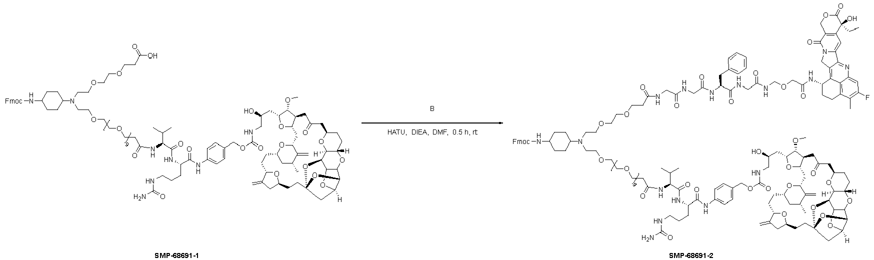

The intermediate SMP-68691-1 (18.0 mg, 0.008 mmol), the intermediate B (8.0 mg, 0.010 mmol), N,N,N',N'-tetramethyl-O-(7-azabenzotriazol-1-yl)hexafluorophosphourea (4.0 mg, 0.010 mmol), and N,N-diisopropylethylamine (2.0 mg, 0.013 mmol), N,N-dimethylformamide (2 mL) were added to a 10 mL reaction flask. A reaction occurred at room temperature for 30 minutes while stirring, and then TLC showed complete reaction. The N,N-dimethylformamide was removed by concentration under reduced pressure, and the residue was purified by HPLC to obtain a compound SMP-68691-2, weighing 12.0 mg, with a yield of 55%.

MS (ESI) m/z: 1363 [M/2+H]⁺.

### Step 2: Preparation of compound SMP-68691-3

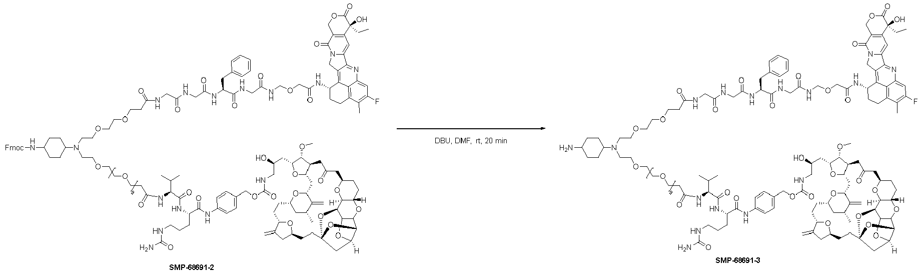

The intermediate SMP-68691-2 (12.0 mg, 0.0044 mmol), 1,8-diazabicyclo[5.4.0]undec-7-ene (1.0 mg, 0.007 mmol), and N,N-dimethylformamide (2 mL) were added to a 10 mL reaction flask. A reaction occurred at room temperature for 20 minutes while stirring, and then TLC showed complete reaction. The N,N-dimethylformamide was removed by concentration under reduced pressure, and the residue was purified by HPLC to obtain a compound SMP-68691-3, weighing 8.0 mg, with a yield of 73%.

MS (ESI) m/z: 1252 [M/2+H]⁺.

### Step 3: Preparation of compound SMP-68691

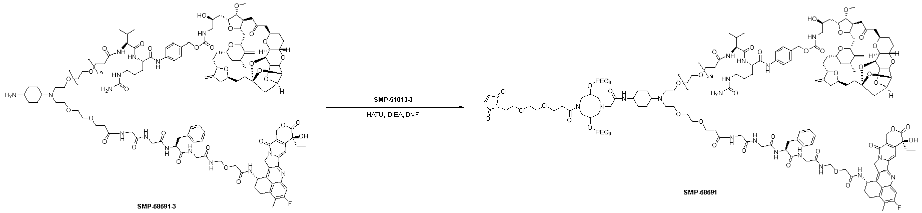

The compound SMP-51013-3 (6.0 mg, 0.0047 mmol) and a solvent N,N-dimethylformamide (1.5 mL) were added to a 10 mL reaction flask, and 2-(7-azabenzotriazole)-N,N,N',N'-tetramethylurea hexafluorophosphate (2.0 mg, 0.0052 mmol) and N,N-diisopropylethylamine (1.0 mg, 0.0077 mmol) were sequentially added. After reaction at room temperature for 5 minutes, the compound SMP-68691-3 (8.0 mg, 0.0029 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-68691, weighing 4.0 mg, with a yield of 33%.

MS (ESI) m/z: 1324 [M/3+H]⁺.

### Example 62: Preparation of compound SMP-60226

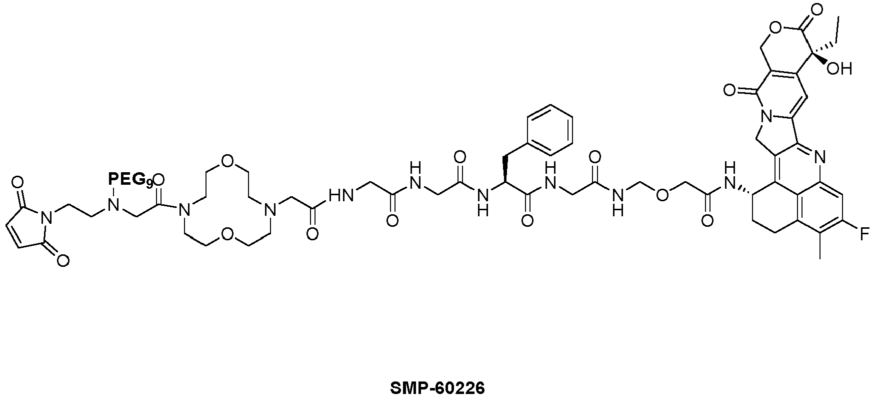

### Step 1: Preparation of compound SMP-60226-1

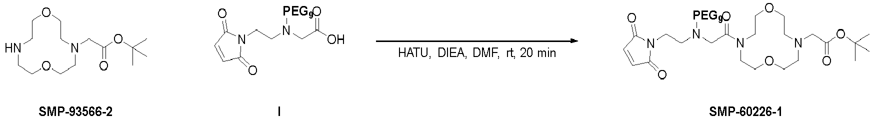

The intermediate SMP-93566-2 (100.0 mg, 0.35 mmol), the intermediate I (212.0 mg, 0.35 mmol), and N,N-dimethylformamide (3 mL) were added to a 10 mL reaction flask. After complete clarification, N,N,N',N'-tetramethyl-O-(7-azabenzotriazol-1-yl)hexafluorophosphourea (133.0 mg, 0.35 mmol) and N,N-diisopropylethylamine (135.4 mg, 1.05 mmol) were added, a reaction occurred at room temperature for 20 minutes while stirring, and then TLC showed complete reaction. The reaction solution was purified by HPLC to obtain a compound SMP-60226-1, weighing 200.0 mg, with a yield of 65%.

MS (ESI) m/z: 878[M+H]⁺.

### Step 2: Preparation of compound SMP-60226-2

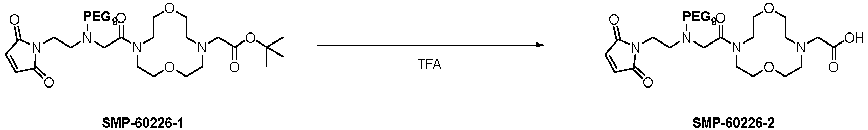

The intermediate SMP-60226-1 (201.9 mg, 0.23 mmol) and trifluoroacetic acid (3 mL) were added to a 10 mL reaction flask. A reaction occurred at room temperature for 2 hours while stirring, and then TLC showed complete reaction. The trifluoroacetic acid was removed by concentration under reduced pressure, and the residue was dissolved in acetonitrile and then purified by HPLC to obtain a compound SMP-60226-2, weighing 80.0 mg, with a yield of 43%.

MS (ESI) m/z: 823 [M+H]⁺.

### Step 3: Preparation of compound SMP-60226

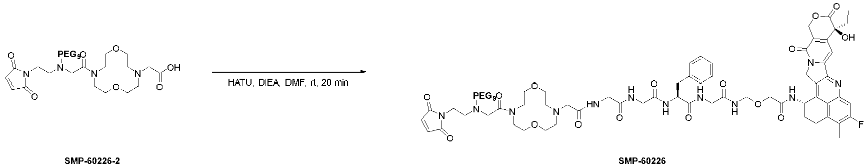

The intermediate SMP-60226-4 (32.9 mg, 0.04 mmol), the intermediate B (33.6 mg, 0.04 mmol), and N,N-dimethylformamide (2 mL) were added to a 10 mL reaction flask. After complete clarification, N,N,N',N'-tetramethyl-O-(7-azabenzotriazol-1-yl)hexafluorophosphourea (15.2 mg, 0.04 mmol) and N,N-diisopropylethylamine (10.3 mg, 0.08 mmol) were added, a reaction occurred at room temperature for 20 minutes while stirring, and then LC-MS test showed complete reaction. The reaction solution was purified by HPLC to obtain a compound SMP-60226, weighing 35.0 mg, with a yield of 53%.

MS (ESI) m/z: 823[M/2+H]⁺.

### Example 63: Preparation of compound SMP-37860

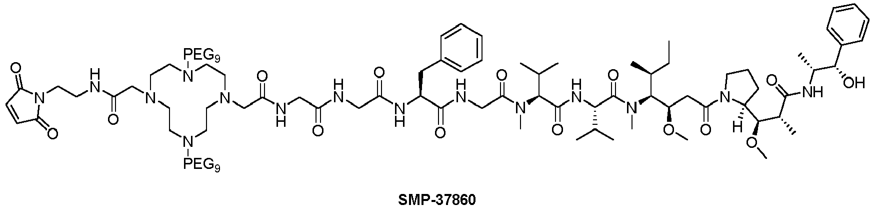

### Step 1: Preparation of compound SMP-37860-2

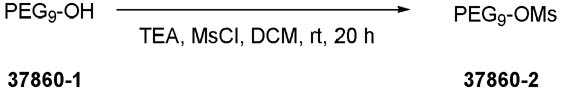

Nonaethylene glycol monomethyl ether (7.0 g, 16.35 mmol) was dissolved in dichloromethane (80 mL) in a 250 mL reaction flask, triethylamine (3.3 g, 32.67 mmol) was added, methylsulfonyl chloride (2.2 g, 19.60 mmol) was slowly added to the reaction solution in an ice bath, a reaction occurred at room temperature for 20 hours while stirring, and then TLC showed complete reaction. The organic phase was washed with water (50 mL) and saturated ammonium chloride aqueous solution (30 mL) and then dried with anhydrous sodium sulfate. After concentration under reduced pressure and column chromatography (dichloromethane: methanol=20: 1) to obtain a compound SMP-37860-2, weighing 8.1 g, with a yield of 98%.

MS (ESI) m/z: 507[M+H]⁺.

¹HNMR(400 MHz, CDCl₃)δ3.52-3.70(m, 32H), 3.40(s, 3H), 3.16(s, 3H).

### Step 2: Preparation of compound SMP-37860-3

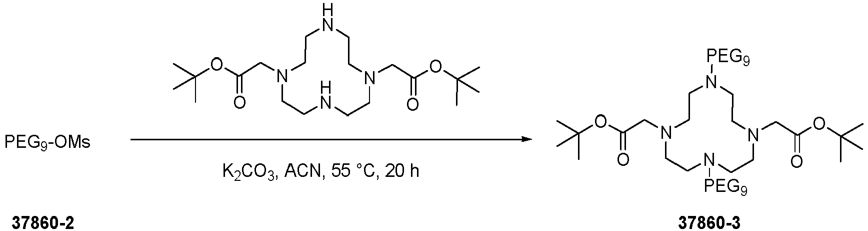

In a 100 mL reaction flask, 1,7-bis-(N-tert-butoxycarbonylmethyl)-1,4,7,10-tetraazacyclododecane (2.1 g, 5.33 mmol) and the compound SMP-37860-2 (8.1 g, 16.01 mmol) were dissolved in acetonitrile (30 mL), and potassium carbonate (2.4 g, 17.07 mmol) was added, followed by heating to 55°C and reaction for 20 hours. LC-MS test showered complete reaction. After cooling to room temperature and filtering, the filtrate was concentrated under reduced pressure to dryness, and the residue was purified by column chromatography (dichloromethane: methanol=5: 1) to obtain a compound SMP-37860-3, weighing 3.2 g, with a yield of 64%. MS (ESI) m/z: 612 [M/2+H]⁺.

¹HNMR(400 MHz, CDCl₃)δ3.52-3.55(m, 60H), 3.40(s, 6H), 3.32(s, 4H), 2.51(t, 4H), 2.46(s, 16H), 1.40(s, 18H).

### Step 3: Preparation of compound SMP-37860-4

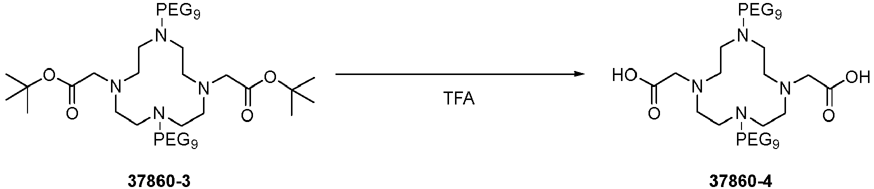

The compound SMP-37860-3 (90.0 mg, 0.079 mmol) was added to a 10 mL reaction flask, then 2 mL trifluoroacetic acid was added, and a reaction occurred at room temperature for 3 hours while stirring. LC-MS test showered complete reaction. After concentration under reduced pressure, the residue was dissolved in a small amount of acetonitrile and purified by HPLC to obtain a compound SMP-37860-4, weighing 20 mg, with a yield of 25%.

MS (ESI) m/z: 555 [M/2+H]⁺.

### Step 4: Preparation of compound SMP-37860-5

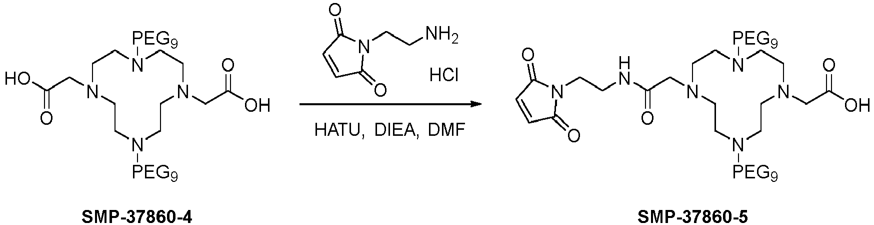

In a 50 mL reaction flask, 1-(2-aminoethyl)-1H-pyrrole-2,5-dione hydrochloride (95.0 mg, 0.54 mmol) and the compound SMP-37860-4 (500.0 mg, 0.45 mmol) were dissolved in N,N-dimethylformamide (10 mL), and N,N-diisopropylethylamine (203.8 mg, 1.58 mmol) was added. N,N,N',N'-tetramethyl-O-(7-azabenzotriazol-1-yl)hexafluorophosphourea (171.0 mg, 0.45 mmol) was dissolved in N,N-dimethylformamide (10 mL) and then dropped into the above solution. A reaction occurred at room temperature for 3 hours while stirring. LC-MS test showered complete reaction, and then the reaction solution was purified by HPLC to obtain a compound SMP-37860-5, weighing 275.0 mg, with a yield of 50%.

MS (ESI) m/z: 616 [M/2+H]⁺.

### Step 5: Preparation of compound SMP-37860-6

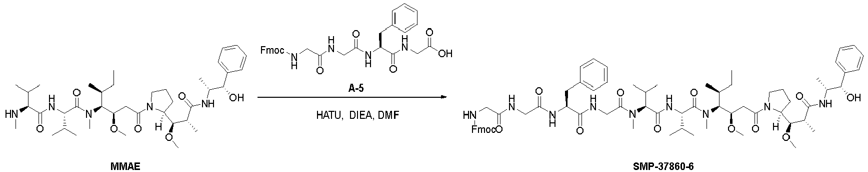

The intermediate A-5 (200.0 mg, 0.36 mmol), N,N,N',N'-tetramethyl-O-(7-azabenzotriazol-1-yl)hexafluorophosphourea (163.4 mg, 0.43 mmol), N,N-diisopropylethylamine (69.7 mg, 0.54 mmol), and N,N-dimethylformamide (5 mL) were added to a 25 mL reaction flask, a reaction occurred at room temperature for 5 minutes while stirring, then monomethyl auristatin E (258.1 mg, 0.36 mmol) was added, and the reaction continued at room temperature for 30 minutes. TLC showed complete reaction. The N,N-dimethylformamide was removed by concentration under reduced pressure, and the residue was purified by column chromatography (dichloromethane: methanol=10:1) to obtain a compound SMP-37860-6, weighing 300.0 mg, with a yield of 65%. MS (ESI) m/z: 630[M/2+H]⁺.

### Step 6: Preparation of compound SMP-37860-7

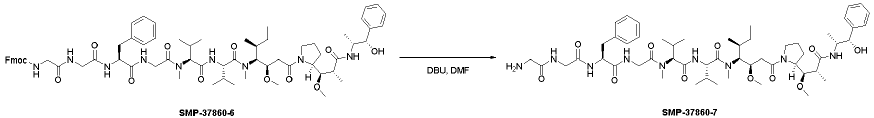

The compound SMP-37860-6 (302.4 mg, 0.24 mmol), 1,8-diazabicyclo[5.4.0]undec-7-ene (41.3 mg, 0.27 mmol), and N,N-dimethylformamide (10 mL) were added to a 50 mL reaction flask, a reaction occurred at room temperature for 30 minutes while stirring, and then TLC showed complete reaction. After concentration under reduced pressure, the residue was purified by HPLC to obtain a compound SMP-37860-7, weighing 200.0 mg, with a yield of 81%.

MS (ESI) m/z: 1037[M+H]⁺.

### Step 7: Preparation of compound SMP-37860

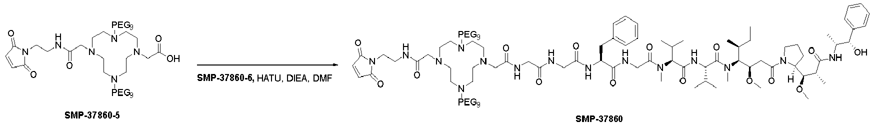

The intermediate SMP-37860-5 (20.7 mg, 0.02 mmol), the intermediate SMP-37860-7 (20.4 mg, 0.02 mmol), N,N-dimethylformamide (3 mL), N,N,N',N'-tetramethyl-O-(7-azabenzotriazol-1-yl)hexafluorophosphourea (15.2 mg, 0.04 mmol), and N,N-diisopropylethylamine (6.5 mg, 0.05 mmol) were added to a 25 mL reaction flask. A reaction occurred at room temperature for 30 minutes while stirring, and then TLC showed complete reaction. The N,N-dimethylformamide was removed by concentration under reduced pressure, and the residue was purified by HPLC to obtain a compound SMP-37860, weighing 20.0 mg, with a yield of 47%.

MS (ESI) m/z: 1126[M/2+H]⁺.

### Example 64: Preparation of compound SMP-18777

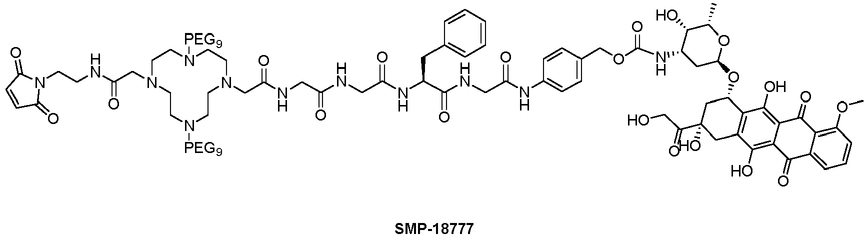

### Step 1: Preparation of compound SMP-18777-1

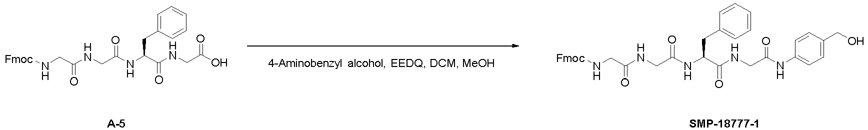

In a 100 mL reaction flask, the intermediate A-5 (2.5 g, 4.38 mmol) and 4-aminobenzyl alcohol (1.1 g, 8.77 mmol) were dissolved in a mixed solution of dichloromethane (30 mL) and methanol (10 mL), 2-ethoxy-1-ethoxycarbonyl-1,2-dihydroquinoline (2.2 g, 8.77 mmol) was added, a reaction occurred at room temperature for 20 hours while stirring, and then TLC showed complete reaction. The solvent was removed by concentration under reduced pressure , and the residue was purified by silica gel column chromatography (dichloromethane: methanol=15:1) to obtain a compound SMP-18777-1, weighing 900.0 mg, with a yield of 31%.

MS (ESI) m/z: 664[M+H]⁺.

### Step 2: Preparation of compound SMP-18777-2

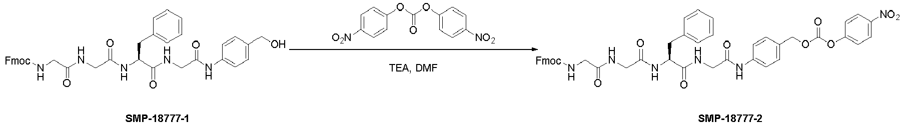

In a 25 mL reaction flask, the intermediate SMP-18777-1 (1.0 g, 1.51 mmol) was dissolved in N,N-dimethylformamide (10 mL), bis(p-nitrophenyl)carbonate (163.0 mg, 0.43 mmol) and triethylamine (1979 mg, 1.96 mmol) were added, a reaction occurred at room temperature for 5 hours while stirring, and then TLC showed complete reaction. The N,N-dimethylformamide was removed by concentration under reduced pressure , and the residue was purified by silica gel column chromatography (dichloromethane: methanol=12:1) to obtain a compound SMP-18777-2, weighing 300.0 mg, with a yield of 24%.

MS (ESI) m/z: 829[M+H]⁺.

### Step 3: Preparation of compound SMP-18777-3

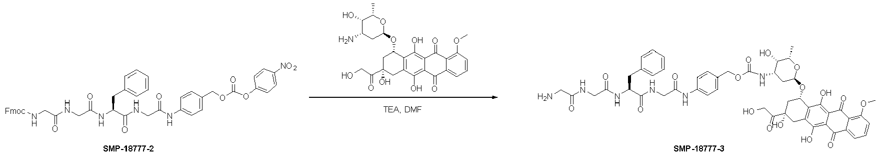

In a 25 mL reaction flask, doxorubicin hydrochloride (52.2 mg, 0.09 mmol) was dissolved in N,N-dimethylformamide (10 mL), the compound SMP-18777-2 (82.9 mg, 0.10 mmol) and triethylamine (17.2 mg, 0.17 mmol) were added, a reaction occurred at room temperature for 18 hours while stirring, and then TLC showed complete reaction. The N,N-dimethylformamide was removed by concentration under reduced pressure, and the residue was purified by HPLC to obtain a compound SMP-18777-3, weighing 80.0 mg, with a yield of 77%.

MS (ESI) m/z: 614, 425 fragment peaks.

### Step 4: Preparation of compound SMP-18777

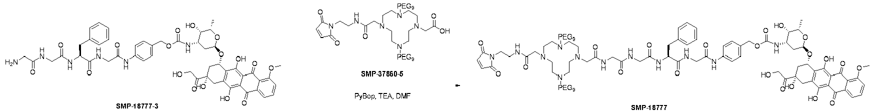

In a 25 mL reaction flask, the compound SMP-18777-3 (20.0 mg, 0.02 mmol) and the compound SMP-37860-5 (9.0 mg, 0.024 mmol) were dissolved in N,N-dimethylformamide (2 mL), 1H-benzotriazol-1-yloxytripyrrolidyl hexafluorophosphate (10.4 mg, 0.02 mmol) and triethylamine (4.0 mg, 0.04 mmol) were added, a reaction occurred at room temperature for 5 hours while stirring, and then LC-MS showed complete reaction. The reaction solution was purified by HPLC to obtain a compound SMP-18777, weighing 1.7 mg, with a yield of 6%.

MS (ESI) m/z: 819, 914 fragment peaks.

### Example 66: Preparation of compound SMP-58054

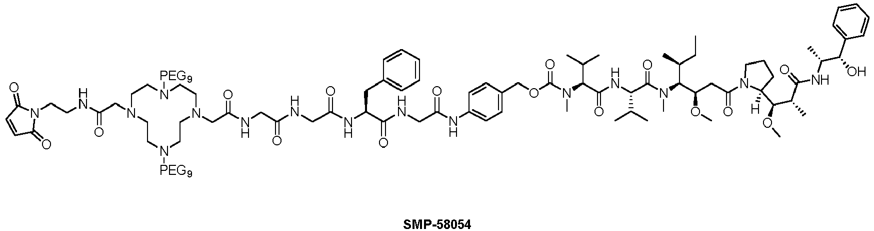

### Step 1: Preparation of compound SMP-58054-1

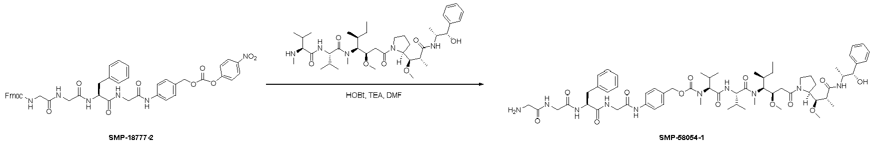

In a 25 mL reaction flask, monomethyl auristatin E (200.7 mg, 0.28 mmol) was dissolved in N,N-dimethylformamide (5 mL), the compound SMP-18777-2 (200.0 mg, 0.24 mmol), triethylamine (48.5 mg, 0.48 mmol) and 1-hydroxybenzotriazole (37.8 mg, 0.28 mmol) were added, a reaction occurred at room temperature for 18 hours while stirring, and then TLC showed complete reaction. The reaction solution was purified by HPLC to obtain a compound SMP-58054-1, weighing 80.0 mg, with a yield of 24%.

MS (ESI) m/z: 425 fragment peak, 1186[M+H]⁺.

### Step 2: Preparation of compound SMP-58054

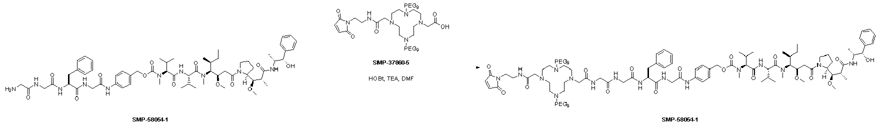

The intermediate SMP-58054-1 (15.0 mg, 0.013 mmol), the intermediate SMP-37860-6 (16.0 mg, 0.013 mmol), N,N-dimethylformamide (1 mL), 1-hydroxybenzotriazole (1.3 mg, 0.014 mmol), and triethylamine (2.0 mg, 0.019 mmol) were added to a 25 mL reaction flask. A reaction occurred at room temperature for 30 minutes while stirring, and then TLC showed complete reaction. The N,N-dimethylformamide was removed by concentration under reduced pressure, and the residue was purified by HPLC to obtain a compound SMP-58054, weighing 20.0 mg, with a yield of 67%.

MS (ESI) m/z: 1200 [M/2+H]⁺.

### Example 67: Preparation of compound SMP-25274

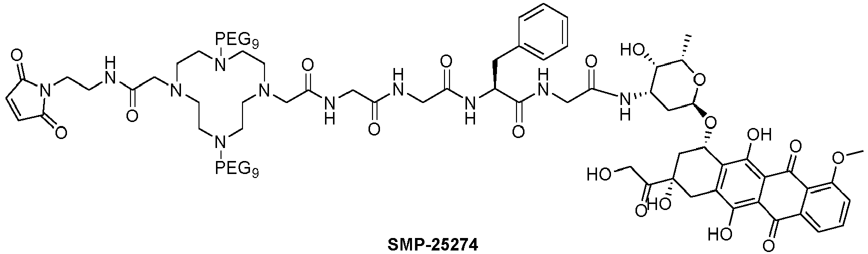

### Step 1: Preparation of compound SMP-25274-1

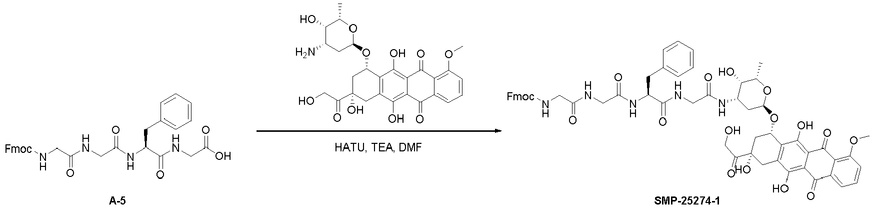

In a 25 mL reaction flask, the compound A-5 (50.0 mg, 0.089 mmol) and doxorubicin hydrochloride (46.4 mg, 0.075 mmol) were dissolved in N,N-dimethylformamide (4 mL), N,N,N',N'-tetramethyl-O-(7-azabenzotriazol-1-yl)hexafluorophosphourea (30.4 mg, 0.08 mmol) and triethylamine (8.0 mg, 0.08 mmol) were added, a reaction occurred at room temperature for 5 hours while stirring, and then TLC showed complete reaction. The reaction solution was purified by HPLC to obtain a compound SMP-25274-1, weighing 70.0 mg, with a yield of 74%.

MS (ESI) m/z:1084[M+H]⁺.

### Step 2: Preparation of compound SMP-25274-2

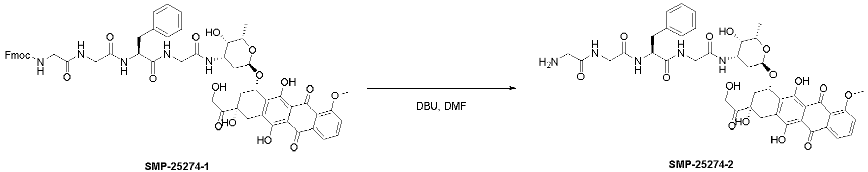

The compound SMP-25274-1 (80.0 mg, 0.07 mmol), 1,8-diazabicyclo[5.4.0]undec-7-ene (10.7 mg, 0.07 mmol), and N,N-dimethylformamide (3 mL) were added to a 10 mL reaction flask, a reaction occurred at room temperature for 30 minutes while stirring, and then TLC showed complete reaction. After concentration under reduced pressure, the residue was purified by HPLC to obtain a compound SMP-25274-2, weighing 50.0 mg, with a yield of 79%.

MS (ESI) m/z:862[M+H]⁺.

### Step 3: Preparation of compound SMP-25274

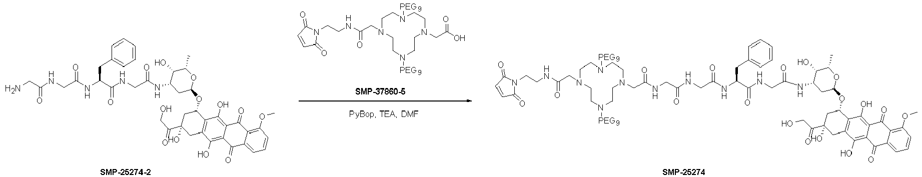

In a 25 mL reaction flask, the compound SMP-25274-2 (20.0 mg, 0.02 mmol) and the compound SMP-37860-5 (30.0 mg, 0.024 mmol) were dissolved in N,N-dimethylformamide (1.5 mL), and 1H-benzotriazol-1-yloxytripyrrolidyl hexafluorophosphate (10.4 mg, 0.021 mmol) and triethylamine (4.0 mg, 0.04 mmol) were added. A reaction occurred at room temperature for 5 hours while stirring, and then TLC-MS showed complete reaction. The reaction solution was purified by HPLC to obtain a compound SMP-25274, weighing 14.0 mg, with a yield of 34%.

MS (ESI) m/z:1038 [M/2+H]⁺.

### Example 68: Preparation of compound SMP-56875

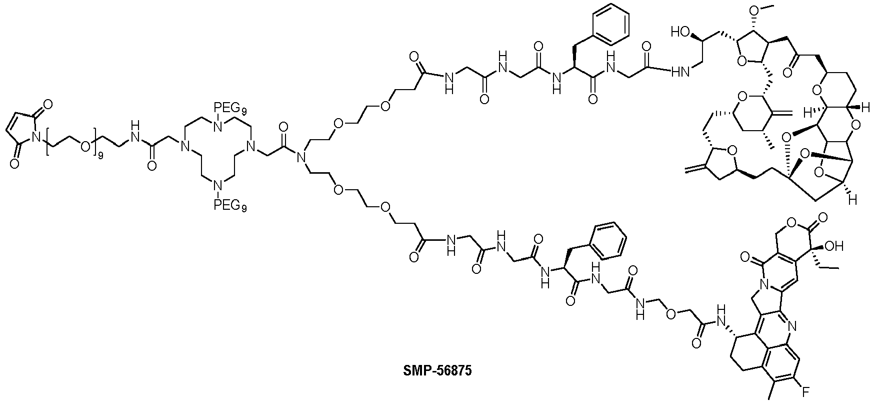

### Step 1: Preparation of compound SMP-56875-1

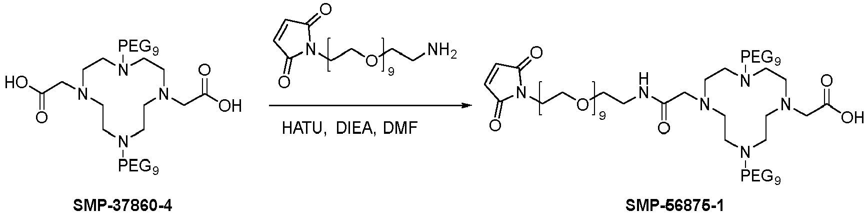

In a 25 mL reaction flask, 1-(29-amino-3,6,9,12,15,18,21,24,27-oxanonacosyl)-1H-pyrrole-2,5-dione (90.0 mg, 0.17 mmol) and the compound SMP-37860-4 (214.0 mg, 0.19 mmol) were dissolved in N,N-dimethylformamide (2 mL), and N,N-diisopropylethylamine (54.2 mg, 0.42 mmol) was added. N,N,N',N'-tetramethyl-O-(7-azabenzotriazol-1-yl)hexafluorophosphourea (64.6 mg, 0.17 mmol) was dissolved in N,N-dimethylformamide (4 mL) and then dropped into the above solution. A reaction occurred at room temperature for 3 hours while stirring, and then LC-MS showed complete reaction. The reaction solution was purified by HPLC to obtain a compound SMP-56875-1, weighing 70.0 mg, with a yield of 26%.

MS (ESI) m/z: 638 [M/2+H]⁺.

### Step 2: Preparation of compound SMP-56875

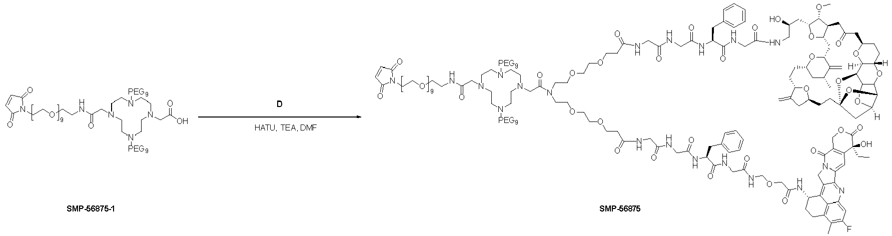

In a 4 mL reaction flask, the compound SMP-56875-1 (11.0 mg, 0.005 mmol) and the intermediate D (16.0 mg, 0.01 mmol) were dissolved in N,N-dimethylformamide (0.5 mL), N,N,N',N'-tetramethyl-O-(7-azabenzotriazol-1-yl)hexafluorophosphourea (3.8 mg, 0.01 mmol) and triethylamine (0.8 mg, 0.01 mmol) were added, a reaction occurred at room temperature for 2 hours while stirring, and then TLC showed complete reaction. The reaction solution was purified by HPLC to obtain a compound SMP-56875, weighing 7.0 mg, with a yield of 35%.

MS (ESI) m/z: 1267 [M/3+H]⁺.

### Example 69: Preparation of compound SMP-82444

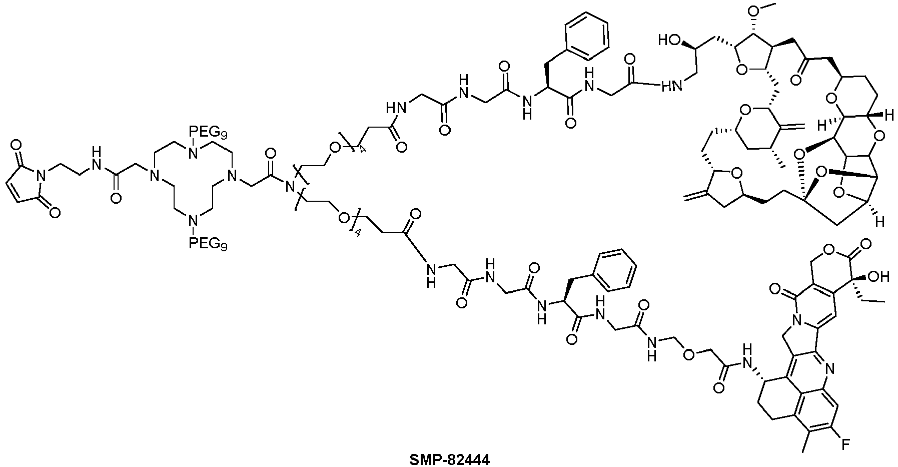

### Step 1: Preparation of compound SMP-82444-1

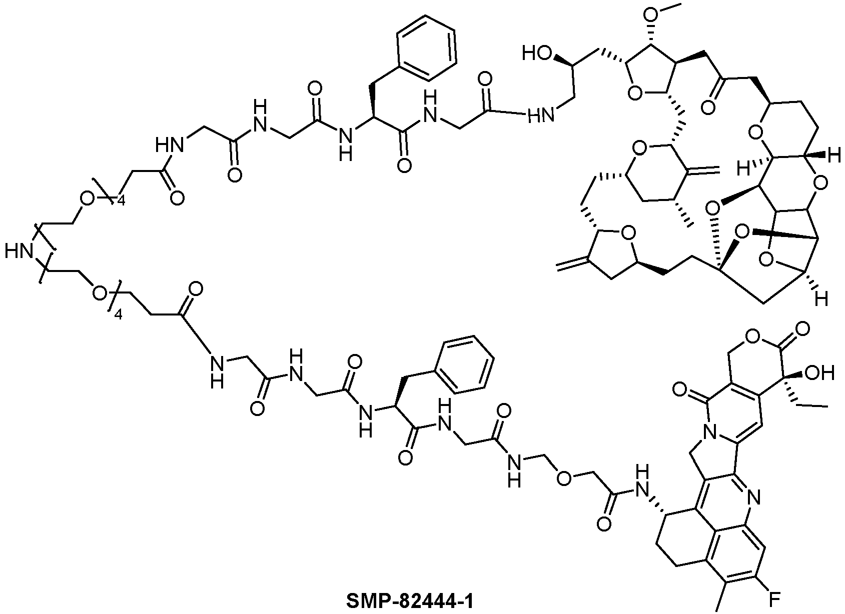

The synthesis method of compound SMP-82444-1 referred to the synthesis of intermediate C.

MS (ESI) m/z: 1184 [M/2+H]⁺.

### Step 2: Preparation of compound SMP-82444

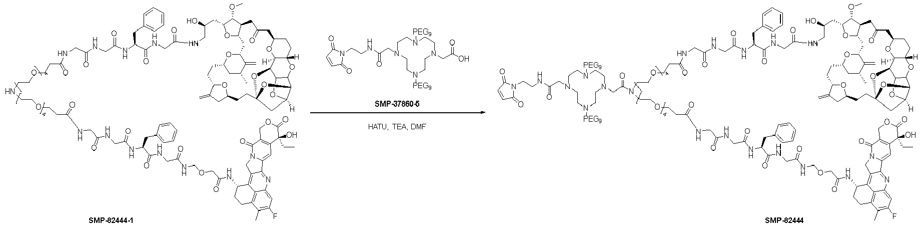

In a 4 mL reaction flask, the compound SMP-82444-1 (9.0 mg, 0.004 mmol) and the compound SMP-37860-5 (8.0 mg, 0.007 mmol) were dissolved in N,N-dimethylformamide (0.5 mL), N,N,N',N'-tetramethyl-O-(7-azabenzotriazol-1-yl)hexafluorophosphourea (3.8 mg, 0.01 mmol) and triethylamine (1.0 mg, 0.011 mmol) were added, a reaction occurred at room temperature for 2 hours while stirring, and then TLC showed complete reaction. The reaction solution was purified by HPLC to obtain a compound SMP-82444, weighing 3.2 mg, with a yield of 24%. MS (ESI) m/z: 1194 [M/3+H]⁺.

### Example 70: Preparation of compound SMP-50309

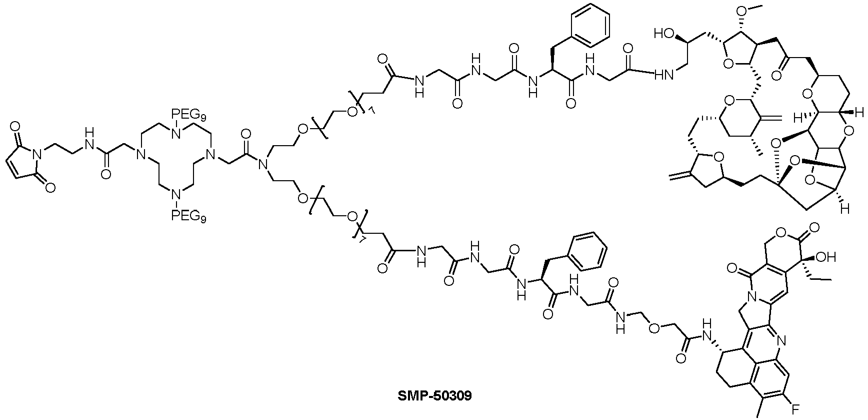

### Step 1: Preparation of compound SMP-50309-1

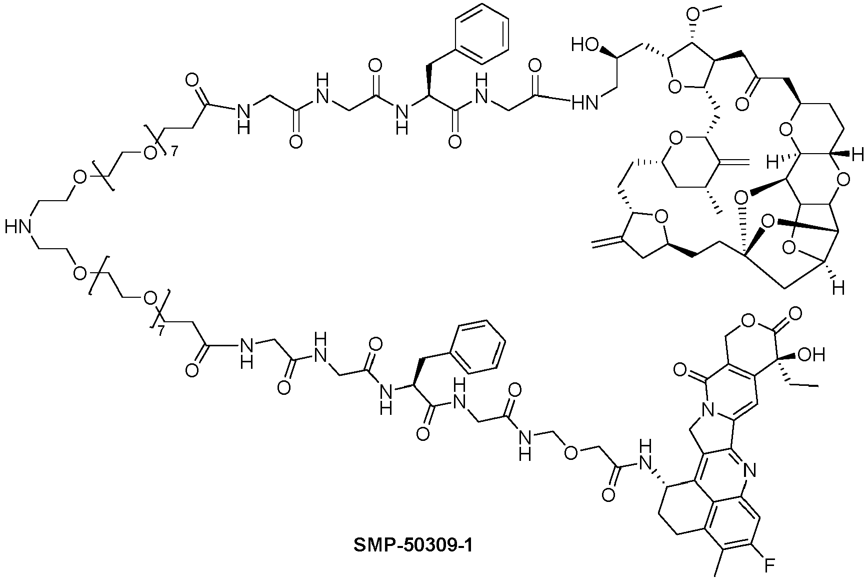

The synthesis method of compound SMP-50309-1 referred to the synthesis of intermediate C.

MS (ESI) m/z: 907 [M/3+H]⁺.

### Step 2: Preparation of compound SMP-50309

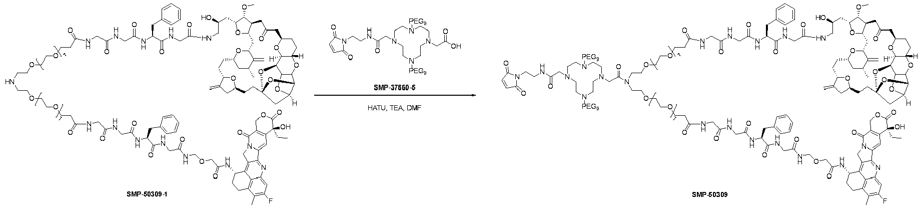

In a 4 mL reaction flask, the compound SMP-82444-1 (9.0 mg, 0.003 mmol) and the compound SMP-37860-5 (8.0 mg, 0.007 mmol) were dissolved in N,N-dimethylformamide (0.5 mL), N,N,N',N'-tetramethyl-O-(7-azabenzotriazol-1-yl)hexafluorophosphourea (3.8 mg, 0.01 mmol) and triethylamine (1.0 mg, 0.011 mmol) were added, and a reaction occurred at room temperature for 2 hours while stirring. The reaction solution was purified by HPLC to obtain a compound SMP-37860, weighing 3.0 mg, with a yield of 29%.

MS (ESI) m/z: 1281 [M/3+H]⁺.

### Example 71: Preparation of compound SMP-20094

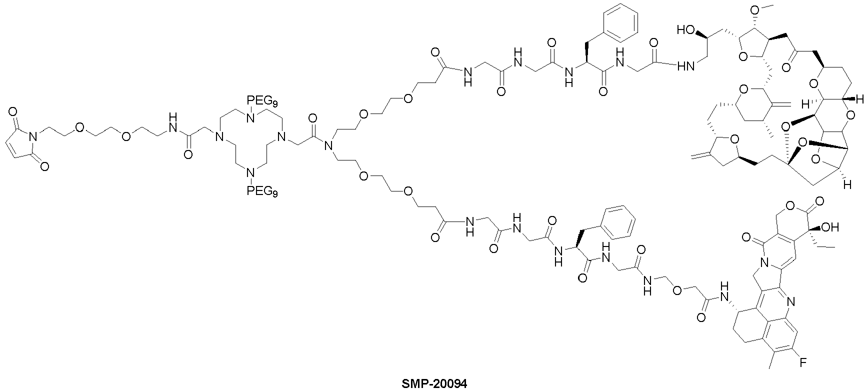

### Step 1: Preparation of compound SMP-20094-1

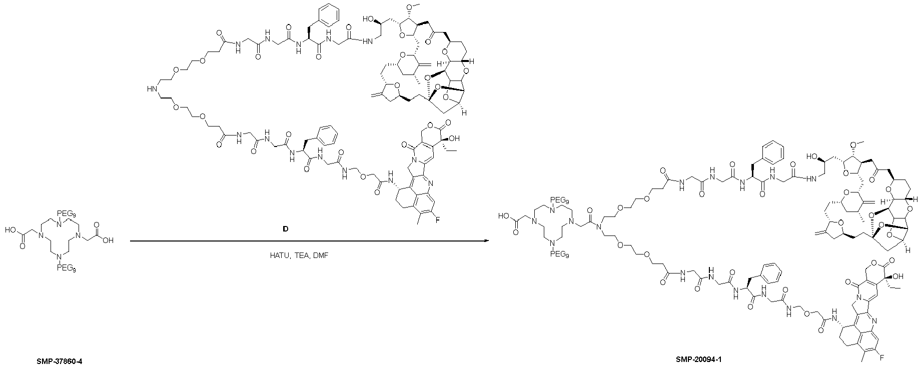

In a 4 mL reaction flask, the intermediate D (10.0 mg, 0.005 mmol) and the compound SMP-37860-4 (14.0 mg, 0.013 mmol) were dissolved in N,N-dimethylformamide (1.5 mL), and triethylamine (1.0 mg, 0.01 mmol) was added. N,N,N',N'-tetramethyl-O-(7-azabenzotriazol-1-yl)hexafluorophosphourea (3.8 mg, 0.01 mmol) was dissolved in N,N-dimethylformamide (1.5 mL) and then dropped into the above solution. A reaction occurred at room temperature for 1.5 hours while stirring. LC-MS showed complete reaction, and then the reaction solution was purified by HPLC to obtain a compound SMP-20094-1, weighing 15.0 mg, with a yield of 91%.

MS (ESI) m/z: 1094 [M/3+H]⁺.

### Step 2: Preparation of compound SMP-20094

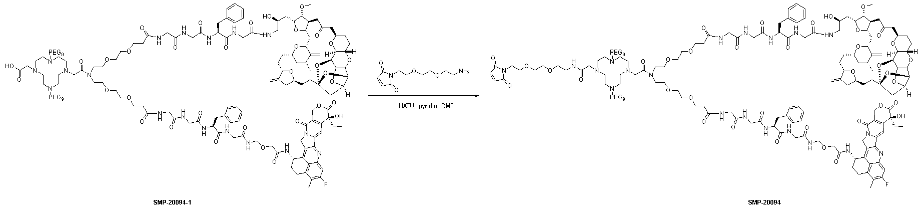

In a 4 mL reaction flask, 1-(2-(2-(2-aminoethoxy)ethoxy)ethyl)-1H-pyrrole-2,5-dione (2.0 mg, 0.009 mmol) and the compound SMP-20094-1 (15.0 mg, 0.005 mmol) were dissolved in N,N-dimethylformamide (3 mL), N,N,N',N'-tetramethyl-O-(7-azabenzotriazol-1-yl)hexafluorophosphourea (3.8 mg, 0.01 mmol) and pyridine (0.9 mg, 0.01 mmol) were added, and a reaction occurred at room temperature for 2 hours while stirring. TLC showed complete reaction, and then the reaction solution was purified by HPLC to obtain a compound SMP-20094, weighing 1.5 mg, with a yield of 10%.

MS (ESI) m/z:1164 [M/3+H]⁺.

### Example 72: Preparation of compound SMP-99559

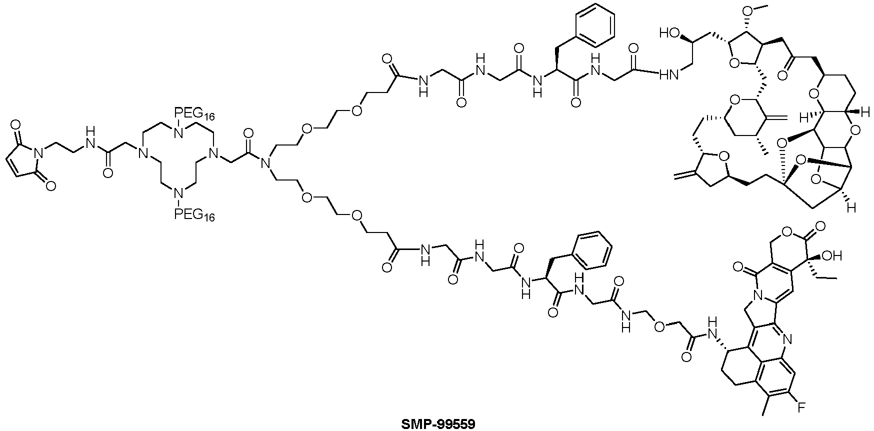

### Step 1: Preparation of compound SMP-99559-1

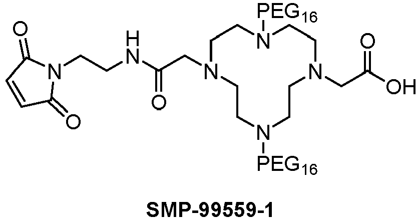

The synthesis method of compound SMP-99559-1 referred to the synthesis of SMP-37860-5.

MS (ESI) m/z: 924 [M/2+H]⁺.

### Step 2: Preparation of compound SMP-99559

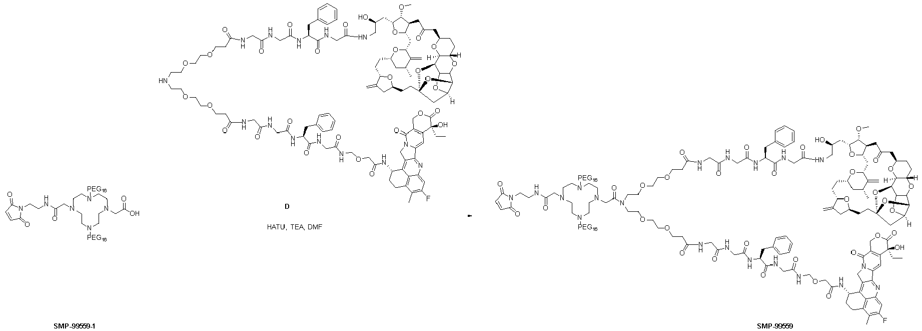

In a 4 mL reaction flask, the compound SMP-99559-1 (9.0 mg, 0.005 mmol) and the intermediate D (10.0 mg, 0.005 mmol) were dissolved in N,N-dimethylformamide (1.0 mL), N,N,N',N'-tetramethyl-O-(7-azabenzotriazol-1-yl)hexafluorophosphourea (3.8 mg, 0.01 mmol) and triethylamine (1.0 mg, 0.012 mmol) were added, and a reaction occurred at room temperature for 2 hours while stirring. TLC showed complete reaction, and then the reaction solution was purified by HPLC to obtain a compound SMP-99559, weighing 6.0 mg, with a yield of 30%.

MS (ESI) m/z: 1005 [M/4+H]⁺.

### Example 73: Preparation of compound SMP-59623

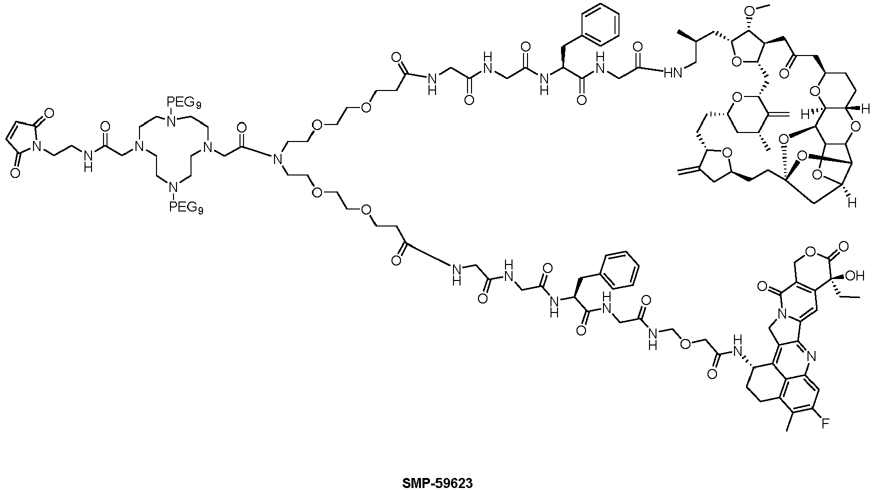

### Step 1: Preparation of compound SMP-59623-1

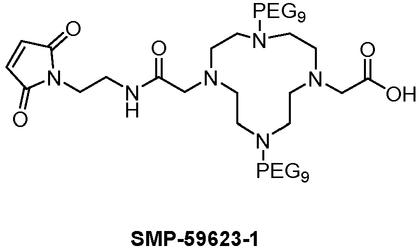

The synthesis method of compound SMP-59623-1 referred to the synthesis of SMP-37860-5.

MS (ESI) m/z: 572 [M/2+H]⁺.

### Step 2: Preparation of compound SMP-59623

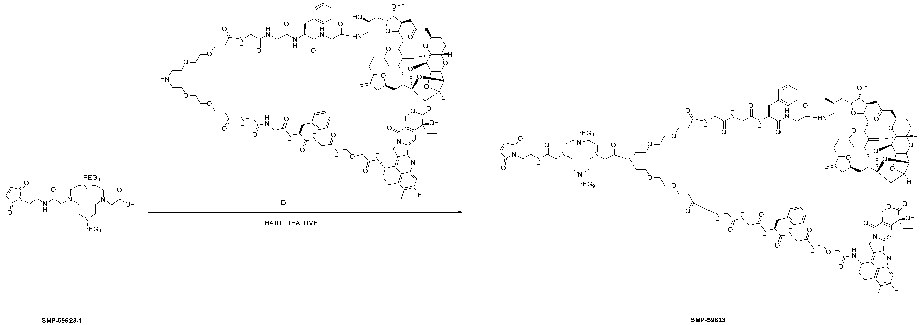

In a 4 mL reaction flask, the compound SMP-59623-1 (7.0 mg, 0.006 mmol) and the intermediate D (10.0 mg, 0.005 mmol) were dissolved in N,N-dimethylformamide (1.0 mL), N,N,N',N'-tetramethyl-O-(7-azabenzotriazol-1-yl)hexafluorophosphourea (3.8 mg, 0.01 mmol) and triethylamine (2.0 mg, 0.02 mmol) were added, and a reaction occurred at room temperature for 2 hours while stirring. TLC showed complete reaction, and then the reaction solution was purified by HPLC to obtain a compound SMP-59623, weighing 7.2 mg, with a yield of 36%.

MS (ESI) m/z: 1105[M/3+H]⁺.

### Example 74: Preparation of compound SMP-47610

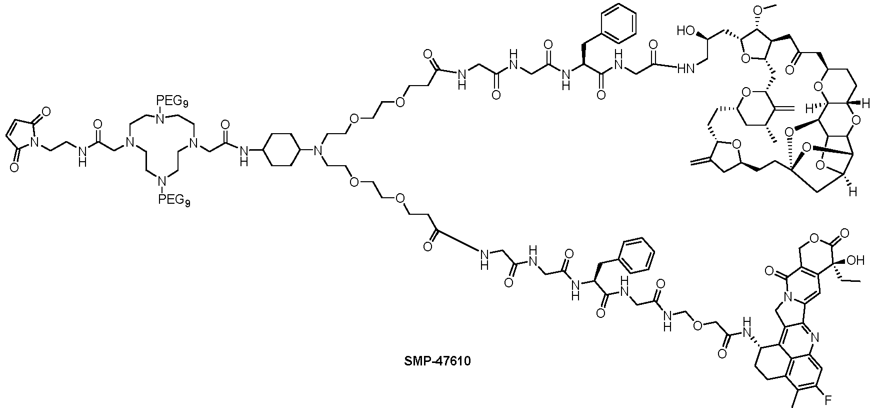

### Step 1: Preparation of compound SMP-47610

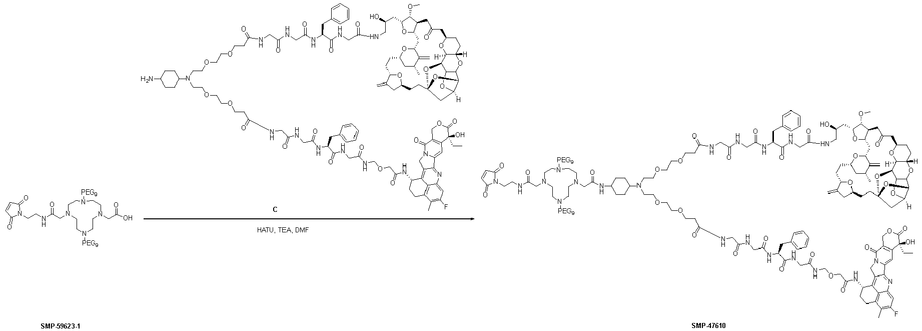

In a 4 mL reaction flask, the compound SMP-59623-1 (6.0 mg, 0.005 mmol) and the intermediate C (10.0 mg, 0.004 mmol) were dissolved in N,N-dimethylformamide (1.0 mL), N,N,N',N'-tetramethyl-O-(7-azabenzotriazol-1-yl)hexafluorophosphourea (3.8 mg, 0.01 mmol) and triethylamine (2 mg, 0.02 mmol) were added, and a reaction occurred at room temperature for 2 hours while stirring. LC-MS showed complete reaction, and then the reaction solution was purified by HPLC to obtain a compound SMP-47610, weighing 6.5 mg, with a yield of 38%.

MS (ESI) m/z: 1138 [M/3+H]⁺.

### Example 75: Preparation of compound SMP-86467

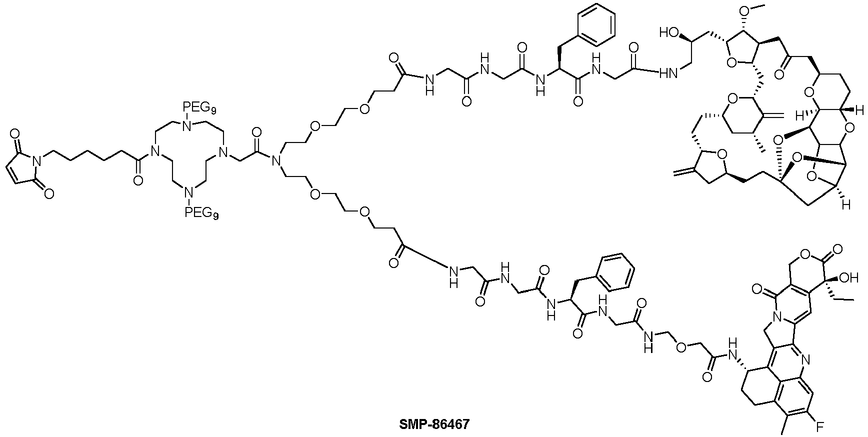

### Step 1: Preparation of compound SMP-86467-1

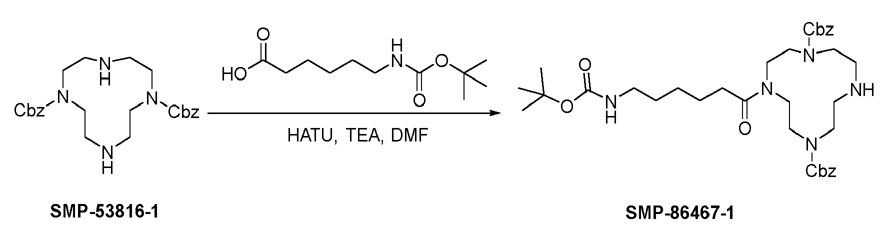

In a 500 mL reaction flask, 6-((tert-butoxycarbonyl)amino)hexanoic acid (584.0 mg, 2.38 mmol) and the compound SMP-53816-1 (1500 mg, 2.15 mmol) were dissolved in N,N-dimethylformamide (20 mL), and triethylamine (325.2 mg, 3.22 mmol) was added. N,N,N',N'-tetramethyl-O-(7-azabenzotriazol-1-yl)hexafluorophosphourea (817.0 mg, 2.15 mmol) was dissolved in N,N-dimethylformamide (20 mL) and then dropped into the above solution. A reaction occurred at room temperature for 2 hours while stirring. TLC showed complete reaction. After concentration under reduced pressure, the reaction solution was purified by HPLC to obtain a compound SMP-86467-1, weighing 788.0 mg, with a yield of 55%.

MS (ESI) m/z:654 [M+H]⁺.

### Step 2: Preparation of compound SMP-86467-2

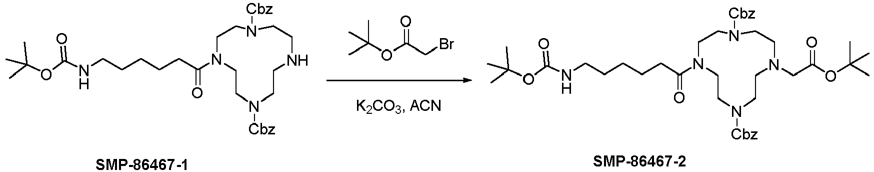

In a 100 mL reaction flask, tert-butyl bromoacetate (312.0 mg, 1.60 mmol) and the compound SMP-86467-1 (788.0 mg, 1.20 mmol) were dissolved in acetonitrile (20 mL), and potassium carbonate (828.0 mg, 6.00 mmol) was added, followed by heating to 55°C and reaction for 20 hours. TLC showed complete reaction. The reaction solution was cooled to room temperature and then filtered, the filtrate was concentrated under reduced pressure to dryness, and the residue was purified by silica gel column chromatography (dichloromethane: methanol=20:1) to obtain a compound SMP-86467-2, weighing 900.0 mg, with a yield of 97%.

MS (ESI) m/z: 768 [M+H]⁺.

### Step 3: Preparation of compound SMP-86467-3

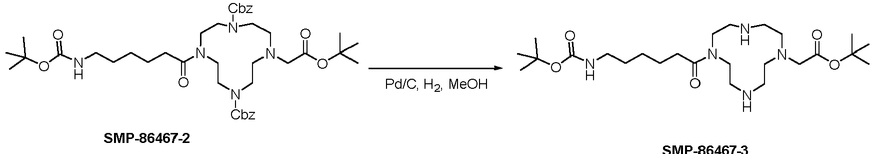

In a 100 mL reaction flask, the compound SMP-86467-2 (900.0 mg, 1.17 mmol) was dissolved in methanol (20 mL), a palladium carbon catalyst (90.0 mg) was added, air in the flask was replaced with a hydrogen balloon, and a reaction occurred at room temperature for 20 hours while stirring. LC-MS showed complete reaction. After filtration, the filtrate was concentrated under reduced pressure to dryness to obtain a compound SMP-86467-3, weighing 467.0 mg, with a yield of 80%.

MS (ESI) m/z:500 [M+H]⁺, 400 [M+H-100]⁺.

### Step 4: Preparation of compound SMP-86467-4

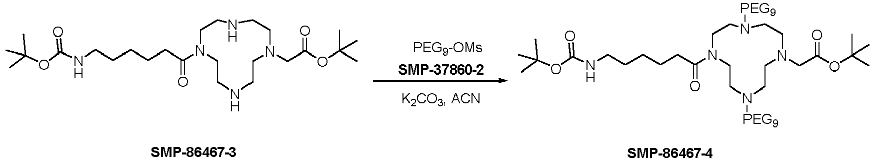

In a 50mL reaction flask, the compound SMP-86467-3 (467.0 mg, 0.93 mmol) and the compound SMP-37860-2 (1.01 g, 2.00 mmol) were dissolved in acetonitrile (12 mL), potassium carbonate (552.0 mg, 4.00 mmol) was added, and a reaction occurred at room temperature for 20 hours. LC-MS test showed complete reaction. After filtration, the filtrate was concentrated under reduced pressure to dryness, followed by silica gel column chromatography (dichloromethane: methanol=12:1) to obtain a compound SMP-86467-4, weighing 737.0 mg, with a yield of 60%.

MS (ESI) m/z: 661 [M/2+H]⁺.

### Step 5: Preparation of compound SMP-86467-5

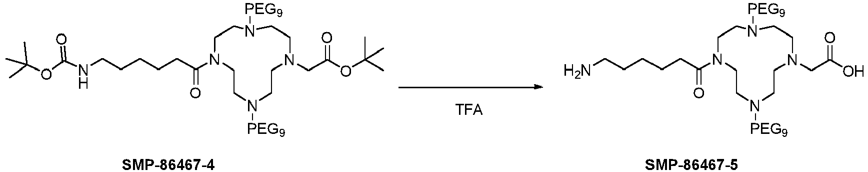

The compound SMP-86467-4 (737.0 mg, 0.56 mmol) was added to a 10 mL reaction flask, then trifluoroacetic acid (4 mL) was added, and a reaction occurred at room temperature for 3 hours while stirring. LC-MS test showed complete reaction. The trifluoroacetic acid was removed by concentration under reduced pressure, and a compound SMP-86467-5 was obtained, weighing 609.0 mg, with a yield of 90%. MS (ESI) m/z: 1165[M+H]⁺, 583 [M/2+H]⁺.

### Step 6: Preparation of compound SMP-86467-6

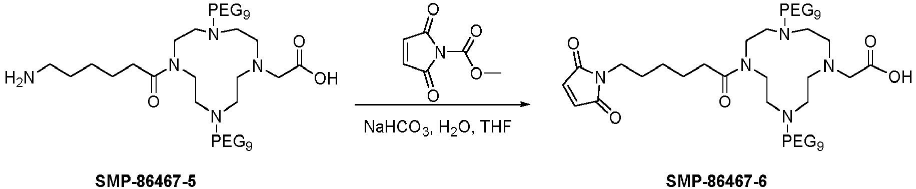

In a 25 mL reaction flask, the compound SMP-86467-5 (609.0 mg, 0.52 mmol) was dissolved in pure water (6 mL), and sodium bicarbonate (189.0 mg, 2.25 mmol) was added. N-methoxycarbonyl maleimide (124 mg, 0.80 mmol) was dissolved in tetrahydrofuran (3 mL) and dropped into the above solution, and a reaction occurred for 2 hours. LC-MS test showed complete reaction. The tetrahydrofuran was removed by concentration under reduced pressure, and the residue was purified by HPLC to obtain a compound SMP-86467-6, weighing 620.0 mg, with a yield of 95%.

MS (ESI) m/z: 623 [M/2+H]⁺.

### Step 7: Preparation of compound SMP-86467

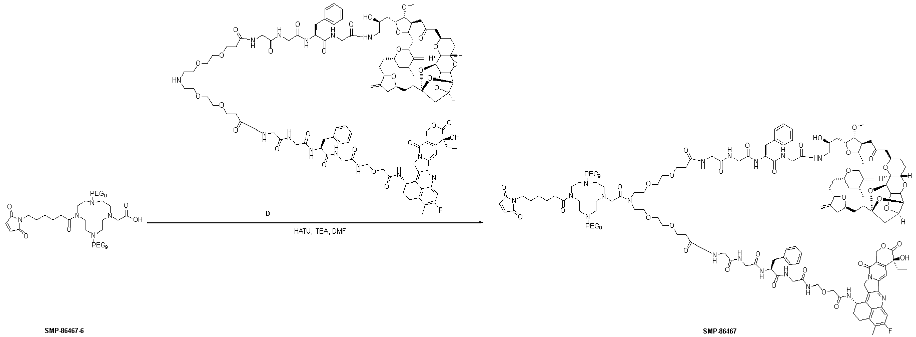

In a 4 mL reaction flask, the compound SMP-86467-6 (15.0 mg, 0.012 mmol) and the intermediate D (20.0 mg, 0.009 mmol) were dissolved in N,N-dimethylformamide (1.0 mL), N,N,N',N'-tetramethyl-O-(7-azabenzotriazol-1-yl)hexafluorophosphourea (3.8 mg, 0.012 mmol) and triethylamine (2.0 mg, 0.015 mmol) were added, and a reaction occurred at room temperature for 2 hours while stirring. LC-MS test showed complete reaction. The reaction solution was purified by HPLC to obtain a compound SMP-59623, weighing 11.0 mg, with a yield of 36%. MS (ESI) m/z: 1139 [M/3+H]⁺.

### Example 76: Preparation of compound SMP-76899

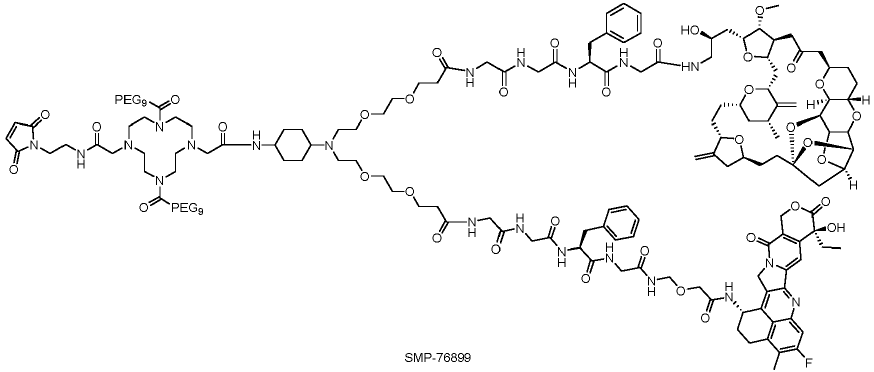

### Step 1: Preparation of compound SMP-76899-1

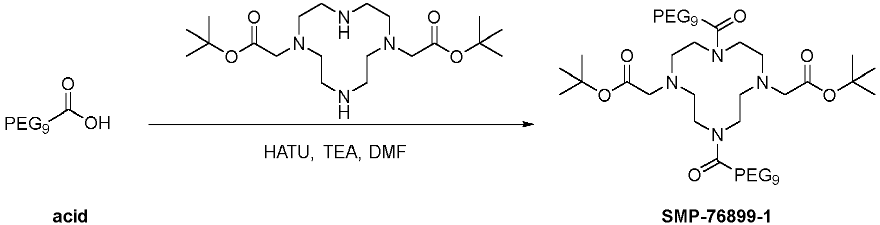

In a 50 mL reaction flask, 3- [(23-methoxy-3,6,9,12,15,18,21-heptaoxatricos-1-yl)oxy]propionic acid (500.0 mg, 1.09 mmol) and 1,7-bis-(N-tert-butoxycarbonylmethyl)-1,4,7,10-tetraazacyclododecane (181.0 mg, 0.45 mmol) were dissolved in N,N-dimethylformamide (15 mL), N,N,N',N'-tetramethyl-O-(7-azabenzotriazol-1-yl)hexafluorophosphourea (437.0 mg, 1.15 mmol) and triethylamine (141.4 mg, 1.40 mmol) were added, and a reaction occurred at room temperature for 7 hours while stirring. LC-MS test showed complete reaction. The reaction solution was purified by HPLC to obtain a compound SMP-76899-1, weighing 448.0 mg, with a yield of 78%.

MS (ESI) m/z: 639 [M/2+H]⁺.

### Step 2: Preparation of compound SMP-76899-2

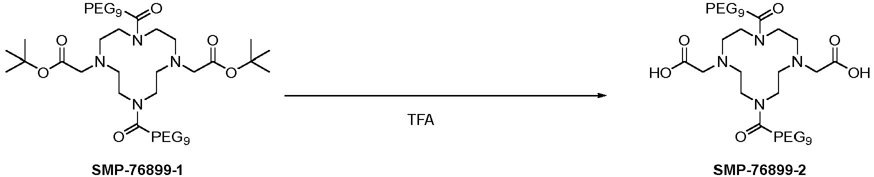

The compound SMP-76899-1 (448.0 mg, 0.34 mmol) was added to a 10 mL reaction flask, then trifluoroacetic acid (4 mL) was added, and a reaction occurred at room temperature for 3 hours while stirring. LC-MS test showed complete reaction. The trifluoroacetic acid was removed by distillation under reduced pressure, and the residual reaction solution was purified by HPLC to obtain a compound SMP-76899-2, weighing 350.0 mg, with a yield of 86%.

MS (ESI) m/z:583 [M/2+H]⁺.

### Step 3: Preparation of compound SMP-76899-3

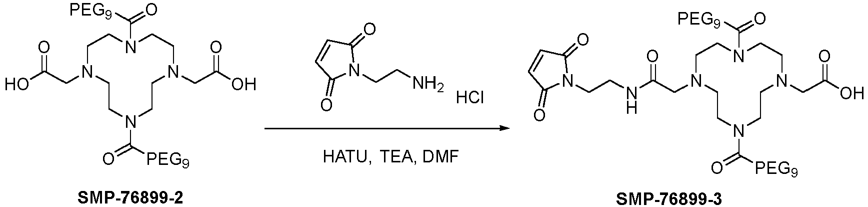

In a 25 mL reaction flask, 1-(2-aminoethyl)-1H-pyrrole-2,5-dione hydrochloride (39.0 mg, 0.22 mmol) and the compound SMP-76899-2 (350.0 mg, 0.29 mmol) were dissolved in N,N-dimethylformamide (5 mL), and triethylamine (50.5 mg, 0.50 mmol) was added. N,N,N',N'-tetramethyl-O-(7-azabenzotriazol-1-yl)hexafluorophosphourea (91.2 mg, 0.24 mmol) was dissolved in N,N-dimethylformamide (3 mL) and then dropped into the above solution. A reaction occurred at room temperature for 3 hours while stirring. LC-MS test showed complete reaction. The reaction solution was purified by HPLC to obtain a compound SMP-76899-3, weighing 250.0 mg, with a yield of 65%.

MS (ESI) m/z: 644 [M/2+H]⁺.

### Step 4: Preparation of compound SMP-76899

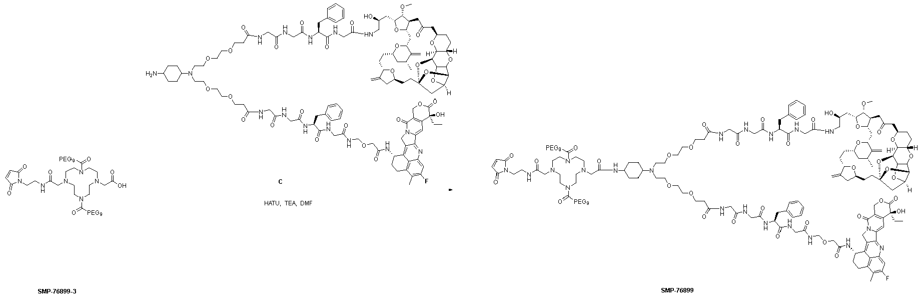

In a 4 mL reaction flask, the compound SMP-76899-3 (9.0 mg, 0.007 mmol) and the intermediate C (15.0 mg, 0.006 mmol) were dissolved in N,N-dimethylformamide (1.0 mL), N,N,N',N'-tetramethyl-O-(7-azabenzotriazol-1-yl)hexafluorophosphourea (3.8 mg, 0.01 mmol) and triethylamine (2 mg, 0.02 mmol) were added, and a reaction occurred at room temperature for 2 hours while stirring. LC-MS test showed complete reaction. The reaction solution was purified by HPLC to obtain a compound SMP-76899, weighing 6.4 mg, with a yield of 28%.

MS (ESI) m/z: 1186 [M/3+H]⁺.

### Example 77: Preparation of compound SMP-27341

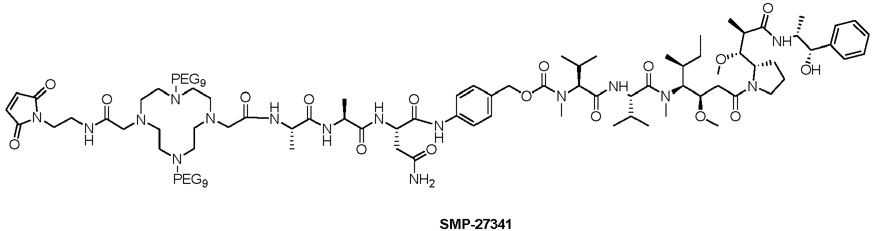

### Step 1: Preparation of compound SMP-27341-1

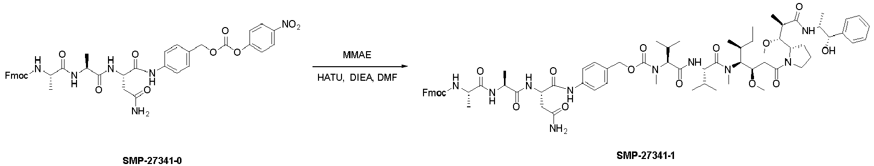

A compound SMP-27341-0 (300.0 mg, 0.40 mmol) and N,N-dimethylformamide (8 mL) were added to a 25 mL reaction flask. After complete clarification, N,N-diisopropylethylamine (7.8 mg, 0.78 mmol) and 1-hydroxybenzotriazole (27.0 mg, 0.20 mmol) were added, and then monomethyl auristatin E (286.0 mg, 0.4 mmol) was added at a time, followed by stirring at room temperature for 2 hours. LC-MS showed complete reaction. The N,N-dimethylformamide was removed by concentration under reduced pressure, and the residue was purified by column chromatography (dichloromethane: methanol=10:1) to obtain a compound SMP-27341-1, weighing 475.0 mg, with a yield of 89%.

MS (ESI) m/z:1344 [M+H]⁺

### Step 2: Preparation of compound SMP-27341-2

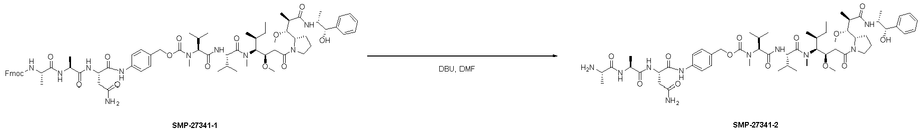

The intermediate SMP-27341-1 (241.0 mg, 0.18 mmol), 1,8-diazabicyclo[5.4.0]undec-7-ene (54.0 mg, 0.36 mmol), and N,N-dimethylformamide (6 mL) were added to a 25 mL reaction flask. A reaction occurred at room temperature for 30 minutes while stirring, and then TLC showed complete reaction. The N,N-dimethylformamide was removed by concentration under reduced pressure, and the residue was purified by HPLC to obtain a compound SMP-27341-2, weighing 172.0 mg, with a yield of 86%.

MS (ESI) m/z: 1122 [M+H]⁺.

### Step 3: Preparation of compound SMP-27341

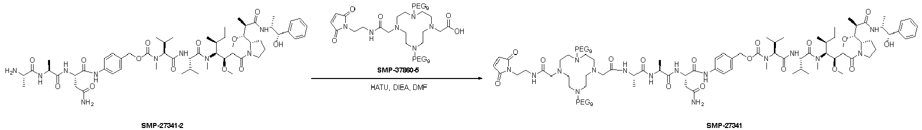

The compound SMP-37860-5 (25.0mg, 0.02 mmol) and a solvent N,N-dimethylformamide (2 mL) were added to a 4 mL reaction flask, and 2-(7-azabenzotriazole)-N,N,N',N'-tetramethylurea hexafluorophosphate (11.4 mg, 0.03 mmol) and N,N-diisopropylethylamine (3.9 mg, 0.03 mmol) were sequentially added. After reaction at room temperature for 5 minutes, the compound SMP-27341-2 (22.0 mg, 0.02 mmol) was added, the reaction continued for 25 minutes while stirring, and then TLC showed complete reaction. The reaction solution was purified by HPLC to obtain a target product SMP-27341, weighing 23.0 mg, with a yield of 51%.

MS (ESI) m/z: 1168 [M/2+H]⁺.

### Example 78: Preparation of compound SMP-39205

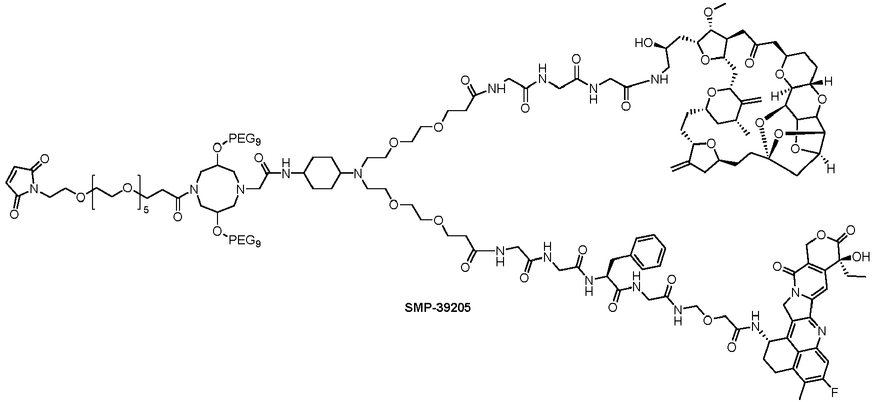

### Step 1: Preparation of compound SMP-39205

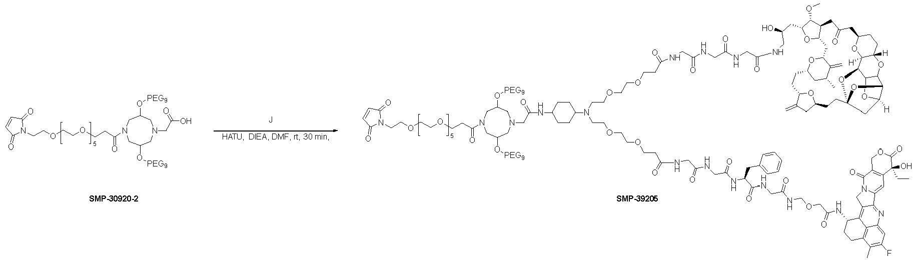

The compound SMP-30920-2 (35.0 mg, 0.024 mmol) and a solvent N,N-dimethylformamide (3 mL) were added to a 10 mL reaction flask, and 2-(7-azabenzotriazole)-N,N,N',N'-tetramethylurea hexafluorophosphate (9.0 mg, 0.024 mmol) and N,N-diisopropylethylamine (4.0 mg, 0.03 mmol) were sequentially added. After reaction at room temperature for 5 minutes, the compound J (43.0 mg, 0.020 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-39205, weighing 51.0 mg, with a yield of 71%. MS (ESI) m/z: 1187 [M/3+H]⁺.

### Example 80: Preparation of compound SMP-68962

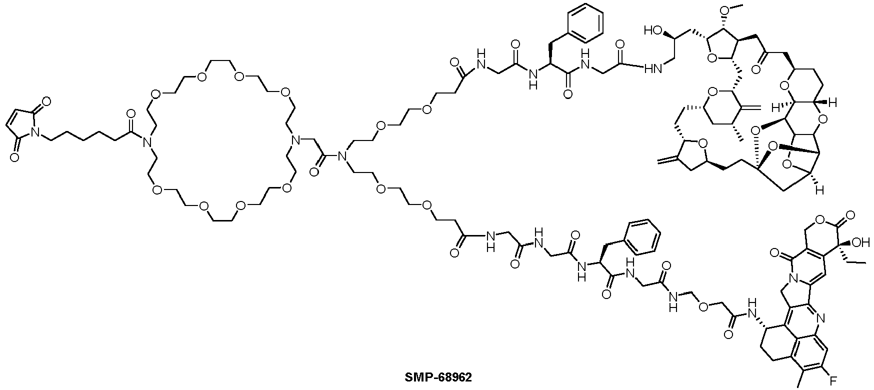

### Step 1: Preparation of compound SMP-68962-1

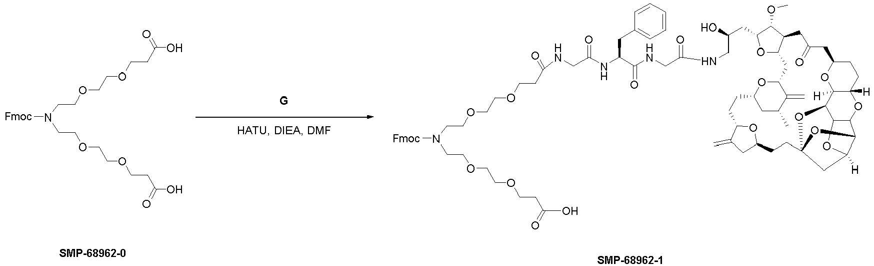

SMP-68962-0 (33.5 mg, 0.060 mmol), the intermediate G (30.0 mg, 0.030 mmol), N,N-dimethylformamide (2 mL), and N,N-diisopropylethylamine (19.4 mg, 0.150 mmol) were sequentially added to a 10 mL reaction flask, N,N,N',N'-tetramethyl-O-(7-azabenzotriazol-1-yl)hexafluorophosphourea (11.4 mg, 0.030 mmol, dissolved in 0.5 mL of N,N-dimethylformamide) was slowly added dropwise in ice water, then the reaction continued for 20 minutes, and LC-MS showed that most was the molecular weight of a target compound. The reaction solution was concentrated under reduced pressure to obtain a crude product, and the crude product was purified by HPLC to obtain a compound SMP-68962-1, weighing 25.0 mg, with a yield of 54.4%.

MS (ESI) m/z: 767[M/2+H]⁺.

### Step 2: Preparation of compound SMP-68962-2

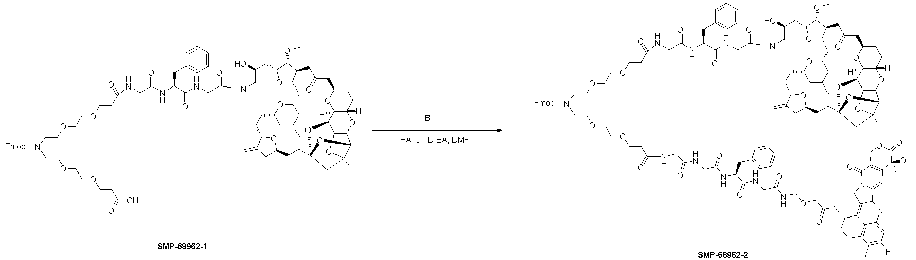

The SMP-68962-1 (25.0 mg, 0.016 mmol), the intermediate B (16.5 mg, 0.019 mmol), N,N-diisopropylethylamine (3.1 mg, 0.024 mmol), and N,N-dimethylformamide (2 mL) were sequentially added to a 10 mL reaction flask, then N,N,N',N'-tetramethyl-O-(7-azabenzotriazol-1-yl)hexafluorophosphourea (7.4 mg, 0.019 mmol) was added, a reaction occurred at room temperature for 20 minutes, and then LC-MS showed that most was the molecular weight of a target compound. The reaction solution was concentrated under reduced pressure to obtain a crude product, and the crude product was purified by HPLC to obtain a compound SMP-68962-2, weighing 20.0 mg, with a yield of 53.1%.

MS (ESI) m/z: 785[M/3+H]⁺.

### Step 3: Preparation of compound SMP-68962-3

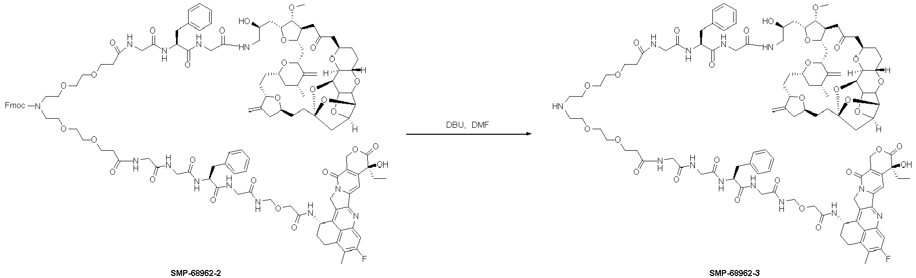

The SMP-68962-2 (20.0 mg, 0.008 mmol), N,N-dimethylformamide (1.5 mL), and 1,8-diazabicyclo[5.4.0]undec-7-ene (2.0 mg, 0.012 mmol) were sequentially added to a 10 mL reaction flask, a reaction occurred at room temperature for 30 minutes while stirring, and then LC-MS showed that most was the molecular weight of a target compound. The reaction solution was concentrated under reduced pressure to obtain a crude product, and the crude product was purified by HPLC to obtain a compound SMP-68962-3, weighing 12.0 mg, with a yield of 70.4%.

MS (ESI) m/z: 711[M/3+H]⁺, 1067[M/2+H]⁺.

### Step 4: Preparation of compound SMP-68962

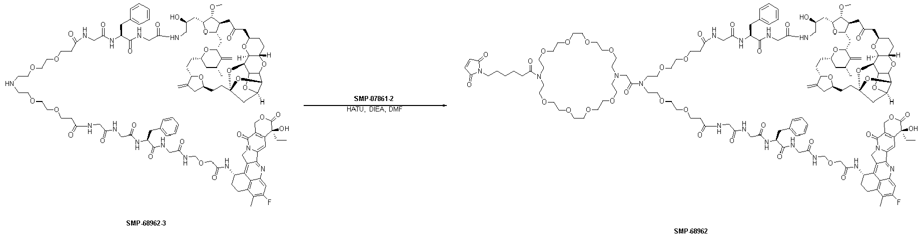

The SMP-68962-3 (12.0 mg, 0.0056 mmol), SMP-07861-2 (5.0 mg, 0.0067 mmol), N,N-diisopropylethylamine (1.1 mg, 0.0084 mmol), and N,N-dimethylformamide (1.5 mL) were sequentially added to a 10 mL reaction flask, then N,N,N',N'-tetramethyl-O-(7-azabenzotriazol-1-yl)hexafluorophosphourea (2.5 mg, 0.0067 mmol) was added, a reaction occurred at room temperature for 20 minutes, and then LC-MS showed that most was the molecular weight of a target compound. The reaction solution was concentrated under reduced pressure to obtain a crude product, and the crude product was purified by HPLC to obtain a compound SMP-68962, weighing 9.6 mg, with a yield of 60.0%. MS (ESI) m/z: 953[M/3+H]⁺, 1430[M/2+H]⁺.

### Example 81: Preparation of compound SMP-31135

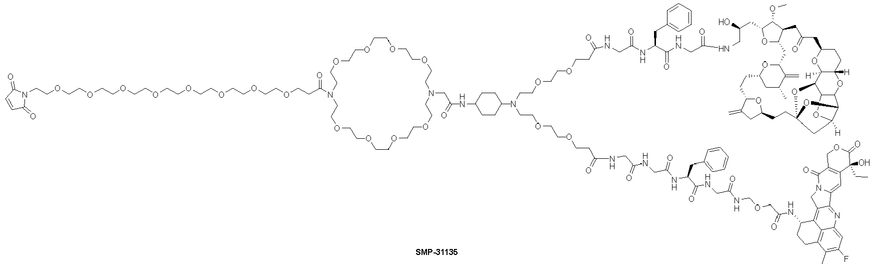

### Step 1: Preparation of compound SMP-31135-1

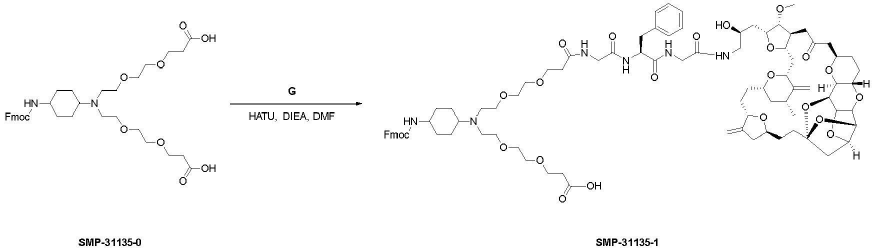

SMP-31135-0 (39.4 mg, 0.060 mmol), the intermediate G (30.0 mg, 0.030 mmol), N,N-diisopropylethylamine (19.4 mg, 0.150 mmol), and N,N-dimethylformamide (2 mL) were sequentially added to a 10 mL reaction flask, N,N,N',N'-tetramethyl-O-(7-azabenzotriazol-1-yl)hexafluorophosphourea (11.4 mg, 0.030 mmol, dissolved in 0.5 mL of N,N-dimethylformamide) was slowly added dropwise in ice water, then the reaction continued at this temperature for 20 minutes, and LC-MS showed that most was the molecular weight of a target compound. The reaction solution was concentrated under reduced pressure to obtain a crude product, and the crude product was purified by HPLC to obtain a compound SMP-31135-1, weighing 27 mg, with a yield of 55.3%.

MS (ESI) m/z: 815[M/2+H]⁺.

### Step 2: Preparation of compound SMP-31135-2

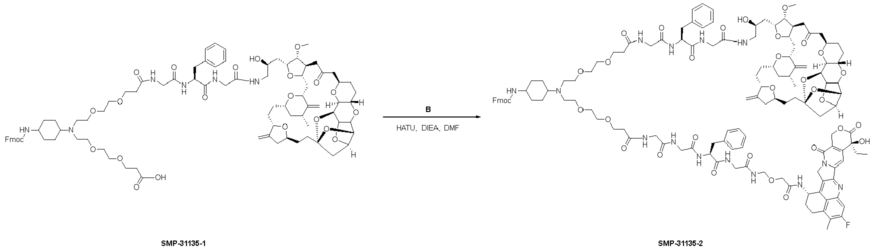

The SMP-31135-1 (27.0 mg, 0.016 mmol), B (16.0 mg, 0.019 mmol), N,N-diisopropylethylamine (3.1 mg, 0.024 mmol), and N,N-dimethylformamide (2 mL) were sequentially added to a 10 mL reaction flask and stirred at room temperature, then N,N,N',N'-tetramethyl-O-(7-azabenzotriazol-1-yl)hexafluorophosphourea (7.2 mg, 0.019 mmol) was added, a reaction occurred under this condition for 20 minutes, and then LC-MS showed that most was the molecular weight of a target compound. The reaction solution was concentrated under reduced pressure to obtain a crude product, and the crude product was purified by HPLC to obtain a compound SMP-31135-2, weighing 22.0 mg, with a yield of 56.1%.

MS (ESI) m/z: 818[M/3+H]⁺.

### Step 3: Preparation of compound SMP-31135-3

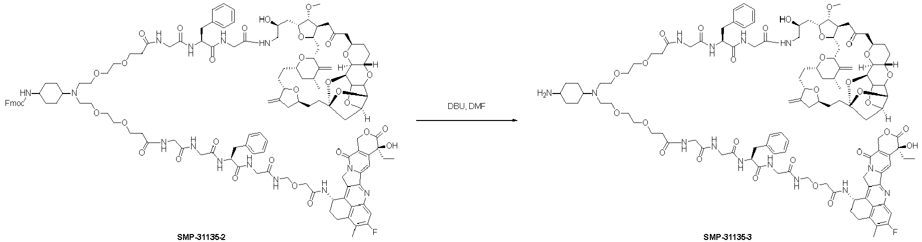

The SMP-31135-2 (22.0 mg, 0.009 mmol), N,N-dimethylformamide (1.5 mL), and 1,8-diazabicyclo[5.4.0]undec-7-ene (2.0 mg, 0.013 mmol) were sequentially added to a 10 mL reaction flask, a reaction occurred at room temperature for 30 minutes while stirring, and then LC-MS showed that most was the molecular weight of a target compound. The reaction solution was concentrated under reduced pressure to obtain a crude product, and the crude product was purified by HPLC to obtain a compound SMP-31135-3, weighing 15.0 mg, with a yield of 74.8%.

MS (ESI) m/z: 744[M/3+H]⁺, 1115[M/2+H]⁺.

### Step 4: Preparation of compound SMP-31135

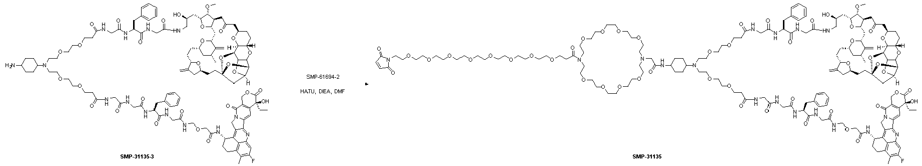

The SMP-31135-3 (15 mg, 0.0067 mmol), SMP-61694-2 (8.1 mg, 0.0081 mmol), N,N-diisopropylethylamine (1.3 mg, 0.010 mmol), and N,N-dimethylformamide (1.5 mL) were sequentially added to a 10 mL reaction flask, then N,N,N',N'-tetramethyl-O-(7-azabenzotriazol-1-yl)hexafluorophosphourea (3.1 mg, 0.0081 mmol) was added, a reaction occurred at room temperature for 20 minutes, and then LC-MS showed that most was the molecular weight of a target compound. The reaction solution was concentrated under reduced pressure to obtain a crude product, and the crude product was purified by HPLC to obtain a compound SMP-31135, weighing 11.2 mg, with a yield of 52.1%. MS (ESI) m/z: 1071[M/3+H]⁺, 803 [M/4+H]⁺.

### Example 82: Preparation of compound SMP-64791

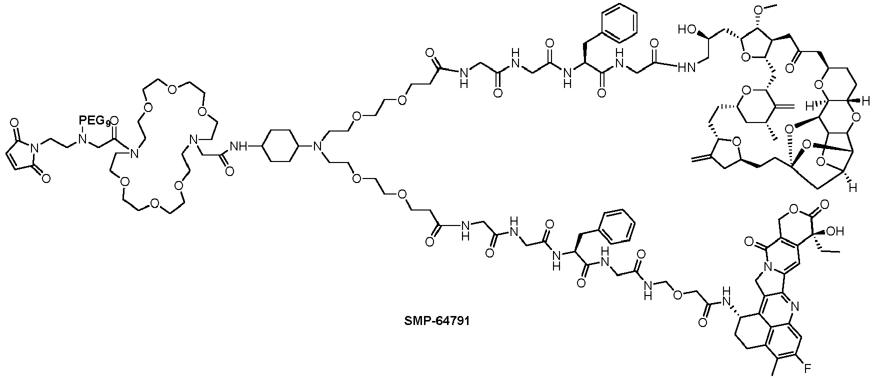

### Step 1: Preparation of compound SMP-64791-1

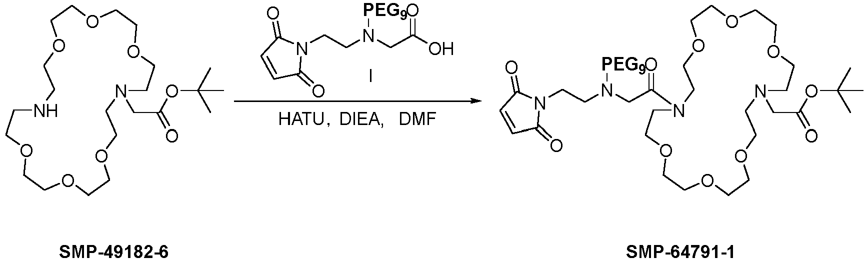

The SMP-49182-6 (50.0 mg, 0.11 mmol), the intermediate I (79 mg, 0.13 mmol), N,N-diisopropylethylamine (20.6 mg, 0.16 mmol), and N,N-dimethylformamide (4 mL) were sequentially added to a 10 mL reaction flask and stirred at room temperature, then N,N,N',N'-tetramethyl-O-(7-azabenzotriazol-1-yl)hexafluorophosphourea (49.4 mg, 0.13 mmol) was added, a reaction occurred under this condition for 20 minutes, and then LC-MS showed that most was the molecular weight of a target compound. The reaction solution was concentrated under reduced pressure to obtain a crude product, and the crude product was purified by HPLC to obtain a compound SMP-64791-1, weighing 45.0 mg, with a yield of 38.8%.

MS (ESI) m/z: 1055[M+H]⁺.

### Step 2: Preparation of compound SMP-64791-2

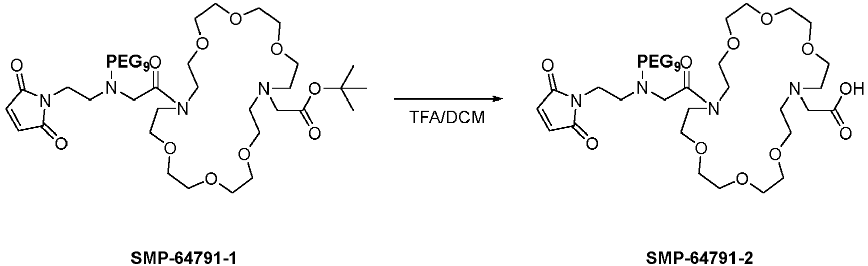

The SMP-64791-1 (45.0 mg, 0.043 mmol), dichloromethane (2 mL), and trifluoroacetic acid (2 mL) were sequentially added to a 10 mL reaction flask and reacted at room temperature for 2 hours while stirring, and then LC-MS showed complete reaction. The reaction solution was concentrated under reduced pressure, and the residue was purified by HPLC to obtain a compound SMP-64791-2, weighing 30.0 mg, with a yield of 70.0%.

MS (ESI) m/z: 999[M+H]⁺.

### Step 3: Preparation of compound SMP-64791

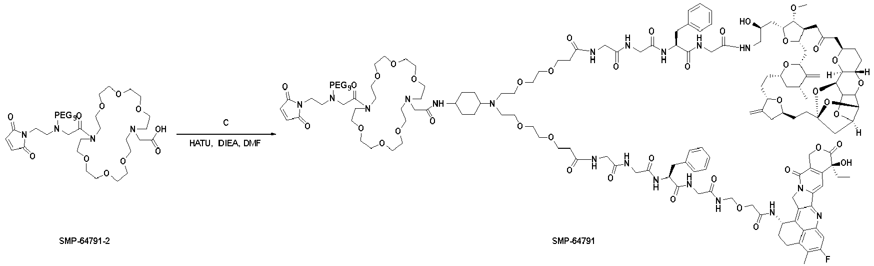

The SMP-64791-2 (6.3 mg, 0.0063 mmol), the intermediate C (12.0 mg, 0.0052 mmol), N,N-diisopropylethylamine (1.0 mg, 0.0078 mmol), and N,N-dimethylformamide (1.5 mL) were sequentially added to a 10 mL reaction flask and stirred at room temperature, then N,N,N',N'-tetramethyl-O-(7-azabenzotriazol-1-yl)hexafluorophosphourea (2.4 mg, 0.0063 mmol) was added, a reaction occurred under this condition for 20 minutes, and then LC-MS showed that most was the molecular weight of a target compound. The reaction solution was concentrated to obtain a crude product, and the crude product was purified by HPLC to obtain a compound SMP-64791, weighing 8.6 mg, with a yield of 52%.

MS (ESI) m/z: 1089[M/3+H]⁺.

### Example 83: Preparation of compound SMP-26598

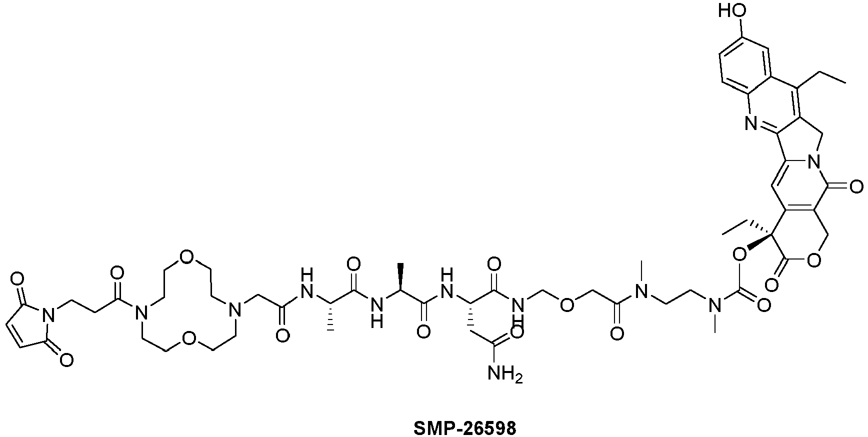

### Step 1: Preparation of compound SMP-26598-2

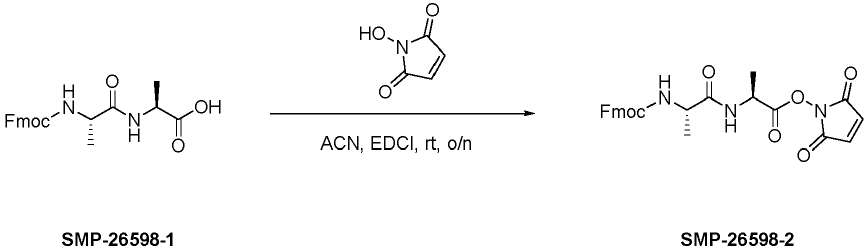

An intermediate SMP-26598-1 (21.5 g, 56.49 mmol) and acetonitrile (200 mL) were added to a 500 mL reaction flask and stirred at room temperature, then 1-(3-dimethylaminopropyl)-3-ethylcarbodiimide hydrochloride (10.8 g, 56.49 mmol) and N-hydroxysuccinimide (6.5 g, 56.49 mmol) were sequentially added, a reaction occurred under this condition overnight while stirring, and then TLC showed complete reaction. The reaction solution was filtered, and the obtained solid was dried in vacuum to obtain a compound SMP-26598-2, weighing 22.0 g, with a yield of 81%.

MS (ESI) m/z: 478 [M+H]⁺.

### Step 3: Preparation of compound SMP-26598-3

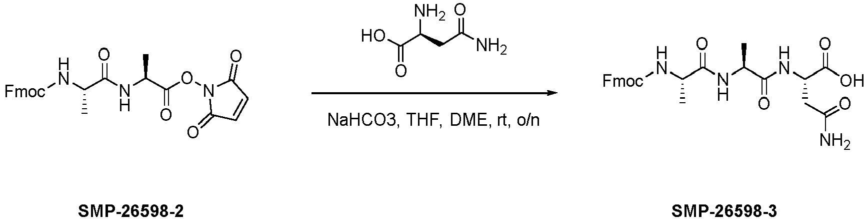

An intermediate L-aspartic acid (6.0 g, 46.12 mmol), sodium bicarbonate (7.7 g, 92.24 mmol), water (20 mL), and tetrahydrofuran (200 mL) were sequentially added to a 500 mL reaction flask and stirred at room temperature. After complete clarification, the compound SMP-26598-2 (22 g, 46.12 mmol) was dissolved in ethylene glycol dimethyl ether (50 mL) and slowly added to the reaction solution. The stirring continued for reaction overnight. TLC showed complete reaction. The reaction solution was concentrated under reduced pressure, and the residue was slowly dropped into a 0.5 M hydrochloric acid aqueous solution (500 mL) to precipitate a large amount of solid. After filtration, the obtained solid was dried in vacuum to obtain a compound SMP-26598-3, weighing 15.0 g, with a yield of 65%.

MS (ESI) m/z: 497 [M+H]⁺.

### Step 4: Preparation of compound SMP-26598-4

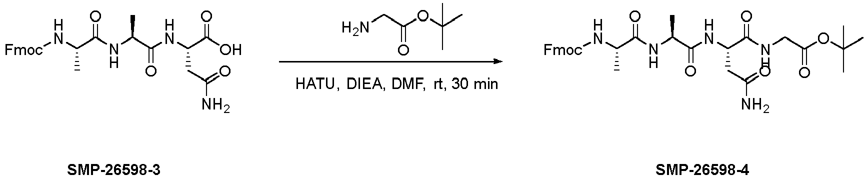

The compound SMP-26598-3 (450.0 mg, 0.90 mmol) and a solvent N,N-dimethylformamide (8 mL) were added to a 25 mL reaction flask and stirred at room temperature. After the solution was completely clarified, 2-(7-azabenzotriazole)-N,N,N',N'-tetramethylurea hexafluorophosphate (410.4 mg, 1.08 mmol) and N,N-diisopropylethylamine (175.4 mg, 1.36 mmol) were sequentially added. After reaction for 5 minutes, a compound tert-butyl glycinate (141.5 mg, 1.08 mmol) was added, the reaction continued for 25 minutes while stirring, and then TLC showed complete reaction. The reaction solution was concentrated under reduced pressure, and the residue was purified by column chromatography (dichloromethane: methanol=15:1) to obtain an intermediate SMP-26598-4, weighing 330.0 mg, with a yield of 60.2%.

MS (ESI) m/z: 610 [M+H]⁺.

### Step 5: Preparation of compound SMP-26598-5

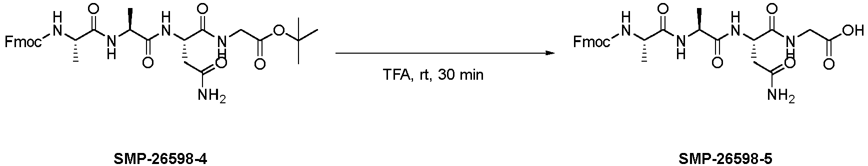

The compound SMP-26598-4 (330.0 mg, 0.54 mmol) and a solvent trifluoroacetic acid (4 mL) were added to a 25 mL reaction flask, a reaction occurred at room temperature for 30 minutes while stirring, and then LC-MS showed complete reaction. The reaction solution was concentrated under reduced pressure, and the residue was purified by HPLC to obtain an intermediate SMP-26598-5, weighing 200.0 mg, with a yield of 67.0%.

MS (ESI) m/z: 554 [M+H]⁺.

### Step 6: Preparation of compound SMP-26598-6

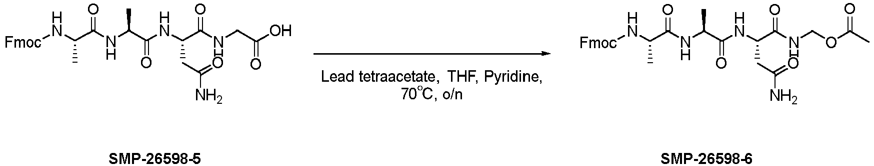

The compound SMP-26598-5 (200.0 mg, 0.36 mmol) and a solvent tetrahydrofuran (20 mL) were added to a 50 mL reaction flask and stirred at room temperature. After the solution was completely clarified, lead tetraacetate (191.8 mg, 0.44 mmol) and pyridine (44.8 mg, 0.56 mmol) were sequentially added, a reaction occurred at 70°C overnight while stirring, and then TLC showed complete reaction. The reaction solution was filtered, the filtrate was concentrated under reduced pressure, and the residue was purified by column chromatography (dichloromethane: methanol=12:1) to obtain an intermediate SMP-26598-6, weighing 132.0 mg, with a yield of 64.7%. MS (ESI) m/z: 568 [M+H]⁺.

### Step 7: Preparation of compound SMP-26598-7

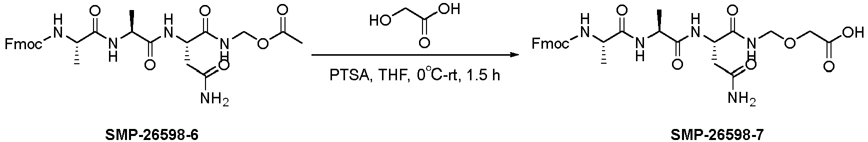

The compound SMP-26598-6 (132.0mg, 0.23 mmol), a solvent tetrahydrofuran (10 mL), and hydroxyacetic acid (21.0 mg, 0.28 mmol) were added to a 25 mL reaction flask and cooled to 0°C in an ice-water bath. After cooling to 0°C, p-toluenesulfonic acid (21.0 mg, 0.12 mmol) was added at a time, followed by slow heating to room temperature. A reaction occurred at this temperature for 1.5 hours, and then TLC showed complete reaction. The reaction solution was filtered and then concentrated under reduced pressure, and the residue was purified by HPLC to obtain an intermediate SMP-26598-7, weighing 86.0 mg, with a yield of 64.1%.

MS (ESI) m/z: 584 [M+H]⁺.

### Step 8: Preparation of compound SMP-26598-8

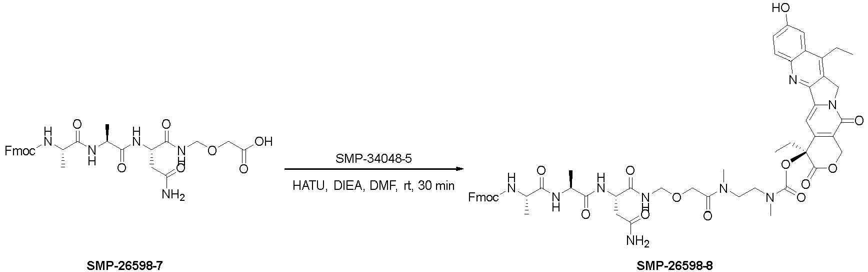

The compound SMP-26598-7 (62.2 mg, 0.107 mmol) and a solvent N,N-dimethylformamide (3 mL) were added to a 25 mL reaction flask and stirred at room temperature, and 2-(7-azabenzotriazole)-N,N,N',N'-tetramethylurea hexafluorophosphate (40.5 mg, 0.107 mmol) and N,N-diisopropylethylamine (17.2 mg, 0.133 mmol) were sequentially added. After reaction for 5 minutes, the compound SMP-34048-5 (45 mg, 0.089 mmol) was added, the reaction continued for 25 minutes while stirring, and then TLC showed complete reaction. The reaction solution was concentrated under reduced pressure, and the residue was purified by HPLC to obtain an intermediate SMP-26598-8, weighing 61.0 mg, with a yield of 64.0%. MS (ESI) m/z: 1072 [M+H]⁺.

### Step 9: Preparation of compound SMP-26598-9

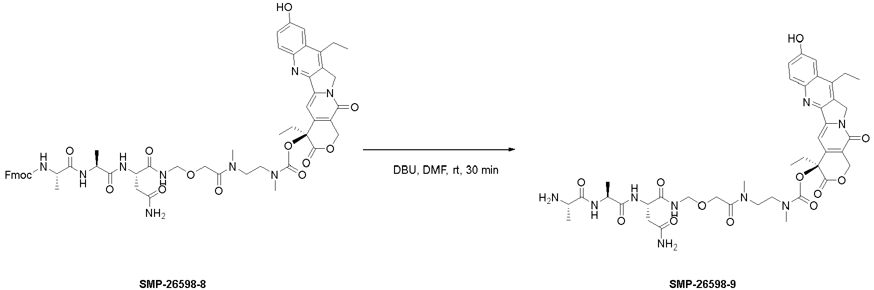

The intermediate SMP-26598-8 (61.0 mg, 0.057 mmol), N,N-dimethylformamide (3 mL), and 1,8-diazabicyclo[5.4.0]undec-7-ene (17.3 mg, 0.114 mmol) were added to a 25 mL reaction flask and reacted at room temperature for 30 minutes while stirring, and then TLC showed complete reaction. The reaction solution was concentrated under reduced pressure, and the residue was purified by HPLC to obtain a compound SMP-26598-9, weighing 33.0 mg, with a yield of 68.2%. MS (ESI) m/z: 850 [M+H]⁺.

### Step 10: Preparation of compound SMP-26598

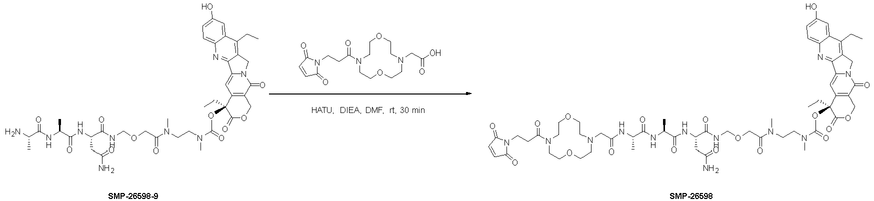

The compound SMP-93566-4 (17.8 mg, 0.046 mmol) and a solvent N,N-dimethylformamide (3 mL) were added to a 25 mL reaction flask and stirred at room temperature, and 2-(7-azabenzotriazole)-N,N,N',N'-tetramethylurea hexafluorophosphate (17.8 mg, 0.046 mmol) and N,N-diisopropylethylamine (7.5 mg, 0.058 mmol) were sequentially added. After reaction at this temperature for 5 minutes, the compound SMP-26598-9 (33.0 mg, 0.039 mmol) was added, the reaction continued for 25 minutes while stirring, and then TLC showed complete reaction. The reaction solution was concentrated under reduced pressure, and the residue was purified by HPLC to obtain an intermediate SMP-26598, weighing 27.0 mg, with a yield of 57.0%.

MS (ESI) m/z: 1215 [M+H]⁺.

### Example 85: Preparation of compound SMP-09168

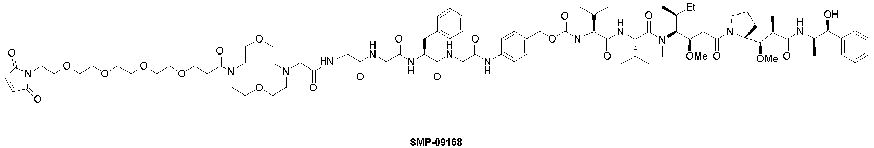

### Step 1: Preparation of compound SMP-09168-1

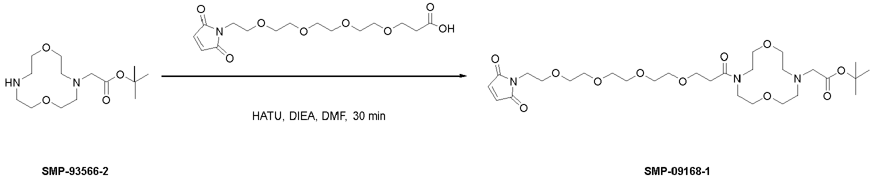

A compound maleimide-tetrapolyethylene glycol-carboxylic acid (30.0 mg, 0.087 mmol) and a solvent N,N-dimethylformamide (3 mL) were added to a 25 mL reaction flask and stirred at room temperature, and 2-(7-azabenzotriazole)-N,N,N',N'-tetramethylurea hexafluorophosphate (39.6 mg, 0.104 mmol) and N,N-diisopropylethylamine (16.8 mg, 0.13 mmol) were sequentially added. After reaction for 5 minutes, the compound SMP-93566-2 (25.0 mg, 0.087 mmol) was added, the reaction continued for 25 minutes while stirring, and then TLC showed complete reaction. The reaction solution was concentrated under reduced pressure, and the residue was purified by column chromatography (petroleum ether: ethyl acetate=1:1) to obtain an intermediate SMP-09168-1, weighing 38.0 mg, with a yield of 71.0%.

MS (ESI) m/z: 616 [M+H]⁺.

### Step 2: Preparation of compound SMP-09168-2

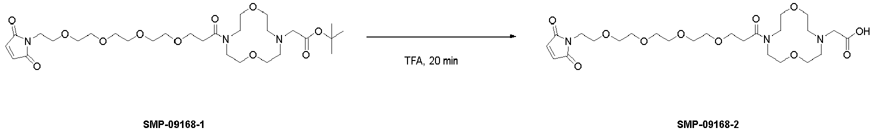

The compound SMP-09168-1 (38.0 mg, 0.062 mmol) and a solvent trifluoroacetic acid (1 mL) were added to a 25 mL reaction flask, a reaction occurred at room temperature for 20 minutes while stirring, and then LC-MS showed complete reaction. The reaction solution was concentrated under reduced pressure to remove excess trifluoroacetic acid, and the residue was purified by HPLC to obtain an intermediate SMP-09168-2, weighing 29.0 mg, with a yield of 83.5%.

MS (ESI) m/z: 560 [M+H]⁺.

### Step 3: Preparation of compound SMP-09168

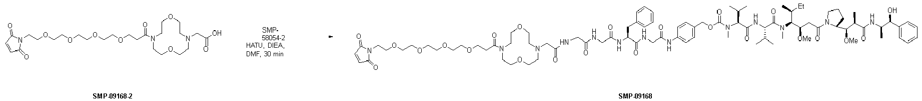

The compound SMP-09168-2 (11.7 mg, 0.021 mmol) and a solvent N,N-dimethylformamide (2 mL) were added to a 25 mL reaction flask and stirred at room temperature, and 2-(7-azabenzotriazole)-N,N,N',N'-tetramethylurea hexafluorophosphate (8.0 mg, 0.021 mmol) and N,N-diisopropylethylamine (3.3 mg, 0.026 mmol) were sequentially added. After reaction for 5 minutes, the compound SMP-58054-2 (20.0 mg, 0.017 mmol) was added, the reaction continued for 25 minutes while stirring, and then TLC showed complete reaction. The reaction solution was concentrated under reduced pressure, and the residue was purified by HPLC to obtain an intermediate SMP-09168, weighing 23.0 mg, with a yield of 78.4%. MS (ESI) m/z: 1726 [M+H]⁺.

### Example 86: Preparation of compound SMP-87055

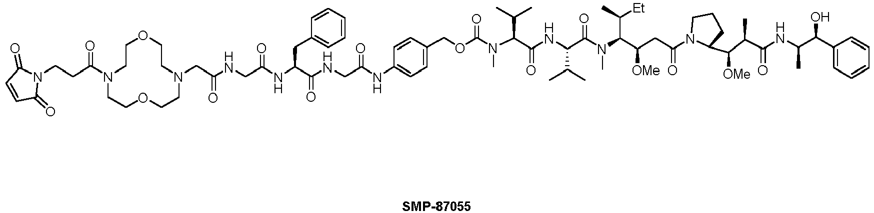

### Step 1: Preparation of compound SMP-87055-2

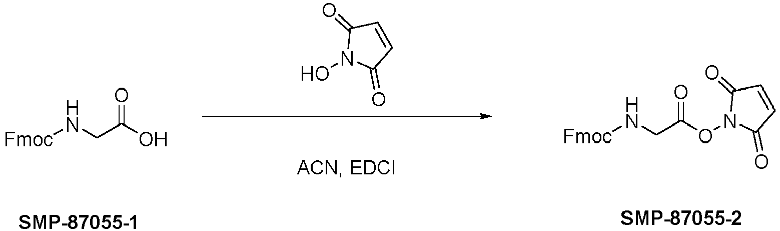

An intermediate SMP-87055-1 (13.5 g, 45.45 mmol) and acetonitrile (200 mL) were added to a 500 mL reaction flask and stirred at room temperature, 1-(3-dimethylaminopropyl)-3-ethylcarbodiimide hydrochloride (10.5 g, 54.54 mmol) and N-hydroxysuccinimide (6.3 g, 54.54 mmol) were sequentially added, a reaction occurred at this temperature overnight while stirring, and then TLC showed complete reaction. The reaction solution was filtered, and the obtained solid was dried in vacuum to obtain a compound SMP-26598-2, weighing 14.0 g, with a yield of 78.4%.

MS (ESI) m/z: 393 [M+H]⁺.

### Step 2: Preparation of compound SMP-87055-3

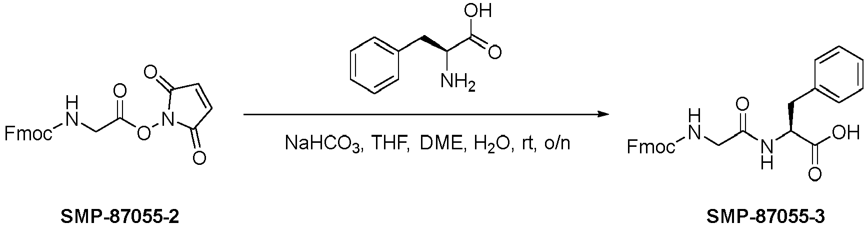

An intermediate L-phenylalanine (6.4 g, 35.71 mmol), sodium bicarbonate (6.0 g, 71.42 mmol), water (20 mL), and tetrahydrofuran (200 mL) were added to a 500 mL reaction flask and stirred at room temperature. After the solution was completely clarified, the compound SMP-87055-2 (14.0 g, 35.71 mmol) was dissolved in ethylene glycol dimethyl ether (50 mL) and slowly added to the reaction solution. The stirring continued for reaction overnight. TLC showed complete reaction. The reaction solution was concentrated under reduced pressure, and the residue was slowly dropped into a 0.5 M hydrochloric acid aqueous solution (500 mL) to precipitate a large amount of solid. After filtration, the obtained solid was dried in vacuum to obtain a compound SMP-87055-3, weighing 13.0 g, with a yield of 82.0%.

MS (ESI) m/z: 445 [M+H]⁺.

### Step 3: Preparation of compound SMP-87055-4

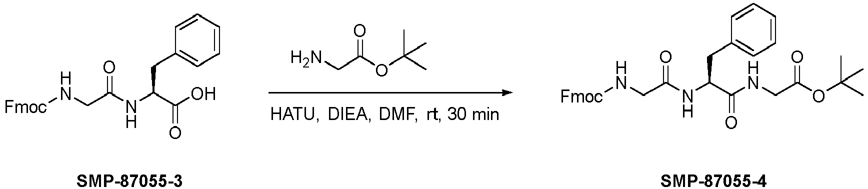

The compound SMP-87055-3 (13.0 g, 29.28 mmol) and a solvent N,N-dimethylformamide (30 mL) were added to a 25 mL reaction flask and stirred at room temperature, and 2-(7-azabenzotriazole)-N,N,N',N'-tetramethylurea hexafluorophosphate (11.1 g, 29.28 mmol) and N,N-diisopropylethylamine (5.6 g, 43.81 mmol) were sequentially added. After reaction for 5 minutes, a compound tert-butyl glycinate (4.6 g, 35.05 mmol) was added, the reaction continued for 25 minutes while stirring, and then TLC showed complete reaction. The reaction solution was concentrated under reduced pressure, and the residue was purified by column chromatography (dichloromethane: methanol=15:1) to obtain an intermediate SMP-87055-4, weighing 9.1 g, with a yield of 55.8%.

MS (ESI) m/z: 558 [M+H]⁺.

### Step 4: Preparation of compound SMP-87055-5

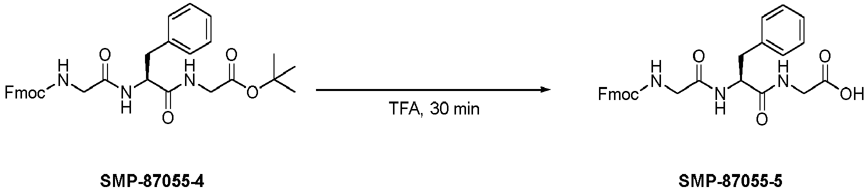

The compound SMP-87055-4 (9.1 g, 16.34 mmol) and a solvent trifluoroacetic acid (30 mL) were added to a 25 mL reaction flask, a reaction occurred at room temperature for 30 minutes while stirring, and then LC-MS showed complete reaction. The reaction solution was concentrated under reduced pressure, and the residue was purified by HPLC to obtain an intermediate SMP-87055-5, weighing 6.7 g, with a yield of 81.8%.

MS (ESI) m/z: 502 [M+H]⁺.

### Step 5: Preparation of compound SMP-87055-6

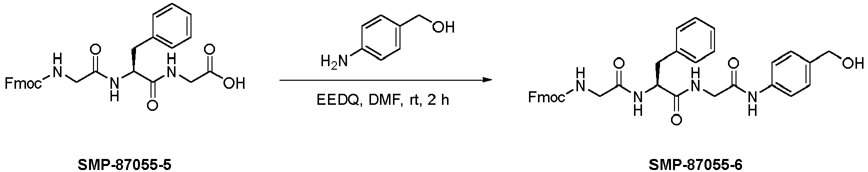

The intermediate SMP-87055-5 (2.1 g, 4.19 mmol), 4-aminobenzyl alcohol (1.0 g, 8.38 mmol), and N,N-dimethylformamide (3 mL) were added to a 100 mL reaction flask and stirred at room temperature, then 2-ethoxy-1-ethoxycarbonyl-1,2-dihydroquinoline (2.1 g, 8.38 mmol) was added, the stirring continued for reaction for 2 hours, the reaction solution was concentrated under reduced pressure to remove the solvent, and the residue was purified by silica gel column chromatography (dichloromethane: methanol=15:1) to obtain a compound SMP-87055-6, weighing 1.7 g, with a yield of 67.0%.

MS (ESI) m/z: 607 [M+H]⁺.

### Step 6: Preparation of compound SMP-87055-7

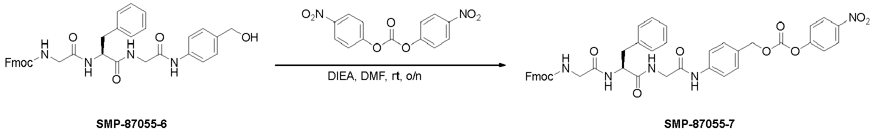

In a 25 mL reaction flask, the intermediate SMP-87055-6 (900.0 mg, 1.48 mmol) was dissolved in N,N-dimethylformamide (10 mL) and stirred at room temperature, then bis(p-nitrophenyl)carbonate (676.1 mg, 2.22 mmol) and triethylamine (298.7 mg, 2.96 mmol) were added, the stirring continued for reaction overnight, and TLC monitored complete reaction of the raw materials. The reaction solution was concentrated under reduced pressure, and the residue was purified by silica gel column chromatography (dichloromethane: methanol=20:1) to obtain a compound SMP-87055-7, weighing 740.0 mg, with a yield of 64.7%. MS (ESI) m/z: 772 [M+H]⁺.

### Step 7: Preparation of compound SMP-87055-8

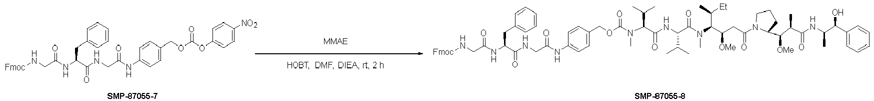

The compound SMP-87055-7 (461 mg, 0.60 mmol) and N,N-dimethylformamide (5 mL) were added to a 25 mL reaction flask and stirred at room temperature. After the solution was completely clarified, N,N-diisopropylethylamine (103.2 mg, 0.80 mmol), 1-hydroxybenzotriazole (27.0 mg, 0.20 mmol), and monomethyl auristatin E (286.8 mg, 0.40 mmol) were sequentially added. The stirring continued at room temperature for 2 hours. LC-MS showed complete reaction. The reaction solution was concentrated under reduced pressure, and the residue was purified by column chromatography (dichloromethane: methanol=10:1) to obtain a compound SMP-87055-8, weighing 480.0 mg, with a yield of 88.9%.

MS (ESI) m/z: 1351 [M+H]⁺.

### Step 8: Preparation of compound SMP-87055-9

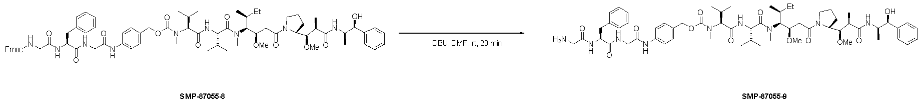

The intermediate SMP-87055-8 (240.0 mg, 0.18 mmol) and N,N-dimethylformamide (4 mL) were added to a 25 mL reaction flask and stirred at room temperature, then 1,8-diazabicyclo[5.4.0]undec-7-ene (54.0 mg, 0.36 mmol) was added, the reaction continued for 20 minutes, and then TLC showed complete reaction. The reaction solution was concentrated under reduced pressure, and the residue was purified by HPLC to obtain a compound SMP-87055-9, weighing 173.0 mg, with a yield of 85.2%.

MS (ESI) m/z: 1129 [M+H]⁺.

### Step 9: Preparation of compound SMP-87055

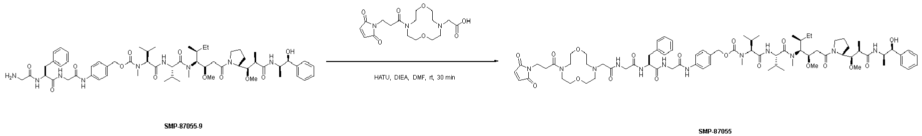

The compound SMP-93566-4 (9.2mg, 0.024 mmol) and a solvent N,N-dimethylformamide (1.5 mL) were added to a 10 mL reaction flask and stirred at room temperature, and 2-(7-azabenzotriazole)-N,N,N',N'-tetramethylurea hexafluorophosphate (9.1 mg, 0.024 mmol) and N,N-diisopropylethylamine (3.9 mg, 0.030 mmol) were sequentially added. After reaction for 5 minutes, the compound SMP-87055-9 (22.0 mg, 0.020 mmol) was added, the reaction continued for 25 minutes while stirring, and then TLC showed complete reaction. The reaction solution was concentrated under reduced pressure, and the residue was purified by HPLC to obtain a target product SMP-87055, weighing 12.0 mg, with a yield of 40.1%. MS (ESI) m/z: 1494 [M+H]⁺.

### Example 87: Preparation of compound SMP-34146

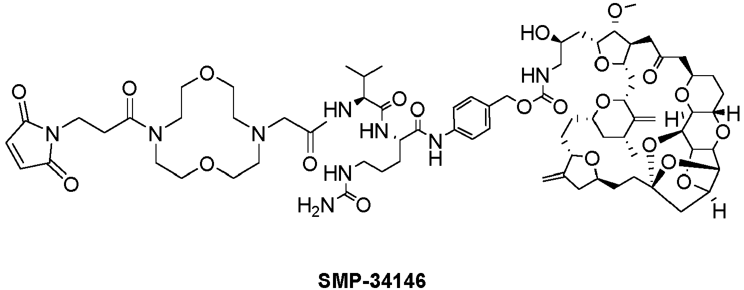

### Step 1: Preparation of compound SMP-34146-2

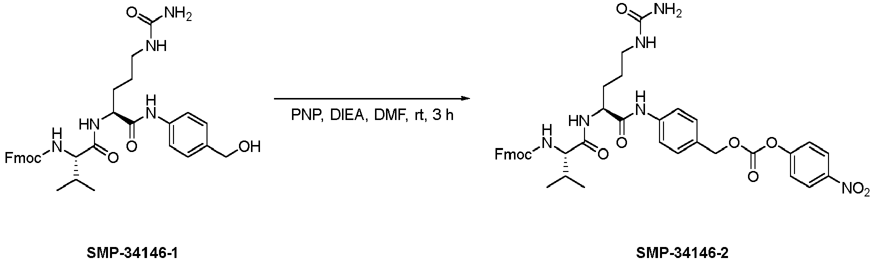

SMP-34146-1 (500.0 mg, 0.83 mmol), p-nitrophenyl chloroformate (251.0 mg, 1.25 mmol), and N,N-dimethylformamide (10 mL) were added to a 50 mL reaction flask. After complete clarification, N,N-diisopropylethylamine (321 mg, 2.49 mmol) was added. A reaction occurred at room temperature for 3 hours while stirring, and then TLC showed complete reaction. The N,N-dimethylformamide was removed by concentration under reduced pressure, and the residue was purified by thin layer chromatography (dichloromethane: methanol=15:1) to obtain a compound SMP-34146-2, weighing 600.0 mg, with a yield of 94%.

MS (ESI) m/z: 767[M+H]⁺.

### Step 2: Preparation of compound SMP-34146-3

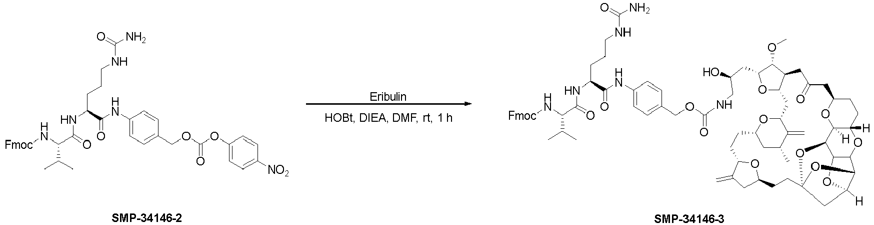

The SMP-34146-2 (300.0 mg, 0.39 mmol), Eribulin (285.0 mg, 0.39 mmol), and N,N-dimethylformamide (5 mL) were added to a 25 mL reaction flask. After complete clarification, 1-hydroxybenzotriazole (58.1 mg, 0.43 mmol) and N,N-diisopropylethylamine (150.9 mg, 1.17 mmol) were sequentially added. A reaction occurred at room temperature for 1 hour while stirring, and then TLC showed complete reaction. The N,N-dimethylformamide was removed by concentration under reduced pressure, and the residue was purified by thin layer chromatography (dichloromethane: methanol=12:1) to obtain a compound SMP-34146-3, weighing 180.0 mg, with a yield of 33.9%.

MS (ESI) m/z: 679[M/2+H]⁺.

### Step 3: Preparation of compound SMP-34146-4

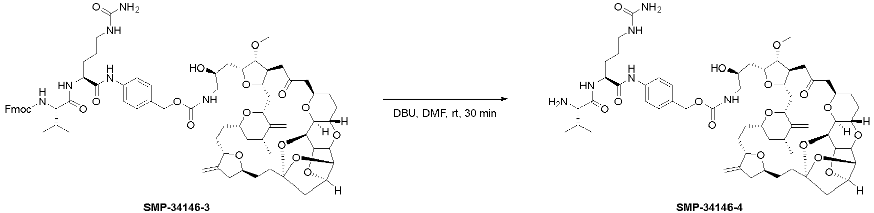

The SMP-34146-3 (176.5 mg, 0.13 mmol), 1,8-diazabicyclo[5.4.0]undec-7-ene (29.1 mg, 0.19 mmol), and N,N-dimethylformamide (3 mL) were added to a 10 mL reaction flask and reacted at room temperature for 30 minutes while stirring, and then LC-MS test showed complete reaction. The reaction solution was purified by HPLC to obtain a compound SMP-34146-4, weighing 90.0 mg, with a yield of 61%.

MS (ESI) m/z: 568[M/2+H]⁺.

### Step 4: Preparation of compound SMP-34146

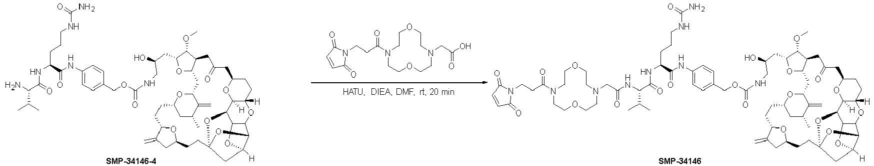

The intermediate SMP-34146-4 (34.1 mg, 0.03 mmol), SMP-93566-4 (11.5 mg, 0.03 mmol), and N,N-dimethylformamide (2 mL) were added to a 10 mL reaction flask. After complete clarification, N,N,N',N'-tetramethyl-O-(7-azabenzotriazol-1-yl)hexafluorophosphourea (11.4 mg, 0.03 mmol) and N,N-diisopropylethylamine (10.3 mg, 0.08 mmol) were sequentially added, a reaction occurred at room temperature for 20 minutes while stirring, and then LC-MS test showed complete reaction. The reaction solution was purified by HPLC to obtain a compound SMP-34146, weighing 33.3 mg, with a yield of 74%.

MS (ESI) m/z: 750 [M/2+H]⁺.

### Example 88: Preparation of compound SMP-02692

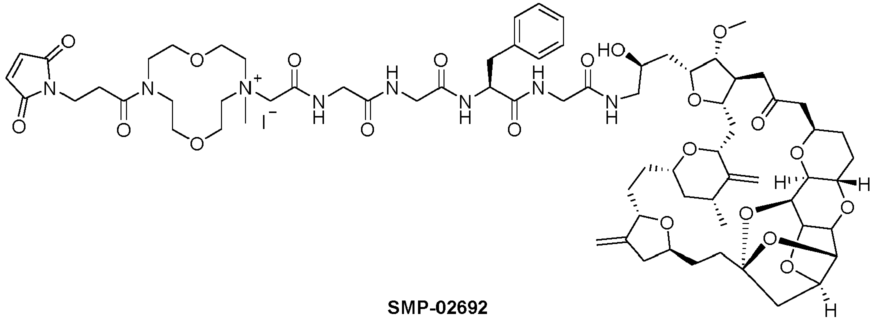

### Step 1: Preparation of compound SMP-02692

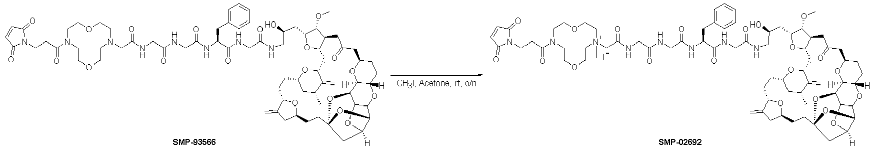

SMP-93566 (10 mg, 0.007 mmol), acetone (1 mL), and iodomethane (2.0 mg, 0.014 mmol) were added to a 10 mL reaction flask and reacted at room temperature overnight while stirring under nitrogen protection. LC-MS test showed complete reaction. A small amount of acetonitrile and water were directly added to the reaction solution. After freeze drying, a compound SMP-02692 was obtained, weighing 10.2 mg, with a yield of 93.7%.

MS (ESI) m/z: 1428[M+H]⁺.

### II. Preparation of targeted linker-drug conjugates

Conjugating method: A disulfide bond on an antibody was conjugated with a drug to generate an antibody-drug conjugate. The antibody was first prepared into a solution with a concentration of 20 mg/mL. 0.1 mL of antibody was pipetted to a 1.5 mL centrifuge tube, and a PBS (pH 7.4)/DTPA solution (293 µL) and a 5 mM TCEP aqueous solution (6.4 µL, one molecule of antibody was 2.4 equivalents) were added. After incubation at 25°C for 2 hours, the disulfide bond in the hinge of the antibody was reduced to a sulfydryl group. Then 10% medical DMSO and 10 mM payload (13.3 µl, one molecule of antibody was 10 eq) were added to the above solution. After reaction at 25°C for 2 hours while shaking (400 rpm), the drug linker was connected to the antibody to obtain the antibody-drug conjugate.

### Example 89 Preparation of antibody-drug conjugates corresponding to Examples 13-88

The compounds prepared in Examples 13-88 above were used as drug link assembly units and conjugated with Her2 antibodies using the aforementioned conjugating method to obtain antibody-drug conjugates of the corresponding examples.

Concentration of an antibody-drug conjugate: The antibody-drug conjugate was put into a 5000K (MiIlipore Co.) ultra-filtration tube and centrifuged at 2°C using a high-speed freezing centrifuge (GENESPEED 1580R) (3500G, for 10 minutes).

The following shows preparation methods for control samples.

### Comparative examples

### I. Preparation of control targeted linker-drug conjugates

### Comparative Example 1: Preparation of compound SMP-81641

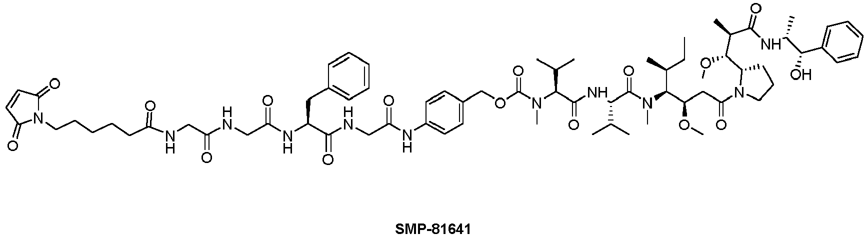

### Step 1: Preparation of compound SMP-81641

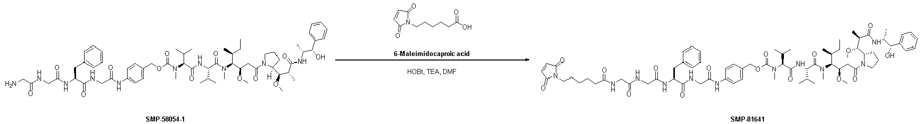

6-maleimidocaproic acid (6.0 mg, 0.020 mmol), the intermediate SMP-58054-1 (21.0 mg, 0.018 mmol), N,N-dimethylformamide (3 mL), 1-hydroxybenzotriazole (2.7 mg, 0.021 mmol), and triethylamine (5.1 mg, 0.050 mmol) were added to a 10 mL reaction flask and reacted at room temperature for 30 minutes while stirring. LC-MS test showed complete reaction. The reaction solution was purified by HPLC to obtain a compound SMP-81641, weighing 8.9 mg, with a yield of 23%.

MS (ESI) m/z: 1378[M+H]⁺.

### Comparative Example 2: Preparation of compound SMP-31896

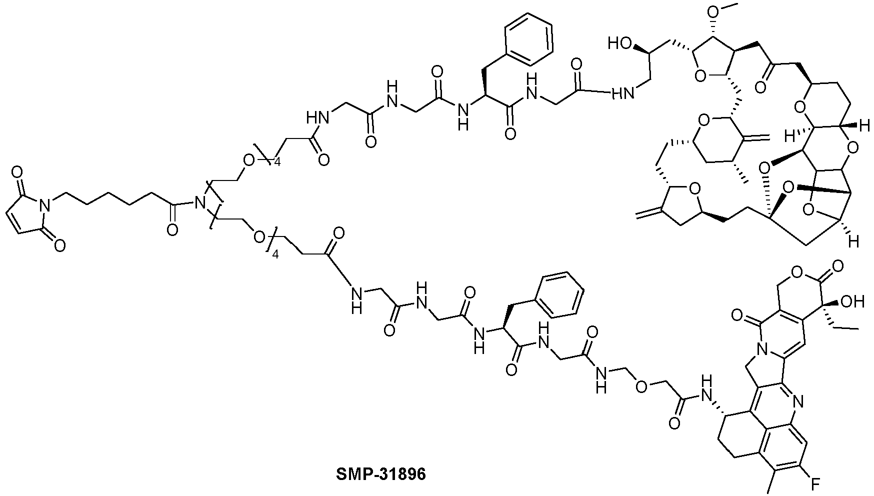

### Step 1: Preparation of compound SMP-31896

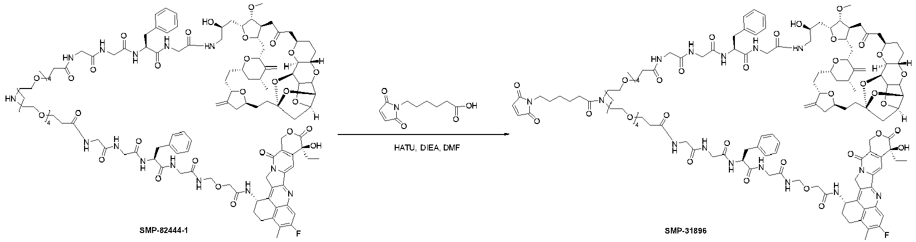

6-maleimidocaproic acid (2.1 mg, 0.006 mmol) and a solvent N,N-dimethylformamide (0.5 mL) were added to a 4 mL reaction flask. After the reaction solution was clarified, N,N,N',N'-tetramethyl-O-(7-azabenzotriazol-1-yl)hexafluorophosphourea (3.8 mg, 0.01 mmol) and N,N-diisopropylethylamine (1.3 mg, 0.01 mmol) are sequentially added. After reaction at room temperature for 5 minutes, the intermediate SMP-82444-1 (14.0 mg, 0.006 mmol) was added, and a reaction occurred for 25 minutes while stirring. TLC showed complete reaction, and the reaction solution was purified by HPLC to obtain a compound SMP-31896, weighing 10.9 mg, with a yield of 72%.

MS (ESI) m/z: 1280 [M/2+H]⁺.

### Comparative Example 3: Preparation of compound SMP-74544

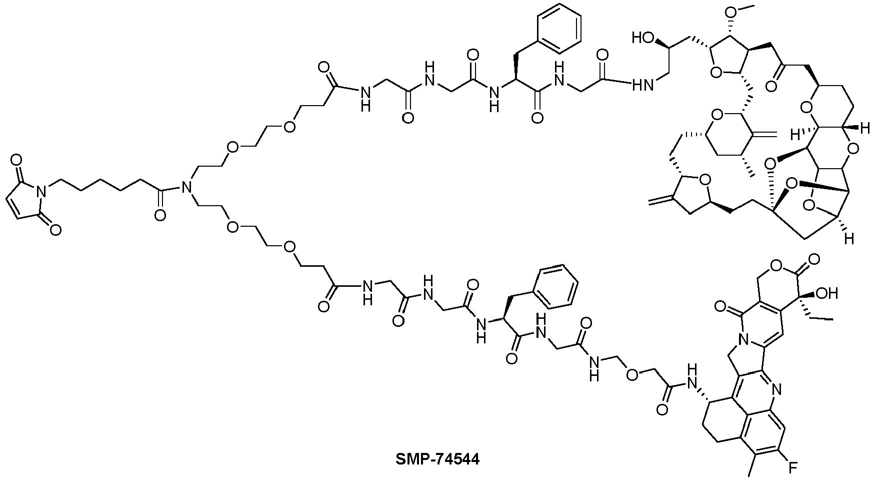

### Step 1: Preparation of compound SMP-74544

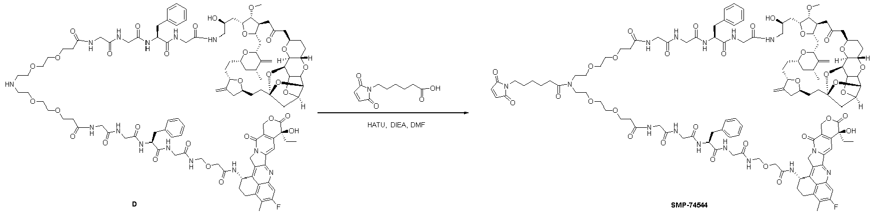

A compound 6-maleimidocaproic acid (13.0 mg, 0.06 mmol) and a solvent N,N-dimethylformamide (3 mL) were added to a 10 mL reaction flask, and 2-(7-azabenzotriazole)-N,N,N',N'-tetramethylurea hexafluorophosphate (22.8 mg, 0.06 mmol) and N,N-diisopropylethylamine (7.6 mg, 0.06 mmol) were sequentially added. After reaction at room temperature for 5 minutes, the intermediate D (131.0 mg, 0.06 mmol) was added, a reaction occurred for 25 minutes while stirring, and then TLC showed complete reaction. Excess N,N-dimethylformamide was removed by concentration under reduced pressure, and the residue was purified by HPLC to obtain a product SMP-74544, weighing 115.0 mg, with a yield of 80%. MS (ESI) m/z: 1192 [M/2+H]⁺.

### Comparative Example 4: Preparation of compound SMP-79786

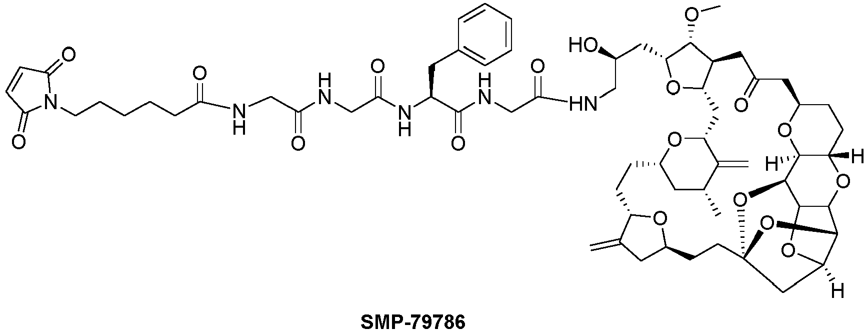

### Step 1: Preparation of compound SMP-79786

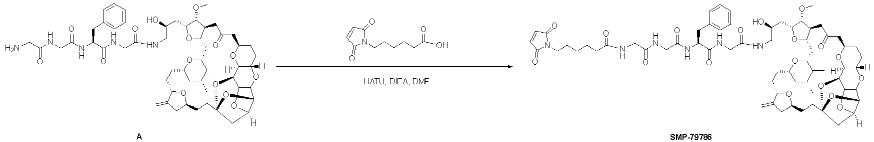

A compound 6-maleimidocaproic acid (13.0 mg, 0.06 mmol) and a solvent N,N-dimethylformamide (5 mL) were added to a 25 mL reaction flask, and 2-(7-azabenzotriazole)-N,N,N',N'-tetramethylurea hexafluorophosphate (22.8 mg, 0.06 mmol) and N,N-diisopropylethylamine (7.7 mg, 0.06 mmol) were sequentially added. After reaction at room temperature for 5 minutes, the intermediate A (62.0 mg, 0.06 mmol) was added, a reaction occurred at room temperature for 25 minutes, and then TLC showed complete reaction. After concentration under reduced pressure, the residue was purified by HPLC to obtain a product SMP-79786, weighing 48.0 mg, with a yield of 65%.

MS (ESI) m/z: 1241 [M+H]⁺.

### Comparative Example 5: Preparation of compound SMP-50206

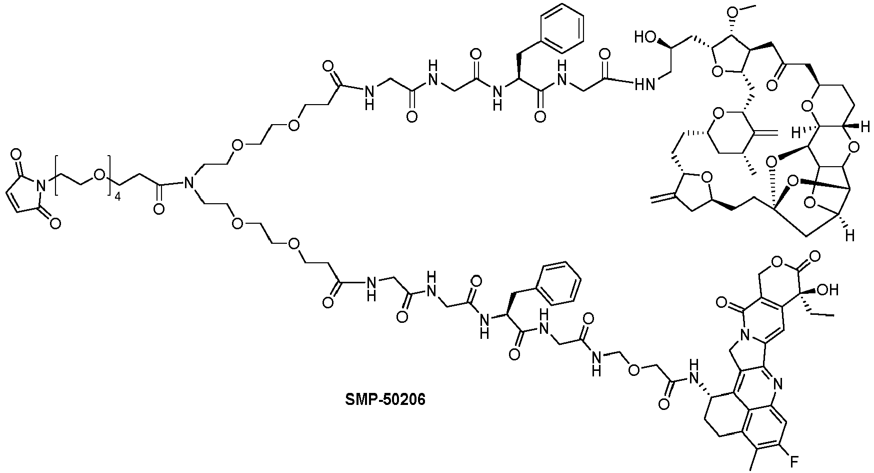

### Step 1: Preparation of compound SMP-50206

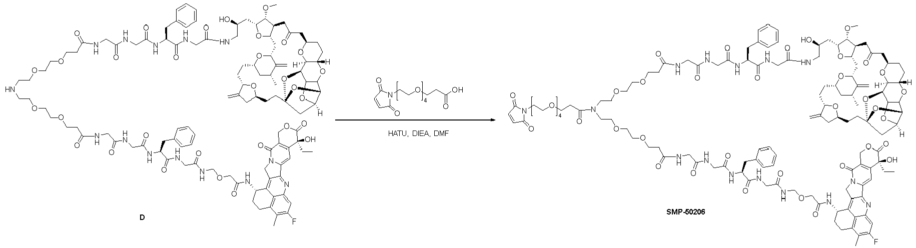

A compound maleimide-tetrapolyethylene glycol-propionic acid (2.0 mg, 0.006 mmol) and a solvent N,N-dimethylformamide (0.5 mL) were added to a 4 mL reaction flask, and 2-(7-azabenzotriazole)-N,N,N',N'-tetramethylurea hexafluorophosphate (2.1 mg, 0.06 mmol) and N,N-diisopropylethylamine (10.3 mg, 0.08 mmol) were sequentially added. After reaction at room temperature for 5 minutes, the intermediate D (13 mg, 0.006 mmol) was added, a reaction occurred for 25 minutes while stirring, and then TLC showed complete reaction. The reaction solution was purified by HPLC to obtain a product SMP-50206, weighing 11.1 mg, with a yield of 72%.

MS (ESI) m/z: 1259 [M/2+H]⁺.

### Comparative Example 6: Preparation of compound SMP-77169

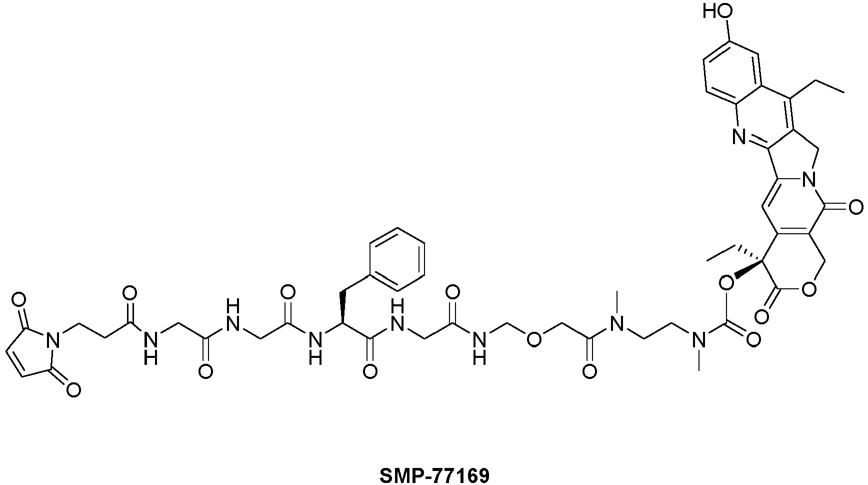

### Step 1: Preparation of compound SMP-77169-1

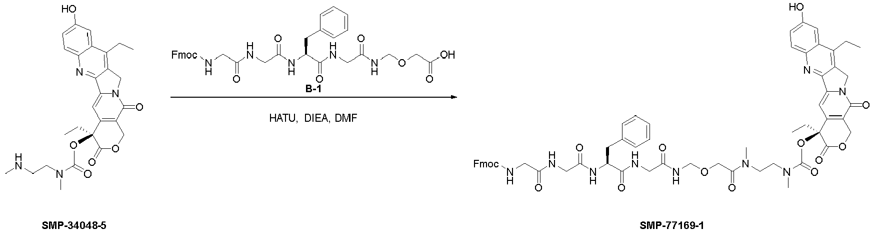

The SMP-34048-5 (50.0 mg, 0.099 mmol), the intermediate B-1 (76.5 mg, 0.119 mmol), N,N-diisopropylethylamine (19.1 mg, 0.148 mmol), and N,N-dimethylformamide (4 mL) were sequentially added to a 10 mL reaction flask and stirred at room temperature, then N,N,N',N'-tetramethyl-O-(7-azabenzotriazol-1-yl)hexafluorophosphourea (45.0 mg, 0.119 mmol) was added, a reaction occurred under this condition for 20 minutes, and then LC-MS showed that most was the molecular weight of a target compound. The reaction solution was concentrated under reduced pressure to obtain a crude product, and the crude product was purified by HPLC to obtain a compound SMP-77169-1, weighing 45.0 mg, with a yield of 40.1%. MS (ESI) m/z: 1134[M+H]⁺.

### Step 2: Preparation of compound SMP-77169-2

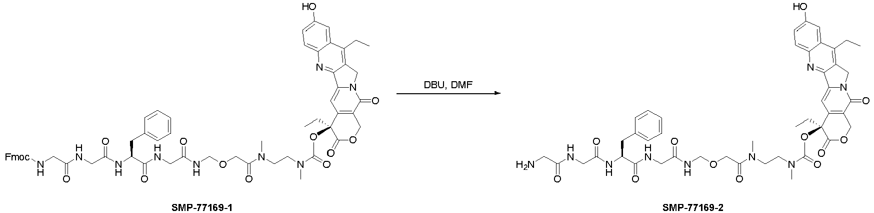

The SMP-77169-1 (45.0 mg, 0.040 mmol), N,N-dimethylformamide (3 mL), and 1,8-diazabicyclo[5.4.0]undec-7-ene (9.0 mg, 0.060 mmol) were sequentially added to a 10 mL reaction flask, a reaction occurred at room temperature for 30 minutes while stirring, and then LC-MS showed that most was the molecular weight of a target compound. The reaction solution was concentrated under reduced pressure to obtain a crude product, and the crude product was purified by HPLC to obtain a compound SMP-77169-2, weighing 25.0 mg, with a yield of 68.5%.

MS (ESI) m/z: 913[M+H]⁺.

### Step 3: Preparation of compound SMP-77169

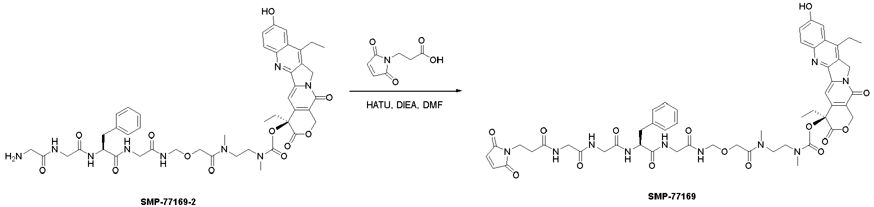

The SMP-77169-2 (25.0 mg, 0.027 mmol), maleimidopropionic acid (5.6 mg, 0.033 mmol), N,N-diisopropylethylamine (5.3 mg, 0.041 mmol), and N,N-dimethylformamide (2 mL) were sequentially added to a 10 mL reaction flask and stirred at room temperature, then N,N,N',N'-tetramethyl-O-(7-azabenzotriazol-1-yl)hexafluorophosphourea (12.5 mg, 0.033 mmol) was added, a reaction occurred under this condition for 20 minutes, and then LC-MS showed that most was the molecular weight of a target compound. The reaction solution was concentrated under reduced pressure to obtain a crude product, and the crude product was purified by HPLC to obtain a compound SMP-77169, weighing 15.0 mg, with a yield of 52.3%. MS (ESI) m/z: 1063[M+H]⁺.

### Comparative Example 7: Preparation of compound SMP-32666

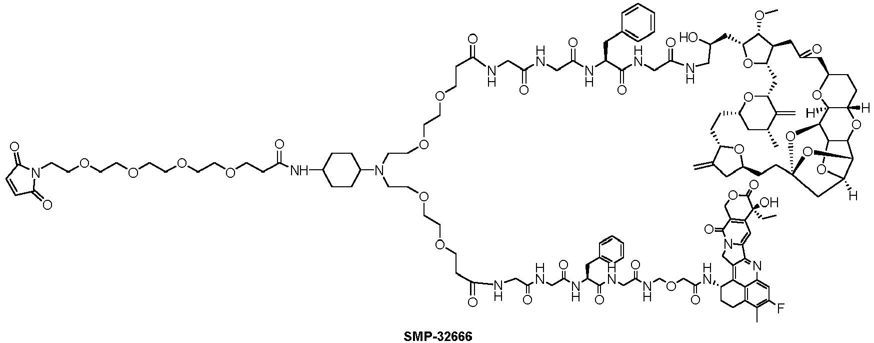

### Step 1: Preparation of compound SMP-32666

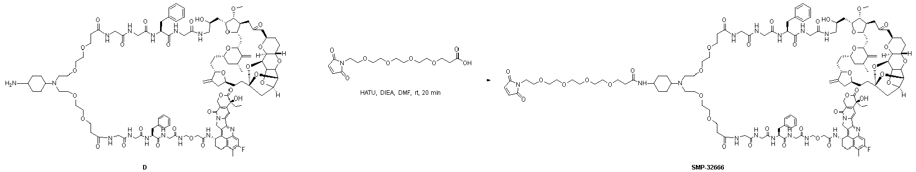

The intermediate C (20 mg, 0.008 mmol), maleimide-tetrapolyethylene glycol-carboxylic acid (2.8 mg, 0.008 mmol), and N,N-dimethylformamide (2 mL) were sequentially added to a 10 mL reaction flask. After complete clarification, N,N,N',N'-tetramethyl-O-(7-azabenzotriazol-1-yl)hexafluorophosphourea (3.0 mg, 0.008 mmol) and N,N-diisopropylethylamine (3.0 mg, 0.024 mmol) were added. A reaction occurred at room temperature for 20 minutes while stirring, and then TLC showed complete reaction. The reaction solution was purified by HPLC to obtain a compound SMP-32666, weighing 10.0 mg, with a yield of 43.7%.

MS (ESI) m/z: 1307[M/2+H]⁺.

### Comparative Example 8: Preparation of compound SMP-88394

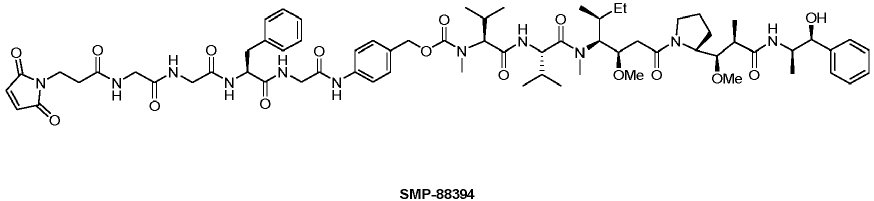

### Step 1: Preparation of compound SMP-88394

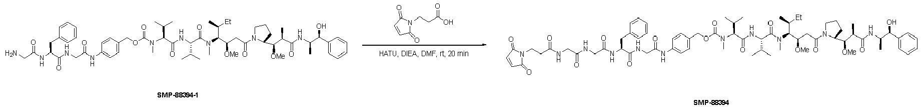

The intermediate SMP-58054-2 (34.0 mg, 0.03 mmol), 3-maleimidopropionic acid (45.1 mg, 0.03 mmol), and N,N-dimethylformamide (2 mL) were added to a 10 mL reaction flask. After complete clarification, N,N,N',N'-tetramethyl-O-(7-azabenzotriazol-1-yl)hexafluorophosphourea (11.4 mg, 0.03 mmol) and N,N-diisopropylethylamine (10.3 mg, 0.081 mmol) were sequentially added, a reaction occurred at room temperature for 20 minutes while stirring, and then TLC showed complete reaction. The reaction solution was purified by HPLC to obtain a compound SMP-88394, weighing 25.0 mg, with a yield of 79%.

MS (ESI) m/z: 668[M/2+H]⁺.

### Comparative Example 9: Preparation of compound SMP-49994

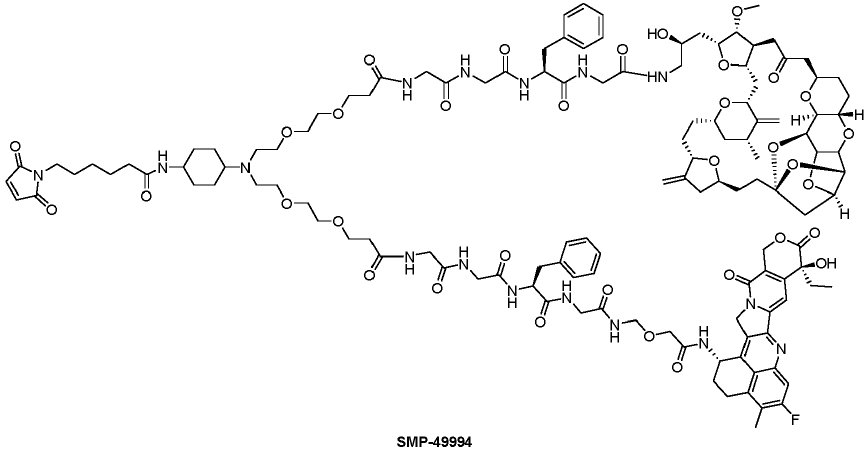

### Step 1: Preparation of compound SMP-49994

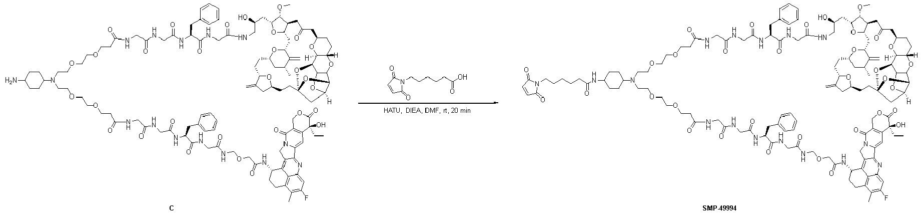

The intermediate C (20 mg, 0.008 mmol), 6-maleimidocaproic acid (1.7 mg, 0.008 mmol), and N,N-dimethylformamide (2mL) were sequentially added to a 10mL reaction flask. After complete clarification, N,N,N',N'-tetramethyl-O-(7-azabenzotriazol-1-yl)hexafluorophosphourea (3.0 mg, 0.008 mmol) and N,N-diisopropylethylamine (3.0 mg, 0.024 mmol) were added. A reaction occurred at room temperature for 20 minutes while stirring, and then TLC showed complete reaction. The reaction solution was purified by HPLC to obtain a compound SMP-49994, weighing 10. 0 mg, with a yield of 46.1%.

MS (ESI) m/z: 1340[M/2+H]⁺.

### Comparative Example 10: Preparation of compound SMP-54731

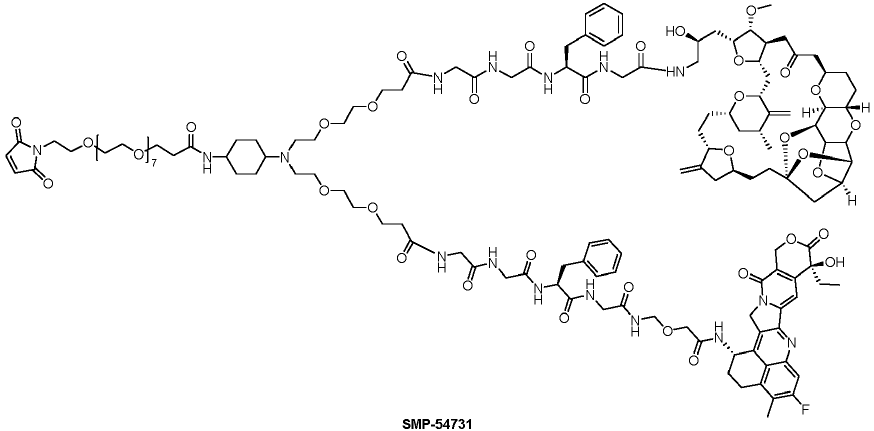

### Step 1: Preparation of compound SMP-54731

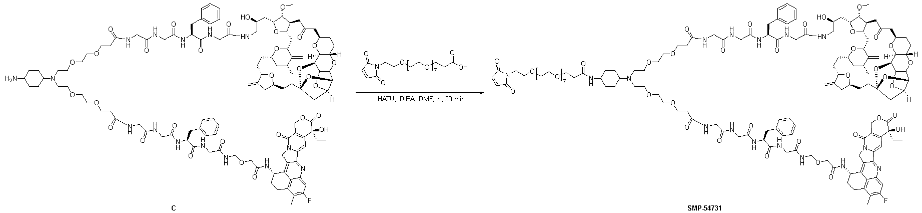

The intermediate C (20 mg, 0.008 mmol), maleimide-octapolyethylene glycol-carboxylic acid (4.2 mg, 0.008 mmol), and N,N-dimethylformamide (2 mL) were sequentially added to a 10 mL reaction flask. After complete clarification, N,N,N',N'-tetramethyl-O-(7-azabenzotriazol-1-yl)hexafluorophosphourea (3.0 mg, 0.008 mmol) and N,N-diisopropylethylamine (3.0 mg, 0.024 mmol) were added, a reaction occurred at room temperature for 20 minutes while stirring, and then TLC showed complete reaction. The reaction solution was purified by HPLC to obtain a compound SMP-54731, weighing 6.0 mg, with a yield of 24.6%.

MS (ESI) m/z: 1395[M/2+H]⁺.

### Comparative Example 11: Preparation of compound SMP-02571

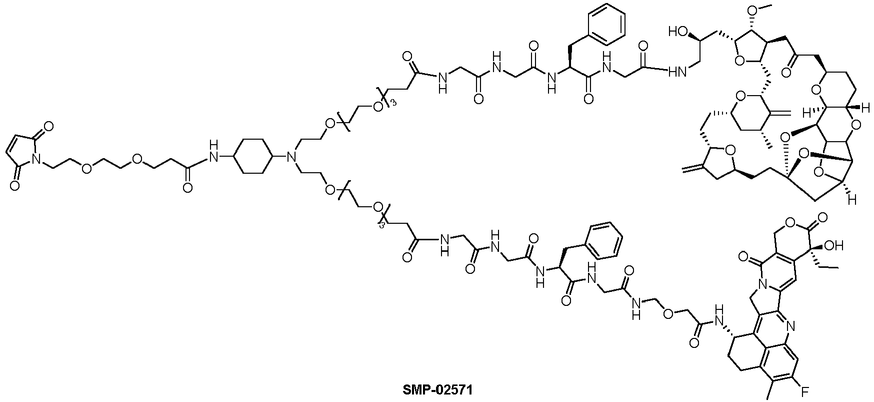

### Step 1: Preparation of compound SMP-02571

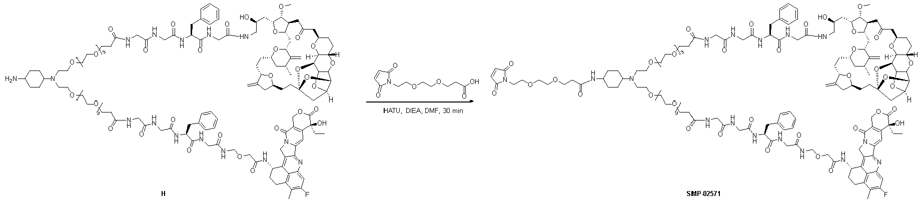

A compound maleimide-dipolyethylene glycol-carboxylic acid (20.0 mg, 0.078 mmol) and a solvent N,N-dimethylformamide (5 mL) were added to a 25 mL reaction flask, and 2-(7-azabenzotriazole)-N,N,N',N'-tetramethylurea hexafluorophosphate (30.0 mg, 0.079 mmol) and N,N-diisopropylethylamine (15.0 mg, 0.12 mmol) were sequentially added. After reaction at room temperature for 5 minutes, the compound H (200.0 mg, 0.081 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-02571, weighing 160.0 mg, with a yield of 74%.

MS (ESI) m/z: 1352 [M/2+H]⁺.

### Comparative Example 12: Preparation of compound SMP-39835

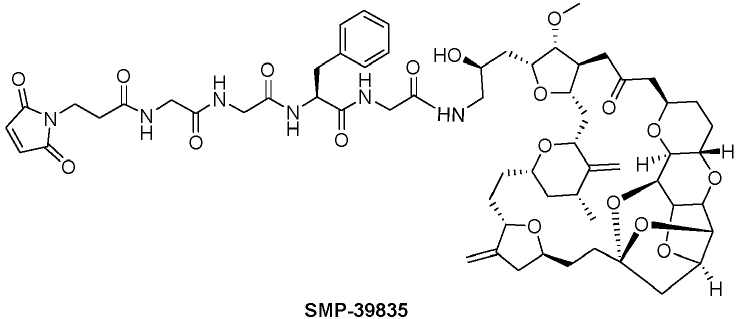

### Step 1: Preparation of compound SMP-39835

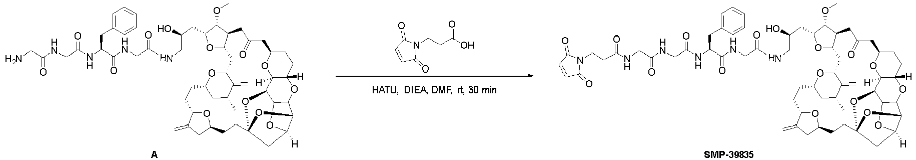

A compound maleimidopropionic acid (17.0 mg, 0.10 mmol) and a solvent N,N-dimethylformamide (5 mL) were added to a 25 mL reaction flask, and 2-(7-azabenzotriazole)-N,N,N',N'-tetramethylurea hexafluorophosphate (38.0 mg, 0.10 mmol) and N,N-diisopropylethylamine (15.0 mg, 0.12 mmol) were sequentially added. After reaction at room temperature for 5 minutes, the compound A (84.0 mg, 0.08 mmol) was added, the reaction continued for 25 minutes while stirring, and then TLC showed complete reaction. Excess N,N-dimethylformamide was removed by concentration under reduced pressure, and the residue was purified by HPLC to obtain a target product SMP-39835, weighing 80.0 mg, with a yield of 83%. MS (ESI) m/z: 1199 [M+H]⁺.

### Comparative Example 13: Preparation of compound SMP-02524

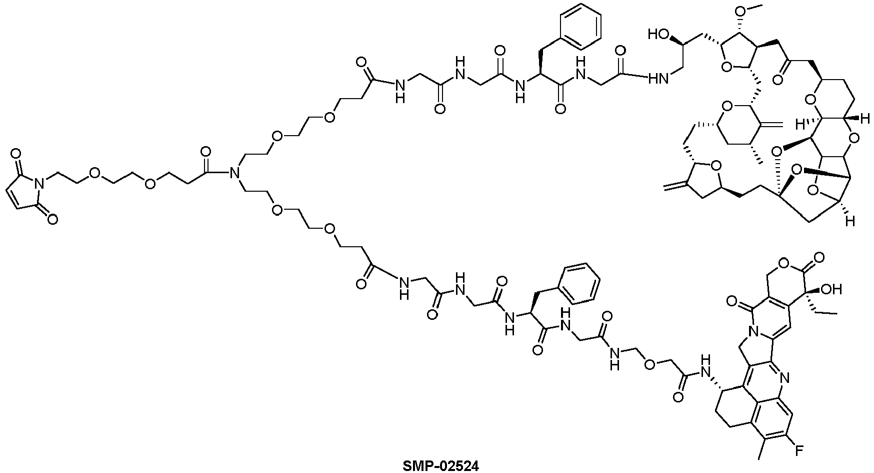

### Step 1: Preparation of compound SMP-02524

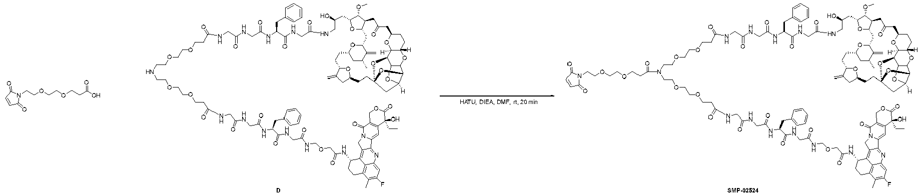

The intermediate D (50.0 mg, 0.023 mmol), maleimide-dipolyethylene glycol-carboxylic acid (6.0 mg, 0.023 mmol), and N,N-dimethylformamide (2 mL) were sequentially added to a 10 mL reaction flask. After complete clarification, N,N,N',N'-tetramethyl-O-(7-azabenzotriazol-1-yl)hexafluorophosphourea (9.0 mg, 0.023 mmol) and N,N-diisopropylethylamine (9.0 mg, 0.069 mmol) were added. A reaction occurred at room temperature for 20 minutes while stirring, and then TLC showed complete reaction. The reaction solution was purified by HPLC to obtain a compound SMP-02524, weighing 35 mg, with a yield of 63.1%.

MS (ESI) m/z: 1215[M/2+H]⁺.

### Comparative Example 14: Preparation of compound DS-8201

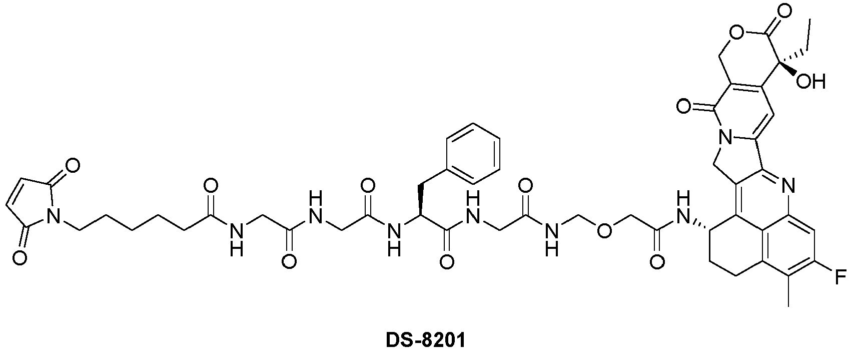

### Step 1: Preparation of compound DS-8201

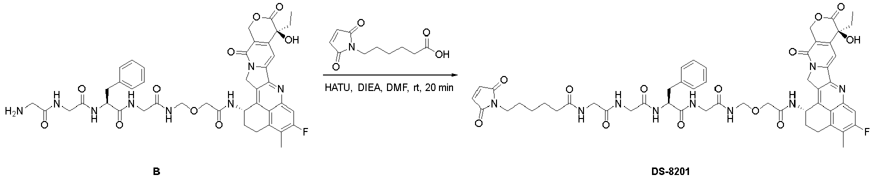

The intermediate B (50.0 mg, 0.06 mmol), maleimidocaproic acid (12.6 mg, 0.06 mmol), and N,N-dimethylformamide (2 mL) were sequentially added to a 10 mL reaction flask. After complete clarification, N,N,N',N'-tetramethyl-O-(7-azabenzotriazol-1-yl)hexafluorophosphourea (22.2 mg, 0.06 mmol) and N,N-diisopropylethylamine (23.2 mg, 0.18 mmol) were sequentially added. A reaction occurred at room temperature for 20 minutes while stirring, and then TLC showed complete reaction. The reaction solution was purified by HPLC to obtain a compound DS-8201, weighing 35 mg, with a yield of 56.4%.

MS (ESI) m/z: 1034[M+H]⁺.

### II. Preparation of control targeted linker-drug conjugates

With reference to the method for preparing targeted linker-drug conjugates in the examples, the compounds prepared in Comparative Examples 1-14 were used as drug link assembly units, which were conjugated with Her2 antibodies using the conjugating method in Example 89 to obtain corresponding control targeted linker-drug conjugates.

Taking antibody-drug conjugates (ADCs) as an example, the following describes effects on targeted linker-drug conjugates that were produced by introducing the structure represented by formula A to linkers of the targeted linker-drug conjugates.

### Experimental Example 1: Test on antibody-drug conjugates

### 1. Test methods for antibody-drug conjugates

Test samples: ADCs prepared in Example 89 of the present invention and ADCs prepared in comparative examples

Polymers (high molecular components) of the antibody-drug conjugates were tested using molecular exclusion high-performance liquid chromatography (SE-HPLC). A Biocore SEC chromatographic column (7.8 × 300 mm, 5 µm) was used, and the mobile phase was 50 mM phosphate containing 300 mM sodium chloride (pH 6.8), with a flow rate of 0.5mL/min. Samples of 50 µg and 10 µl were injected. UV absorption at 280 nm was observed for at least 30 minutes. The polymer content of an antibody-drug conjugate was calculated using an area normalization method.

A drug-antibody conjugating ratio (also known as drug-antibody ratio, abbreviated as DAR) of the antibody-drug conjugate was tested using reversed-phase high-performance liquid chromatography-mass chromatography (RP-HPLC-MS). The ADC sample was diluted with 50 mmol/L Tris buffer (pH 8.0) to 1 mg/ml. A fresh dithiothreitol (DTT) stock solution was added to achieve a final DTT concentration of 50 mmol/L. Incubation was performed at 37°C for 30 minutes. A PLRP-S chromatographic column (1000 Å pores, 2.1 × 50 mm, 5) µm) was used, and the column temperature was 70°C. The flow rate was 0.25 mL/min. 10-20 µg of sample was injected; the mobile phase A was an aqueous solution of 0.1% formic acid (V/V) and 0.025% trifluoroacetic acid (V/V), and the mobile phase B was an acetonitrile solution of 0.1% formic acid (V/V) and 0.025% trifluoroacetic acid (V/V); the running time was 0-3 minutes, where 27% of mobile phase B ran for 3 minutes, and the mobile phase B ran from 27% to 49% within 3-25 minutes, from 49% to 95% within 25-26 minutes, at 95% within 26-31 minutes, from 95% to 27% within 30-31.5 minutes, and at 27% for 31.5-45 minutes. UV absorption was observed at 280 nm. Mass spectrometry drying gas was at a temperature of 350°C and a flow rate of 7.0 L/min, the pressure of an atomizer was 40 psi, capillary voltage was positive 3000V and negative 3000V, and the mass range was 500-1600 m/z. The molecular weight of each mass spectrometry peak (ranging from 20000 to 70000) was calculated by deconvolution and compared with light and heavy chain molecular weights of un-conjugated antibodies to calculate a drug load at each peak, so as to determine the load of a corresponding ultraviolet peak. Area percentages of heavy and light chain ultraviolet peaks were calculated through integration, and a weighted average DAR of the antibody-drug conjugate was calculated by combining the area percentages with the drug load distribution of each peak.

The content of naked antibodies in the antibody-drug conjugate was tested using hydrophobic interaction chromatography (HIC). A Biocore HIC-Butyl chromatographic column (4.6 × 150 mm, 5 µm) was used. The mobile phase A was a 100 mM phosphate aqueous solution containing 2 M ammonium sulfate (pH 7.0), and the mobile phase B was a 100 mM phosphate aqueous solution (pH 7.0): isopropanol=80:20 (V/V). Samples of 50 µg and 10 µl were injected. The mobile phase A at the flow rate of 1 mL/min ran at 100% for 3 minutes. Within 3-25 minutes, the mobile phase B ran from 0% to 100%, while the mobile phase B decreased to 0% at 25.1 and ran to 30 minutes. UV absorption was observed at 280 nm. The naked antibody content of the antibody-drug conjugate was calculated using an area normalization method based on the retention time of a naked antibody peak located by the antibody of the antibody-drug conjugate.

### 2. Test results of antibody-drug conjugates

**Table 1. Test results of antibody-drug conjugates**

| | ADC raw material source | DAR | Polymer content (%) | Naked antibody content (%) |
|---|---|---|---|---|
| | Example 13 | 4.02 | 0.77 | 5.02 |
| | Example 14 | 3.82 | 0.59 | 4.97 |
| | Example 15 | 4.07 | 1.20 | 3.63 |
| | Example 16 | 3.84 | 1.35 | 4.63 |
| | Example 17 | 3.88 | 1.22 | 3.73 |
| | Example 18 | 3.94 | 1.26 | 4.09 |
| | Example 19 | 3.92 | 1.53 | 5.22 |
| | Example 20 | 3.85 | 1.48 | 4.23 |
| | Example 21 | 4.08 | 0.87 | 2.56 |
| | Example 22 | 4.14 | 0.76 | 0.00 |
| | Example 23 | 4.12 | 0.41 | 0.00 |
| | Example 24 | 4.20 | 0.86 | 2.01 |
| Group 1 | Example 25 | 4.17 | 0.56 | 0.00 |
| | Example 26 | 3.92 | 0.70 | 1.26 |
| | Example 27 | 2.98 | 1.34 | 2.12 |
| | Example 28 | 3.84 | 0.56 | 2.76 |
| | Example 39 | 4.30 | 0.90 | 0.00 |
| | Example 40 | 3.78 | 1.29 | 4.27 |
| | Example 41 | 3.84 | 1.52 | 5.05 |
| | Example 42 | 4.12 | 1.25 | 1.06 |
| | Example 62 | 4.04 | 0.00 | 4.17 |
| | Example 80 | 3.93 | 0.89 | 5.04 |
| | Example 81 | 4.02 | 0.56 | 5.33 |
| | Example 82 | 3.83 | 1.67 | 4.56 |
| | Example 83 | 3.96 | 0.56 | 2.23 |
| | Example 85 | 4.04 | 1.43 | 1.98 |
| | Example 86 | 4.20 | 0.97 | 3.45 |
| | Example 87 | 3.85 | 1.12 | 2.11 |
| | Example 88 | 4.03 | 0.71 | 2.35 |
| Group 2 | Example 63 | 3.85 | 0.47 | 5.16 |
| | Example 64 | 4.11 | 1.01 | 2.98 |
| | Example 66 | 4.18 | 0.41 | 1.77 |
| | Example 67 | 4.01 | 0.79 | 2.55 |
| | Example 68 | 3.72 | 0.00 | 3.33 |
| | Example 69 | 4.13 | 0.40 | 2.16 |
| | Example 70 | 4.14 | 0.47 | 4.14 |
| | Example 71 | 4.03 | 0.00 | 3.3 |
| | Example 72 | 4.23 | 0.00 | 2.52 |
| | Example 73 | 4.17 | 1.25 | 0.57 |
| | Example 74 | 4.24 | 1.16 | 0.87 |
| | Example 75 | 3.89 | 0.00 | 4.17 |
| | Example 76 | 3.94 | 1.41 | 3.58 |
| | Example 77 | 3.88 | 0.93 | 5.07 |
| Group 3 | Example 29 | 3.79 | 1.66 | 5.23 |
| | Example 30 | 4.10 | 0.00 | 3.29 |
| | Example 31 | 3.91 | 0.00 | 3.35 |
| | Example 32 | 4.16 | 0.00 | 3.13 |
| | Example 33 | 4.00 | 1.24 | 4.06 |
| | Example 34 | 3.85 | 0.00 | 2.67 |
| | Example 35 | 3.72 | 1.06 | 4.23 |
| | Example 36 | 3.90 | 0.84 | 5.48 |
| | Example 37 | 3.72 | 1.13 | 4.35 |
| | Example 38 | 3.86 | 1.17 | 5.19 |
| | Example 43 | 3.78 | 1.77 | 5.55 |
| | Example 44 | 4.01 | 1.23 | 2.35 |
| | Example 45 | 4.18 | 0.77 | 3.15 |
| | Example 60 | 3.97 | 1.72 | 2.32 |
| | Example 61 | 3.98 | 1.38 | 3.56 |
| | Example 78 | 3.86 | 1.06 | 5.66 |
| Group 4 | Example 46 | 4.14 | 1.77 | 1.71 |
| | Example 47 | 3.81 | 1.11 | 5.16 |
| | Example 48 | 4.17 | 1.07 | 2.89 |
| | Example 49 | 4.19 | 0.00 | 2.11 |
| | Example 50 | 4.08 | 0.00 | 2.47 |
| | Example 51 | 4.02 | 1.12 | 2.49 |
| | Example 52 | 4.18 | 0.00 | 2.94 |
| | Example 53 | 4.18 | 0.00 | 3.15 |
| | Example 54 | 4.22 | 0.00 | 2.79 |
| | Example 55 | 4.16 | 0.00 | 3.29 |
| | Example 56 | 3.86 | 2.04 | 3.27 |
| | Example 57 | 3.84 | 1.52 | 4.21 |
| | Example 58 | 3.84 | 1.23 | 4.23 |
| | Example 59 | 3.83 | 1.18 | 2.57 |
| Group 5 | Comparative Example 1 | 1.03 | 38.31 | 23.50 |
| | Comparative Example 2 | 0.77 | 40.25 | 45.62 |
| | Comparative Example 3 | 0.81 | 50.73 | 60.77 |
| | Comparative Example 4 | 3.01 | 10.73 | 8.01 |
| | Comparative Example 5 | 1.01 | 30.85 | 47.60 |
| | Comparative Example 6 | 2.76 | 13.74 | 9.06 |
| | Comparative Example 7 | 0.86 | 60.31 | 53.82 |
| | Comparative Example 8 | 2.57 | 12.37 | 10.15 |
| | Comparative Example 9 | 0.91 | 70.65 | 57.78 |
| | Comparative Example 10 | 1.13 | 50.68 | 55.46 |
| | Comparative Example 11 | 0.96 | 53.80 | 60.72 |
| | Comparative Example 12 | 2.67 | 13.18 | 7.00 |
| | Comparative Example 13 | 1.01 | 78.50 | 60.37 |
| | Comparative Example 14 | 8.00 | 0.53 | 0.00 |

In Table 1 above, the polymer content represents the percentage of polymers (high molecular components) in ADC that were tested using molecular exclusion high-performance liquid chromatography. Excessive polymer content may cause immunogenic responses. The naked antibody content represents the percentage of naked antibodies in ADC that were tested using hydrophobic interaction chromatography. Excessive naked antibody content may make the naked antibodies become competitive inhibitors of ADC. Excessive polymer content and naked antibody content will affect the binding of ADC and target, thereby affecting therapeutic effects.

The DAR value represents the average number of drug link assembly units conjugated to an antibody. If the DAR value is too low, it cannot achieve effective cell killing, resulting in insufficient efficacy of ADC. As ADC allows, the higher DAR value can achieve more efficient drug delivery. However, as the DAR value increases, the content of hydrophobic payload increases, leading to an increase in the hydrophobicity of ADC, thereby accelerating the clearance of ADC in vivo, shortening the half-life of ADC and increasing toxicity.

The ADCs in the examples of the present invention differ from the ADCs in comparative examples in whether the linker has the structure represented by formula A.

Meanwhile, the increase in the hydrophobicity can be manifested by the polymer content in the conjugating step. When the hydrophobicity of small molecules increases, hydrophobic amino acids inside the antibodies may be exposed due to the influence of small molecules, thereby increasing the hydrophobicity of the entire ADC, accelerating the aggregation of ADC and increasing polymers. The exposure of hydrophobic amino acids inside the antibodies may further change the advanced structure of antibodies, resulting in a decrease in antibody binding activity, thereby reducing the targeting ability of ADC. By introducing the structure of formula A on the linker, the hydrophobicity of small molecules can be reduced, non-covalent binding of hydrophobic payload with antibodies can be prevented, and the exposure of hydrophobic amino acids inside the antibodies can be prevented, thereby reducing the hydrophobicity of the entire ADC. By comparing the data of polymer content, naked antibody content, and DAR value in Table 1, it can be seen that the ADCs in Comparative Examples 1-13 have high polymer content and high naked antibody content, while the corresponding ADCs in the examples of the present invention have low polymer content and low naked antibody content; although the ADC in Comparative Example 14 has low polymer content and naked antibody content, its plasma stability is not as good as 88480 introduced with the structure of formula A, as a proof.

The experimental results show that, by introducing the structure represented by formula A on the linker of ADC, the polymer content and naked antibody content of ADC can be reduced, so that the DAR value of the prepared ADC can be maintained within an appropriate range, thereby improving the stability of ADC, reducing its immunogenicity, reducing competitive inhibition, and improving drug efficacy.

The ADCs prepared in the present invention have low polymer content and naked antibody content, appropriate DAR values, good stability, low immunogenicity, and high purity.

### Experimental Example 2: Test on stability of antibody-drug conjugates

### 1. Experimental method

Test samples: ADCs prepared in examples of the present invention and ADCs prepared in comparative examples

Stability testing method: The stability of the obtained ADCs was tested for 14 days. The ADCs were placed at 40°C in dark. Sampling points were days 0, 7, and 14. The polymer content and DAR value of samples at different sampling points were tested according to the method in Experimental Example 1.

### 2. Experimental results

**Table 2. Stability test results**

| Example | SMP number | Day 0 | | Day 7 | | Day 14 | |
|---|---|---|---|---|---|---|---|
| | | DAR | Polymer content (%) | DAR | Polymer content (%) | DAR | Polymer content (%) |
| Example 13 | SMP-22682 | 4.02 | 0.77 | 4.02 | 0.78 | 4.04 | 0.91 |
| Example 14 | SMP-07861 | 3.82 | 0.59 | 3.83 | 0.63 | 3.84 | 0.72 |
| Example 15 | SMP-61694 | 4.07 | 1.20 | 4.07 | 1.21 | 4.11 | 1.27 |
| Example 16 | SMP-45539 | 3.84 | 1.35 | 3.87 | 1.36 | 3.81 | 1.33 |
| Example 17 | SMP-59034 | 3.88 | 1.22 | 3.90 | 1.23 | 3.83 | 1.22 |
| Example 18 | SMP-2943 8 | 3.94 | 1.26 | 3.97 | 1.28 | 3.92 | 1.23 |
| Example 19 | SMP-49182 | 3.92 | 1.53 | 3.91 | 1.56 | 3.87 | 1.49 |
| Example 20 | SMP-40359 | 3.85 | 1.48 | 3.87 | 1.50 | 3.89 | 1.43 |
| Example 21 | SMP-93566 | 4.08 | 0.87 | 4.06 | 0.90 | 4.10 | 0.88 |
| Example 22 | SMP-88480 | 4.14 | 0.76 | 4.12 | 0.81 | 4.15 | 0.78 |
| Example 23 | SMP-34048 | 4.12 | 0.41 | 4.15 | 0.45 | 4.16 | 0.56 |
| Example 24 | SMP-97962 | 4.20 | 0.86 | 4.18 | 0.87 | 4.18 | 0.86 |
| Example 25 | SMP-00171 | 4.17 | 0.56 | 4.17 | 0.57 | 4.18 | 0.68 |
| Example 26 | SMP-24600 | 3.92 | 0.70 | 3.92 | 0.72 | 3.96 | 0.66 |
| Example 27 | SMP-36297 | 2.98 | 1.34 | 3.00 | 1.39 | 2.99 | 1.38 |
| Example 28 | SMP-12395 | 3.84 | 0.56 | 3.89 | 0.57 | 3.82 | 0.65 |
| Example 29 | SMP-56822 | 3.79 | 1.66 | 3.79 | 1.70 | 3.74 | 1.68 |
| Example 30 | SMP-82891 | 4.10 | 0.00 | 4.12 | 0.05 | 4.13 | 0.04 |
| Example 31 | SMP-51013-A | 3.91 | 0.00 | 3.94 | 0.03 | 3.91 | 0.15 |
| Example 32 | SMP-51013-B | 4.16 | 0.00 | 4.16 | 0.04 | 4.16 | 0.11 |
| Example 33 | SMP-36256 | 4.00 | 1.24 | 4.02 | 1.27 | 4.05 | 1.37 |
| Example 34 | SMP-45582 | 3.85 | 0.00 | 3.82 | 0.03 | 3.80 | 0.14 |
| Example 35 | SMP-01984 | 3.72 | 1.06 | 3.74 | 1.07 | 3.67 | 1.12 |
| Example 36 | SMP-30920 | 3.90 | 0.84 | 3.94 | 0.88 | 3.92 | 0.85 |
| Example 37 | SMP-14324 | 3.72 | 1.13 | 3.70 | 1.18 | 3.71 | 1.12 |
| Example 38 | SMP-61393 | 3.86 | 1.17 | 3.85 | 1.18 | 3.85 | 1.24 |
| Example 39 | SMP-33693 | 4.30 | 0.90 | 4.32 | 0.92 | 4.30 | 0.98 |
| Example 40 | SMP-52573 | 3.78 | 1.29 | 3.75 | 1.31 | 3.74 | 1.41 |
| Example 41 | SMP-42930 | 3.84 | 1.52 | 3.81 | 1.53 | 3.81 | 1.59 |
| Example 42 | SMP-80439 | 4.12 | 1.25 | 4.11 | 1.28 | 4.08 | 1.26 |
| Example 43 | SMP-81404 | 3.78 | 1.77 | 3.81 | 1.79 | 3.74 | 1.86 |
| Example 44 | SMP-64906 | 4.01 | 1.23 | 3.97 | 1.28 | 3.97 | 1.25 |
| Example 45 | SMP-66348 | 4.18 | 0.77 | 4.13 | 0.81 | 4.22 | 0.91 |
| Example 46 | SMP-34421 | 4.14 | 1.77 | 4.10 | 1.79 | 4.19 | 1.76 |
| Example 47 | SMP-53612 | 3.81 | 1.11 | 3.77 | 1.12 | 3.83 | 1.10 |
| Example 48 | SMP-10215 | 4.17 | 1.07 | 4.22 | 1.11 | 4.14 | 1.12 |
| Example 49 | SMP-34710 | 4.19 | 0.00 | 4.23 | 0.04 | 4.15 | 0.04 |
| Example 50 | SMP-44419 | 4.08 | 0.00 | 4.09 | 0.04 | 4.04 | 0.03 |
| Example 51 | SMP-35055 | 4.02 | 1.12 | 4.05 | 1.13 | 4.07 | 1.12 |
| Example 52 | SMP-94170-A | 4.18 | 0.00 | 4.21 | 0.01 | 4.18 | 0.00 |
| Example 53 | SMP-94170-B | 4.18 | 0.00 | 4.17 | 0.04 | 4.13 | 0.09 |
| Example 54 | SMP-46940 | 4.22 | 0.00 | 4.23 | 0.02 | 4.20 | 0.07 |
| Example 55 | SMP-13069 | 4.16 | 0.00 | 4.15 | 0.02 | 4.19 | 0.13 |
| Example 56 | SMP-28945 | 3.86 | 2.04 | 3.84 | 2.08 | 3.82 | 2.17 |
| Example 57 | SMP-28823 | 3.84 | 1.52 | 3.85 | 1.55 | 3.81 | 1.67 |
| Example 58 | SMP-53816 | 3.84 | 1.23 | 3.83 | 1.28 | 3.80 | 1.38 |
| Example 59 | SMP-23135 | 3.83 | 1.18 | 3.83 | 1.22 | 3.78 | 1.27 |
| Example 60 | SMP-21139 | 3.97 | 1.72 | 3.94 | 1.77 | 3.93 | 1.87 |
| Example 61 | SMP-68691 | 3.98 | 1.38 | 3.93 | 1.42 | 4.00 | 1.49 |
| Example 62 | SMP-60226 | 4.04 | 0.00 | 4.05 | 0.01 | 4.05 | -0.04 |
| Example 63 | SMP-37860 | 3.85 | 0.47 | 3.83 | 0.52 | 3.84 | 0.54 |
| Example 64 | SMP-18777 | 4.11 | 1.01 | 4.09 | 1.02 | 4.13 | 1.06 |
| Example 66 | SMP-58054 | 4.18 | 0.41 | 4.19 | 0.45 | 4.19 | 0.38 |
| Example 67 | SMP-25274 | 4.01 | 0.79 | 4.06 | 0.83 | 3.96 | 0.75 |
| Example 68 | SMP-56875 | 3.72 | 0.00 | 3.70 | 0.01 | 3.67 | -0.05 |
| Example 69 | SMP-82444 | 4.13 | 0.40 | 4.08 | 0.42 | 4.09 | 0.53 |
| Example 70 | SMP-50309 | 4.14 | 0.47 | 4.09 | 0.51 | 4.13 | 0.60 |
| Example 71 | SMP-20094 | 4.03 | 0.00 | 4.06 | 0.03 | 4.04 | 0.11 |
| Example 72 | SMP-99559 | 4.23 | 0.00 | 4.18 | 0.03 | 4.26 | 0.15 |
| Example 73 | SMP-59623 | 4.17 | 1.25 | 4.18 | 1.26 | 4.15 | 1.36 |
| Example 74 | SMP-47610 | 4.24 | 1.16 | 4.22 | 1.20 | 4.20 | 1.16 |
| Example 75 | SMP-86467 | 3.89 | 0.00 | 3.93 | 0.03 | 3.87 | 0.15 |
| Example 76 | SMP-76899 | 3.94 | 1.41 | 3.96 | 1.45 | 3.90 | 1.55 |
| Example 77 | SMP-27341 | 3.88 | 0.93 | 3.90 | 0.94 | 3.86 | 0.91 |
| Example 78 | SMP-39205 | 3.86 | 1.06 | 3.86 | 1.11 | 3.91 | 1.20 |
| Example 80 | SMP-68962 | 3.93 | 0.89 | 3.90 | 0.94 | 3.91 | 1.00 |
| Example 81 | SMP-31135 | 4.02 | 0.56 | 4.05 | 0.60 | 3.97 | 0.62 |
| Example 82 | SMP-64791 | 3.83 | 1.67 | 3.87 | 1.68 | 3.87 | 1.70 |
| Example 83 | SMP-26598 | 3.96 | 0.56 | 3.98 | 0.59 | 3.96 | 0.58 |
| Example 85 | SMP-09168 | 4.04 | 1.43 | 4.08 | 1.48 | 4.00 | 1.45 |
| Example 86 | SMP-87055 | 4.20 | 0.97 | 4.23 | 0.99 | 4.16 | 1.11 |
| Example 87 | SMP-34146 | 3.85 | 1.12 | 3.87 | 1.15 | 3.81 | 1.24 |
| Example 88 | SMP-02692 | 4.03 | 0.71 | 4.01 | 0.75 | 4.04 | 0.73 |

In Table 2 above, the stability of the ADCs was evaluated based on their polymer content and DAR value after the ADCs were placed at 40°C in dark for 0, 7, and 14 days. After placed for 14 days, if an ADC can still maintain low polymer content and low DAR value, it indicates that the ADC has excellent placement stability.

From the data in Table 2, it can be seen that the ADCs in the examples of the present invention can maintain low polymer content and low DAR value after placed for 14 days, and their polymer content and DAR value have no significant changes, indicating that the ADCs in the examples of the present invention have excellent placement stability.

The experimental results indicate that the placement stability of an ADC can be improved by introducing the structure represented by formula A on the linker of the ADC.

The ADCs prepared by the present invention not only have low polymer content and low DAR value, but also can maintain the low polymer content and low DAR value within 14 days, and have excellent placement stability.

### Experimental Example 3: Test on plasma stability

### 1. Experimental method

### Test samples: ADCs prepared in examples of the present invention and ADCs prepared in comparative examples

In order to further evaluate the characteristics of ADCs, the plasma stability of ADCs was tested. The plasma stability was tested by the following steps:

### Step 1: Preparation of a stock solution and a working solution

A test sample stock solution was diluted with ultra-pure water into a working solution having a concentration of 2.5 mg/mL. The working solution was prepared as needed and used at room temperature.

### Step 2: Incubation test

Follow the following steps to test plasma samples:
(1) 980 µL of plasma was collected from blank CD-1 mice, monkeys, and humans (experimental group N=2, control group N=1) and pre-incubated at 37°C for 5 minutes;
(2) 20 µL of test compound working solution (or positive control drug (payload) working solution) was added, followed by vortex mixing (final concentration of the test compound was 100 µg/mL, and final concentration of the positive control drug was 1 µM). 50 µL of plasma sample was immediately added into 150 µL of ice precipitant, followed by vortex mixing as a 0 h sample;
(3) The remaining plasma samples were sealed and further incubated at 37°C, and 50 µL of each plasma sample was pipetted into 150 µL of ice precipitant according to preset time points in Table 3, followed by vortex mixing;

**Table 3. Sampling time points**

| Group | Time point |
|---|---|
| Test group | 0 h, 4 h, 8 h, 24 h, 2 d, 4 d, 7 d, 14 d, 21 d |
| Positive control group | 0, 5, 15, 30, 60, 120 min |

(4) The collected samples were stored in a refrigerator at -60oC to -90oC for testing.

### Step 3: Sample analysis

The samples were analyzed using liquid chromatography-tandem mass spectrometry (LC-MS/MS) to measure the quantity of a generated payload, where the quantities of two payloads were measured for dual toxin ADCs respectively.

### 2. Experimental results

Plasma stability is a key factor affecting the druggability of ADCs. Improving the plasma stability of a compound helps to improve its pharmacokinetic properties and efficacy in vivo. According to the quantities of drugs in the drug link assembly units of ADCs, the ADCs can be divided into two classes: (1) single toxin ADCs, referring to ADCs containing 1 drug in a drug link assembly unit; and (2) dual toxin ADCs, referring to ADCs containing 2 drugs in a drug link assembly unit. This experiment tested the stability of single toxin ADCs and dual toxin ADCs in the ADCs of the examples and the ADCs of the comparative examples in the plasma of mice, monkeys, and humans in the plasma of mice, monkeys, and humans.

The ADCs in the examples of the present invention differ from the ADCs in comparative examples in whether the linker has the structure represented by formula A.

### (1) Plasma stability of single toxin ADCs

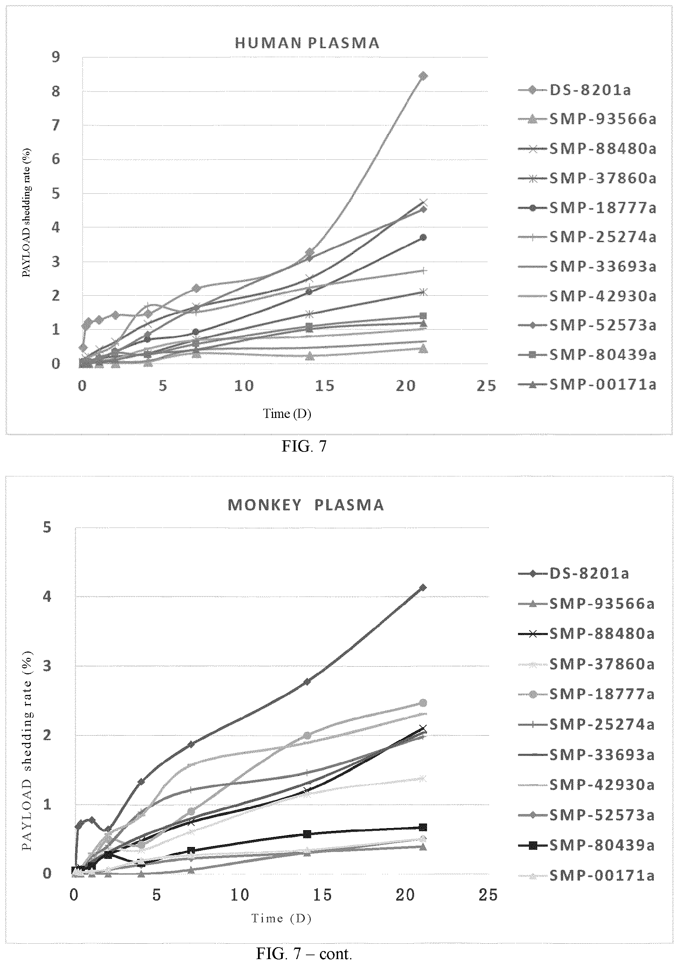

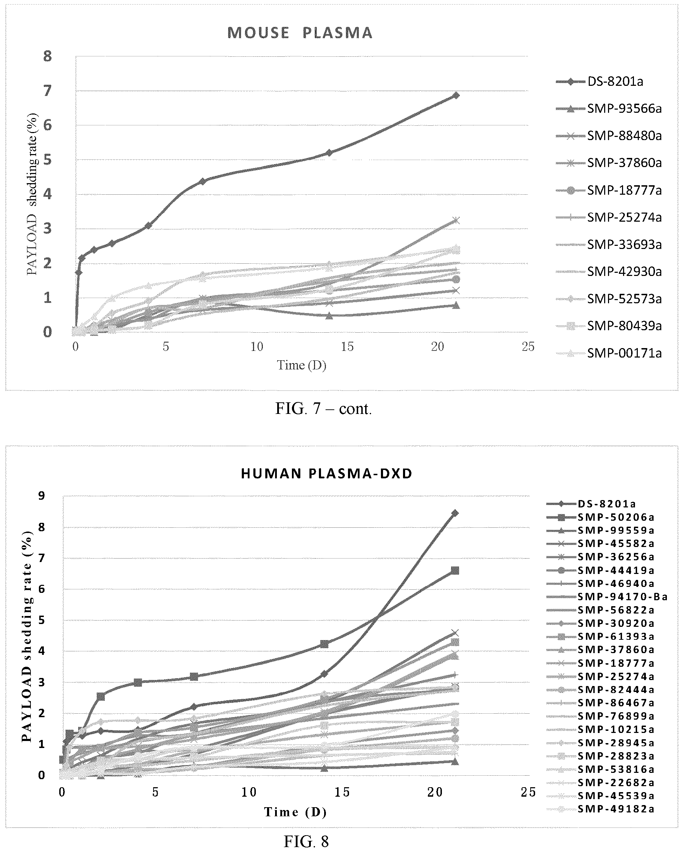

The plasma stability test results of single toxin ADCs are shown in FIG. 7. It can be seen that the single toxin ADC in the comparative example (DS-8201 in Comparative Example 14) has poor stability in the plasma of mice, monkeys, and humans, while the single toxin ADCs in the examples of the present invention have excellent stability in the plasma of mice, monkeys, and humans. Compared with the single toxin ADC in the comparative example (DS-8201 in Comparative Example 14), the stability of the single toxin ADCs in the examples of the present invention in the plasma of mice, monkeys, and humans is significantly improved.

In particular, by comparing the single toxin ADC obtained from SMP-88480 (compound in Example 22) with the single toxin ADC obtained from DS-8201 (in Comparative Example 14), it is found that the difference between the structures of the two only lines in that the linker of the single toxin ADC obtained from SMP-88480 (compound in Example 22) contains the structure represented by formula A. However, the stability of the single toxin ADC obtained from SMP-88480 (Example 22) in the plasma of mice, monkeys, and humans is significantly better than that of the single toxin ADC obtained from DS-8201 (Comparative Example 14), indicating that introducing the structure represented by formula A on the linker of the single toxin ADC can significantly improve the plasma stability of the ADC.

### (2) Plasma stability of dual toxin ADCs

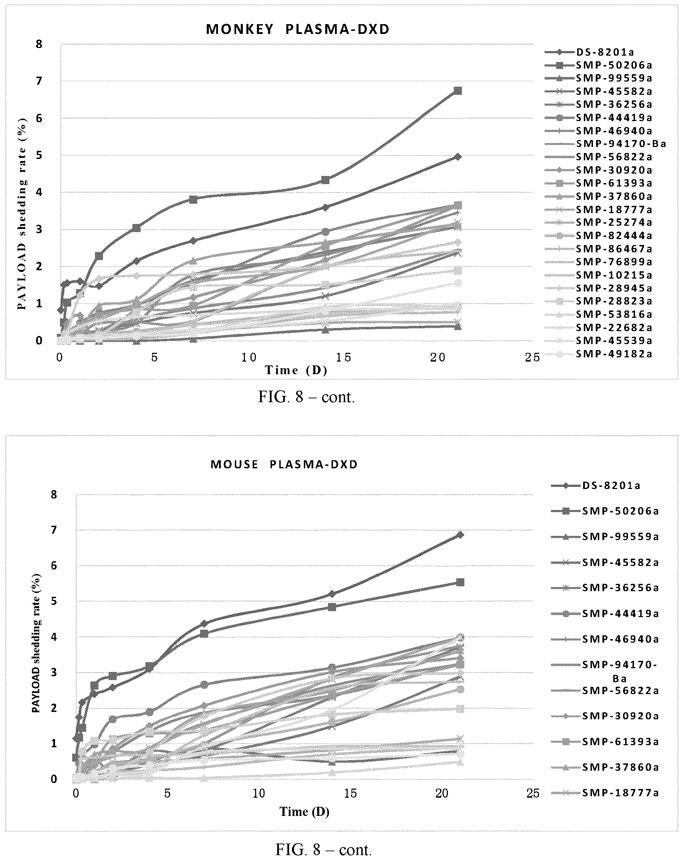

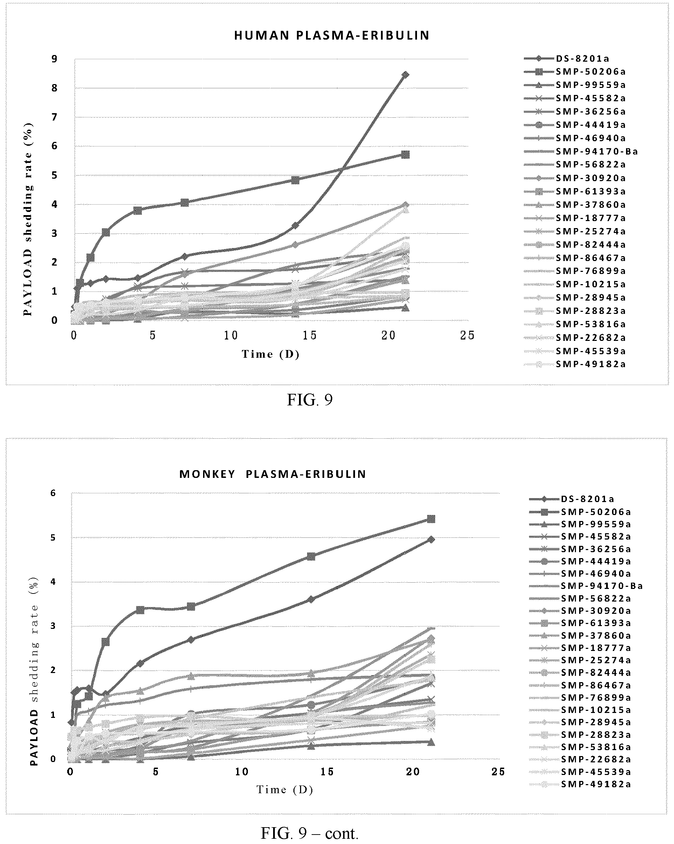

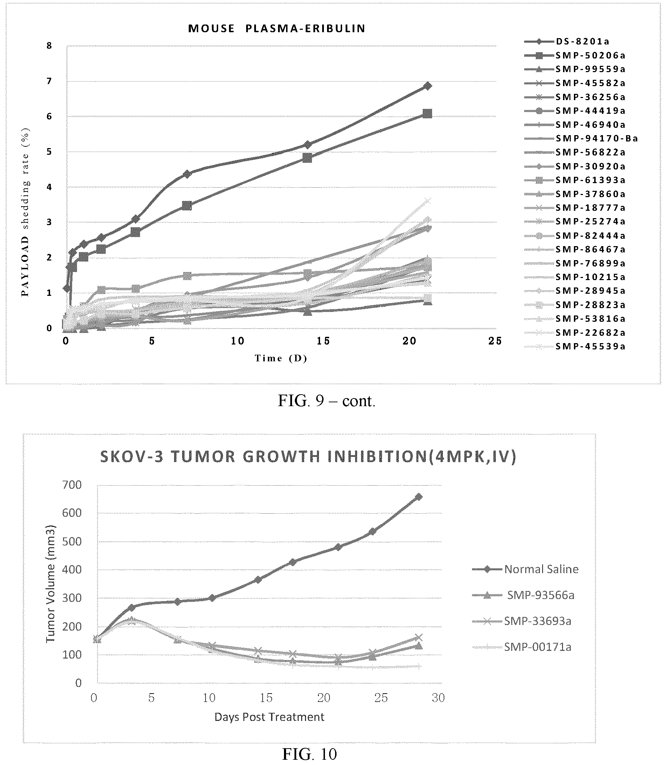

The plasma stability results of Dxd in the dual toxin ADCs are shown in FIG. 8, and the plasma stability results of Eribulin in the dual toxin ADCs are shown in FIG. 9. It can be seen that the dual toxin ADC in the comparative example (SMP-50206 in Comparative Example 5) has poor stability in the plasma of mice, monkeys, and humans, while the dual toxin ADCs in the examples of the present invention have excellent stability in the plasma of mice, monkeys, and humans. Compared with the dual toxin ADC in the comparative example (SMP-50206 in Comparative Example 5), the stability of the dual toxin ADCs in the examples of the present invention in the plasma of mice, monkeys, and humans is significantly improved.

In particular, by comparing the dual toxin ADC obtained from SMP-22682 (Example 13) with the dual toxin ADC obtained from SMP-50206 (Comparative Example 5), it is found that the difference between the structures of the two only lines in that the linker of the dual toxin ADC obtained from SMP-22682 (Example 13) contains the structure represented by formula A. However, the stability of the dual toxin ADC obtained from SMP-22682 (Example 13) in the plasma of mice, monkeys, and humans is significantly better than that of the dual toxin ADC obtained from SMP-50206 (Comparative Example 5), indicating that introducing the structure represented by formula A on the linker of the dual toxin ADC can significantly improve the plasma stability of the ADC.

In addition, according to FIG. 8 and FIG. 9, it can be further seen that the plasma stability of the dual toxin ADCs in the examples of the present invention is even better than that of the single toxin ADC in the comparative example (DS-8201 in Comparative Example 14). It further indicates that introducing the structure represented by formula A on the linker of a dual toxin ADC can significantly improve the plasma stability of the ADC.

The experimental results indicate that the plasma stability of an ADC can be improved by introducing the structure represented by formula A on the linker of the ADC.

The ADCs prepared by the present invention have excellent stability in the plasma of mice, monkeys, and humans, which helps to improve their pharmacokinetic properties and efficacy in vivo.

### Experimental Example 4: Test on in vivo anti-tumor effects

### 1. Experimental method

Test samples: ADCs prepared in examples of the present invention and ADCs prepared in comparative examples

### 1.1 Building of tumor models

Tumor cells (ovarian cancer SKOV-3, gastric cancer NCI-N87, and breast cancer JIMT-1) were cultured in a medium and maintained in a 37°C saturated humidity incubator containing 5% COz, respectively.

Tumor cells in a logarithmic growth phase were collected and re-suspended in a basic medium, and the concentration of cells was adjusted to 4×10⁷/mL. Under sterile conditions, 0.1 mL of cell suspension was inoculated subcutaneously into the right armpit of a mouse at a concentration of 4×10⁶Cells/0.1mL/mouse.

### 1.2 Grouping and administration observation

When the tumor volume reached around 200 mm³, animals were randomly grouped according to the tumor volume, where the difference in tumor volume between the groups was less than 10% of the mean. The grouping day was recorded as Day 0, and administration started according to the body weight of the animals. In the administration period, if the body weight of individual animals decreased by more than 15% compared to that on Day 0 (BWL ≥ 15%), the administration was stopped until the body weight of the animals recovered (BWL< 15%), and then the administration recovered.

During the experiment, the body weight of the animals and the tumor volume were measured 2 times a week, and clinical symptoms of the animals were observed and recorded every day.

### 2. Experimental results

The anti-tumor effects on animal models can relatively directly reflect the therapeutic effects of ADCs. This experiment tested the anti-tumor effects of single toxin ADCs and dual toxin ADCs in the ADCs of the examples and the ADCs of the comparative examples in model animals with ovarian cancer, gastric cancer and breast cancer.

### (1) In vivo anti-tumor effects of single toxin ADCs

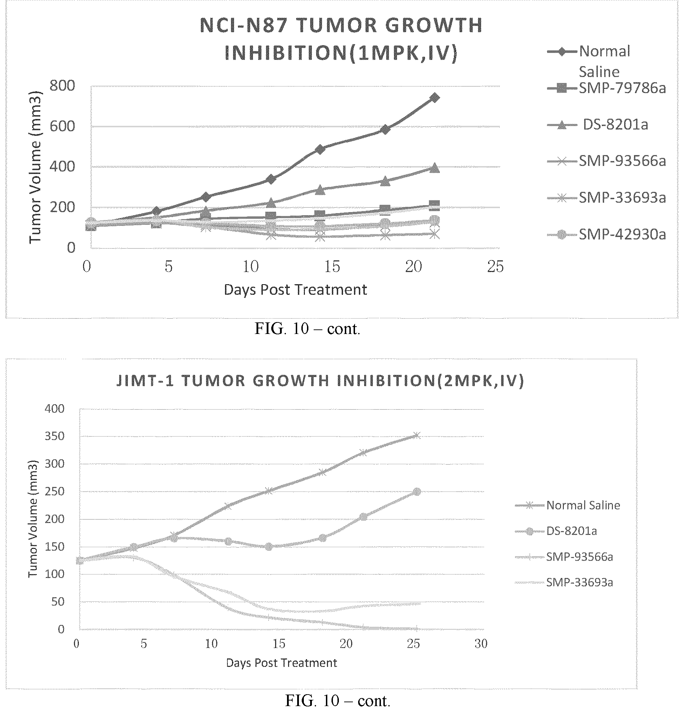

The in vivo anti-tumor effects of single toxin ADCs on the ovarian cancer, gastric cancer and breast cancer are shown in FIG. 10. It can be seen that the single toxin ADCs in the comparative examples (SMP-79786 in Comparative Example 4, and DS-8201 in Comparative Example 14) have poor anti-tumor effects on the gastric cancer and breast cancer, while the single toxin ADCs in the examples of the present invention have excellent anti-tumor effects on the ovarian cancer, gastric cancer and breast cancer. Compared to the single toxin ADCs in the comparative examples (SMP-79786 in Comparative Example 4, and DS-8201 in Comparative Example 14), the anti-tumor effects of the single toxin ADCs in the examples of the present invention on the gastric cancer are significantly improved. Compared to the single toxin ADC in the comparative example (DS-8201 in Comparative Example 14), the anti-tumor effects of the single toxin ADCs in the examples of the present invention on the breast cancer are significantly improved.

In particular, by comparing the single toxin ADC obtained from SMP-52573 (Example 40) with the single toxin ADC obtained from SMP-79786 (Comparative Example 4), it is found that the difference between the structures of the two only lines in that the linker of the single toxin ADC obtained from SMP-52573 (Example 40) contains the structure represented by formula A. However, the in vivo anti-tumor effect of the single toxin ADC obtained from SMP-52573 (Example 40) on the gastric cancer is significantly better than that of the single toxin ADC obtained from SMP-79786 (Comparative Example 4), indicating that introducing the structure represented by formula A on the linker of the single toxin ADC can significantly improve the anti-tumor effect of the ADC.

### (2) In vivo anti-tumor effects of dual toxin ADCs

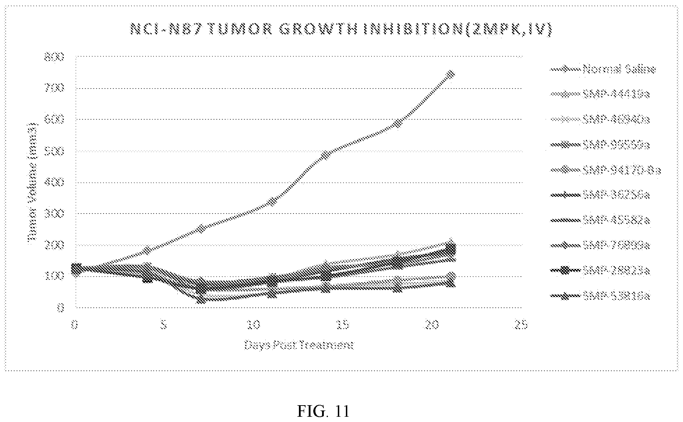

The in vivo anti-tumor effects of dual toxin ADCs on gastric cancer are shown in FIG. 11. It can be seen that the dual toxin ADCs in the examples of the present invention have an excellent anti-tumor effect on the gastric cancer.

In combination with the anti-tumor effect of the single toxin ADC in the comparative example (DS-8201 in Comparative Example 14) on the gastric cancer in FIG. 10, it can be seen that the in vivo anti-tumor effects of the dual toxin ADCs in the examples of the present invention on the gastric cancer are significantly better than that of the single toxin ADC in the comparative example (DS-8201 in Comparative Example 14), indicating that introducing the structure represented by formula A on the linker of a dual toxin ADC can significantly improve the anti-tumor effect of the ADC.

The experimental results indicate that the anti-tumor effect of an ADC can be improved by introducing the structure represented by formula A on the linker of the ADC.

The ADCs prepared in the present invention have excellent in vivo anti-tumor effects and broad clinical application prospects.

In summary, the present invention specifically provides a drug link assembly unit having a specific linker structure. The drug link assembly unit can be connected to an antibody to form a targeted linker-drug conjugate, which can effectively avoid aggregation caused by increasing the antibody-drug ratio of the targeted linker-drug conjugate, improve the stability of the targeted linker-drug conjugate, and ultimately improve its therapeutic effect. The preparation method for the targeted linker-drug conjugate in the present invention is simple and has broad application prospects in the preparation of anti-tumor drugs for prevention and/or treatment.

## Claims

**1.** A drug link assembly unit or a stereoisomer, optical isomer, salt, or deuterated compound thereof, **characterized in that** the drug link assembly unit consists of a linker and a drug, a linker end can be connected to a targeted linker, and the targeted linker is a substance capable of targeted binding to a lesion site; the linker has a structure represented by formula A, as shown in formula A-1: 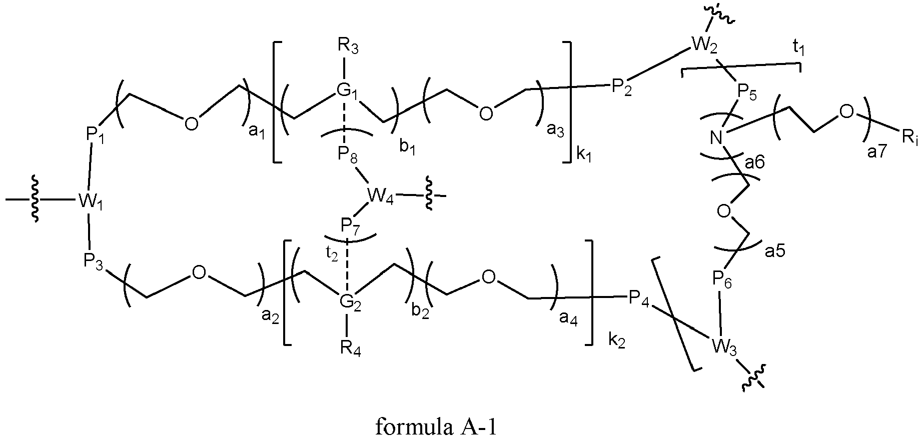
wherein W₁, W₃, and W₄ are independently selected from N, N⁺E⁻R₁, or CR₁; R₁ is selected from hydrogen, C₁₋₁₀ alkyl, or C₁₋₁₀ alkoxy; N⁺ is a monovalent nitrogen cation, and E⁻ is a monovalent anion;
W₂ is selected fromN, N⁺E⁻R₂, or CR₂; R₂ is selected from hydrogen, C₁₋₁₀ alkyl, or C₁₋₁₀ alkoxy;
P₁, P₂, P₃, P₄, P₅, P₆, P₇, and P₈ are independently none or selected from C₁₋₁₀ alkylene that is unsubstituted or substituted by 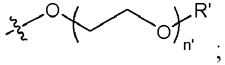 n' is an integer from 1 to 50; R' is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R_{5'})₂; R_{5'} is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
G₁ and G₂ are independently selected from N or N⁺B⁻R₇; N⁺ is a monovalent nitrogen cation, and B⁻ is a monovalent anion; R₇ is selected from C₁₋₁₀ alkyl, C₁₋₁₀ alkoxy, or 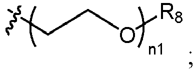 n1 is an integer from 1 to 50; R₈ is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₉)₂; R₈ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
R₃ and R₄ are independently selected from hydrogen, C₁₋₁₀ alkyl, C₁₋₁₀ alkoxy, or 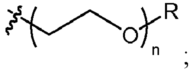 n is an integer from 1 to 50; R is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₅)₂; R₅ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl; or R₃ and R₄ are connected into 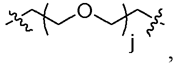 and j is an integer selected from 0 to 10;
t₁ is selected from 0 or 1;
------ represents none or a covalent bond; when ------ represents none, t₂ is 0; when ------ represents the covalent bond, t₂ is 1;
t₁ and t₂ are not simultaneously 1;
k₁ and k₂ are integers independently selected from 0 to 10;
a₁, a₂, a₃, a₄, a₅, and a₆ are integers independently selected from 0 to 10;
a₇ is an integer selected from 1 to 50;
Rᵢ is selected from hydrogen or C₁₋₁₀ alkyl; and
b₁ and b₂ are integers independently selected from 0 to 10.

**2.** A drug link assembly unit or a stereoisomer, optical isomer, salt, or deuterated compound thereof, **characterized in that** the drug link assembly unit consists of a linker and a drug, a linker end can be connected to a targeted linker, and the targeted linker is a substance capable of targeted binding to a lesion site; the linker has a structure represented by formula A, as shown in formula A-1: 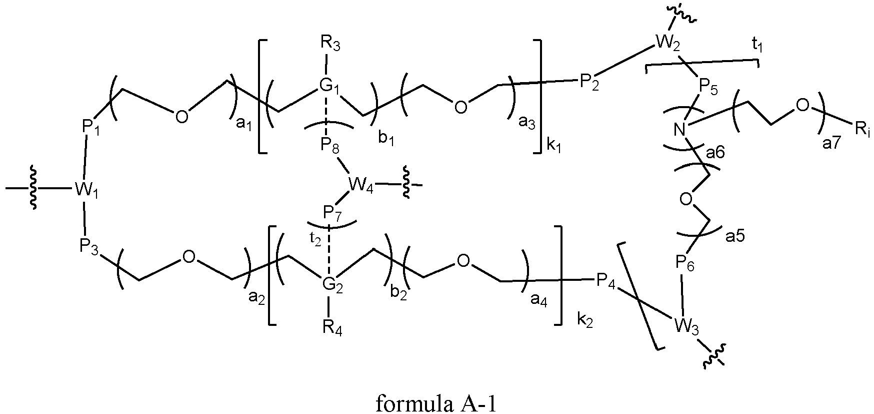
wherein W₁, W₃, and W₄ are independently selected from N, N⁺E⁻R₁, or CR₁; R₁ is selected from hydrogen, C₁₋₁₀ alkyl, or C₁₋₁₀ alkoxy; N⁺ is a monovalent nitrogen cation, and E⁻ is a monovalent anion;
W₂ is selected fromN, N⁺E⁻R₂, or CR₂; R₂ is selected from hydrogen, C₁₋₁₀ alkyl, or C₁₋₁₀ alkoxy;
P₁, P₂, P₃, P₄, P₅, P₆, P₇, and P₈ are independently none or selected from C₁₋₁₀ alkylene that is unsubstituted or substituted by 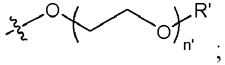 n' is an integer from 1 to 50; R' is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R_{5'})₂; R_{5'} is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
G₁ and G₂ are independently selected from N or N⁺B⁻R₇; N⁺ is a monovalent nitrogen cation, and B⁻ is a monovalent anion; R₇ is selected from C₁₋₁₀ alkyl, C₁₋₁₀ alkoxy, or 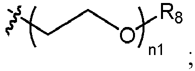 n1 is an integer from 1 to 50; R₈ is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₉)₂; R₈ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
R₃ and R₄ are independently selected from hydrogen, C₁₋₁₀ alkyl, C₁₋₁₀ alkoxy, or 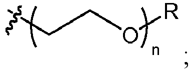 n is an integer from 1 to 50; R is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₅)₂; R₅ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl; or R₃ and R₄ are linked into 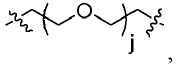 and j is an integer selected from 0 to 10;
t₁ is selected from 0 or 1;
------ represents none or a covalent bond; when ------ represents none, t₂ is 0; when ------ represents the covalent bond, t₂ is 1;
t₁ and t₂ are not simultaneously 1;
k₁ and k₂ are integers independently selected from 0 to 10;
a₁, a₂, a₃, a₄, a₅, and a₆ are integers independently selected from 0 to 10;
a₇ is an integer selected from 1 to 50;
Rᵢ is selected from hydrogen or C₁₋₁₀ alkyl;
b₁ and b₂ are integers independently selected from 0 to 10;
when W₁ is N, W₂ is N, a₁, a₂, a₃, a₄, b₁, b₂, t₁, and t₂ are simultaneously 0,represents none, and P₂ and P₄ are none, P_{1 i}s not C₁₋₂ alkylene, and P₃ is not C₁₋₂ alkylene; and
when W₁ is N, W₂ is N, W₃ is N, a₁, a₂, a₃, a₄, b₁, b₂, a₅, a₆, and t₂ are simultaneously 0, ------ represents none, and P₂ and P₄ are none, P₁ is not C₁₋₂ alkylene, P₃ is not C₁₋₂ alkylene, P₅ is not C₁₋₂ alkylene, and P₆ is not C₁₋₂ alkylene.

**3.** The drug link assembly unit or the stereoisomer, optical isomer, salt, or deuterated compound thereof according to claim 2, **characterized in that** the structure represented by formula A is as shown in formula B-1: 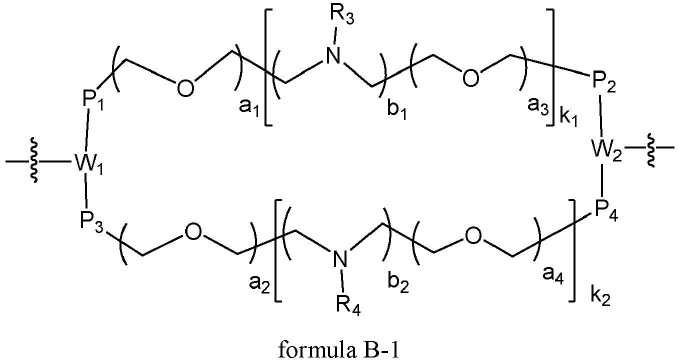
wherein W₁ is selected from N, N⁺E⁻R₁, or CR₁; R₁ is selected from hydrogen, C₁₋₁₀ alkyl, or C₁₋₁₀ alkoxy; N⁺ is a monovalent nitrogen cation, and E⁻ is a monovalent anion;
W₂ is selected fromN or CR₂; R₂ is selected from hydrogen, C₁₋₁₀ alkyl, or C₁₋₁₀ alkoxy;
P₁, P₂, P₃, and P₄ are independently none or selected from C₁₋₁₀ alkylene that is unsubstituted or substituted by 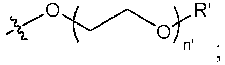 n' is an integer from 1 to 50; R' is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R_{5'})₂; R_{5'} is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
R₃ and R₄ are independently selected from hydrogen, C₁₋₁₀ alkyl, C₁₋₁₀ alkoxy, or 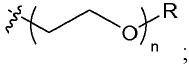 n is an integer from 1 to 50; R is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₅)₂; R₅ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl; or R₃ and R₄ are connected into 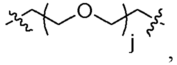 and j is an integer selected from 0 to 10;
k₁ and k₂ are integers independently selected from 0 to 10;
a₁, a₂, a₃, and a₄ are integers independently selected from 0 to 10;
b₁ and b₂ are integers independently selected from 0 to 10; and
when W₁ is N, W₂ is N, a₁, a₂, a₃, a₄, b₁, and b₂ are simultaneously 0, and P₂ and P₄ are none, P₁ is not none or C₁₋₂ alkylene, and P₃ is not none or C₁₋₂ alkylene.

**4.** The drug link assembly unit or the stereoisomer, optical isomer, salt, or deuterated compound thereof according to claim 3, **characterized in that** the structure represented by formula A is as shown in formula B-2: 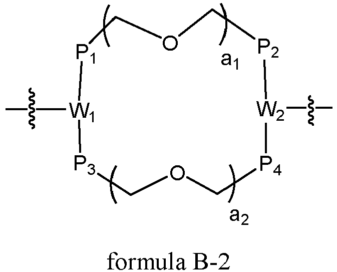
wherein W₁ is selected fromN or CR₁, and W₂ is selected fromN or CR₂; R₁ is hydrogen or C₁₋₃ alkyl, and R₂ is hydrogen or C₁₋₃ alkyl;
P₁, P₂, P₃, and P₄ are independently selected from none or C₁₋₂alkylene;
a₁ and a₂ are integers independently selected from 1 to 6;
preferably, W₁ is selected from N or CR₁, and W₂ is selected from N or CR₂; R₁ is hydrogen, and R₂ is hydrogen;
P₁, P₂, P₃, and P₄ are independently selected from none or methylene; and
a₁ and a₂ are integers independently selected from 1 to 4.

**5.** The drug link assembly unit or the stereoisomer, optical isomer, salt, or deuterated compound thereof according to claim 3, **characterized in that** the structure represented by formula A is as shown in formula B-3: 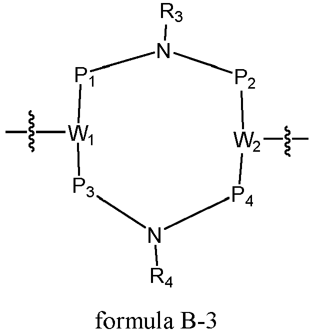
wherein W₁ is selected fromN or CR₁, and W₂ is selected fromN or CR₂; R₁ is hydrogen or C₁₋₃ alkyl, and R₂ is hydrogen or C₁₋₃ alkyl;
P₁, P₂, P₃, and P₄ are independently selected from C₁₋₃ alkylene;
R₃ and R₄ are independently selected from hydrogen, C₁₋₃ alkyl, C₁₋₃ alkoxy, or 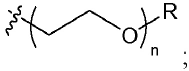 n is an integer from 1 to 30, and R is selected from hydrogen or methyl; or R₃ and R₄ are connected into 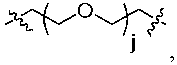 and j is an integer selected from 0 to 5;
preferably, W₁ is selected from N, and W₂ is selected from N;
P₁, P₂, P₃, and P₄ are independently selected from C₂ alkylene; and
R₃ and R₄ are independently selected from 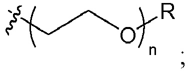 n is an integer from 1 to 30, and R is selected from hydrogen or methyl; or R₃ and R₄ are connected into 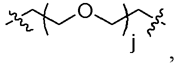 and j is 1.

**6.** The drug link assembly unit or the stereoisomer, optical isomer, salt, or deuterated compound thereof according to claim 2, **characterized in that** the structure represented by formula A is as shown in formula B-4: 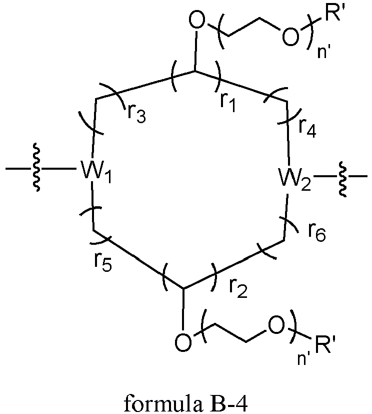
wherein W₁ is selected from N or N⁺E⁻R₁, and W₂ is selected from N or N⁺E⁻R₂; R₁ is selected from C₁₋₃ alkyl, R₂ is selected from C₁₋₃ alkyl, N⁺ is a monovalent nitrogen cation, and E⁻ is a monovalent anion;
r₃, r₄, r₅, and r₆ are integers independently selected from 1 to 3;
r₁ and r₂ are independently selected from 0 or 1;
when W₁ is N and W₂ is N, r₁ and r₂ are not simultaneously 0;
n' is an integer from 1 to 40, and R' is selected from hydrogen or methyl;
preferably, W₁ is selected from N or N⁺E⁻R₁, and W₂ is selected from N or N⁺E⁻R₂; R₁ is methyl, R₂ is methyl, N⁺ is a monovalent nitrogen cation, and E⁻ is a monovalent anion;
r₃, r₄, r₅, and r₆ are independently selected from 1;
r₁ and r₂ are independently selected from 0 or 1;
when W₁ is N and W₂ is N, r1 and r2 are not simultaneously 0; and
n' is an integer from 1 to 30, and R' is selected from hydrogen or methyl.

**7.** The drug link assembly unit or the stereoisomer, optical isomer, salt, or deuterated compound thereof according to claim 2, **characterized in that** the structure represented by formula A is as shown in formula C-1: 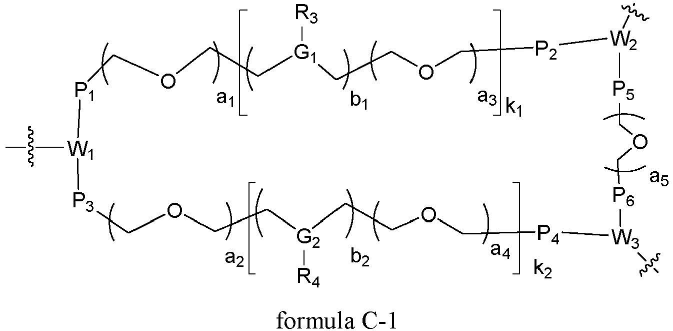
wherein W₁ and W₃ are independently selected from N, N⁺E⁻R₁, or CR₁; R₁ is selected from hydrogen, C₁₋₁₀ alkyl, or C₁₋₁₀ alkoxy; N⁺ is a monovalent nitrogen cation, and E⁻ is a monovalent anion;
W₂ is selected fromN or CR₂; R₂ is selected from hydrogen, C₁₋₁₀ alkyl, or C₁₋₁₀ alkoxy;
P₁, P₂, P₃, P₄, P₅, and P₆ are independently none or selected from C₁₋₁₀ alkylene that is unsubstituted or substituted by 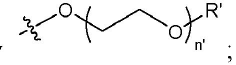 n' is an integer from 1 to 50; R' is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R_{5'})₂; R_{5'} is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
G₁ and G₂ are independently selected from N or N⁺B⁻R₇; N⁺ is a monovalent nitrogen cation, and B⁻ is a monovalent anion; R₇ is selected from C₁₋₁₀ alkyl, C₁₋₁₀ alkoxy, or 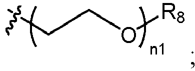 n1 is an integer from 1 to 50; R₈ is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₉)₂; R₈ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
R₃ and R₄ are independently selected from hydrogen, C₁₋₁₀ alkyl, C₁₋₁₀ alkoxy, or 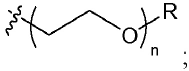 n is an integer from 1 to 50; R is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₅)₂; R₅ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl; or R₃ and R₄ are connected into 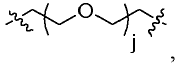 and j is an integer selected from 0 to 10;
k₁ and k₂ are integers independently selected from 0 to 10;
a₁, a₂, a₃, a₄, and a₅ are integers independently selected from 0 to 10; and
b₁ and b₂ are integers independently selected from 0 to 10.

**8.** The drug link assembly unit or the stereoisomer, optical isomer, salt, or deuterated compound thereof according to claim 7, **characterized in that** the structure represented by formula A is as shown in formula C-2: 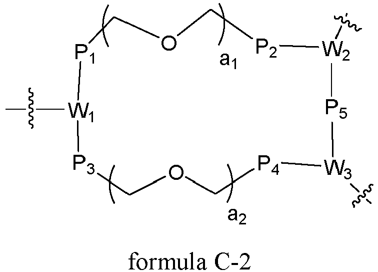
wherein W₁ is N, W₂ is N, and W₃ is N;
P₁, P₂, P₃, P₄, and P₅ are independently selected from C₁₋₃ alkylene;
a₁ and a₂ are integers independently selected from 1 to 6;
preferably, W₁ is N, W₂ is N, and W₃ is N;
P₁, P₂, P₃, P₄, and P₅ are independently selected from C₁₋₂ alkylene; and
a₁ and a₂ are integers independently selected from 1 to 4.

**9.** The drug link assembly unit or the stereoisomer, optical isomer, salt, or deuterated compound thereof according to claim 7, **characterized in that** the structure represented by formula A is as shown in formula C-3: 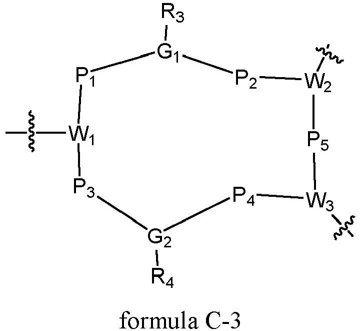
wherein W₁ is N, W₂ is N, and W₃ is N;
P₁, P₂, P₃, P₄, and P₅ are independently selected from C₁₋₃ alkylene;
G₁ and G₂ are independently selected from N or N⁺B⁻R₇; N⁺ is a monovalent nitrogen cation, and B⁻ is a monovalent anion; R₇ is selected from C₁₋₁₀ alkyl, C₁₋₁₀ alkoxy, or 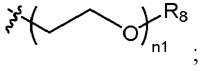 n1 is an integer from 1 to 50; R₈ is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₉)₂; R₉ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
R₃ and R₄ are independently selected from hydrogen, C₁₋₃ alkyl, C₁₋₃ alkoxy, or 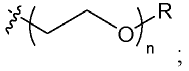 n is an integer from 1 to 30, and R is selected from hydrogen or methyl; or R₃ and R₄ are connected into 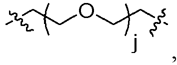 and j is an integer selected from 0 to 5;
preferably, W₁ is N, W₂ is N, and W₃ is N;
P₁, P₂, P₃, P₄, and P₅ are independently selected from C₂ alkylene; and
R₃ and R₄ are independently selected from 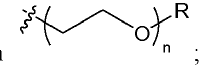 n is an integer from 1 to 30, and R is selected from hydrogen or methyl; or R₃ and R₄ are connected into 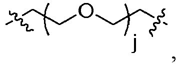 and j is 1.

**10.** The drug link assembly unit or the stereoisomer, optical isomer, salt, or deuterated compound thereof according to claim 2, **characterized in that** the structure represented by formula A is as shown in formula C-4: 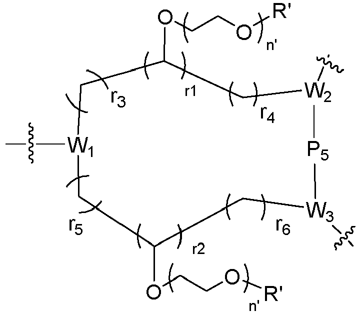 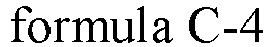
wherein W₁ is N, W₂ is N, and W₃ is N;
r₃, r₄, r₅, and r₆ are integers independently selected from 1 to 3;
P₅ is selected from C₁₋₃ alkylene;
r₁ and r₂ are independently selected from 0 or 1;
n' is an integer from 1 to 40, and R' is selected from hydrogen or methyl;
preferably, the structure represented by formula A is as shown in formula C-5: 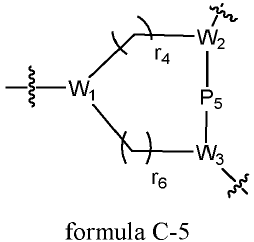
W₁ is N, W₂ is N, and W₃ is N;
r₄ and r₆ are integers independently selected from 1 to 3; and
P₅ is selected from C₁₋₃ alkylene.

**11.** The drug link assembly unit or the stereoisomer, optical isomer, salt, or deuterated compound thereof according to claim 2, **characterized in that** the structure represented by formula A is as shown in formula D-1: 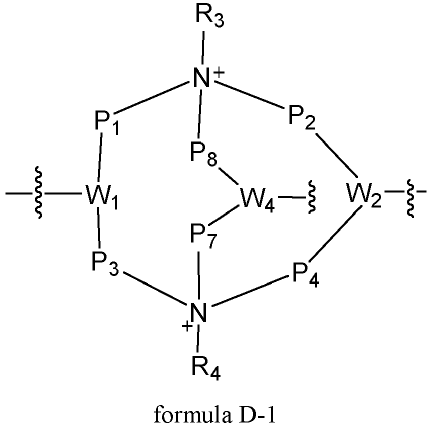
wherein W₁ and W₄ are independently selected from N, N⁺E⁻R₁, or CR₁; R₁ is selected from hydrogen, C₁₋₁₀ alkyl, or C₁₋₁₀ alkoxy; N⁺ is a monovalent nitrogen cation, and E⁻ is a monovalent anion;
W₂ is selected fromN or CR₂; R₂ is selected from hydrogen, C₁₋₁₀ alkyl, or C₁₋₁₀ alkoxy;
P₁, P₂, P₃, P₄, P₇, and P₈ are independently selected from C₁₋₁₀ alkylene that is unsubstituted or substituted by 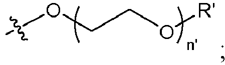 n' is an integer from 1 to 50; R' is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R_{5'})₂; R_{5'} is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
R₃ and R₄ are independently selected from hydrogen, C₁₋₁₀ alkyl, C₁₋₁₀ alkoxy, or 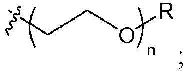 n is an integer from 1 to 50; R is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₅)₂; R₅ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl; or R₃ and R₄ are connected into 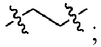
preferably, W₁ is N, W₂ is N, and W₄ is N;
P₁, P₂, P₃, P₄, P₆, and P₇ are independently selected from C₁₋₂ alkylene; and
R₃ and R₄ are independently selected from 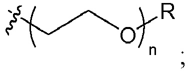 n is an integer from 1 to 30, and R is selected from hydrogen or methyl.

**12.** The drug link assembly unit or the stereoisomer, optical isomer, salt, or deuterated compound thereof according to claim 2, **characterized in that** the structure represented by formula A is as follows: 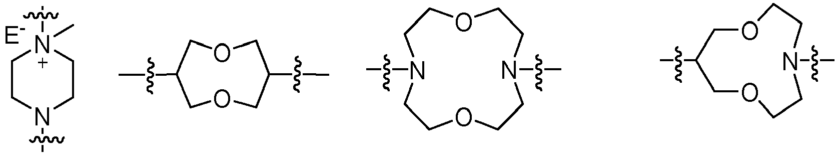 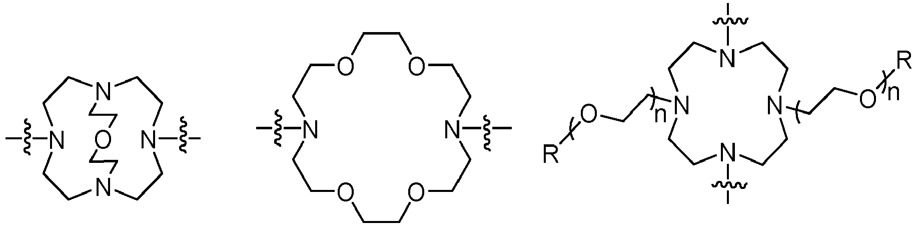 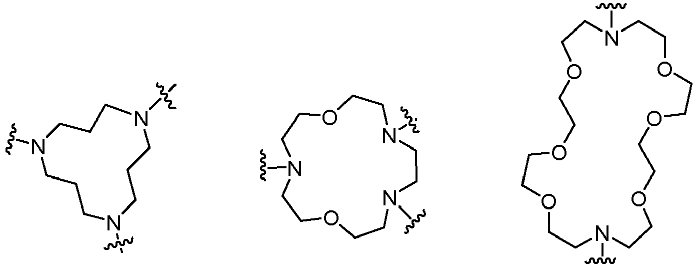 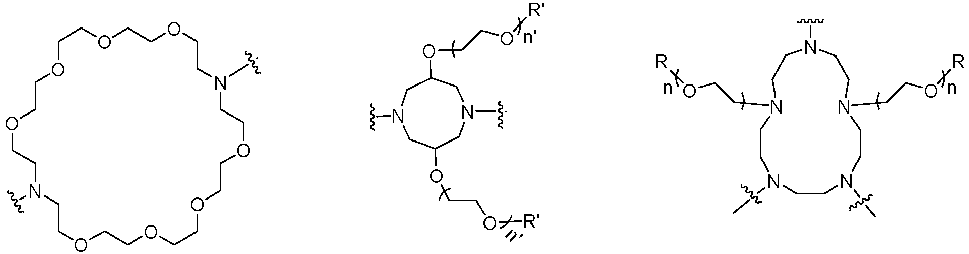 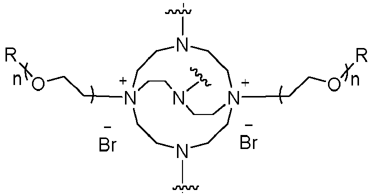 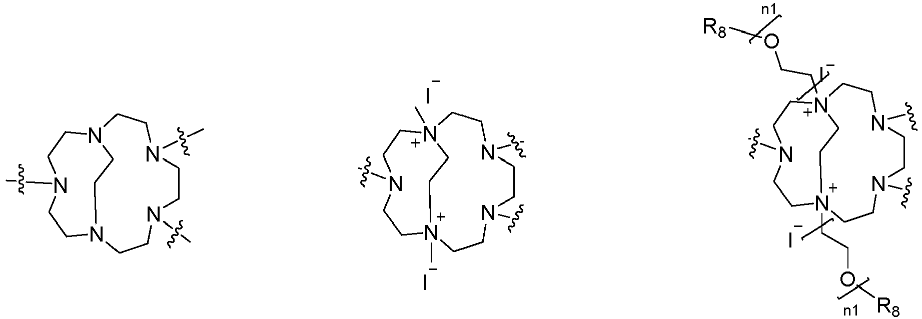 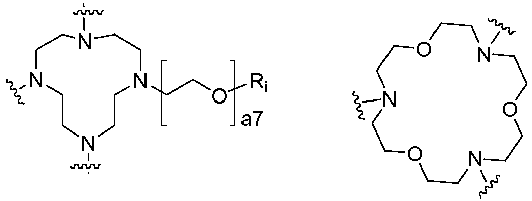 wherein n is an integer selected from 1 to 24, R is hydrogen or methyl, n' is an integer selected from 1 to 24, R' is hydrogen or methyl, a7 is an integer selected from 1 to 24, Rᵢ is hydrogen or methyl, n1 is an integer selected from 1 to 24, R₈ is hydrogen or methyl, and E is halogen.

**13.** A drug link assembly unit or a stereoisomer, optical isomer, salt, or deuterated compound thereof, **characterized in that** the drug link assembly unit consists of a linker and a drug, a linker end can be connected to a targeted linker, and the targeted linker is a substance capable of targeted binding to a lesion site; the linker has a structure represented by formula A, as shown in formula E-0: 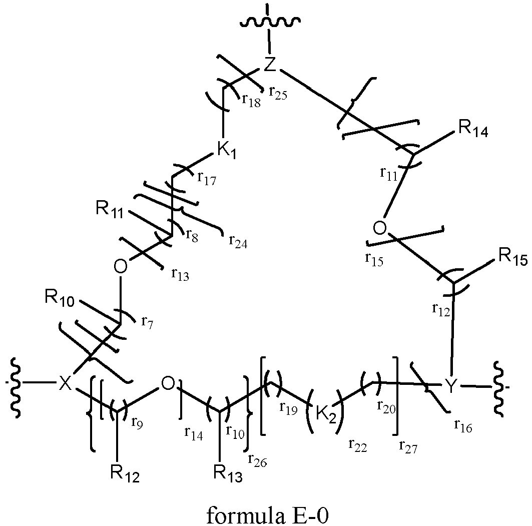
wherein X, Y, and Z are independently selected from N, N⁺B₀⁻R₀, or CH; R₁₀, R₁₁, R₁₂, R₁₃, R₁₄, and R₁₅ are independently selected from hydrogen or 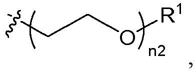 n2 is an integer from 1 to 40, and R¹ is hydrogen or methyl; N⁺ is a monovalent nitrogen cation, B₀⁻is a monovalent anion, and R₀ is C₁₋₅ alkyl;
r₇, r₈, r₉, r₁₀, r₁₃, and r₁₄ are integers independently selected from 1 to 5; r₁₁, r₁₂, and r₁₅ are integers independently selected from 0 to 5; r₁₆ is 0 or 1;
r₁₇, r₁₈, r₁₉, and r₂₀ are integers independently selected from 1 to 3;
r₂₂ is selected from 0 or 1;
r₂₄ is selected from 0 or 1, r₂₅ is selected from 0 or 1, and r₂₄ and r₂₅ are not simultaneously 0;
r₂₆ is selected from 0 or 1, r₂₇ is selected from 0 or 1, and r₂₆ and r₂₇ are not simultaneously 0;
K₁ is selected from NR₁₆, N⁺R₁₆B⁻R₁₈, or CHR₂₀; K₂ is selected from NR₁₇, N⁺R₁₇B⁻R₁₈, or CHR₂₁;
N⁺ is a monovalent nitrogen cation, B⁻ is a monovalent anion, and R₁₈ is selected from C₁₋₃ alkyl, C₁₋₃ alkoxy, or 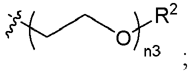 n3 is an integer from 1 to 50, and R² is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₁₉)₂; R₁₉ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
R₁₆ and R₁₇ are independently selected from C₁₋₃ alkyl, C₁₋₃ alkoxy, or 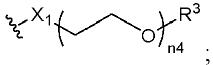 n4 is an integer from 1 to 40, and R³ is selected from hydrogen or methyl; or R₁₆ and R₁₇ are connected into 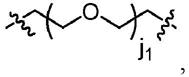 and j₁ is an integer selected from 0 to 5; X₁ is -CO-, -CONH-, or none;
R₂₀ and R₂₁ are independently selected from 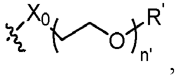 n' is an integer from 1 to 40, R' is selected from hydrogen or methyl, and X₀ is O or none.

**14.** The drug link assembly unit or the stereoisomer, optical isomer, salt, or deuterated compound thereof according to claim 13, **characterized in that** the structure represented by formula A is as shown in formula E-1: 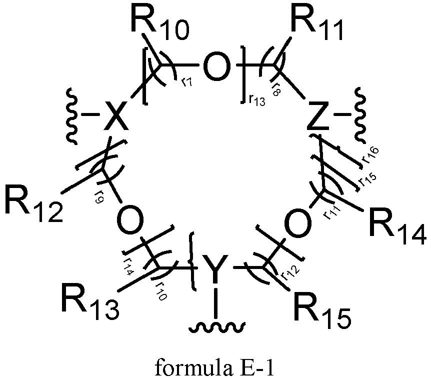
wherein X, Y, and Z are independently selected from N, N⁺B₀⁻R₀, or CH; R₁₀, R₁₁, R₁₂, R₁₃, R₁₄, and R₁₅ are independently selected from hydrogen or 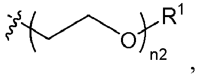 n2 is an integer from 1 to 40, and R¹ is hydrogen or methyl; N⁺ is a monovalent nitrogen cation, B₀⁻is a monovalent anion, and R₀ is C₁₋₅ alkyl;
r₇, r₈, r₉, r₁₀, r₁₃, and r₁₄ are integers independently selected from 1 to 5; r₁₁, r₁₂, and r₁₅ are integers independently selected from 0 to 5; and r₁₆ is 0 or 1.

**15.** The drug link assembly unit or the stereoisomer, optical isomer, salt, or deuterated compound thereof according to claim 14, **characterized in that** R₁₀, R₁₁, R₁₂, R₁₃, R₁₄, and R₁₅ are hydrogen.

**16.** The drug link assembly unit or the stereoisomer, optical isomer, salt, or deuterated compound thereof according to claim 15, **characterized in that** the structure represented by formula A is as shown in formula E-1-a or E-1-a': 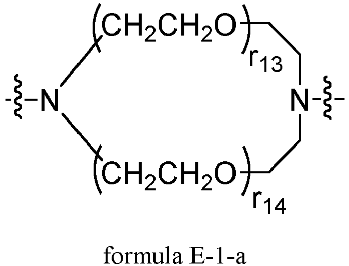 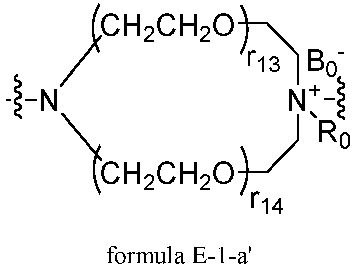
wherein r₁₃ and r₁₄ are integers independently selected from 1 to 5, preferably integers from 1 to 4; and
B₀⁻ is a monovalent anion, and R₀ is C₁₋₅ alkyl.

**17.** The drug link assembly unit or the stereoisomer, optical isomer, salt, or deuterated compound thereof according to claim 15, **characterized in that** the structure represented by formula A is as shown in formula E-1-b: 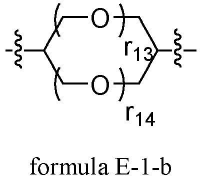 wherein r₁₃ and r₁₄ are integers independently selected from 1 to 5.

**18.** The drug link assembly unit or the stereoisomer, optical isomer, salt, or deuterated compound thereof according to claim 15, **characterized in that** the structure represented by formula A is as shown in formula E-1-c: 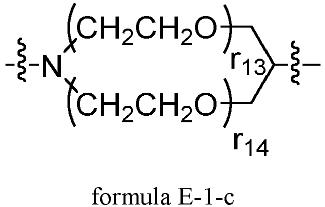 wherein r₁₃ and r₁₄ are integers independently selected from 1 to 5.

**19.** The drug link assembly unit or the stereoisomer, optical isomer, salt, or deuterated compound thereof according to claim 15, **characterized in that** the structure represented by formula A is as shown in formula E-1-d: 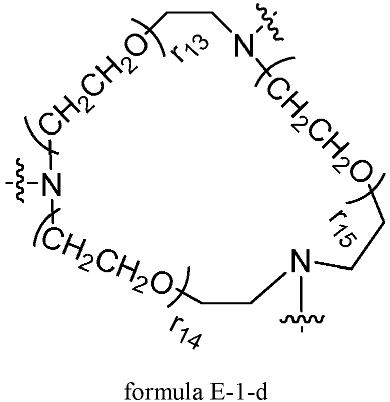 wherein r₁₃ and r₁₄ are integers independently selected from 1 to 5, and r₁₅ is an integer selected from 0 to 5.

**20.** The drug link assembly unit or the stereoisomer, optical isomer, salt, or deuterated compound thereof according to claim 13, **characterized in that** the structure represented by formula A is as shown in formula F-1: 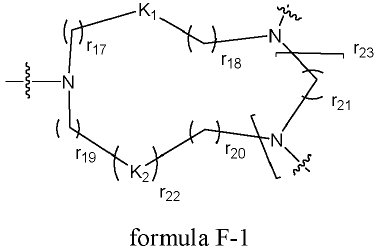
wherein n₁₇, r₁₈, r₁₉, r₂₀, and r₂₁ are integers independently selected from 1 to 3;
r₂₂ is selected from 0 or 1;
r₂₃ is selected from 0 or 1;
K₁ is selected from NR₁₆, N⁺R₁₆B⁻R₁₈, or CHR₂₀; K₂ is selected from NR₁₇, N⁺R₁₇B⁻R₁₈, or CHR₂₁;
N⁺ is a monovalent nitrogen cation, B⁻ is a monovalent anion, and R₁₈ is selected from C₁₋₃ alkyl, C₁₋₃ alkoxy, or 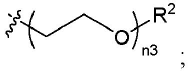 n3 is an integer from 1 to 50, and R² is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₁₉)₂; R₁₉ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl;
R₁₆ and R₁₇ are independently selected from C₁₋₃ alkyl, C₁₋₃ alkoxy, or 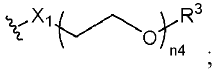 n4 is an integer from 1 to 40, and R³ is selected from hydrogen or methyl; or R₁₆ and R₁₇ are connected into 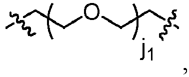 and j₁ is an integer selected from 0 to 5; X₁ is -CO-, -CONH-, or none;
R₂₀ and R₂₁ are independently selected from 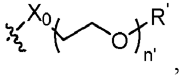 n' is an integer from 1 to 40, R' is selected from hydrogen or methyl, and X₀ is O or none.

**21.** The drug link assembly unit or the stereoisomer, optical isomer, salt, or deuterated compound thereof according to claim 20, **characterized in that** the structure represented by formula A is as shown in formula F-1-a: 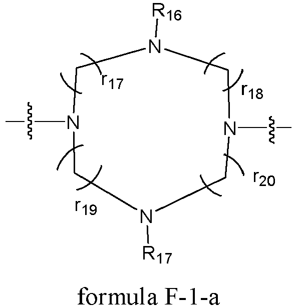
wherein r₁₇, r₁₈, r₁₉, and r₂₀ are integers independently selected from 1 to 3; and
R₁₆ and R₁₇ are independently selected from C₁₋₃ alkyl, C₁₋₃ alkoxy, or 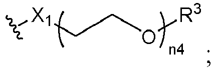 n4 is an integer from 1 to 40, preferably an integer from 9 to 16, and R³ is selected from hydrogen or methyl; or R₁₆ and R₁₇ are connected into 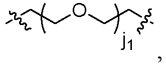 and j₁ is an integer selected from 0 to 5; X₁ is -CO-, -CONH-, or none.

**22.** The drug link assembly unit or the stereoisomer, optical isomer, salt, or deuterated compound thereof according to claim 20, **characterized in that** the structure represented by formula A is as shown in formula F-1-b: 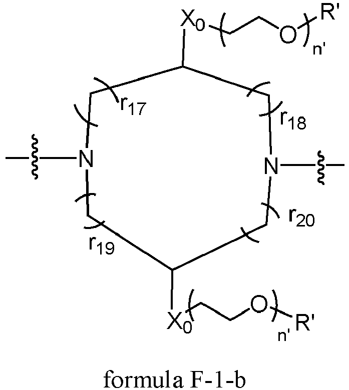
wherein r₁₇, r₁₈, r₁₉, and r₂₀ are integers independently selected from 1 to 3;
n' is an integer from 1 to 40, preferably an integer from 9 to 16, and R' is selected from hydrogen or methyl; and
X₀ is O or none.

**23.** The drug link assembly unit or the stereoisomer, optical isomer, salt, or deuterated compound thereof according to claim 20, **characterized in that** the structure represented by formula A is as shown in formula F-1-c: 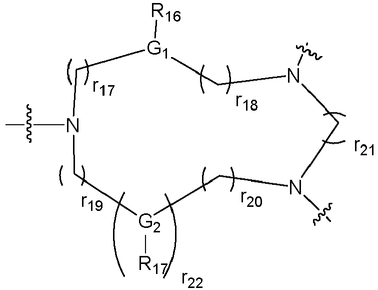 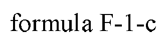
wherein r₁₇, r₁₈, r₁₉, r₂₀, and r₂₁ are integers independently selected from 1 to 3;
r₂₂ is selected from 0 or 1;
G₁ is selected from Nor N⁺B⁻R₁₈; G₂ is selected from N or N⁺B⁻R₁₈;
N⁺ is a monovalent nitrogen cation, B⁻ is a monovalent anion, and R₁₈ is selected from C₁₋₃ alkyl, C₁₋₃ alkoxy, or 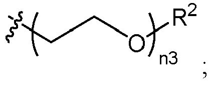 n3 is an integer from 1 to 50, preferably an integer from 3 to 16, and R² is selected from hydrogen, C₁₋₁₀ alkyl, C₁₋₁₀ alkoxy, C₂-C₁₀ alkenyl, C₂-C₁₀ alkynyl, 3-10-membered aryl, 3-10-membered heteroaryl, or N(R₁₉)₂; R₁₉ is selected from hydrogen, C₁₋₁₀ alkyl, C₁₋₁₀ alkoxy, C₂-C₁₀ alkenyl, C₂-C₁₀ alkynyl, 3-10-membered aryl, or 3-10-membered heteroaryl; and
R₁₆ and R₁₇ are independently selected from C₁₋₃ alkyl, C₁₋₃ alkoxy, or 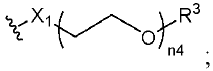 n4 is an integer from 1 to 40, preferably an integer from 9 to 16, and R³ is selected from hydrogen or methyl; or R₁₆ and R₁₇ are linked into 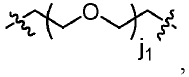 and j 1 is an integer selected from 0 to 5; X1 is -CO-, -CONH-, or none.

**24.** The drug link assembly unit or the stereoisomer, optical isomer, salt, or deuterated compound thereof according to any one of claims 13-23, **characterized in that** the structure represented by formula A is as follows: 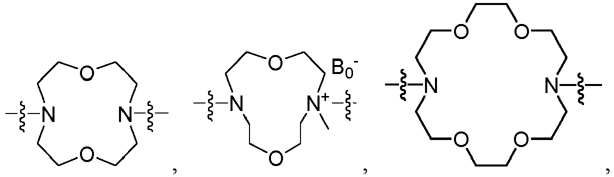 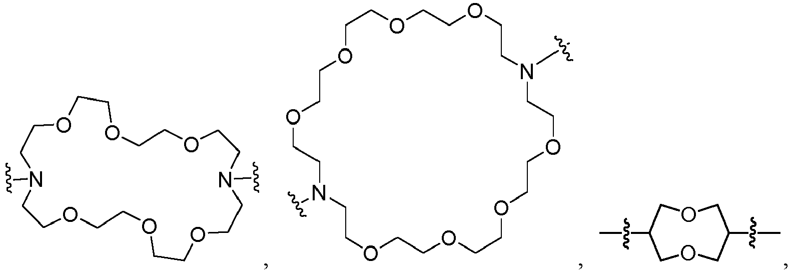 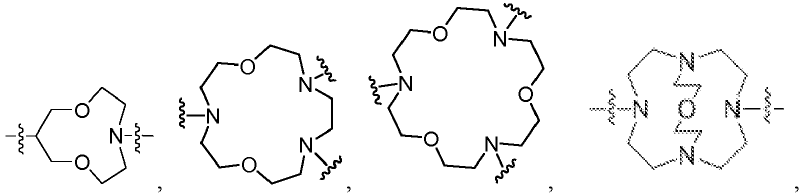 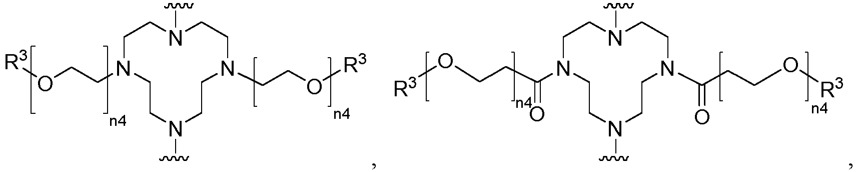 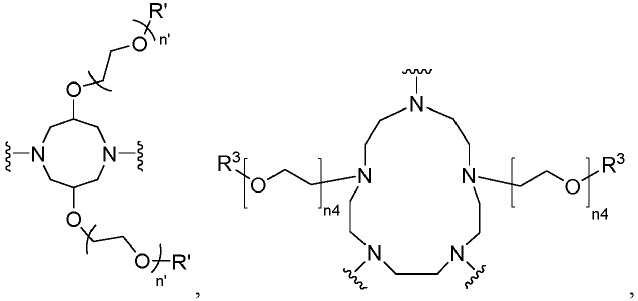 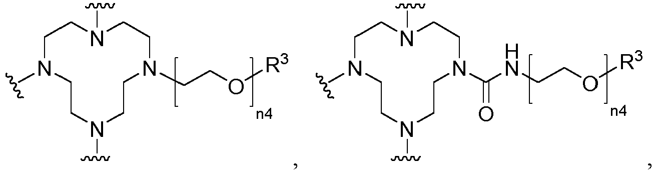 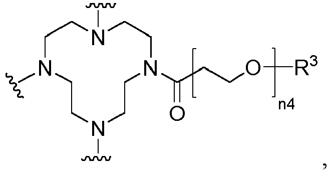 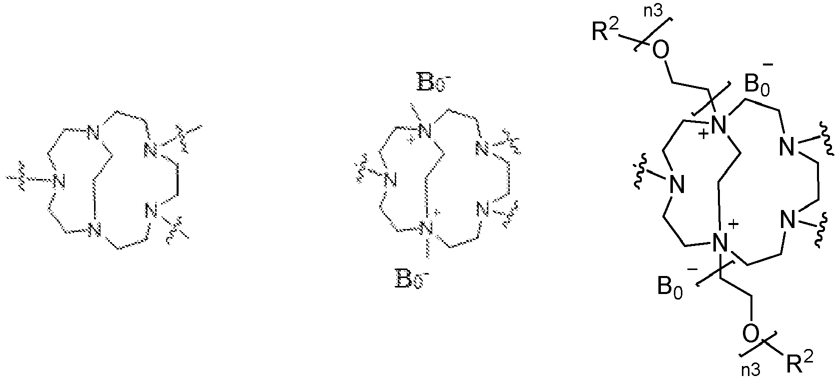 n4 is an integer selected from 1 to 24, preferably an integer from 9 to 16; R³ is hydrogen or methyl; n' is an integer selected from 1 to 24, preferably an integer from 9 to 16; R' is hydrogen or methyl; n3 is an integer selected from 1 to 24, preferably an integer from 3 to 16; R² is hydrogen or methyl; and B₀⁻ is a monovalent anion.

**25.** The drug link assembly unit or the stereoisomer, optical isomer, salt, or deuterated compound thereof according to any one of claims 1-24, **characterized in that** the structure of the linker is as shown in formula G-1 or G-2: 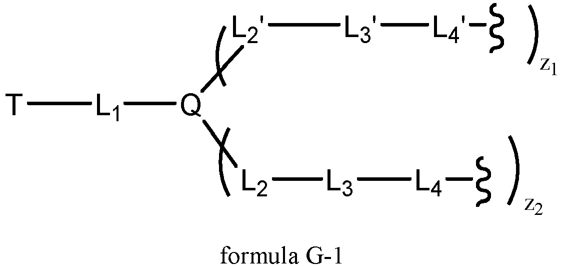 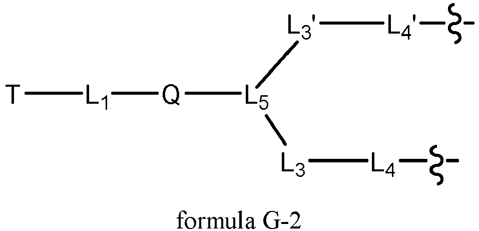
wherein T is a tying group, which can be connected to the targeted linker; the targeted linker is a substance capable of targeted binding to a lesion site;
L₁ is none or a breakable or unbreakable linker fragment;
L₂ is none or a breakable or unbreakable linker fragment;
L₂' is none or a breakable or unbreakable linker fragment;
L₃ is none or a breakable or unbreakable linker fragment;
L₃' is none or a breakable or unbreakable linker fragment;
L₄ is none or a breakable or unbreakable linker fragment;
L₄' is none or a breakable or unbreakable linker fragment;
L₅ is none or a breakable or unbreakable trident linker fragment;
Z₁ is 0 or 1, Z₂ is 0 or 1, and Z₁ and Z₂ are not simultaneously 0; and
the structure of Q is as shown in formula A.

**26.** The drug link assembly unit or the stereoisomer, optical isomer, salt, or deuterated compound thereof according to claim 25, **characterized in that** the structure of L₅ is selected from 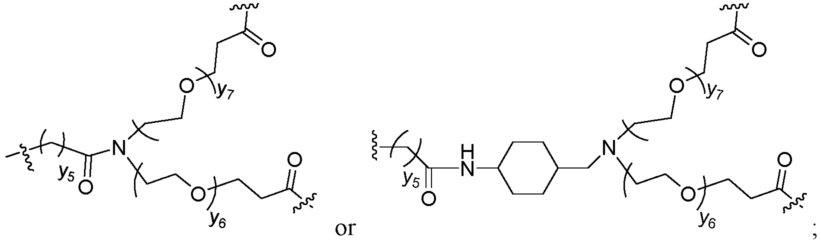
y₅ is an integer selected from 1 to 10, preferably 1;
y₆ is an integer selected from 1 to 50, preferably an integer from 2 to 9; and
y₇ is an integer selected from 1 to 50, preferably an integer from 2 to 9.

**27.** The drug link assembly unit or the stereoisomer, optical isomer, salt, or deuterated compound thereof according to claim 25, **characterized in that** the structure of the linker is as shown in formula G-3: 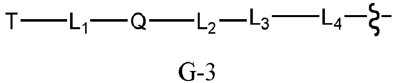
wherein T is a tying group, which can be connected to the targeted linker; the targeted linker is a substance capable of targeted binding to a lesion site;
L₁ is a breakable or unbreakable linker fragment;
L₂ is none or a breakable or unbreakable linker fragment;
L₃ is a breakable or unbreakable linker fragment;
L₄ is none or a breakable or unbreakable linker fragment; and
the structure of Q is as shown in formula A.

**28.** The drug link assembly unit or the stereoisomer, optical isomer, salt, or deuterated compound thereof according to any one of claims 25-27, **characterized in that** the structure of T is as follows:

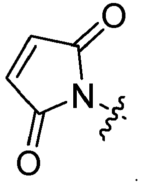

**29.** The drug link assembly unit or the stereoisomer, optical isomer, salt, or deuterated compound thereof according to any one of claims 25-27, **characterized in that** the structure of L₁ is as follows: 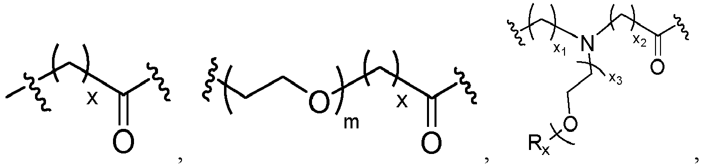 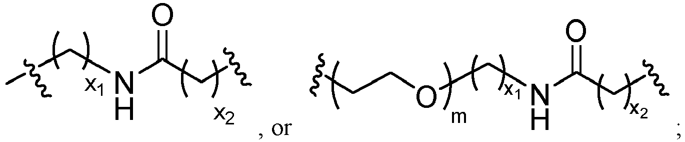
wherein x is an integer selected from 1 to 10, preferably an integer from 2 to 6;
m is an integer selected from 1 to 25, preferably an integer from 2 to 10, and more preferably an integer from 2 to 9;
x₁ is an integer selected from 1 to 10, preferably an integer from 2 to 6. and more preferably 2;
x₂ is an integer selected from 1 to 10, preferably an integer from 1 to 5. and more preferably 1;
x₃ is an integer selected from 0 to 50, preferably an integer from 8 to 30, and more preferably 9; and
Rₓ is selected from hydrogen or methyl.

**30.** The drug link assembly unit or the stereoisomer, optical isomer, salt, or deuterated compound thereof according to claim 25, **characterized in that** the structure of L₂ is 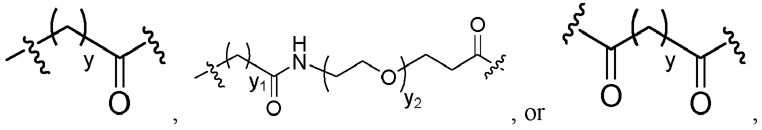 or L₂ is none;
the structure of L₂' is 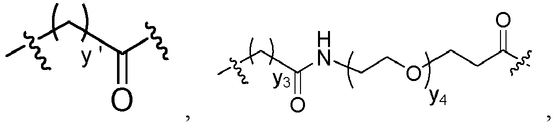 or 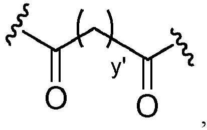 or L₂' is none;
wherein y and y' are integers independently selected from 1 to 10, preferably 1 or 2;
y₁ and y₃ are integers independently selected from 0 to 10, preferably 0, 1 or 2, and more preferably 0 or 1; and
y₂ and y₄ are integers independently selected from 1 to 50, preferably integers from 1 to 20, and more preferably integers from 2 to 12.

**31.** The drug link assembly unit or the stereoisomer, optical isomer, salt, or deuterated compound thereof according to claim 25, **characterized in that** the structures of L₃ and L₃' are independently selected from: 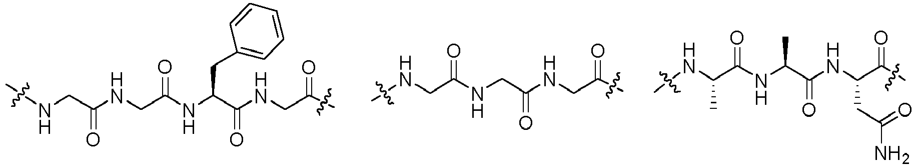 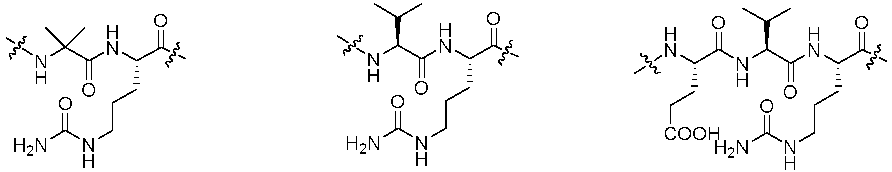 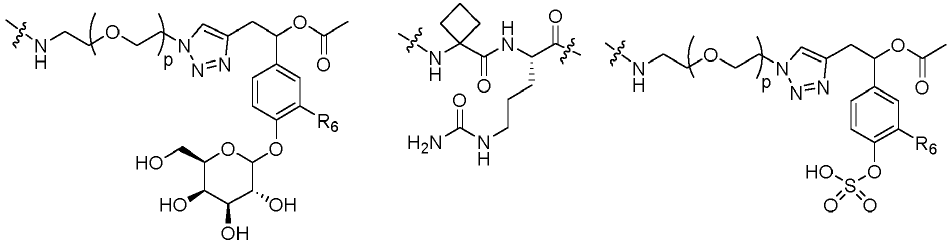 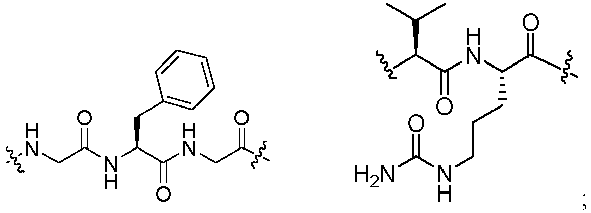 wherein p is an integer selected from 1 to 24, and R₆ is selected from hydrogen or nitro.

**32.** The drug link assembly unit or the stereoisomer, optical isomer, salt, or deuterated compound thereof according to claim 25, **characterized in that** the structures of L₄ and L₄' are independently selected from none,

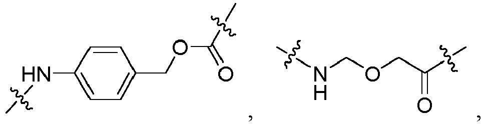

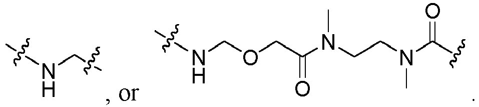

**33.** The drug link assembly unit or the stereoisomer, optical isomer, salt, or deuterated compound thereof according to any one of claims 25-32, **characterized in that** the structure of the linker is selected from: 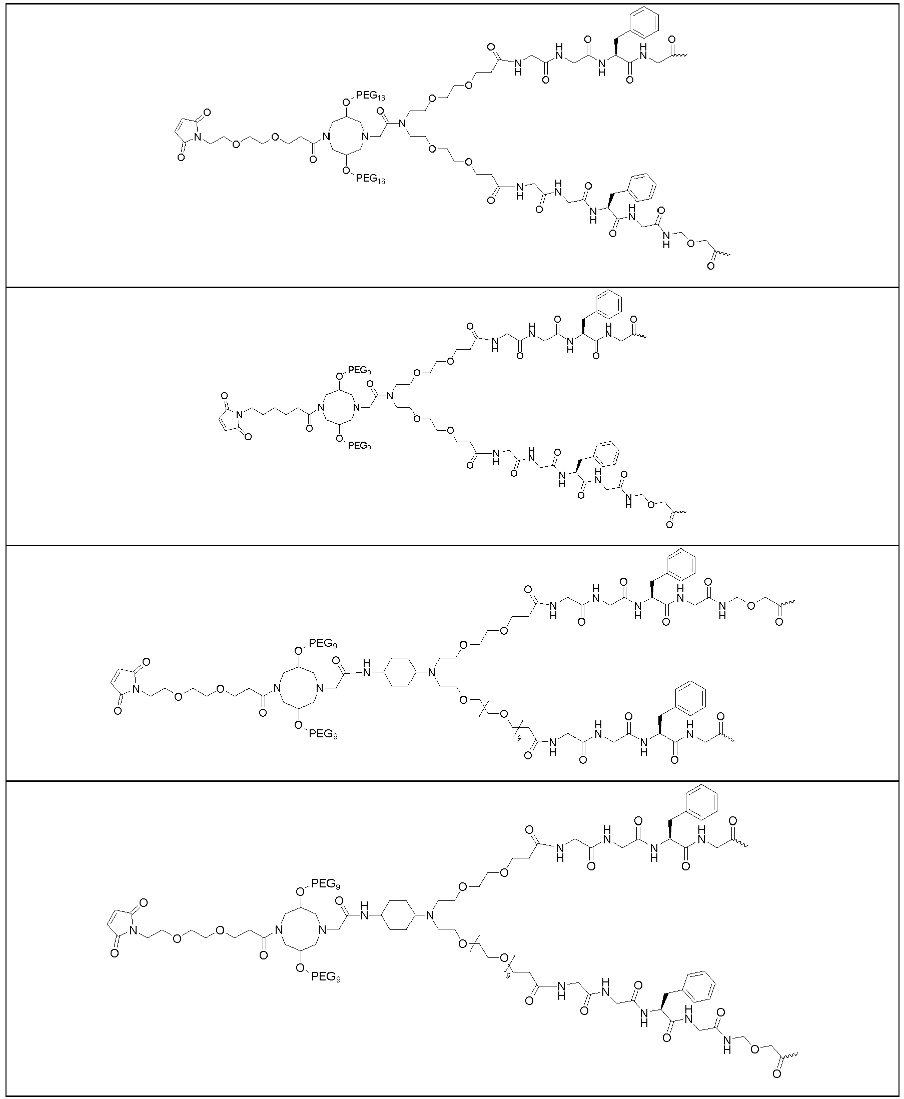 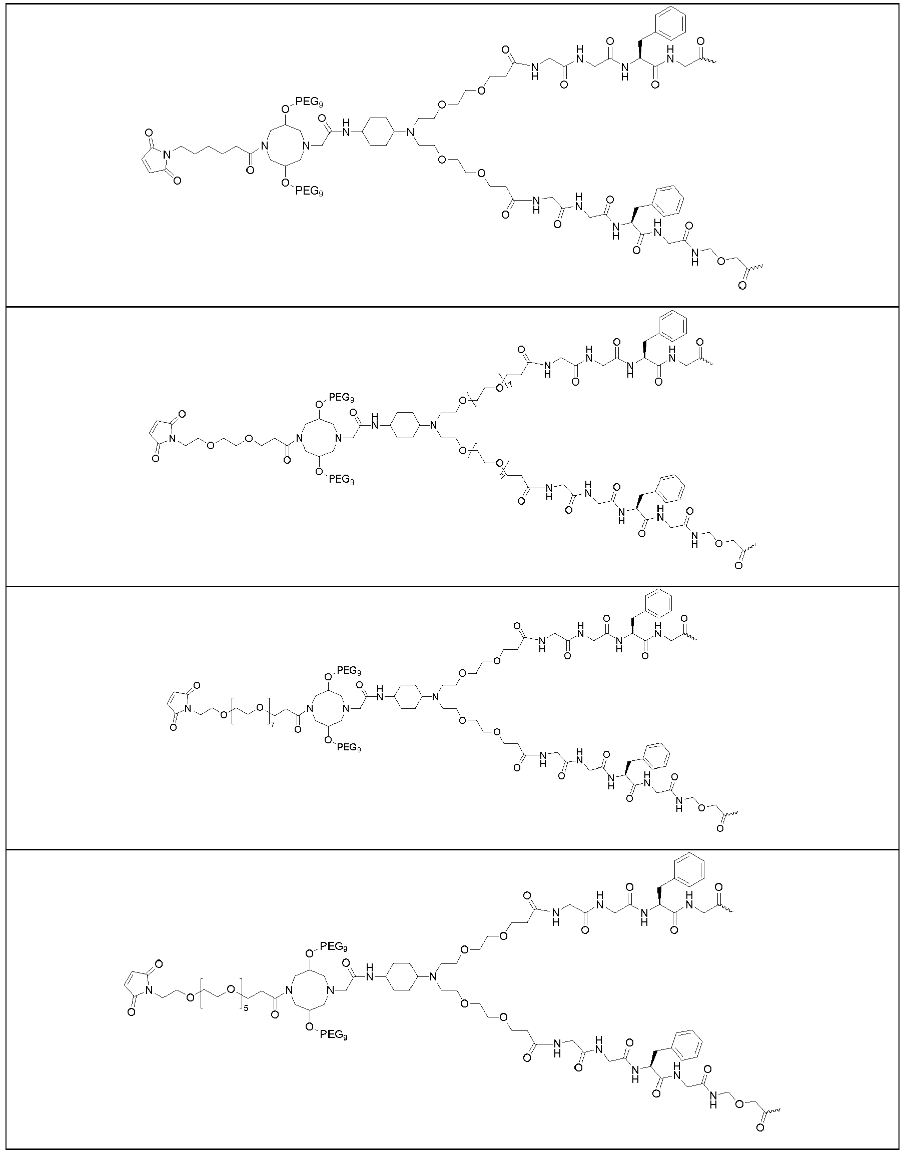 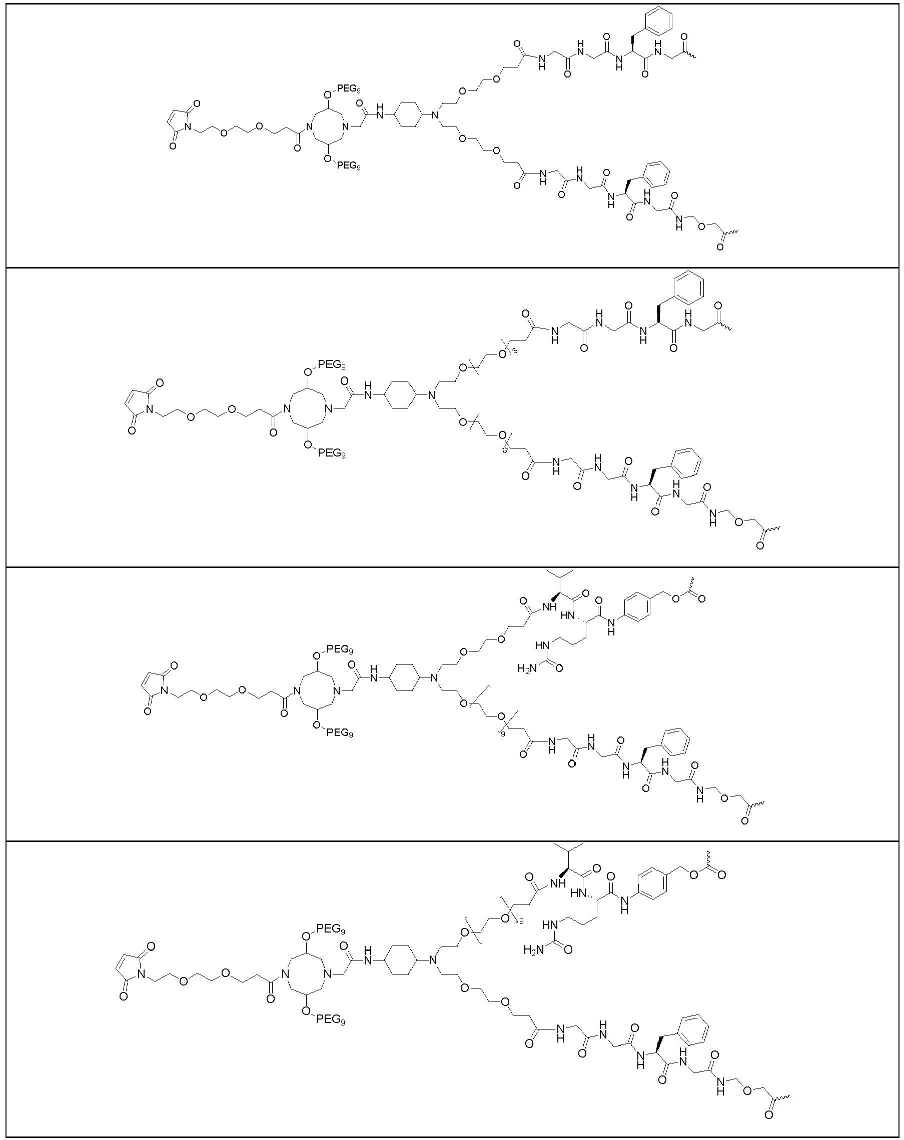 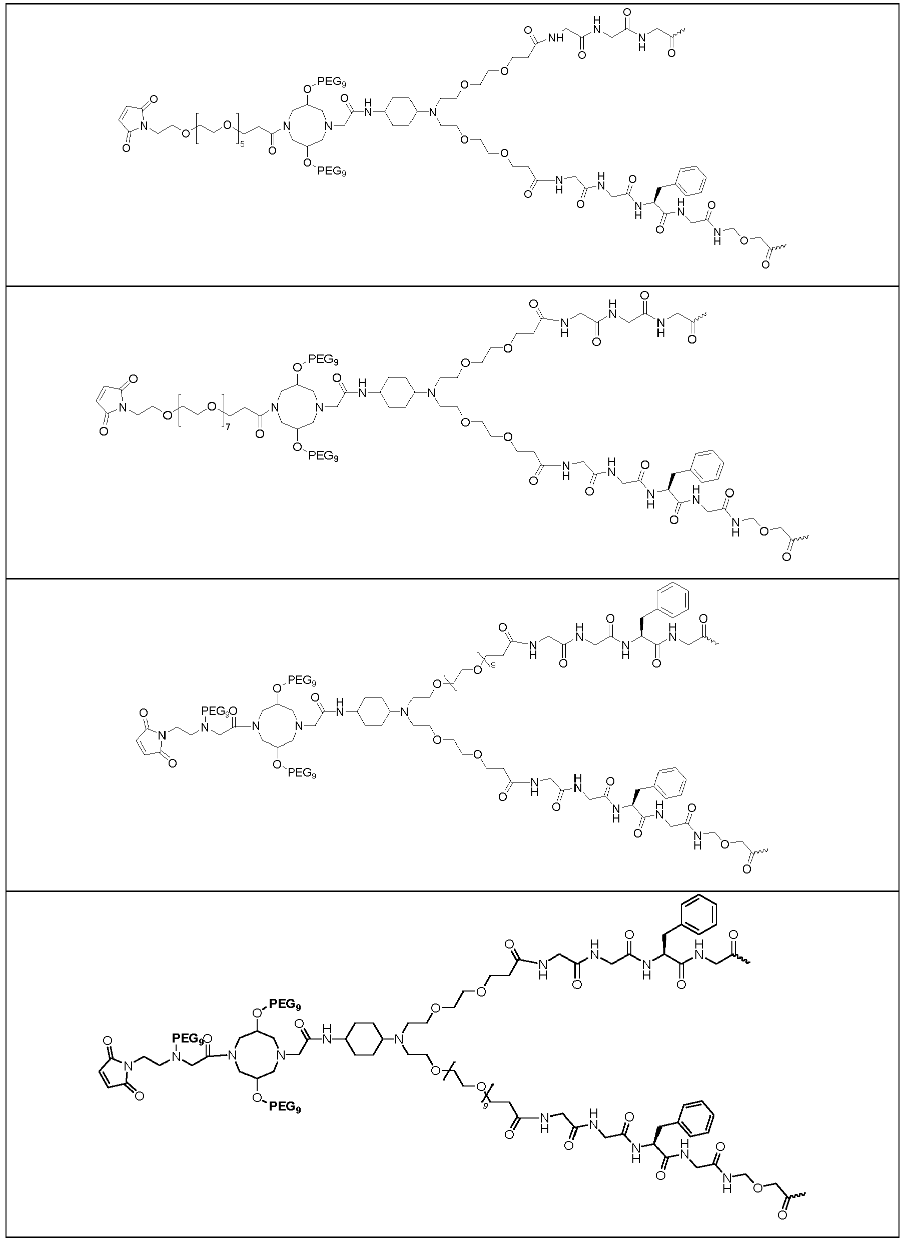 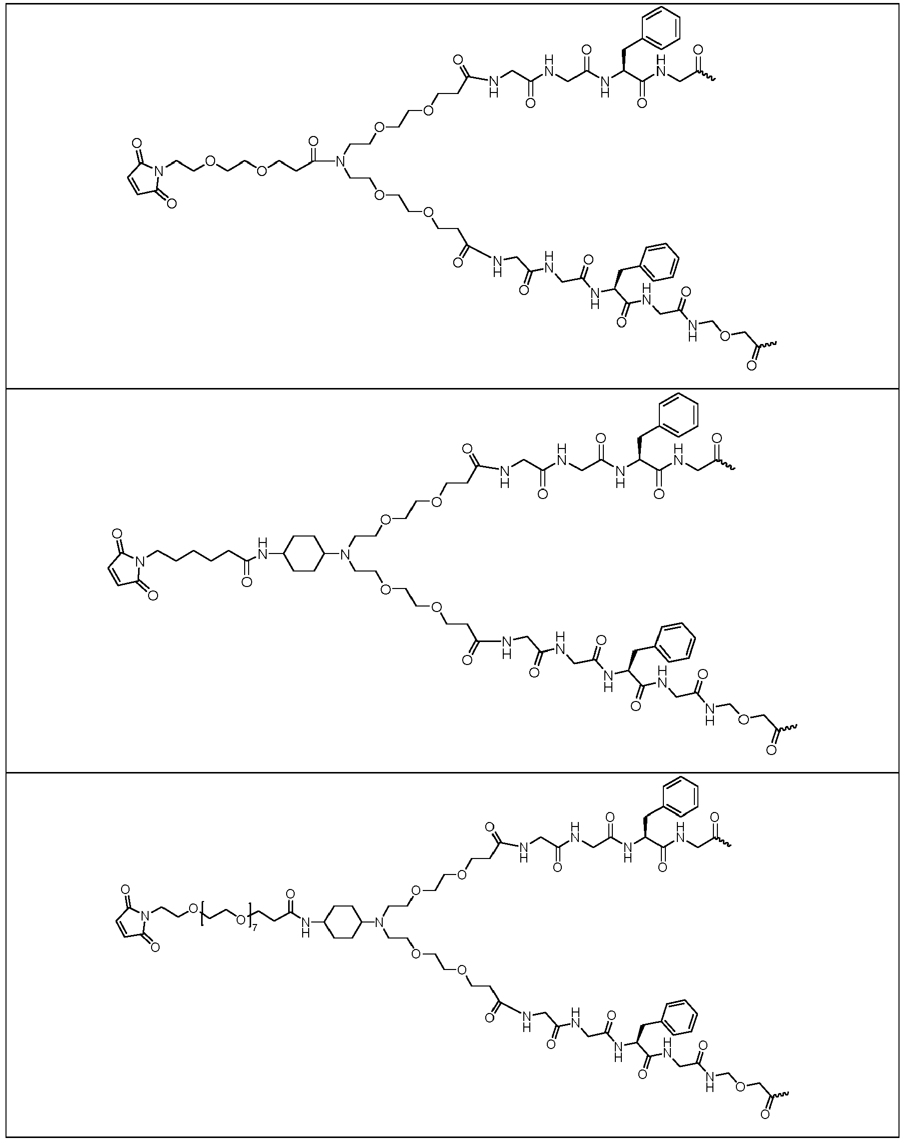 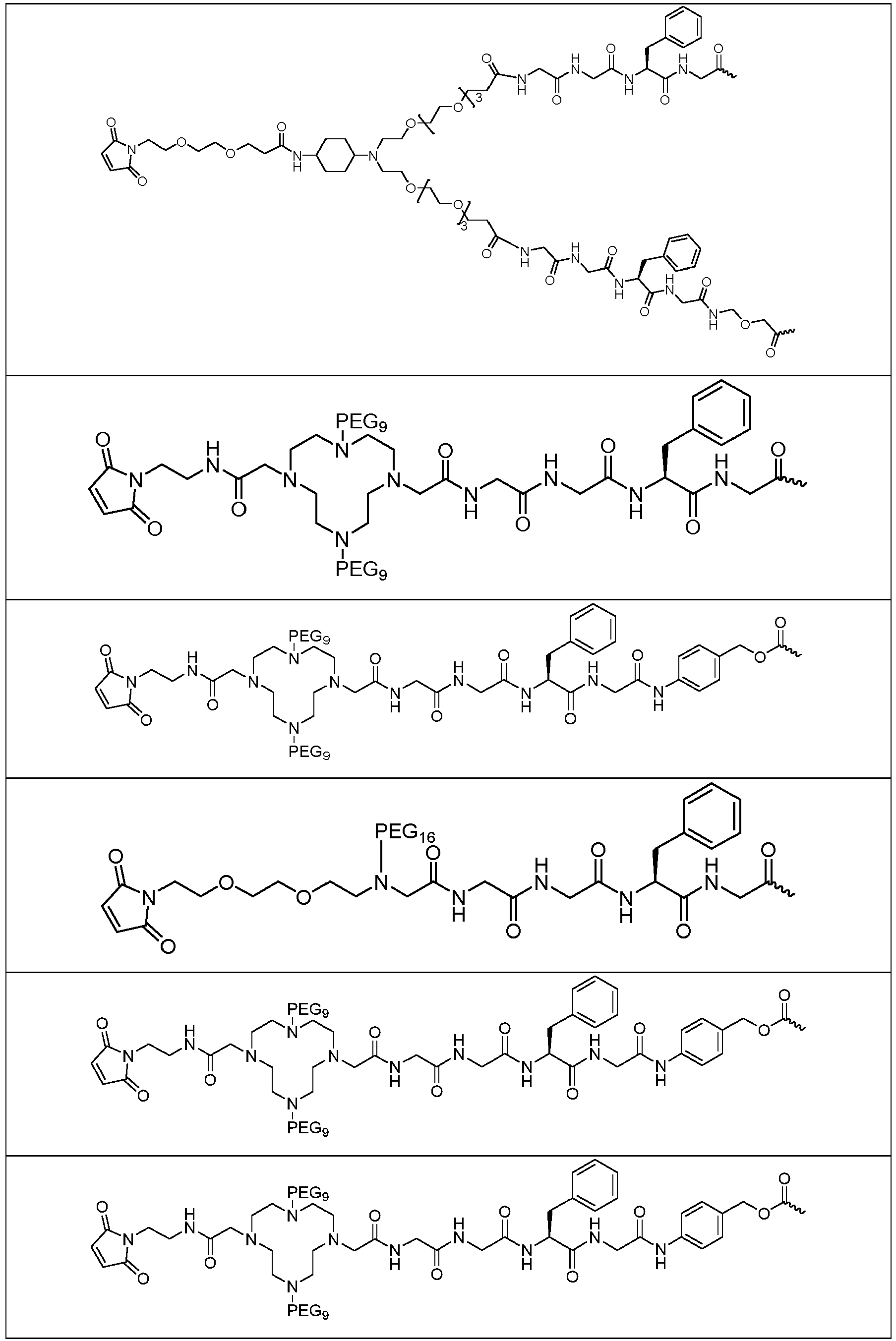 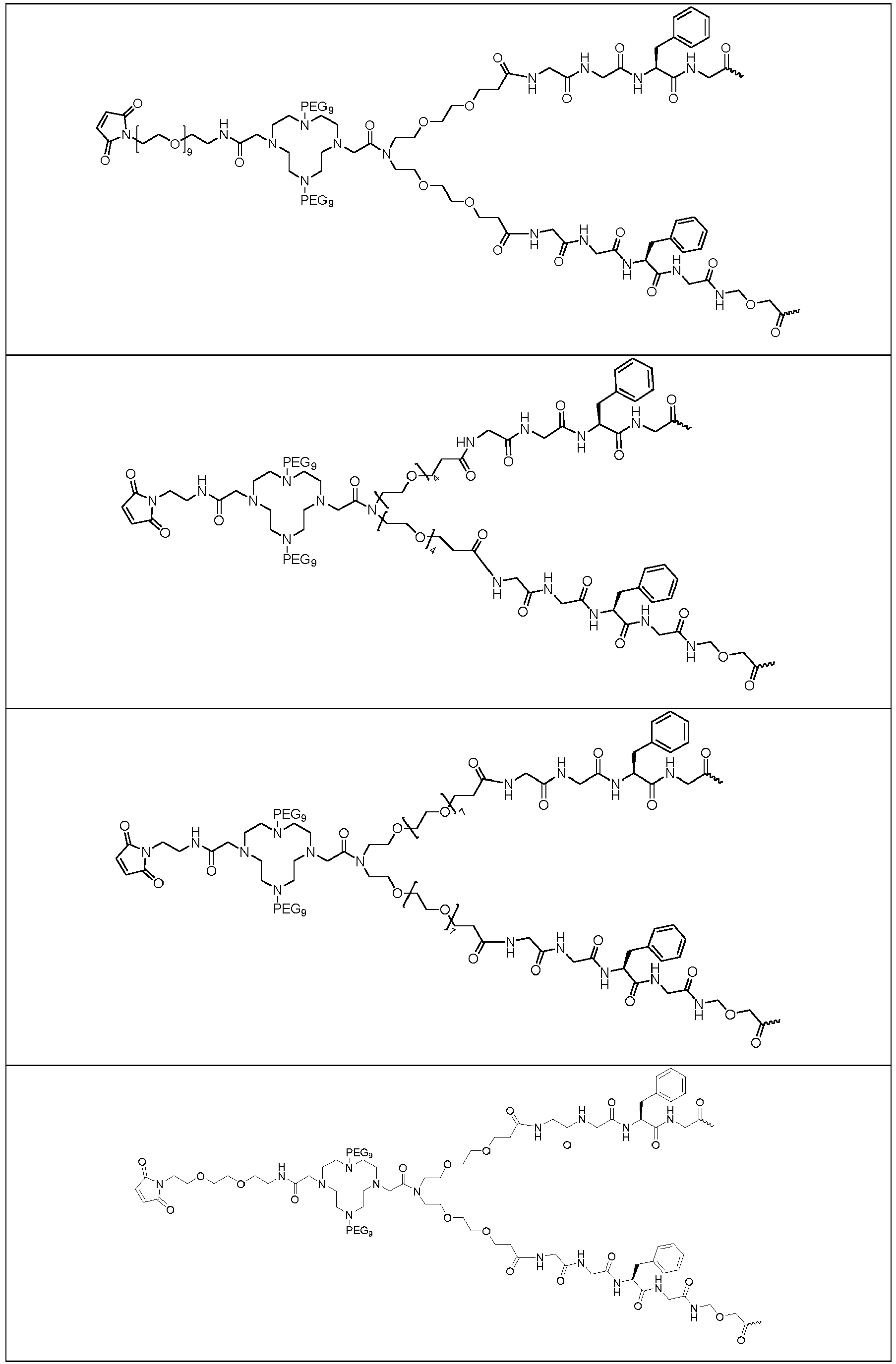 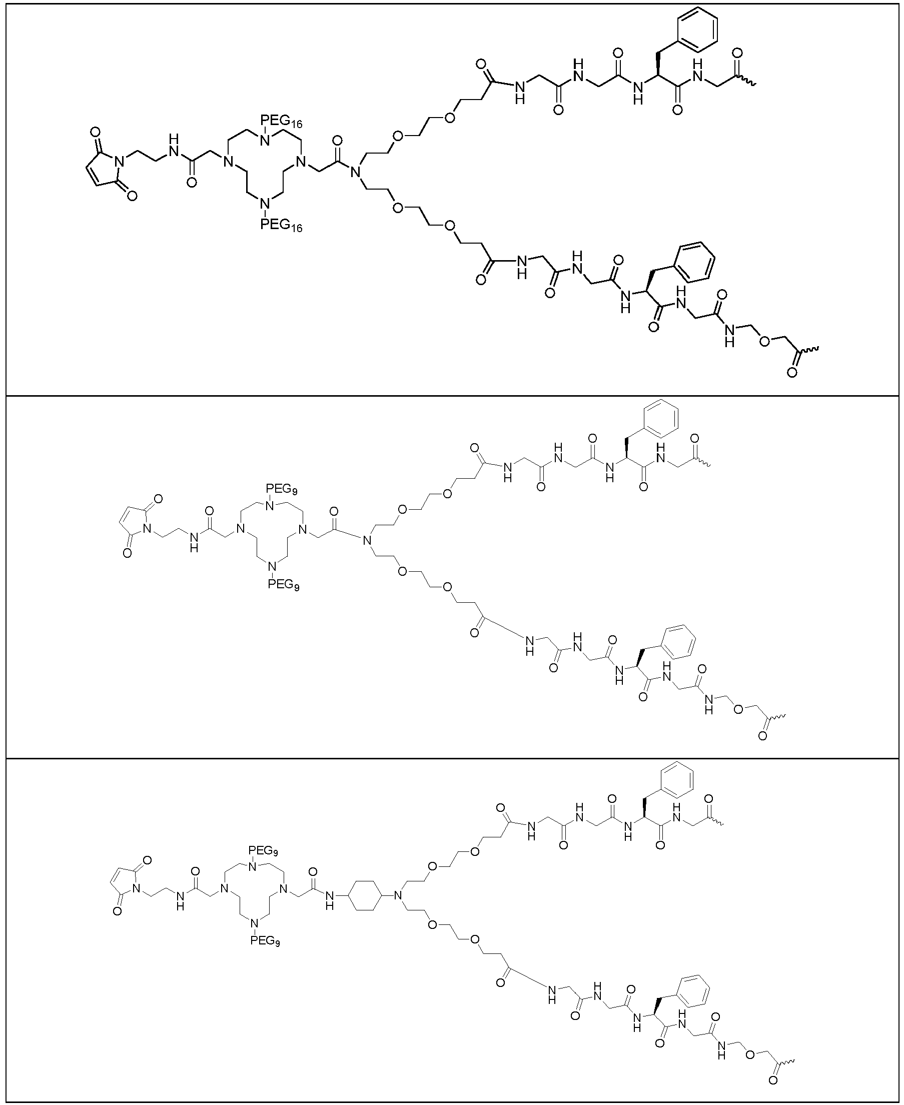 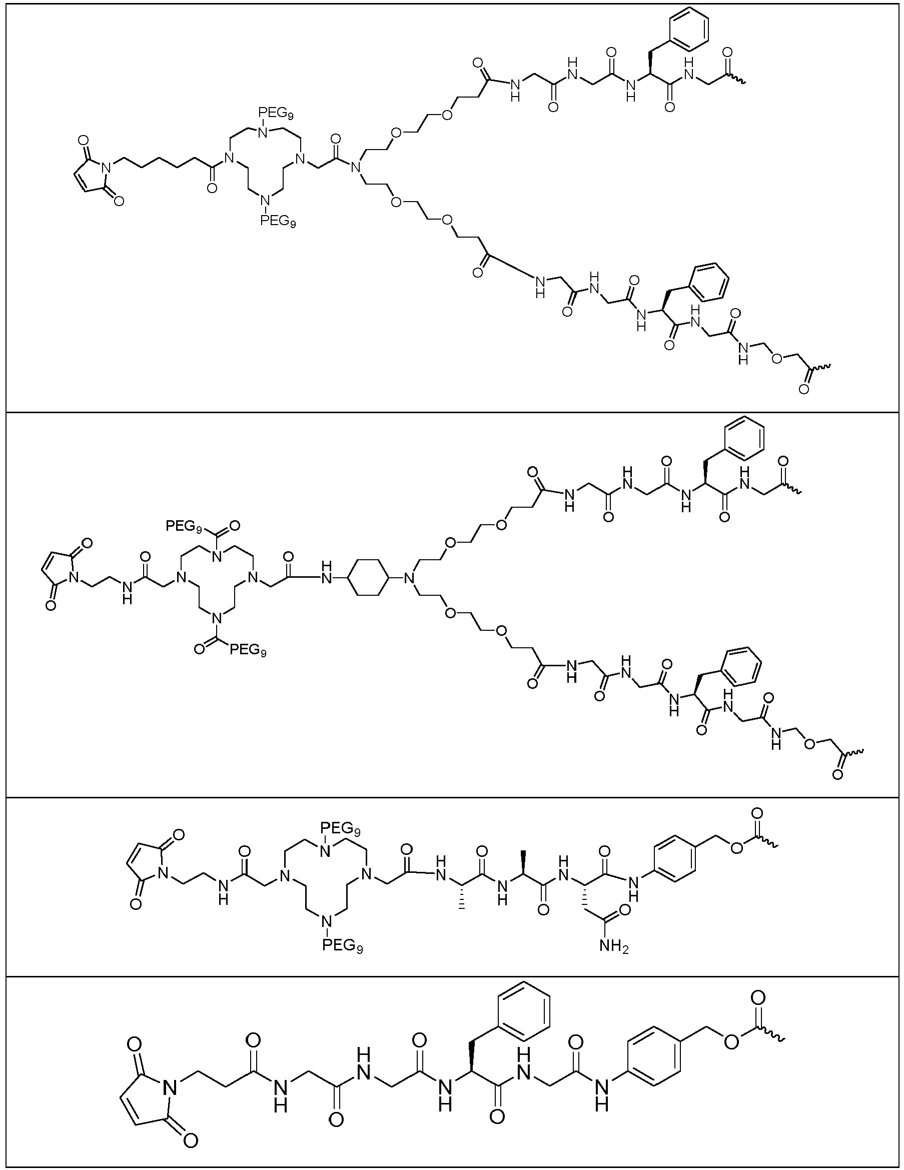 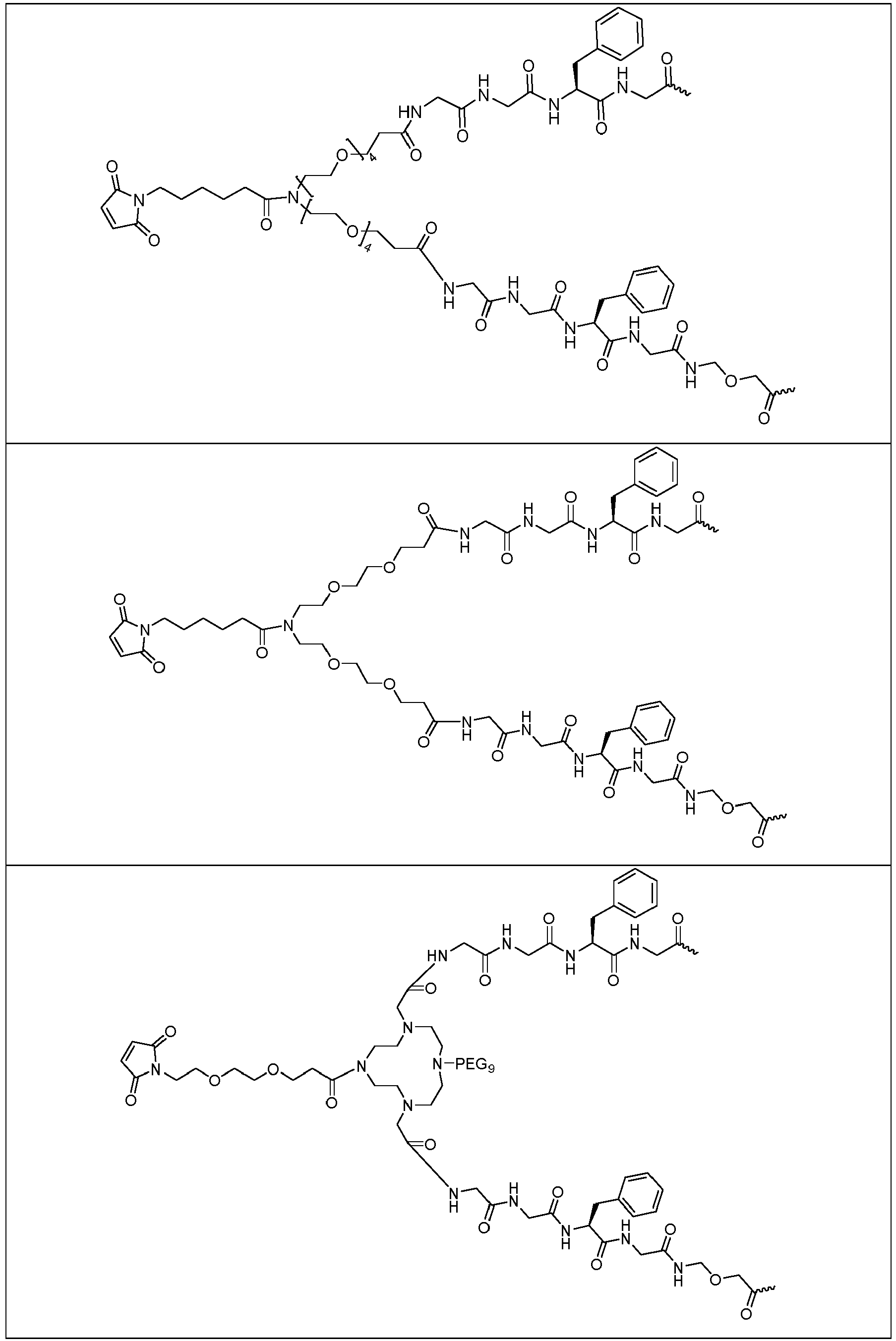 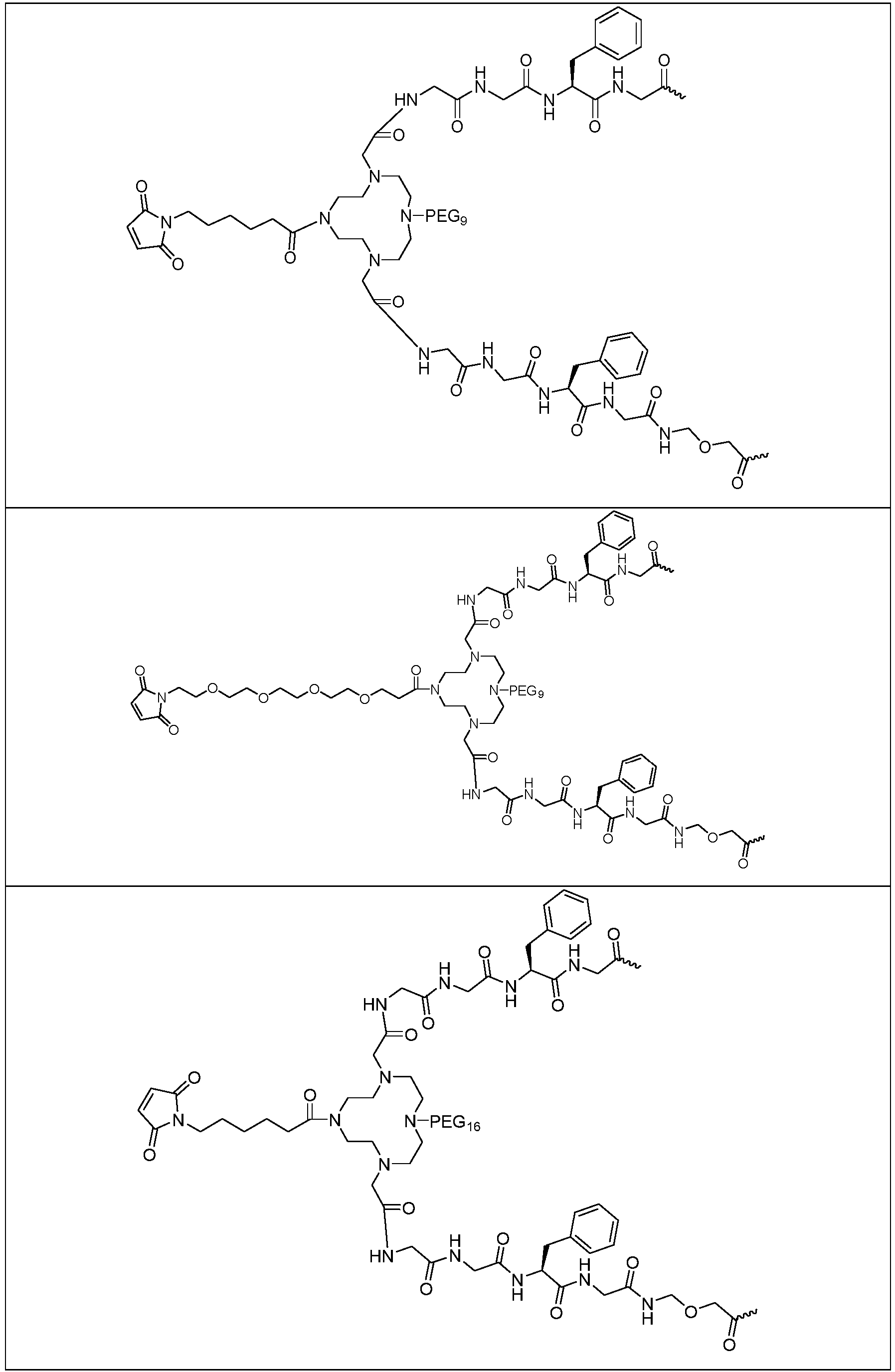 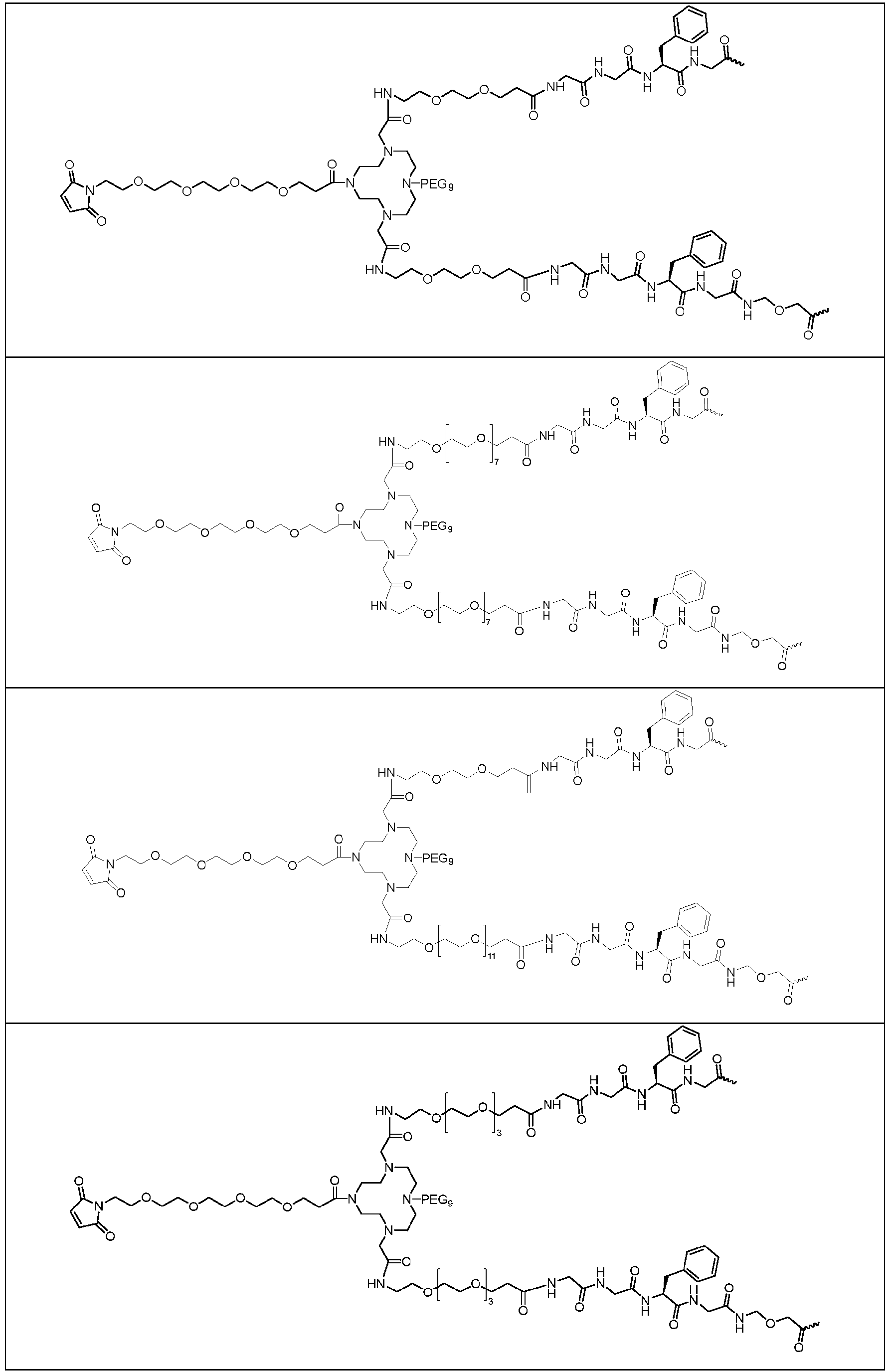 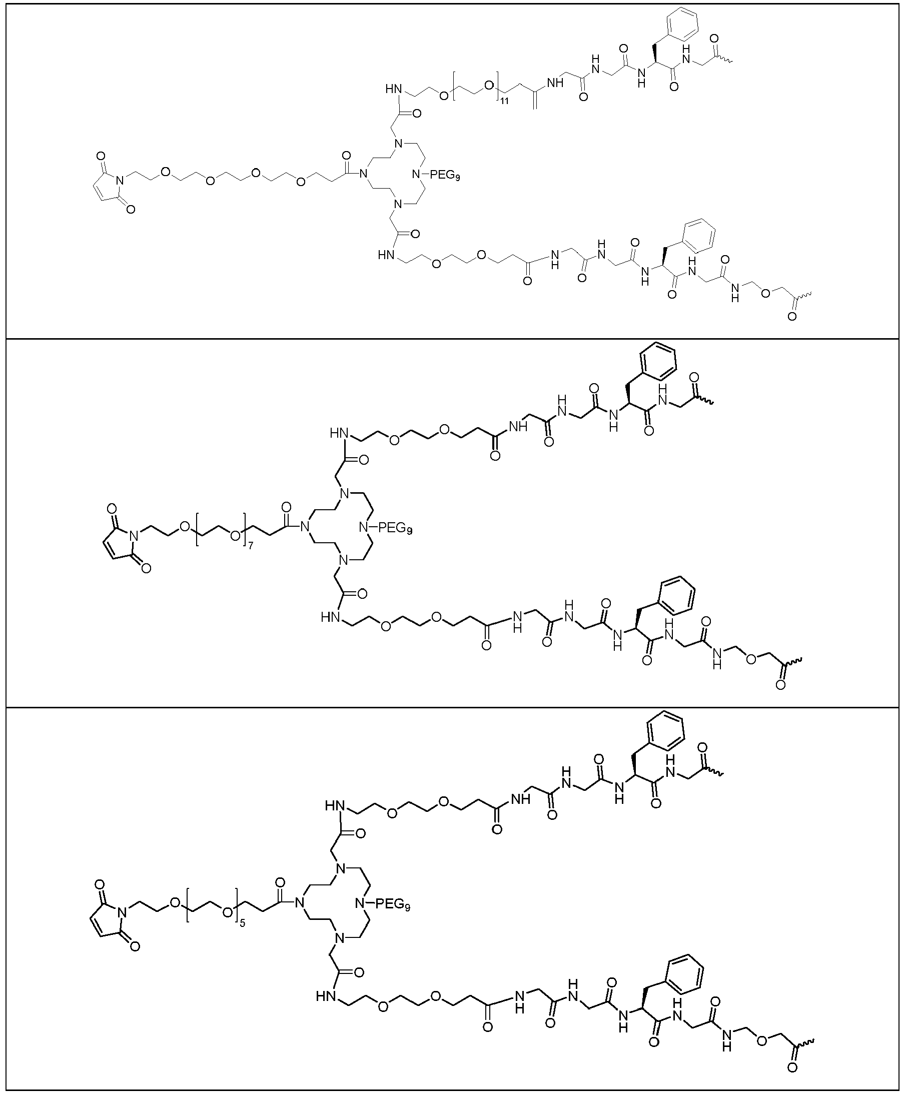 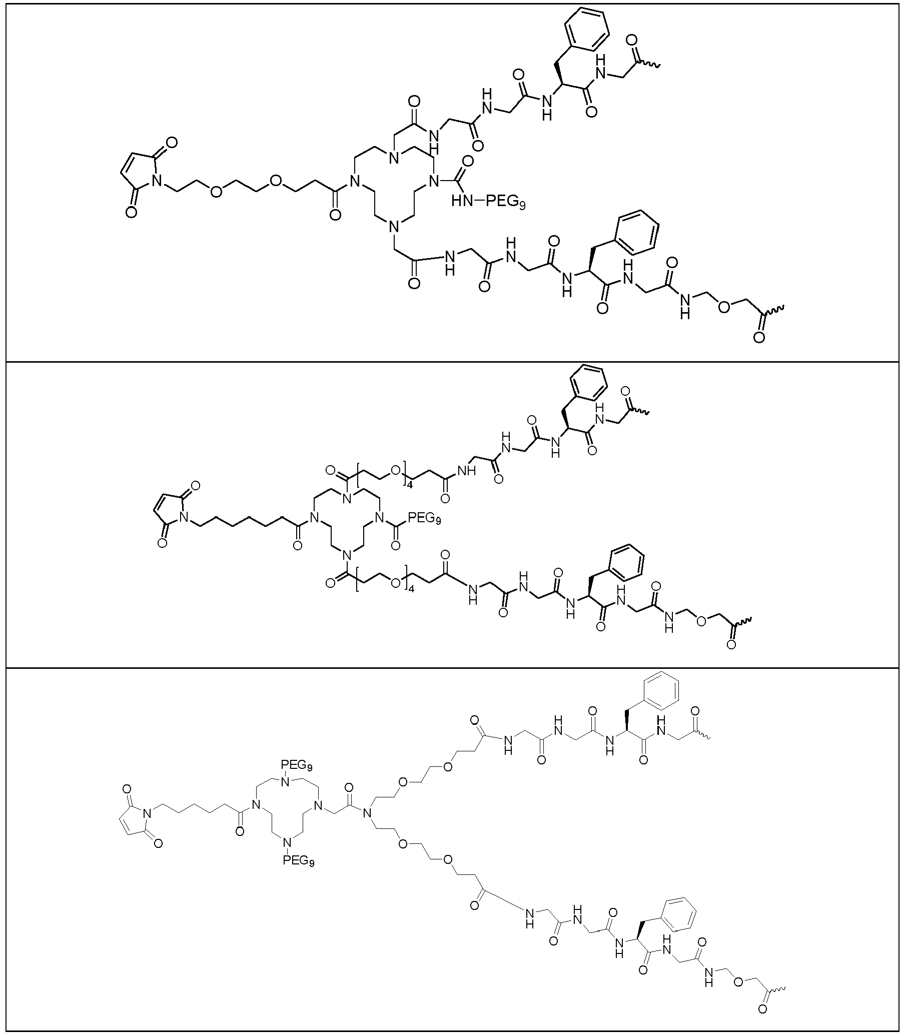 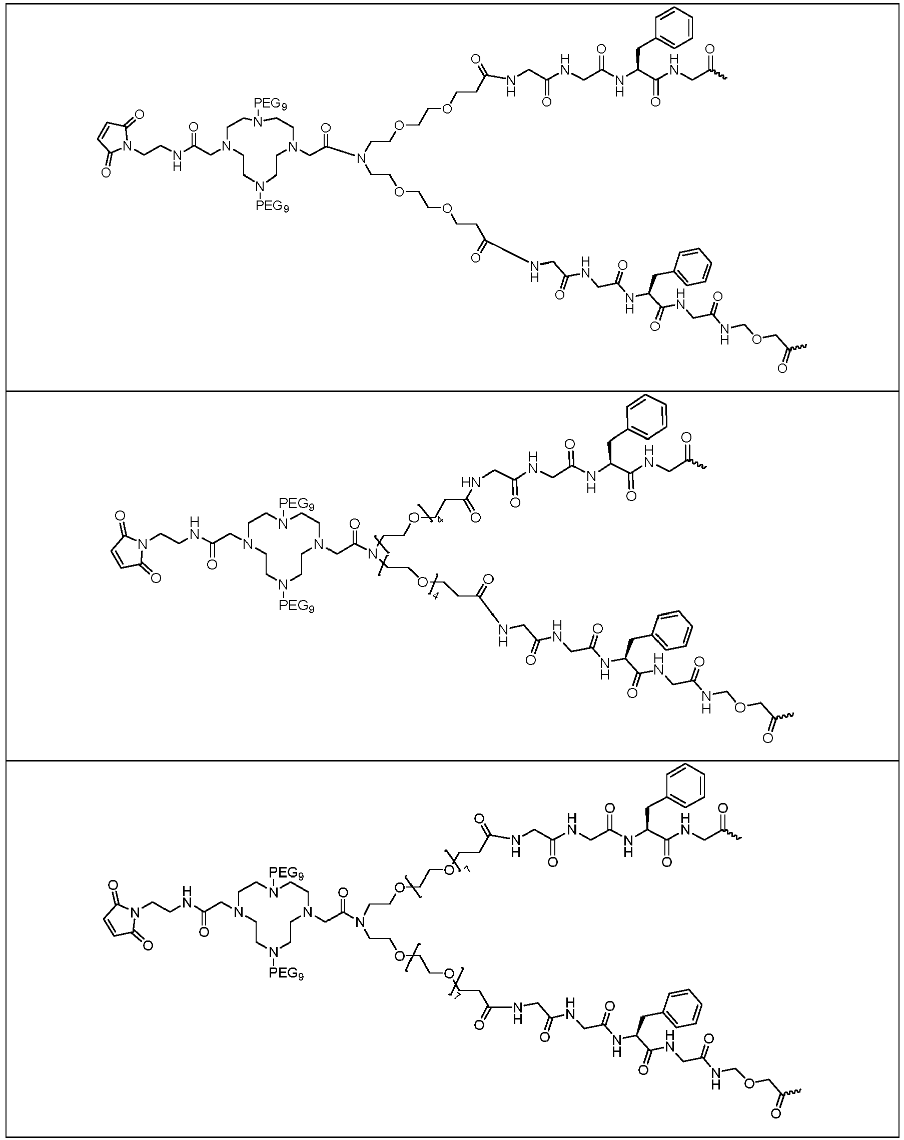 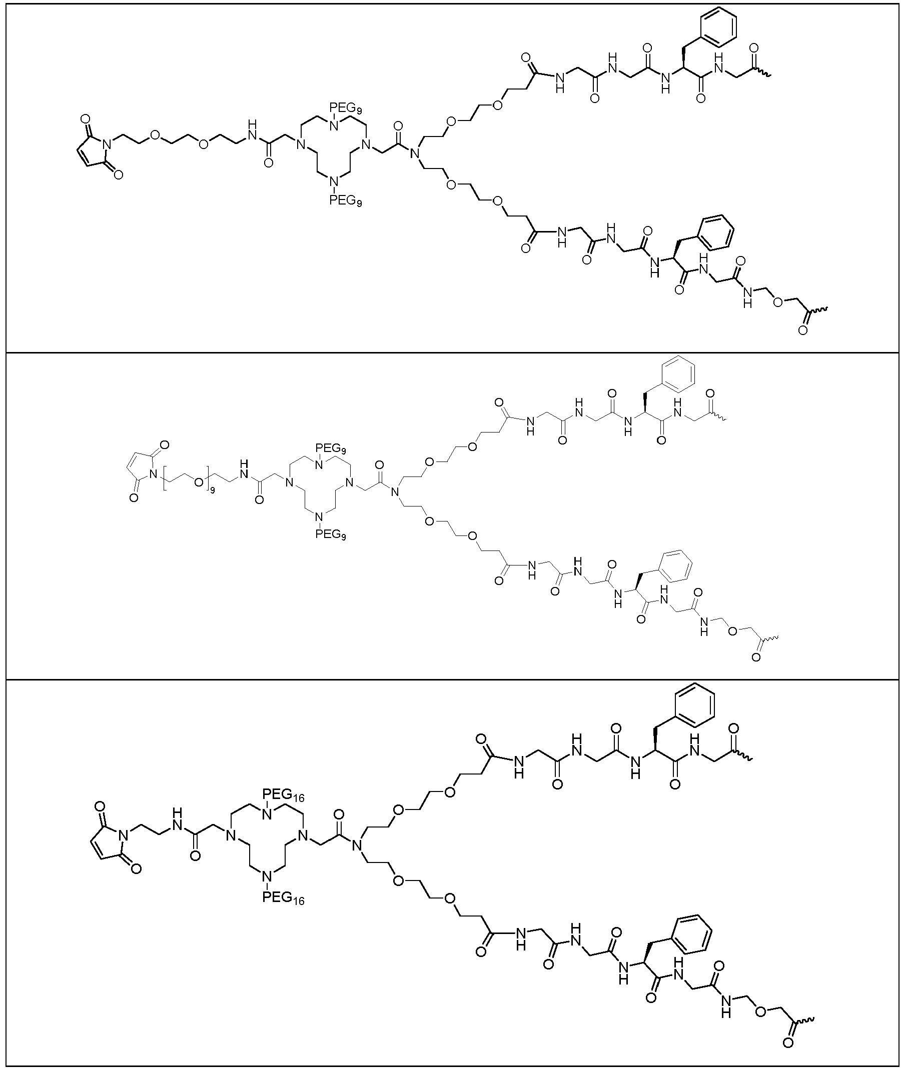 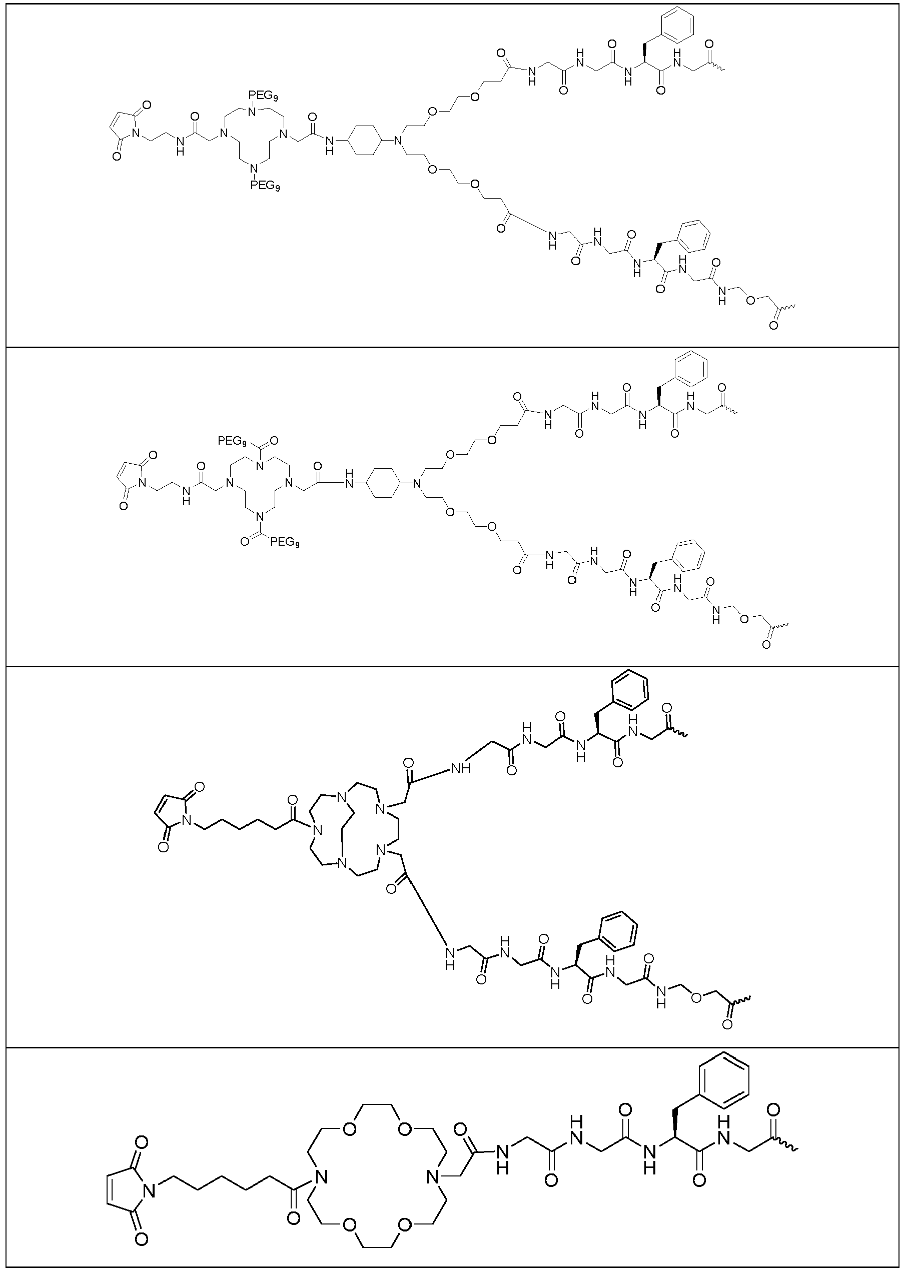 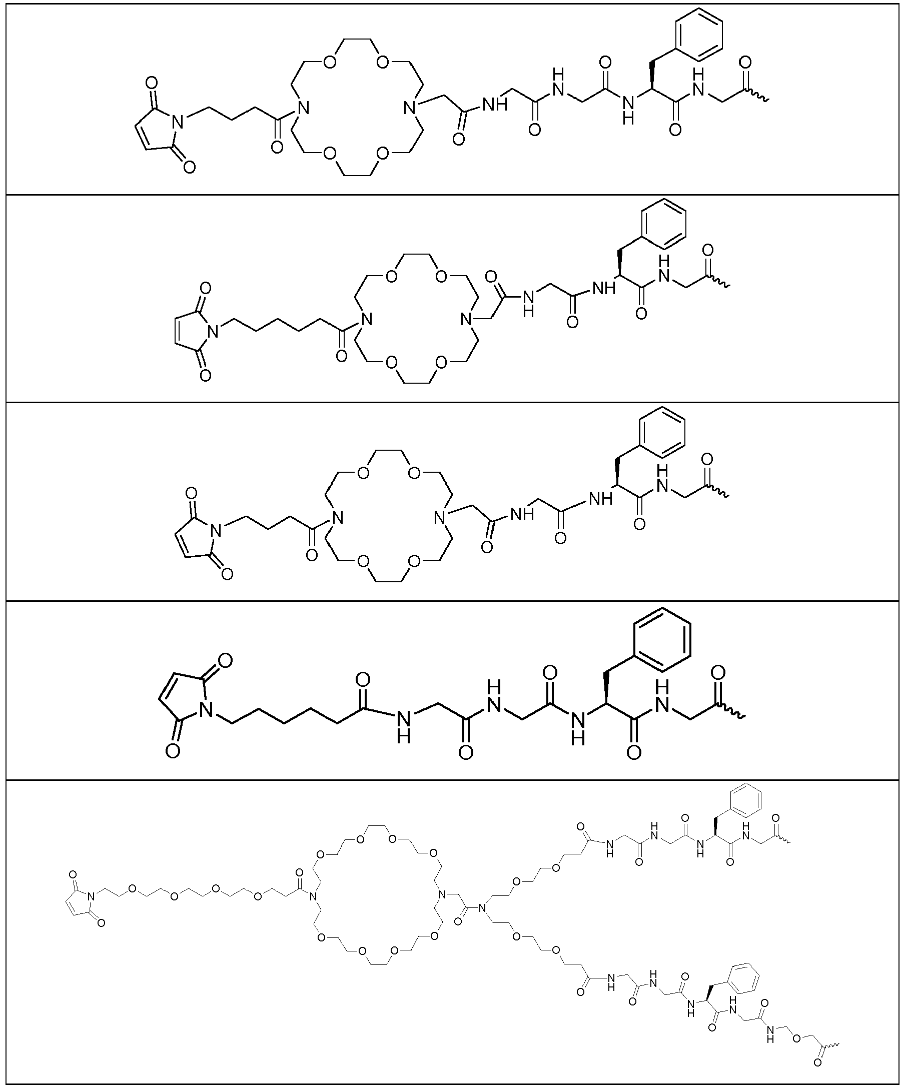 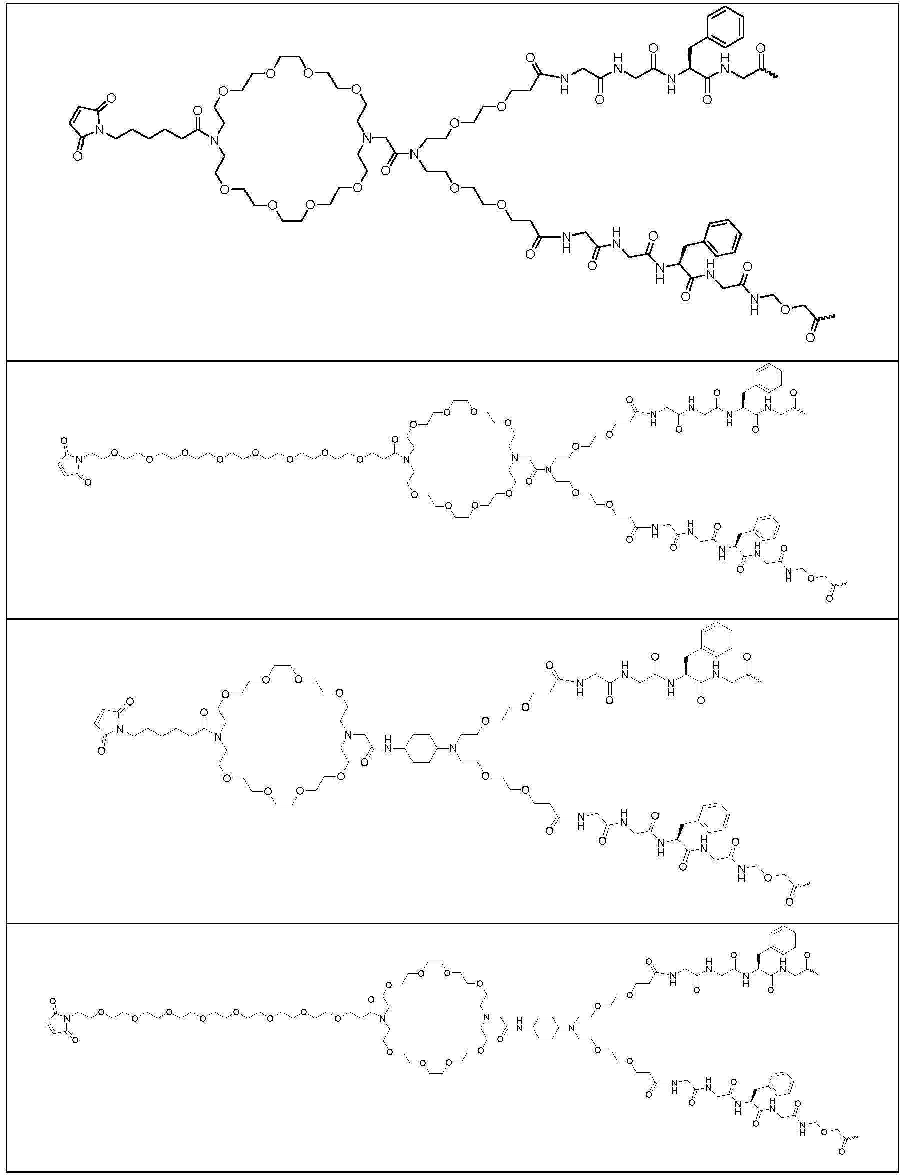 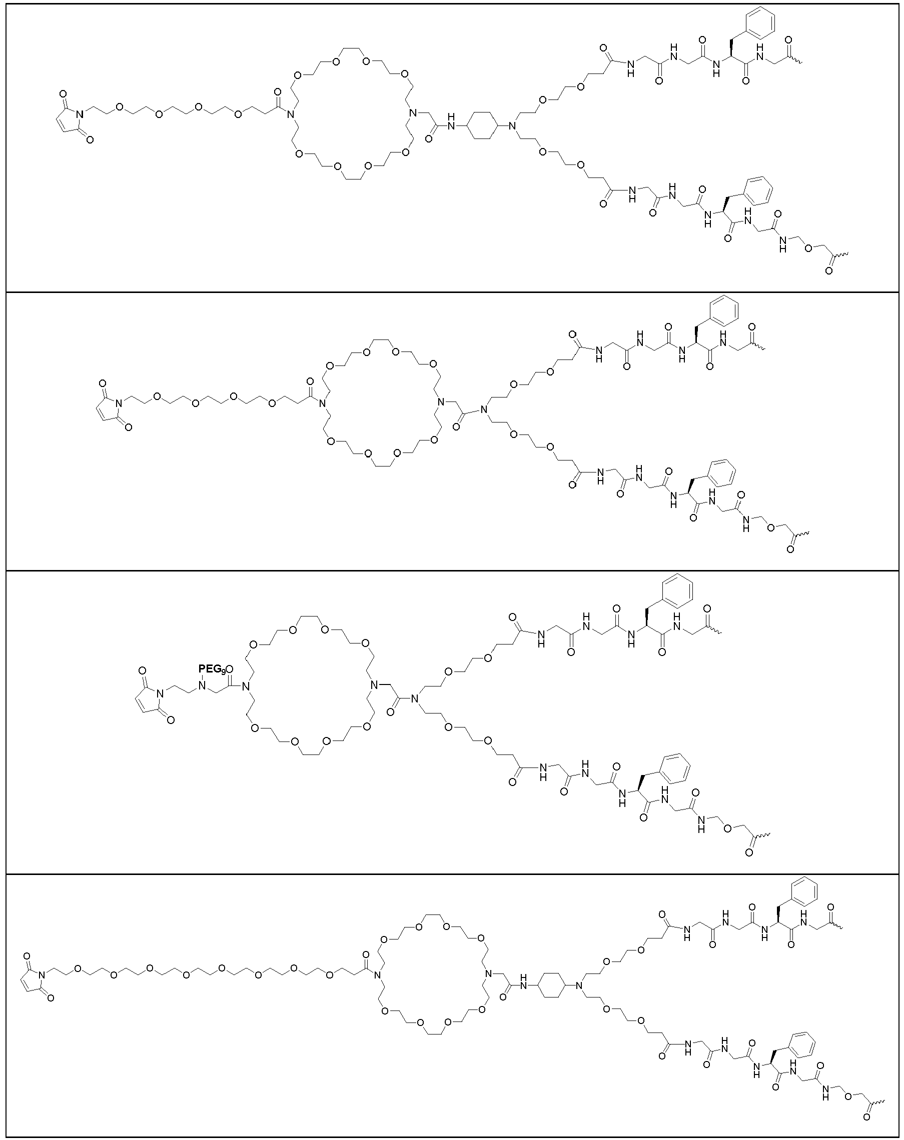 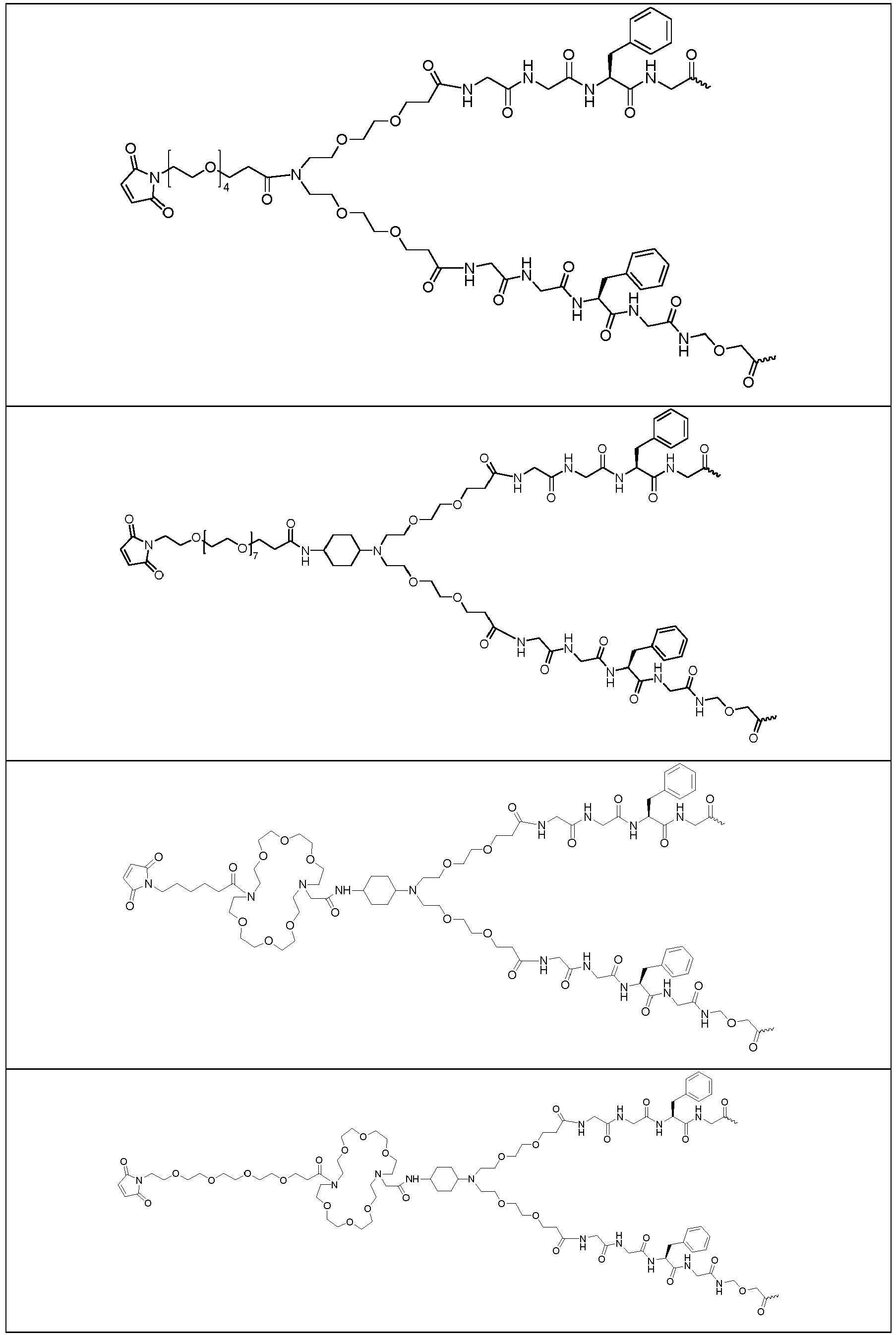 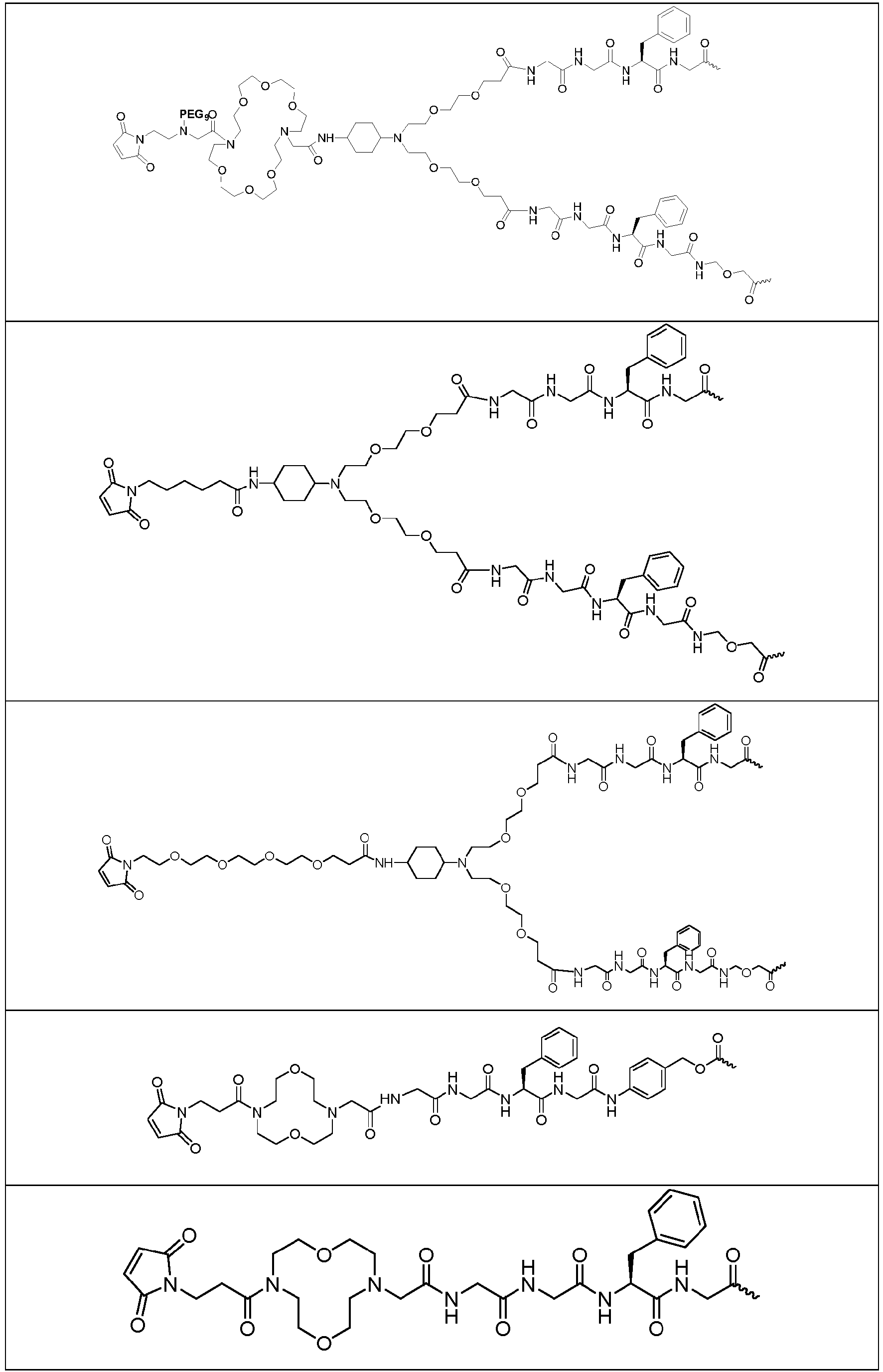 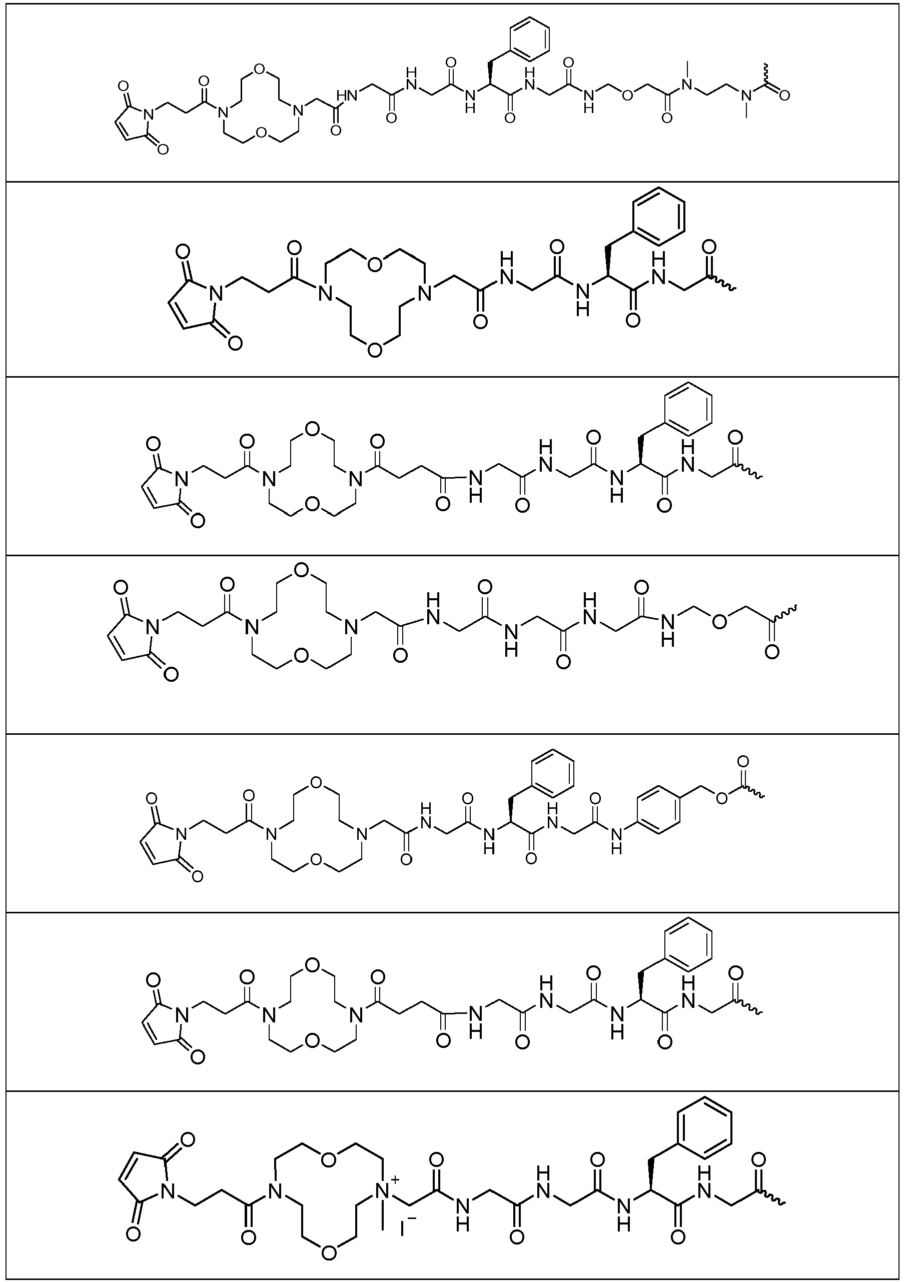 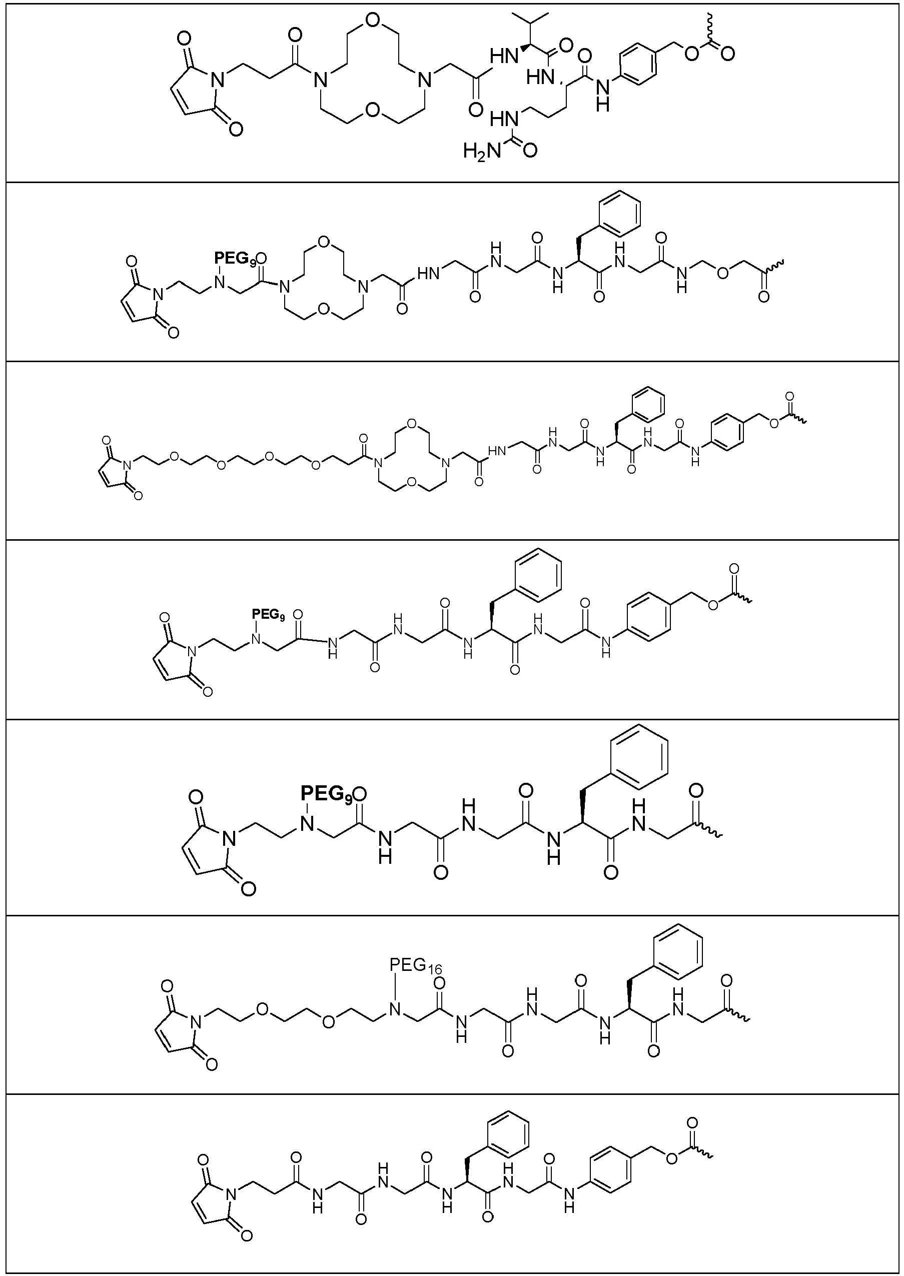 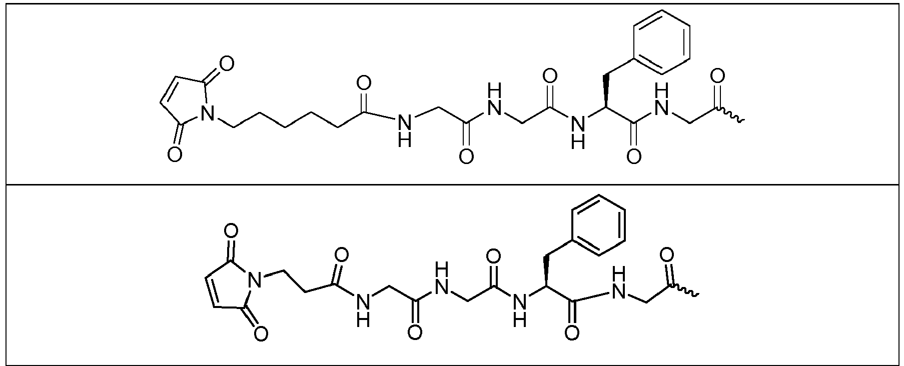
- PEG₉ represents 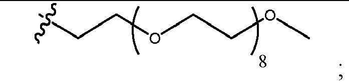 and -PEG₁₆ represents

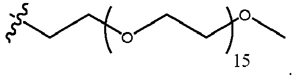

**34.** The drug link assembly unit or the stereoisomer, optical isomer, salt, or deuterated compound thereof according to any one of claims 25-33, **characterized in that**
the structure of the drug link assembly unit is as shown in formula G-4 or G-5: 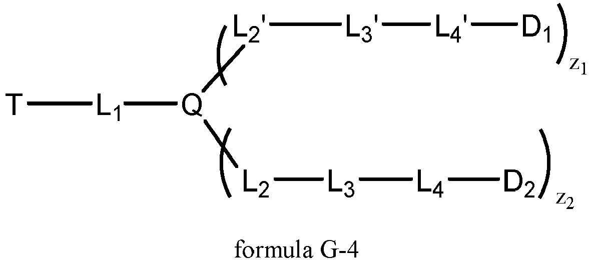 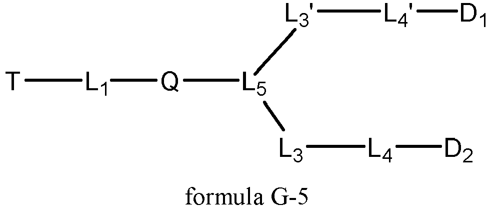
wherein T, Q, L₁, L₂, L₂', L₃, L₃', L₄, L₄', L₅, Z₁, and Z₂ are as described in any one of claims 25-35;
D₁ is a drug; and
D₂ is a drug.

**35.** The drug link assembly unit or the stereoisomer, optical isomer, salt, or deuterated compound thereof according to any one of claims 1-34, **characterized in that** D₁ and D₂ are independently selected from a cytotoxic drug, a drug for treating autoimmune toxicity, or an anti-inflammatory drug;
preferably, D₁ and D₂ are independently selected from a drug targeting DNA or a drug targeting a microtubule protein; and
more preferably, D₁ and D₂ are independently selected from one of the following compounds or derivatives thereof

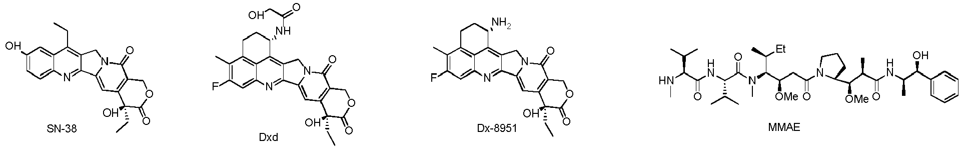

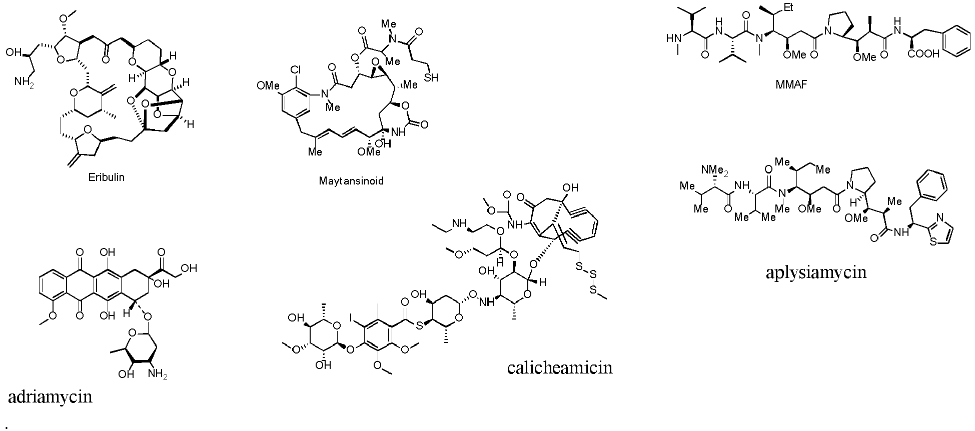

**36.** The drug link assembly unit or the stereoisomer, optical isomer, salt, or deuterated compound thereof according to any one of claims 1-35, **characterized in that** the structure of the drug link assembly unit is selected from: 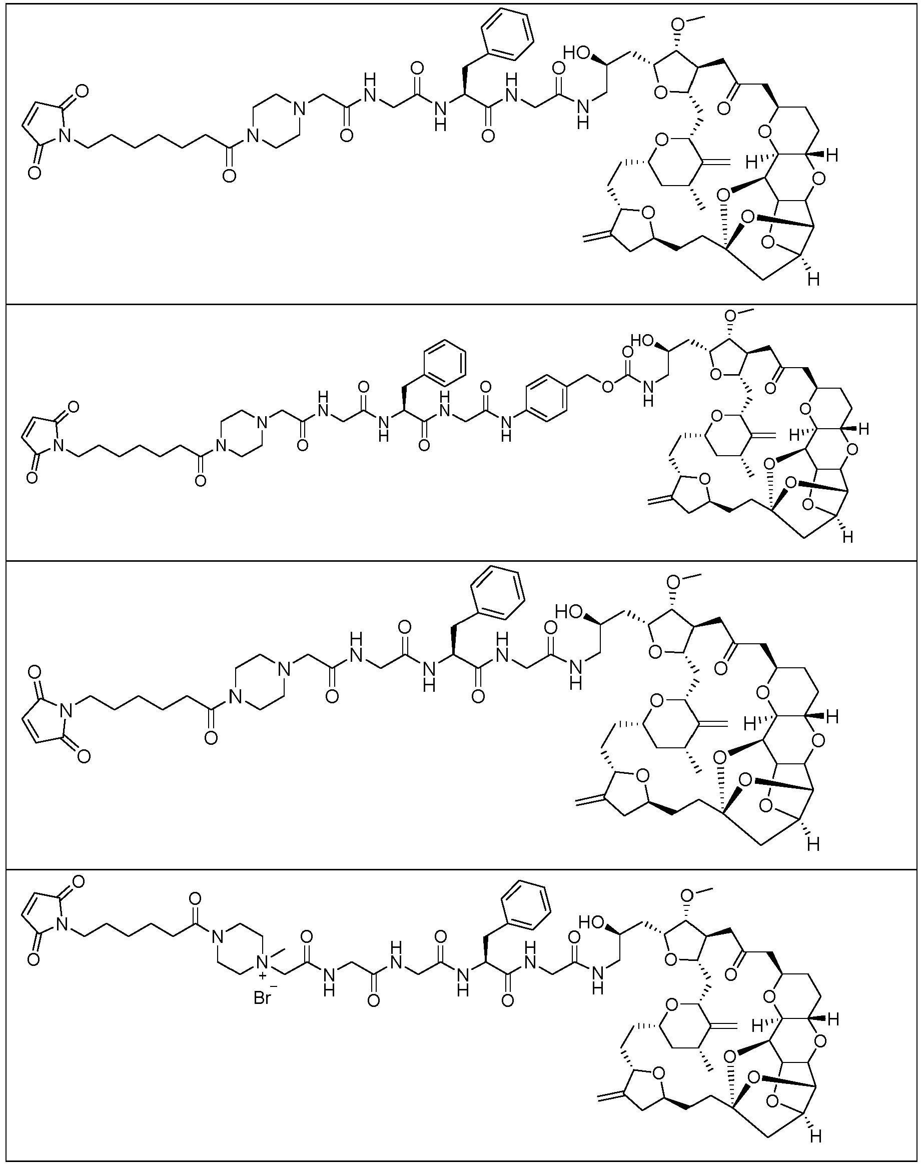 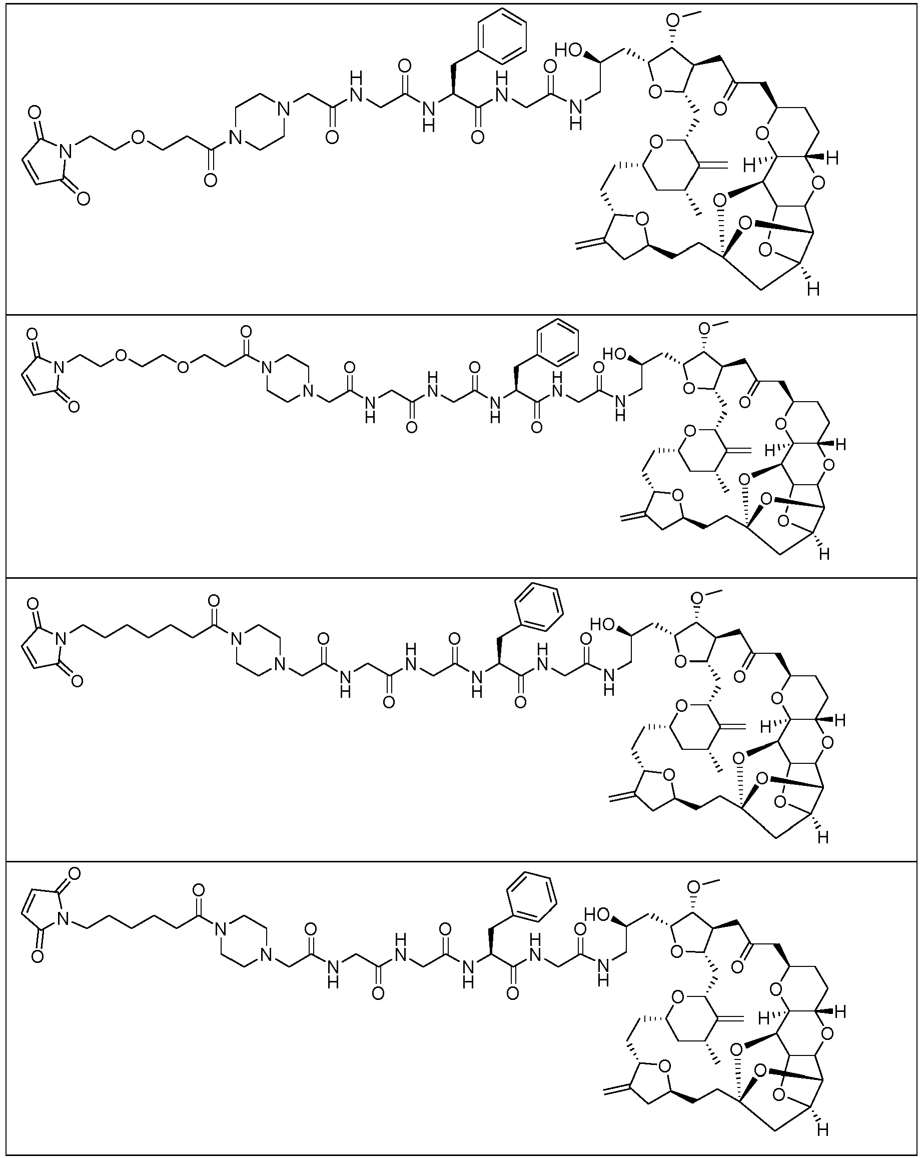 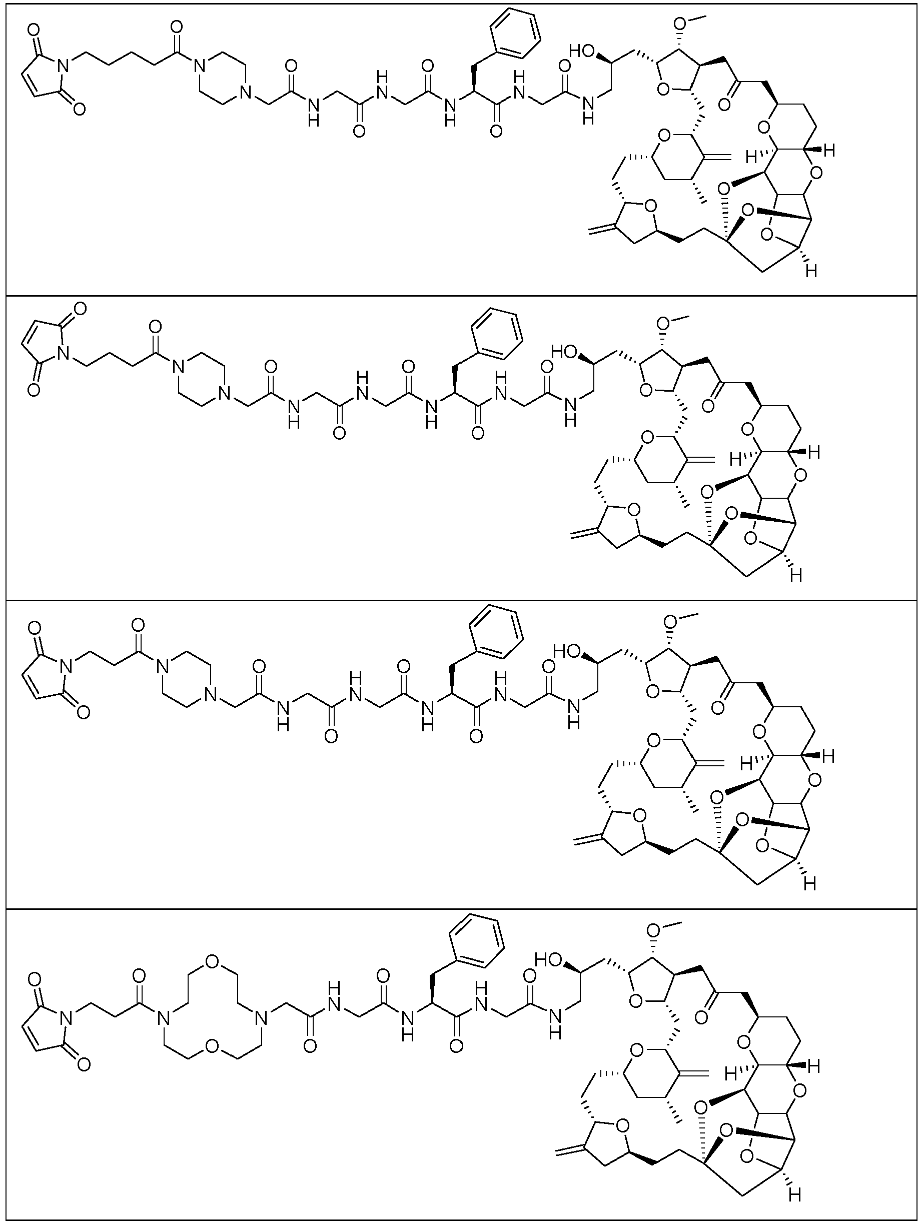 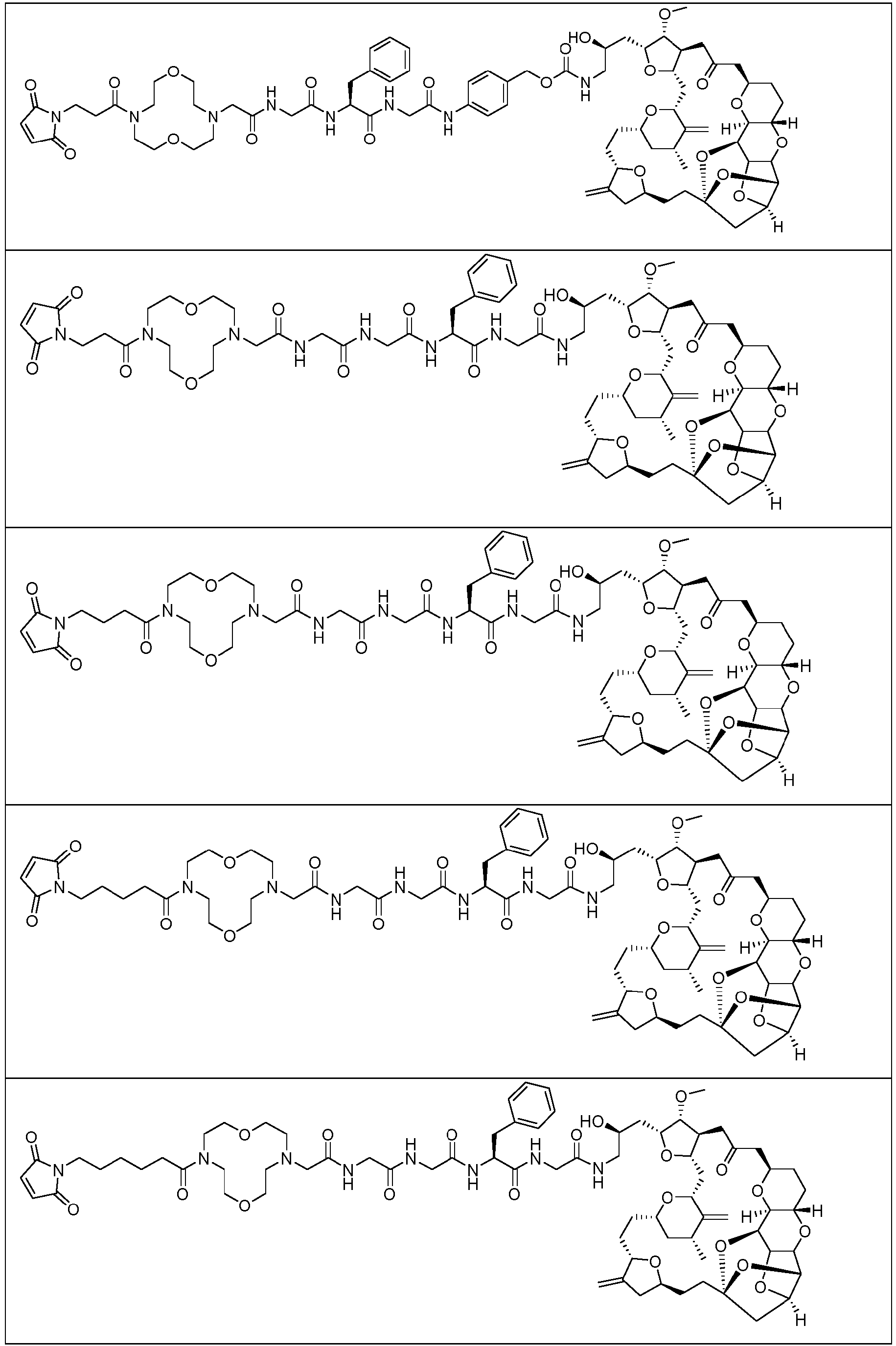 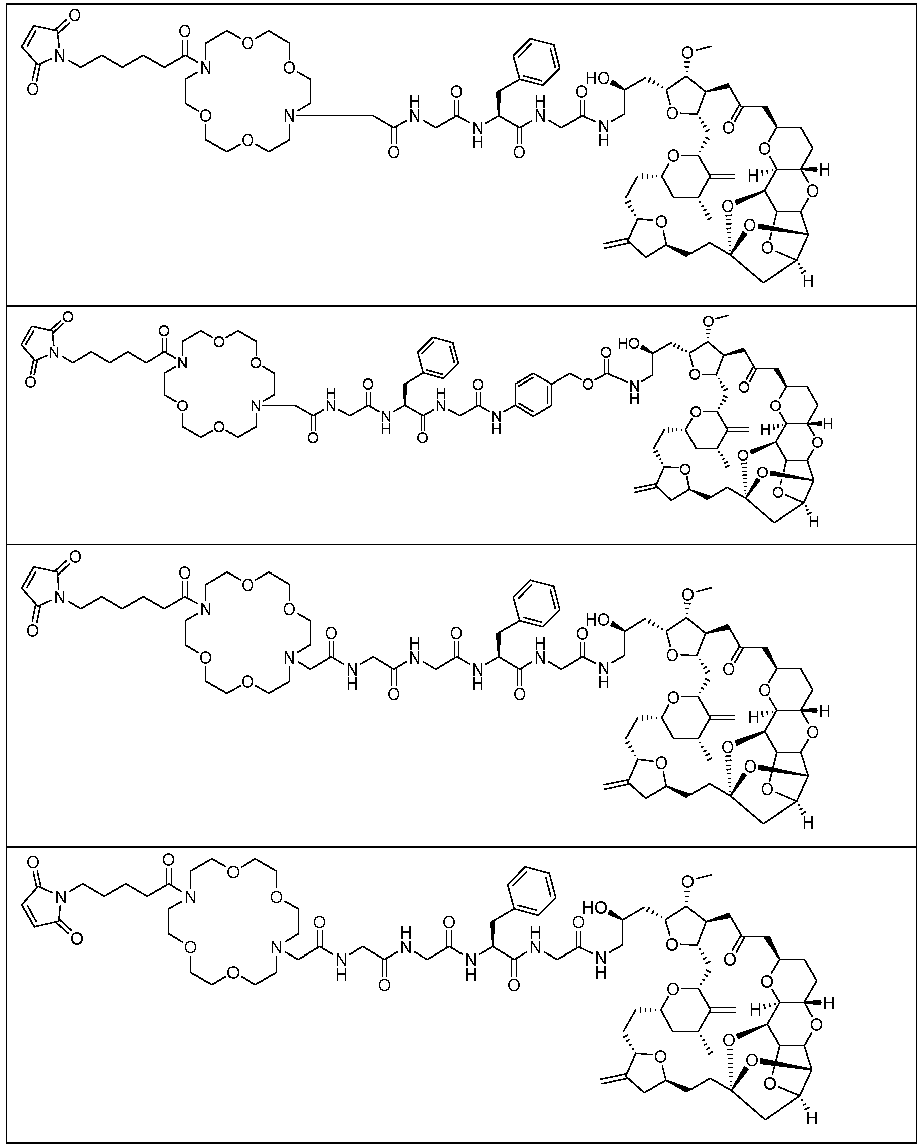 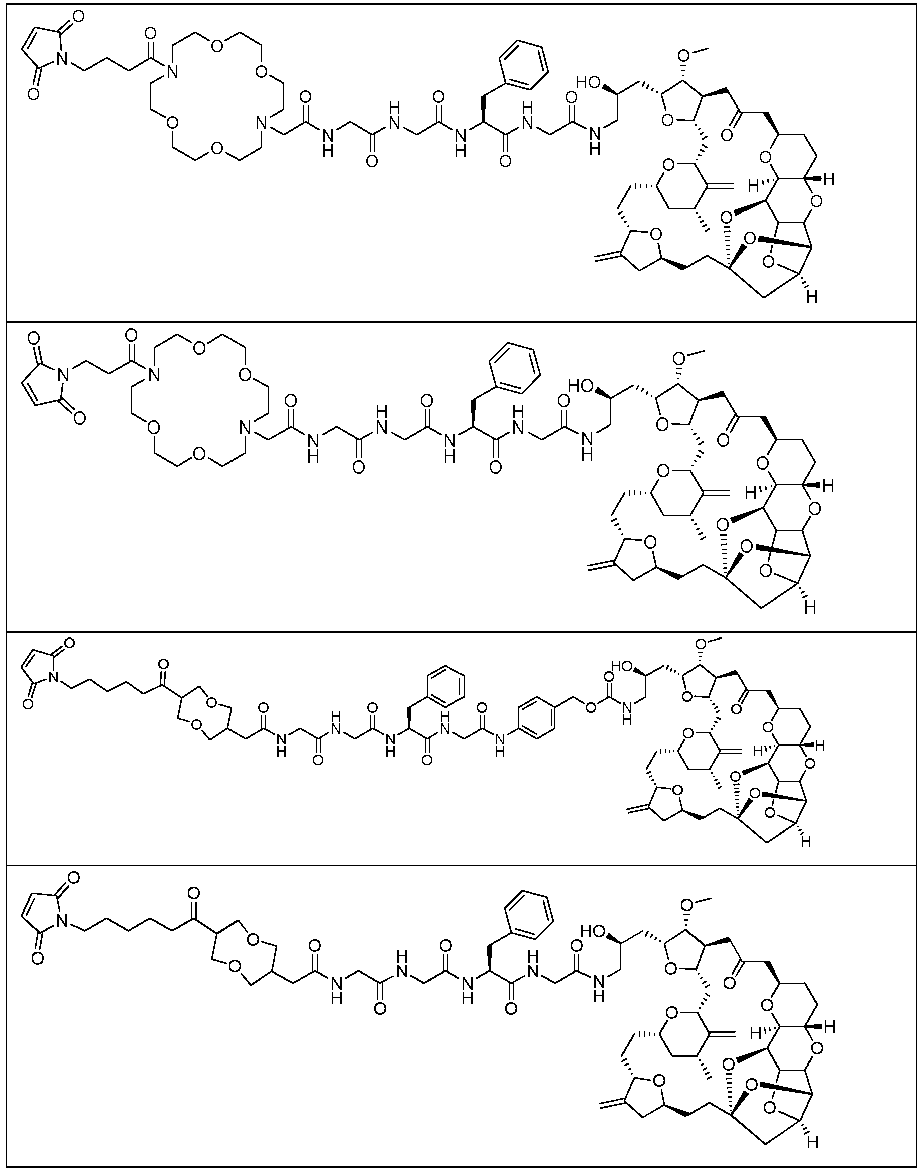 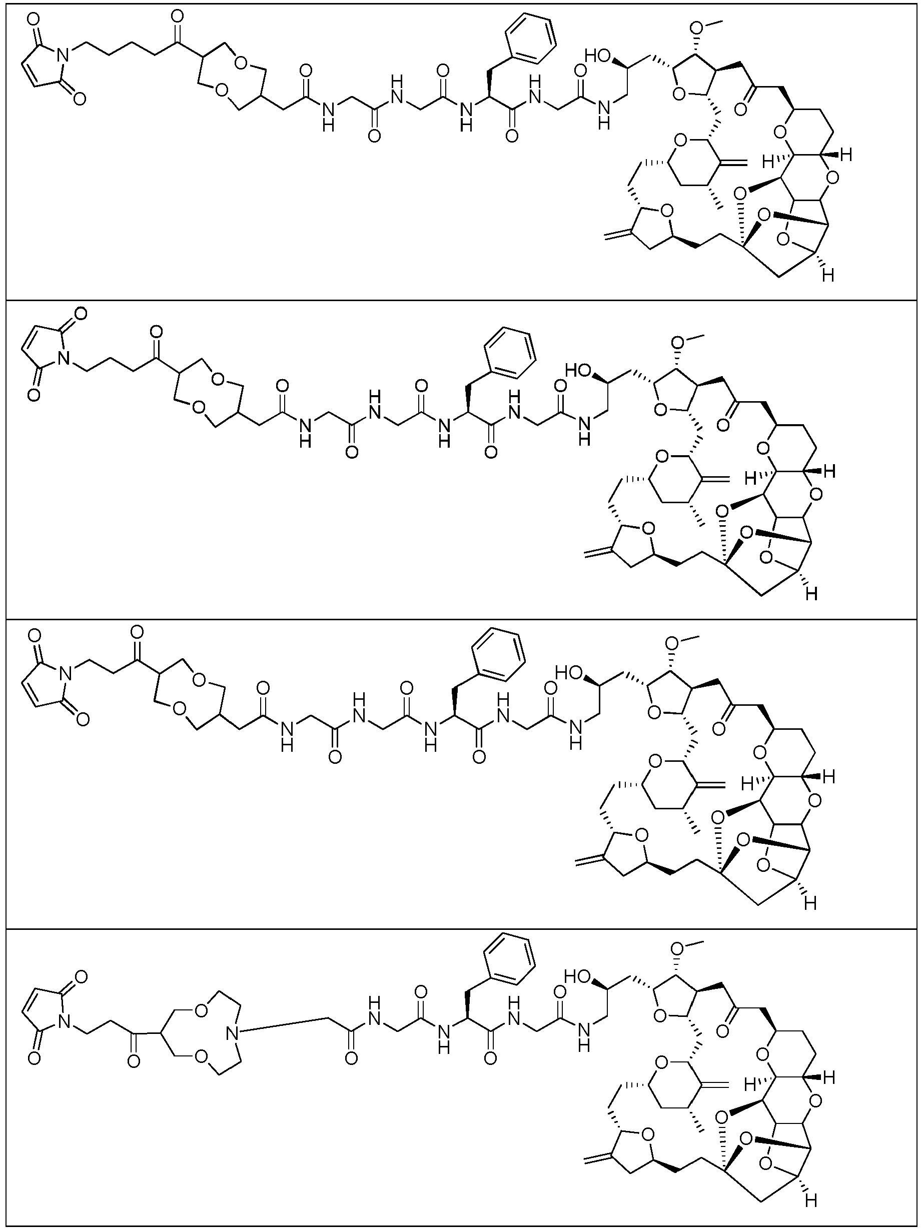 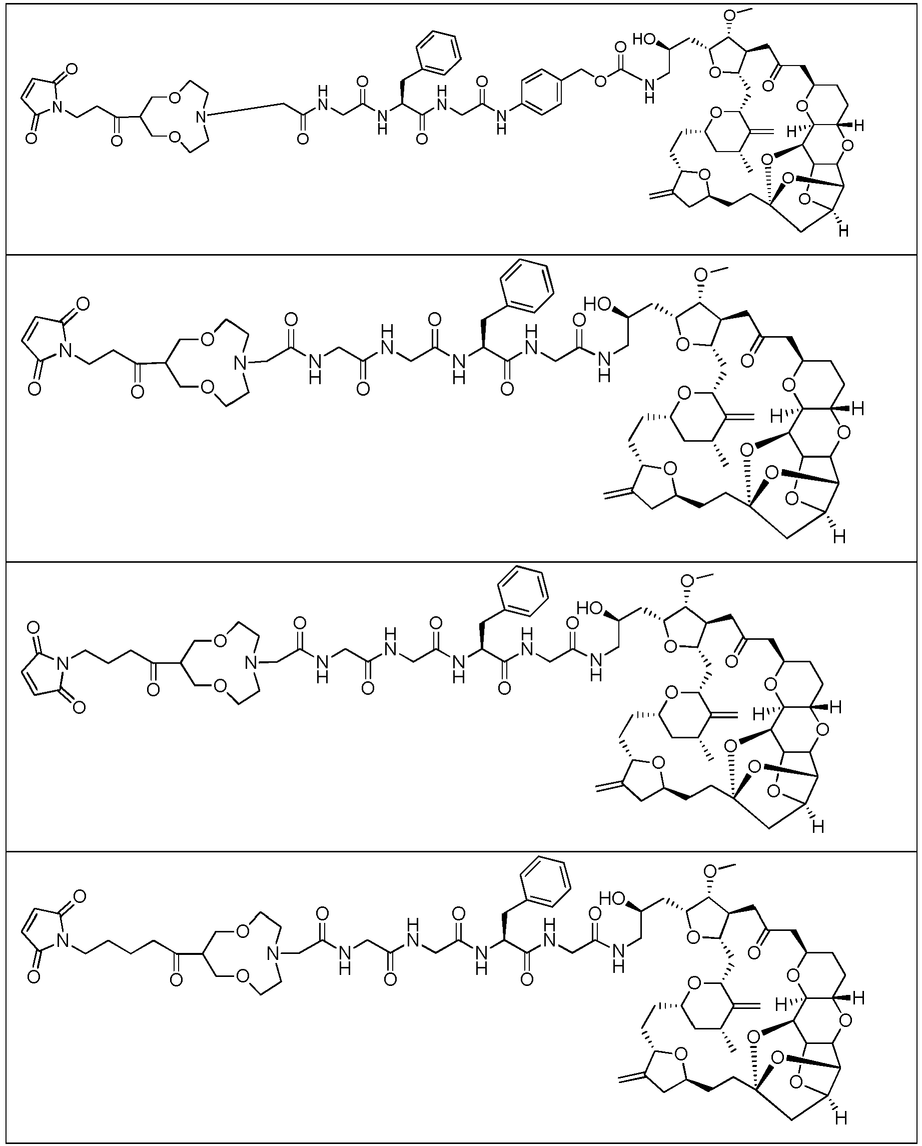 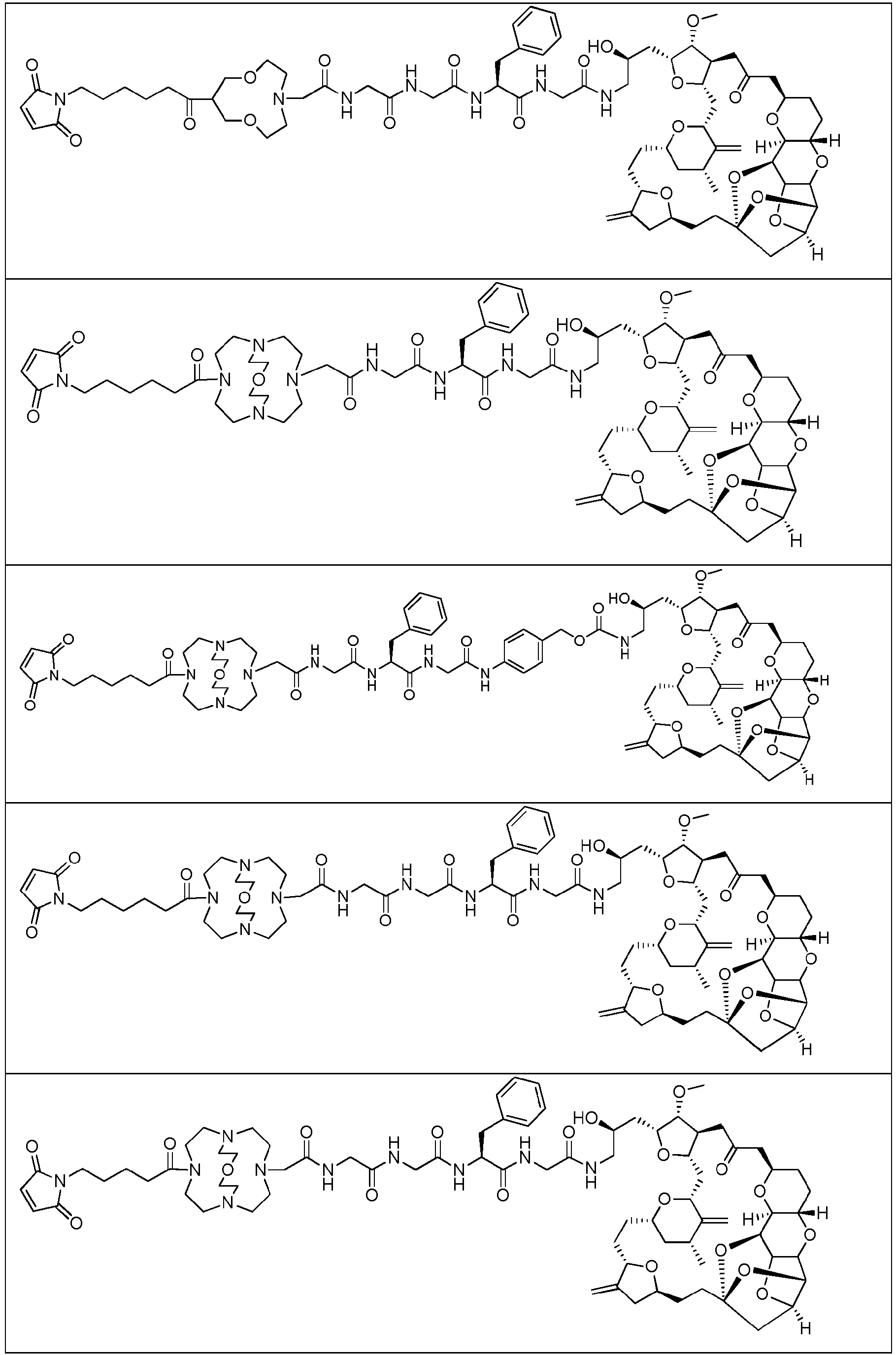 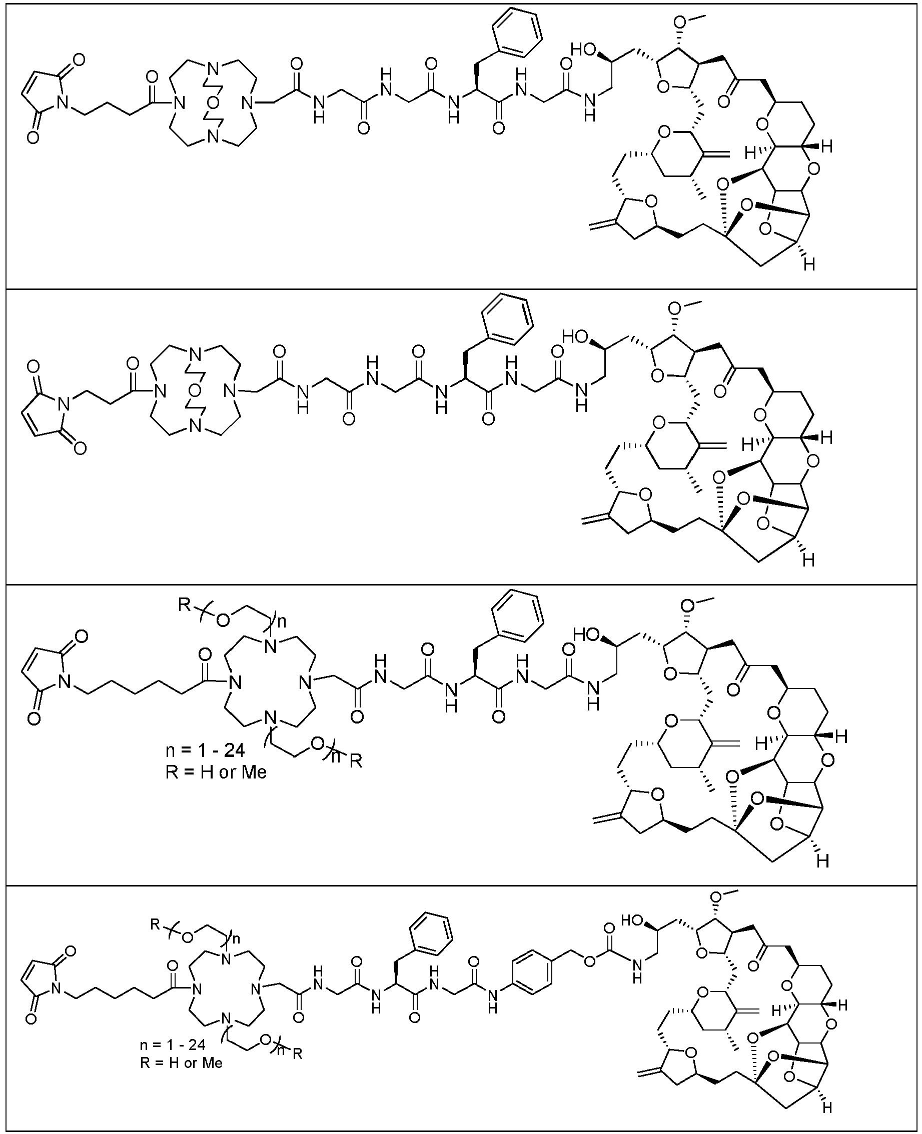 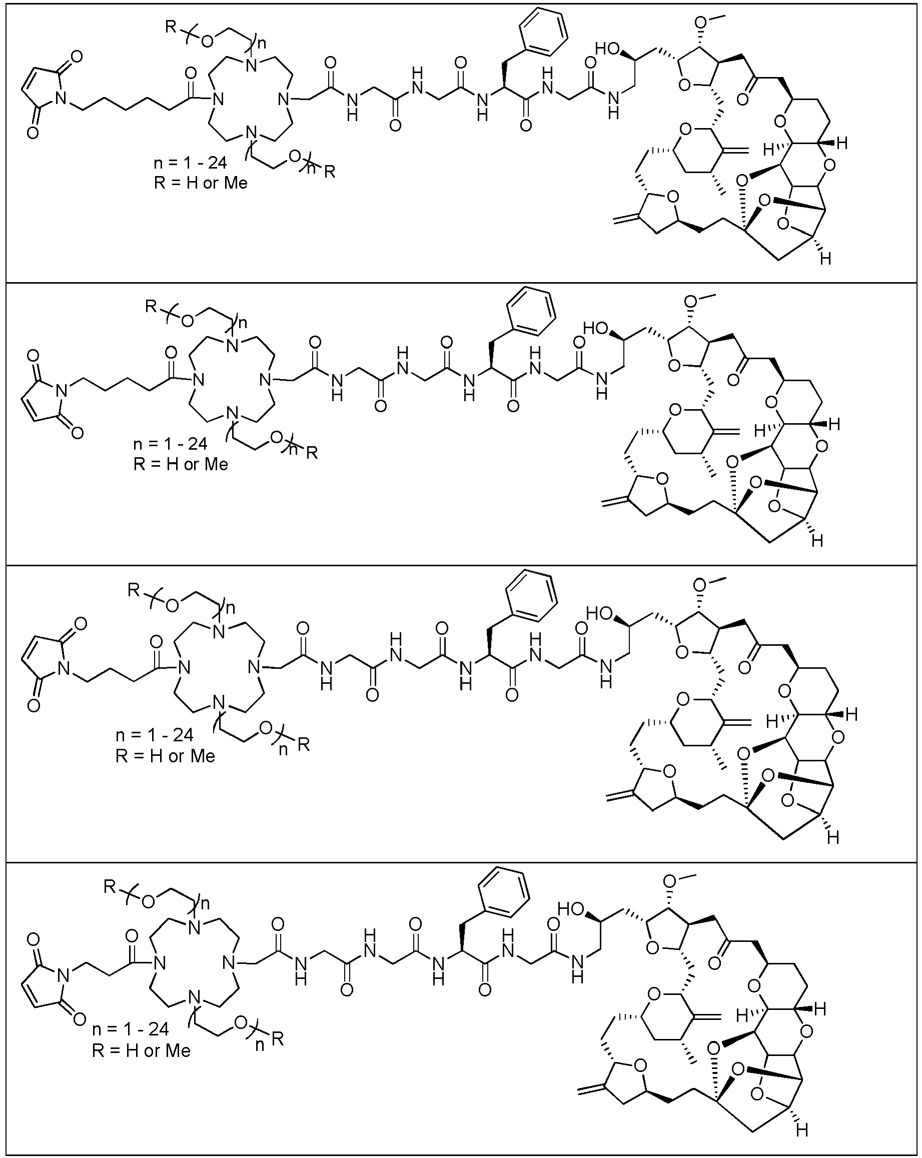 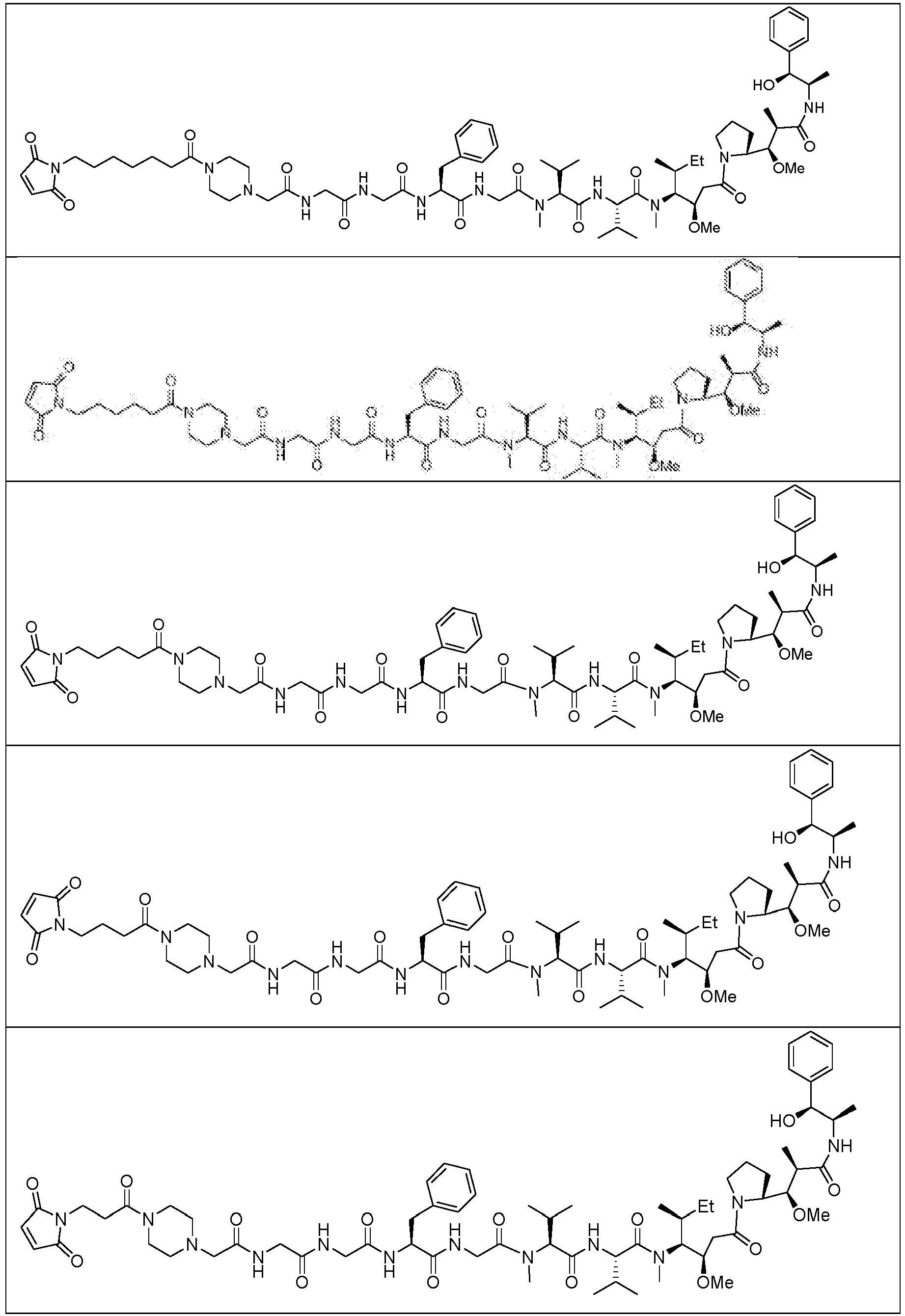 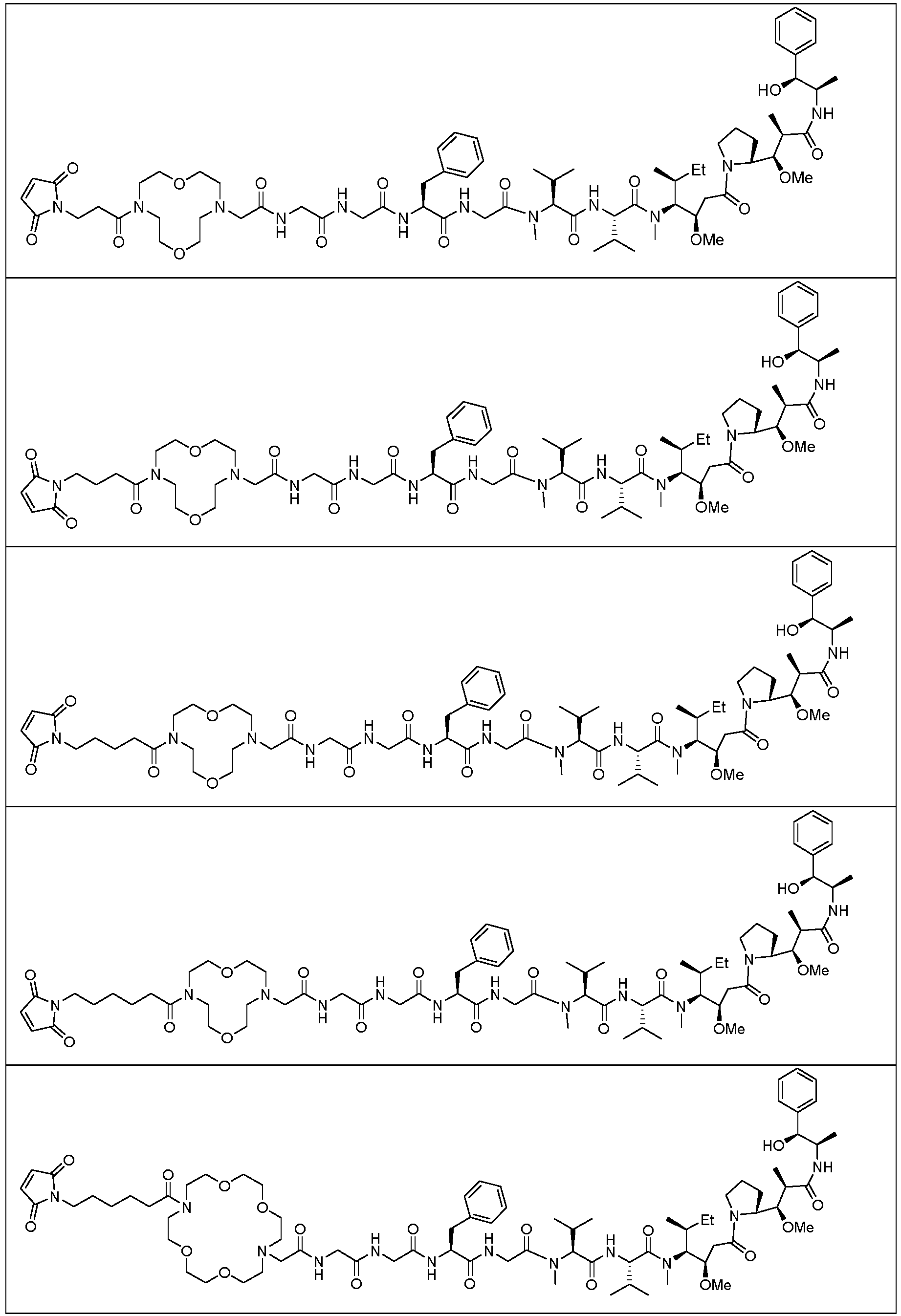 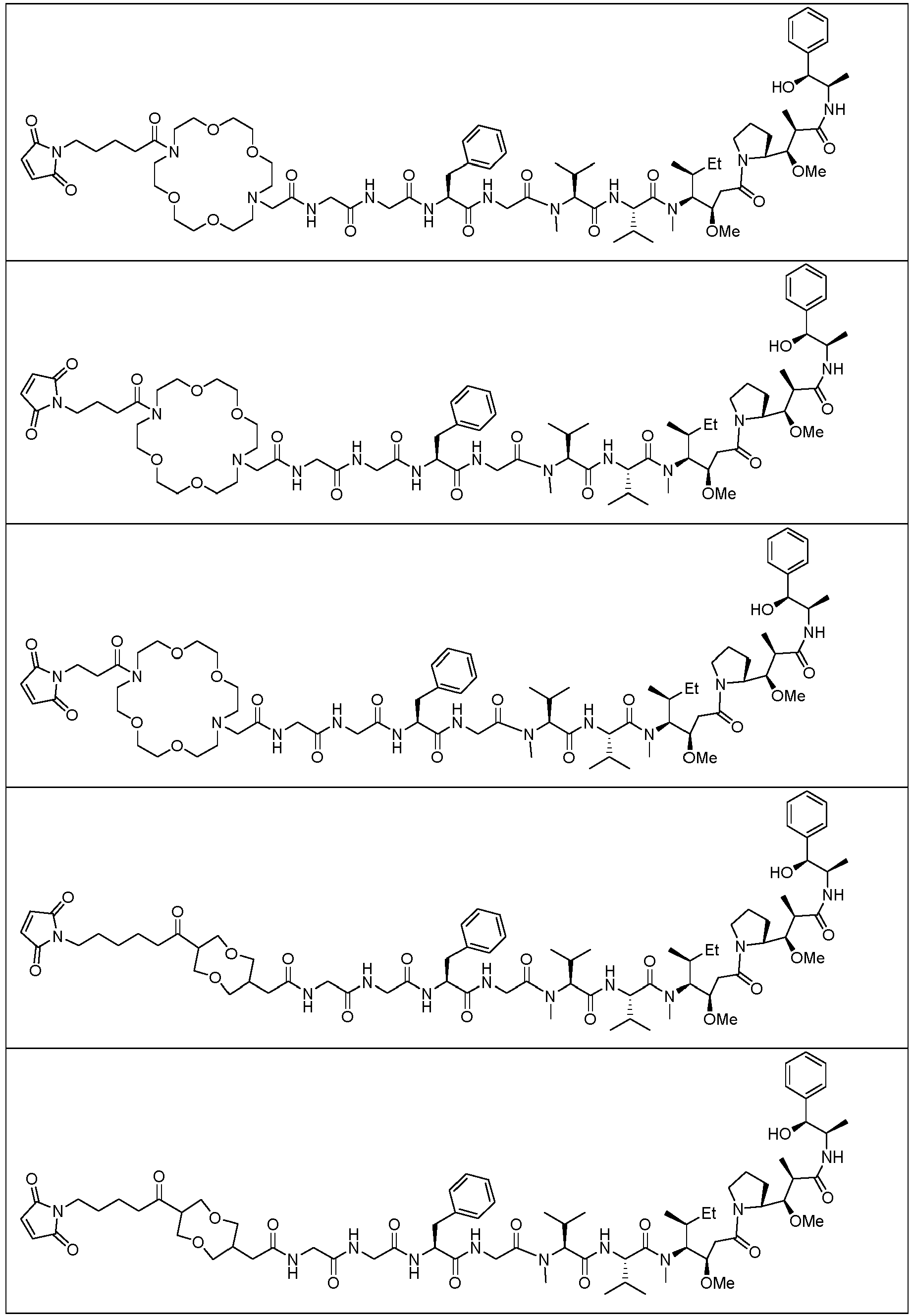 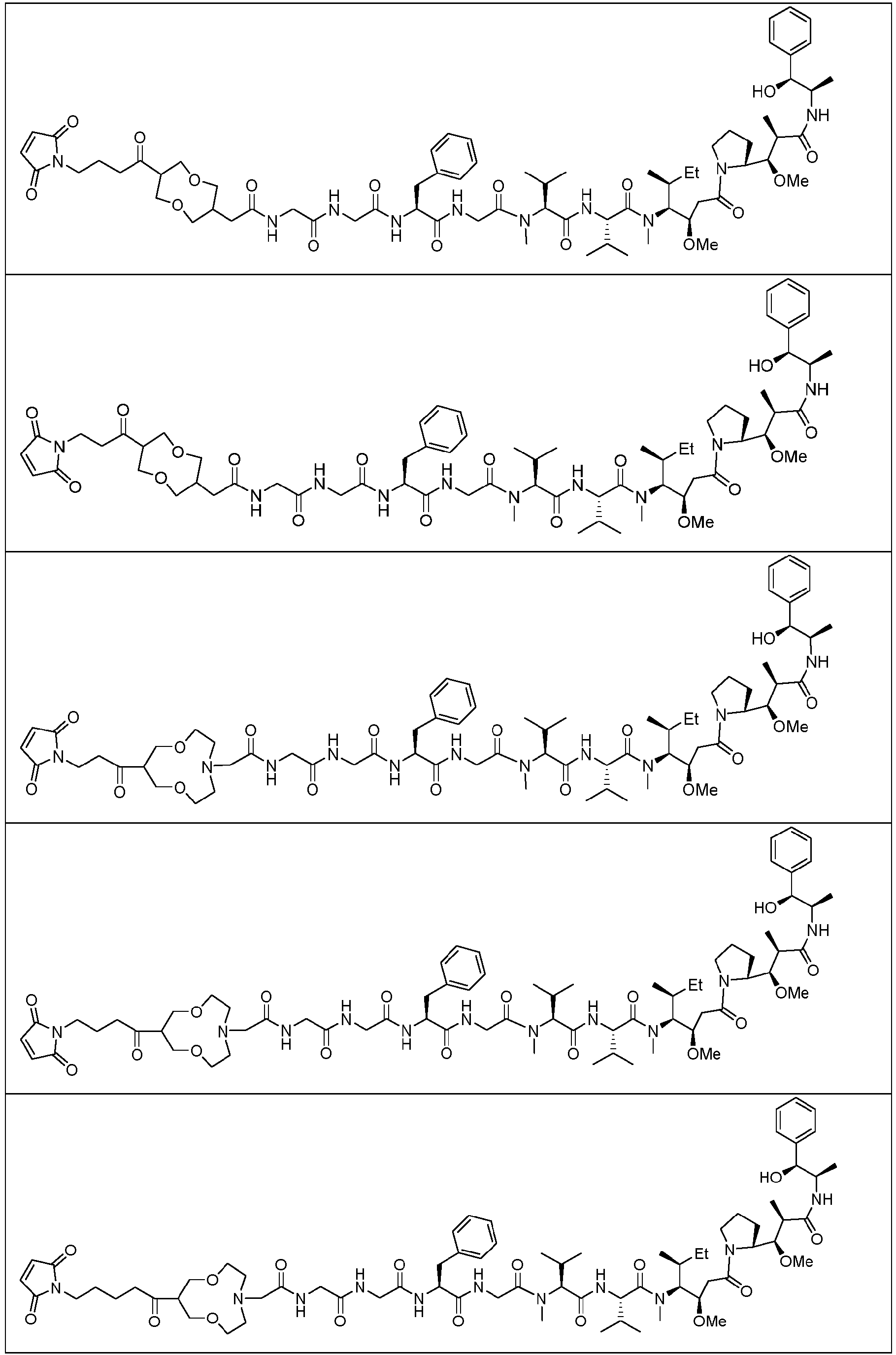 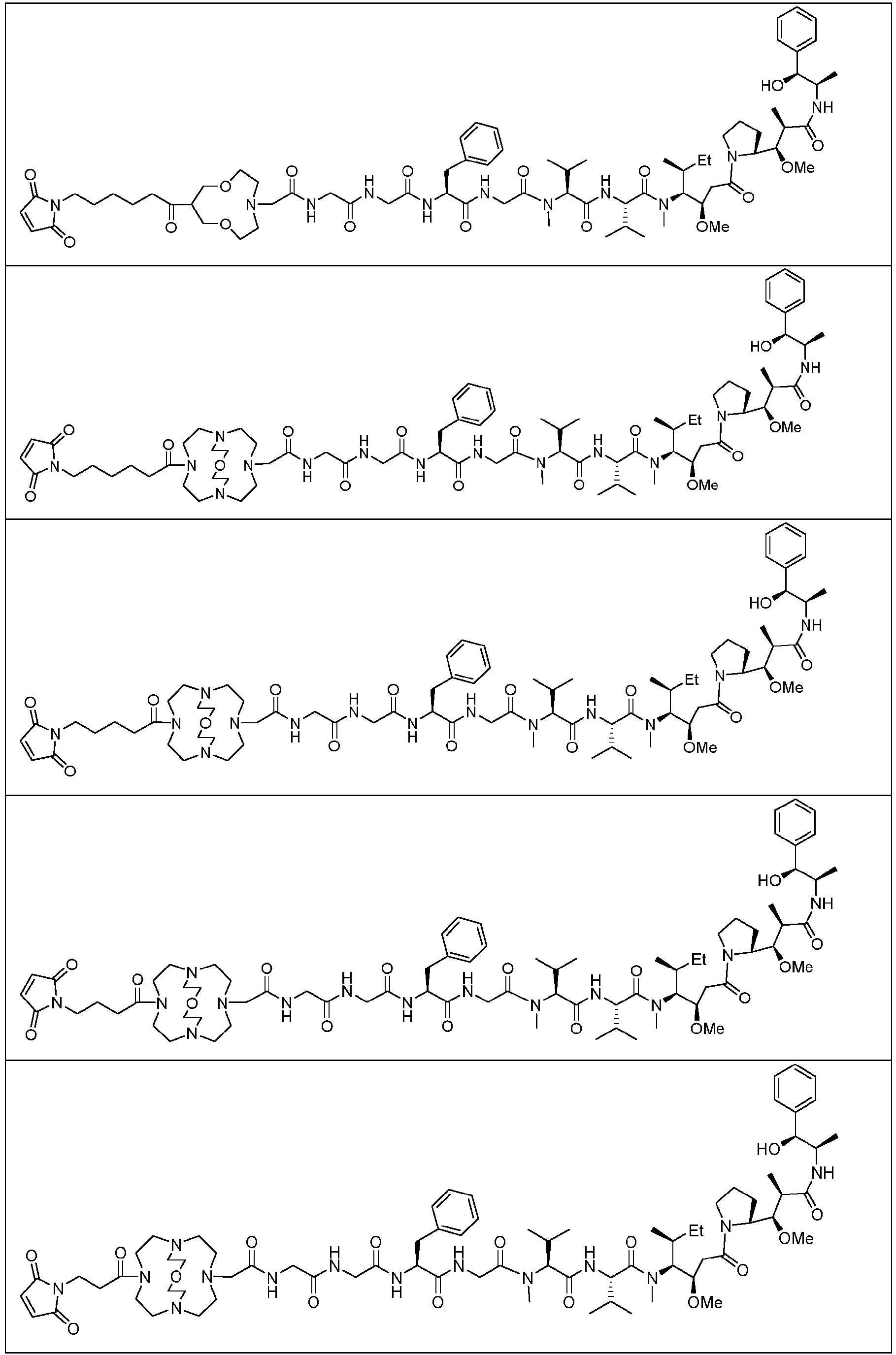 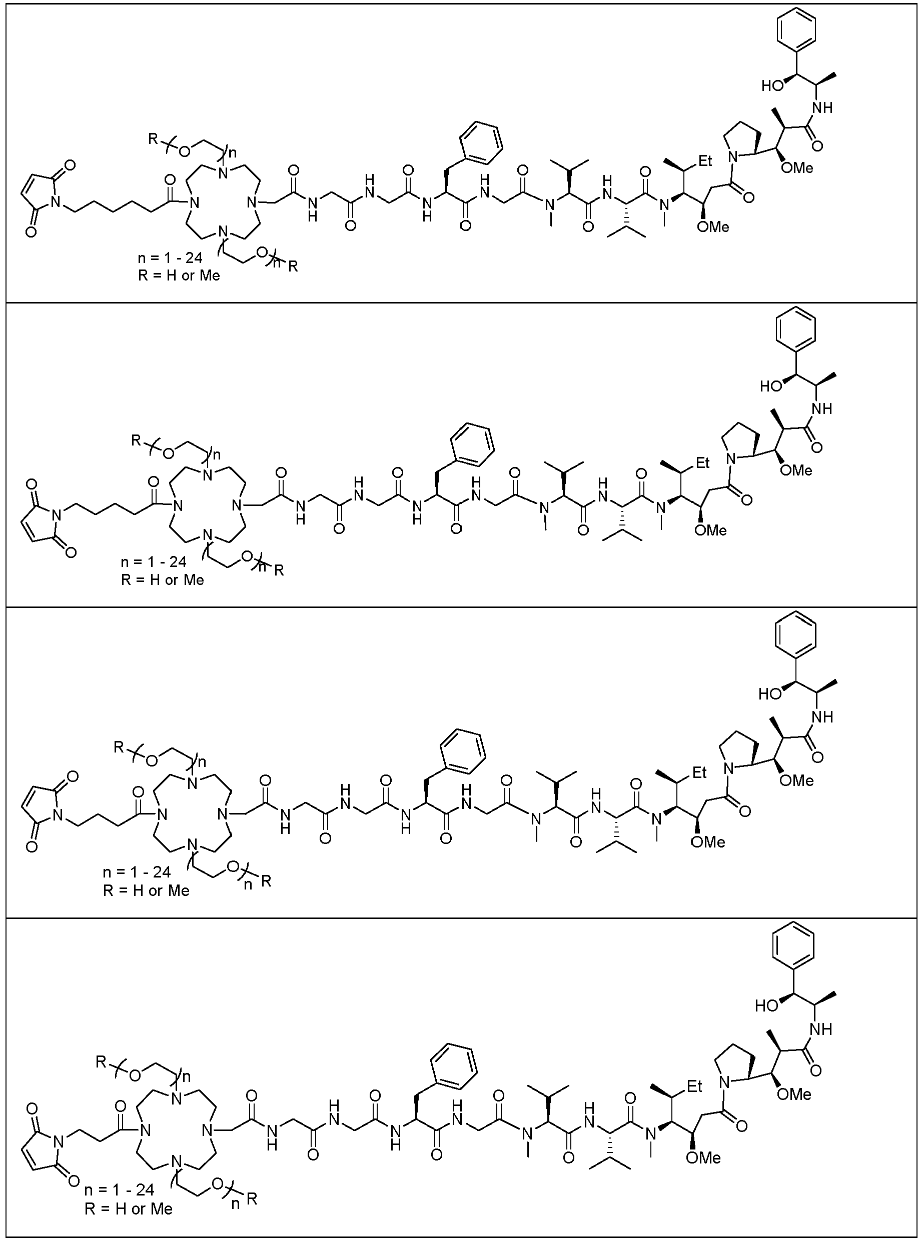 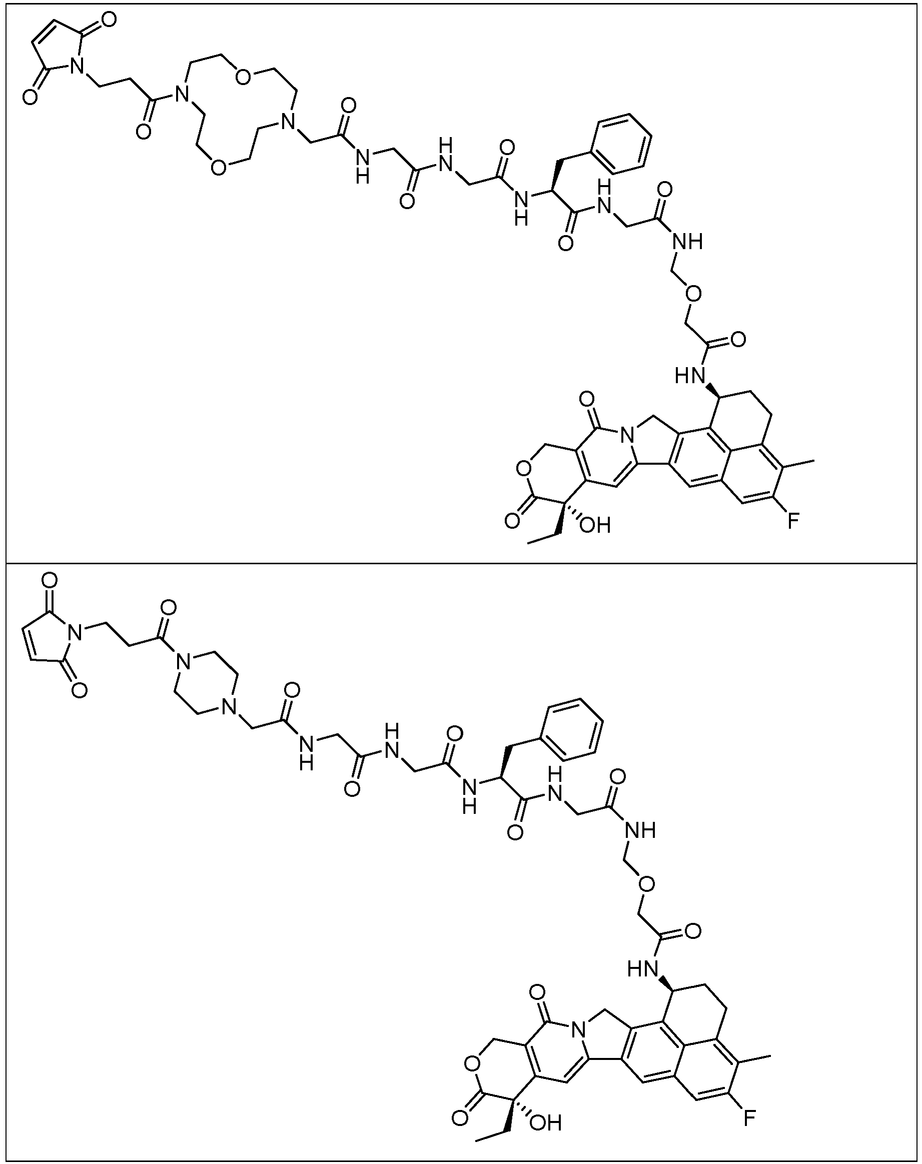 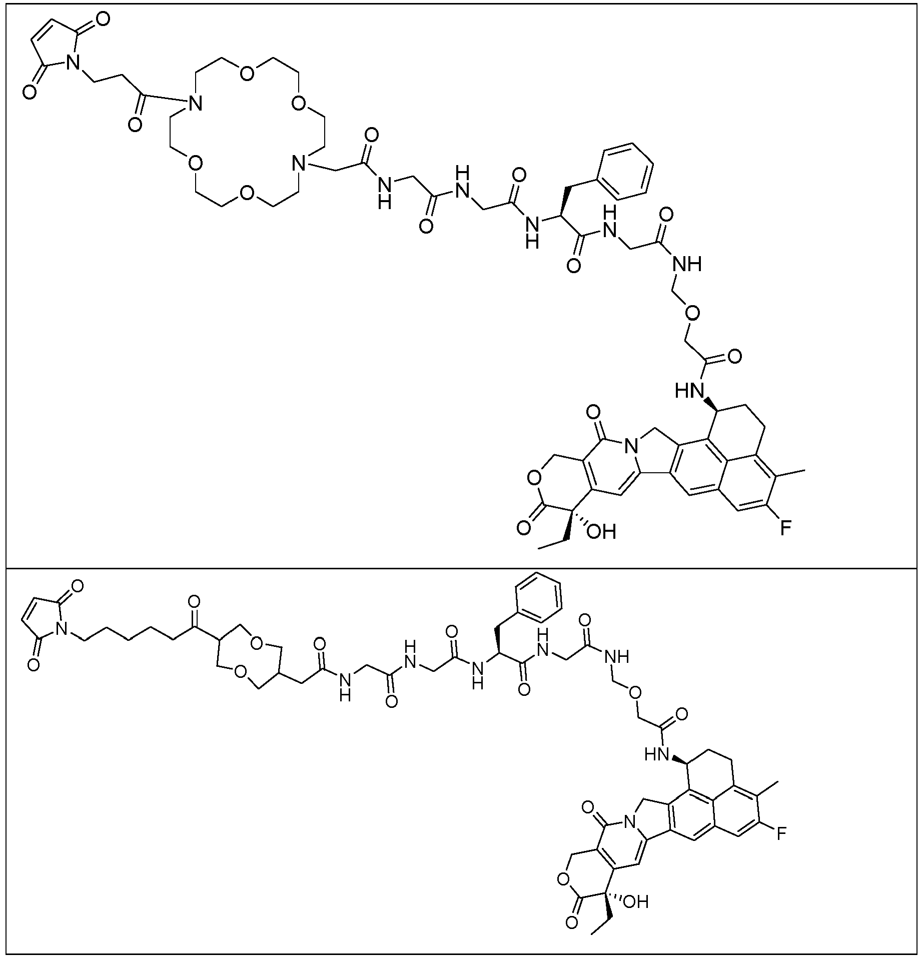 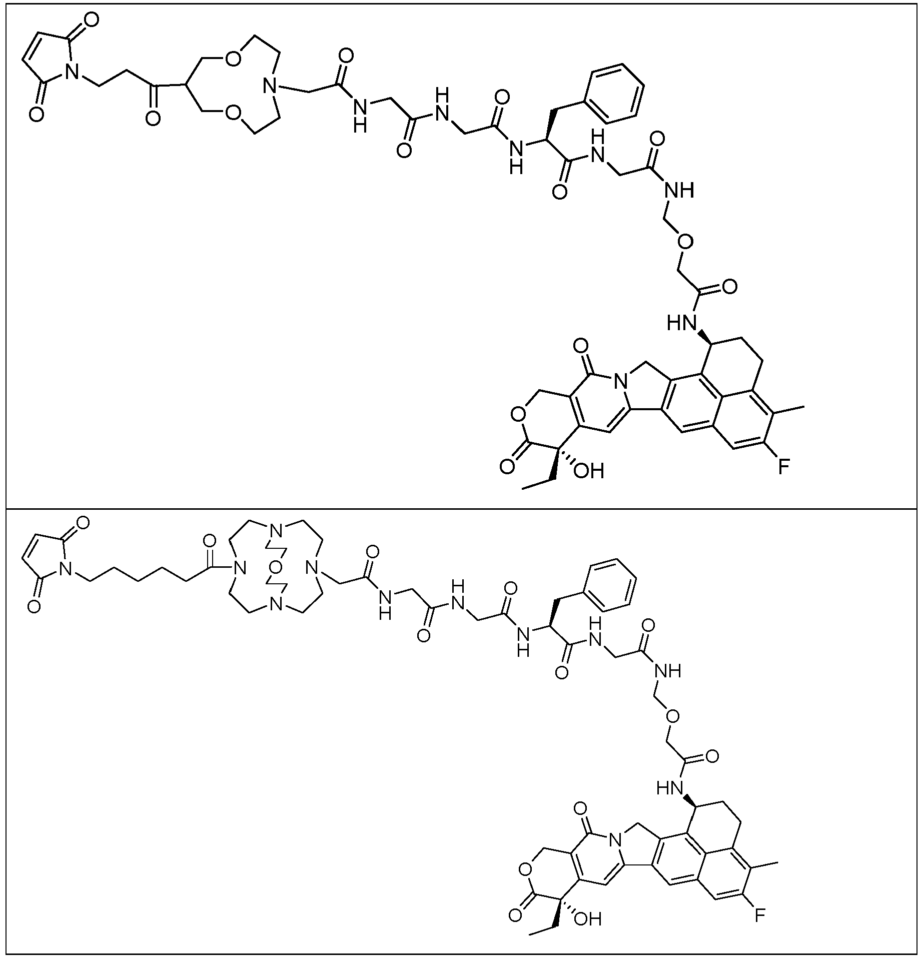 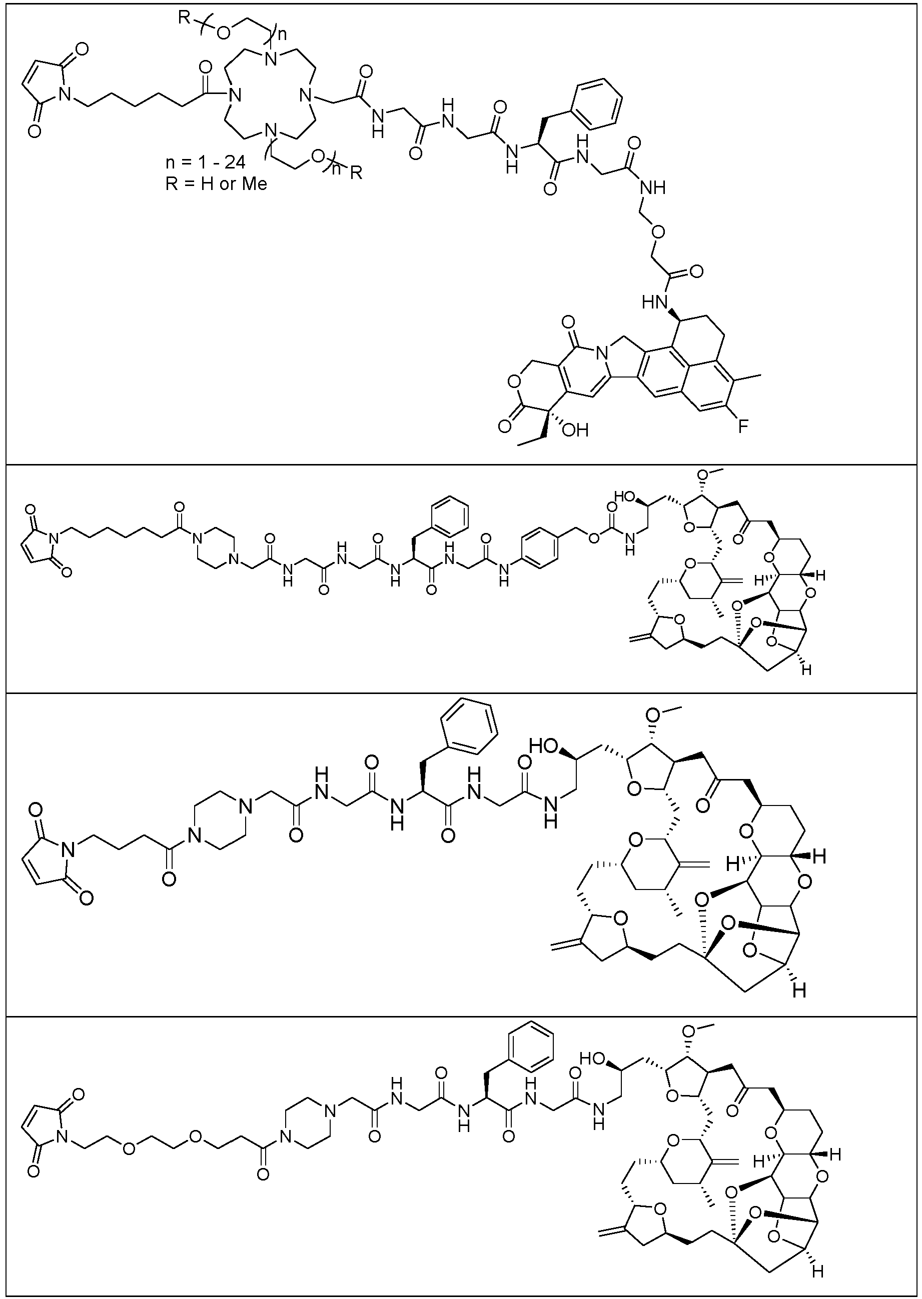 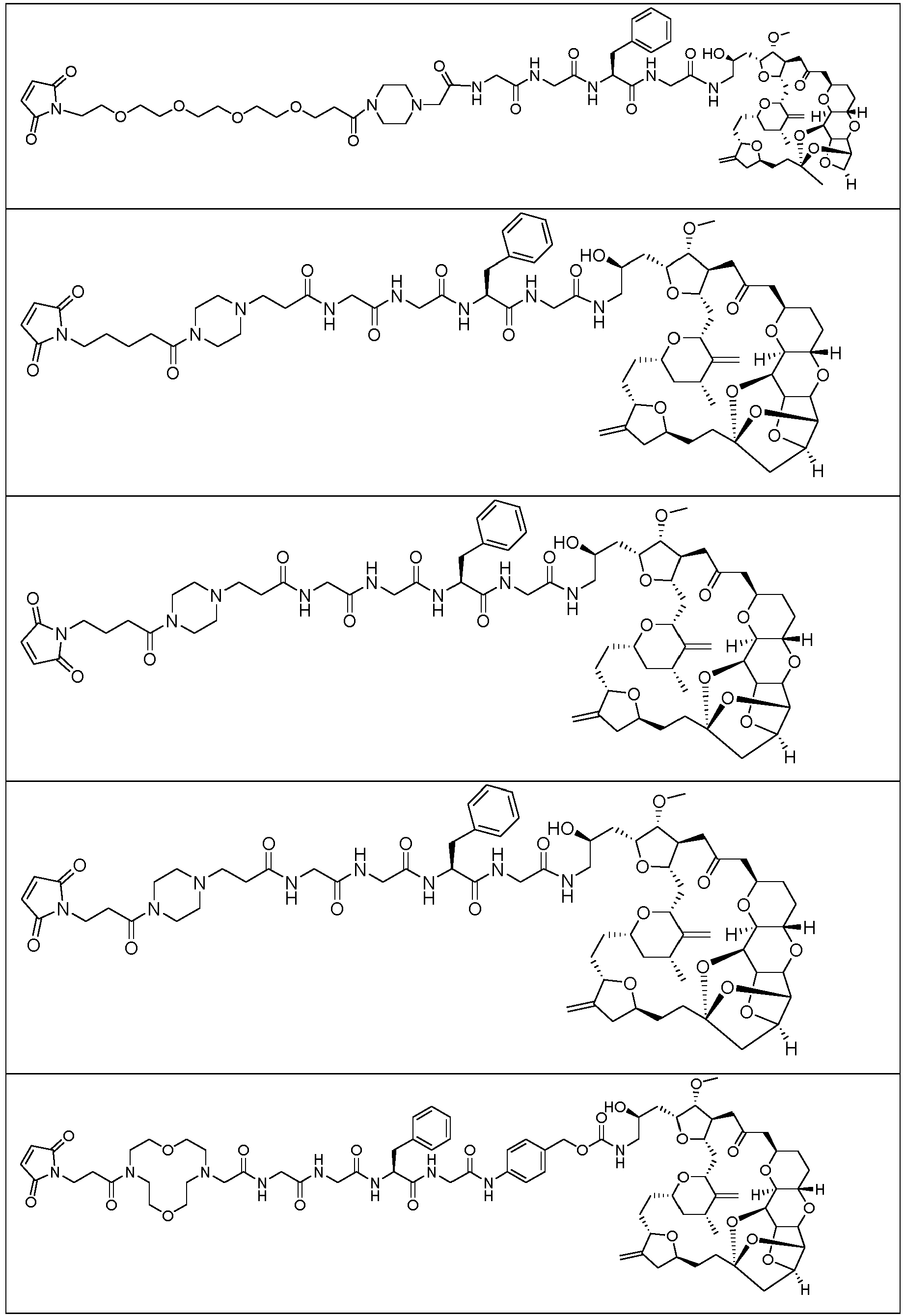 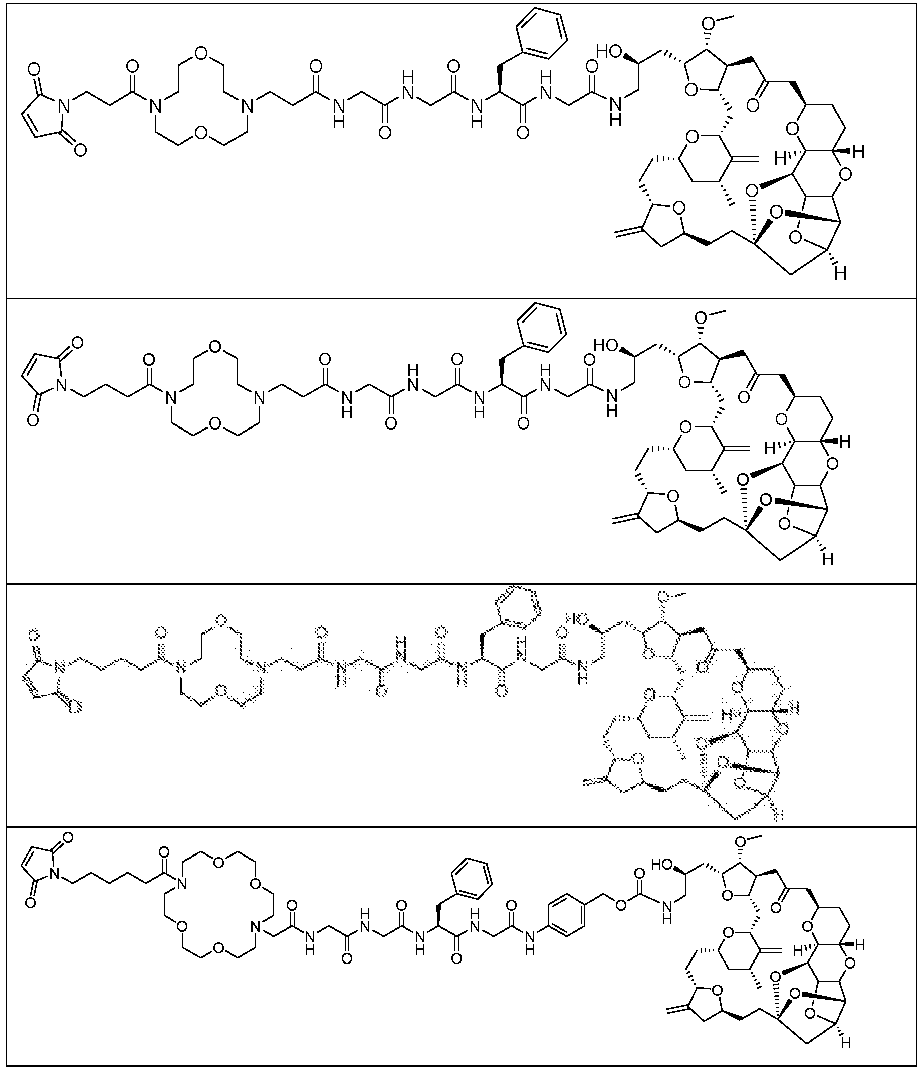 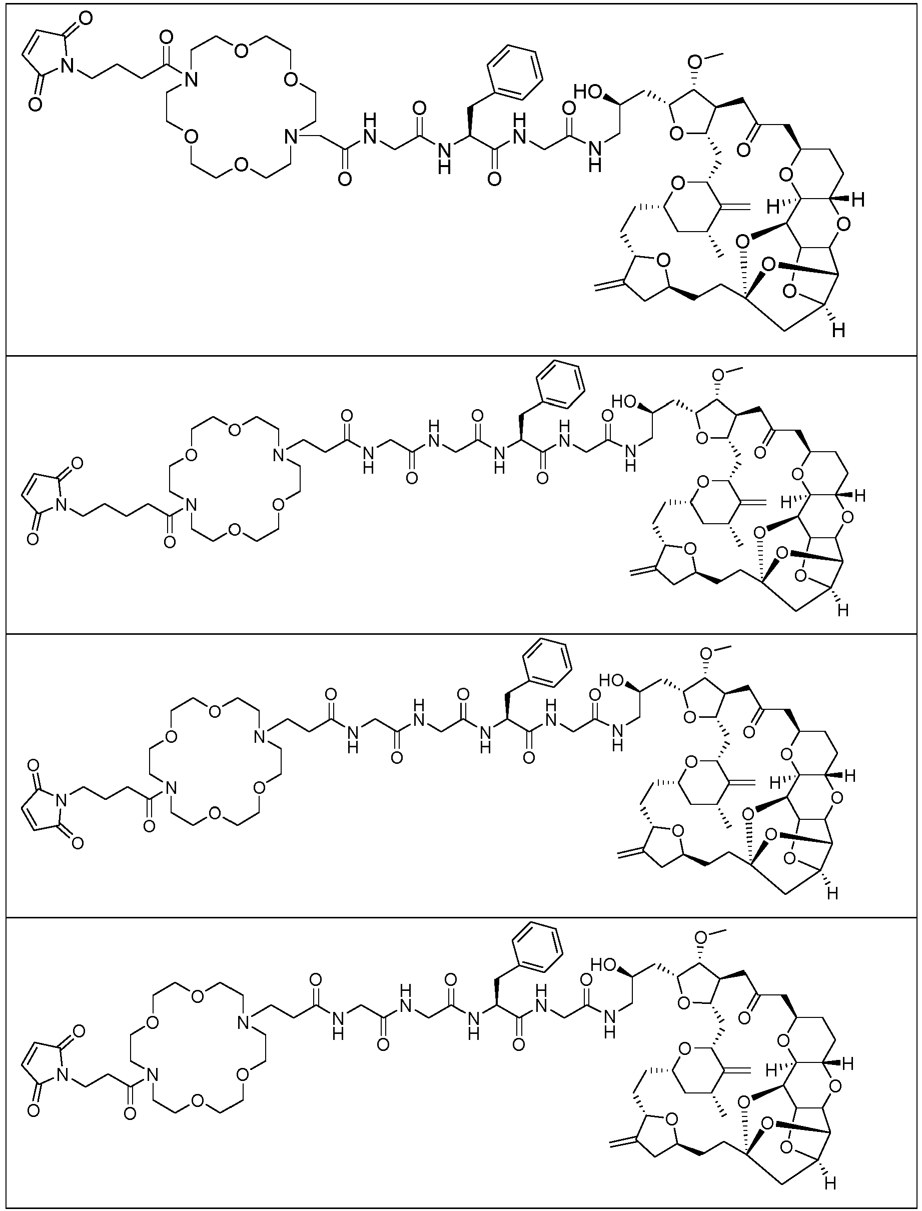 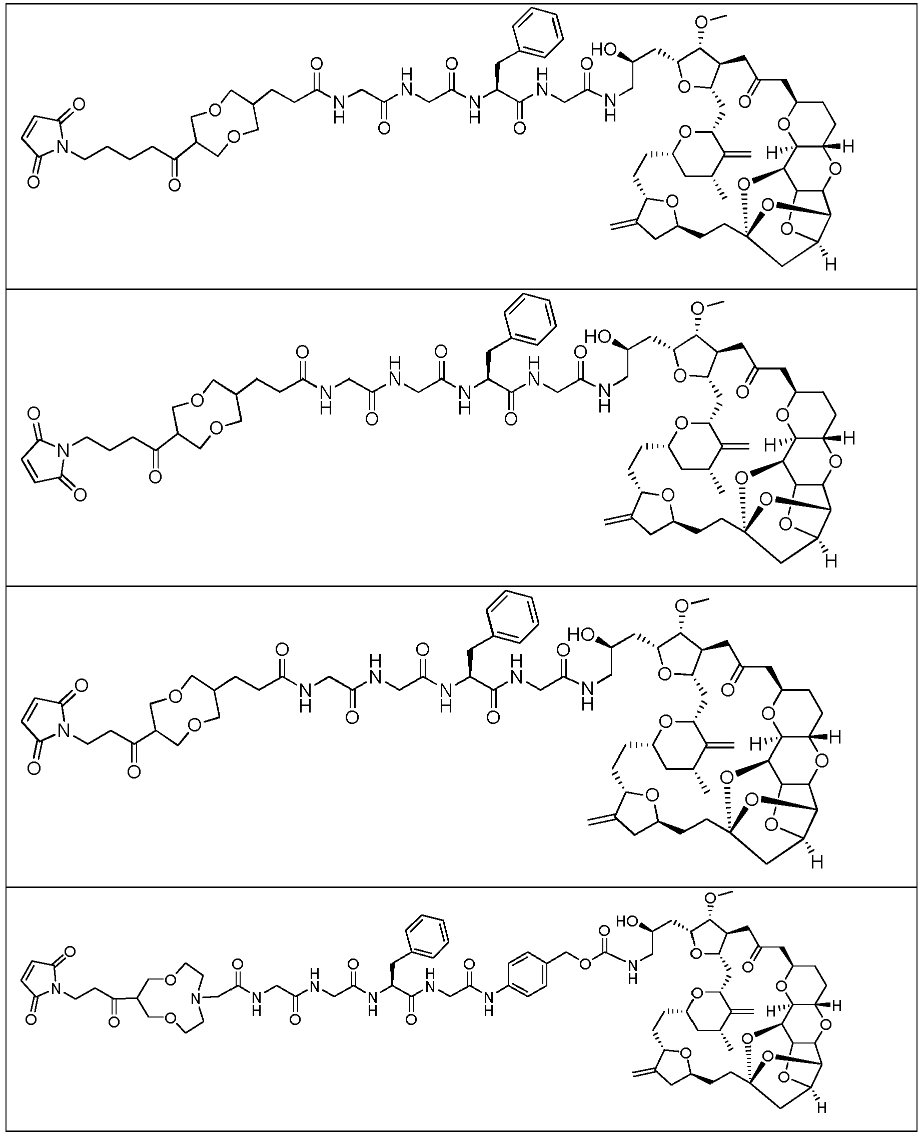 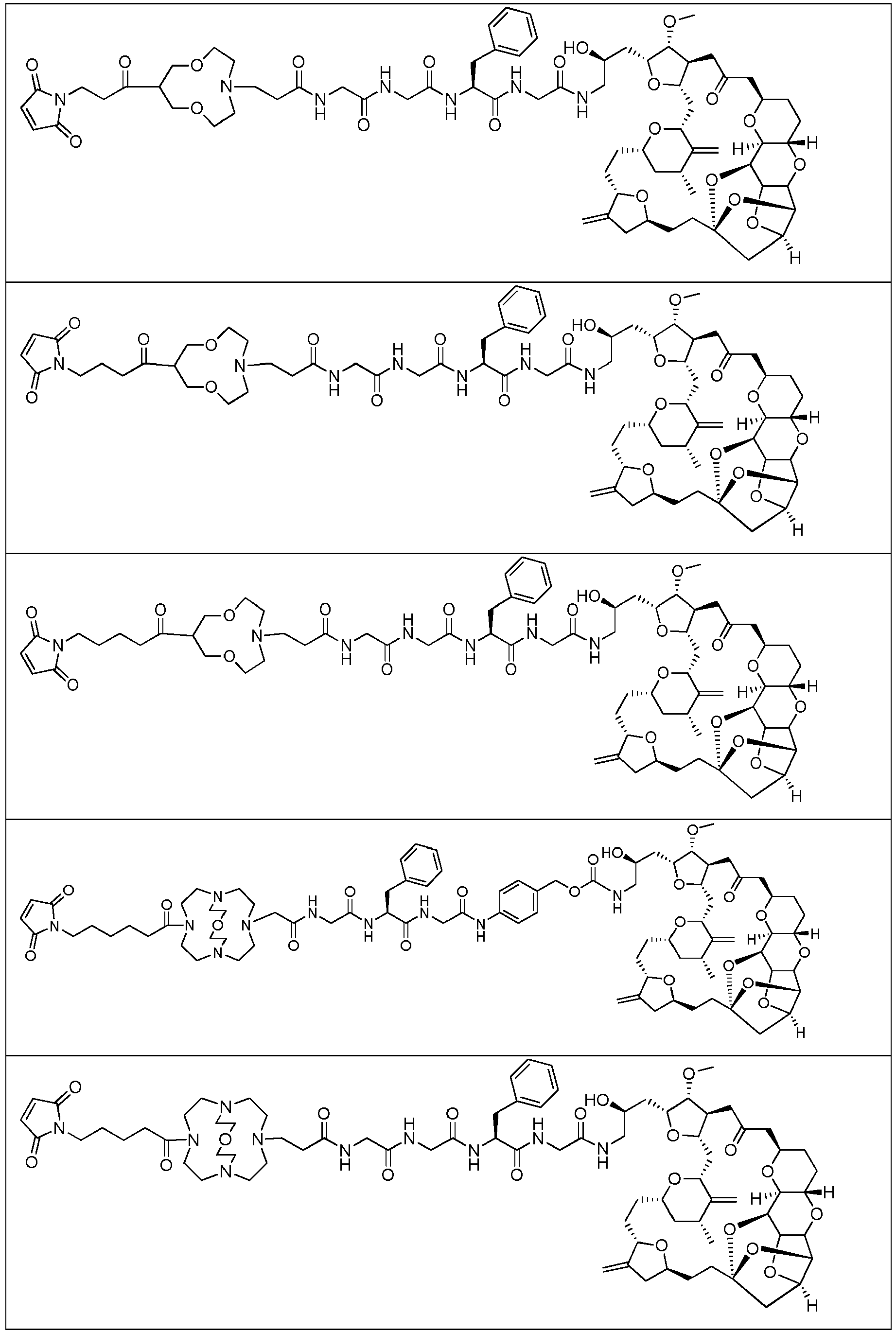 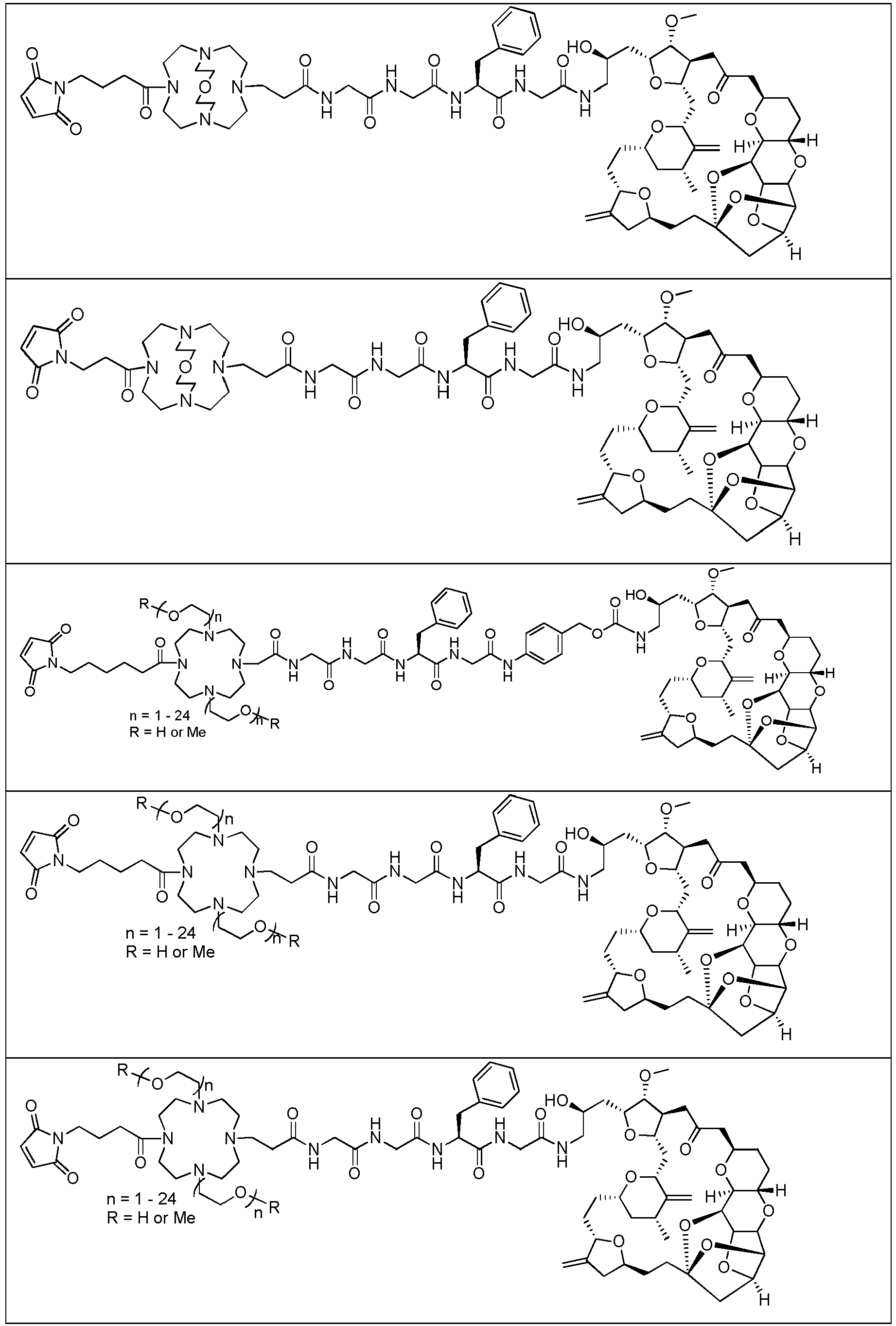 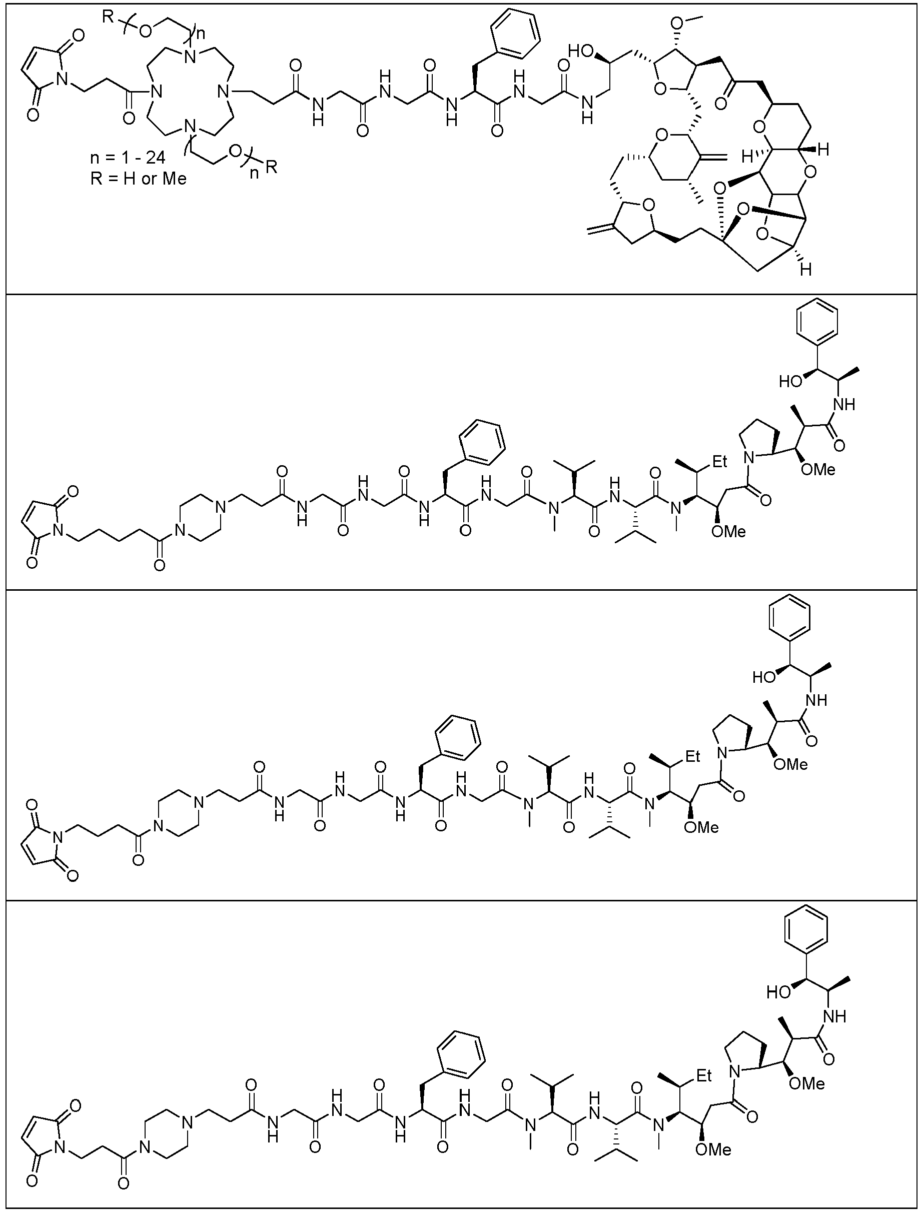 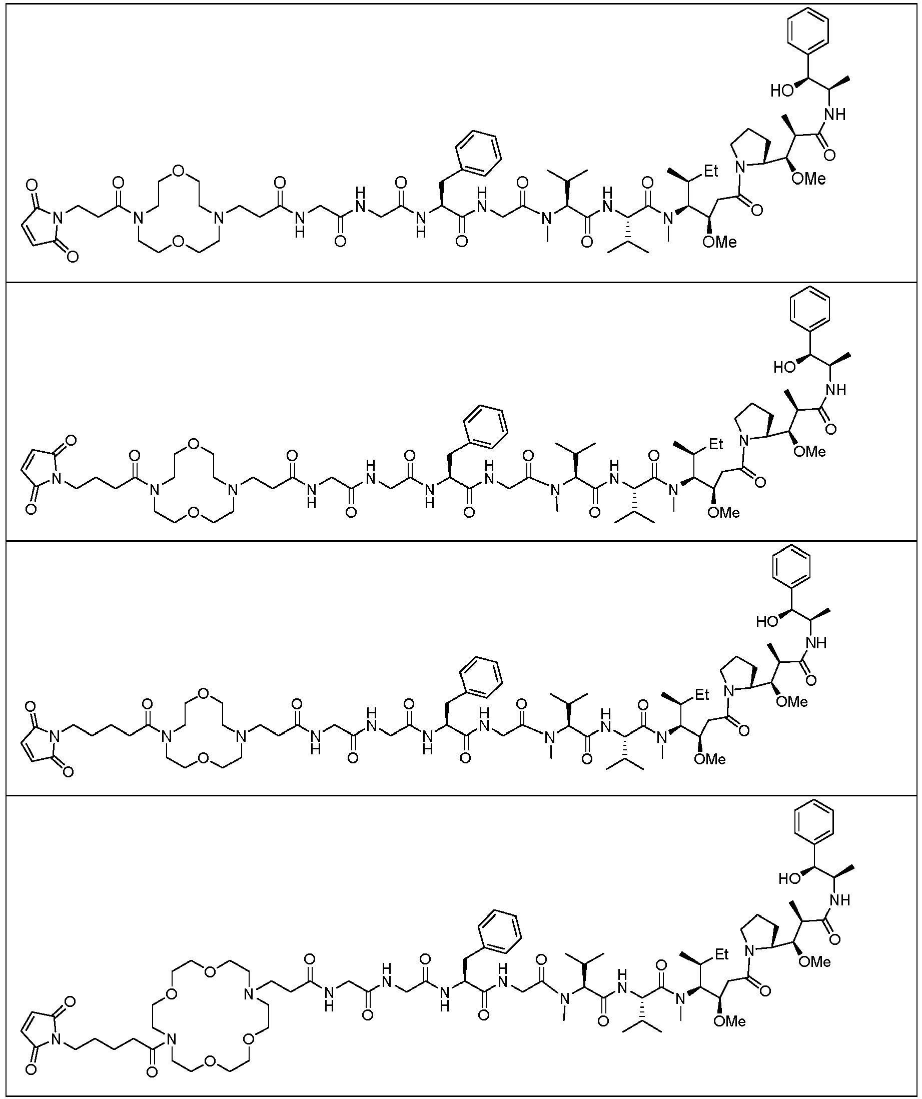 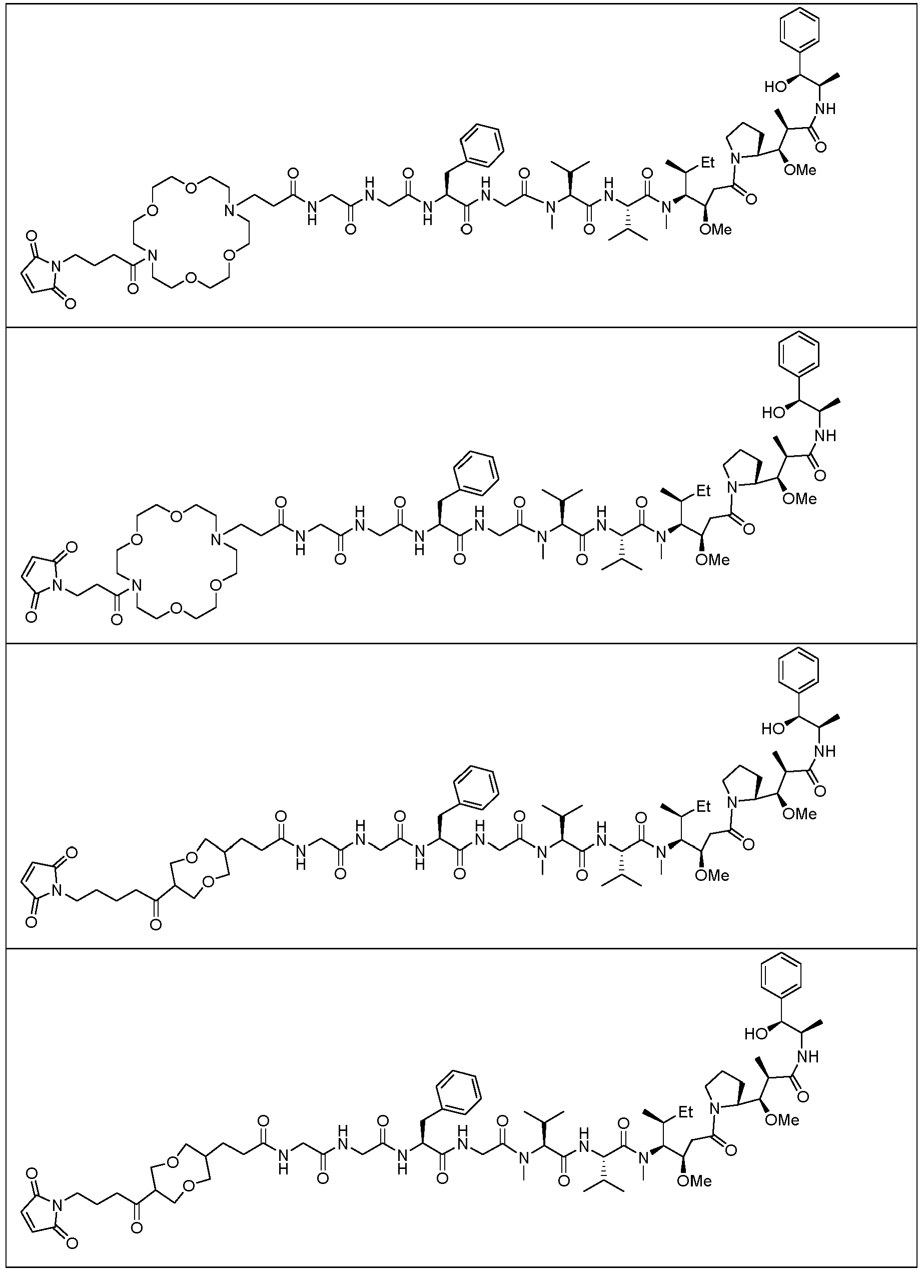 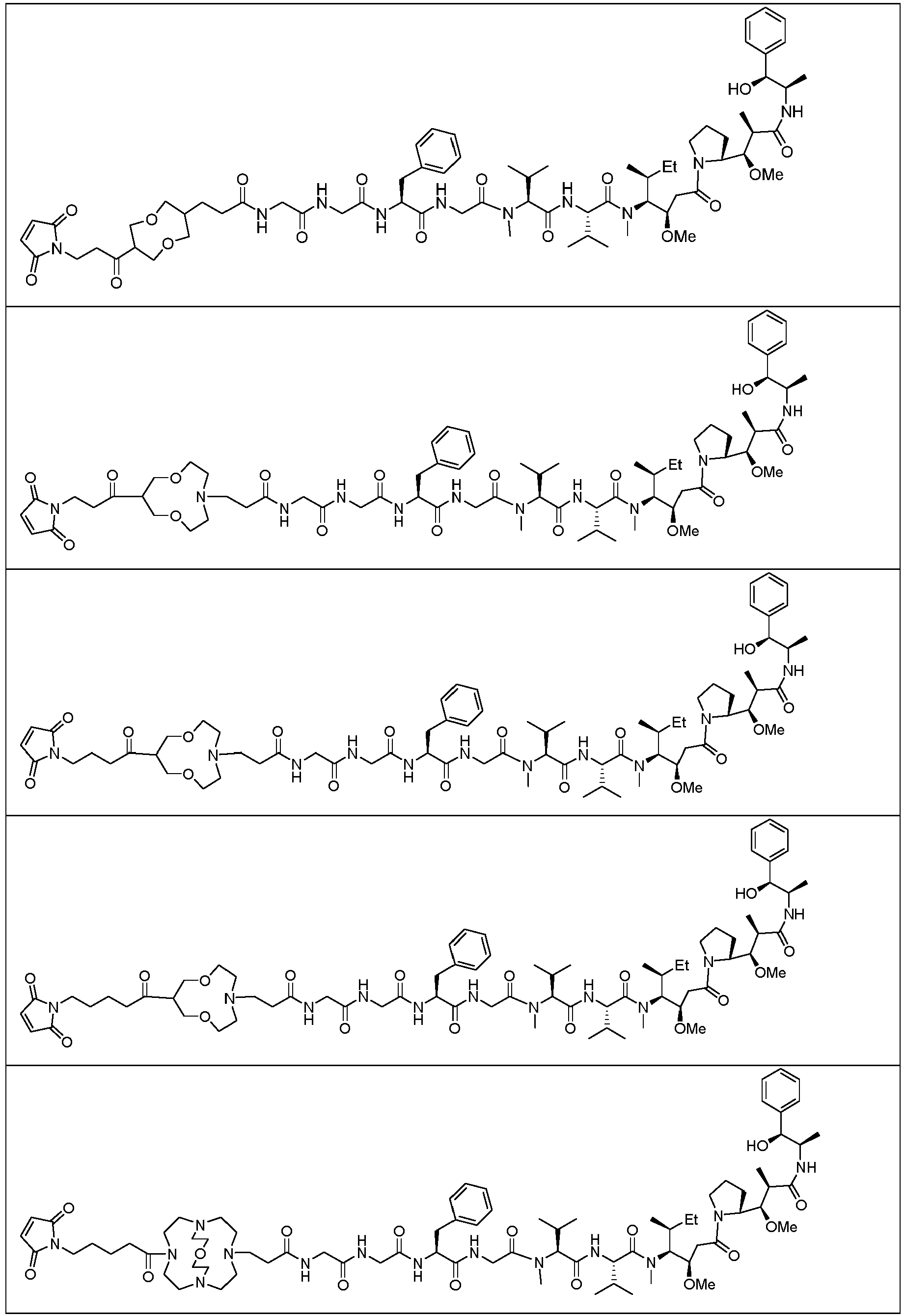 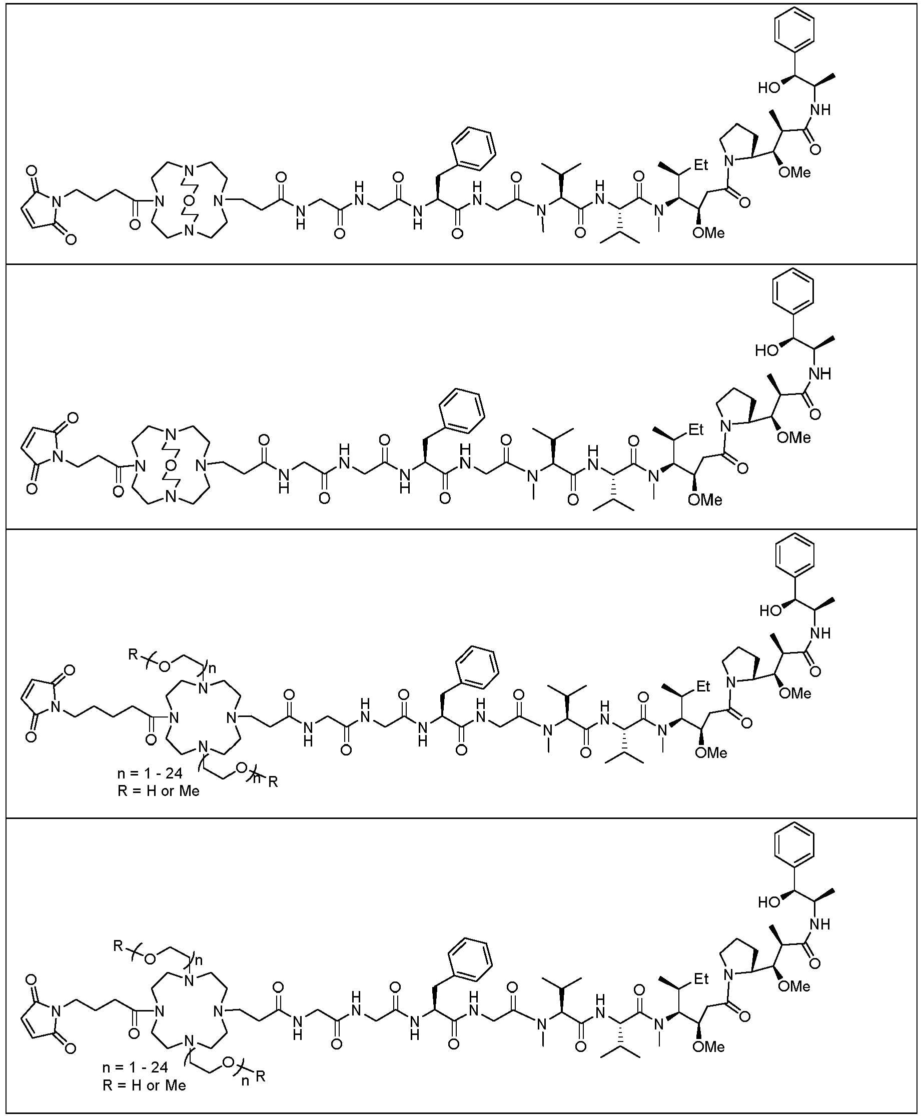 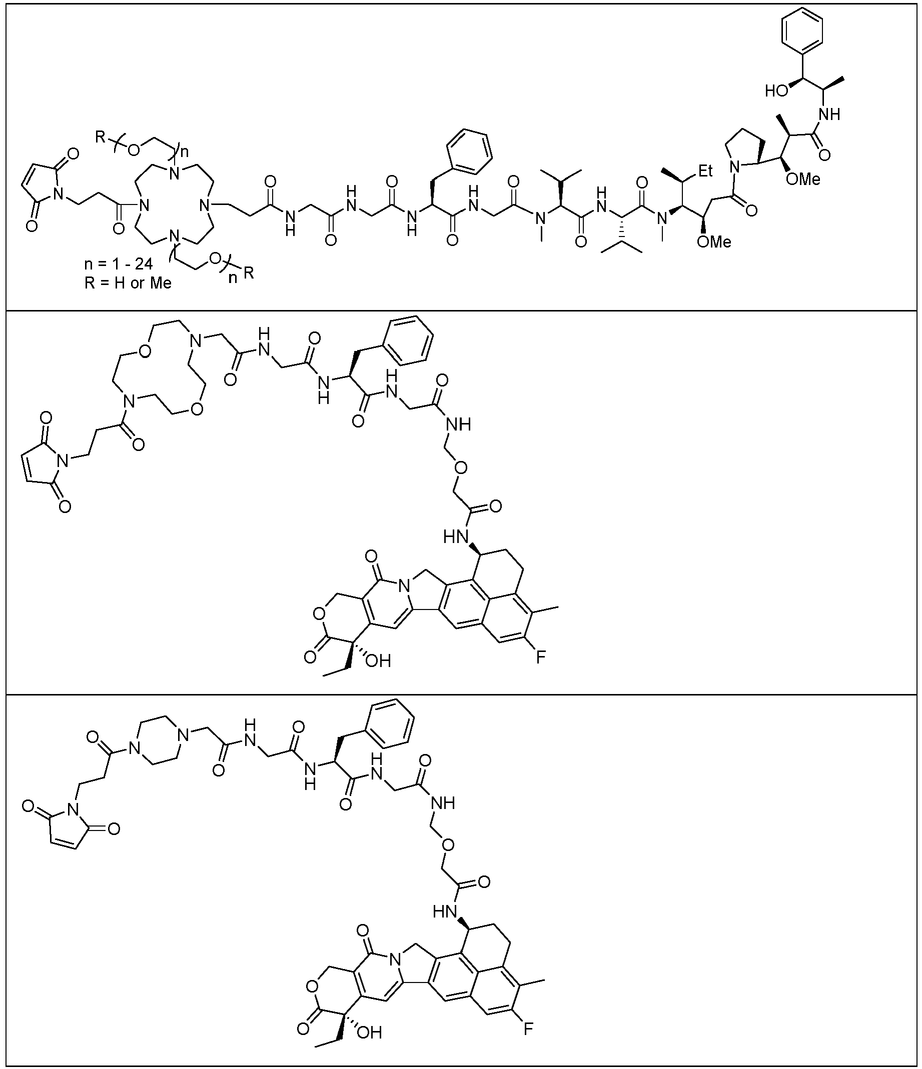 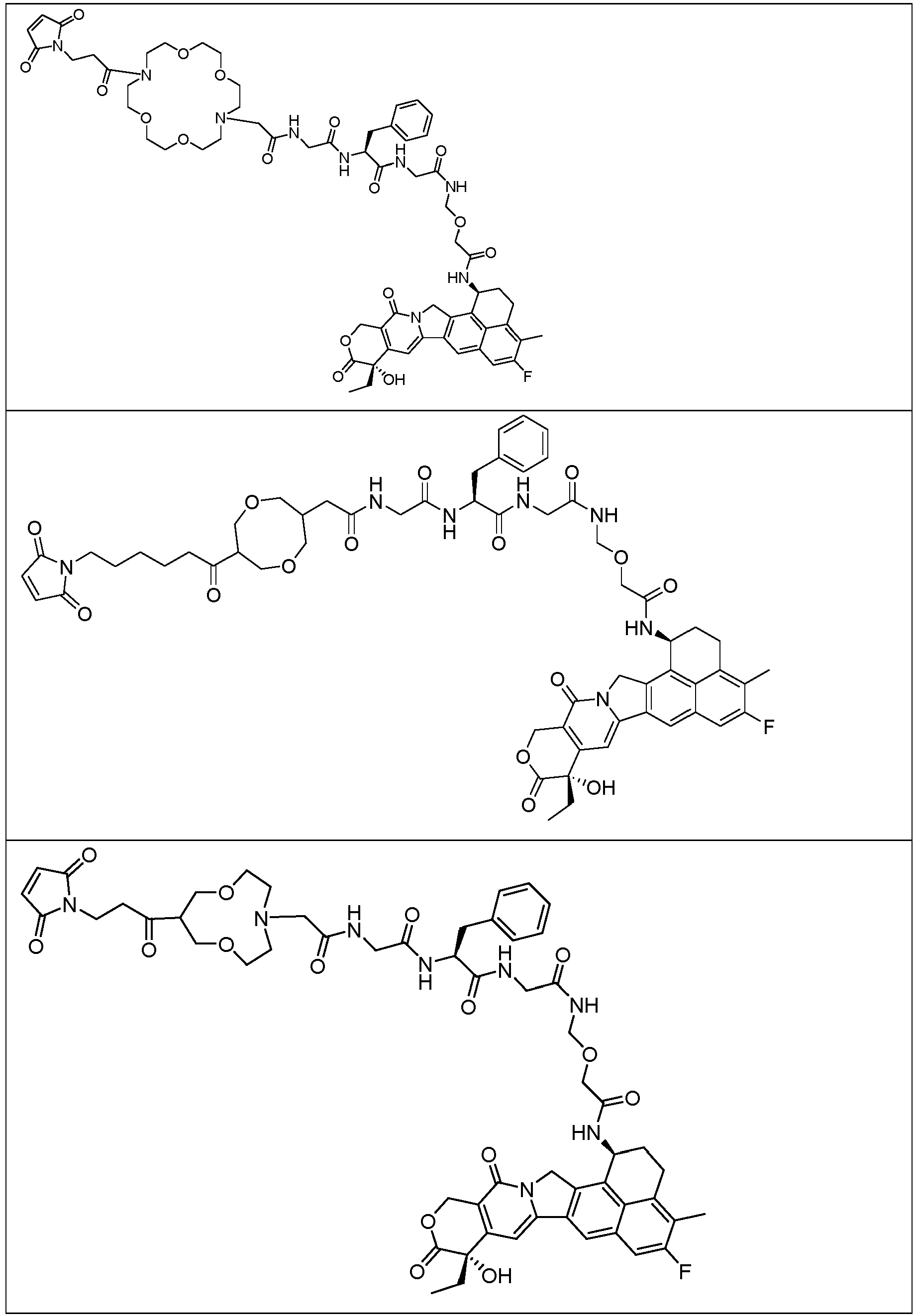 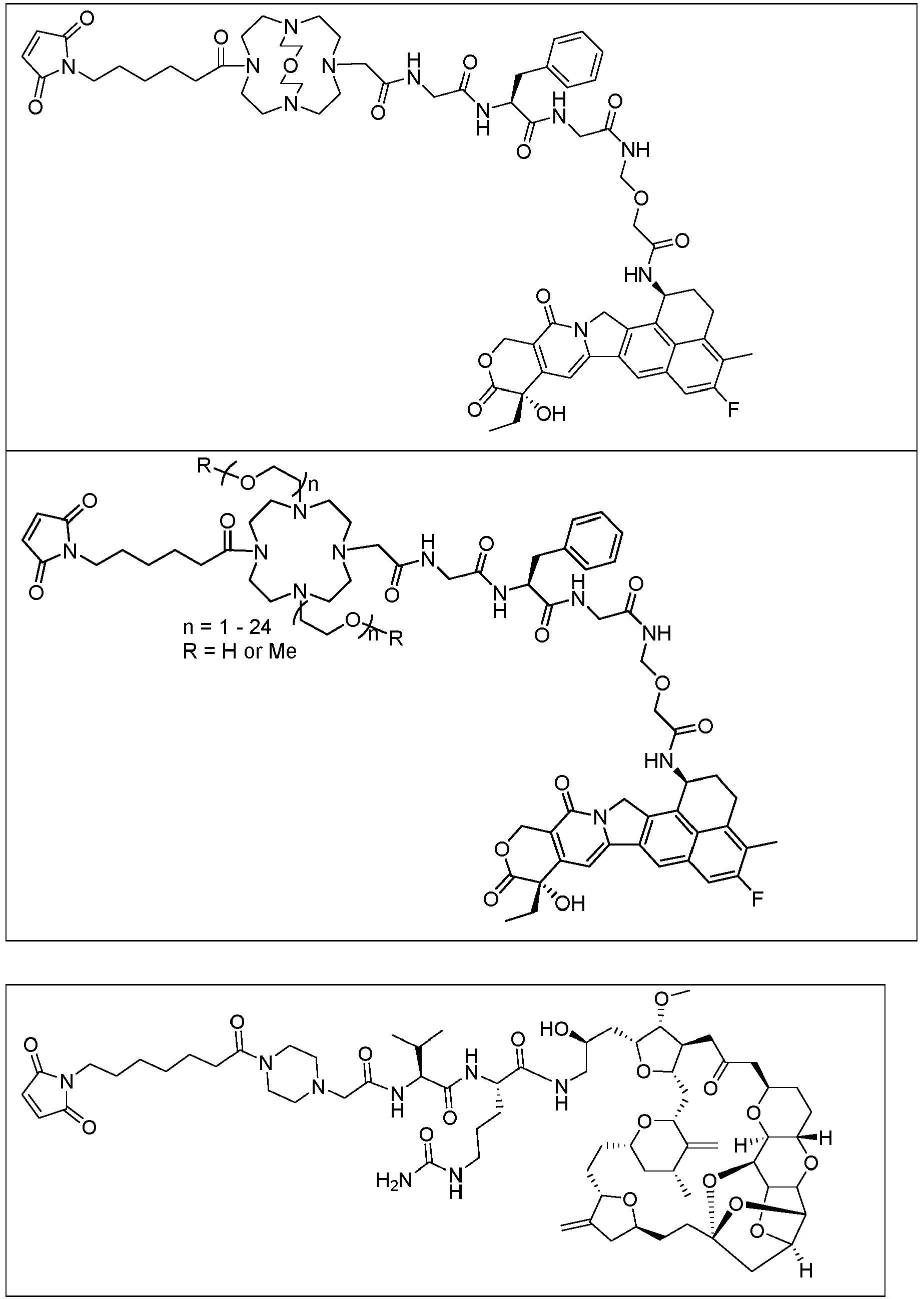 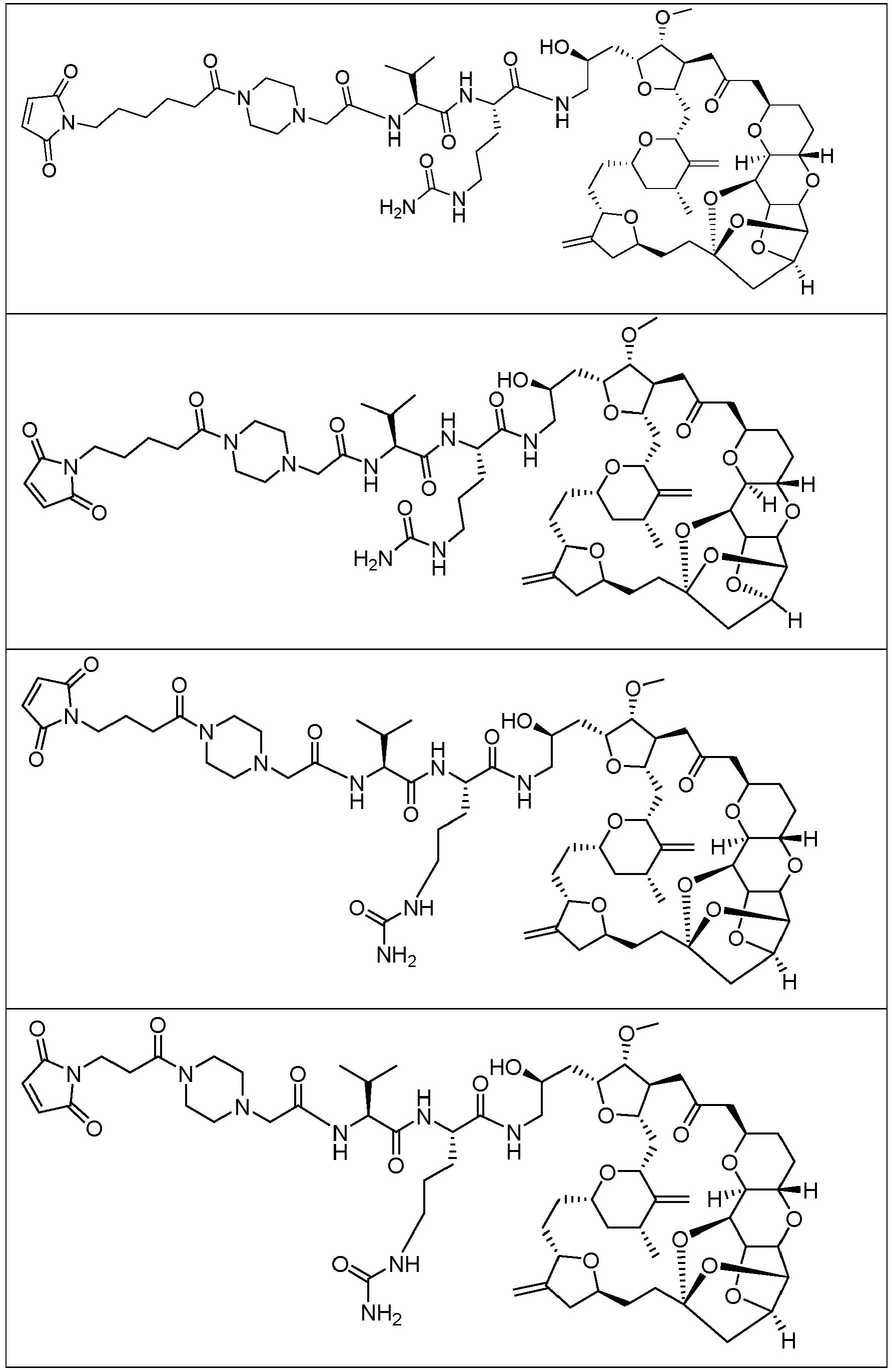 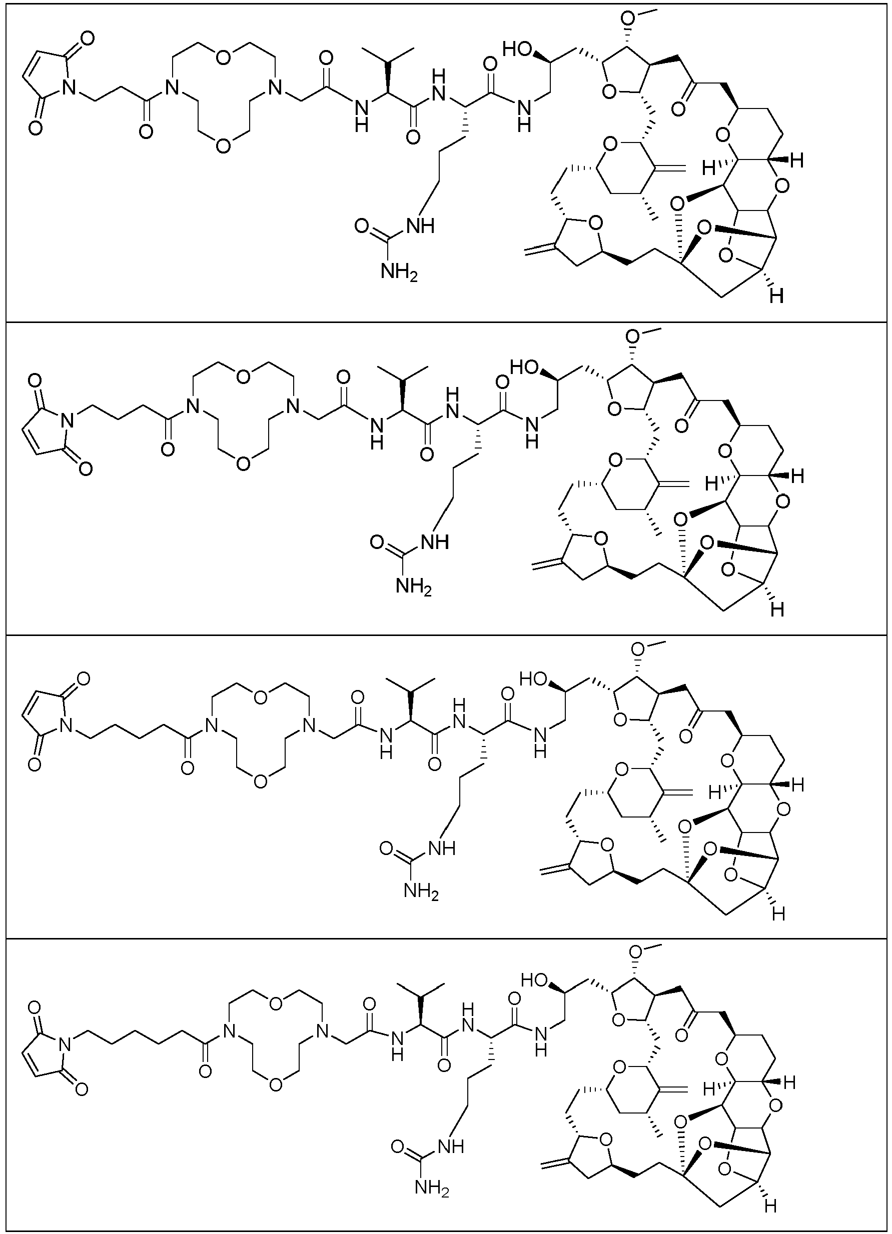 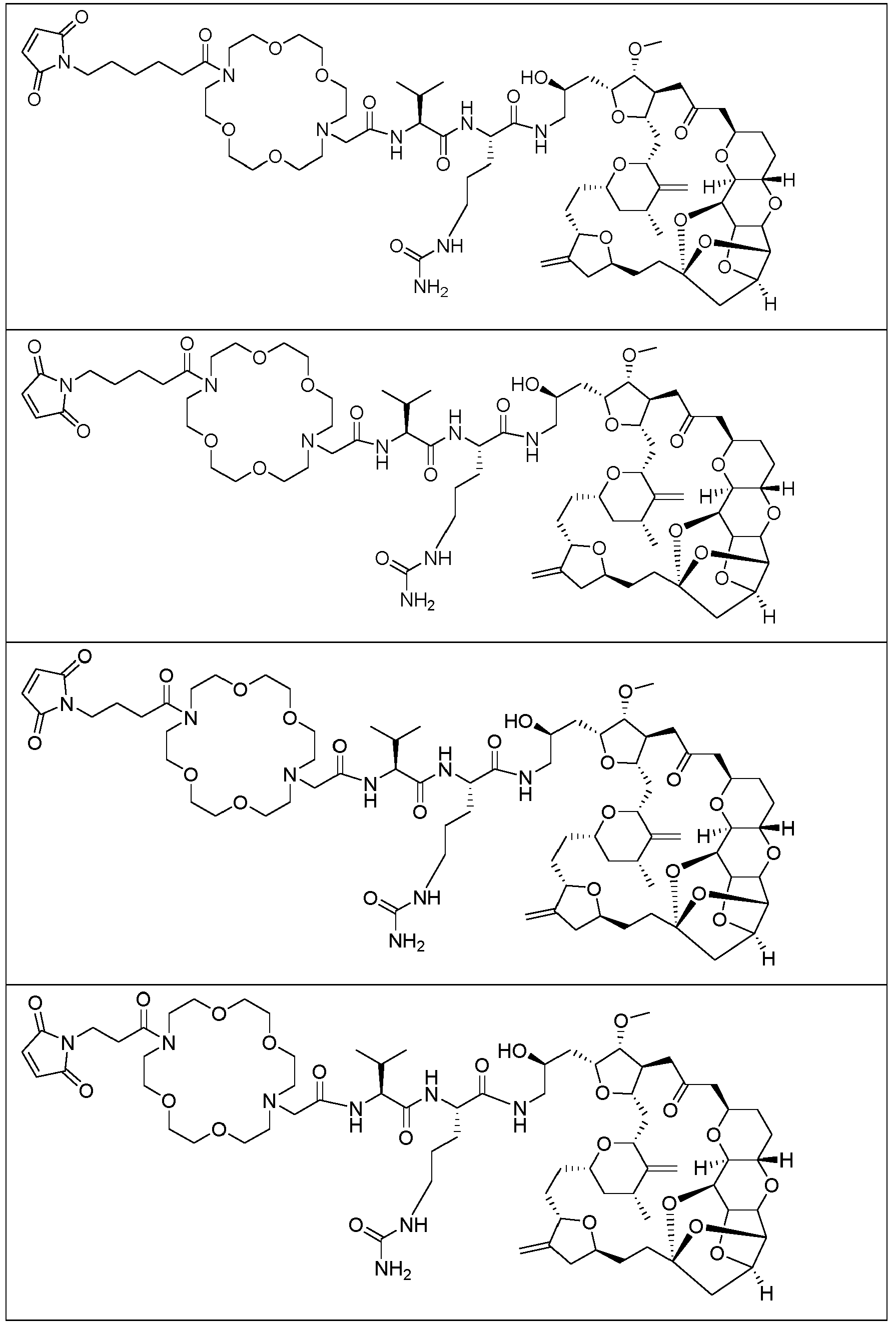 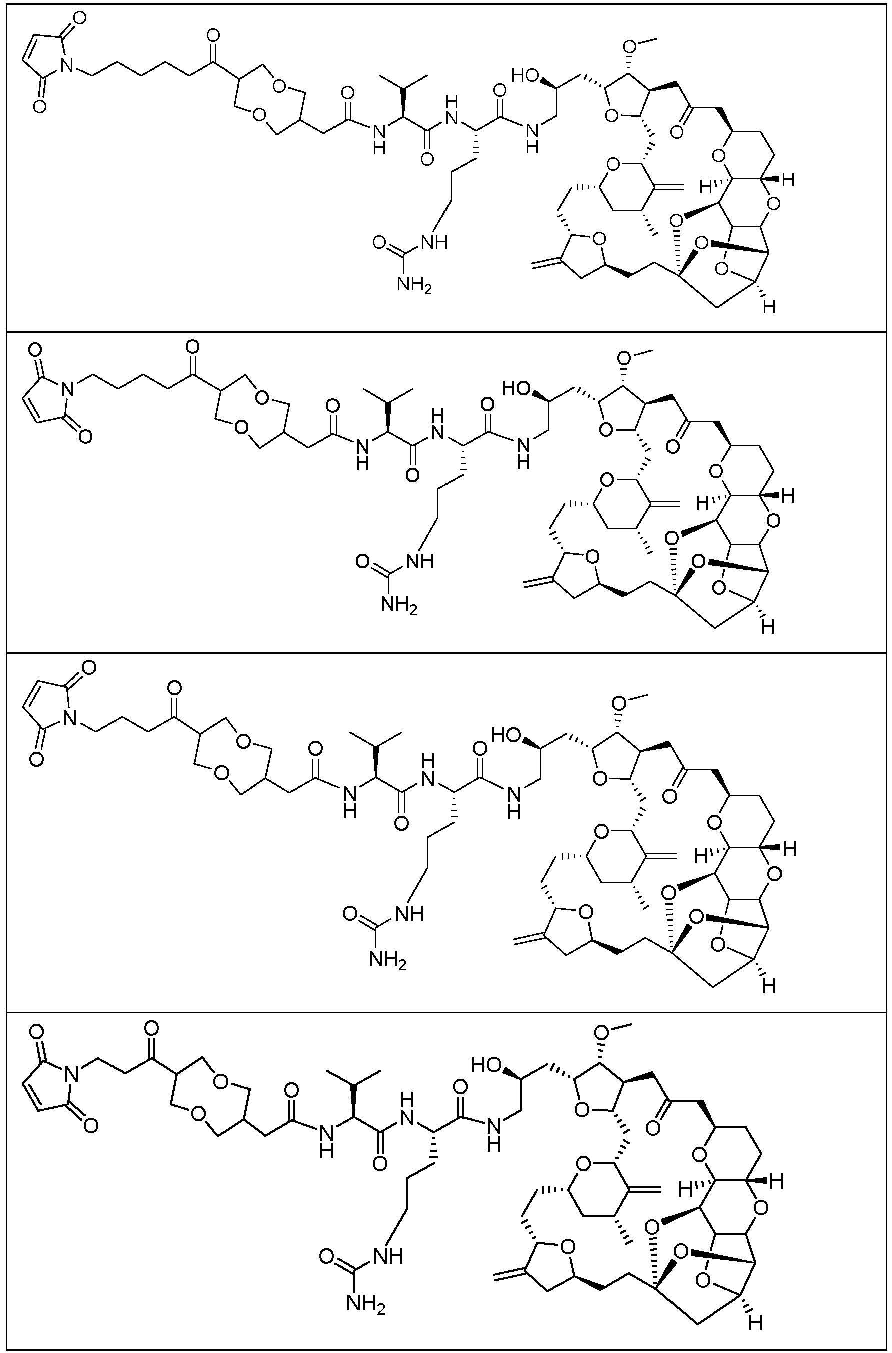 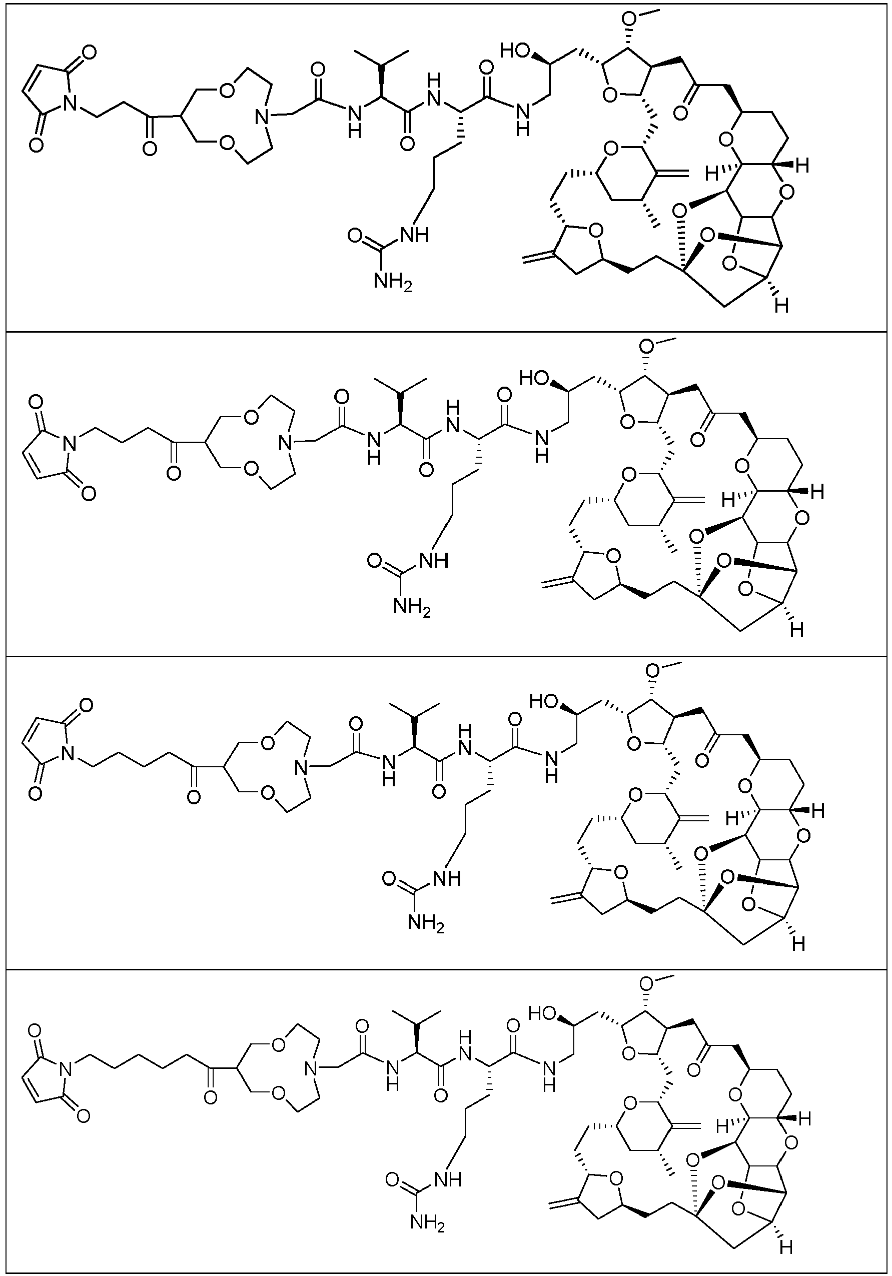 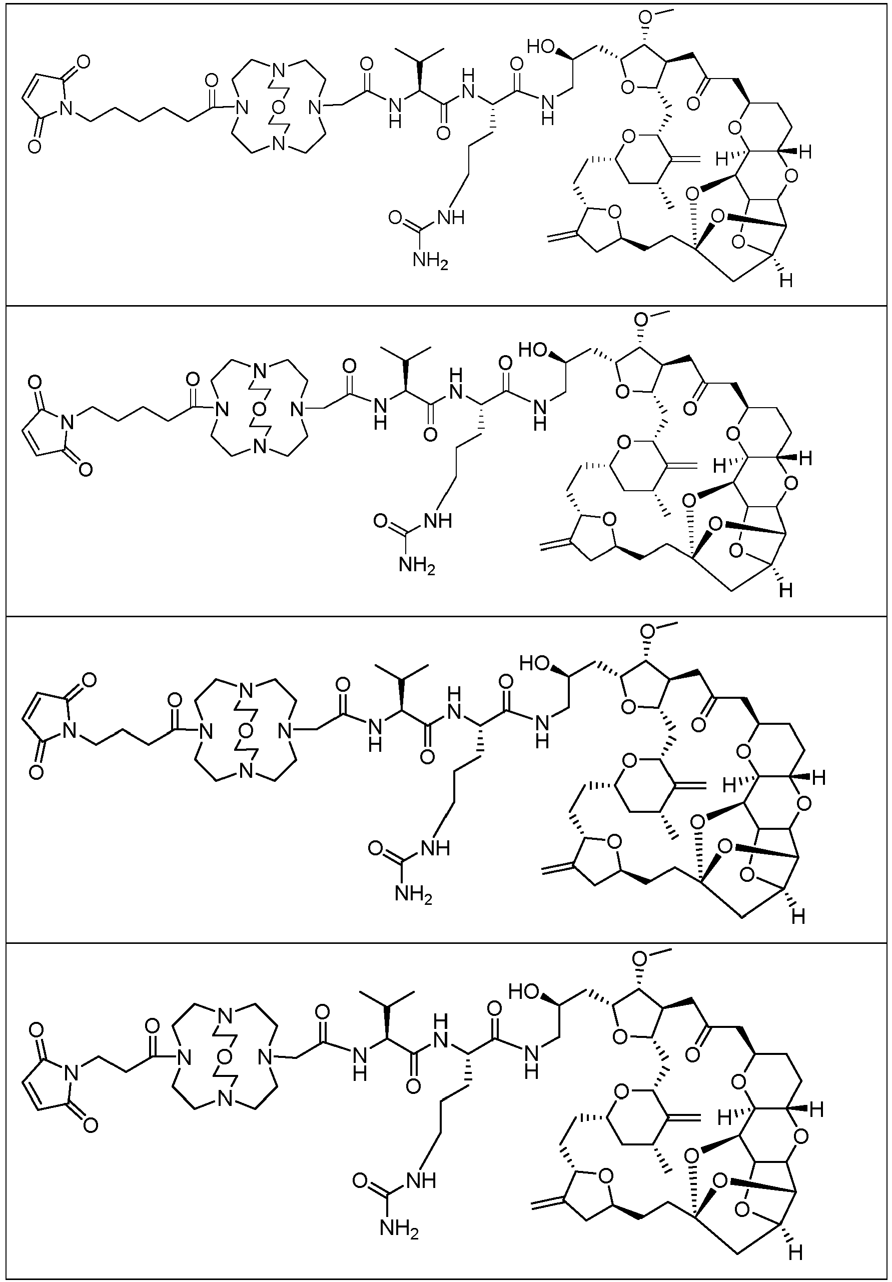 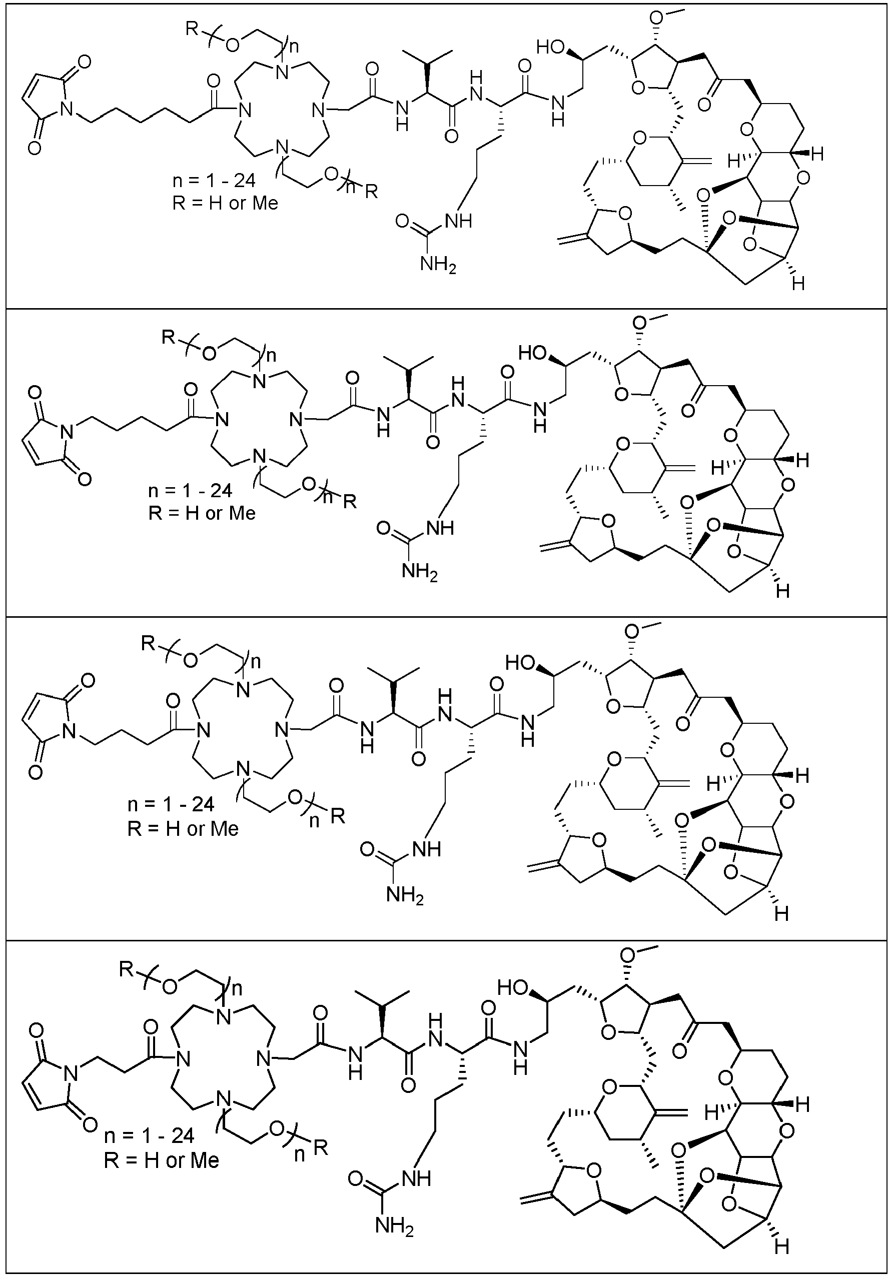 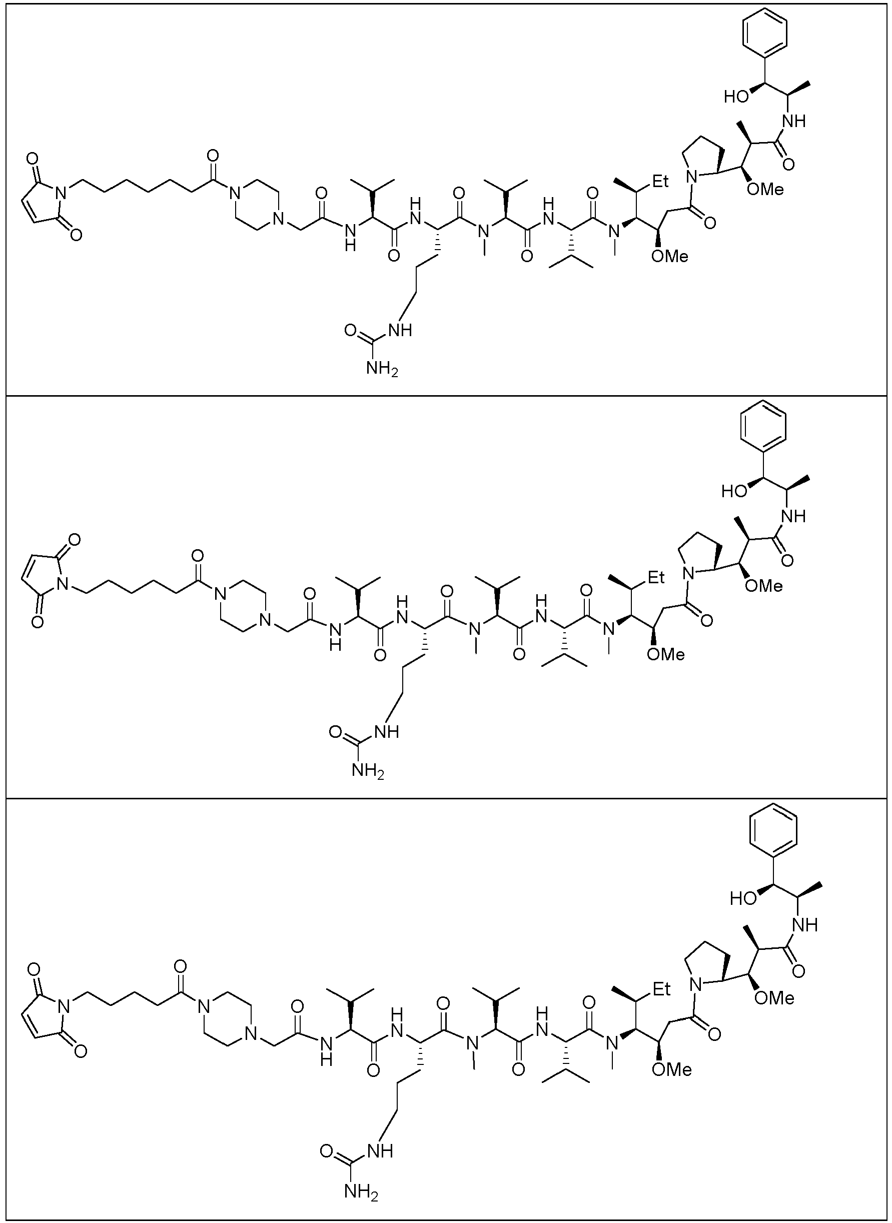 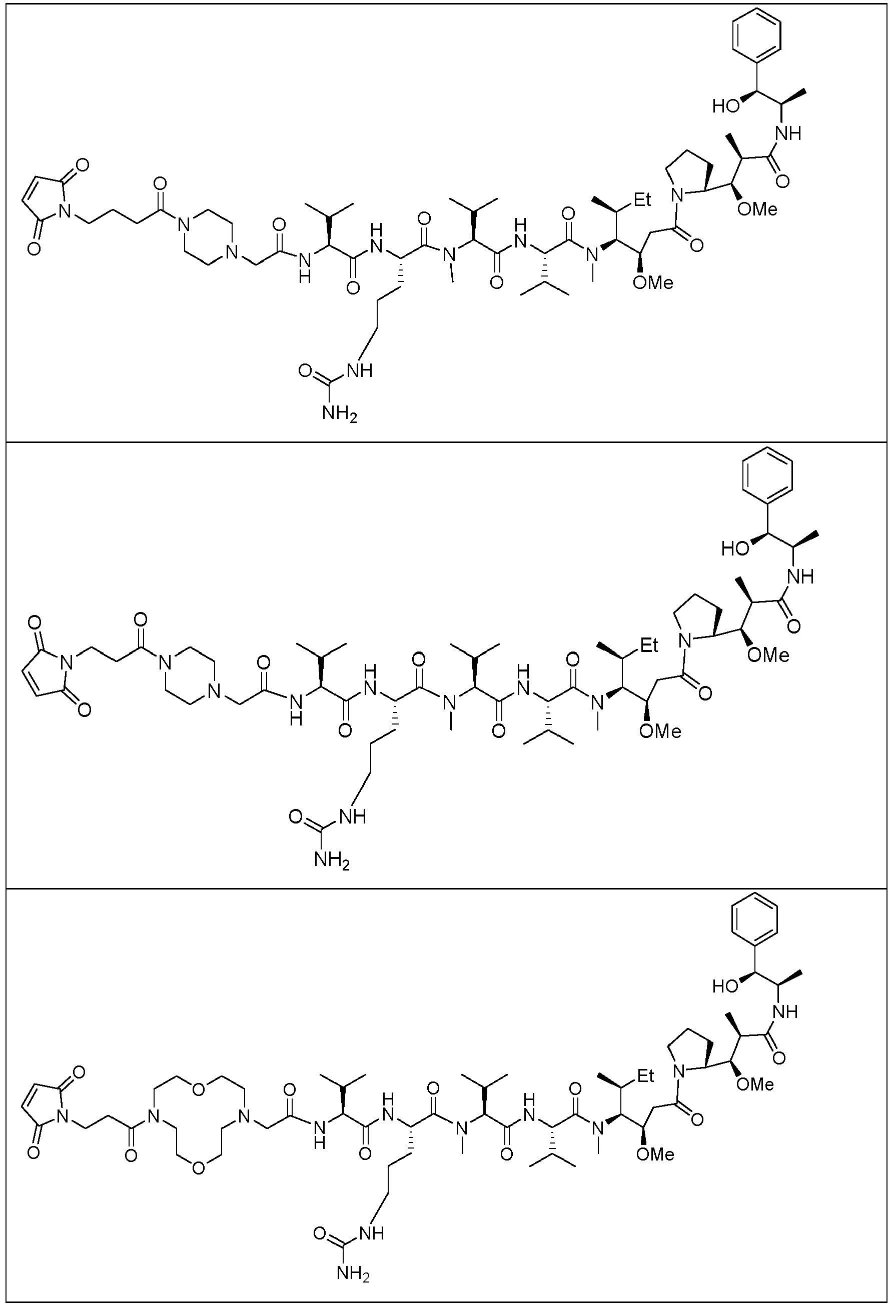 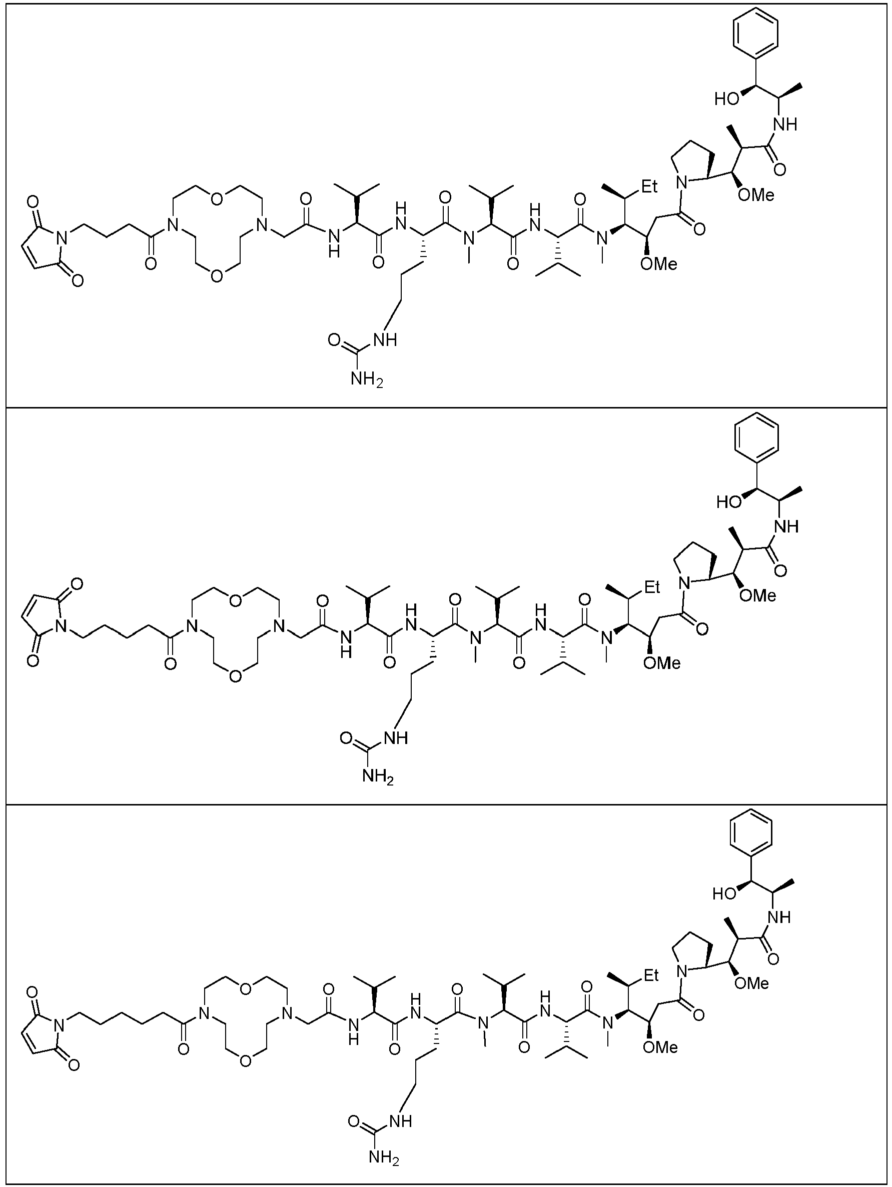 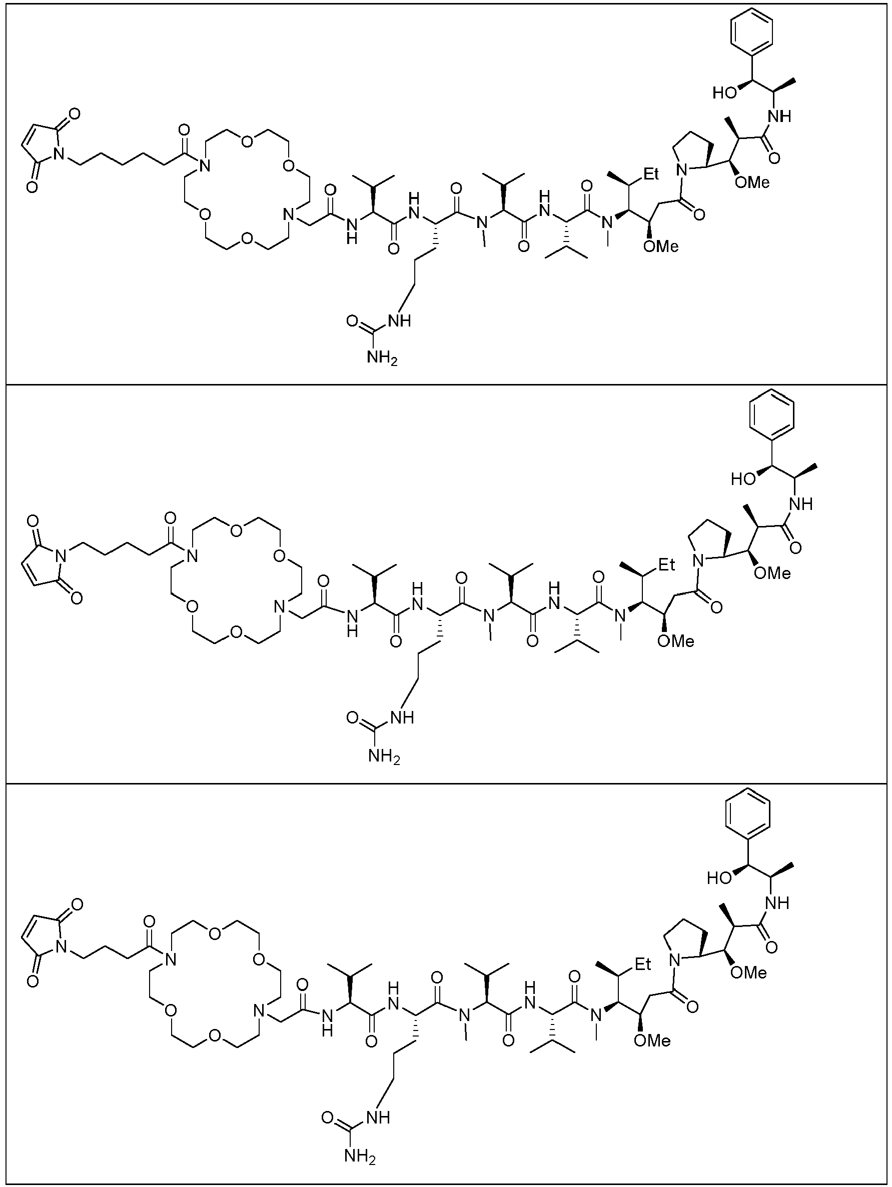 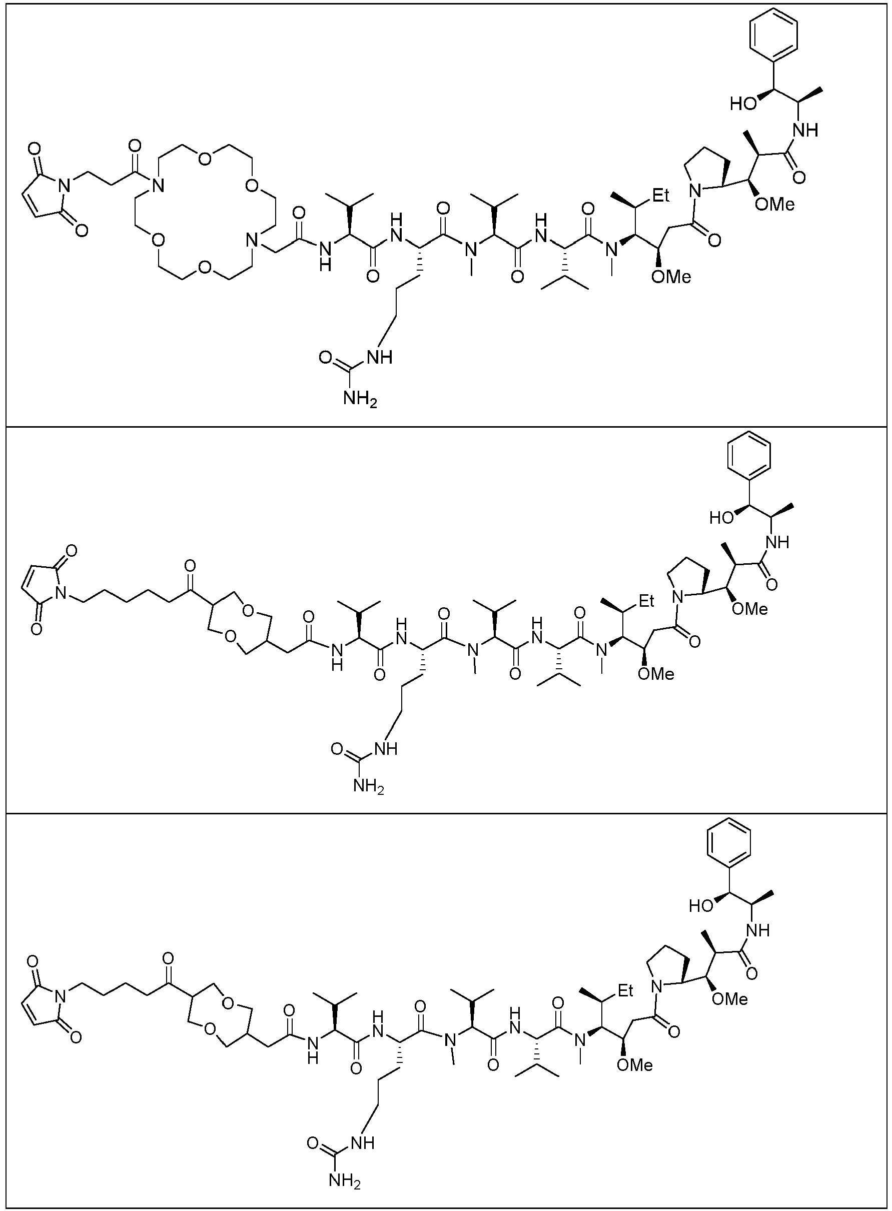 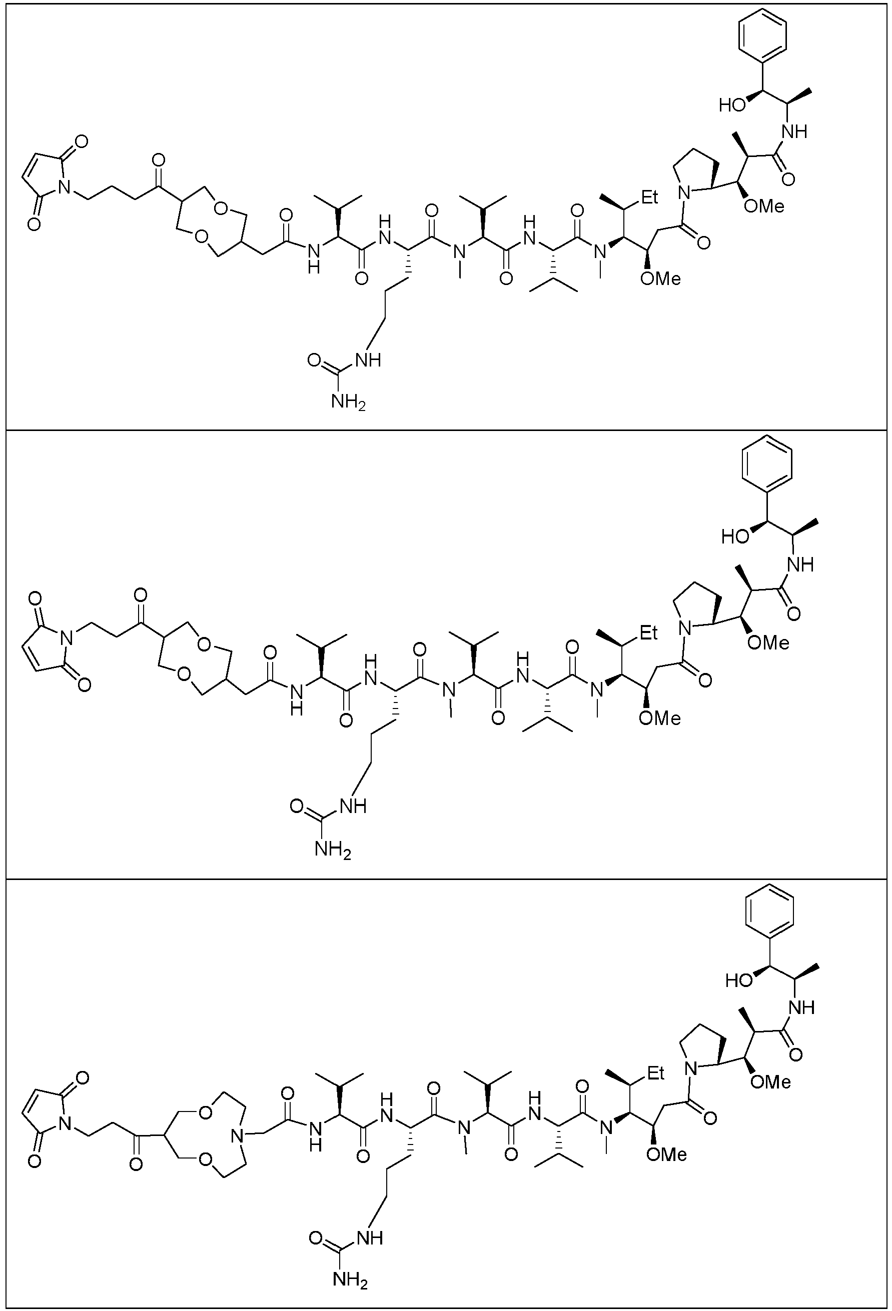 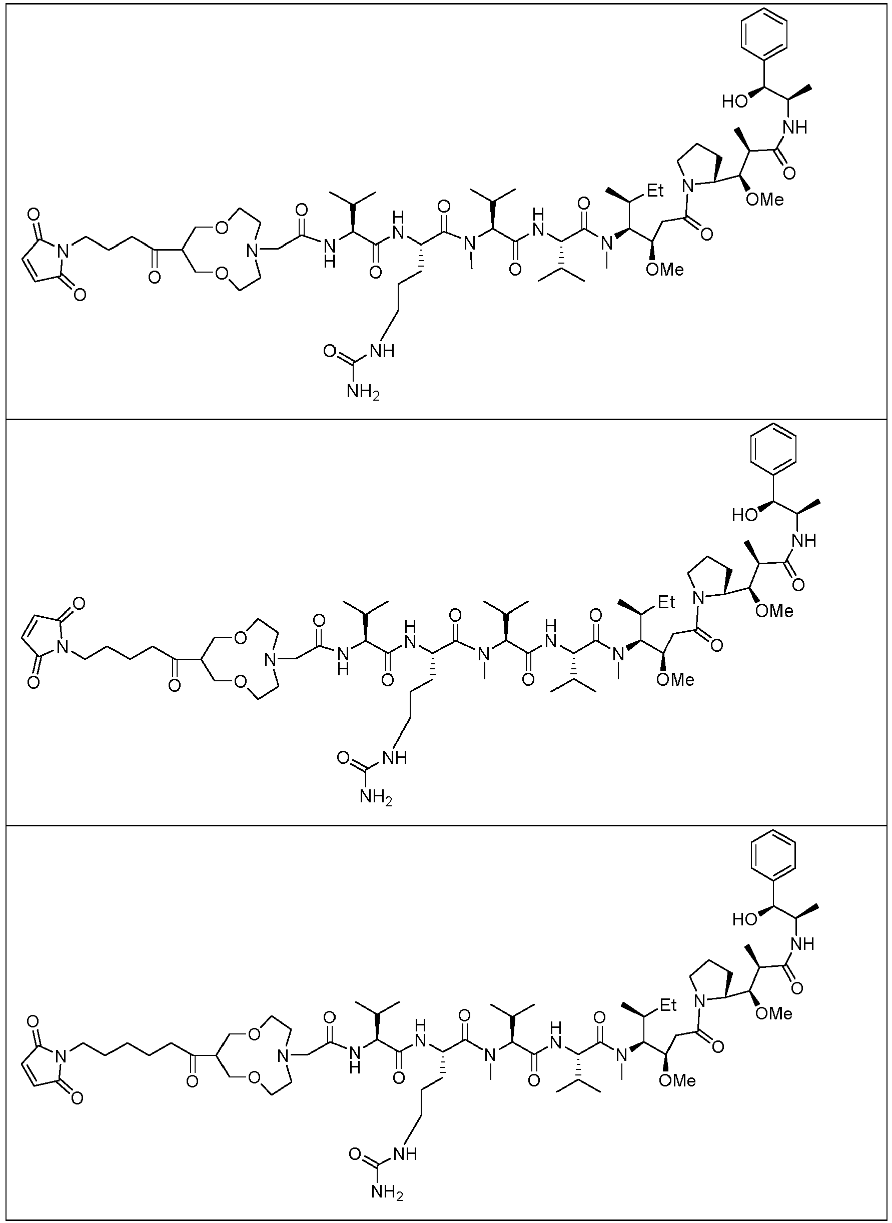 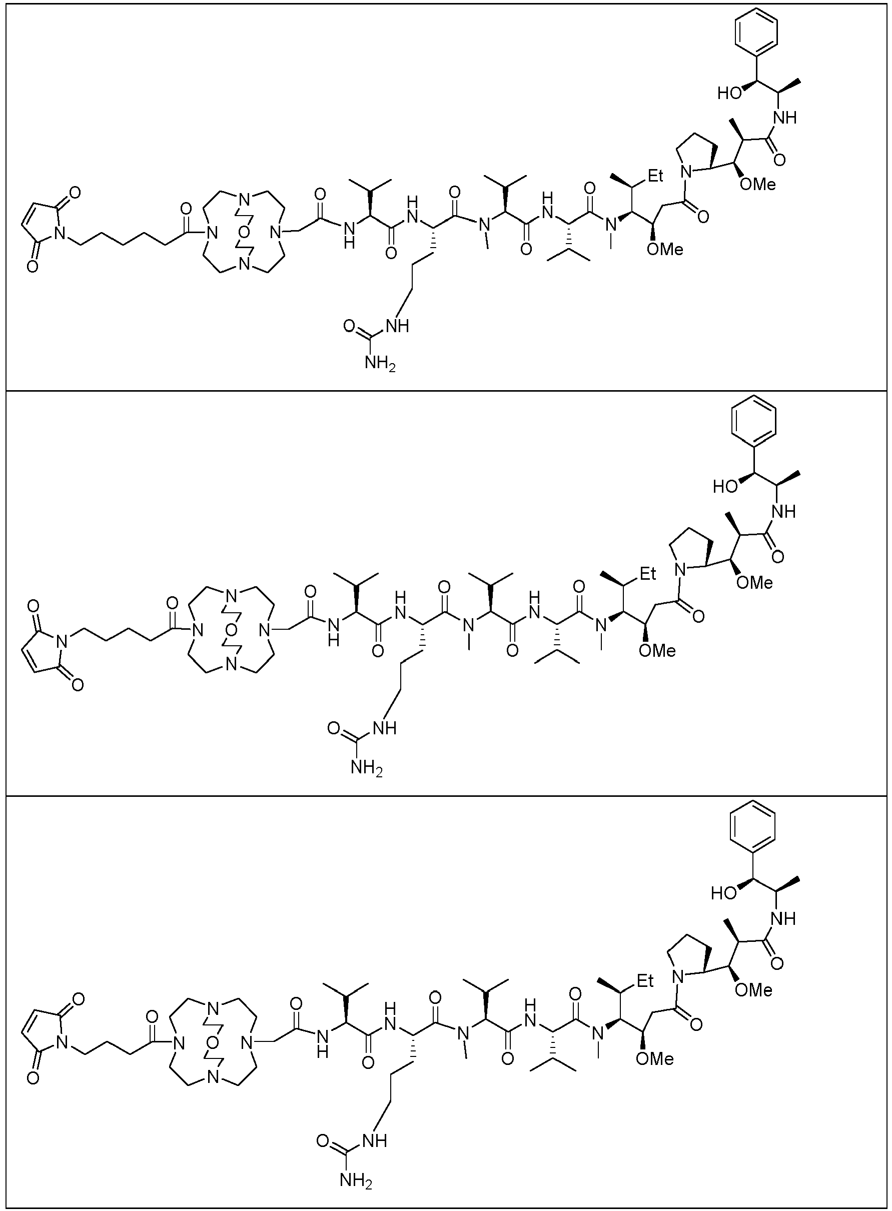 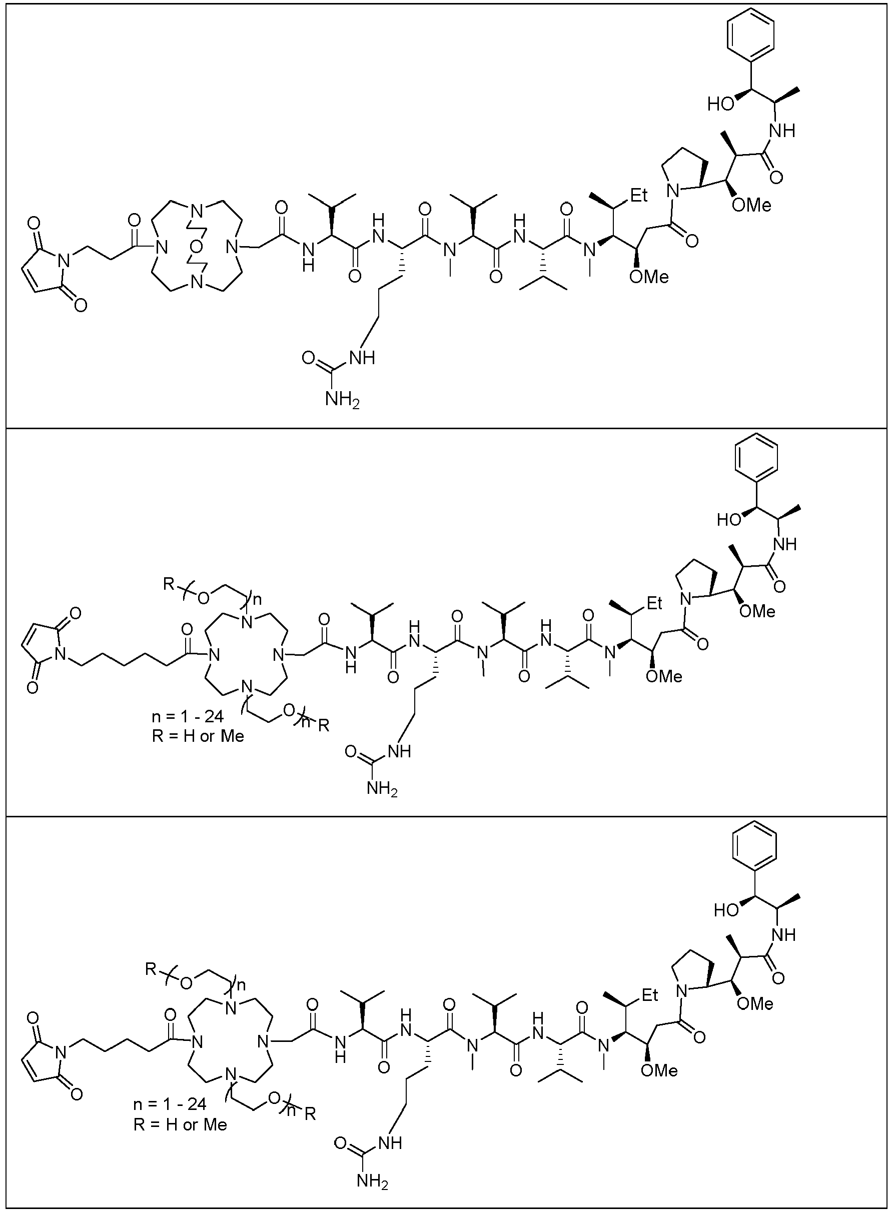 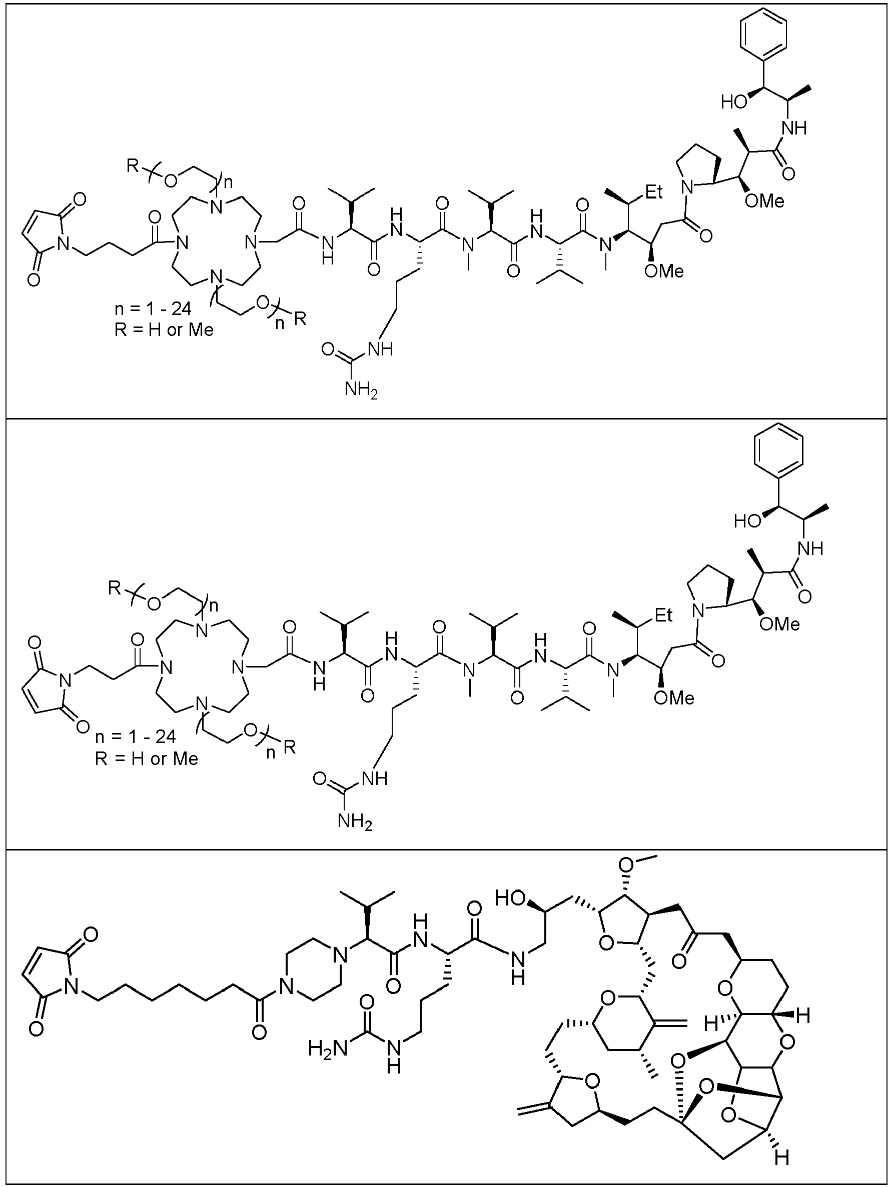 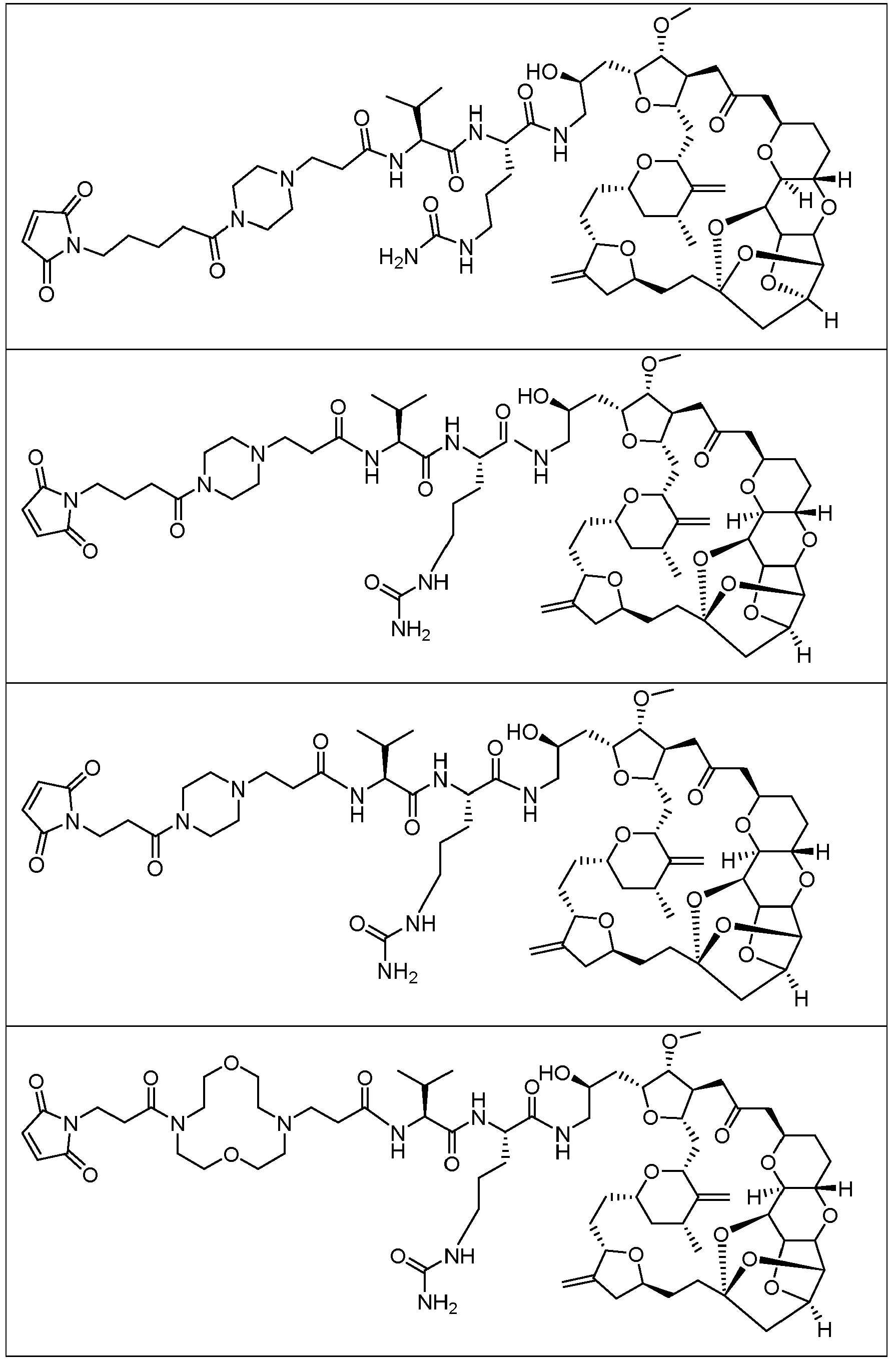 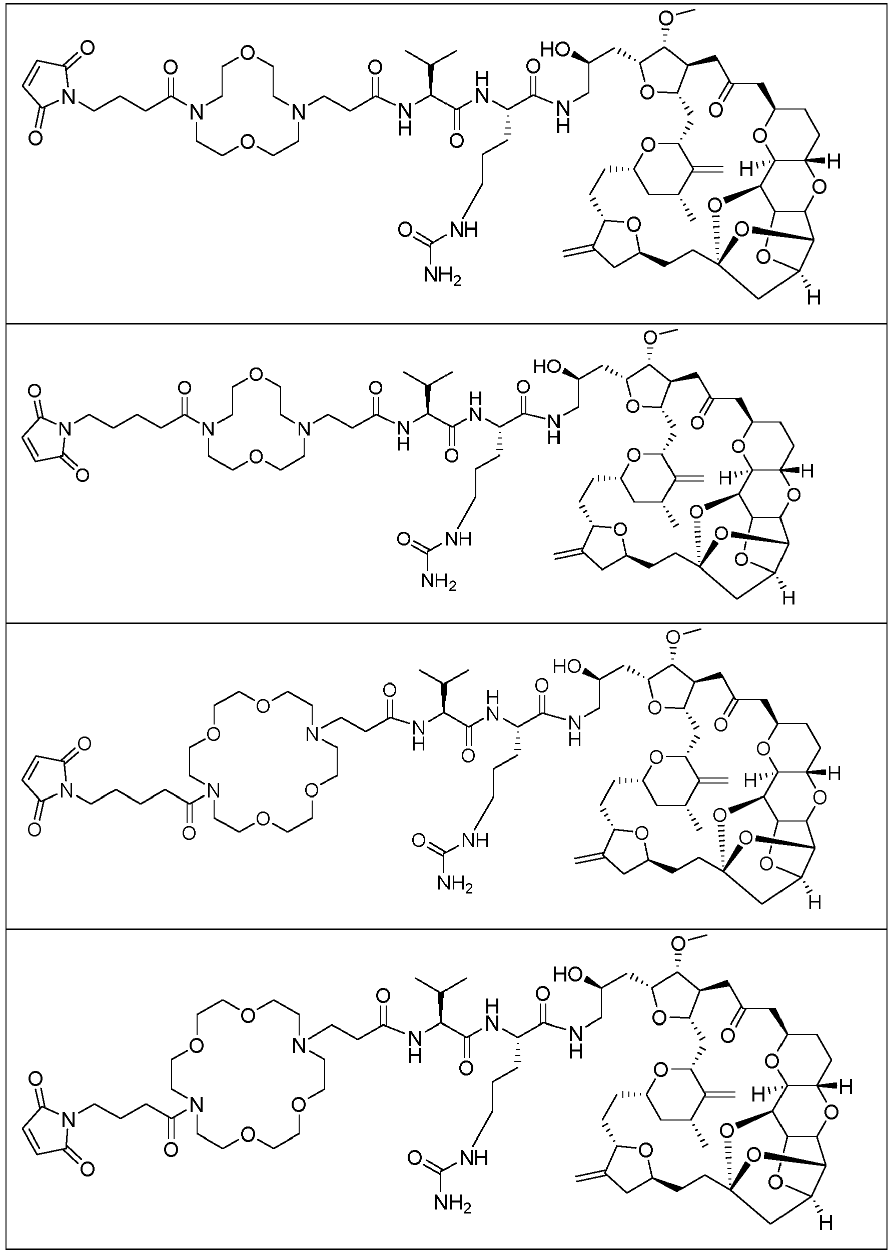 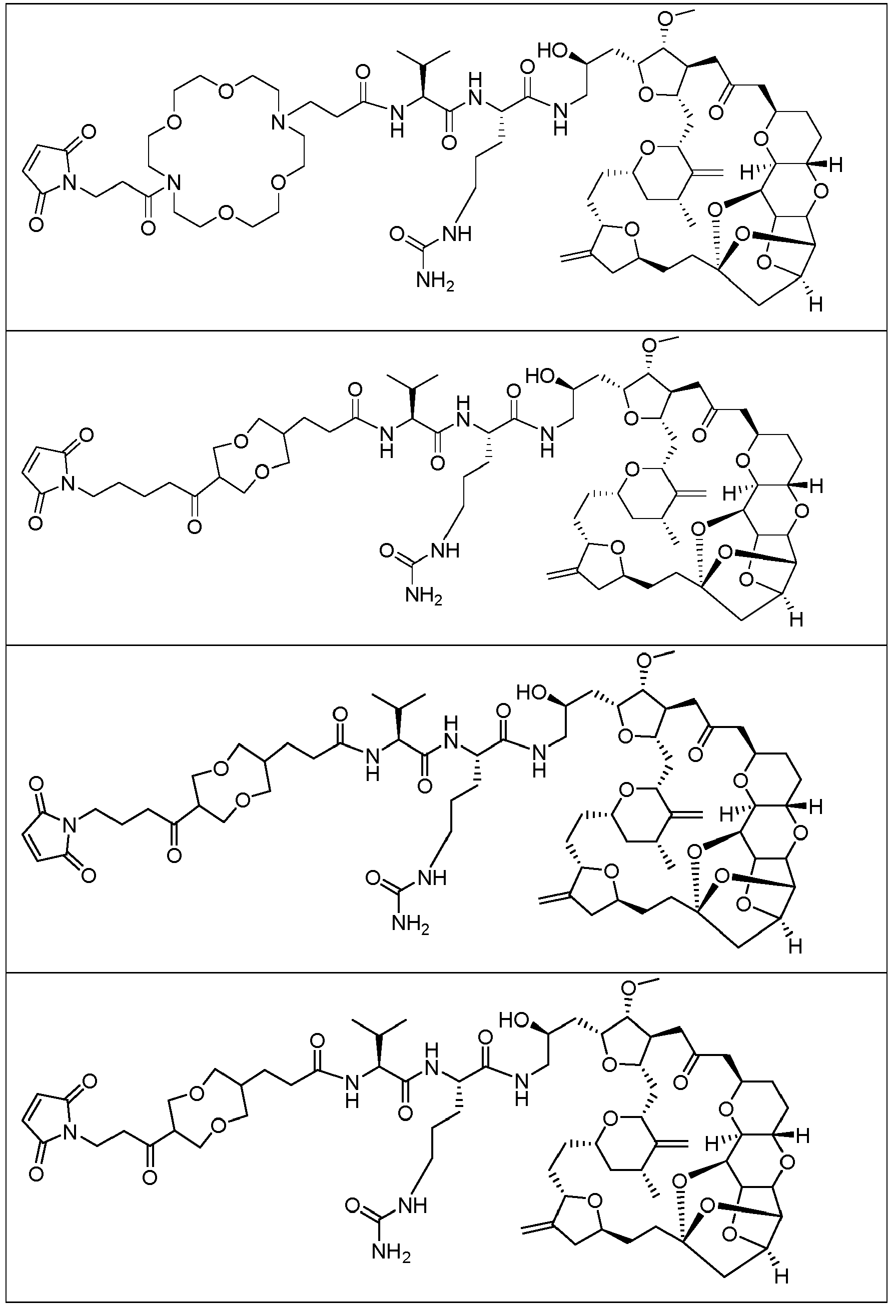 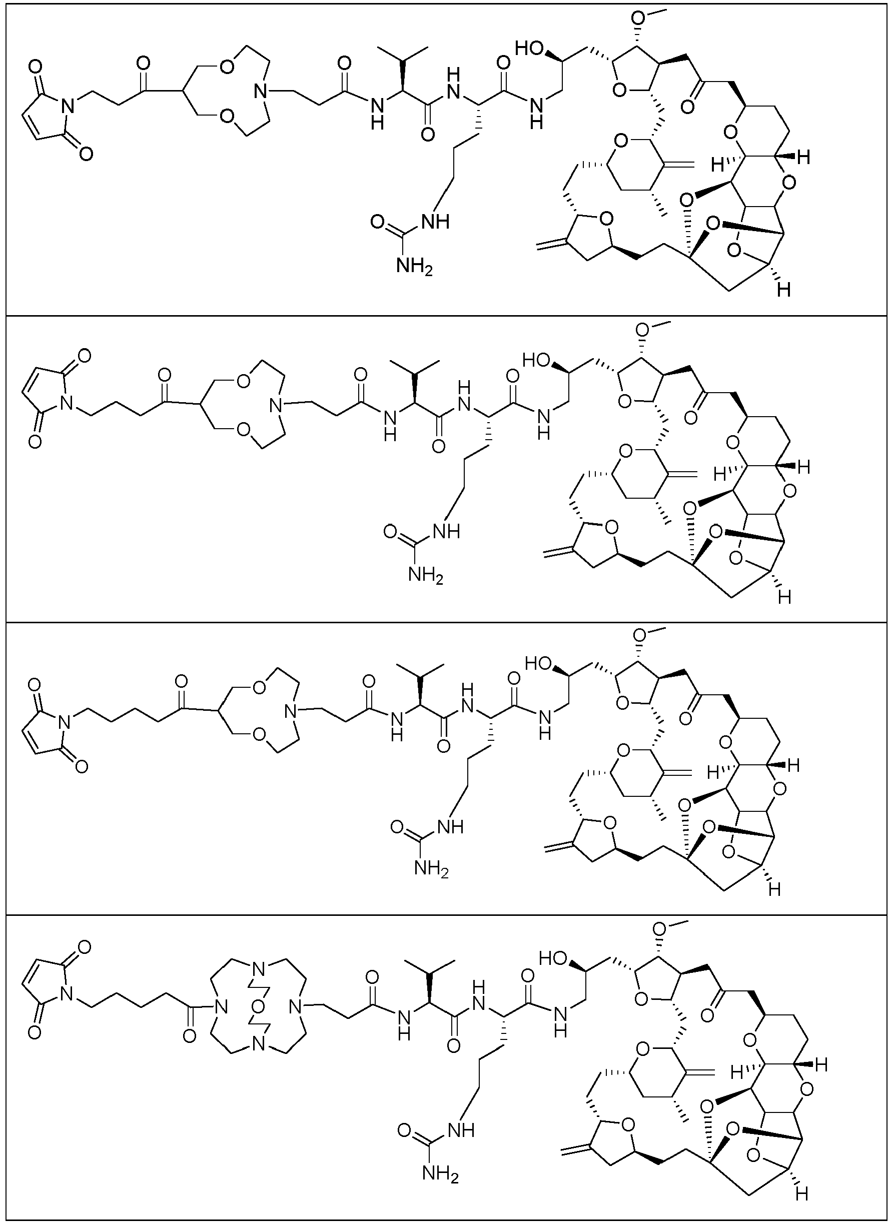 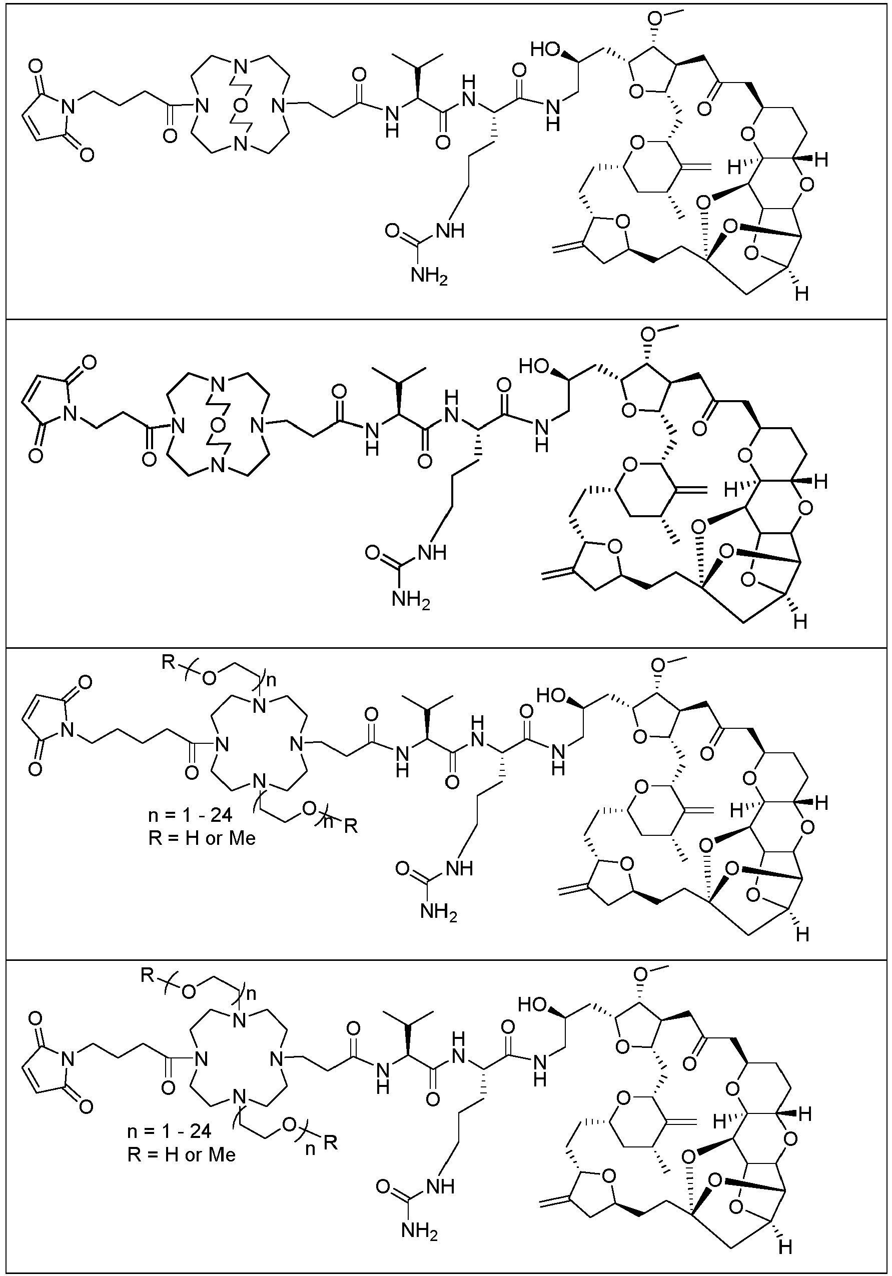 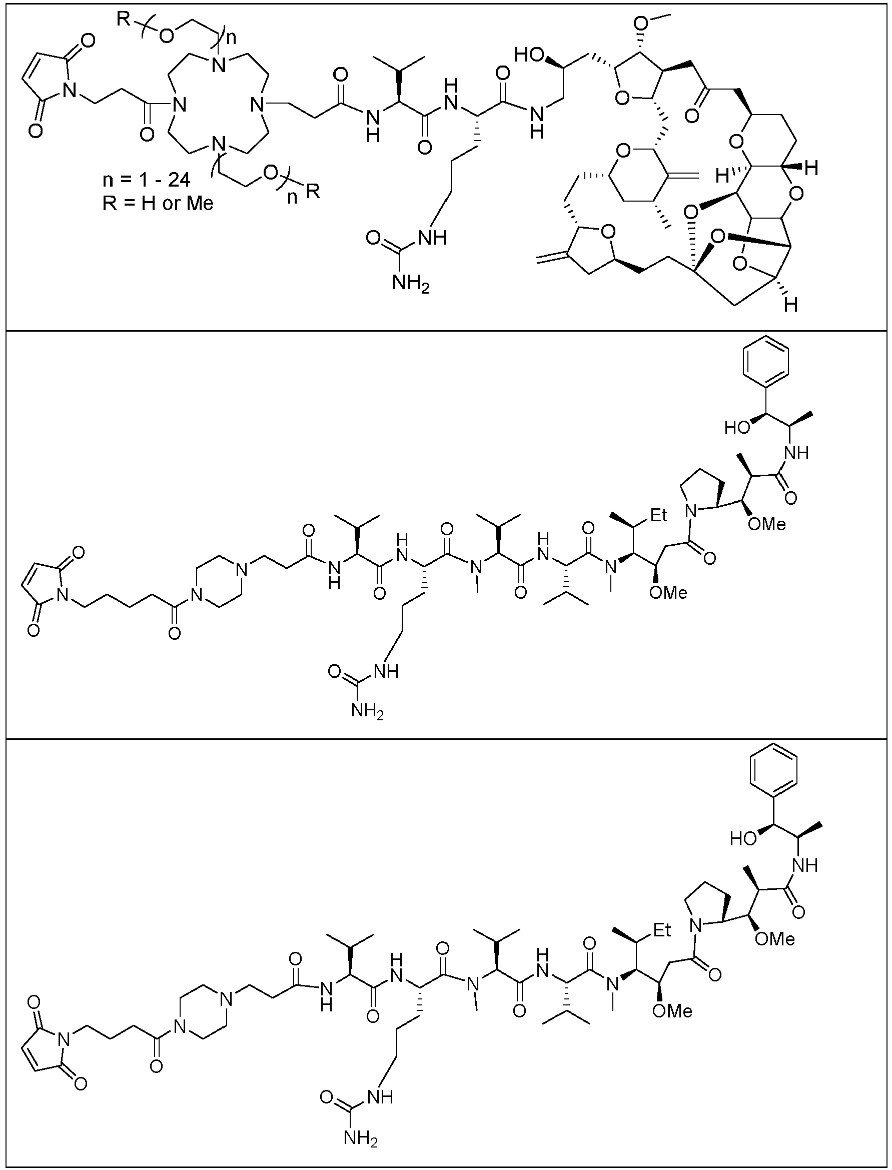 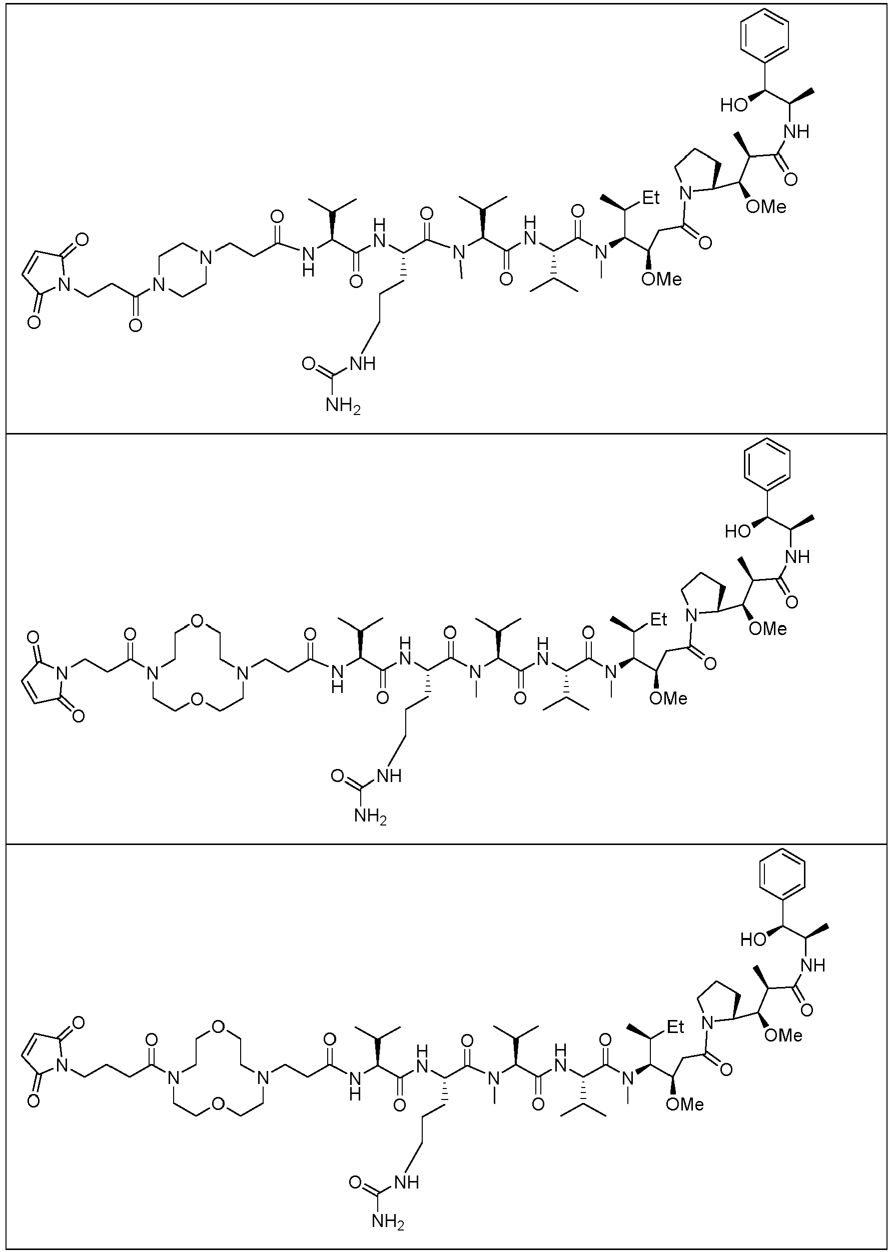 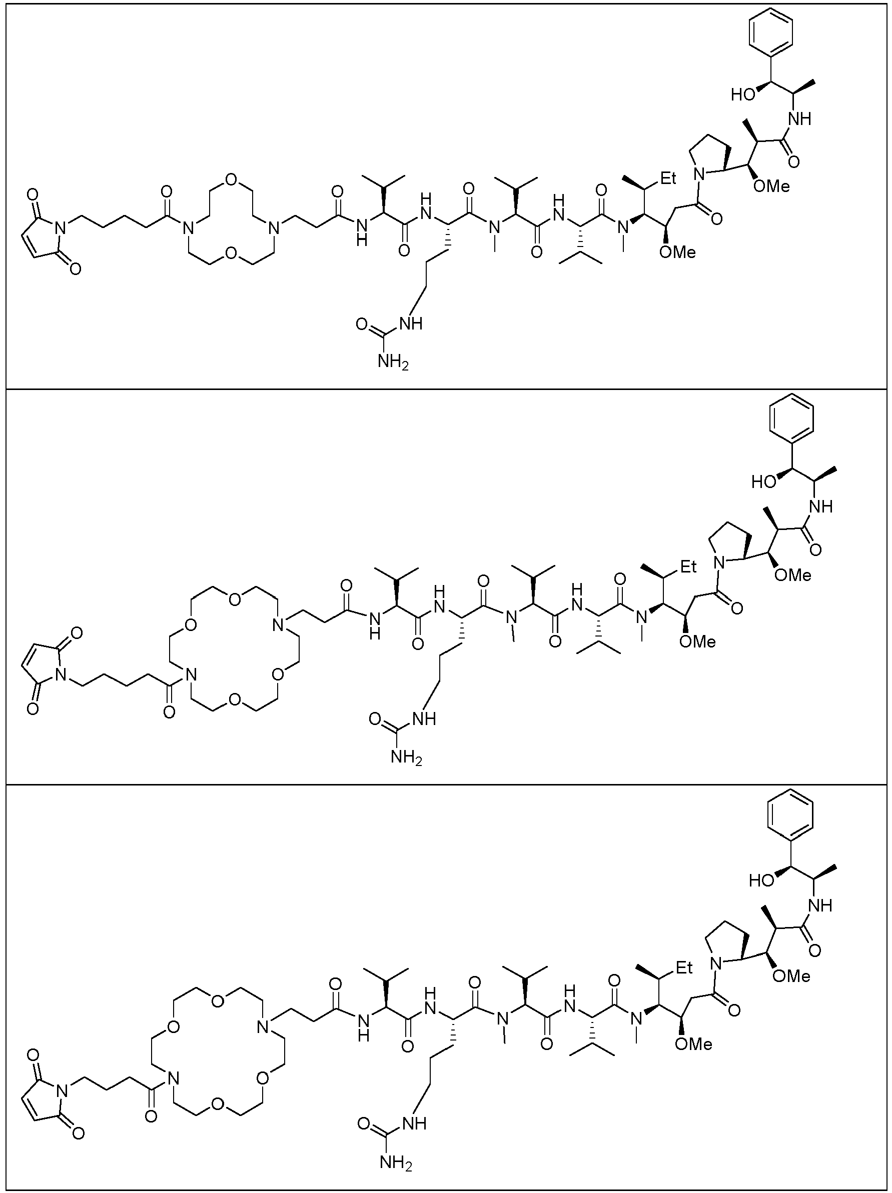 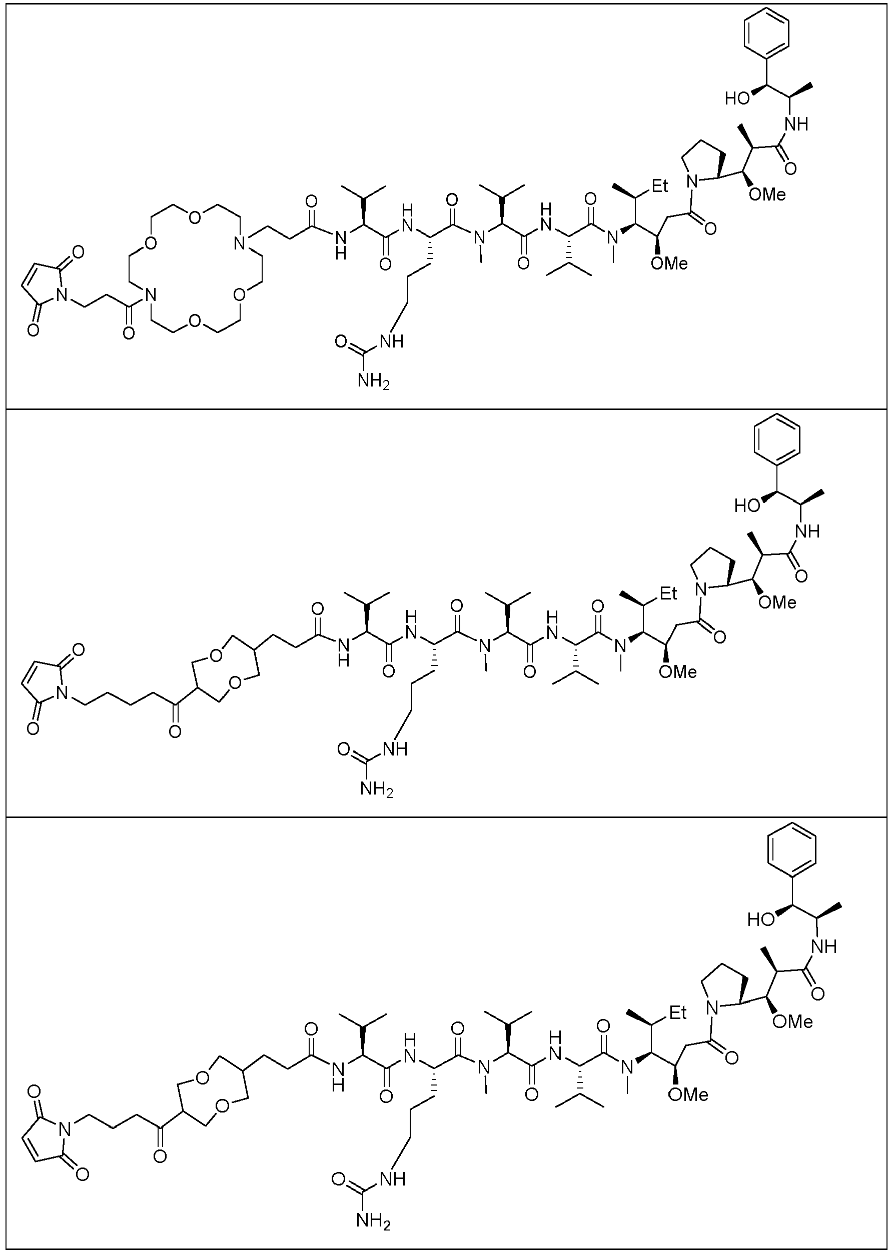 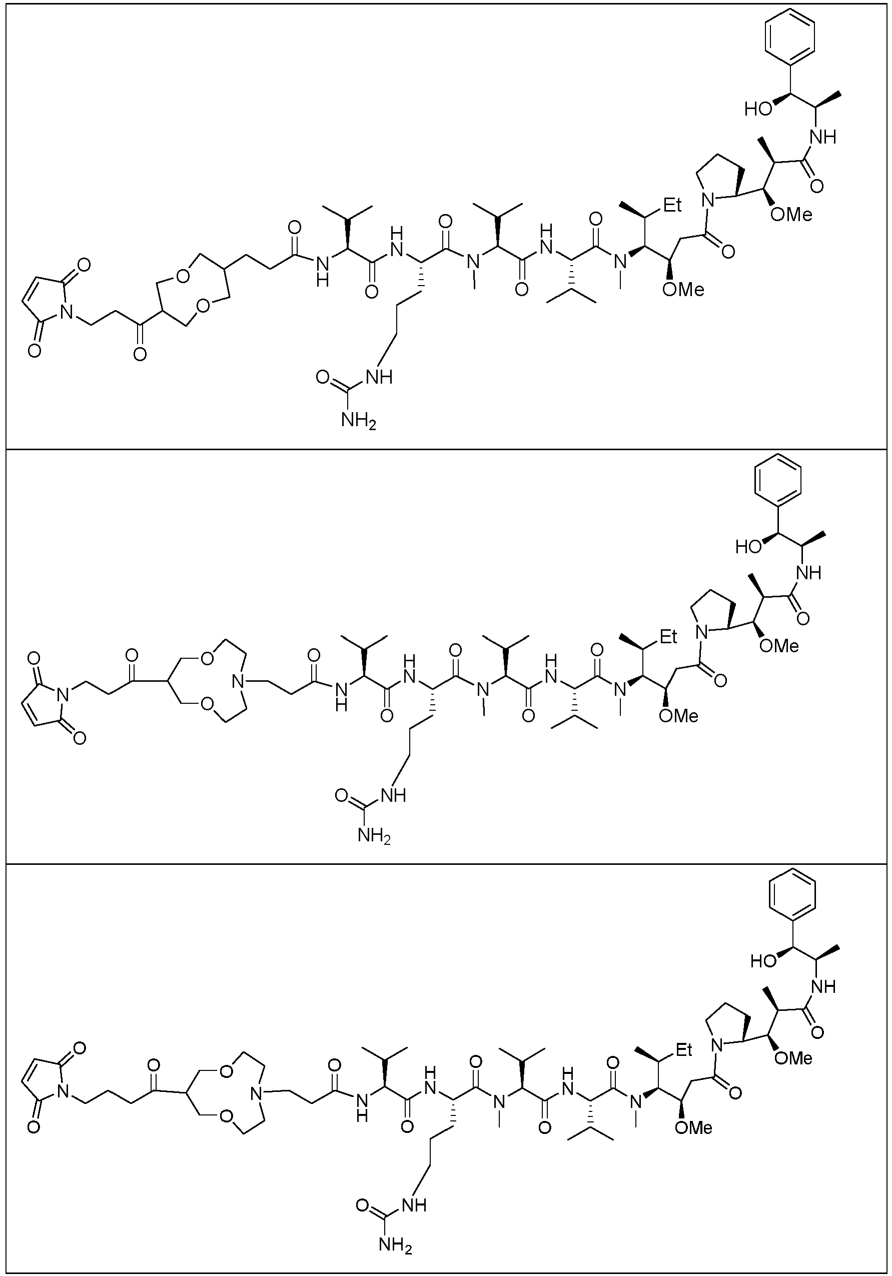 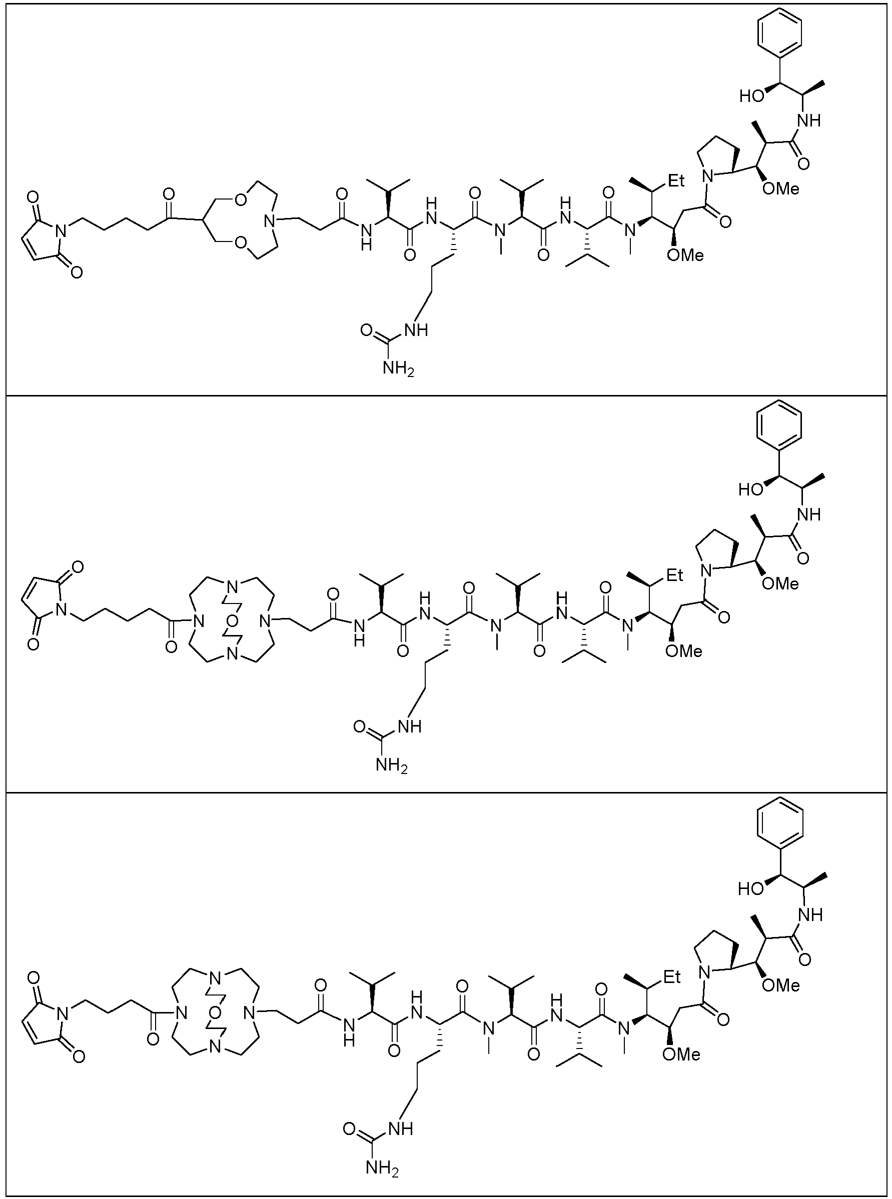 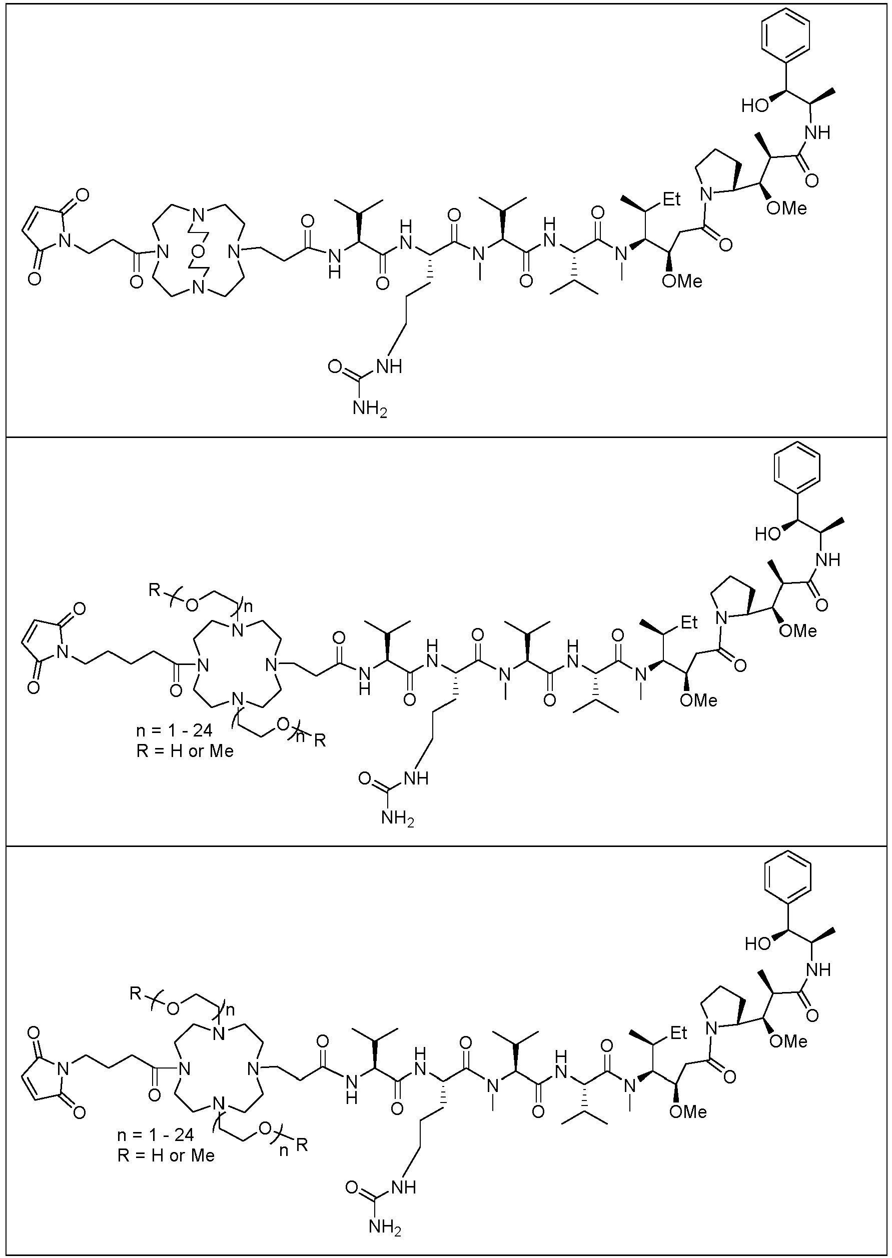 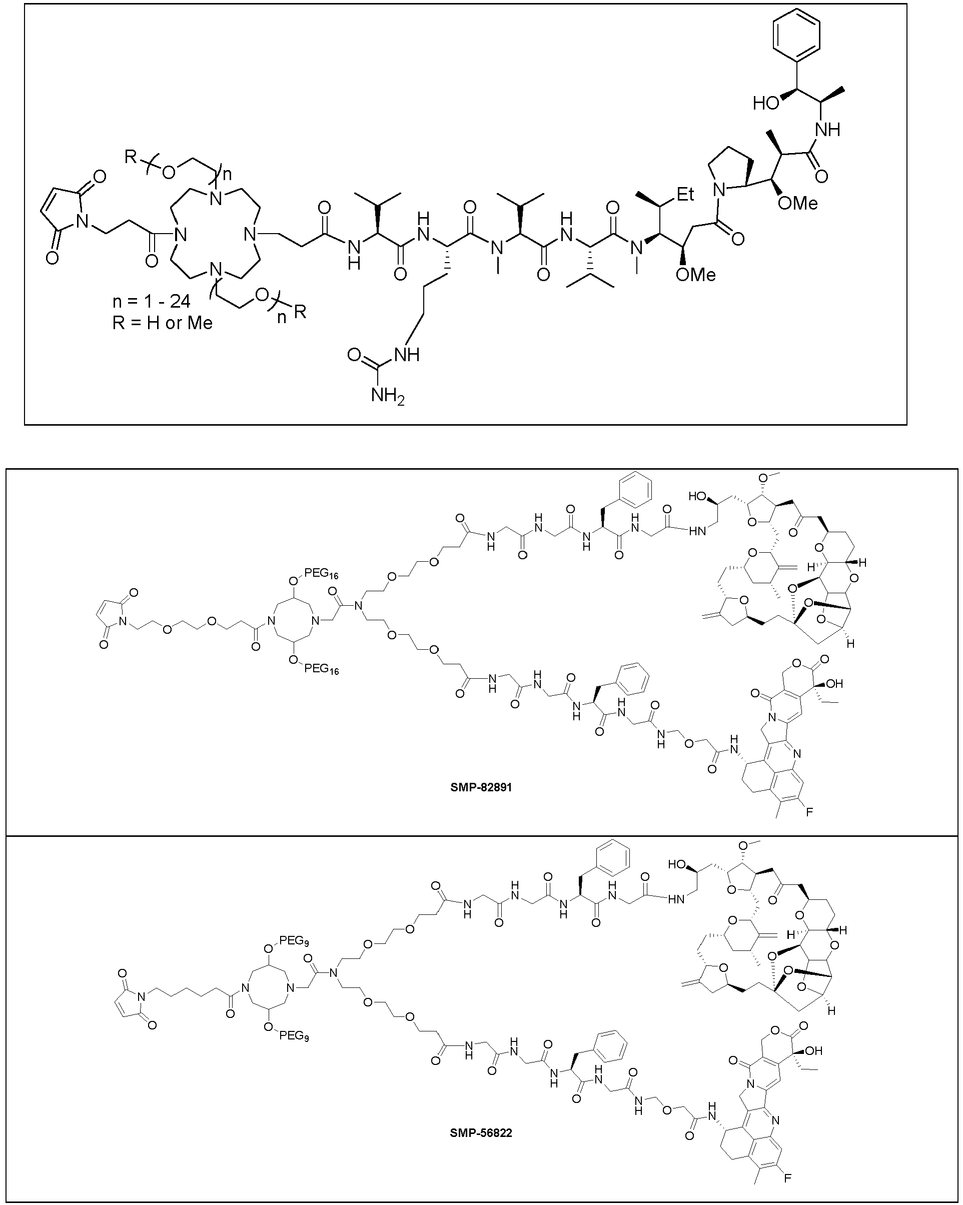 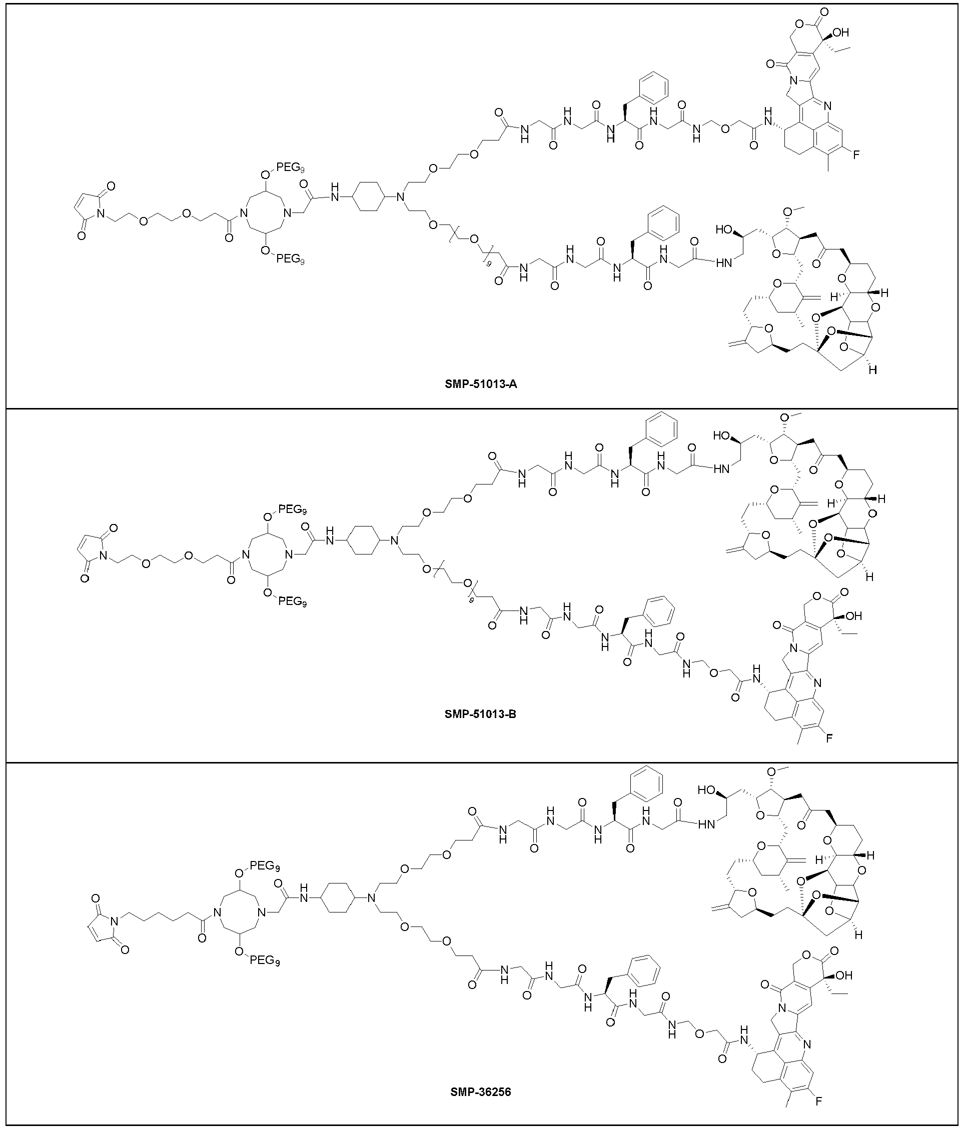 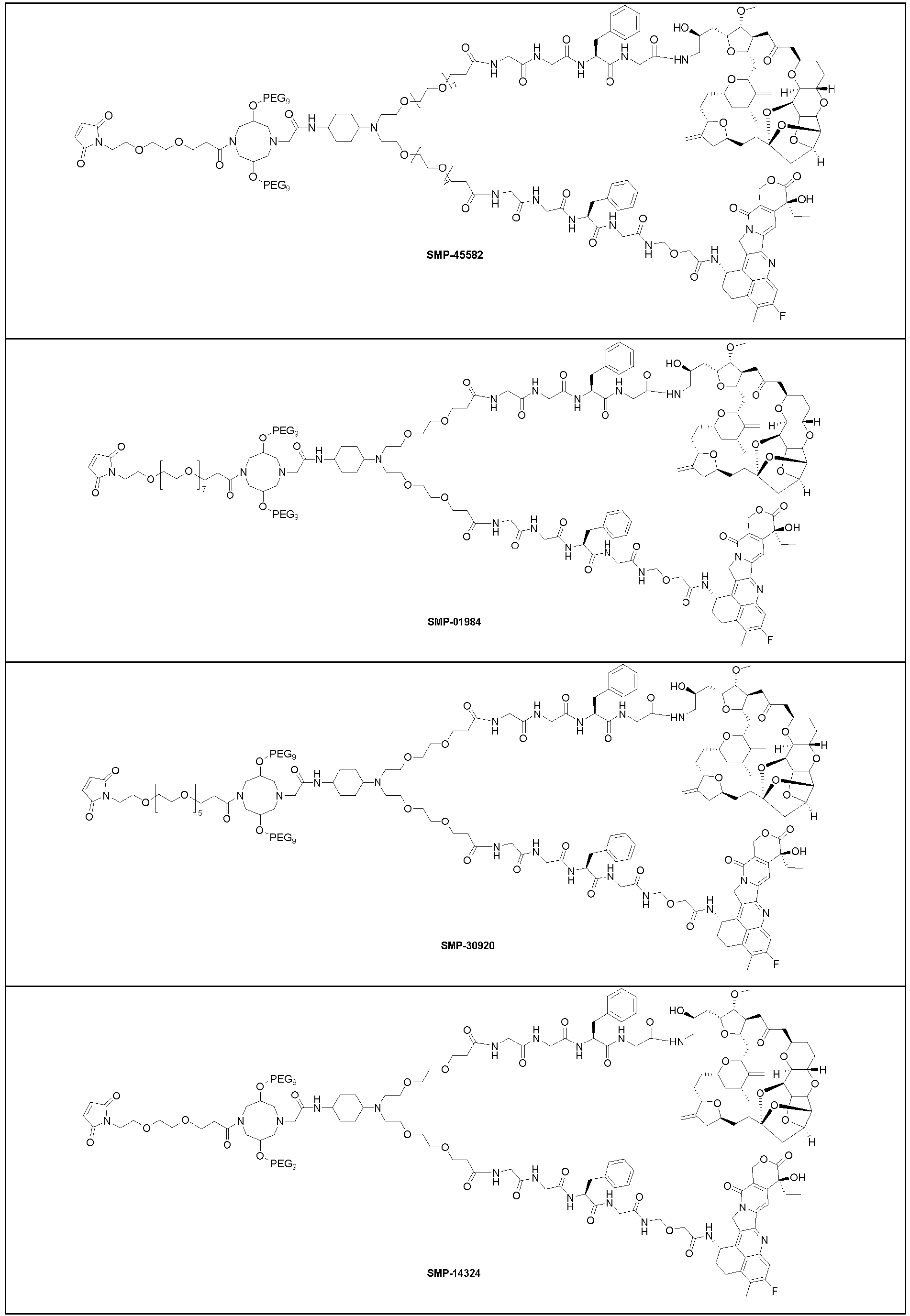 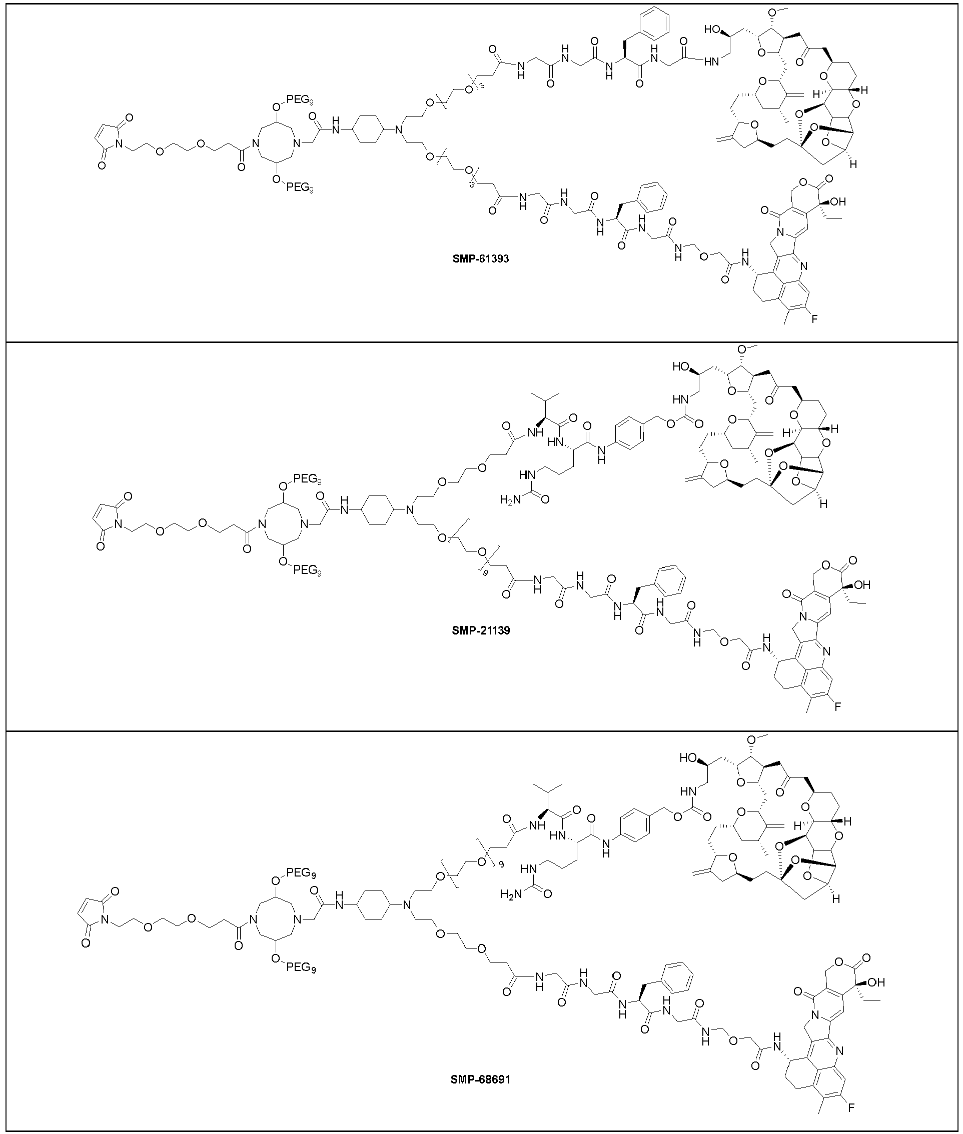 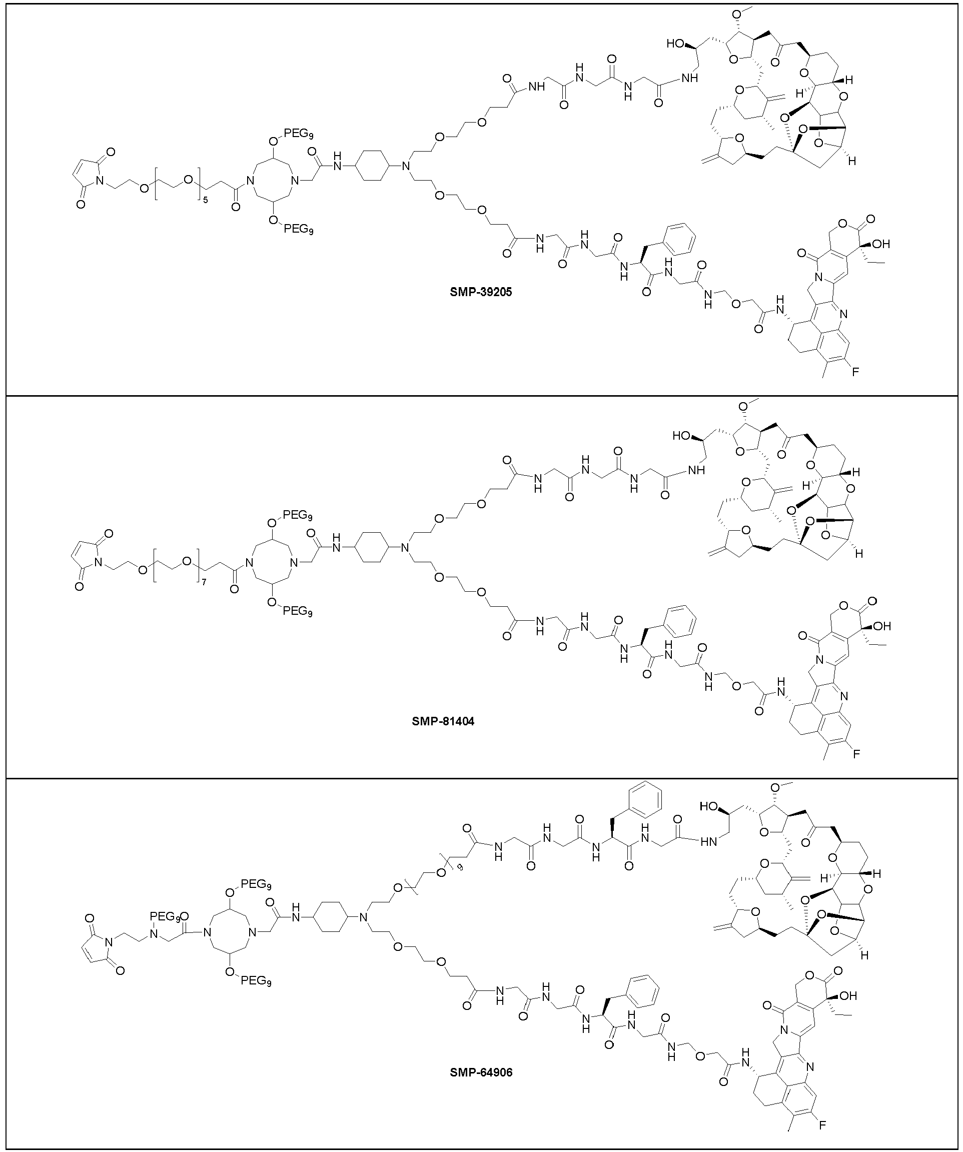 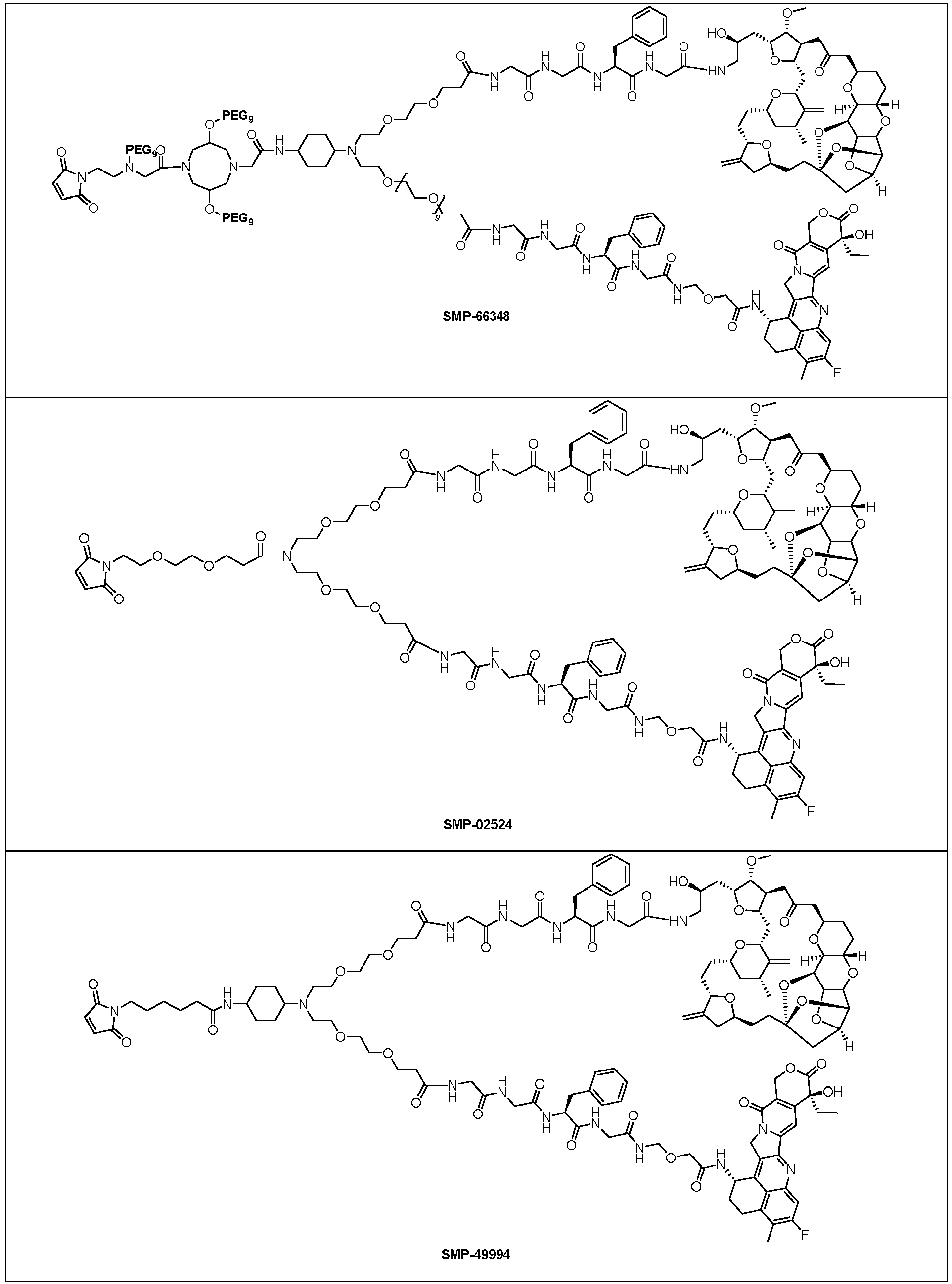 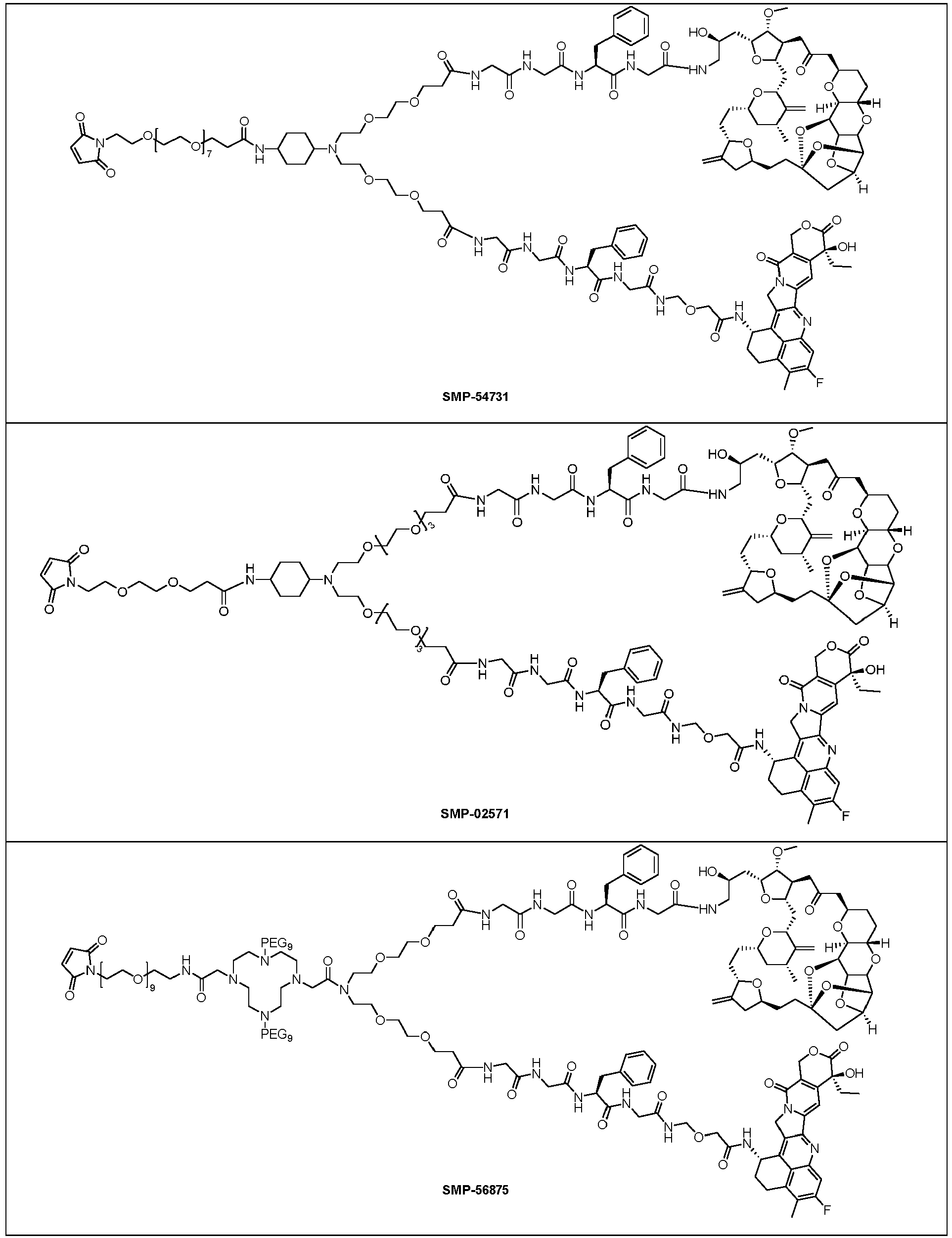 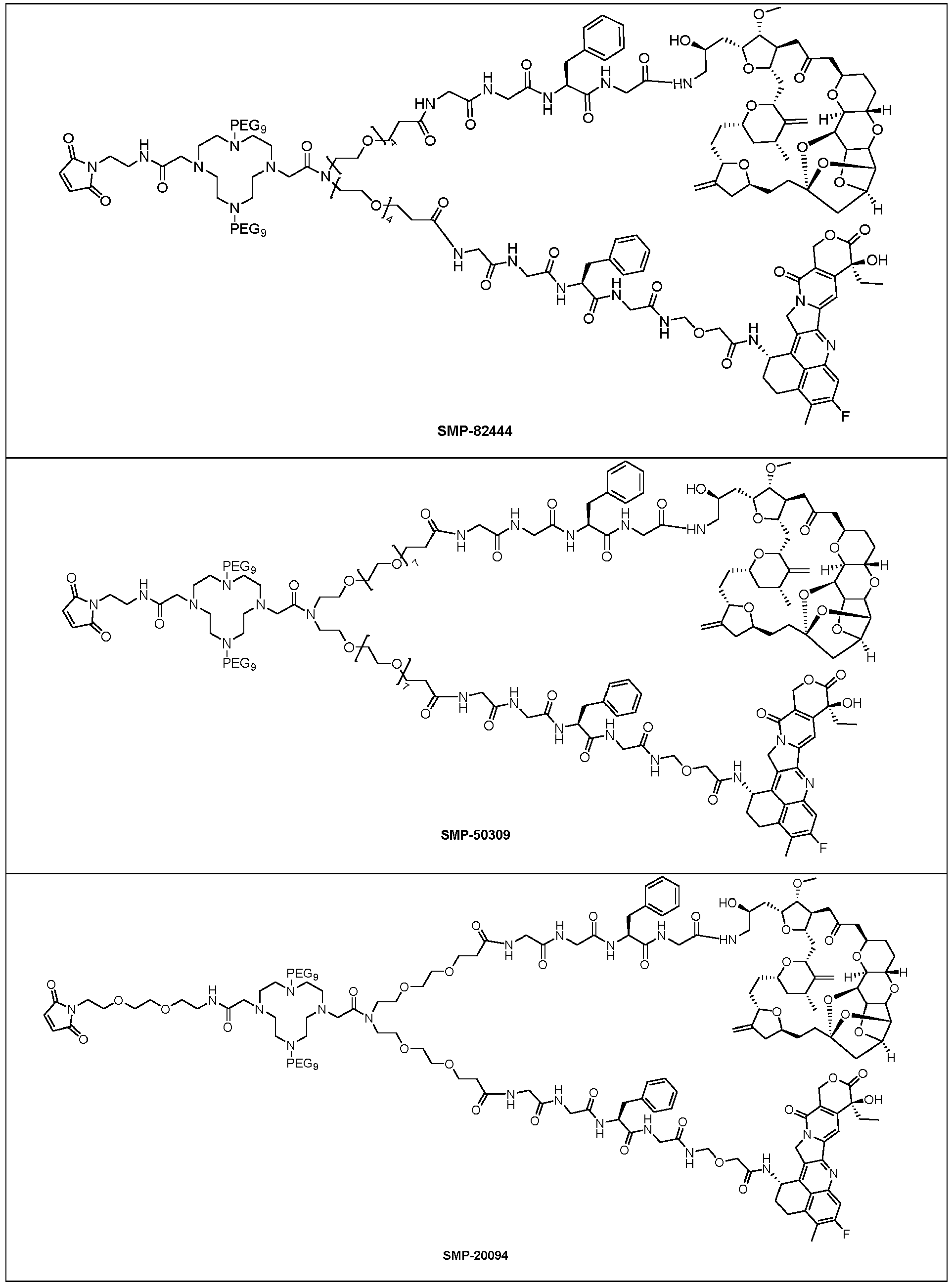 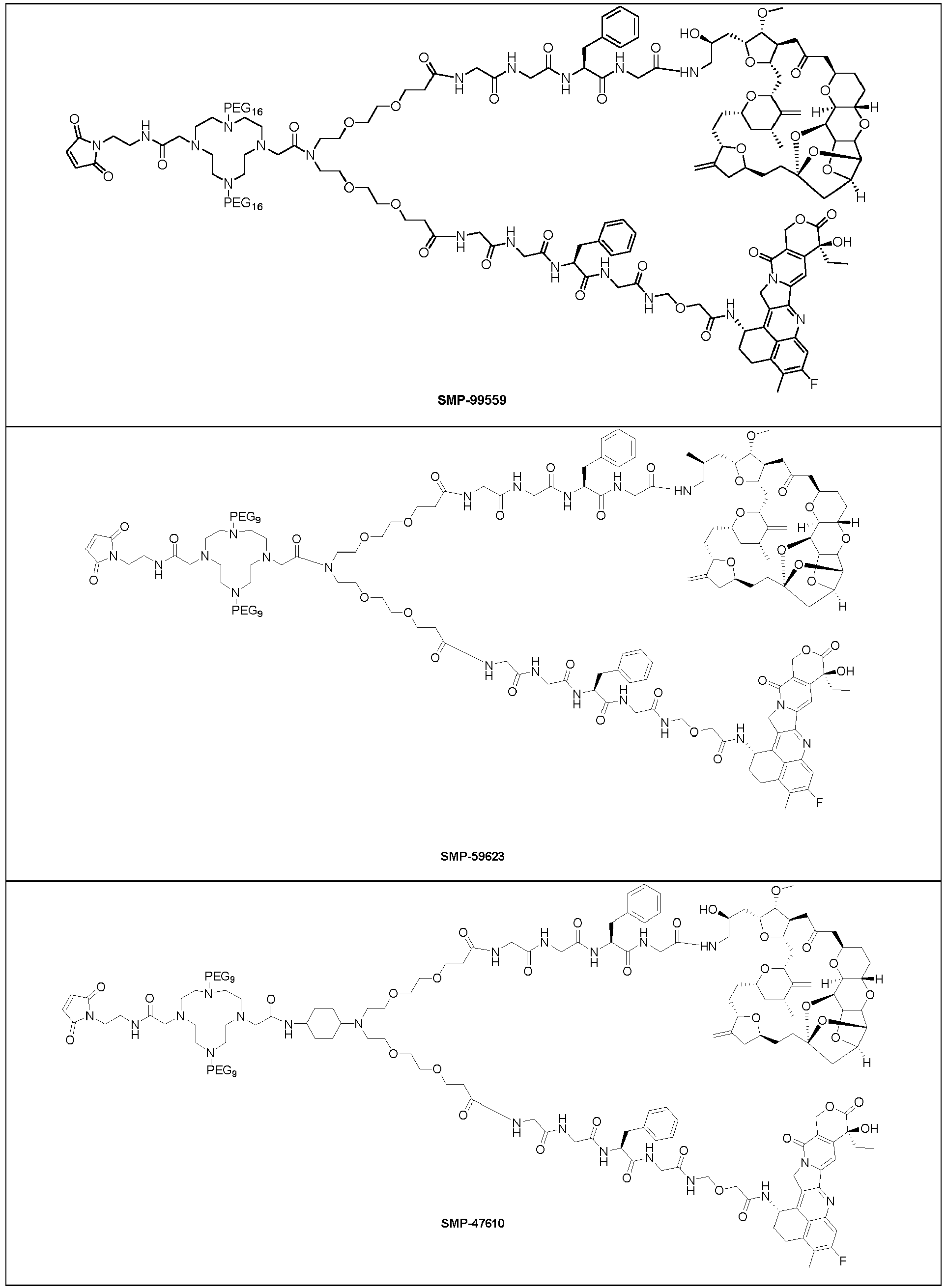 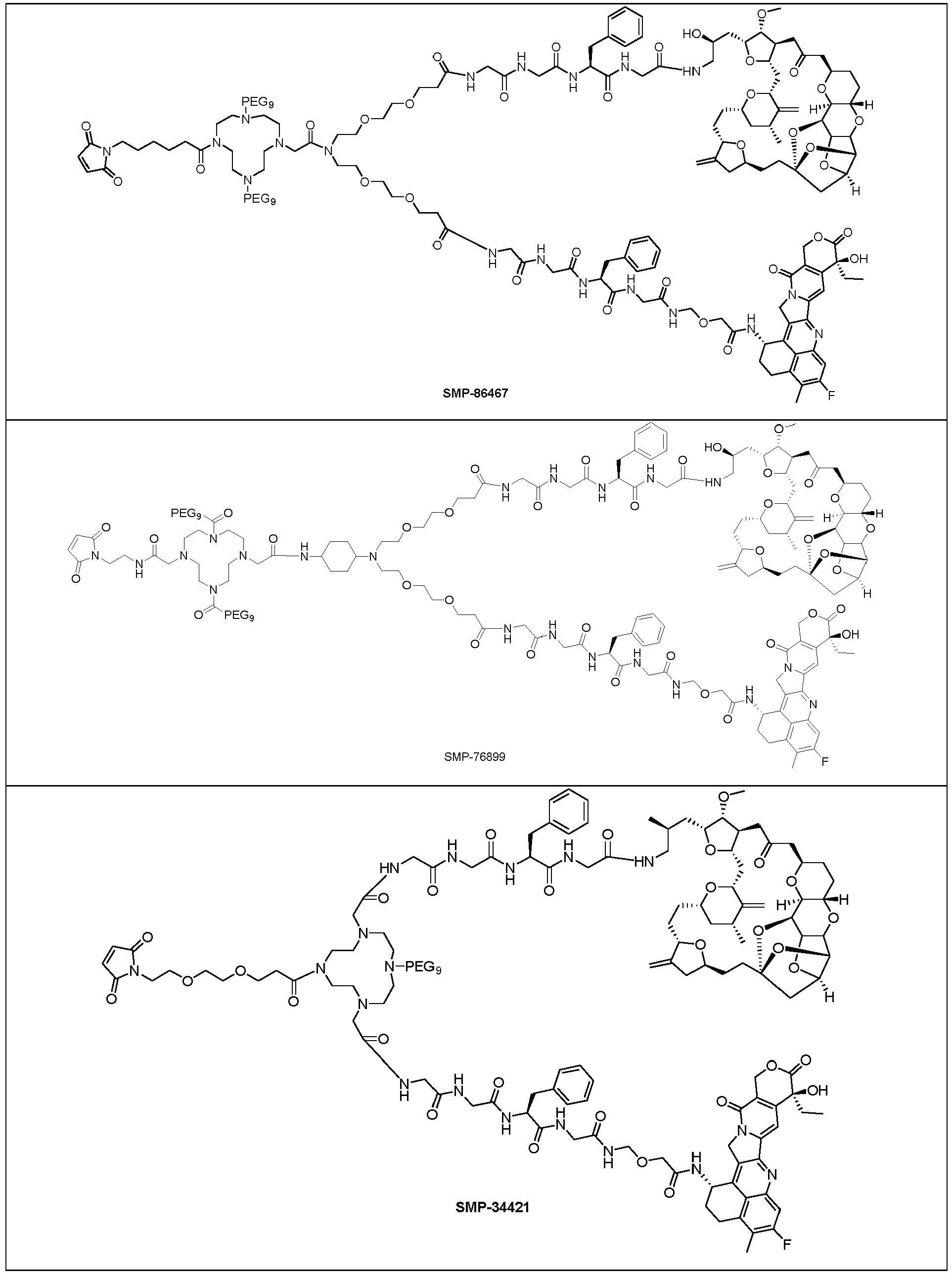 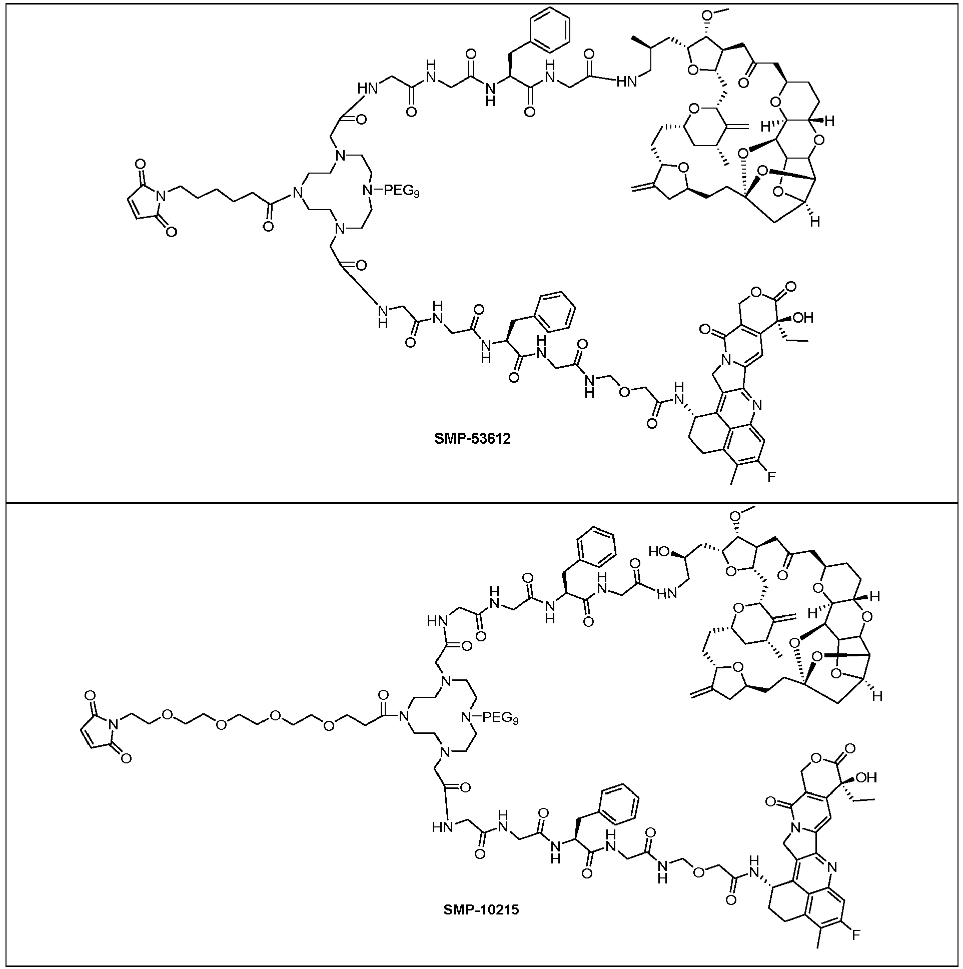 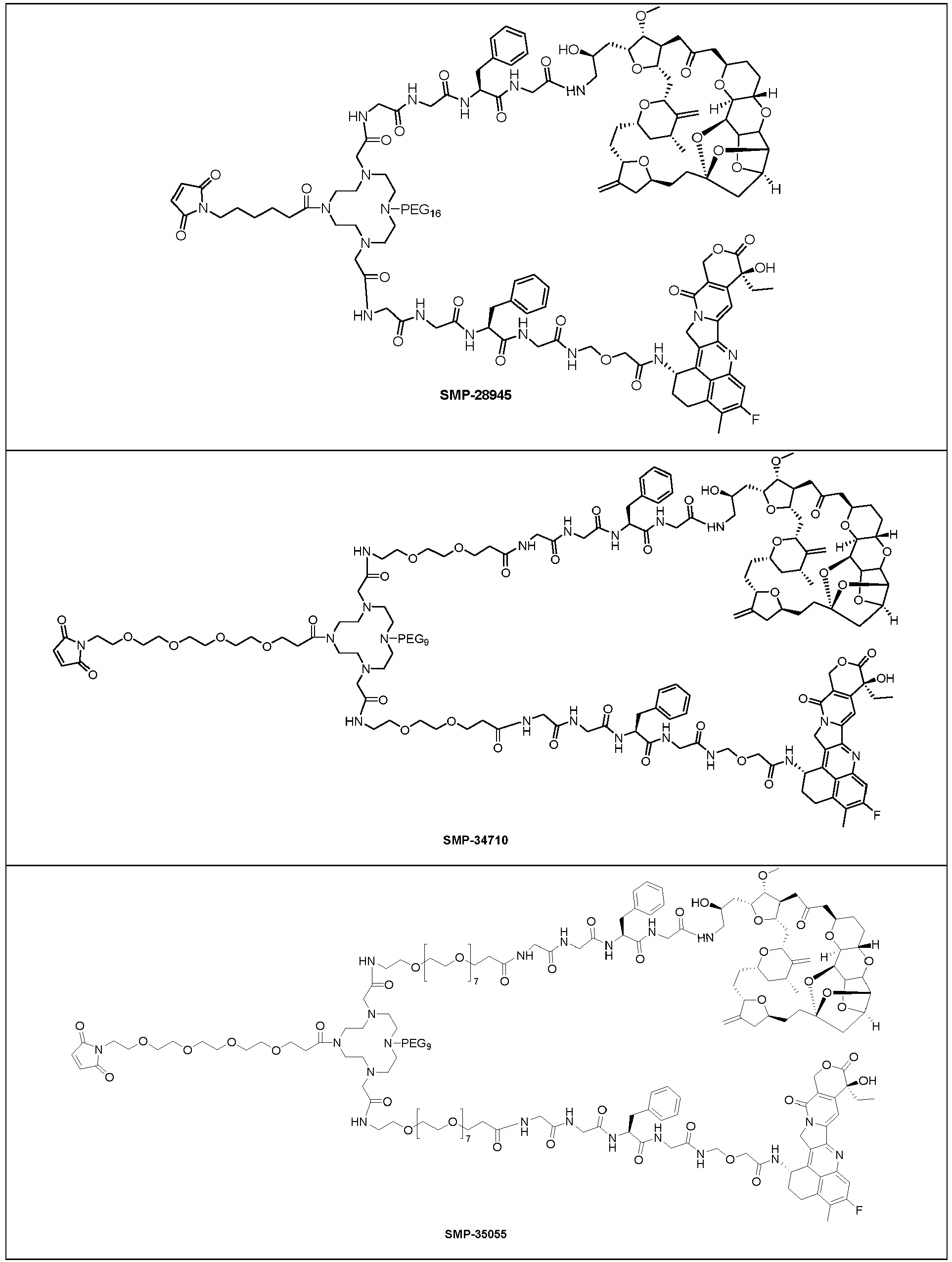 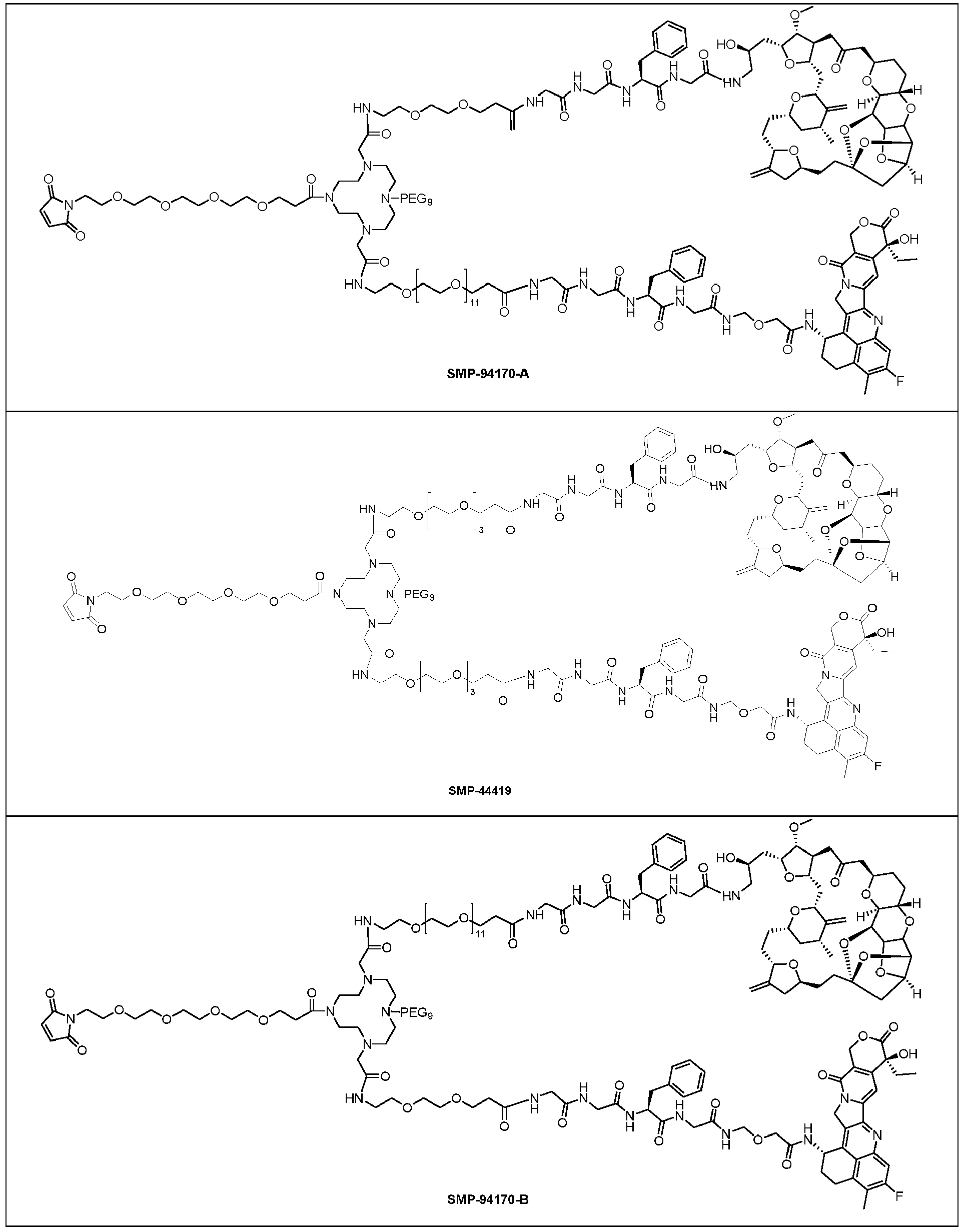 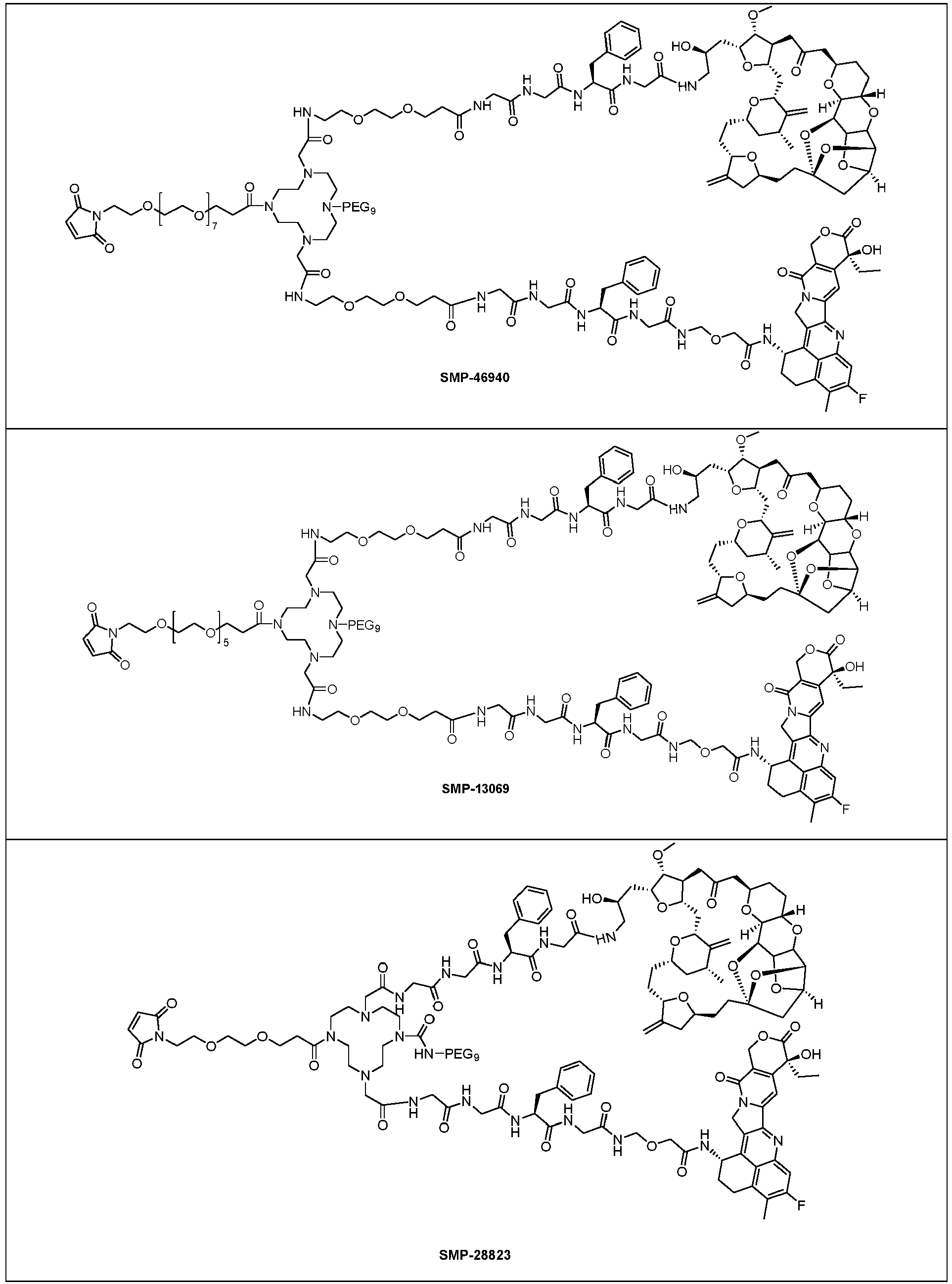 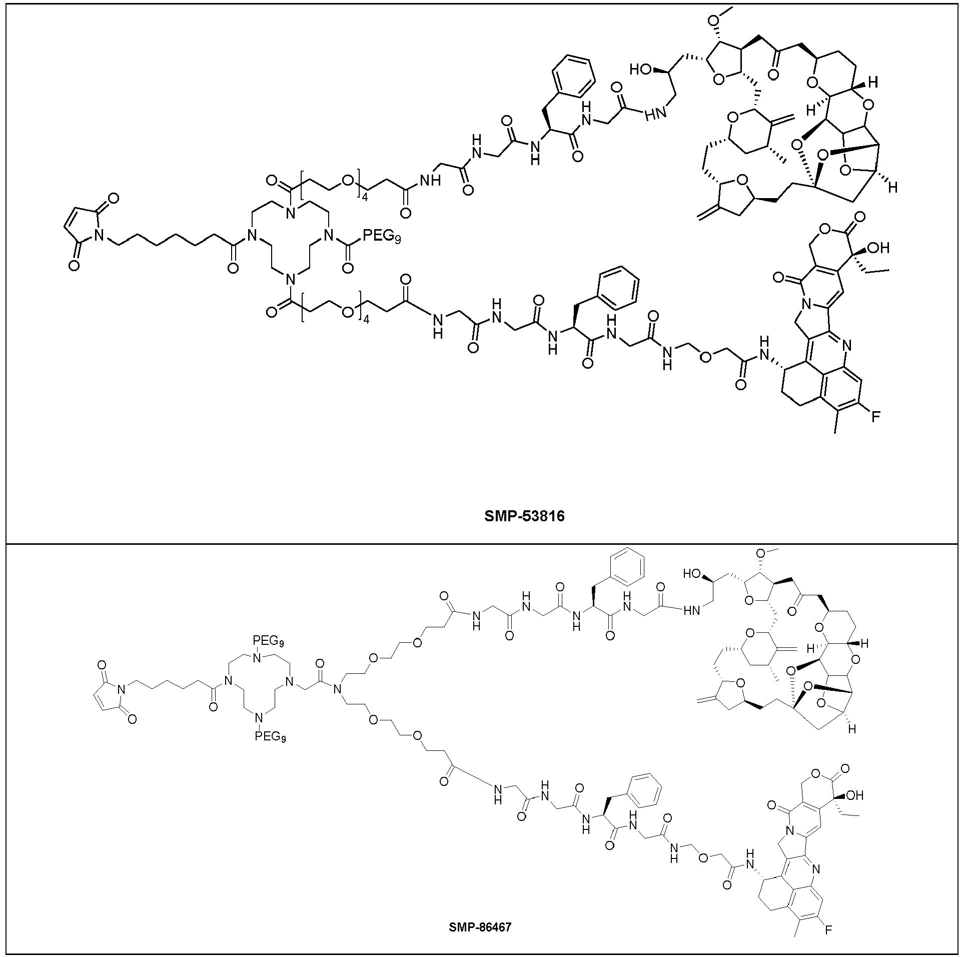 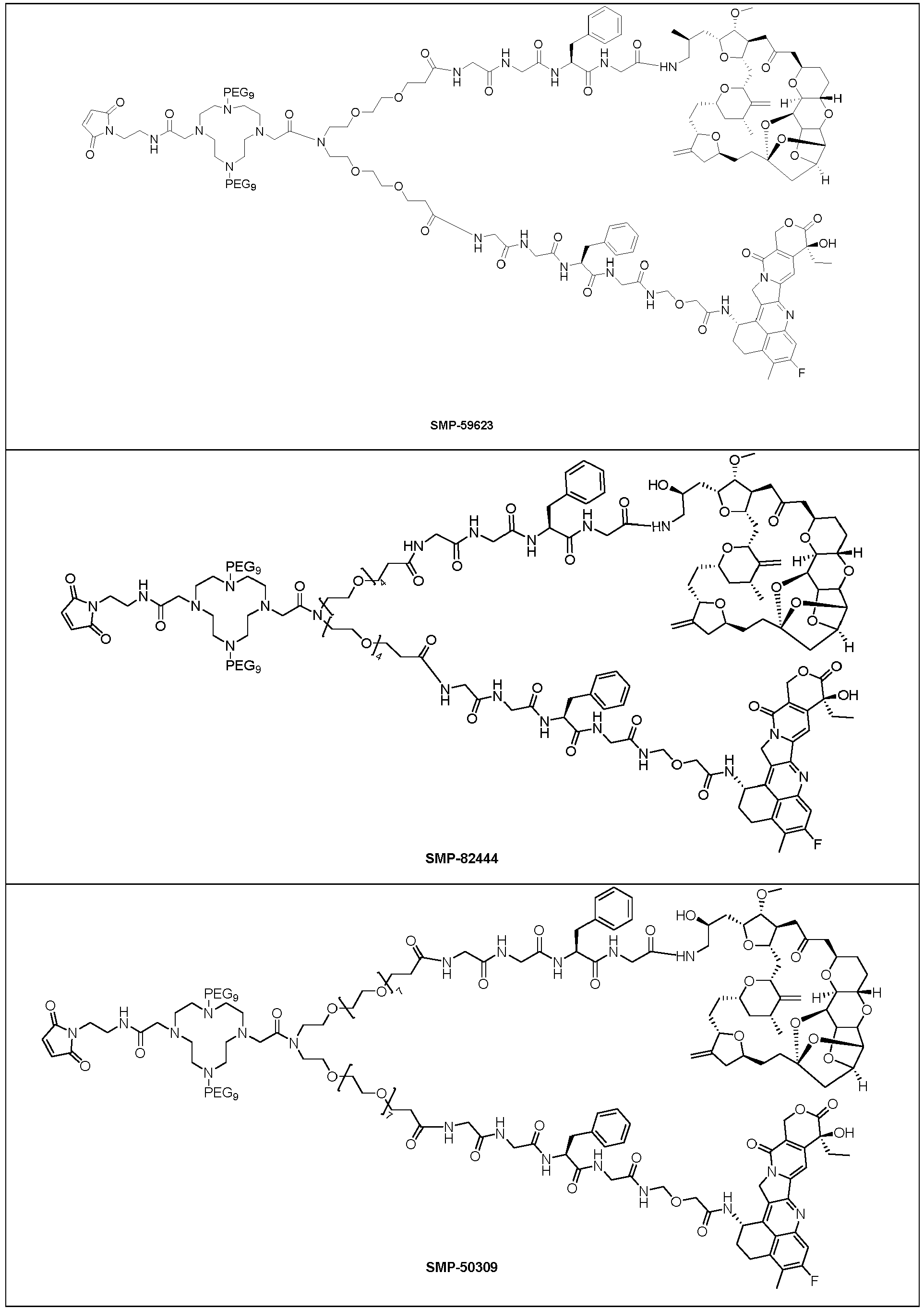 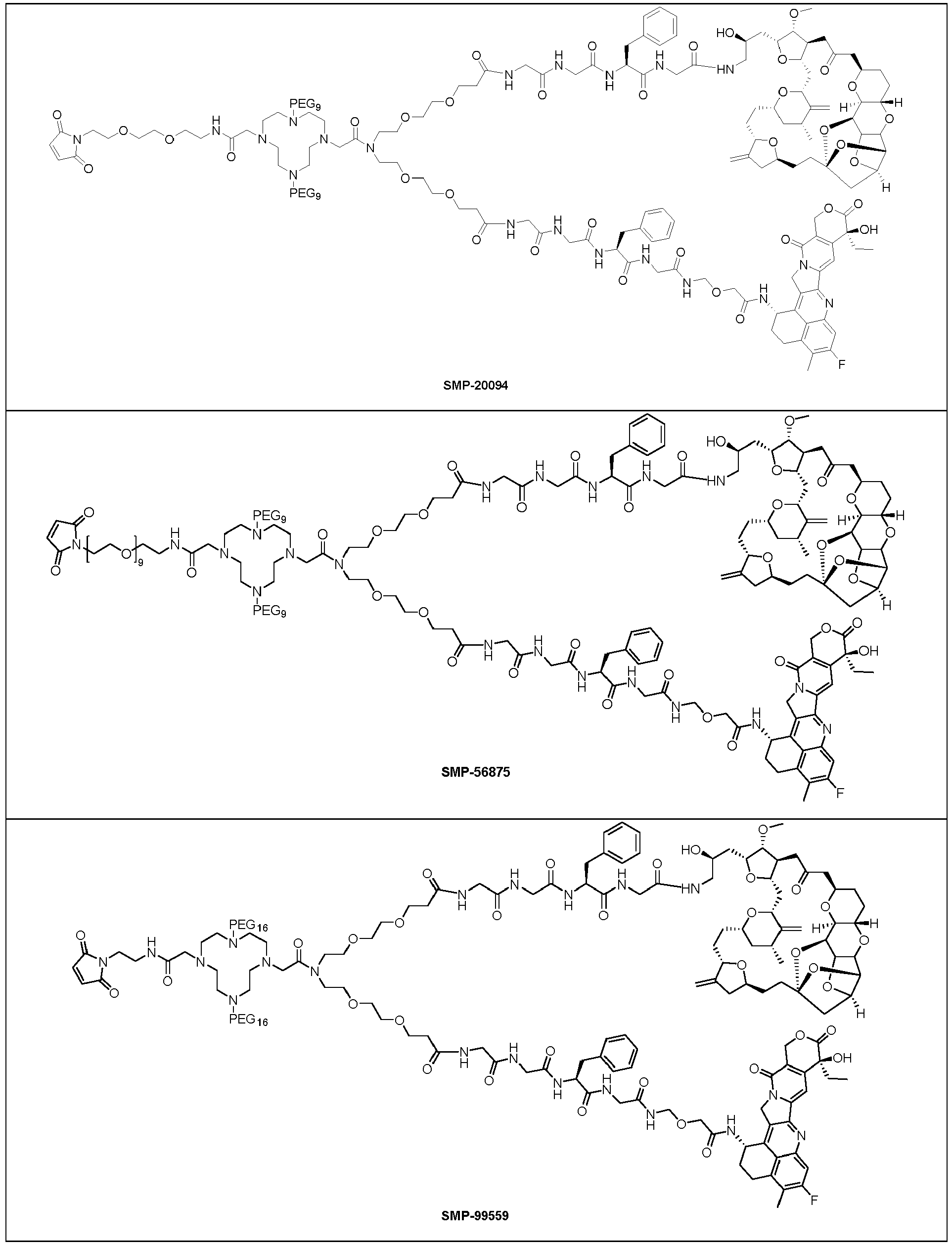 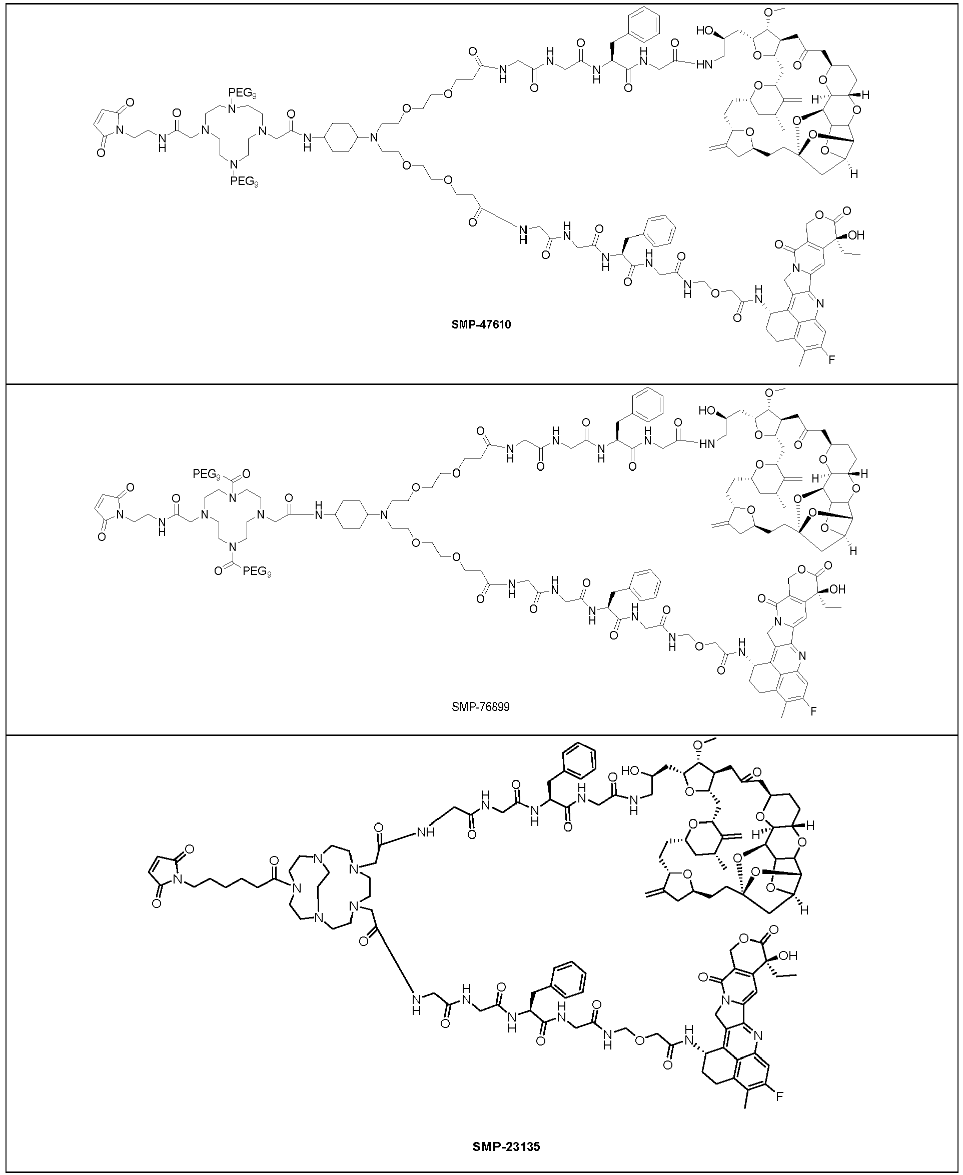 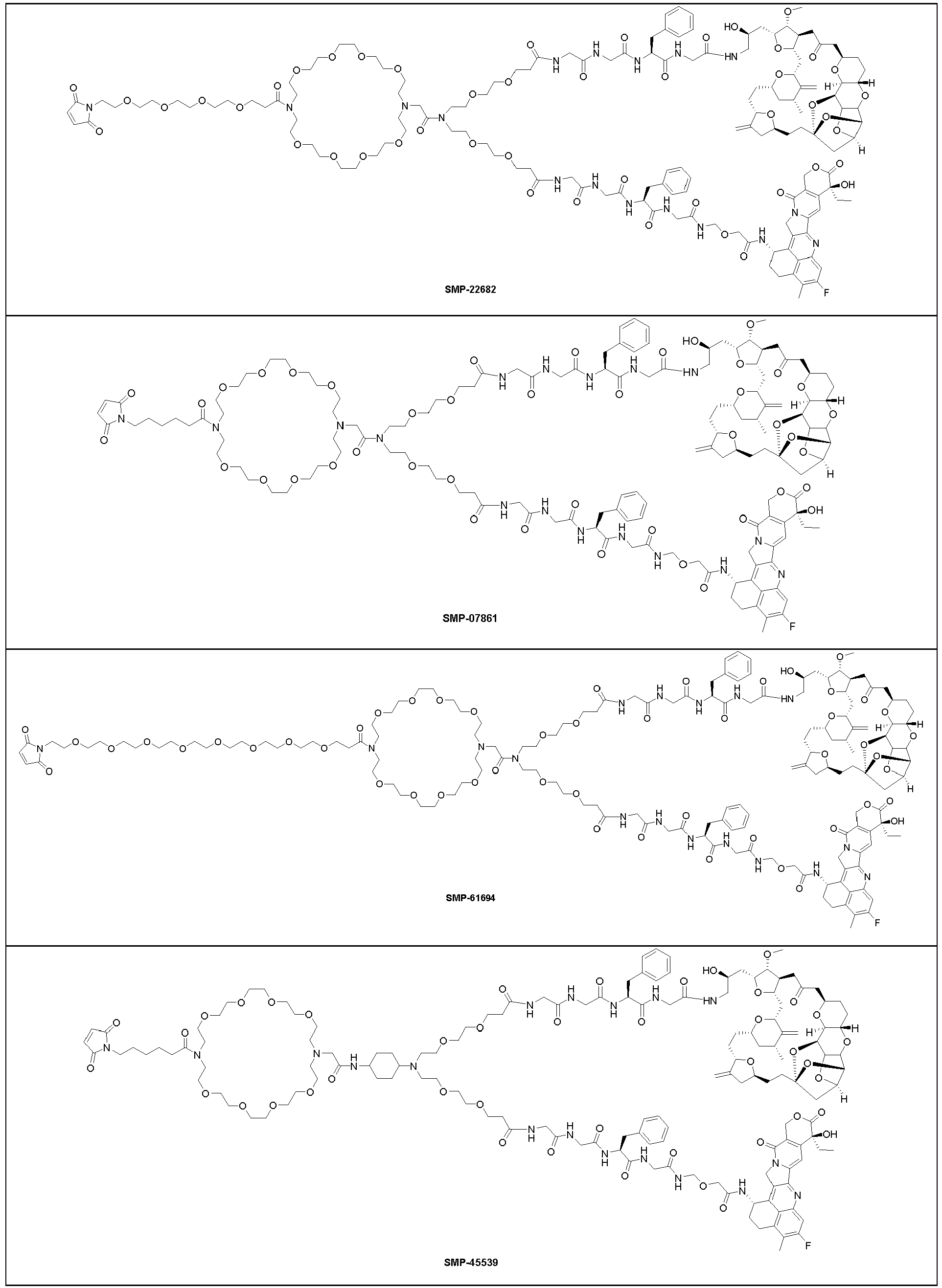 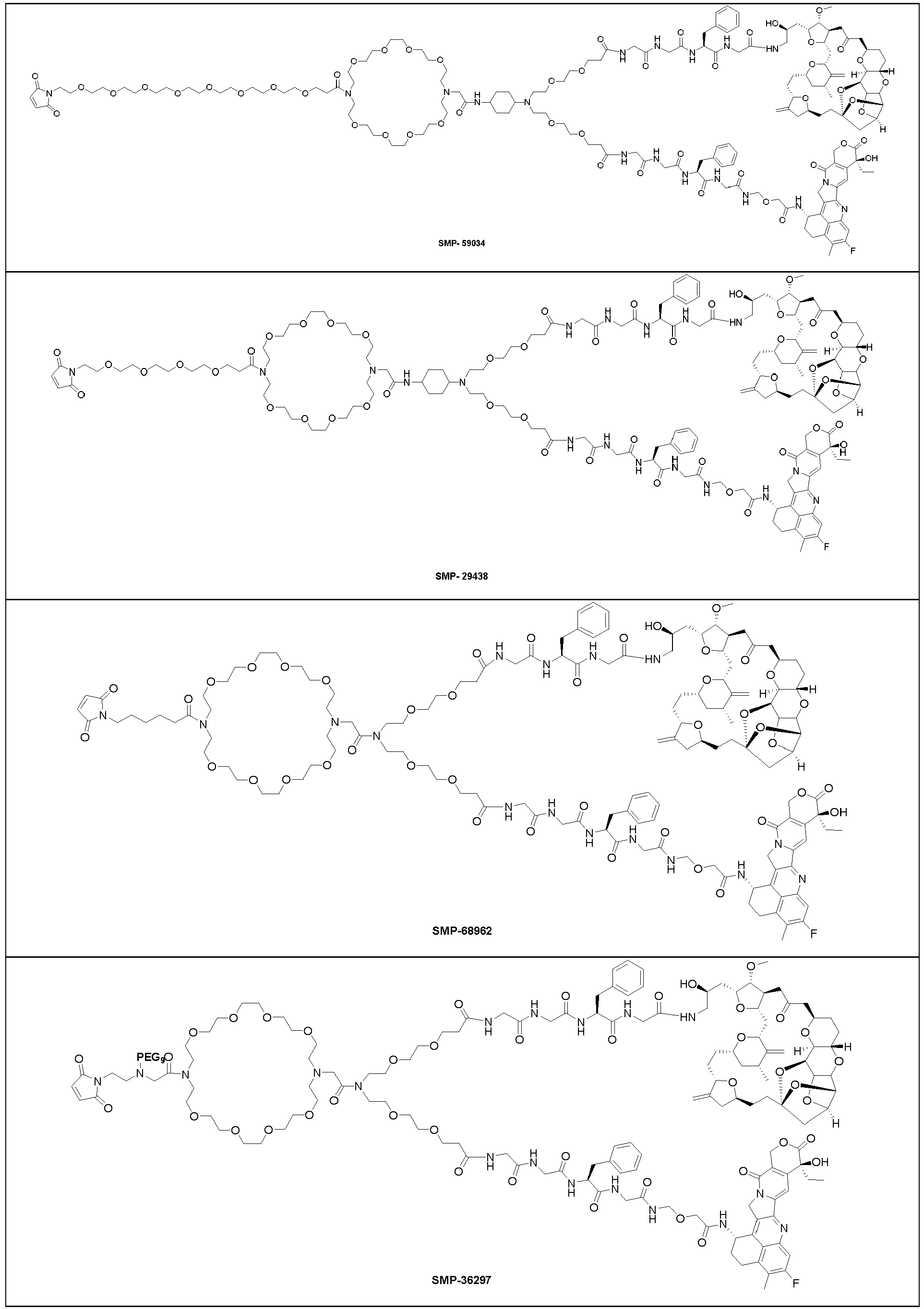 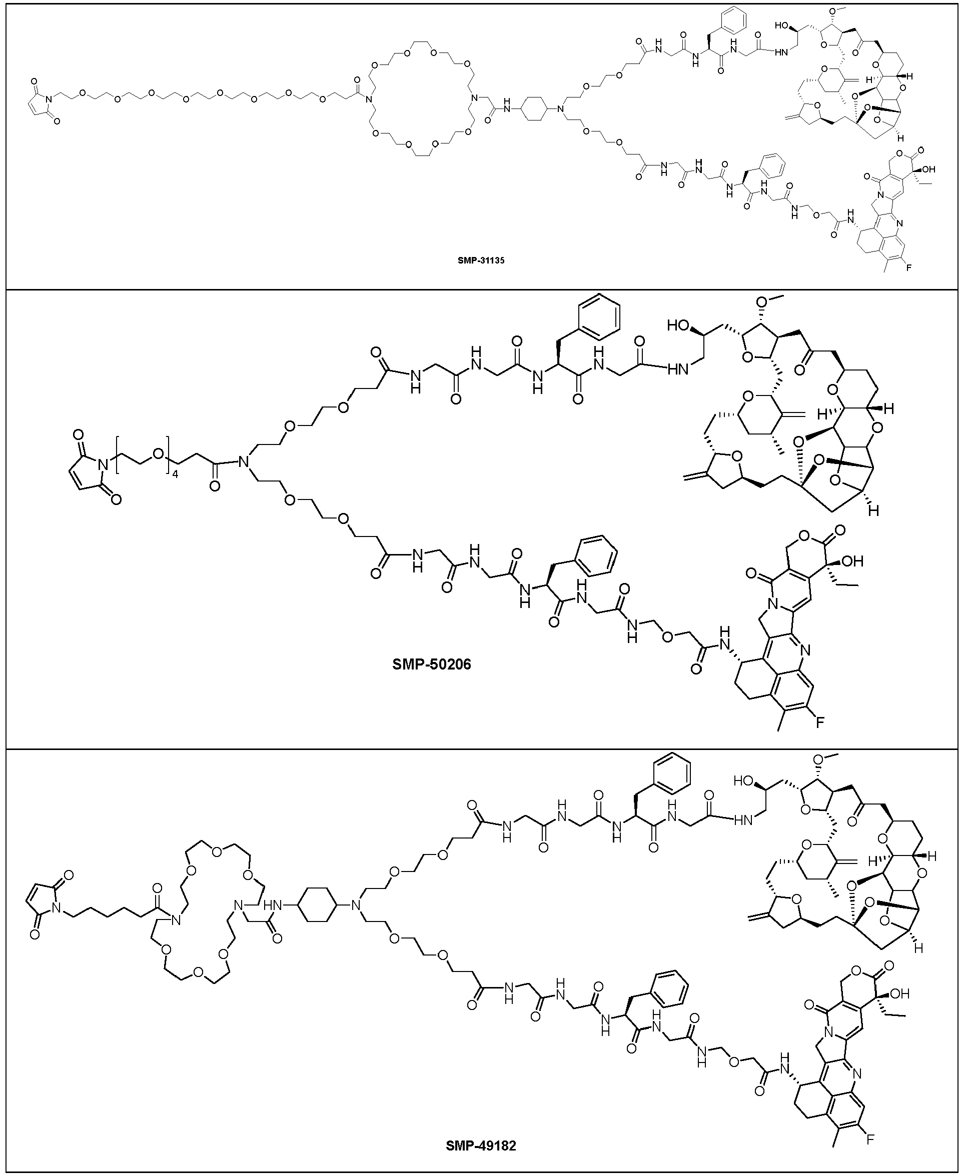 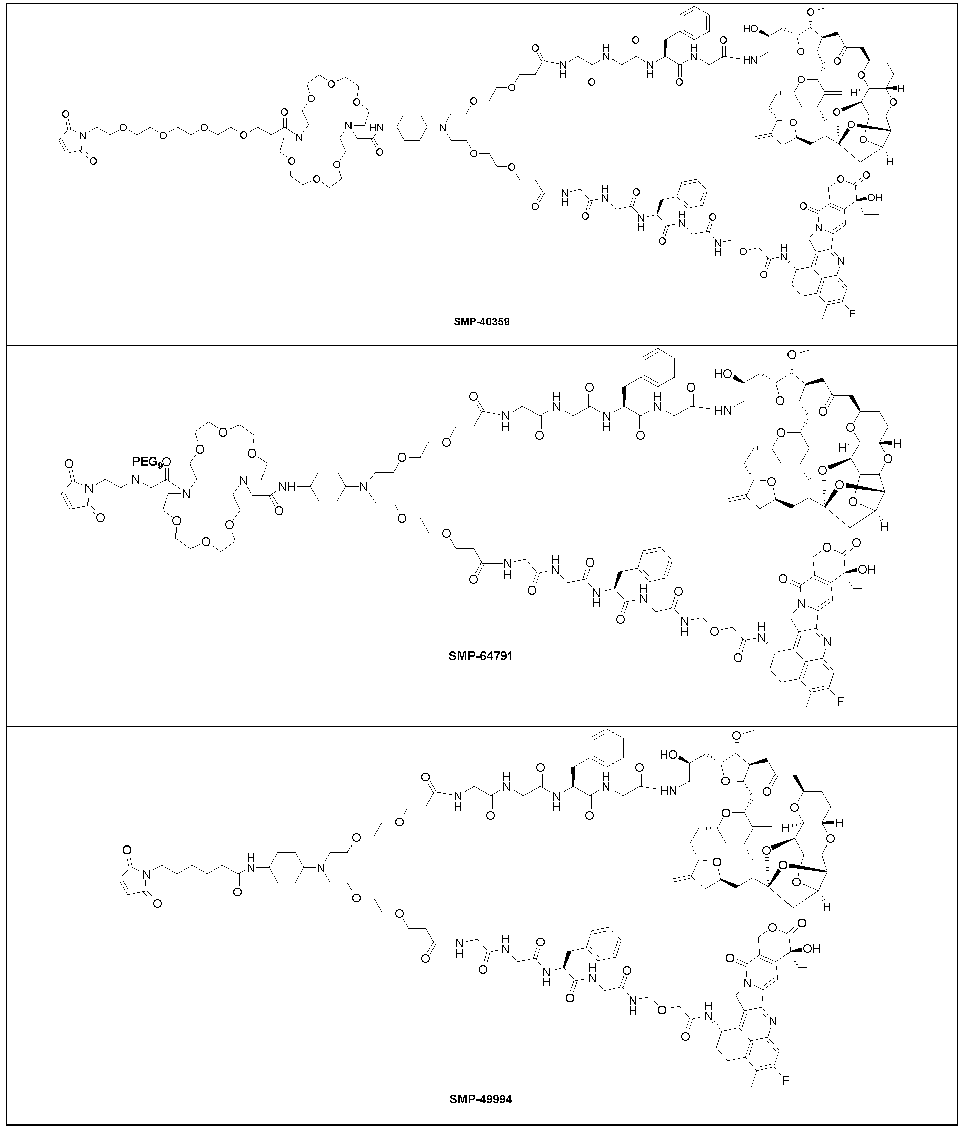 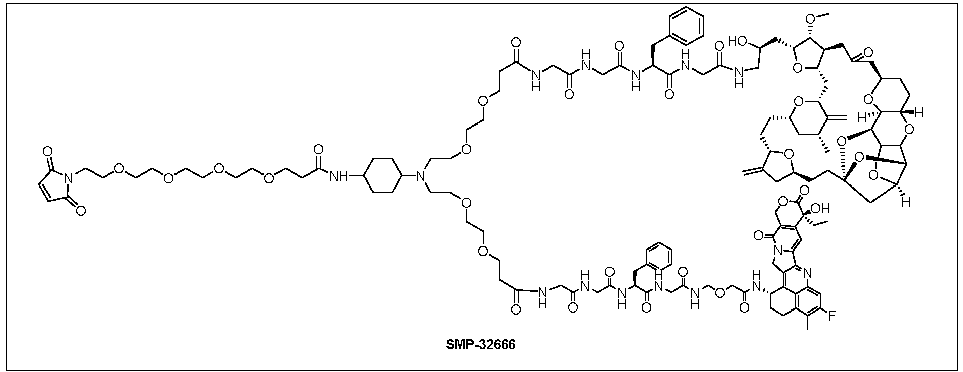
- PEG₉ represents 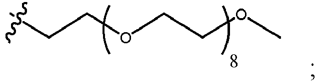 and -PEG₁₆ represents

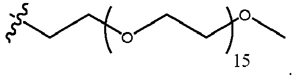

**37.** A targeted linker-drug conjugate or a stereoisomer, optical isomer, salt, or deuterated compound thereof, **characterized in that** the targeted linker-drug conjugate is obtained by connecting a targeted linker to the drug link assembly unit according to any one of claims 1-36, and has a structure as shown in formula J-1; 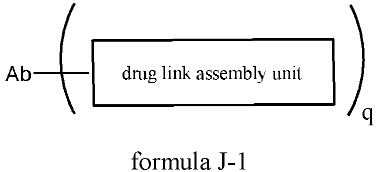
Ab is the targeted linker; q is an integer from 1 to 20; and
the targeted linker is a substance capable of targeted binding to a lesion site.

**38.** The targeted linker-drug conjugate or the stereoisomer, optical isomer, salt, or deuterated compound thereof according to claim 37, **characterized in that** the structure of the targeted linker-drug conjugate is as shown in formula J-2 or J-3: 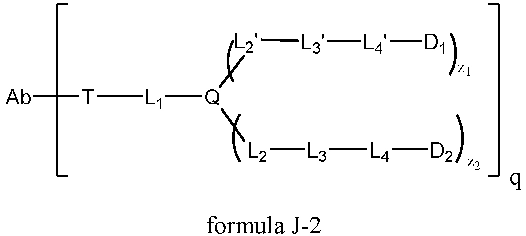 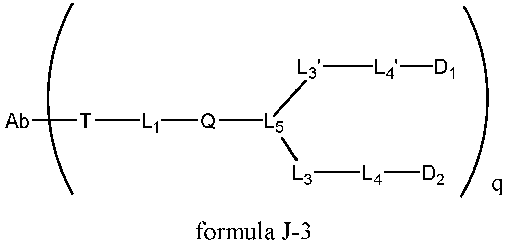
wherein Ab is the targeted linker; q is an integer from 1 to 20;
T, L₁, Q, L₂, L₃, L₄, L₂', L₃', L₄', L₅, D₁, D₂, Z₁, and Z₂ are as described in any one of claims 1-35;
preferably, the structure of the targeted linker-drug conjugate is as shown in formula J-4:
Ab-(T-L₁-Q-L₂-L₃-L₄-D₂)_{q} formula J-4
wherein Ab is the targeted linker; q is an integer of 1 to 20; and
T, L₁, Q, L₂, L₃, L₄, and D₂ are as described in any one of claims 1-35. 39. The targeted linker-drug conjugate or the stereoisomer, optical isomer, salt, or deuterated compound thereof according to any one of claims 37 and 38, **characterized in that** a DAR value of the targeted linker-drug conjugate is 1.00 to 20.00, preferably 2.0 to 8.0, and more preferably 2.0 to 5.0.

**40.** The targeted linker-drug conjugate or the stereoisomer, optical isomer, salt, or deuterated compound thereof according to any one of claims 37-39, **characterized in that** the structure of the targeted linker-drug conjugate is selected from:
| | |
|---|---|
| SMP-82891a | 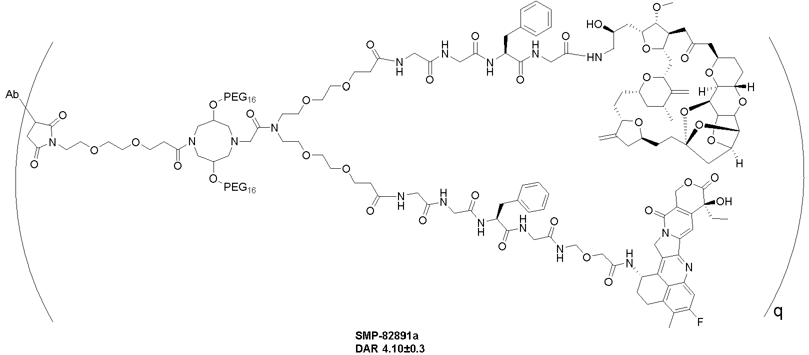 |
| SMP-56822a | 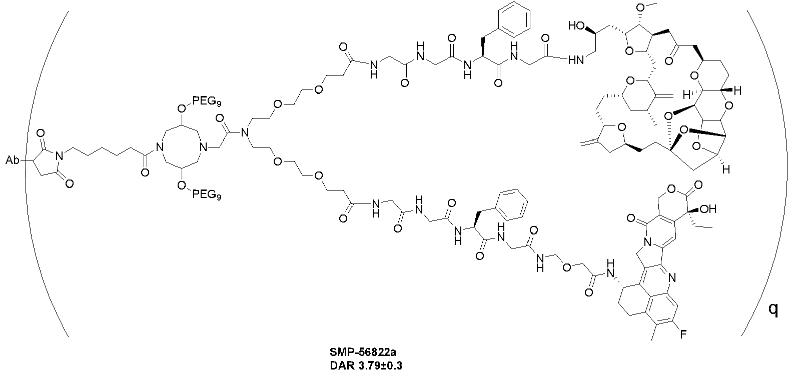 |
| SMP-51013-Aa | 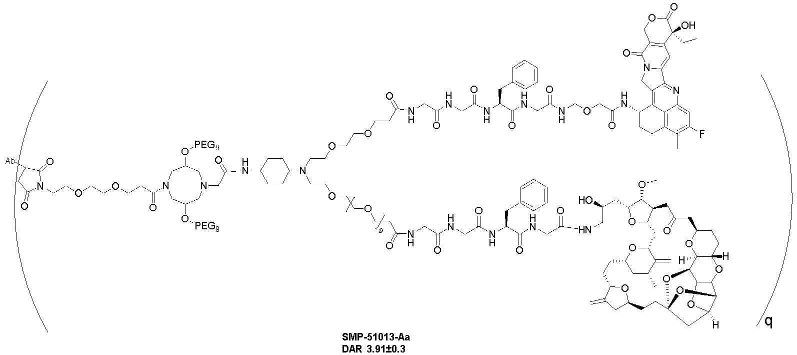 |
| SMP-51013-Ba | 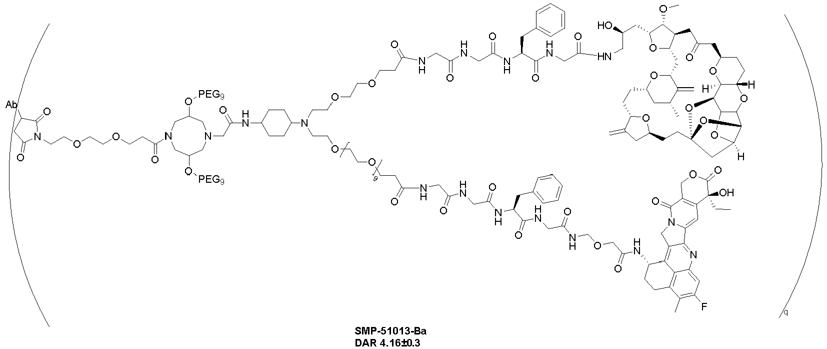 |
| SMP-36256a | 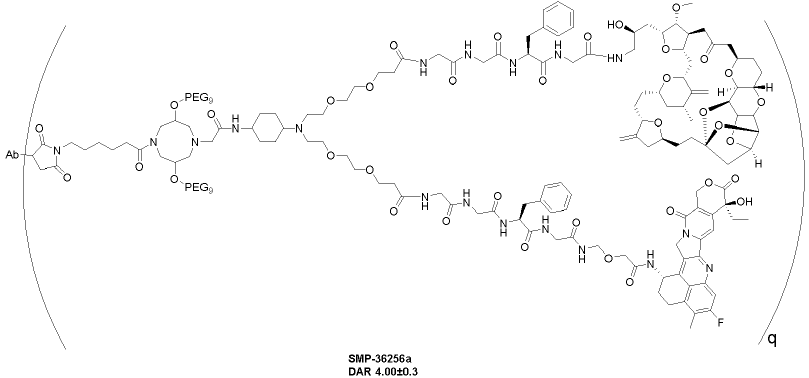 |
| SMP-45582a | 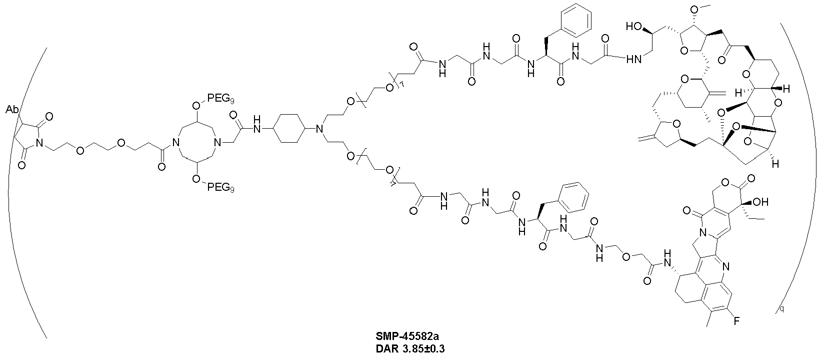 |
| SMP-01984a | 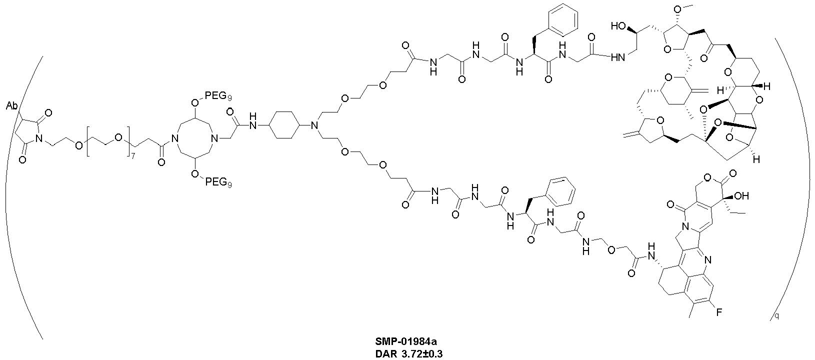 |
| SMP-30920a | 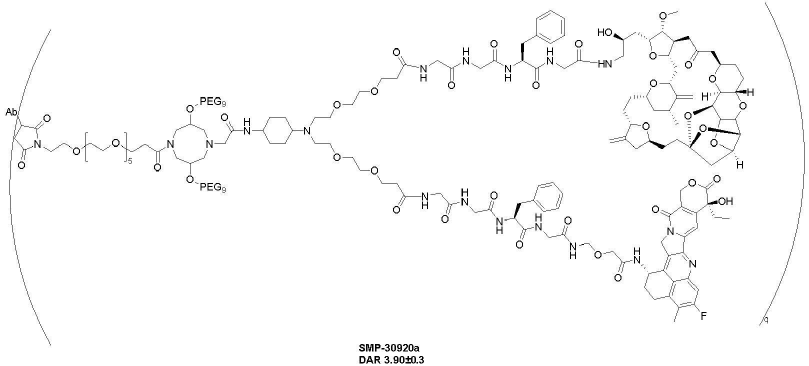 |
| SMP-14324a | 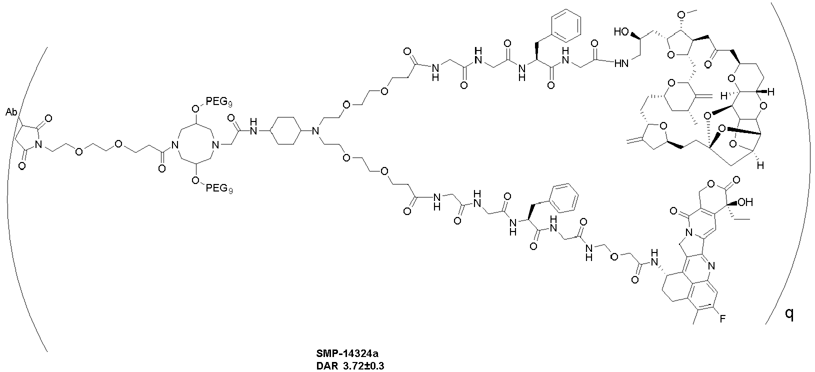 |
| SMP-61393a | 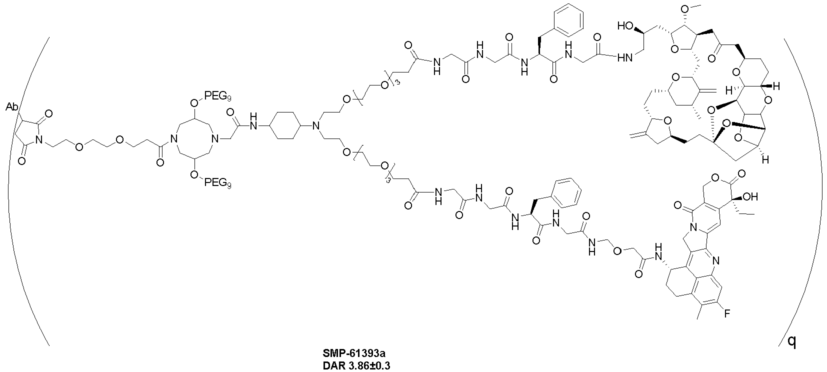 |
| SMP-21139a | 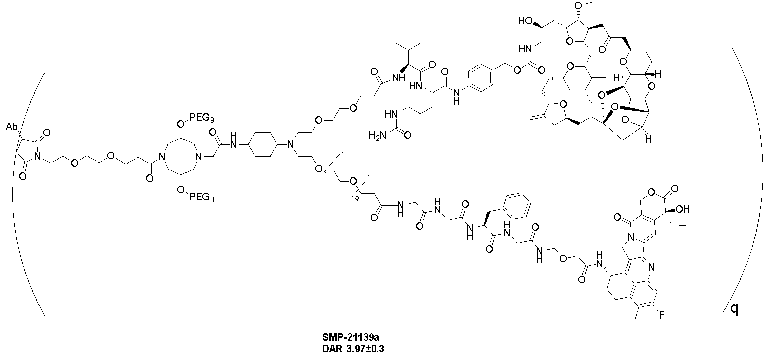 |
| SMP-68691a | 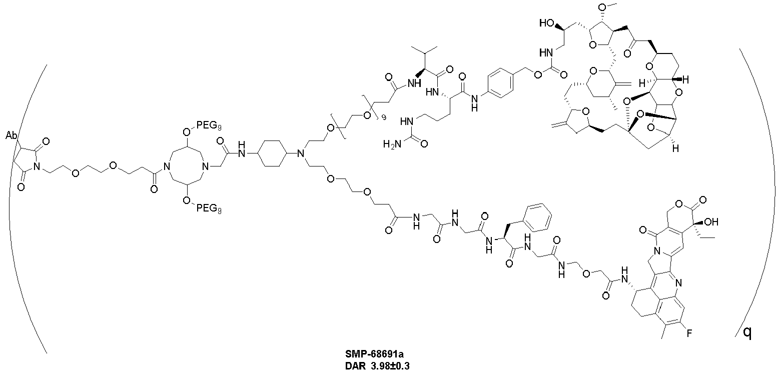 |
| SMP-39205a | 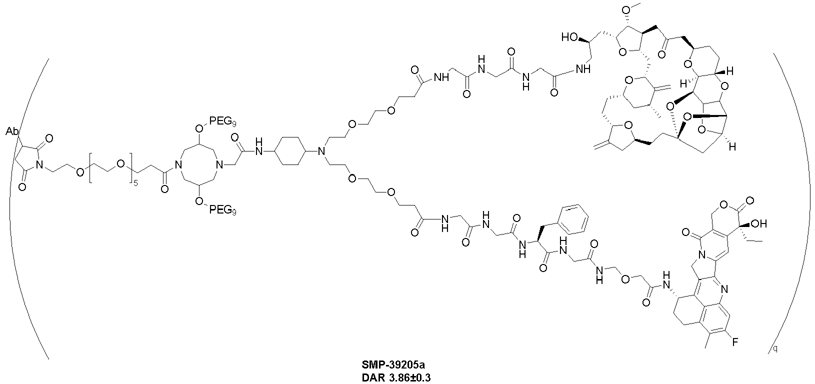 |
| SMP-81404a | 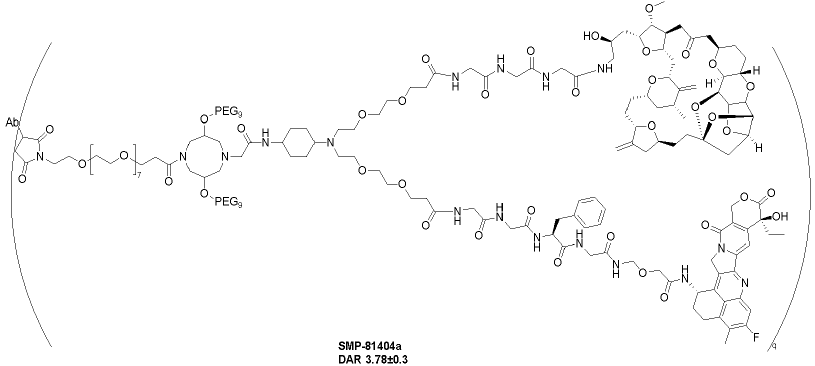 |
| SMP-64906a | 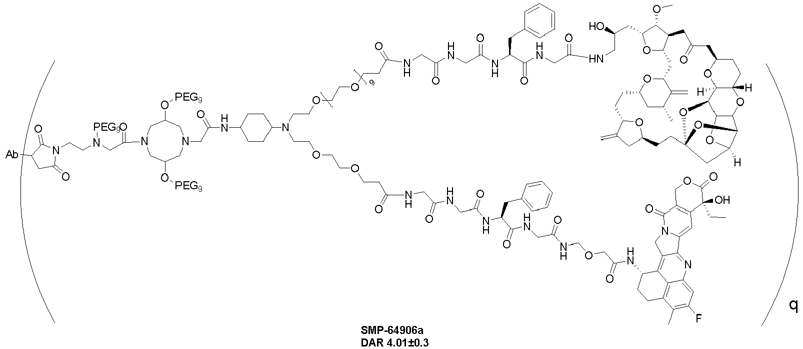 |
| SMP-66348a | 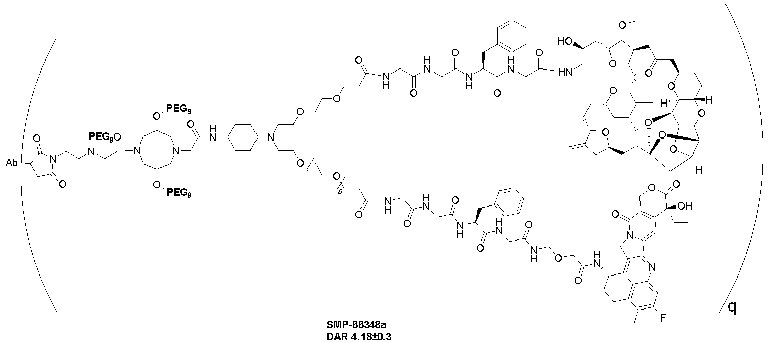 |
| SMP-37860a | 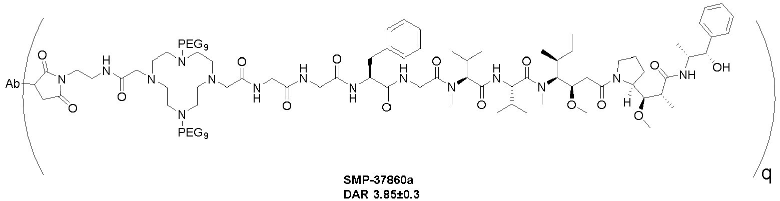 |
| SMP-18777a | 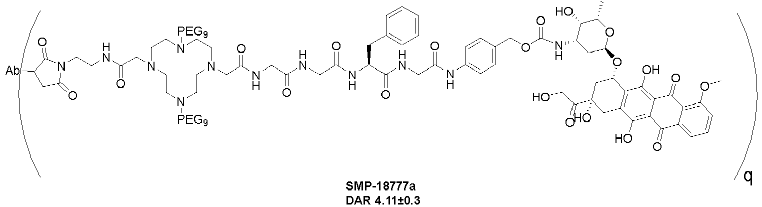 |
| SMP-56368a | 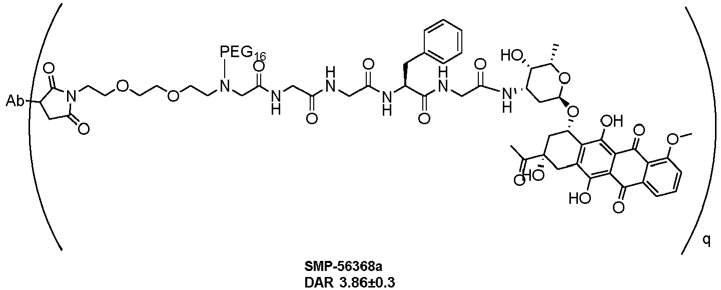 |
| SMP-58054a | 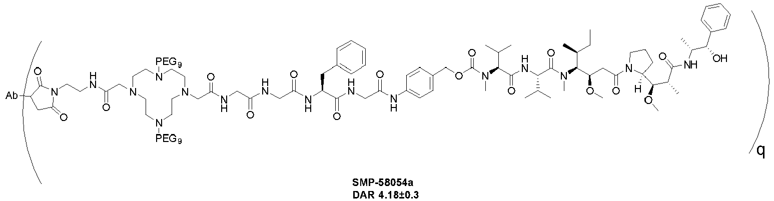 |
| SMP-25274a | 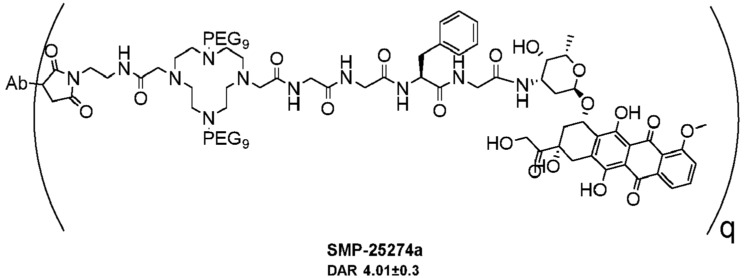 |
| SMP-56875a | 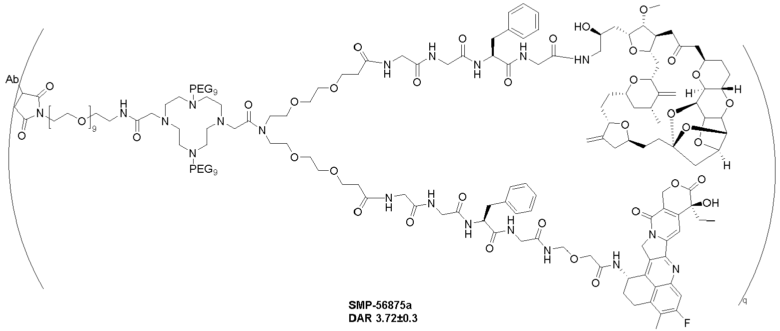 |
| SMP-82444a | 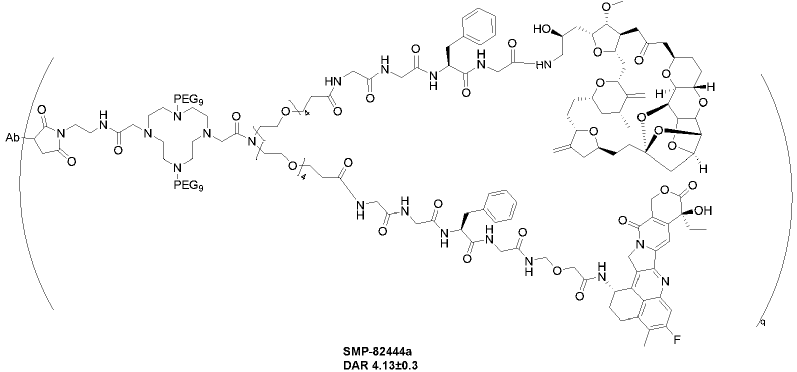 |
| SMP-50309a | 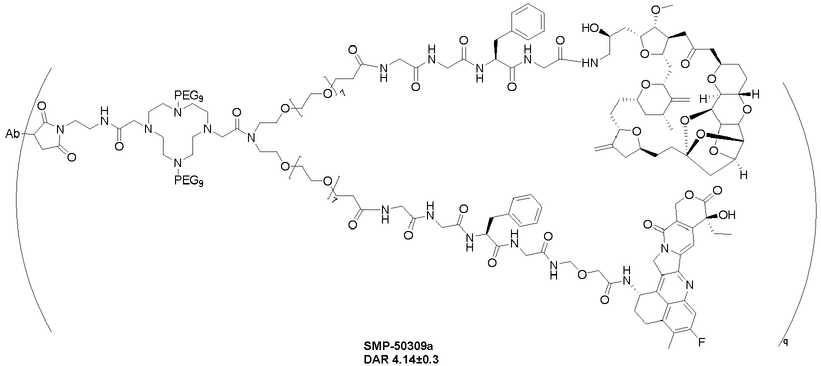 |
| SMP-20094a | 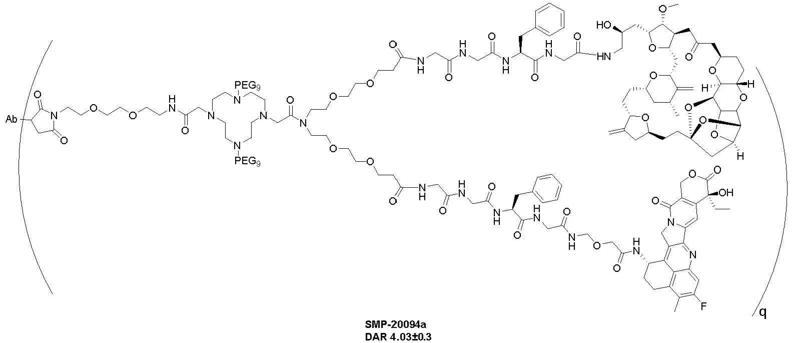 |
| SMP-99559a | 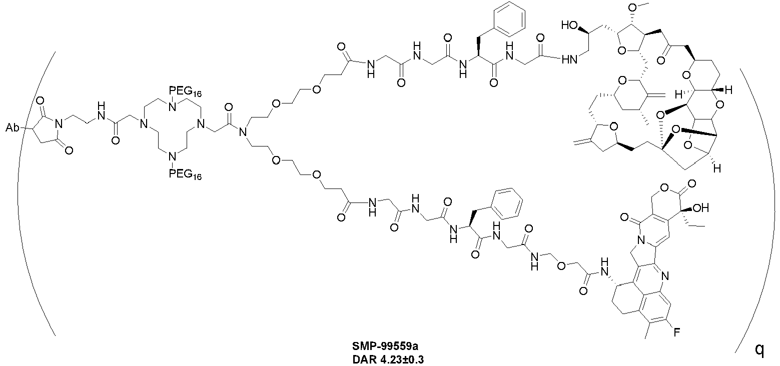 |
| SMP-59623a | 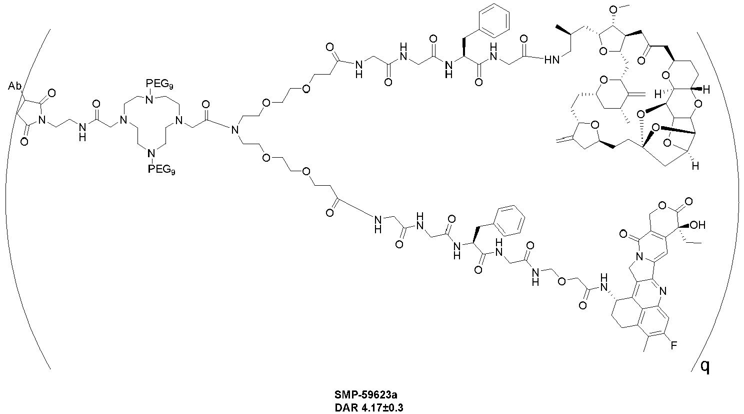 |
| SMP-47610a | 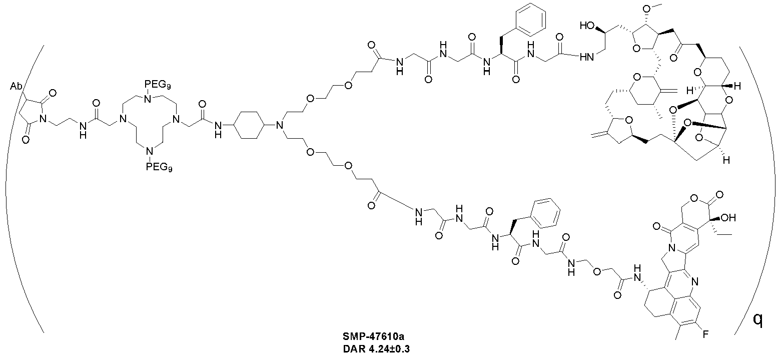 |
| SMP-86467a | 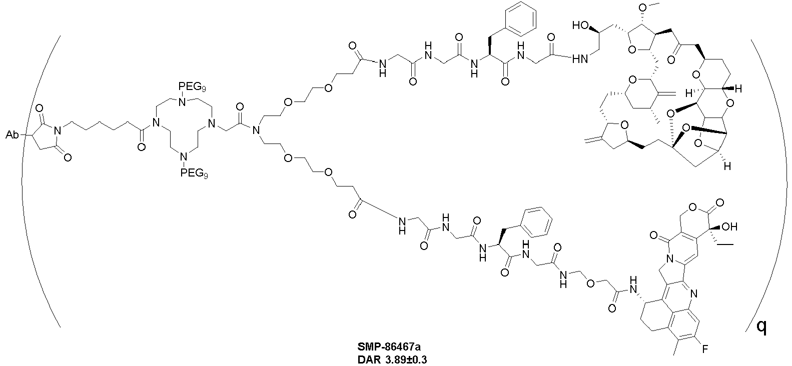 |
| SMP-76899a | 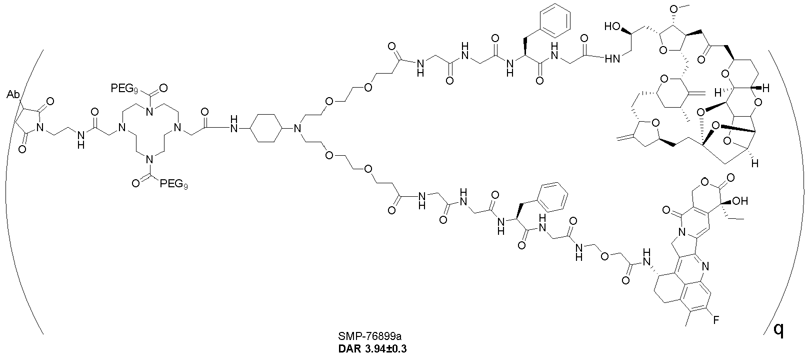 |
| SMP-27341a | 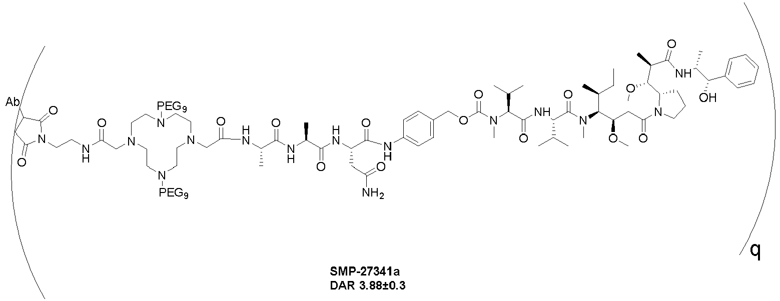 |
| SMP-34421a | 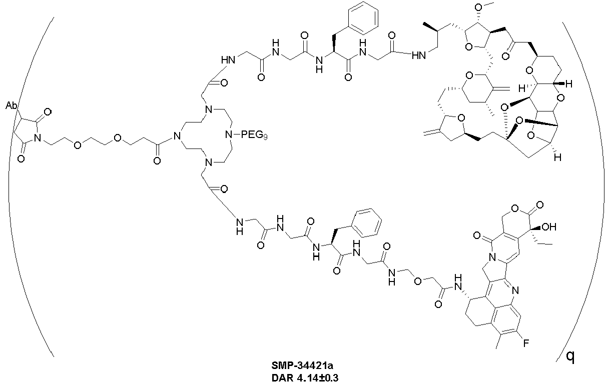 |
| SMP-53612a | 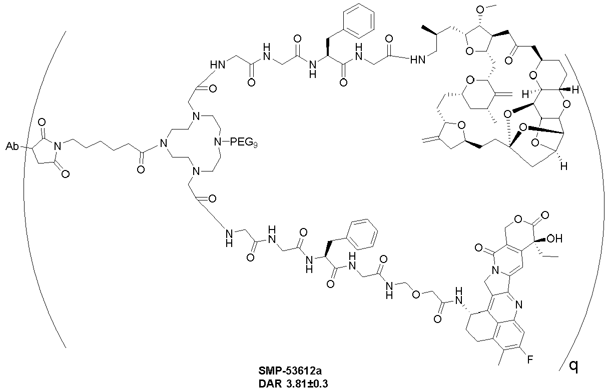 |
| SMP-10215a | 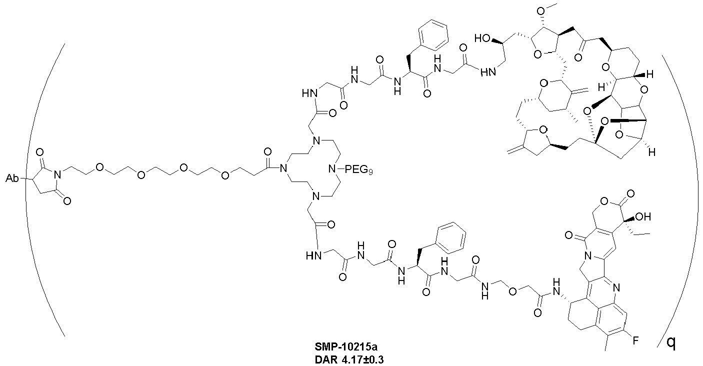 |
| SMP-28945a | 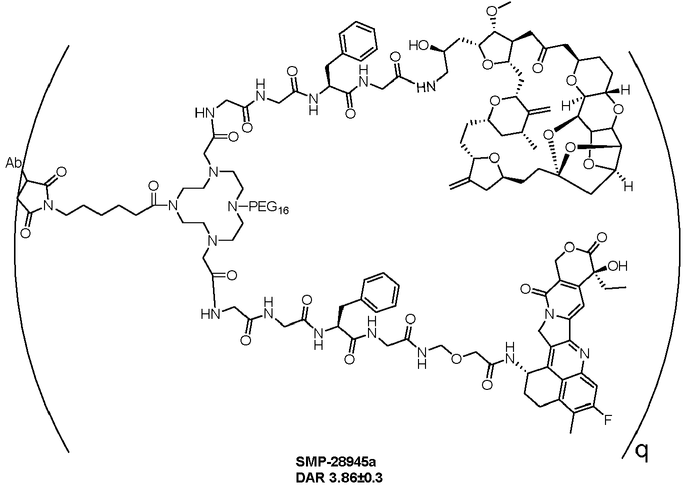 |
| SMP-34710a | 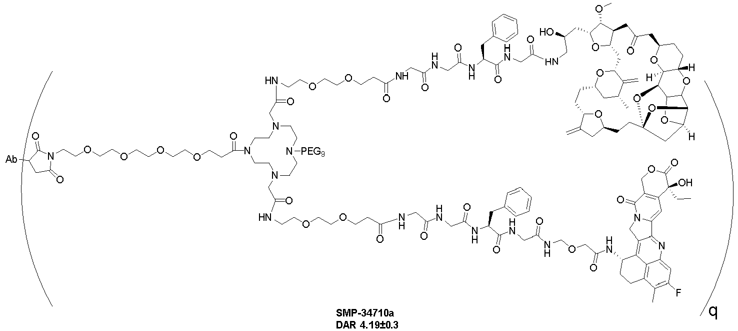 |
| SMP-35055a | 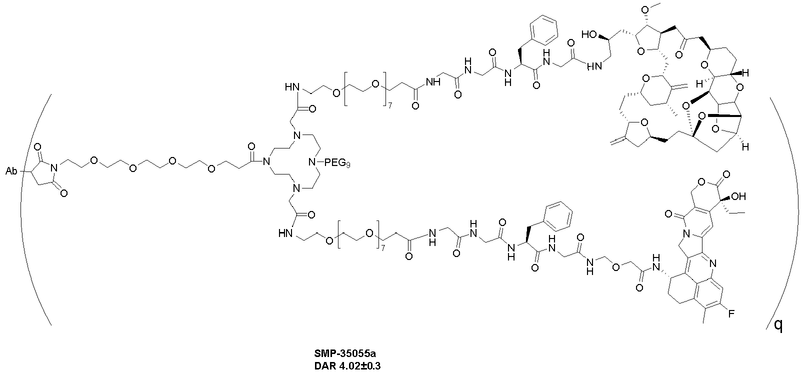 |
| SMP-94170-Aa | 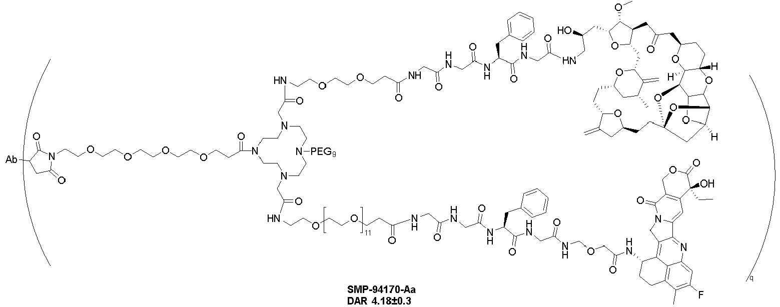 |
| SMP-44419a | 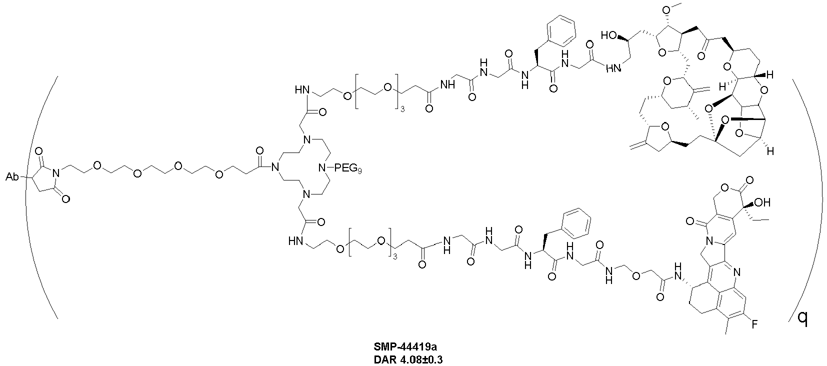 |
| SMP-94170-Ba | 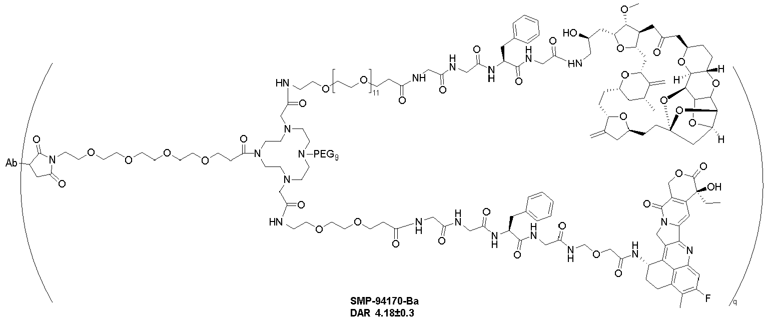 |
| SMP-46940a | 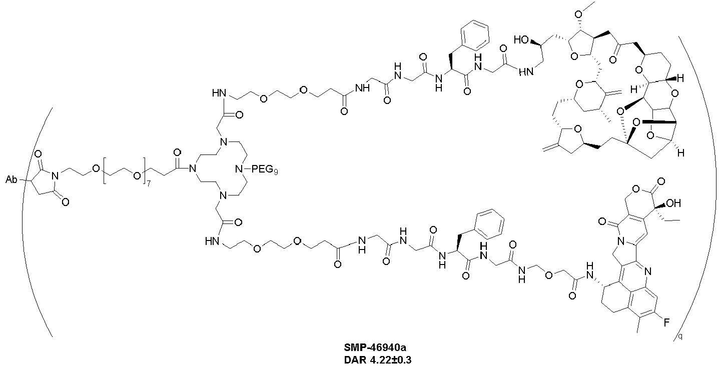 |
| SMP-13069a | 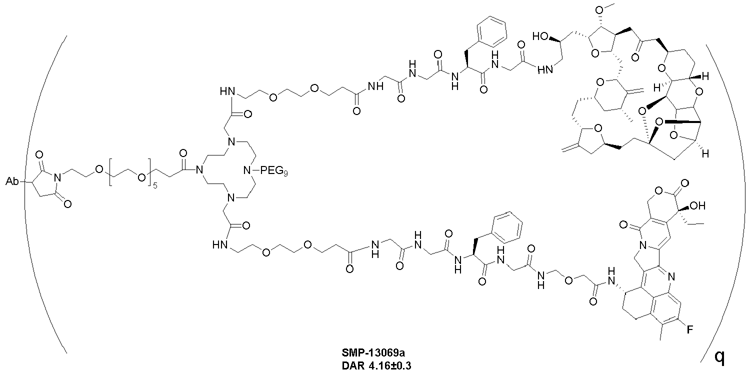 |
| SMP-28823a | 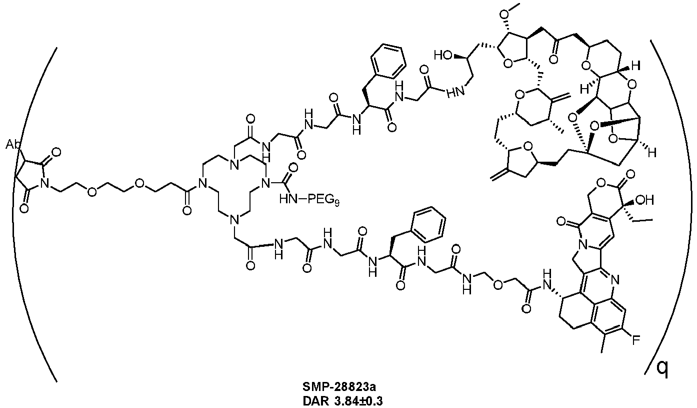 |
| SMP-53816a | 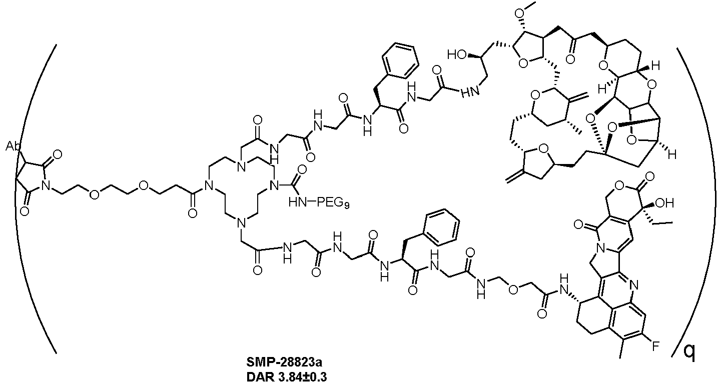 |
| SMP-23135a | 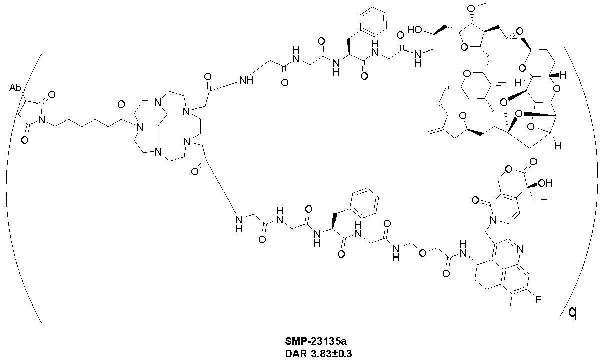 |
| SMP-33693a | 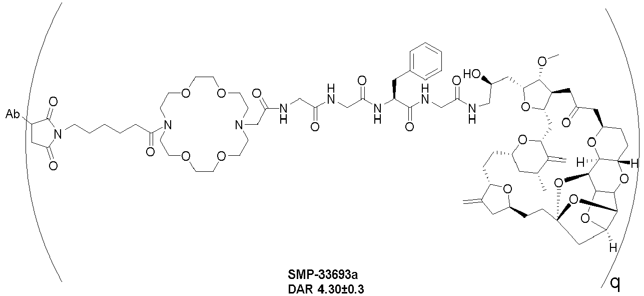 |
| SMP-42930a | 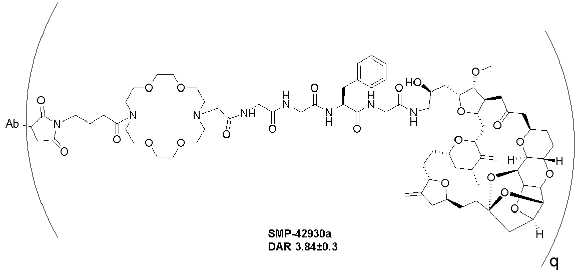 |
| SMP-52573a | 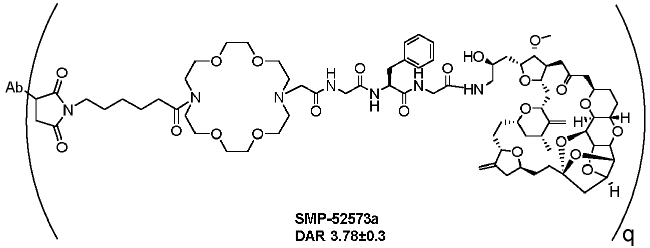 |
| SMP-80439a | 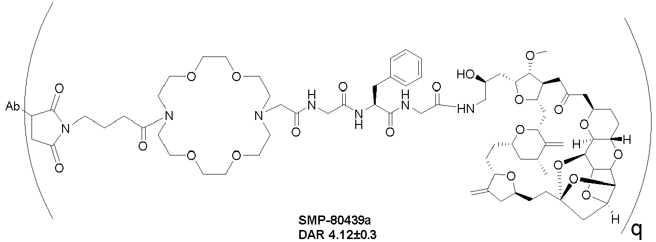 |
| SMP-22682a | 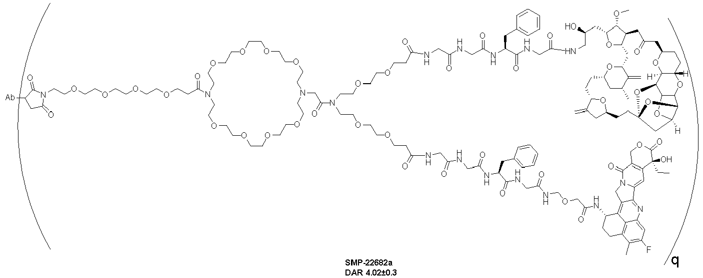 |
| SMP-07861a | 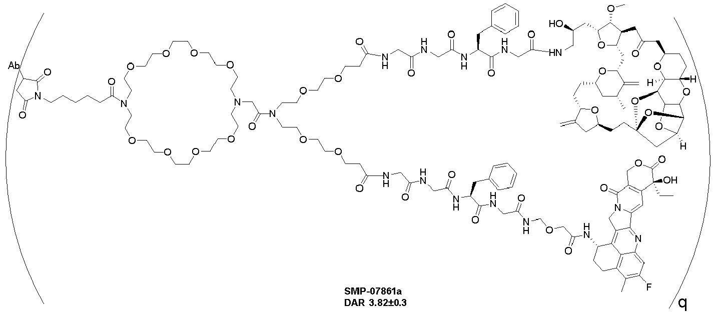 |
| SMP-61694a | 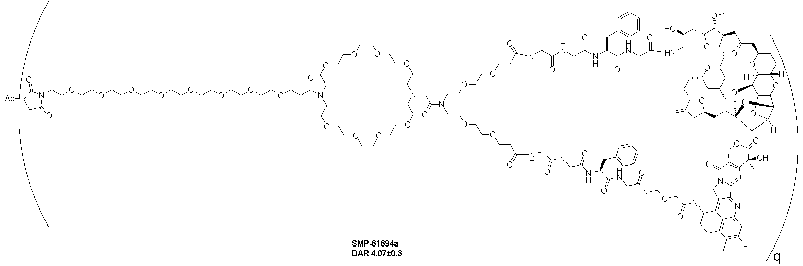 |
| SMP-45539a | 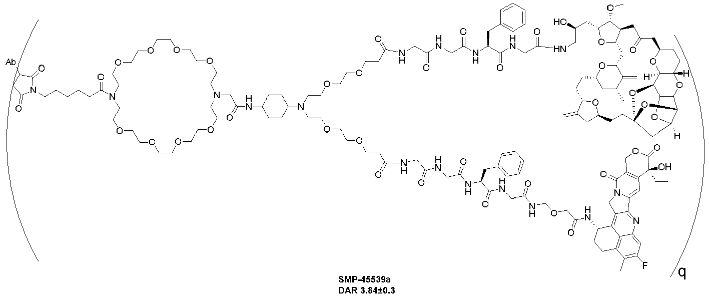 |
| SMP-59034a | 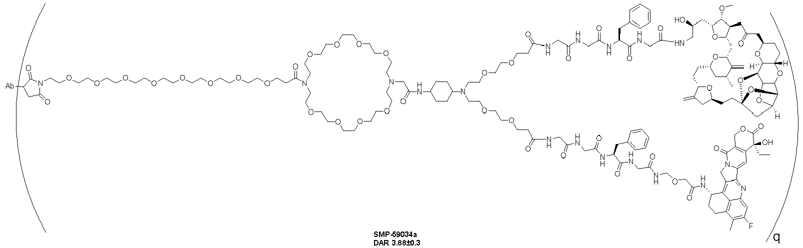 |
| SMP-29438a | 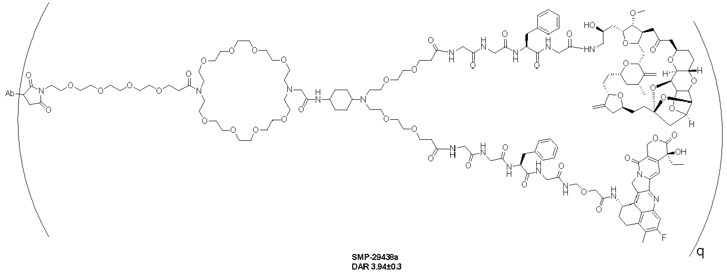 |
| SMP-68962a | 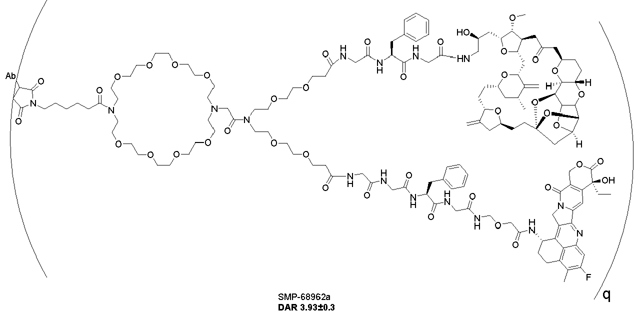 |
| SMP-36297a | 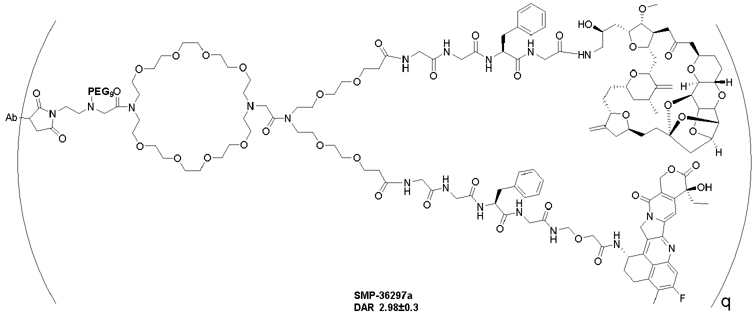 |
| SMP-31135a | 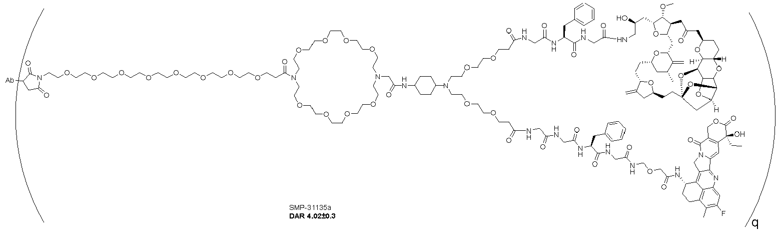 |
| SMP-49182a | 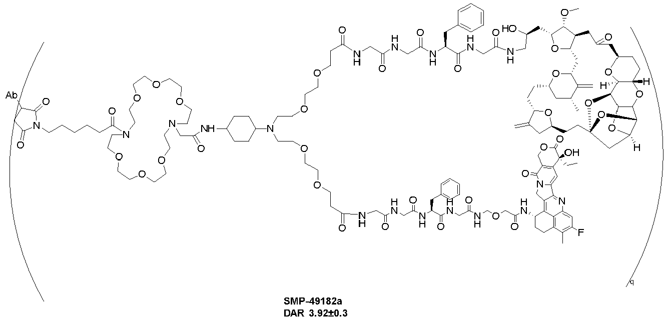 |
| SMP-40359a | 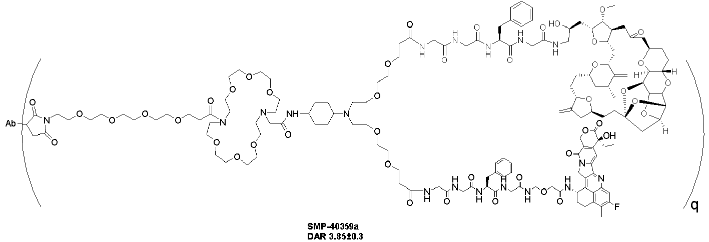 |
| SMP-64791a | 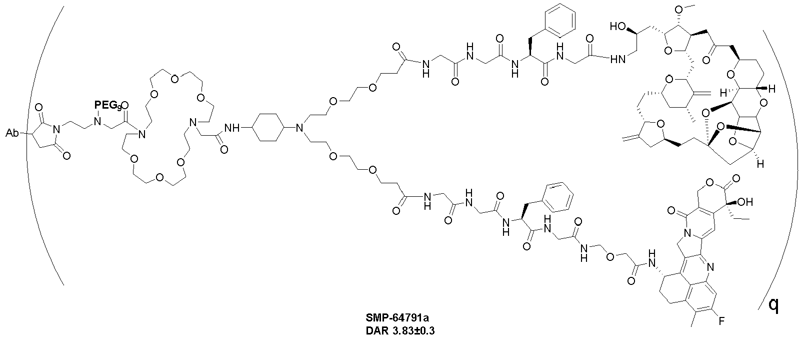 |
| SMP-00171a | 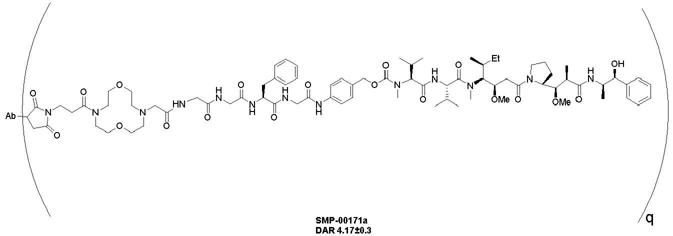 |
| SMP-93566a | 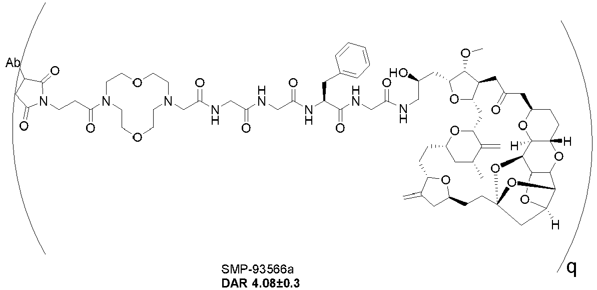 |
| SMP-88480a | 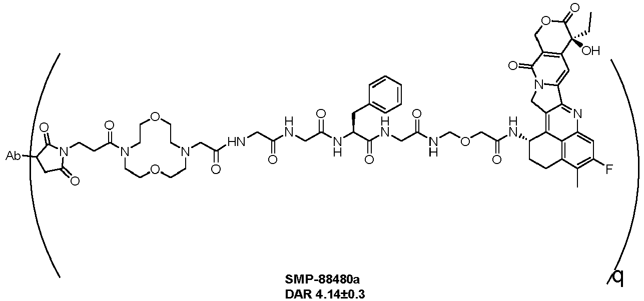 |
| SMP-34048a | 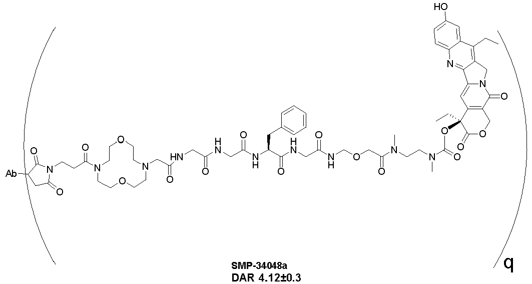 |
| SMP-97962a | 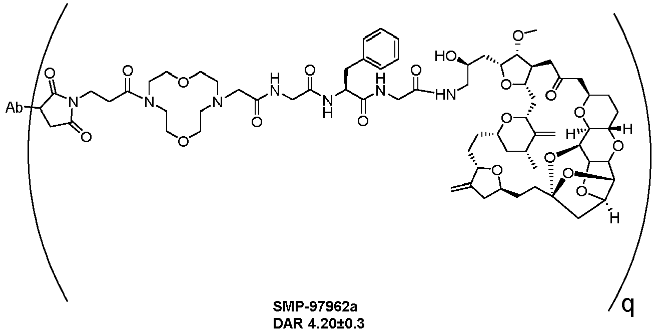 |
| SMP-24600a | 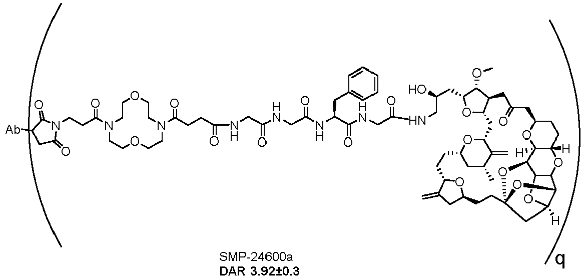 |
| SMP-12395a | 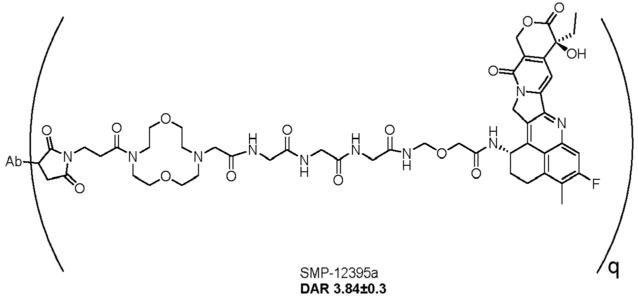 |
| SMP-87055a | 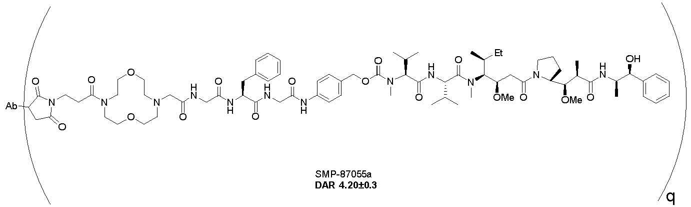 |
| SMP-02692a | 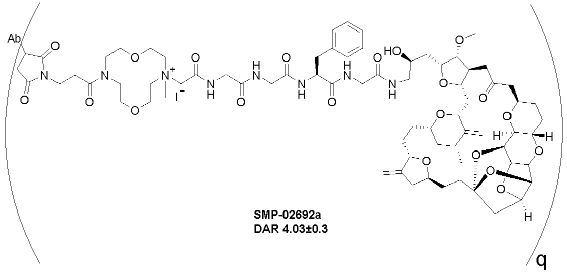 |
| SMP-34146a | 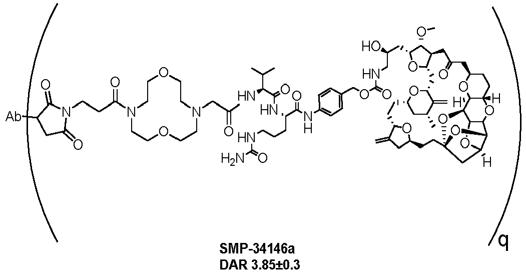 |
| SMP-26598a | 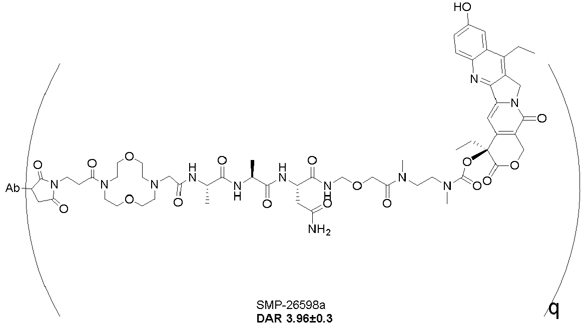 |
| SMP-60226a | 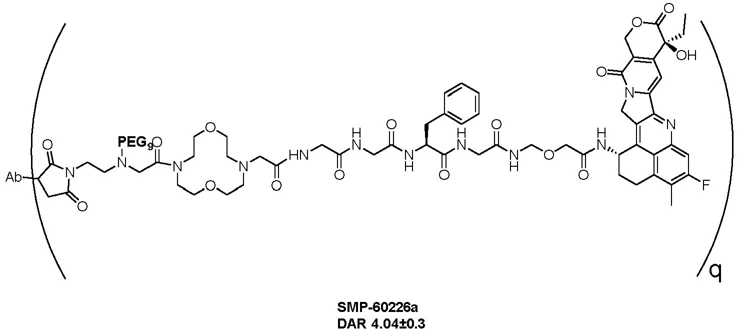 |
| SMP-09168a | 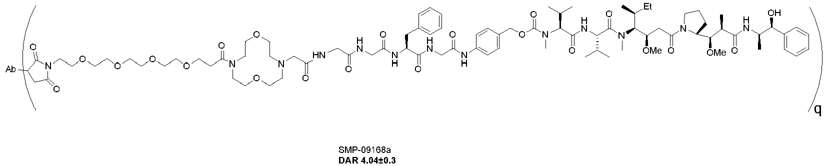 |
| SMP-79575a | 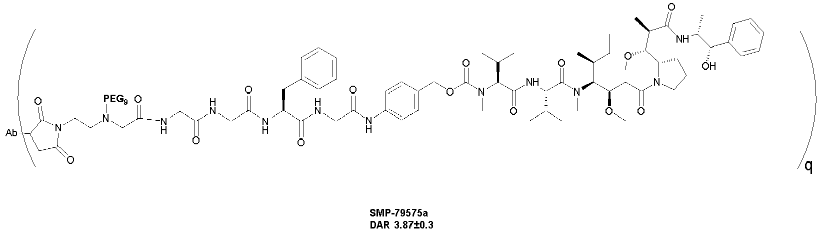 |
| SMP-80035a | 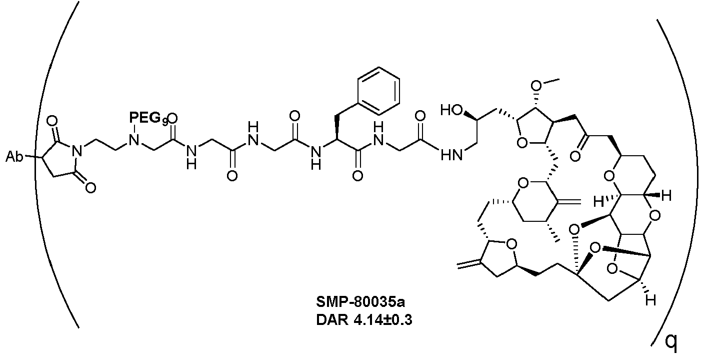 |
q is an integer from 1 to 20; and
-PEG₉ represents 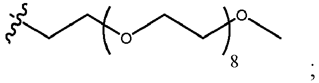 and -PEG₁₆ represents

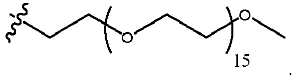

**41.** The targeted linker-drug conjugate or the stereoisomer, optical isomer, salt, or deuterated compound thereof according to any one of claims 37-40, **characterized in that** the targeted linker is an antibody, antibody fragment, protein, small molecular polypeptide, glycopeptide, mimetic peptide, small molecular compound, or nucleic acid oligonucleotide adapter; and
preferably, the antibody is an antibody targeting cell surface receptors and tumor associated antigens.

**42.** A linker used for a targeted linker-drug conjugate, or a stereoisomer, optical isomer, salt, or deuterated compound thereof, **characterized in that** the structure of the linker is as shown in formula K-1 or K-2: 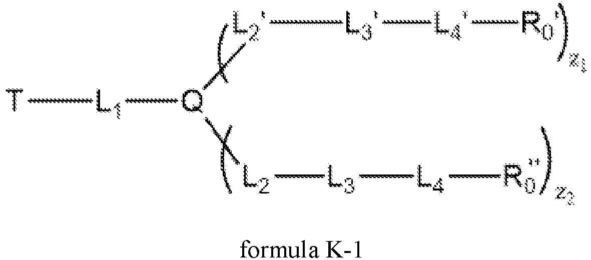 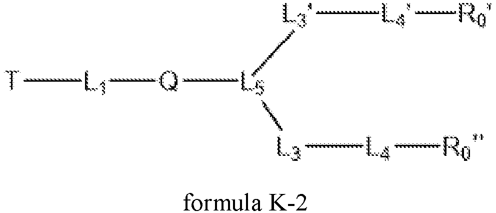
wherein T is a tying group, which can be connected to the targeted linker; the targeted linker is a substance capable of targeted binding to a lesion site;
L₁ is none or a breakable or unbreakable linker fragment;
L₂ is none or a breakable or unbreakable linker fragment;
L₂' is none or a breakable or unbreakable linker fragment;
L₃ is none or a breakable or unbreakable linker fragment;
L₃' is none or a breakable or unbreakable linker fragment;
L₄ is none or a breakable or unbreakable linker fragment;
L₄' is none or a breakable or unbreakable linker fragment;
L₅ is none or a breakable or unbreakable trident linker fragment;
R₀" is a leaving group;
R₀' is a leaving group;
Z₁ is 0 or 1, Z₂ is 0 or 1, and Z₁ and Z₂ are not simultaneously 0; and
the structure of Q is as shown in any one of claims 1-24.

**43.** The linker or the stereoisomer, optical isomer, salt, or deuterated compound thereof according to claim 42, **characterized in that** the leaving group is hydroxyl.

**44.** The linker or the stereoisomer, optical isomer, salt, or deuterated compound thereof according to claim 42, **characterized in that** the structure of L₅ is selected from 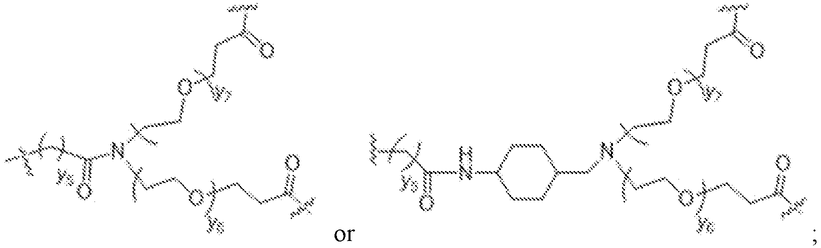
y₅ is an integer selected from 1 to 10, preferably 1;
y₆ is an integer selected from 1 to 50, preferably an integer from 2 to 9; and
y₇ is an integer selected from 1 to 50, preferably an integer from 2 to 9.

**45.** The linker or the stereoisomer, optical isomer, salt, or deuterated compound thereof according to claim 42, **characterized in that** the structure of the linker is as shown in formula K-3:
T-L₁-Q-L₂-L₃-L₄-R₀" formula K-3
wherein T is a tying group, which can be connected to the targeted linker; the targeted linker is a substance capable of targeted binding to a lesion site;
L₁ is a breakable or unbreakable linker fragment;
L₂ is none or a breakable or unbreakable linker fragment;
L₃ is a breakable or unbreakable linker fragment;
L₄ is none or a breakable or unbreakable linker fragment;
R₀" is a leaving group; and
the structure of Q is as shown in any one of claims 1-24.

**46.** The linker or the stereoisomer, optical isomer, salt, or deuterated compound thereof according to any one of claims 42-45, **characterized in that** the structure of T is:

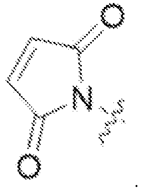

**47.** The linker or the stereoisomer, optical isomer, salt, or deuterated compound thereof according to any one of claims 42-45, **characterized in that** the structure of L₁ is 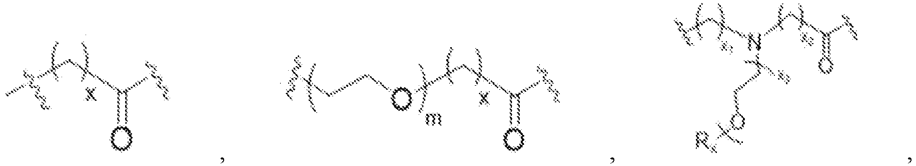 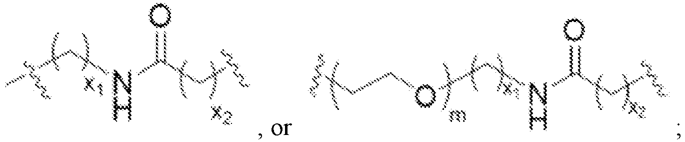
wherein x is an integer selected from 1 to 10, preferably an integer from 2 to 6;
m is an integer selected from 1 to 25, preferably an integer from 2 to 10, and more preferably an integer from 2 to 9;
x₁ is an integer selected from 1 to 10, preferably an integer from 2 to 6, and more preferably 2;
x₂ is an integer selected from 1 to 10, preferably an integer from 1 to 5, and more preferably 1;
x₃ is an integer selected from 0 to 50, preferably an integer from 8 to 30, and more preferably 9; and
Rₓ is selected from hydrogen or methyl.

**48.** The linker or the stereoisomer, optical isomer, salt, or deuterated compound thereof according to claim 42, **characterized in that** the structure of L₂ is 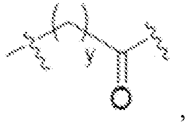 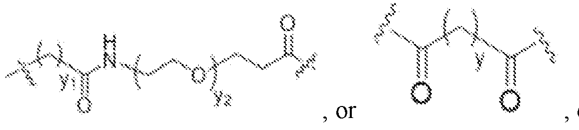 or L2 is none;
the structure of L₂' is 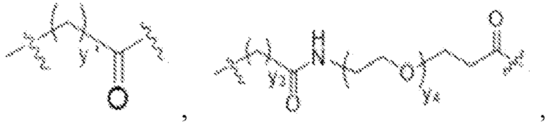 or 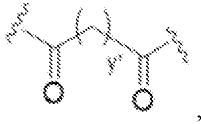 or L₂' is none;
wherein y and y' are integers independently selected from 1 to 10, preferably 1 or 2;
y₁ and y₃ are integers independently selected from 0 to 10, preferably 0, 1 or 2, and more preferably 0 or 1; and
y₂ and y₄ are integers independently selected from 1 to 50, preferably integers from 1 to 20, and more preferably integers from 2 to 12.

**49.** The linker or the stereoisomer, optical isomer, salt, or deuterated compound thereof according to claim 42, **characterized in that** the structures of L₃ and L₃' are independently selected from: 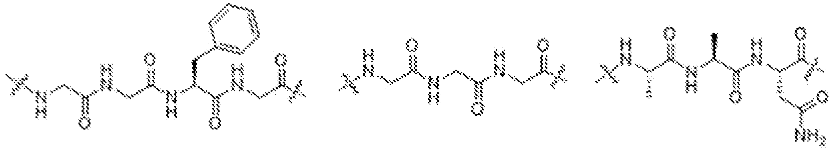 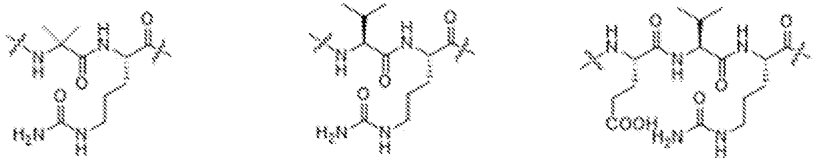 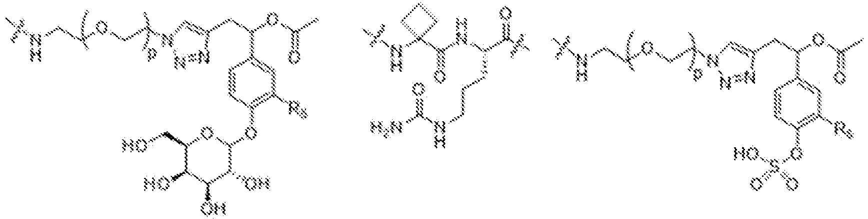 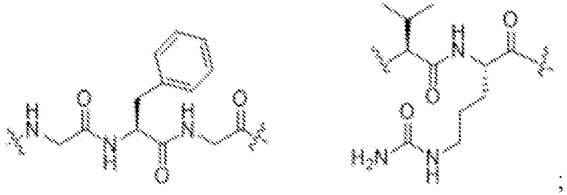 wherein p is an integer selected from 1 to 24, and R₆ is selected from hydrogen or nitro.

**50.** The linker or the stereoisomer, optical isomer, salt, or deuterated compound thereof according to claim 42, **characterized in that** the structures of L₄ and L₄' are independently selected from none,

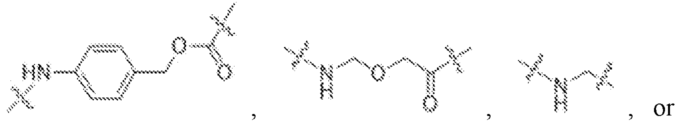

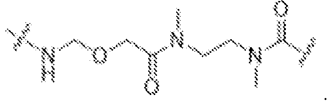

**51.** The linker or the stereoisomer, optical isomer, salt, or deuterated compound thereof according to any one of claims 42-50, **characterized in that** the structure of the linker is selected from: 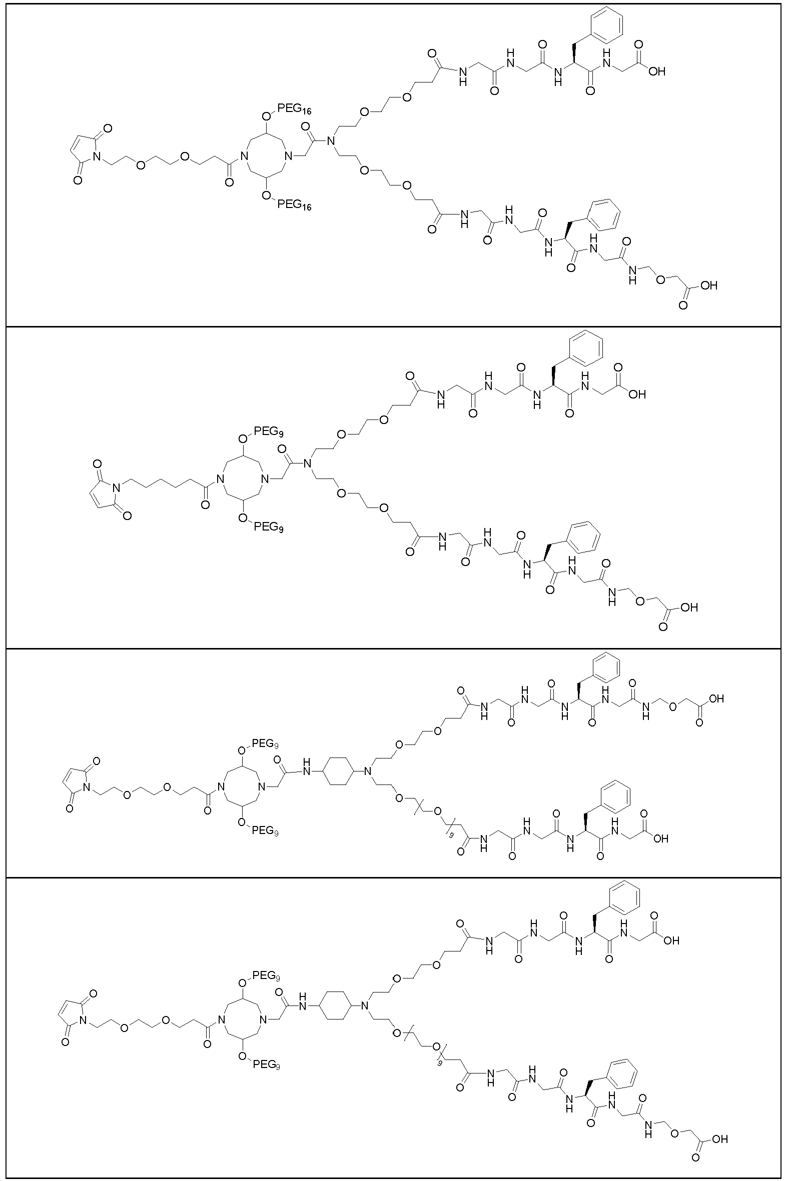 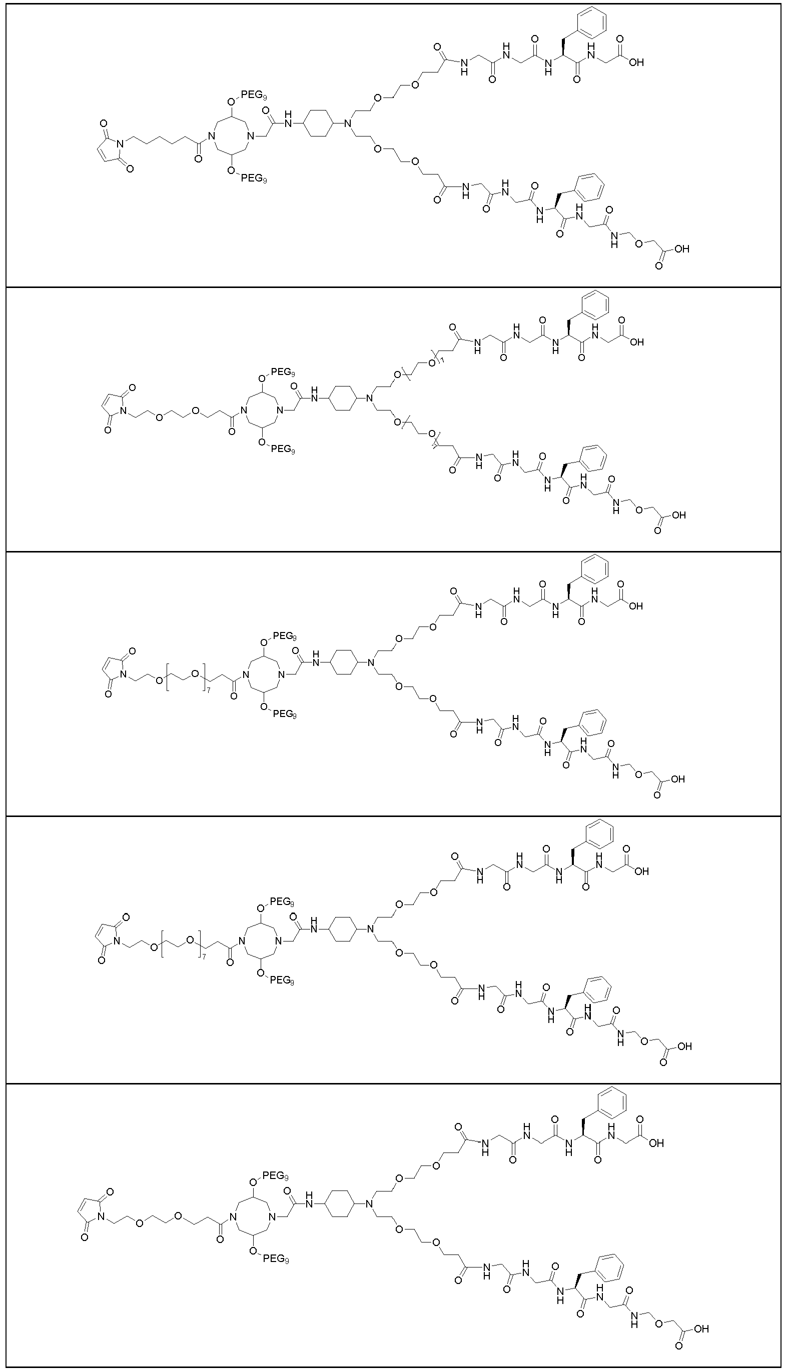 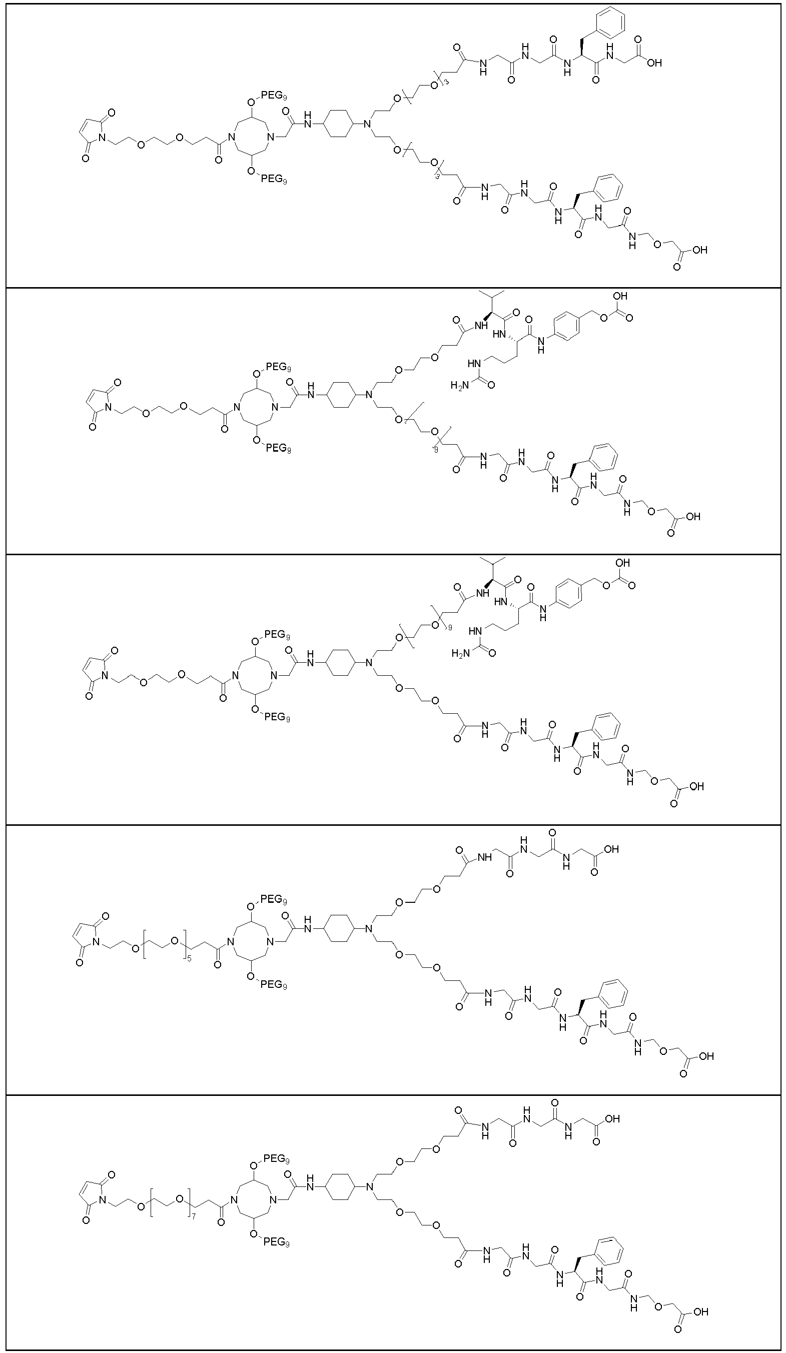 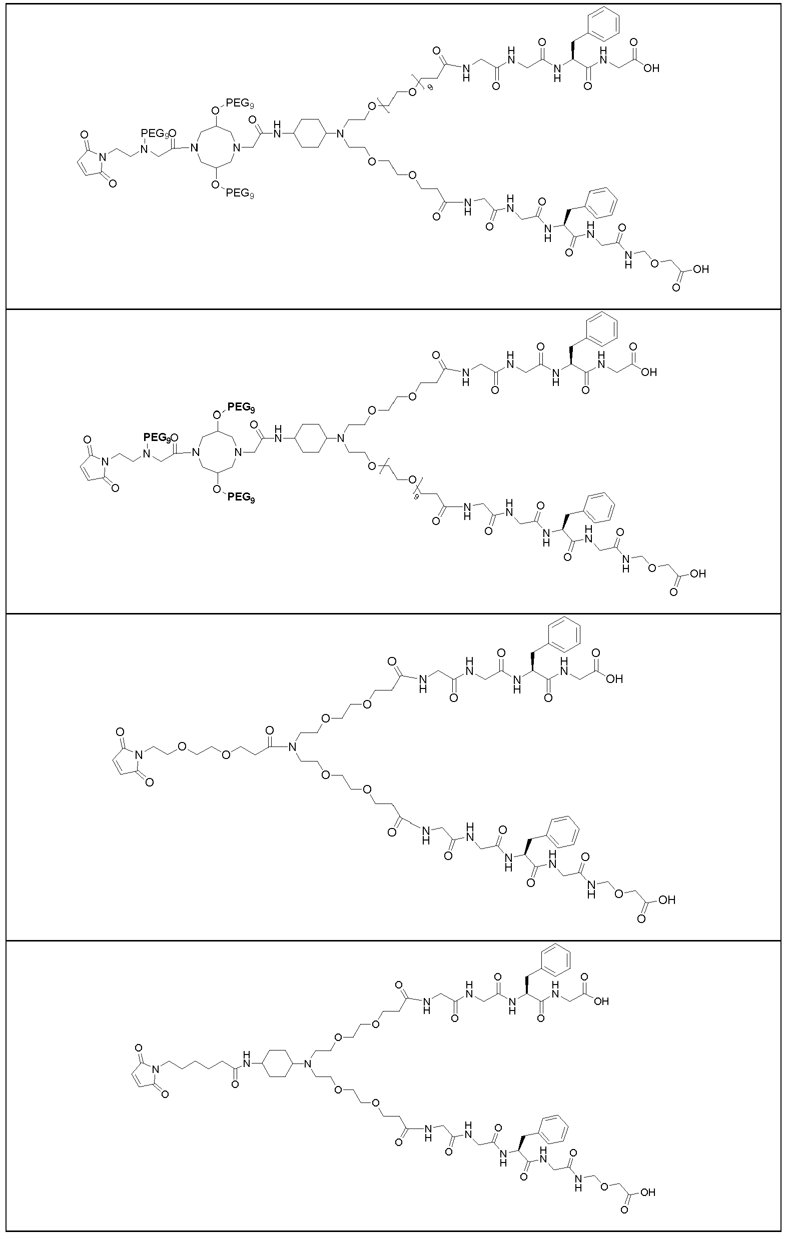 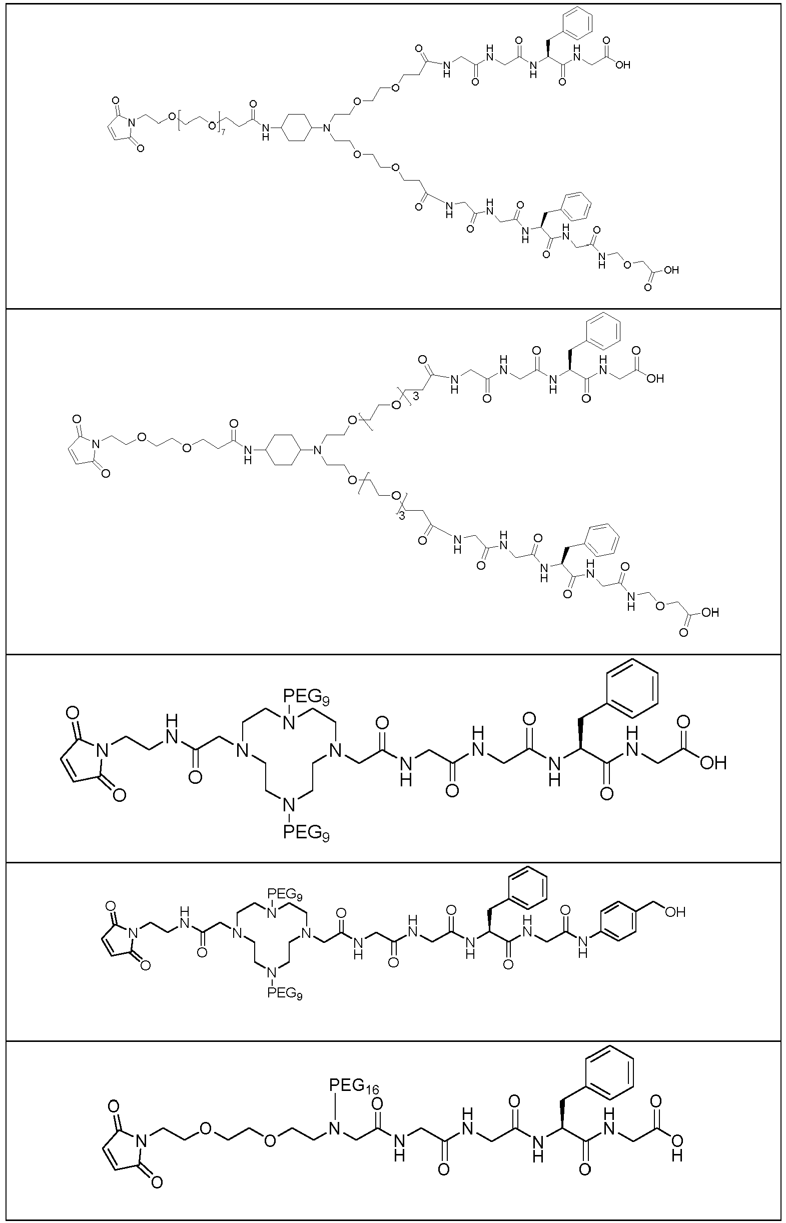 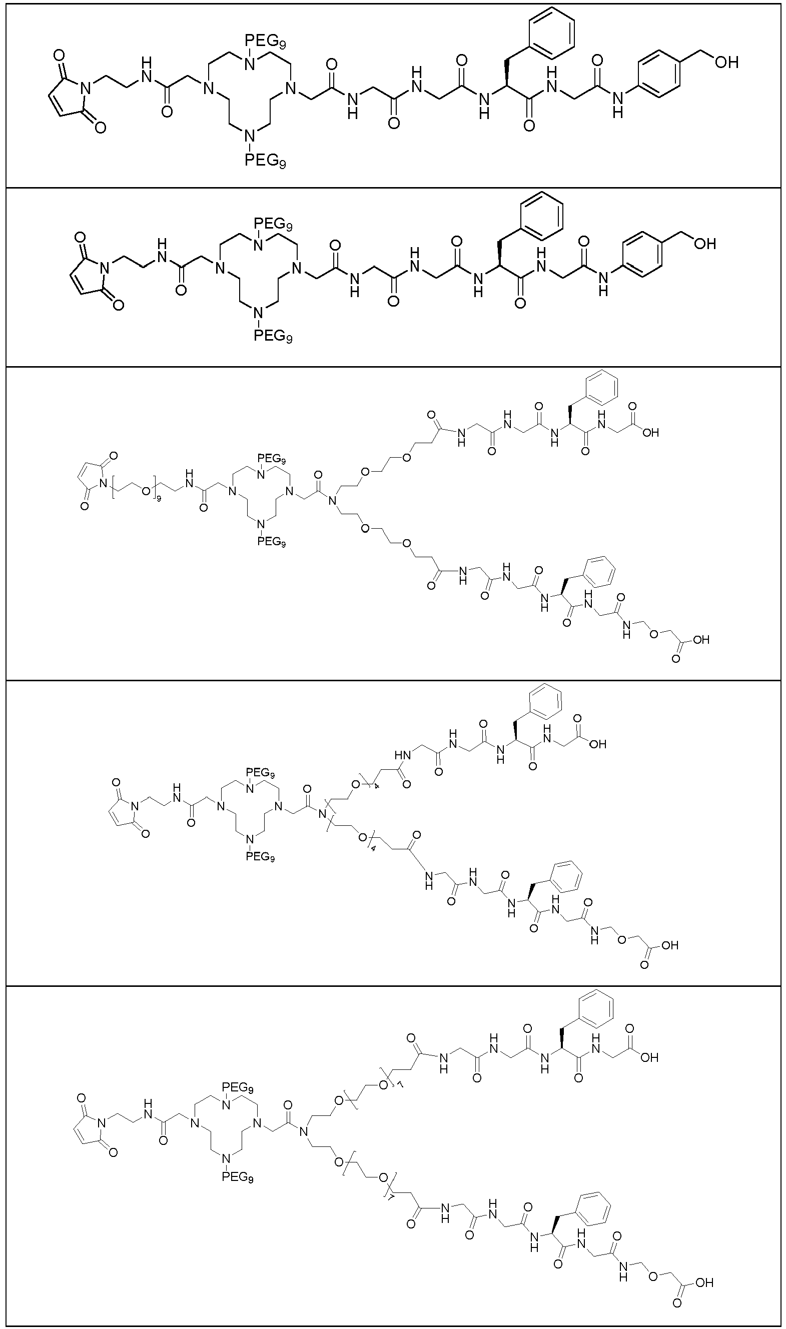 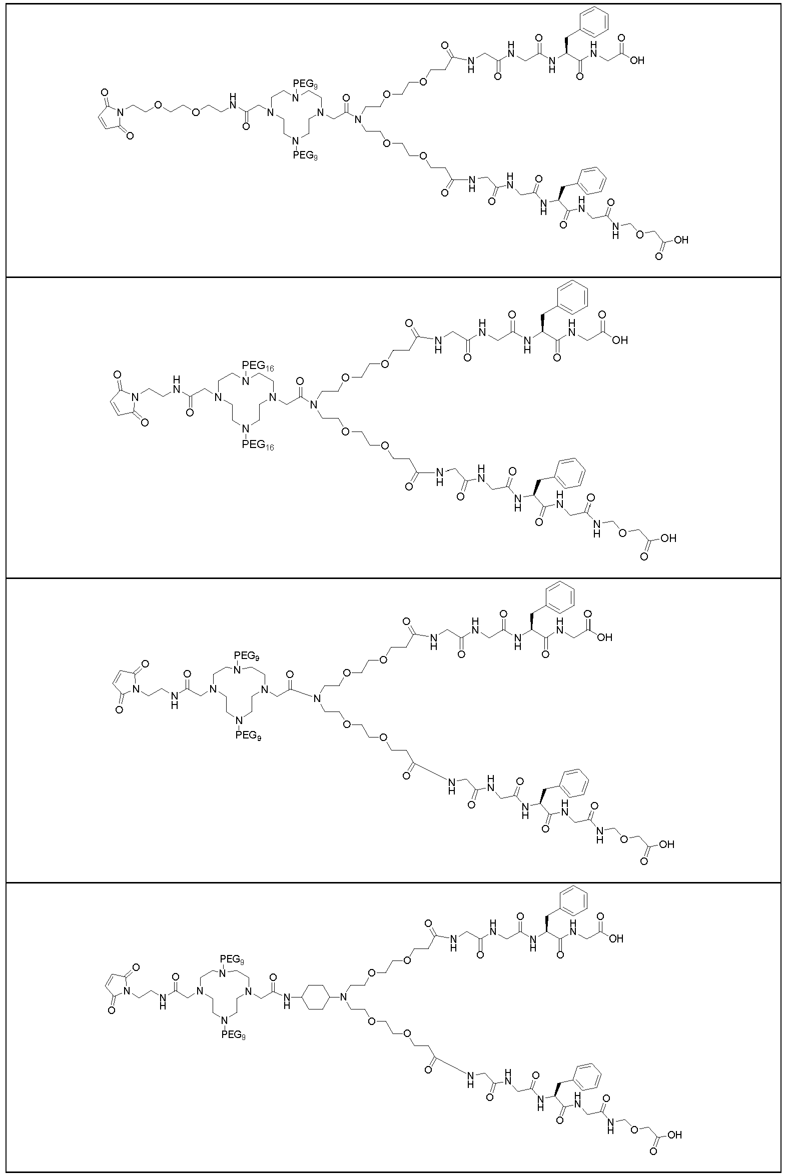 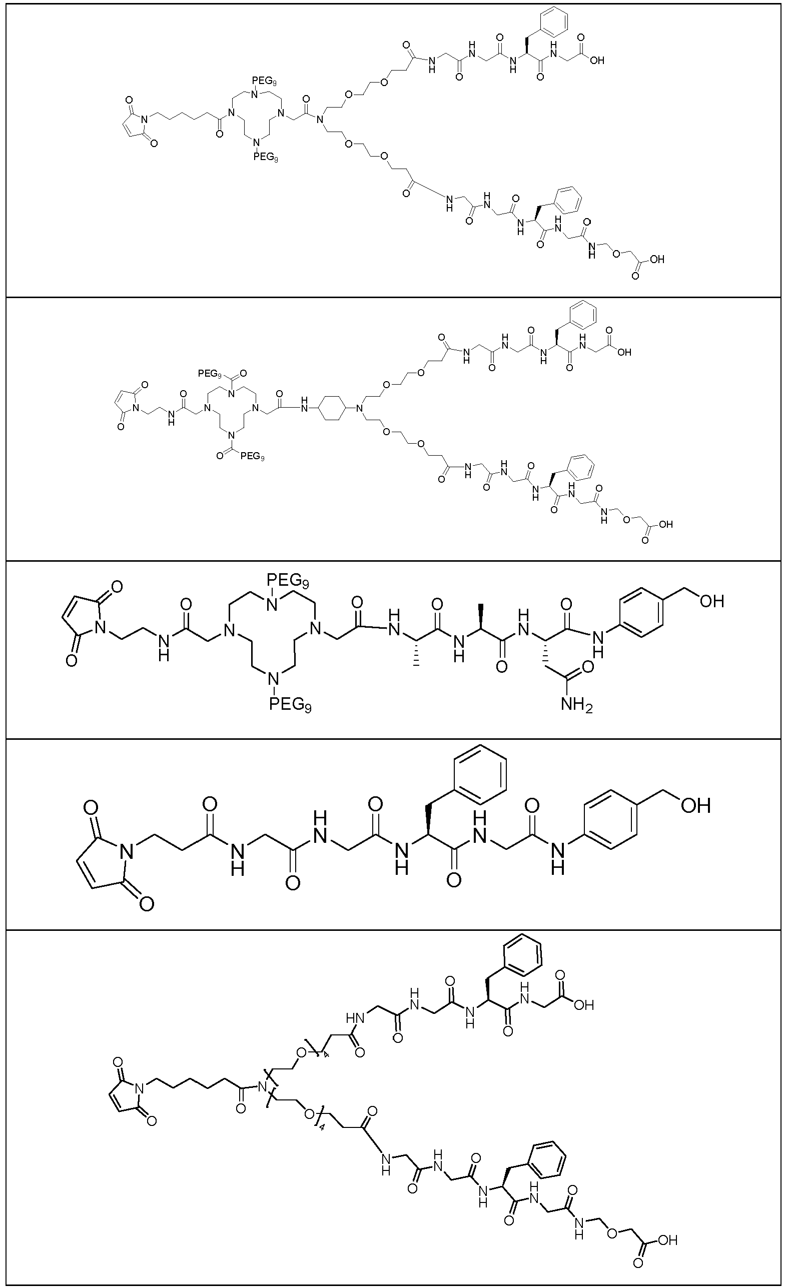 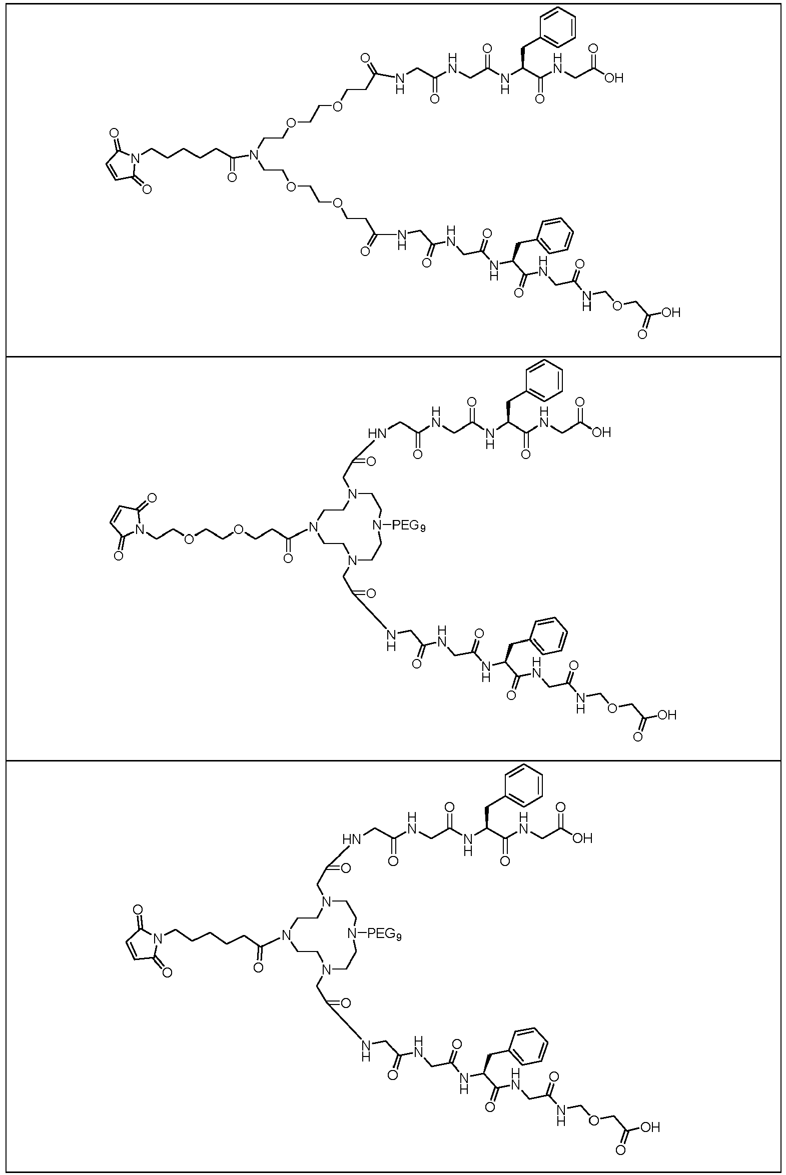 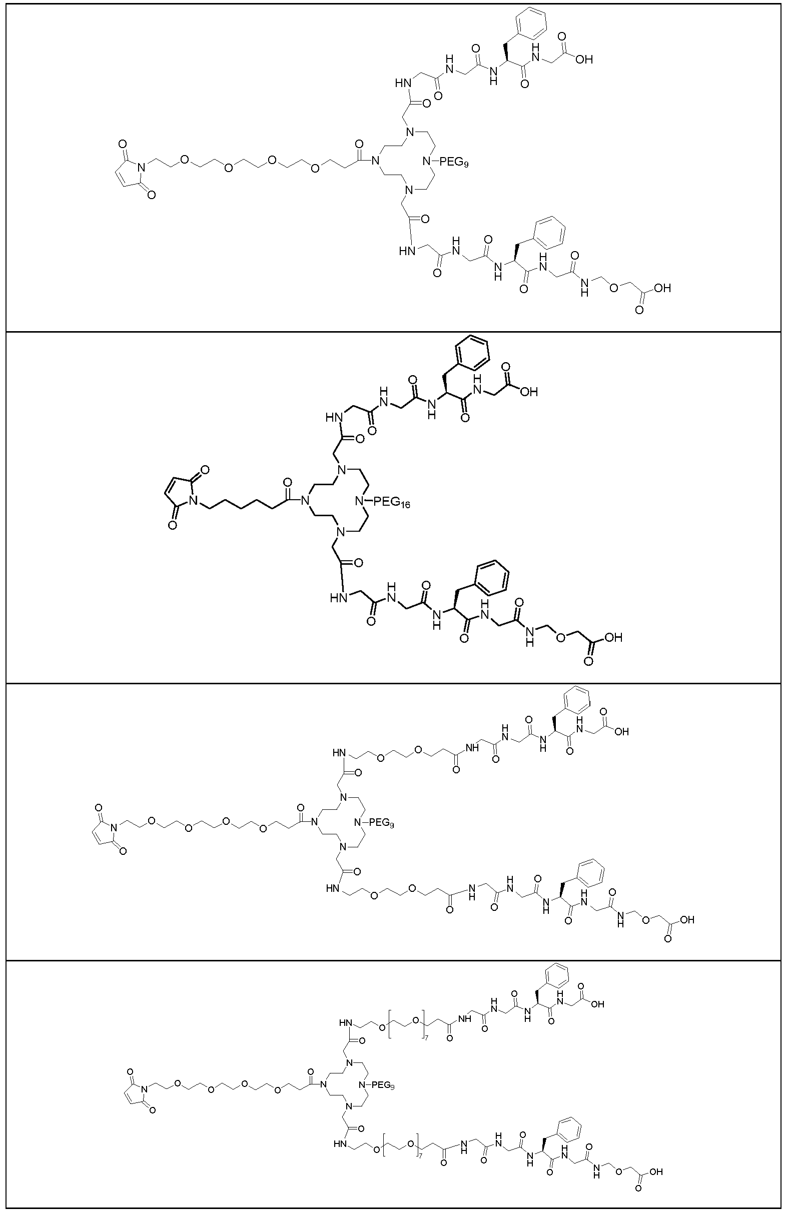 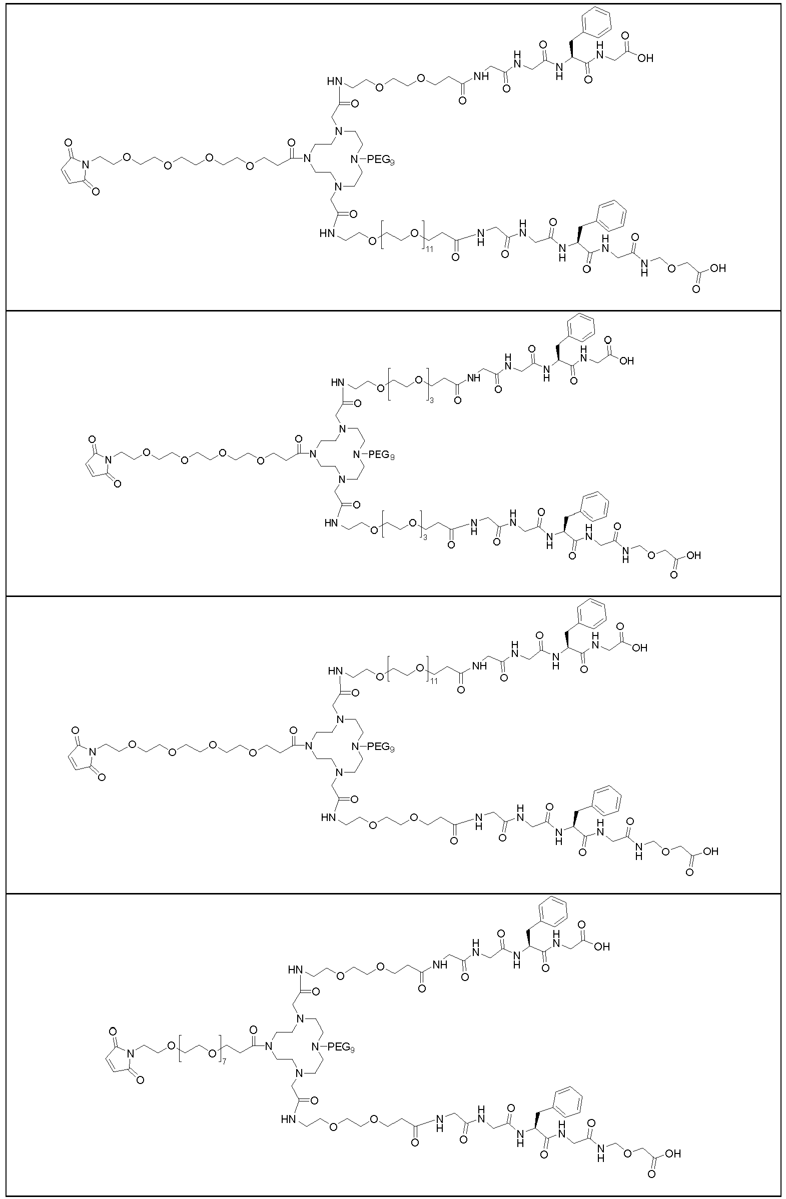 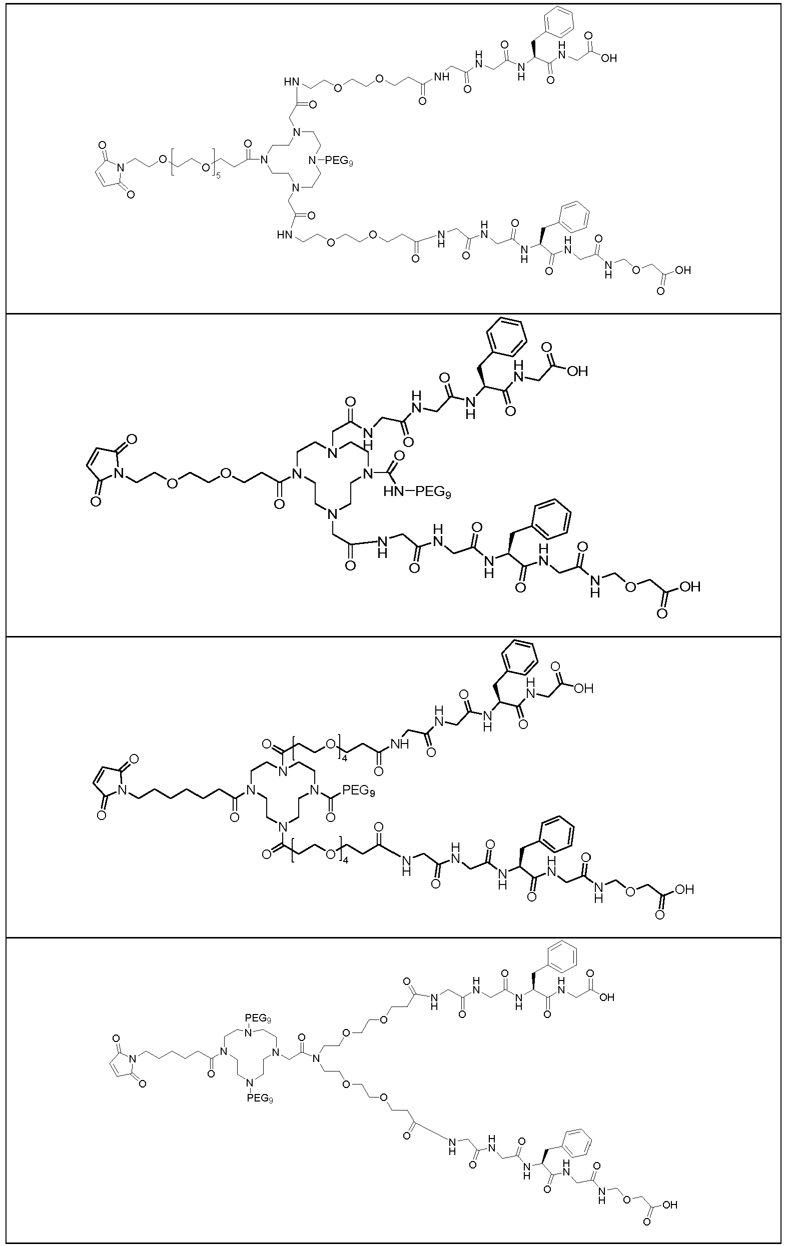 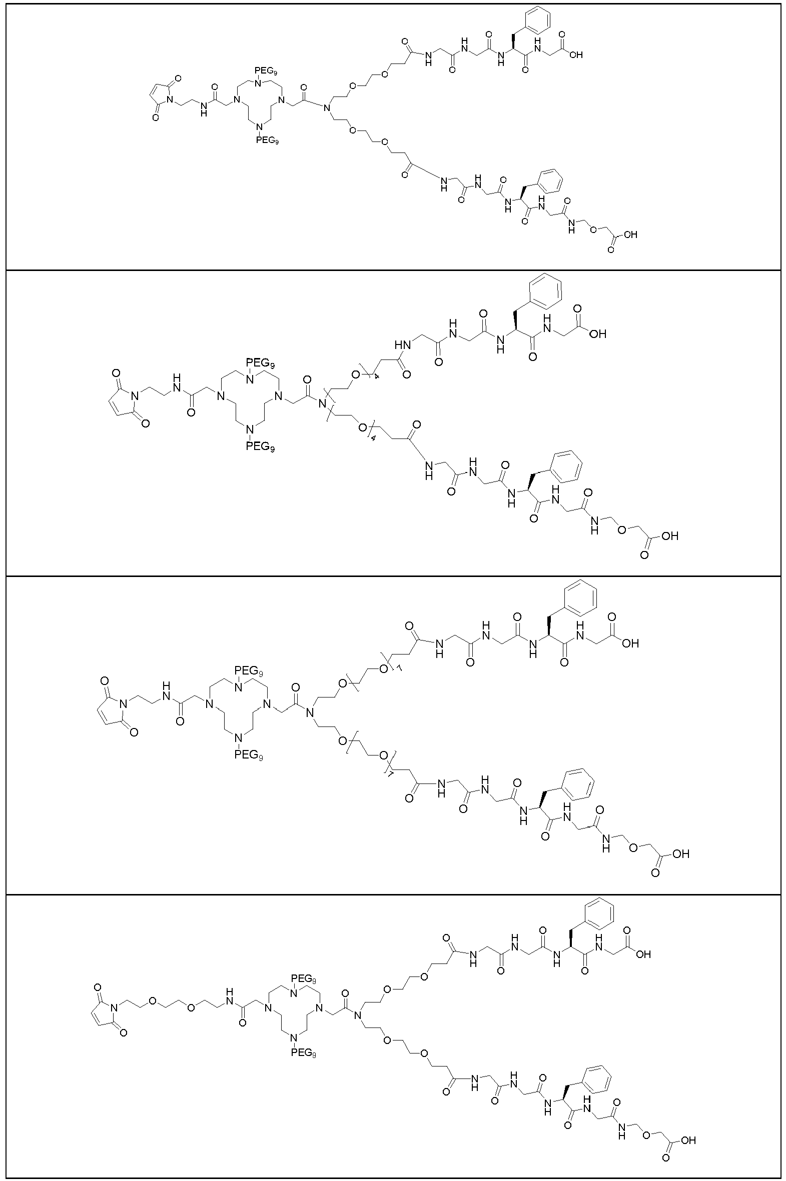 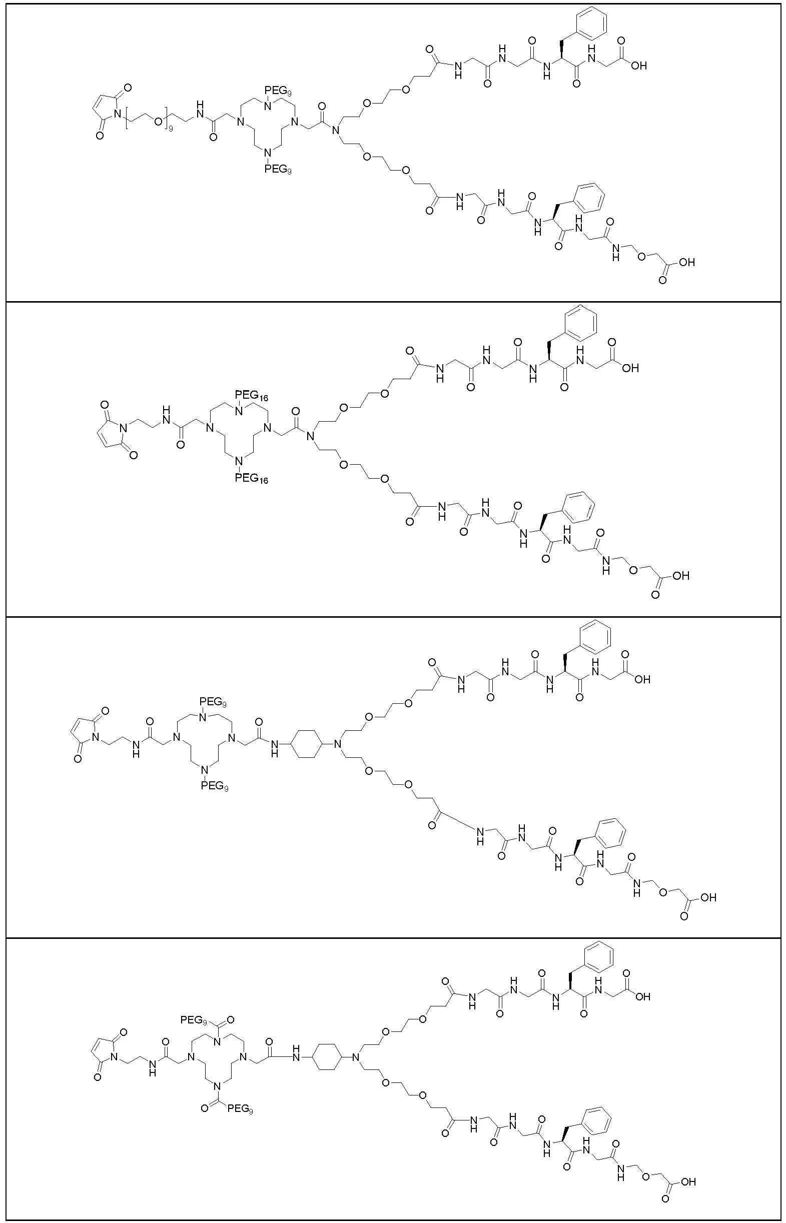 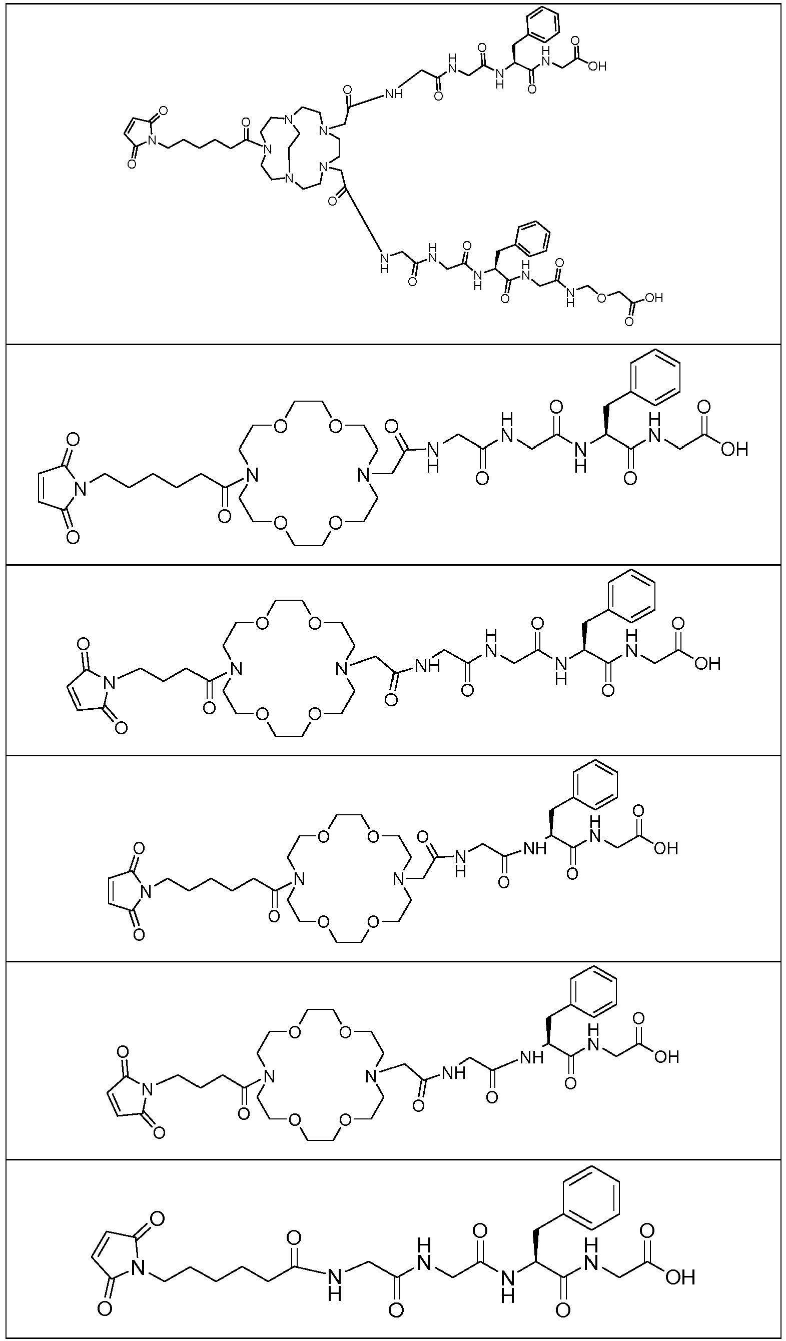 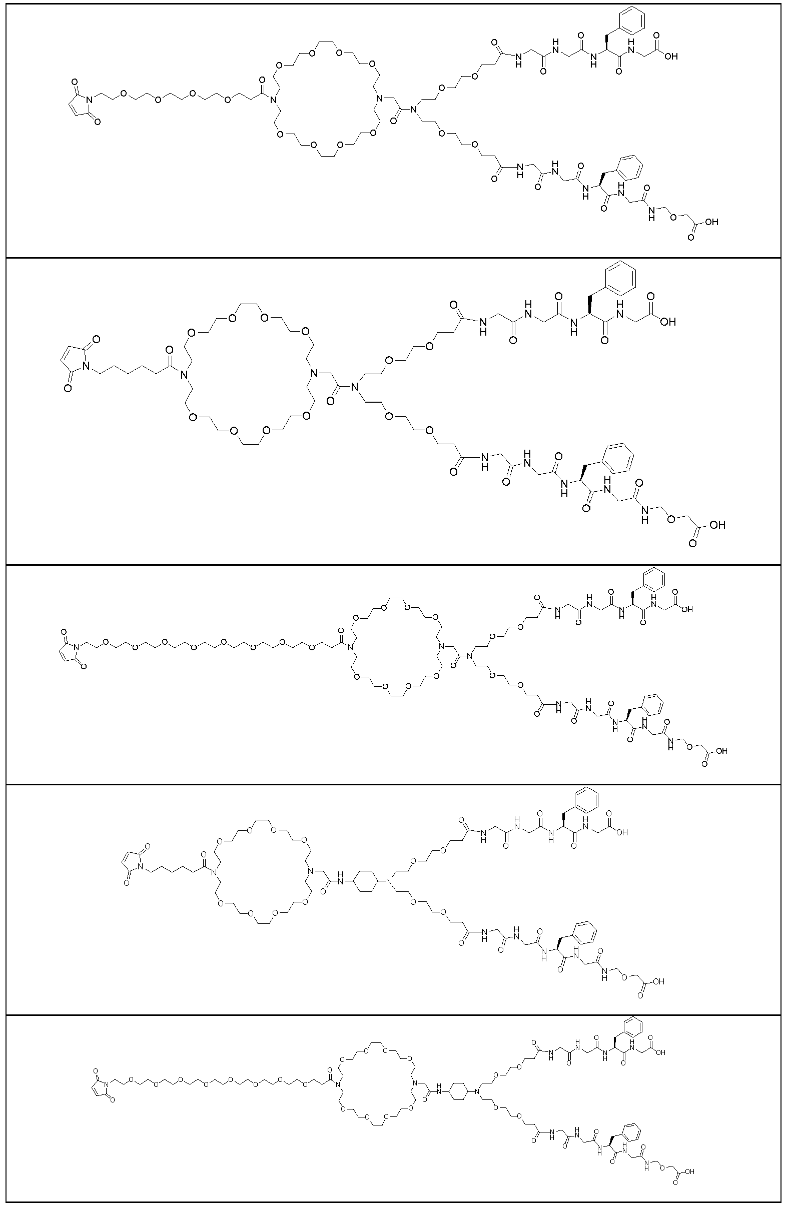 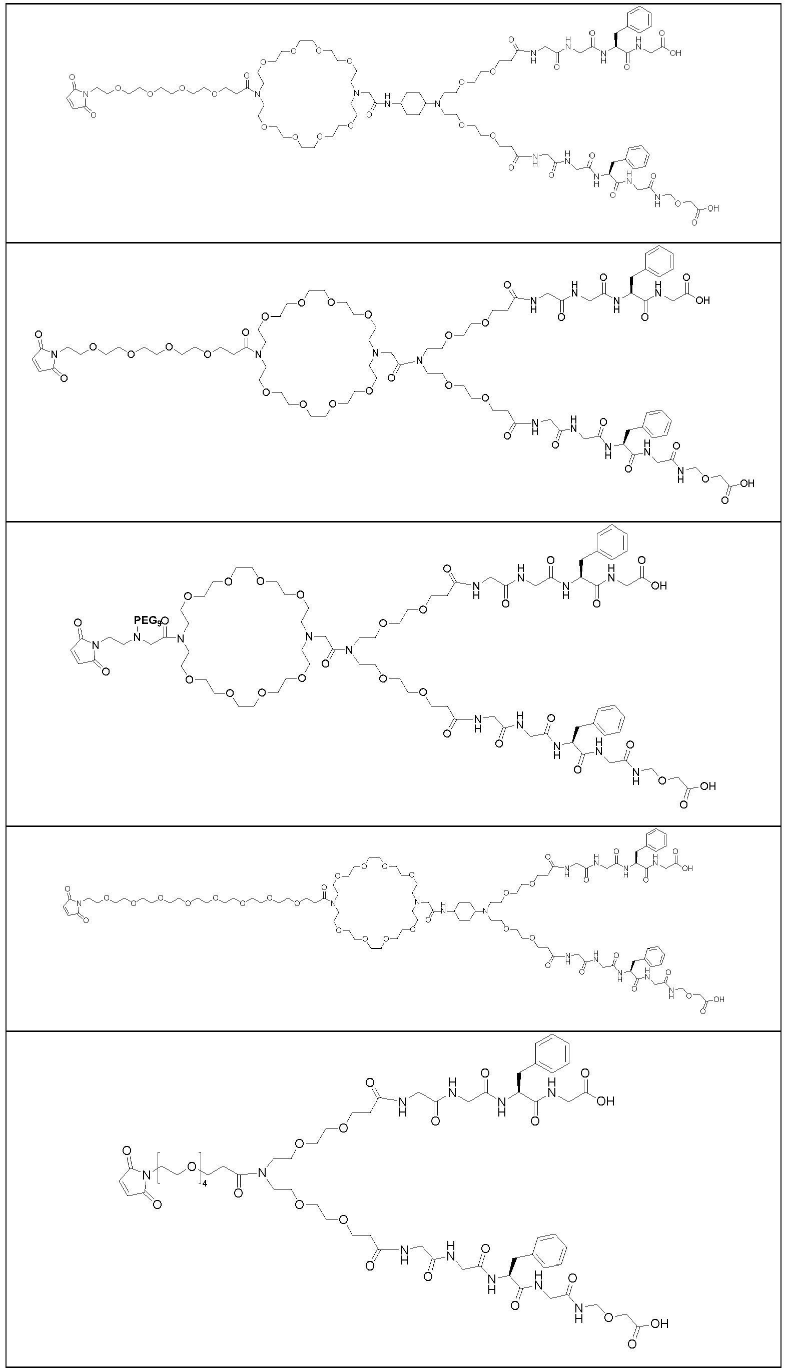 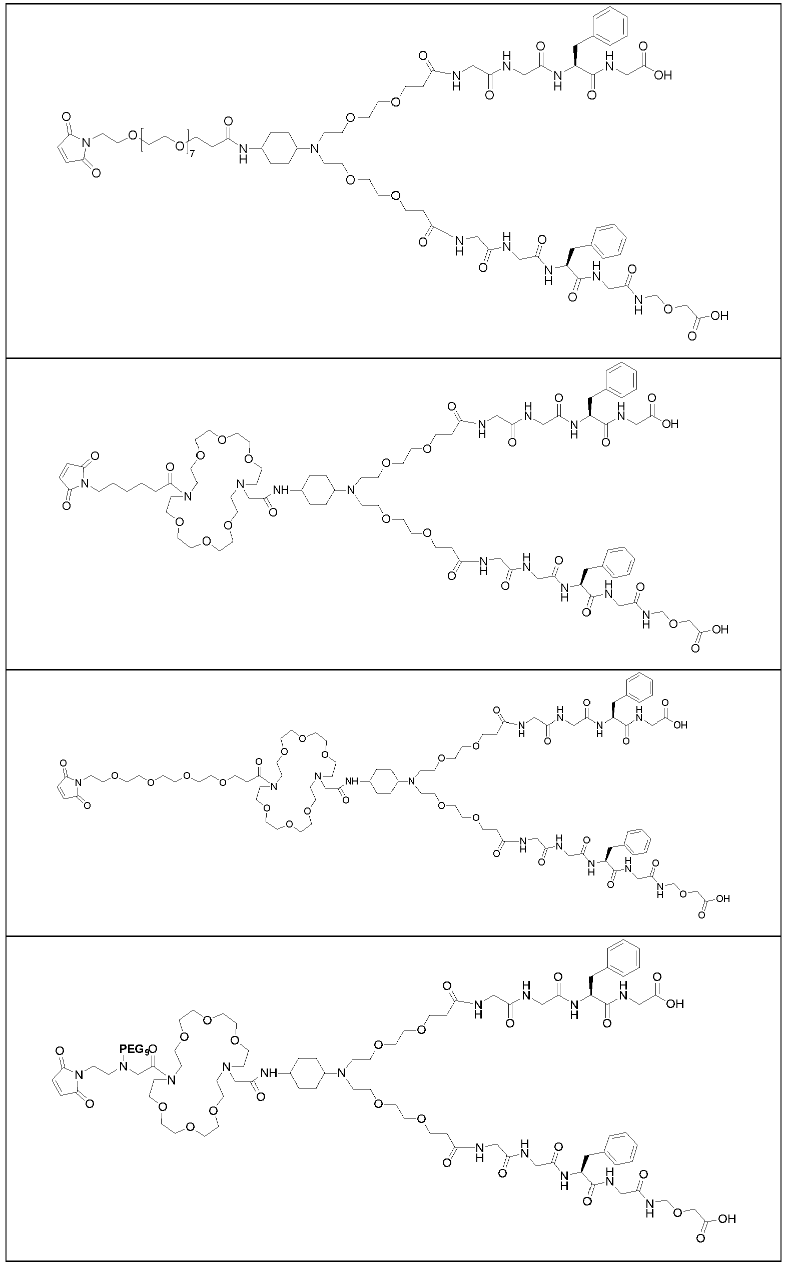 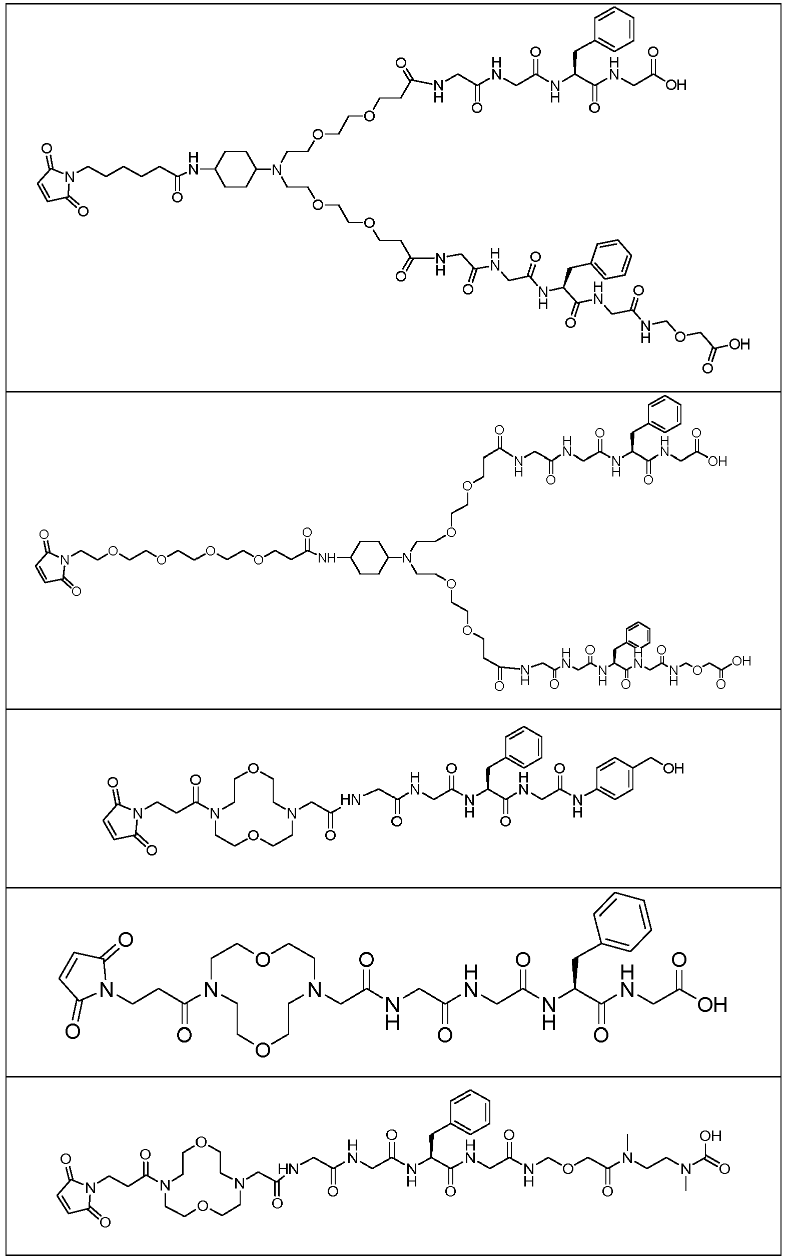 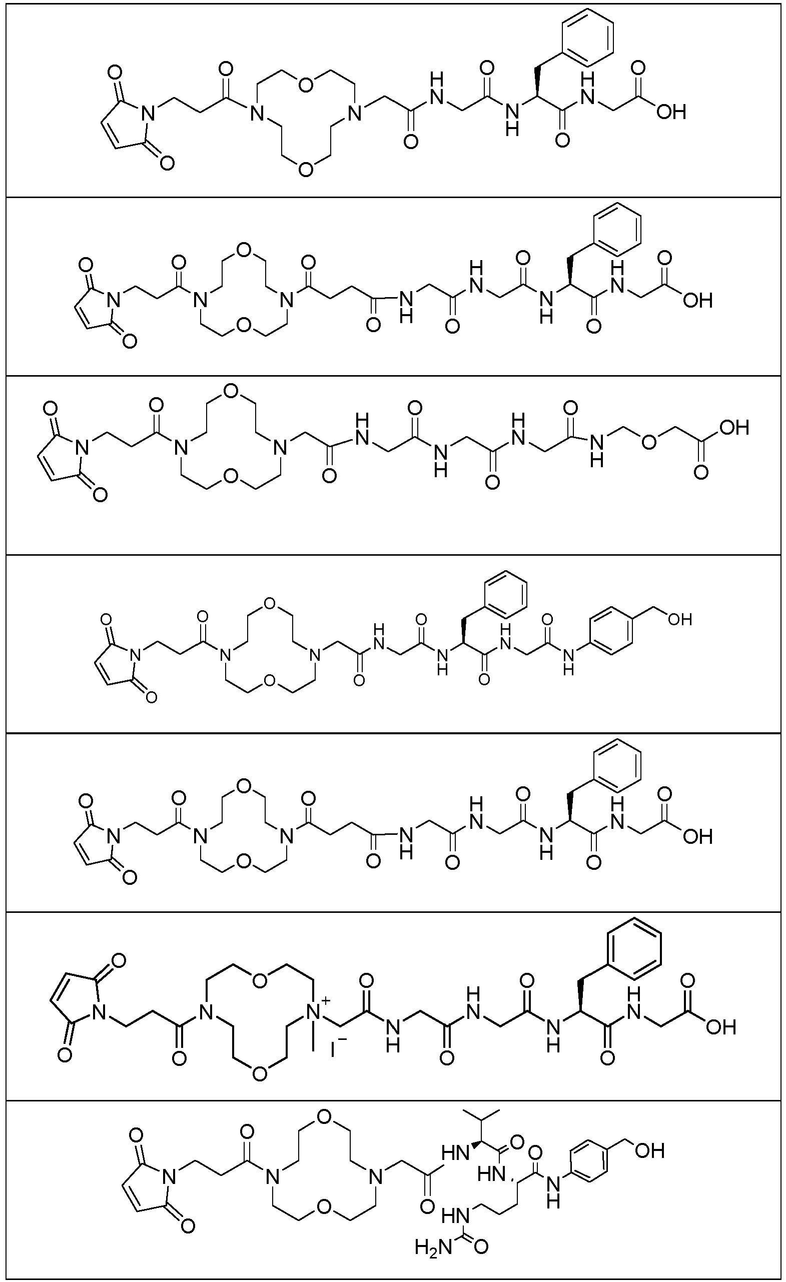 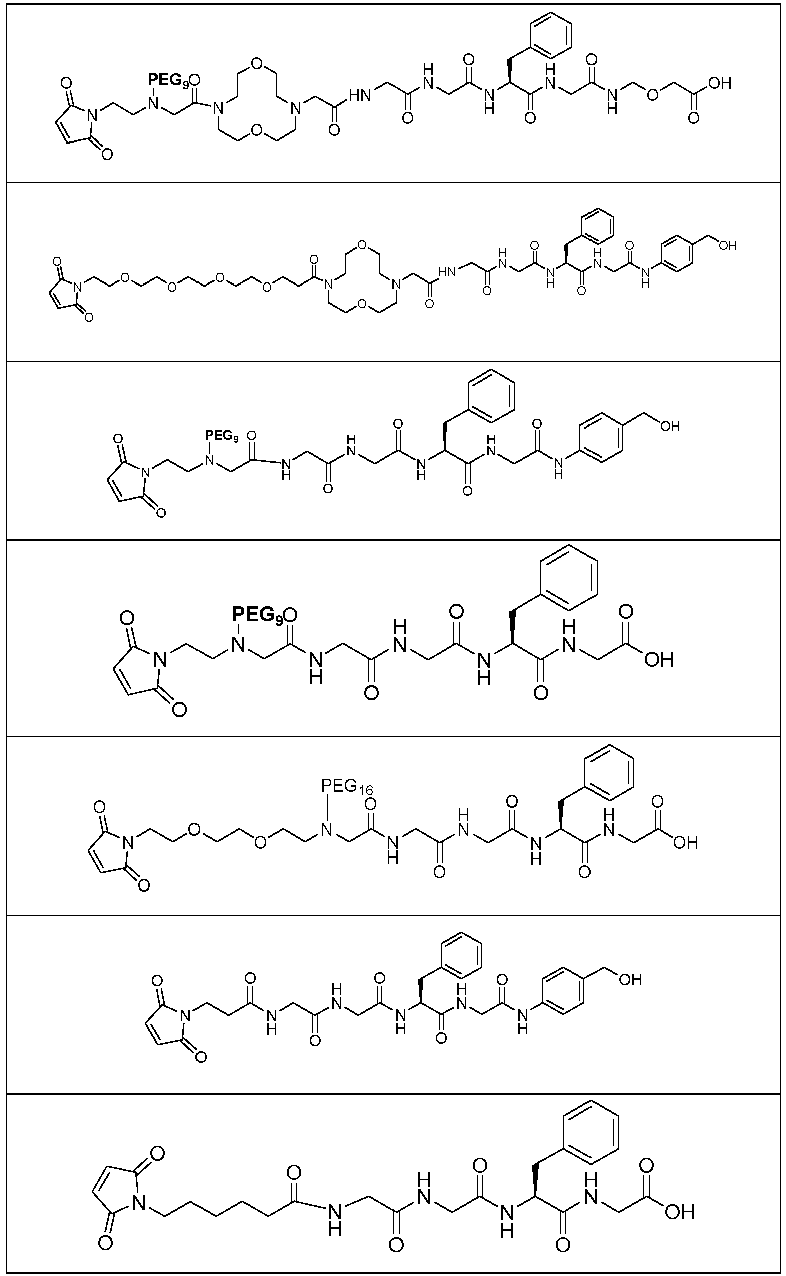 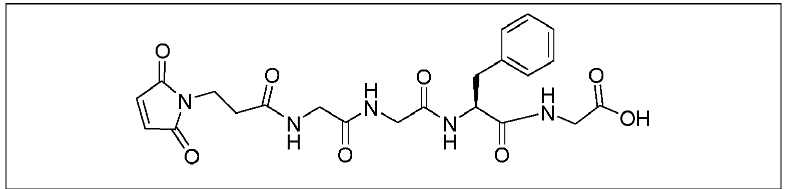
- PEG₉ represents 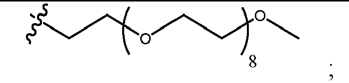 and -PEG₁₆ represents

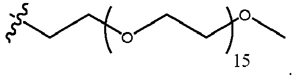

**52.** A drug formulation for preventing and/or treating a tumor, **characterized in that** the drug formulation is a formulation prepared from the targeted linker-drug conjugate or the stereoisomer, optical isomer, salt, or deuterated compound thereof according to any one of claims 37-41 as an active ingredient, and pharmaceutically acceptable accessories.

**53.** A use of the targeted linker-drug conjugate or the stereoisomer, optical isomer, salt, or deuterated compound thereof according to any one of claims 37-41 in preparation of a drug formulation for preventing and/or treating a tumor.

**54.** The use according to claim 53, **characterized in that** the tumor is selected from lung cancer, urethral cancer, large intestine cancer, prostate adenocarcinoma, ovarian cancer, pancreatic cancer, breast cancer, bladder cancer, stomach cancer, gastrointestinal stromal tumor, cervical cancer, esophageal cancer, squamous cell cancer, abdominal membrane cancer, liver cancer, colon cancer, rectal cancer, colorectal cancer, uterine cancer, salivary gland cancer, kidney cancer, vulva cancer, thyroid cancer, penis cancer, leukemia, malignant lymphoma, plasma cell tumor, myeloma, or sarcoma.

**55.** A method for preparing the targeted linker-drug conjugate or the stereoisomer, optical isomer, salt, or deuterated compound thereof according to any one of claims 37-41, **characterized in that** the method comprises the following steps:
(1) conjugating a drug with a linker to obtain a drug link assembly unit; and
(2) conjugating the drug link assembly unit with a targeted linker to obtain the targeted linker-drug conjugate; or the method comprises the following steps:
(1') conjugating a targeted linker with a linker to obtain a targeted linker-linker unit; and
(2') conjugating the targeted linker-linker unit with a drug to obtain the targeted linker-drug conjugate.
